# EUROPEAN PATENT APPLICATION

(11) **EP 3 865 579 A1**
(43) Date of publication of application: **18.08.2021**
(21) Application number: 20156987.8
(22) Date of filing: 12.02.2020
(51) Int. Cl.: C12N 15/67

(54) **RECOMBINANT NUCLEIC ACID CONSTRUCT AND USE THEREOF**

(71) Applicant: Pantherna Therapeutics GmbH, 16761 Hennigsdorf (DE)
(72) Inventor: The designation of the inventor has not yet been filed
(74) Representative: Bohmann, Armin K.

(57) **Abstract**

The present invention is related to a recombinant nucleic acid construct comprising in 5'-> 3' direction
- a 5' UTR,
- a coding region coding for an effector molecule, and
- a 3' UTR,
wherein the 5' UTR is selected from the group consisting of a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for Ang-2 or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group consisting of a 3' UTR of a gene coding for vWF or a derivative thereof having a nucleotide identity of at least 85 %, a 3' UTR of a gene coding for MCP-1 or a derivative thereof having a nucleotide identity of at least 85 %,, a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell, and
wherein the recombinant nucleic acid construct is different from a wild type mRNA coding for the effector molecule.

## Description

The present invention is related to a recombinant nucleic acid construct, a vector, preferably an expression vector, comprising a recombinant nucleic acid construct, a cell comprising the recombinant nucleic acid construct and/or the vector, a delivery vehicle comprising the recombinant nucleic acid construct, a pharmaceutical composition comprising the recombinant nucleic acid construct, the recombinant nucleic acid construct for use in a method for the treatment and/or prevention of a disease, the recombinant nucleic acid construct for use in a method for restoring a cellular function of a cell, a method for the treatment and/or prevention of a disease and a method for restoring a cellular function of a cell.

Nucleic acid molecules serve various purposes in biological systems. One out of several purposes is to store genetic information, another one is to convey genetic information from deoxyribonucleic acid to the ribosome where such genetic information is translated into an amino acid sequence. The latter step involves the use of messenger ribonucleic acid (mRNA).

mRNA shows a very basic design in eukaryotic cells and typically comprises, in 5'-> 3' direction, a Cap structure, a 5' untranslated region (5' UTR), a coding sequence typically starting with a AUG codon attached to a coding sequence (CDS) terminating with a stop codon, a 3' untranslated region (3' UTR) and a poly-A-tail.

mRNA based therapy have been proposed as therapeutics since the beginning of the biotech. In principle, the administered mRNA sequence can cause a cell to make a protein, which in turn could directly treat a disease or could function as a vaccine; more indirectly the protein could interfere with an element of a pathway in such way that the pathway is either inhibited or stimulated, thereby treating or ameliorating a disease.

The problem underlying the present invention is the provision of a recombinant nucleic acid construct, preferably of an mRNA, whereby such recombinant nucleic acid construct allows for high expression of the coding sequence of the recombinant nucleic acid construct.

Another problem underlying the present invention is the provision of a recombinant nucleic acid construct, preferably of an mRNA, whereby such recombinant nucleic acid construct allows for the expression of the coding sequence of the recombinant nucleic acid construct in an endothelial cell.

These and other problems underlying the present invention are solved by the subject matter of the attached independent claims; preferred embodiments may be taken from the attached dependent claims.

Additionally, and more specifically, the problem underlying the present invention is solved in a first aspect, which is also the first embodiment of the first aspect, by a recombinant nucleic acid construct comprising in 5'-> 3' direction
- a 5' UTR,
- a coding region coding for an effector molecule, and
- a 3' UTR,
wherein the 5' UTR is selected from the group comprising a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for Ang-2 or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group comprising a 3' UTR of a gene coding for vWF or a derivative thereof having a nucleotide identity of at least 85 %, a 3' UTR of a gene coding for MCP-1 or a derivative thereof having a nucleotide identity of at least 85 %, a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell,
wherein the effector molecule is a mutant effector molecule, wherein the mutant effector molecule is a gain of function mutant effector molecule and/or a hypermorphic mutant effector molecule, and
wherein the recombinant nucleic acid construct is different from a wild type mRNA coding for the effector molecule.

In a second embodiment of the first aspect, which is also an embodiment of the first embodiment of the first aspect, the 5' UTR and the 3' UTR of the recombinant construct are of different origin, preferably are from different endogenous species.

In a third embodiment of the first aspect, which is also an embodiment of the first and second embodiment of the first aspect, the 5' UTR and the 3' UTR of the recombinant construct are of the same origin, preferably are from the same endogenous genes species.

In a fourth embodiment of the first aspect, which is also an embodiment of the first, second and third embodiment of the first aspect,
a) the 5' UTR is a 5' UTR of a gene coding for MCP-1 or a derivative thereof having a nucleotide identity of at least 85 %, and wherein the 3' UTR is selected from the group comprising a 3' UTR of a gene coding for vWF or a derivative thereof having a nucleotide identity of at least 85 %, a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, and a 3' UTR of a gene coding for MCP-1 or a derivative thereof having a nucleotide identity of at least 85 %.
b) the 5' UTR is a 5' UTR of a gene coding for RPL12s.c. or a derivative thereof having a nucleotide identity of at least 85 %, and wherein the 3' UTR is selected from the group comprising a 3' UTR of a gene coding for vWF or a derivative thereof having a nucleotide identity of at least 85 %, a 3' UTR of a gene coding for HSP70 or a derivative thereof having a nucleotide identity of at least 85 %, a 3' UTR of a gene coding for RPL12s.c. or a derivative thereof having a nucleotide identity of at least 85 % and a 3' UTR of a gene coding for MCP-1 or a derivative thereof having a nucleotide identity of at least 85 %,
c) the 5' UTR is a 5' UTR of a gene coding for ANG-2 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL 12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %, or
d) the 5' UTR is a 5' UTR of a gene coding for HSP70 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL 12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %.

In a fifth embodiment of the first aspect, which is also an embodiment of the first, second and third embodiment of the first aspect,
a) the 3'UTR is a 3' UTR of a gene coding for vWF or a derivative thereof having a nucleotide identity of at least 85 % and the 5' UTR is selected from the group comprising a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for HSP70 or a derivative thereof having a nucleotide identity of at least 85 %, a 5' UTR of a gene coding for ANG-2 of a derivative thereof having a nucleotide identity of at least 85 %,
b) the 3'UTR is a 3' UTR of a gene coding for HSP70 or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
c) the 3'UTR is a 3' UTR of a gene coding for RPL12s.c. or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
   or
d) the 3'UTR is a 3' UTR of a gene coding for MCP-1 or a derivative thereof having a nucleotide identity of at least 85 % and the 5' UTR is selected from the group comprising a 5' UTR of a gene coding for HSP70 or a derivative thereof having a nucleotide identity of at least 85 %, a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %.

In a sixth embodiment of the first aspect, which is also an embodiment of the first, second, third, fourth and fifth embodiment of the first aspect, the construct is one selected from the group comprising
a construct, wherein the 5' UTR is a 5' UTR of a gene coding for MCP-1 or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for RPL12s.c. or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for MCP-1 or a derivative thereof having a nucleotide identity of at least 85 %, and the 3' UTR is a 3' UTR of a gene coding for MCP-1 or a derivative thereof having a nucleotide identity of at least 85 %,
a construct, wherein the 5' UTR is a 5' UTR of a gene coding for ANG-2 or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for RPL12s.c. or a derivative thereof having a nucleotide identity of at least 85 %, and the 3' UTR is a 3' UTR of a gene coding for RPL12s.c. or a derivative thereof having a nucleotide identity of at least 85 %,
a construct, wherein the 5' UTR is a 5' UTR of a gene coding for HSP70 or a derivative thereof having a nucleotide identity of at least 85 %, and the 3' UTR is a 3' UTR of a gene coding for HSP70 or a derivative thereof having a nucleotide identity of at least 85 %,
a construct, wherein the 5' UTR is a 5' UTR of a gene coding for H3.3. or a derivative thereof having a nucleotide identity of at least 85 %, and the 3' UTR is a 3' UTR of a gene coding for H3.3. or a derivative thereof having a nucleotide identity of at least 85 %, and
a construct, wherein the 5' UTR is a 5' UTR of a gene coding for Galectin 9 or a derivative thereof having a nucleotide identity of at least 85 %, and the 3' UTR is a 3' UTR of a gene coding for Galectin 9 or a derivative thereof having a nucleotide identity of at least 85 %.

In a seventh embodiment of the first aspect, which is also an embodiment of the first, second, third, fourth, fifth and sixth embodiment of the first aspect, the construct comprises a poly-A tail, preferably at the 3' terminal end of the recombinant nucleic acid construct.

In an eighth embodiment of the first aspect, which is also an embodiment of the first, second, third, fourth, fifth, sixth and seventh embodiment of the first aspect, the construct comprises a CAP structure, preferably at the 5' terminal end of the recombinant nucleic acid construct.

In a ninth embodiment of the first aspect, which is also an embodiment of the first, second, third, fourth, fifth, sixth, seventh and eighth embodiment of the first aspect, the construct comprises a IRES (internal ribosomal entry site) sequence, preferably at the 5' terminal end of the recombinant nucleic acid construct.

In a tenth embodiment of the first aspect, which is also an embodiment of the first, second, third, fourth, fifth, sixth, seventh, eighth and ninth embodiment of the first aspect, the construct comprises nucleic acid sequence coding for a signal peptide, preferably the signal peptide is in-frame with nucleic acid sequence coding for a signal peptide and is arranged between the 5' UTR and the coding region coding for an effector molecule.

In an eleventh embodiment of the first aspect, which is also an embodiment of the tenth embodiment of the first aspect, the signal peptide allows secretion of the effector molecule.

In a 12^{th} embodiment of the first aspect, which is also an embodiment of the tenth and the eleventh embodiment of the first aspect, the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %, a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %.

In a 13^{th} embodiment of the first aspect, which is also an embodiment of the first, second, third, fourth, fifth, sixth, seventh, eighth, ninth, tenth, eleventh and 12^{th} embodiment of the first aspect, the cell a cellular function of which is restored and/or the cell in or on which a therapeutic effect is exercised is an endothelial cell, preferably a vascular endothelial cell, more preferably the vascular endothelial cell is a microvascular endothelial cell.

In 14^{th} embodiment of the first aspect, which is also an embodiment of the first, second, third, fourth, fifth, sixth, seventh, eighth, ninth, tenth, eleventh, 12^{th} and 13^{th} embodiment of the first aspect, the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect, preferably the effect is linked to or associated with the Tie-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway.

More specifically, the problem underlying the present invention is solved in a second aspect, which is also a first embodiment of the second aspect, by a recombinant nucleic acid construct comprising in 5'-> 3' direction
- a 5' non-translated region,
- a coding region coding for an effector molecule, and
- a 3' non-translated region,
wherein the effector molecule is effective in restoring a cellular function of a cell and wherein the effector molecule is a mutant effector molecule, wherein the mutant effector molecule is a gain of function mutant effector molecule and/or a hypermorphic mutant effector molecule, for use in a method for the treatment and/or prevention of a disease, wherein the effector molecule is expressed by an endothelial cell. In an embodiment and as preferably used herein restoring a cellular function comprises and, respectively, encompasses killing of a cell, more preferably killing of tumor endothelial cells and/or tumor cells; in another embodiment and as preferably used herein, restoring cellular function comprises and, respectively, encompasses neoangiogenesis, more preferably neoangiogenesis in case of vascular diseases.

In a second embodiment of the second aspect which is also an embodiment of the first embodiment of the second aspect, the treatment and/or prevention of a disease involves restoration of the cellular function of a cell.

More specifically, the problem underlying the present invention is solved in a third aspect, which is also a first embodiment of the third aspect, by a recombinant nucleic acid construct comprising in 5'-> 3' direction
- a 5' non-translated region,
- a coding region coding for an effector molecule, and
- a 3' non-translated region,
wherein the effector molecule is effective in restoring a cellular function of a cell and wherein the effector molecule is a mutant effector molecule, wherein the mutant effector molecule is a gain of function mutant effector molecule and/or a hypermorphic mutant effector molecule, for use in a method for restoring the cellular function of a cell, wherein the effector molecule is expressed by an endothelial cell.

More specifically, the problem underlying the present invention is solved in a fourth aspect, which is also a first embodiment of the fourth aspect, by a recombinant nucleic acid construct comprising in 5'-> 3' direction
- a 5' non -translated region,
- a coding region coding for an effector molecule, and
- a 3' non-translated region,
wherein the effector molecule is effective in exercising a therapeutic effect in or on a cell and wherein the effector molecule is a mutant effector molecule, wherein the mutant effector molecule is a gain of function mutant effector molecule and/or a hypermorphic mutant effector molecule, for use in a method for the treatment and/or prevention of a disease, wherein the effector molecule is expressed by an endothelial cell.

In a second embodiment of the fourth aspect which is also an embodiment of the first embodiment of the fourth aspect, the treatment and/or prevention of a disease involves restoration of a cellular function of a cell.

More specifically, the problem underlying the present invention is solved in a fifth aspect, which is also a first embodiment of the fifth aspect, by a recombinant nucleic acid construct comprising in 5'-> 3' direction
- a 5' non -translated region,
- a coding region coding for an effector molecule, and
- a 3' non-translated region,
wherein the effector molecule is effective in exercising a therapeutic effect in or on a cell and wherein the effector molecule is a mutant effector molecule, wherein the mutant effector molecule is a gain of function mutant effector molecule and/or a hypermorphic mutant effector molecule, for use of in a method for restoring a cellular function of a cell, wherein the effector molecule is expressed by an endothelial cell.

In a third embodiment of the second aspect which is also an embodiment of the first and second embodiment of the second aspect, in a second embodiment of the third aspect which is also an embodiment of the first embodiment of the third aspect, in a third embodiment of the fourth aspect which is also an embodiment of the first and second embodiment of the fourth aspect, and in a second embodiment of the fifth aspect which is also an embodiment of the first embodiment of the fifth aspect, the effector molecule is expressed in the method by an endothelial cell.

In a fourth embodiment of the second aspect which is also an embodiment of the first, second and third embodiment of the second aspect, in a third embodiment of the third aspect which is also an embodiment of the first and the second embodiment of the third aspect, in a fourth embodiment of the fourth aspect which is also an embodiment of the first, second and third embodiment of the fourth aspect, and in a third embodiment of the fifth aspect which is also an embodiment of the first and second embodiment of the fifth aspect, the endothelial cell is a vascular endothelial cell, preferably the vascular endothelial cell is a microvascular endothelial cell.

In a fifth embodiment of the second aspect which is also an embodiment of the first, second, third and fourth embodiment of the second aspect, in a fourth embodiment of the third aspect which is also an embodiment of the first, second and third embodiment of the third aspect, in a fifth embodiment of the fourth aspect which is also an embodiment of the first, second, third and fourth embodiment of the fourth aspect, and in a fourth embodiment of the fifth aspect which is also an embodiment of the first, second and third embodiment of the fifth aspect, the endothelial cell is a non-dividing endothelial cell, a non-proliferating endothelial cell or a resting endothelial cell. In an embodiment thereof and as preferably used herein the endothelial is a vascular endothelial cell, a lymphatic endothelial cells, an endothelial stem cell (ESC) or an endothelial progenitor cells (EPC).

In a sixth embodiment of the second aspect which is also an embodiment of the first, second, third, fourth and fifth embodiment of the second aspect, in a fifth embodiment of the third aspect which is also an embodiment of the first, second, third and fourth embodiment of the third aspect, in a sixth embodiment of the fourth aspect which is also an embodiment of the first, second, third, fourth and fifth embodiment of the fourth aspect, and in a fifth embodiment of the fifth aspect which is also an embodiment of the first, second, third and fourth embodiment of the fifth aspect, the endothelial cell is a senescent endothelial cell.

In a seventh embodiment of the second aspect which is also an embodiment of the first, second, third, fourth, fifth and sixth embodiment of the second aspect, in a sixth embodiment of the third aspect which is also an embodiment of the first, second, third, fourth and fifth embodiment of the third aspect, in a seventh embodiment of the fourth aspect which is also an embodiment of the first, second, third, fourth, fifth and sixth embodiment of the fourth aspect, and in a sixth embodiment of the fifth aspect which is also an embodiment of the first, second, third, fourth and fifth embodiment of the fifth aspect, the endothelial cell is impaired by age and/or showing stress-related defects.

In an eighth embodiment of the second aspect which is also an embodiment of the first, second, third, fourth, fifth, sixth and seventh embodiment of the second aspect, in a seventh embodiment of the third aspect which is also an embodiment of the first, second, third, fourth, fifth and sixth embodiment of the third aspect, in an eighth embodiment of the fourth aspect which is also an embodiment of the first, second, third, fourth, fifth, sixth and seventh embodiment of the fourth aspect, and in a seventh embodiment of the fifth aspect which is also an embodiment of the first, second, third, fourth, fifth and sixth embodiment of the fifth aspect, the recombinant nucleic acid construct additionally comprises one element selected from the group comprising a cap structure, an IRES sequence, a further start codon providing sequence, a nucleic acid sequence coding for a signal peptide and a poly-A tail.

In a ninth embodiment of the second aspect which is also an embodiment of the first, second, third, fourth, fifth, sixth, seventh and eighth embodiment of the second aspect, in an eighth embodiment of the third aspect which is also an embodiment of the first, second, third, fourth, fifth, sixth and seventh embodiment of the third aspect, in a ninth embodiment of the fourth aspect which is also an embodiment of the first, second, third, fourth, fifth, sixth, seventh and eighth embodiment of the fourth aspect, and in an eighth embodiment of the fifth aspect which is also an embodiment of the first, second, third, fourth, fifth, sixth and seventh embodiment of the fifth aspect,
wherein the recombinant nucleic acid construct is one selected from the group comprising in 5'-> 3' direction
a)
   - a 5' non -translated region,
   - a coding region coding for an effector molecule,
   - a 3' non-translated region, and
   - a poly-A tail;
b)
   - a 5' non -translated region,
   - a further start codon providing sequence,
   - a coding region coding for an effector molecule,
   - a 3' non-translated region, and
   - a poly-A tail;
c)
   - a cap structure,
   - a 5' non -translated region,
   - a coding region coding for an effector molecule, and
   - a 3' non-translated region;
d)
   - a cap structure,
   - a 5' non -translated region,
   - a further start codon providing sequence,
   - a coding region coding for an effector molecule, and
   - a 3' non-translated region;
e)
   - an IRES sequence,
   - a 5' non -translated region,
   - a coding region coding for an effector molecule, and
   - a 3' non-translated region;
f)
   - an IRES sequence,
   - a 5' non -translated region,
   - a further start codon providing sequence,
   - a coding region coding for an effector molecule, and
   - a 3' non-translated region;
g)
   - a cap structure,
   - a 5' non -translated region,
   - a coding region coding for an effector molecule,
   - a 3' non-translated region, and
   - a poly-A tail;
h)
   - a cap structure,
   - a 5' non -translated region,
   - a further start codon providing sequence,
   - a coding region coding for an effector molecule,
   - a 3' non-translated region, and
   - a poly-A tail;
i)
   - an IRES sequence,
   - a 5' non -translated region,
   - a coding region coding for an effector molecule,
   - a 3' non-translated region, and
   - a poly-A tail;
j)
   - an IRES sequence,
   - a 5' non -translated region,
   - a further start codon providing sequence,
   - a coding region coding for an effector molecule,
   - a 3' non-translated region, and
   - a poly-A tail;
k)
   - a 5' non -translated region,
   - a nucleic acid sequence coding for a signal peptide,
   - a coding region coding for an effector molecule,
   - a 3' non-translated region, and
   - a poly-A tail;
l)
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail;
m)
   - a cap structure,
   - a 5' non -translated region,
   - a nucleic acid sequence coding for a signal peptide,
   - a coding region coding for an effector molecule, and
   - a 3' non-translated region;
   - a cap structure
   - a 5' non -translated region,
   - a further start codon providing sequence,
   - a nucleic acid sequence coding for a signal peptide,
   - a coding region coding for an effector molecule, and
   - a 3' non-translated region;
o)
   - an IRES sequence,
   - a 5' non -translated region,
   - a nucleic acid sequence coding for a signal peptide,
   - a coding region coding for an effector molecule, and
   - a 3' non-translated region;
p)
   - an IRES sequence,
   - a 5' non -translated region,
   - a further start codon providing sequence,
   - a nucleic acid sequence coding for a signal peptide,
   - a coding region coding for an effector molecule, and
   - a 3' non-translated region;
q)
   - a cap structure,
   - a 5' non -translated region,
   - a nucleic acid sequence coding for a signal peptide,
   - a coding region coding for an effector molecule,
   - a 3' non-translated region, and
   - a poly-A tail;
r)
   - a cap structure,
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail;
s)
   - an IRES sequence,
   - a 5' non -translated region,
   - a nucleic acid sequence coding for a signal peptide,
   - a coding region coding for an effector molecule,
   - a 3' non-translated region, and
   - a poly-A tail; and
t)
   - an IRES sequence,
   - a 5' non -translated region,
   - a further start codon providing sequence,
   - a nucleic acid sequence coding for a signal peptide,
   - a coding region coding for an effector molecule,
   - a 3' non-translated region, and
   - a poly-A tail.

In a tenth embodiment of the second aspect which is also an embodiment of the first, second, third, fourth, fifth, sixth, seventh, eighth and ninth embodiment of the second aspect, in an ninth embodiment of the third aspect which is also an embodiment of the first, second, third, fourth, fifth, sixth, seventh and eighth embodiment of the third aspect, in a tenth embodiment of the fourth aspect which is also an embodiment of the first, second, third, fourth, fifth, sixth, seventh, eighth and ninth embodiment of the fourth aspect, and in an ninth embodiment of the fifth aspect which is also an embodiment of the first, second, third, fourth, fifth, sixth, seventh and eighth embodiment of the fifth aspect, the recombinant nucleic acid comprises in 5' -> 3' direction
- a cap structure,
- a 5' non -translated region,
- a nucleic acid sequence coding for a signal peptide,
- a coding region coding for an effector molecule,
- a 3' non-translated region, and
- a poly-A tail.

In an eleventh embodiment of the second aspect which is also an embodiment of the first, second, third, fourth, fifth, sixth, seventh, eighth, ninth and tenth embodiment of the second aspect, in a tenth embodiment of the third aspect which is also an embodiment of the first, second, third, fourth, fifth, sixth, seventh, eighth and ninth embodiment of the third aspect, in an eleventh embodiment of the fourth aspect which is also an embodiment of the first, second, third, fourth, fifth, sixth, seventh, eighth, ninth and tenth embodiment of the fourth aspect, and in a tenth embodiment of the fifth aspect which is also an embodiment of the first, second, third, fourth, fifth, sixth, seventh, eighth and ninth embodiment of the fifth aspect, the recombinant nucleic acid comprises in 5' -> 3' direction
- an IRES sequence,
- a 5' non -translated region,
- a nucleic acid sequence coding for a signal peptide,
- a coding region coding for an effector molecule,
- a 3' non-translated region, and
- a poly-A tail.

In a 12th embodiment of the second aspect which is also an embodiment of the first, second, third, fourth, fifth, sixth, seventh, eighth, ninth, tenth and eleventh embodiment of the second aspect, in an eleventh embodiment of the third aspect which is also an embodiment of the first, second, third, fourth, fifth, sixth, seventh, eighth, ninth and tenth embodiment of the third aspect, in a 12^{th} embodiment of the fourth aspect which is also an embodiment of the first, second, third, fourth, fifth, sixth, seventh, eighth, ninth, tenth and eleventh embodiment of the fourth aspect, and in an eleventh embodiment of the fifth aspect which is also an embodiment of the first, second, third, fourth, fifth, sixth, seventh, eighth, ninth and tenth embodiment of the fifth aspect, recombinant nucleic acid comprises in 5' -> 3' direction
- a cap structure,
- a 5' non -translated region,
- a further start codon providing sequence,
- a nucleic acid sequence coding for a signal peptide,
- a coding region coding for an effector molecule,
- a 3' non-translated region, and
- a poly-A tail.

In a 13^{th} embodiment of the second aspect which is also an embodiment of the first, second, third, fourth, fifth, sixth, seventh, eighth, ninth, tenth, eleventh and 12^{th} embodiment of the second aspect, in a 12^{th} embodiment of the third aspect which is also an embodiment of the first, second, third, fourth, fifth, sixth, seventh, eighth, ninth, tenth and eleventh embodiment of the third aspect, in a 13^{th} embodiment of the fourth aspect which is also an embodiment of the first, second, third, fourth, fifth, sixth, seventh, eighth, ninth, tenth, eleventh and 12^{th} embodiment of the fourth aspect, and in an 12^{th} embodiment of the fifth aspect which is also an embodiment of the first, second, third, fourth, fifth, sixth, seventh, eighth, ninth, tenth and eleventh embodiment of the fifth aspect, the recombinant nucleic acid comprises in 5' -> 3' direction
- an IRES sequence,
- a 5' non -translated region,
- a further start codon providing sequence,
- a nucleic acid sequence coding for a signal peptide,
- a coding region coding for an effector molecule,
- a 3' non-translated region, and
- a poly-A tail.

In a 14^{th} embodiment of the second aspect which is also an embodiment of the first, second, third, fourth, fifth, sixth, seventh, eighth, ninth, tenth, eleventh, 12^{th} and 13^{th} embodiment of the second aspect, in a 13^{th} embodiment of the third aspect which is also an embodiment of the first, second, third, fourth, fifth, sixth, seventh, eighth, ninth, tenth, eleventh and 12^{th} embodiment of the third aspect, in a 14^{th} embodiment of the fourth aspect which is also an embodiment of the first, second, third, fourth, fifth, sixth, seventh, eighth, ninth, tenth, eleventh, 12^{th} and 13^{th} embodiment of the fourth aspect, and in an 13^{th} embodiment of the fifth aspect which is also an embodiment of the first, second, third, fourth, fifth, sixth, seventh, eighth, ninth, tenth, eleventh and 12^{th} embodiment of the fifth aspect, the further start codon providing sequence is in-frame with the coding region coding for an effector molecule.

In a 15^{th} embodiment of the second aspect which is also an embodiment of the first, second, third, fourth, fifth, sixth, seventh, eighth, ninth, tenth, eleventh, 12^{th}, 13^{th} and 14^{th} embodiment of the second aspect, in a 14^{th} embodiment of the third aspect which is also an embodiment of the first, second, third, fourth, fifth, sixth, seventh, eighth, ninth, tenth, eleventh, 12^{th} and 13^{th} embodiment of the third aspect, in a 15^{th} embodiment of the fourth aspect which is also an embodiment of the first, second, third, fourth, fifth, sixth, seventh, eighth, ninth, tenth, eleventh, 12^{th}, 13^{th} and 14^{th} embodiment of the fourth aspect, and in an 14^{th} embodiment of the fifth aspect which is also an embodiment of the first, second, third, fourth, fifth, sixth, seventh, eighth, ninth, tenth, eleventh 12^{th} and 13^{th} embodiment of the fifth aspect, the 5' non -translated region is a 5' UTR.

In a 16^{th} embodiment of the second aspect which is also an embodiment of the first, second, third, fourth, fifth, sixth, seventh, eighth, ninth, tenth, eleventh, 12^{th}, 13^{th}, 14^{th} and 15^{th} embodiment of the second aspect, in a 15^{th} embodiment of the third aspect which is also an embodiment of the first, second, third, fourth, fifth, sixth, seventh, eighth, ninth, tenth, eleventh, 12^{th}, 13^{th} and 14^{th} embodiment of the third aspect, in a 16^{th} embodiment of the fourth aspect which is also an embodiment of the first, second, third, fourth, fifth, sixth, seventh, eighth, ninth, tenth, eleventh, 12^{th}, 13^{th}, 14^{th} and 15^{th} embodiment of the fourth aspect, and in an 15^{th} embodiment of the fifth aspect which is also an embodiment of the first, second, third, fourth, fifth, sixth, seventh, eighth, ninth, tenth, eleventh 12^{th}, 13^{th} and 14^{th} embodiment of the fifth aspect, the 5' non-translated region is from a eukaryotic organism, preferably a mammalian organism, more preferably a mammalian organism selected from the group comprising human, non-human primate such as cynomolgus, chimpanzee rhesus monkey, rat and mouse. In an alternative embodiment the 5' non-translated region is from a viral gene, preferably the virus is a virus capable of infecting a eukaryotic organism.

In a 17^{th} embodiment of the second aspect which is also an embodiment of the first, second, third, fourth, fifth, sixth, seventh, eighth, ninth, tenth, eleventh, 12^{th}, 13^{th}, 14^{th}, 15^{th} and 16^{th} embodiment of the second aspect, in a 16^{th} embodiment of the third aspect which is also an embodiment of the first, second, third, fourth, fifth, sixth, seventh, eighth, ninth, tenth, eleventh, 12^{th}, 13^{th}, 14^{th} and 15^{th} embodiment of the third aspect, in a 17^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th} and 16^{th} embodiment of the fourth aspect, and in an 16^{th} embodiment of the fifth aspect which is also an embodiment of the first, second, third, fourth, fifth, sixth, seventh, eighth, ninth, tenth, eleventh 12^{th}, 13^{th}, 14^{th} and 15^{th} embodiment of the fifth aspect, the 5' UTR is selected from the group comprising a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for Ang-2 or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, and a 5' UTR of a gene coding for Galectin-9 or a derivative thereof having a nucleotide identity of at least 85 %.

In an 18^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th} and 17^{th} embodiment of the second aspect, in a 17^{th} embodiment of the third aspect which is also an embodiment of the first, second, third, fourth, fifth, sixth, seventh, eighth, ninth, tenth, eleventh, 12^{th}, 13^{th}, 14^{th}, 15^{th} and 16^{th} embodiment of the third aspect, in an 18^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} and 17^{th} embodiment of the fourth aspect, and in an 17^{th} embodiment of the fifth aspect which is also an embodiment of the first, second, third, fourth, fifth, sixth, seventh, eighth, ninth, tenth, eleventh 12^{th}, 13^{th}, 14^{th} and 15^{th}, 16^{th} embodiment of the fifth aspect, the 3' non-translated region is a 3'-UTR.

In a 19^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th} and 18^{th} embodiment of the second aspect, in an 18^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th} and 17^{th} embodiment of the third aspect, in a 19^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th} and 18^{th} embodiment of the fourth aspect, and in an 18^{th} embodiment of the fifth aspect which is also an embodiment of the first, second, third, fourth, fifth, sixth, seventh, eighth, ninth, tenth, eleventh 12^{th}, 13^{th}, 14^{th} and 15^{th}, 16^{th}, 17^{th} embodiment of the fifth aspect, the 3' non-translated region is from a eukaryotic organism, preferably a mammalian organism, more preferably a mammalian organism selected from the group comprising human, non-human primate such as cynomolgus, chimpanzee rhesus monkey, rat and mouse. In an alternative embodiment the 5' non-translated region is from a viral gene, preferably the virus is a virus capable of infecting a eukaryotic organism.

In a 20^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th} and 19^{th} embodiment of the second aspect, in a 19^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th} and 18^{th} embodiment of the third aspect, in a 20^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th} and 19^{th} embodiment of the fourth aspect, and in a 19^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th} and 18^{th} embodiment of the fifth aspect, the 3' UTR is selected from the group comprising a 3' UTR of a gene coding for vWF or a derivative thereof having a nucleotide identity of at least 85 %, a 3' UTR of a gene coding for MCP-1 or a derivative thereof having a nucleotide identity of at least 85 %, a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, and a 3' UTR of a gene coding for H3.3. or a derivative thereof having a nucleotide identity of at least 85 %.

In a 21^{st} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th} and 20^{th} embodiment of the second aspect, in a 20^{th} embodiment of the third aspect which is also an embodiment of the first, second, third, fourth, fifth, sixth, seventh, eighth, ninth, tenth, eleventh, 12^{th}, 3^{th}, 14^{th}, 15^{th}, 16^{th}, 17^{th}, 18^{th} and 19^{th} embodiment of the third aspect, in a 21^{st} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th} 19^{th} and 20^{th} embodiment of the fourth aspect, and in a 20^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th} and 19^{th} embodiment of the fifth aspect, the 5' UTR and 3' UTR of the recombinant nucleic acid construct are of different origin, preferably are from different species.

In a 22^{nd} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th} and 21^{st} embodiment of the second aspect, in a 21^{st} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th} and 20^{th} embodiment of the third aspect, in a 22^{nd} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th} 19^{th}, 20^{th} and 21^{st} embodiment of the fourth aspect, and in a 21^{st} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th} and 20^{th} embodiment of the fifth aspect, the 5' UTR and 3' UTR of the recombinant nucleic acid construct are of the same origin, preferably are from the same species.

In a 23^{rd} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st} and 22^{nd} embodiment of the second aspect, in a 22^{nd} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th} 20^{th} and 21^{st} embodiment of the third aspect, in a 23^{rd} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th} 19^{th} and 20^{th}, 21^{st} and 22^{nd} embodiment of the fourth aspect, and in a 22^{nd} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th} and 21^{st} embodiment of the fifth aspect,
a) the 5' UTR is a 5' UTR of a gene coding for MCP-1 or a derivative thereof having a nucleotide identity of at least 85 %, and wherein the 3' UTR is selected from the group comprising a 3' UTR of a gene coding for vWF or a derivative thereof having a nucleotide identity of at least 85 %, a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, and a 3' UTR of a gene coding for MCP-1 or a derivative thereof having a nucleotide identity of at least 85 %.
b) the 5' UTR is a 5' UTR of a gene coding for RPL12s.c. or a derivative thereof having a nucleotide identity of at least 85 %, and wherein the 3' UTR is selected from the group comprising a 3' UTR of a gene coding for vWF or a derivative thereof having a nucleotide identity of at least 85 %, a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, and a 3' UTR of a gene coding for MCP-1 or a derivative thereof having a nucleotide identity of at least 85 %,
c) the 5' UTR is a 5' UTR of a gene coding for HSP70 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL 12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %, or
d) the 5' UTR is a 5' UTR of a gene coding for ANG-2 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %.

In a 24^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd} embodiment of the second aspect, in a 23^{rd} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st} and 22^{nd} embodiment of the third aspect, in a 24^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd} embodiment of the fourth aspect, and in a 23^{rd} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st} and 22^{nd} embodiment of the fifth aspect,
a) 3'UTR is a 3' UTR of a gene coding for vWF or a derivative thereof having a nucleotide identity of at least 85 % and the 5' UTR is selected from the group comprising a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for HSP70 or a derivative thereof having a nucleotide identity of at least 85 %, and a 5' UTR of a gene coding for ANG-2 of a derivative thereof having a nucleotide identity of at least 85 %, and,
b) 3'UTR is a 3' UTR of a gene coding for HSP70 or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
c) 3'UTR is a 3' UTR of a gene coding for RPL12s.c. or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %,
   or
d) 3'UTR is a 3' UTR of a gene coding for MCP-1 or a derivative thereof having a nucleotide identity of at least 85 % and the 5' UTR is selected from the group comprising a 5' UTR of a gene coding for HSP70 or a derivative thereof having a nucleotide identity of at least 85 %, a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %.

In a 25^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd} and 24^{th} embodiment of the second aspect, in a 24^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th},^{,} 21^{st}, 22^{nd} and 23^{rd} embodiment of the third aspect, in a 25^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} 23^{rd} and 24^{th} embodiment of the fourth aspect, and in a 24th embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd} and 23^{rd} embodiment of the fifth aspect, the construct is one selected from the group comprising
a construct, wherein the 5' UTR is a 5' UTR of a gene coding for MCP-1 or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for RPL12s.c. or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for MCP-1 or a derivative thereof having a nucleotide identity of at least 85 %, and the 3' UTR is a 3' UTR of a gene coding for MCP-1 or a derivative thereof having a nucleotide identity of at least 85 %,
a construct, wherein the 5' UTR is a 5' UTR of a gene coding for ANG-2 or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for RPL12s.c. or a derivative thereof having a nucleotide identity of at least 85 %, and the 3' UTR is a 3' UTR of a gene coding for RPL12s.c. or a derivative thereof having a nucleotide identity of at least 85 %,
a construct, wherein the 5' UTR is a 5' UTR of a gene coding for HSP70 or a derivative thereof having a nucleotide identity of at least 85 %, and the 3' UTR is a 3' UTR of a gene coding for HSP70 or a derivative thereof having a nucleotide identity of at least 85 %,
a construct, wherein the 5' UTR is a 5' UTR of a gene coding for H3.3. or a derivative thereof having a nucleotide identity of at least 85 %, and the 3' UTR is a 3' UTR of a gene coding for H3.3. or a derivative thereof having a nucleotide identity of at least 85 %, or
a construct, wherein the 5' UTR is a 5' UTR of a gene coding for Galectin 9 or a derivative thereof having a nucleotide identity of at least 85 %, and the 3' UTR is a 3' UTR of a gene coding for Galectin 9 or a derivative thereof having a nucleotide identity of at least 85 %.

In a 26^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th} and 25^{th} embodiment of the second aspect, in a 25^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd} and 24^{th} embodiment of the third aspect, in a 26^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, and 23^{rd}, 24^{th} and 25^{th} embodiment of the fourth aspect, and in a 25^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st} 22^{nd}, 23^{rd} and 24^{th} embodiment of the fifth aspect, the construct comprises a poly-A tail.

In a 27^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th} and 26^{th} embodiment of the second aspect, in a 26^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th} and 25^{th} embodiment of the third aspect, in a 27^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th} and 26^{th} embodiment of the fourth aspect, and in a 26^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd}, 23^{rd}, 24^{th} and 25^{th} embodiment of the fifth aspect, the construct comprises CAP structure.

In a 28^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd}, 24^{th}, 25^{th}, 26^{th} and 27^{th} embodiment of the second aspect, in a 27^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th} and 26^{th} embodiment of the third aspect, in a 28th embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th} and 27^{th} embodiment of the fourth aspect, and in a 27th embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd}, 23^{rd}, 24^{th}, 25^{th} and 26^{th} embodiment of the fifth aspect, the construct comprises one or more IRESs (internal ribosomal entry sites) sequences. In a preferred embodiment, the IRES sequences replaced the Cap structure.

In a 29^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd}, 24^{th}, 25^{th}, 26^{th}, 27^{th} and 28^{th} embodiment of the second aspect, in a 28^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th} and 27^{th} embodiment of the third aspect, in a 29^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th}, 19^{th}, 20^{th}, 21^{st}, 22^{nd}, 23^{rd}, 24^{th}, 25^{th}, 26^{th}, 27^{th} and 28^{th} embodiment of the fourth aspect, and in a 28^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd}, 23^{rd}, 24^{th}, 25^{th}, 26^{th} and 27^{th} embodiment of the fifth aspect, the construct comprises nucleic acid sequence coding for a signal peptide.

In a 30^{th} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd}, 24^{th}, 25^{th}, 26^{th}, 27^{th}, 28^{th} and 29^{th} embodiment of the second aspect, in a 29^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27tz and 28^{th} embodiment of the third aspect, in a 30^{th} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th}, 19^{th}, 20^{th}, 21^{st}, 22^{nd}, 23^{rd}, 24^{th}, 25^{th}, 26^{th}, 27^{th} 28^{th} and 29^{th} embodiment of the fourth aspect, and in a 29^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st}, 22^{nd}, 23^{rd}, 24^{th}, 25^{th} 26^{th}, 27^{th} and 28^{th} embodiment of the fifth aspect, the signal peptide allows secretion of the effector molecule.

In a 31^{st} embodiment of the second aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd} and 23^{rd}, 24^{th}, 25^{th}, 26^{th}, 27^{th}, 28^{th}, 29^{th} and 30^{th} embodiment of the second aspect, in a 30^{th} embodiment of the third aspect which is also an embodiment of the first, second, third, fourth, fifth, sixth, seventh, eighth, ninth, tenth, eleventh, 12^{th}, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th}, 27^{th}, 28^{th} and 29^{th} embodiment of the third aspect, in a 31^{st} embodiment of the fourth aspect which is also an embodiment of the first, second, third, fourth, fifth, sixth, seventh, eighth, ninth, tenth, eleventh, 12^{th}, 13^{th}, 14^{th}, 15^{th}, 16^{th} , 17^{th}, 18^{th} , 19^{th}, 20^{th}, 21^{st}, 22^{nd}, 23^{rd}, 24^{th}, 25^{th}, 26^{th}, 27^{th}, 28^{th}, 29^{th} and 30^{th} embodiment of the fourth aspect, and in a 30^{th} embodiment of the fifth aspect which is also an embodiment of the first, second, third, fourth, fifth, sixth, seventh, eighth, ninth, tenth, eleventh 12^{th}, 13^{th}, 14^{th} 15^{th}, 16^{th}, 17^{th}, 18^{th}, 19^{th}, 20^{th}, 21^{st} 22^{nd}, 23^{rd}, 24^{th}, 25^{th}, 26^{th}, 27^{th}, 28^{th} and 29^{th} embodiment of the fifth aspect, the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %, , a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %.

In an embodiment of the first, second, third, fourth and fifth aspect, including any embodiment thereof, the cell a cellular function of which is restored and/or the cell in or on which a therapeutic effect is exercised is an endothelial cell, preferably a vascular endothelial cell, more preferably the vascular endothelial cell is a microvascular endothelial cell.

In an embodiment of the first, second, third, fourth and fifth aspect, including any embodiment thereof, the endothelial cell is a non-dividing endothelial cell, a non-proliferating endothelial cell or a resting endothelial cell.

In an embodiment of the first, second, third, fourth and fifth aspect, including any embodiment thereof, the endothelial cell is an endothelial cell stimulated by an inflammatory stimulus.

In an embodiment of the first, second, third, fourth and fifth aspect, including any embodiment thereof, the endothelial cell is a senescent endothelial cell.

In an embodiment of the first, second, third, fourth and fifth aspect, including any embodiment thereof, the endothelial cell is impaired by age and/or showing stress-related defects.

In an embodiment of the first, second, third, fourth and fifth aspect, including any embodiment thereof, the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect. In an embodiment thereof, the effector molecule stimulates vessel survival, inhibits regression, inhibits apoptosis, stimulates migration, stimulates remodelling, stimulates angiogenesis, stimulates tube-formation/invasion, stimulates proliferation, inhibits leucocyte adhesion, inhibits adhesion molecule expression, inhibits tissue factor expression, inhibits NFKappaB activity or promotes monolayer integrity.

In an embodiment of the first, second, third, fourth and fifth aspect, including any embodiment thereof, the effect is linked to or associated with the Tie-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway, preferably the pathway is selected from the group comprising Ang/Tie-2 signalling pathway, VEGF/VEGF receptor pathway and Notch/Notch ligand pathway.

In an embodiment of the first, second, third, fourth and fifth aspect, including any embodiment thereof, the effector molecule is an element of a pathway, wherein the pathway is selected from the group comprising the Tie-2 signalling pathway, VEGF-Receptor pathway, NOTCH signalling pathway, PI3-kinase pathway, eNOS signalling pathway, sirtuin-dependent metabolic and energy homeostasis pathway, oxidative stress pathway, shear stress response pathway ET-1 signal transduction pathway, NO-mediated signal transduction pathway, and mechanochemical transduction pathway.

In an embodiment of the first, second, third, fourth and fifth aspect, including any embodiment thereof, the effector molecule is selected from the group comprising Ang-1, Ang-4, COMP-Ang-1, hCOMP-Ang-1, COMP-Ang-1, COMP-ANG-2, Tie-2 receptor, Tie-1 receptor, PI3-kinase, preferably constitutive active PI3-kinase, hyperactive Tie-2 receptor (e.g. R849W), VE-cadherin, GRB2, GRB7, GRB14, GRB7, IQGAP1, RAC1, RAP1, DOK2, ABIN1 and ABIN2, KLF2 (Krueppel-like factor 2), alpha5 beta1 integrin, CD73, Akt 1, Akt 2 and Akt 3.

In an embodiment of the first, second, third, fourth and fifth aspect, including any embodiment thereof, the cellular function is vascular leakage and, preferably, the pathway is the Tie-2 pathway.

In an embodiment of the first, second, third, fourth and fifth aspect, including any embodiment thereof, the effector molecule is selected from the group comprising VEGF, VEGF-A, VEGF-B, PDGF, bFGF, Sirtuin, eNOS, RAS and c-MYC.

In an embodiment of the first, second, third, fourth and fifth aspect, including any embodiment thereof, wherein the effector molecule restores vascular regeneration or provides for vascular regeneration, preferably vascular endothelial vascular regeneration

In an embodiment of the first, second, third, fourth and fifth aspect, including any embodiment thereof, the cell a cellular function of which is restored and/or the cell in or on which a therapeutic effect is exercised is a non-endothelial cell.

In an embodiment of the first, second, third, fourth and fifth aspect, including any embodiment thereof, the non-endothelial cell is selected from the group comprising epithelial cells, alveolar macrophages, Alveolar type I cells, alveolar type II cells, alveolar macrophages, Pneumocytes, lung epithelial cells, hematopoietic cells, bone marrow cells, bone cells, stem cells, mesenchymal cells, neural cells, glia cells, neuron cells, Astrocytes, cells of the peripheral nervous system, cardiac cells, adipocytes, vascular smooth muscle cells, cardiomyocytes, skeletal muscle cells, beta cells, pituitary cells, synovial lining cells, ovarian cells, testicular cells, B cells, T cells, reticulocytes, leukocytes, granulocytes, macrophages, neutrophils, antigen presenting cells (dendritic cells), fibroblasts, hepatocytes, tumor cells and combination thereof.

In an embodiment of the first, second, third, fourth and fifth aspect, including any embodiment thereof, the effector molecule is selected from the group comprising a tumor death ligand, a factor involved in hematopoiesis and blood clotting, a stem cell factor, a growth factor and a cytokine.

In an embodiment of the first, second, third, fourth and fifth aspect, including any embodiment thereof, the tumor death ligand is selected from the group comprising IL-12, members of the TNF gene superfamily of death ligand proteins such as Apo2L/TRAIL, IL-13, IL-10, IL-8, IL-2, interferon beta, secreted frizzled-related protein (SFRP) 1, SFRP 2, SFRP 3, SFRP 4 and SFRP5.

In an embodiment of the first, second, third, fourth and fifth aspect, including any embodiment thereof, wherein the factor involved in hematopoiesis and blood clotting is selected from the group comprising erythropoietin, Factor VII, Factor VIII, Factor IX and heparan-N-sulfatase.

In an embodiment of the first, second, third, fourth and fifth aspect, including any embodiment thereof, the stem cell factor is selected from the group comprising Oct4, Sox2, Klf4 and c-Myc.

In an embodiment of the first, second, third, fourth and fifth aspect, including any embodiment thereof, the growth factor and cytokine is selected from the group comprising adrenomedullin, angiopoietin family, autocrine motility factor, bone morphogenetic proteins, ciliary neurotrophic factor (CNTF), leukemia inhibitory factor (LIF), interleukin-6 (IL-6), macrophage colony-stimulating factor (m-CSF), granulocyte colony-stimulating factor (G-CSF), granulocyte macrophage colony-stimulating factor (GM-CSF), epidermal growth fac-tor (EGF), ephrins, erythropoietin (EPO), fibroblast growth factor family (FGF1-23), somatotropin, GDNF family of ligands (such as glial cell line-derived neurotrophic factor (GDNF), neurturin, persephin, and artemin), growth differentiation factor-9 (GDF9), hepatocyte growth factor (HGF), hepatoma-derived growth factor (HDGF), insulin, insulin-like growth factors -1 and-2 (IGF-1; IGF-2), keratinocyte growth factor (KGF), migration-stimulating factor (MSF), macrophage-stimulating protein (MSP), also known as hepatocyte growth factor-like protein (HGFLP), myostatin (GDF-8), neuregulins 1-4 (NRG1-4), neurotrophins (brain-derived neurotrophic factor (BDNF), nerve growth factor (NGF), neurotrophin-3 (NT-3), neurotrophin-4 (NT-4)), placental growth factor (PGF), platelet-derived growth factor (PDGF), renalase (RNLS) - anti-apoptotic survival factor, T-cell growth factor (TCGF), thrombopoietin (TPO), transforming growth factor alpha (TGF-α), transforming growth factor beta (TGF-β), tumor necrosis factor-alpha (TNF-α), vascular endothelial growth factor family (VEGF), Wnt Signaling Pathway signaling glycoproteins (WNT1, WNT2, WNT2B, WNT3, WNT3A, WNT4, WNT5A, WNT5B, WNT6, WNT7A, WNT7B, WNT8A, WNT8B, WNT9A, WNT9B, WNT10A, WNT10B, WNT11, WNT16), granulocyte colony-stimulating factor (G-CSF), granulocyte macrophage colony-stimulating factor (GM-CSF), interferon- alfa, interferon-beta, interleukin 2 (IL-2), Interleukin 11 (IL-11), Interferon-gamma, IL-1, IL-3, IL-4, IL-5, IL-6, IL-7, IL-8, IL-9, IL-10, IL-12, IL-13, IL-14, IL-15, IL-16, IL-17, IL-18, IL-19, IL-20, IL-21, IL-22, IL-23,IL-24, IL-25, IL-26, IL-27, IL-28, IL-29, IL-30, IL-31, IL-32, IL-33, IL-35 and IL-36.

In an embodiment of the first, second, third, fourth and fifth aspect, including any embodiment thereof, the disease is characterized by or caused by vascular leakage, preferably leakage of vascular endothelial cells, more preferably leakage of micro vascular endothelial cells.

In an embodiment of the first, second, third, fourth and fifth aspect, including any embodiment thereof, a subject suffering from the disease shows inflammation in the lung.

In an embodiment of the first, second, third, fourth and fifth aspect, including any embodiment thereof, the disease wherein the disease is selected from the group comprising pneumonia, sepsis and trauma.

In an embodiment of the first, second, third, fourth and fifth aspect, including any embodiment thereof, a subject suffering from the disease shows acute respiratory distress syndrome (ARDS).

In an embodiment of the first, second, third, fourth and fifth aspect, including any embodiment thereof, disease is pneumonia, preferably pneumonia selected from the group comprising severe community-acquired pneumonia (sCAP), community acquired pneumonia (CAP), hospital-acquired pneumonia (HAP).

In an embodiment of the first, second, third, fourth and fifth aspect, including any embodiment thereof, the disease is characterized by or shows lung damage.

In an embodiment of the first, second, third, fourth and fifth aspect, including any embodiment thereof, lung damage is immediate lung damage.

In an embodiment of the first, second, third, fourth and fifth aspect, including any embodiment thereof, immediate lung damage results from inhalation of toxic gases, toxic lung edema, lung infection of a virus or bacterium, aspiration of stomach contents, aspiration of fresh water, aspiration of salt water, pulmonary contusion, fat embolism, amniotic fluid embolism and inhalation of hyperbaric oxygen.

In an embodiment of the first, second, third, fourth and fifth aspect, including any embodiment thereof, lung damage is indirect lung damage.

In an embodiment of the first, second, third, fourth and fifth aspect, including any embodiment thereof, indirect lung damage results from sepsis, bacteremia, endotoxinemia, severe trauma, polytrauma including shock, burns, pancreatitis, malaria tropica, drugs and immunosuppression, chronic alcohol abuse, chronic pulmonary diseases, low pH of serum.

In an embodiment of the first, second, third, fourth and fifth aspect, including any embodiment thereof, the disease or effect is vascular regeneration or longevity, wherein preferably the effector molecule is selected from the group comprising VEGF, VEGF-A, VEGF-B, PDGF, bFGF, Sirtuin (Sirt 1), PPAR-gamma, AMPK, eNOS, RAS, c-MYC, FOXO3, PI3 kinase *, Akt, Akt* (* indicates fusion or truncated versions of the proteins to generate hyperactive and constitutively active derivatives of the kinases), adenosine A1 receptor, adenosine A2A receptor, adenosine A2B receptor, telomerase, Yamanaka factors (namely Oct4, sox2, klf4, c-My).

In an embodiment of the first, second, third, fourth and fifth aspect, including any embodiment thereof, the effector molecule is an antiapoptotic factor, preferably protecting normal, i.e. non-diseased endothelium, wherein the antiapoptotic factor is preferably selected from the group comprising PTEN, survivin, IAP, cIAP2 and XIAP, neuroglobin (binds cytochrome c), prosurvival pro-teins Bcl-2, Bcl-xl, Bcl-w, mcl-1 A1, NR-13, BHRF1, LMW5-HL, ORF16, KS-Bcl-2, E1b-19K and P53.

In an embodiment of the first, second, third, fourth and fifth aspect, including any embodiment thereof, the effector molecule is an apoptotic factor, preferably targeting tumor endothelium, wherein the apoptotic factor is preferably selected from the group comprising PTEN, survivin, IAP, cIAP2 and XIAP, neuroglobin (binds cytochrome c), prosurvival pro-teins Bcl-2, Bcl-xl, Bcl-w, mcl-1 A1, NR-13, BHRF1, LMW5-HL, ORF16, KS-BCl-2, E1b-19K, P53.

In an embodiment of the first, second, third, fourth and fifth aspect, including any embodiment thereof, the effector molecule is a tumor death ligand, preferably the tumor death ligand is proapoptotic or anti-angiogenic, more preferably the tumor death ligand is selected from the group comprising Members of the TNF gene superfamily of death ligand proteins such as TNF-alpha, Fas-L, Apo2L/TRAIL, IL-12, IL-13, IL-10, IL-8, IL-2, , secreted frizzled-related protein (SFRP) family (SFRP1, 2, 3, 4, 5), ELTD1, TGF-β, TNF-a, IL-6, PTEN, p53 and other tumor suppressor genes with proapoptotic function, thrombospondin C, TIMP-1, TIMP-2, interferon-alpha, interferon-beta, interferon-gamma, Ang-1 and derivatives with Tie-2 agonistic function, cytochrome c, BH3-only proteins, pro-apoptotic Bcl-2 proteins Bad, Bid, Bax, Bak and Bim, Apaf1, procaspase-8, -9 -10, caspases 2, 3, 6, 7, 8, 9 and 10.

In an embodiment of the first, second, third, fourth and fifth aspect, including any embodiment thereof, the effector molecule is anti-inflammatory, preferably the effector molecule is selected from the group comprising IkappaB and derivative with mutated phosphorylation sites, PI3 kinase and Akt kinases and hyperactive forms.

In an embodiment of the first, second, third, fourth and fifth aspect, including any embodiment thereof, the effector molecule is active in blood coagulation and/or hematopoiesis, preferably the effector molecule is selected from the group comprising erythropoietin, factor VII, factor VIII, factor IX and heparan-N-sulfatase.

In an embodiment of the first, second, third, fourth and fifth aspect, including any embodiment thereof, the effector molecule is a gene editing nuclease such as Cas9.

In an embodiment of the first, second, third, fourth and fifth aspect, including any embodiment thereof, the effector molecule is a secreted protein

In an embodiment of the first, second, third, fourth and fifth aspect, including any embodiment thereof, the effector molecule is effective as an autocrine factor.

In an embodiment of the first, second, third, fourth and fifth aspect, including any embodiment thereof, the effector molecule is effective as a paracrine factor.

In an embodiment of the first, second, third, fourth and fifth aspect, including any embodiment thereof, the recombinant nucleic acid construct comprises, preferably consists of ribonucleotides.

In an embodiment of the first, second, third, fourth and fifth aspect, including any embodiment thereof, the recombinant nucleic acid construct comprises, preferably consists of deoxyribonucleotides.

In an embodiment of the first, second, third, fourth and fifth aspect, including any embodiment thereof, the recombinant nucleic acid construct comprises, preferably consists of ribonucleotides and deoxyribonucleotides.

More specifically, the problem underlying the present invention is solved in a sixth aspect which is also a first embodiment of the sixth aspect, by a vector comprising a recombinant nucleic acid construct of the first aspect, including any embodiment thereof, and/or a recombinant nucleic acid construct as described in connection with each and any aspect, including any embodiment thereof, preferably of the second, third, fourth and fifth aspect.

In a second embodiment of the sixth aspect which is also an embodiment of the first embodiment of the sixth aspect, the vector is an expression vector.

In a third embodiment of the sixth aspect which is also an embodiment of the second embodiment of the sixth aspect, the vector allows expression of the recombinant nucleic acid construct in a cell, preferably an endothelial cell, more preferably the vector is a plasmid or a virus.

More specifically, the problem underlying the present invention is solved in a seventh aspect which is also a first embodiment of the seventh aspect, by a cell comprising a recombinant nucleic acid construct according to the first, second, third, fourth and fifth aspect, including any embodiment thereof, and/or a vector according to the sixth aspect, including any embodiment thereof.

In a second embodiment of the seventh aspect which is also an embodiment of the first embodiment of the seventh aspect, the cell is an endothelial cell or a mesenchymal stem cell.

In a third embodiment of the seventh aspect which is also an embodiment of the first and second embodiment of the seventh aspect, the cell is a recombinant cell.

In a fourth embodiment of the seventh aspect which is also an embodiment of the first, second and third embodiment of the seventh aspect, the cell is an isolated cell.

More specifically, the problem underlying the present invention is solved in an eighth aspect which is also a first embodiment of the eighth aspect, by a delivery vehicle comprising a recombinant nucleic acid construct according to the first, second, third, fourth and fifth aspect, including any embodiment thereof, wherein the delivery vehicle is a cationic lipid delivery particle.

In a second embodiment of the eighth aspect which is also an embodiment of the first embodiment of the eighth aspect, the particle is a nanoparticle.

In a third embodiment of the eighth aspect which is also an embodiment of the first and second embodiment of the eighth aspect, the average size of the nanoparticle is from about 30 nm to about 200 nm, preferably from about 30 nm to about 140 nm and more preferably from about 30 nm to about 60 nm.

More specifically, the problem underlying the present invention is solved in a ninth aspect which is also a first embodiment of the ninth aspect, by a composition comprising a recombinant nucleic acid construct according to the first, second, third, fourth and fifth aspect, including any embodiment thereof, a vector according to the sixth aspect, including any embodiment thereof, a cell according to the seventh aspect, including any embodiment thereof, and/or a delivery vehicle according to the eight aspect, including any embodiment thereof. In a preferred embodiment, the composition is a pharmaceutical composition comprising a pharmaceutically acceptable excipient.

More specifically, the problem underlying the present invention is also solved in a tenth aspect by the use in the manufacture of a medicament of a recombinant nucleic acid construct according to the first, second, third, fourth and fifth aspect, including any embodiment thereof, a vector according to the sixth aspect, including any embodiment thereof, a cell according to the seventh aspect, including any embodiment thereof, a cell according to the seventh aspect, including any embodiment thereof, and/or a delivery vehicle according to the eight aspect, including any embodiment thereof. In an embodiment thereof, the medicament is for the treatment of a disease, preferably a disease described herein in connection with the second, third, fourth and fifth aspect, including any embodiment thereof.

More specifically, the problem underlying the present invention is also solved in an eleventh aspect by a method for treating a subject, preferably a human subject, wherein the method comprises administering to the subject a therapeutically effective amount of a recombinant nucleic acid construct according to the first, second, third, fourth and fifth aspect, including any embodiment thereof, a vector according to the sixth aspect, including any embodiment thereof, a cell according to the seventh aspect, including any embodiment thereof, a delivery vehicle according to the eight aspect, including any embodiment thereof and/or a composition according to the ninth aspect, including any embodiment thereof. In an embodiment thereof, the method is for the treatment of a disease, preferably a disease described herein in connection with the second, third, fourth and fifth aspect, including any embodiment thereof.

More specifically, the problem underlying the present invention is also solved in an twelfth aspect by a method for vaccinating a subject, preferably a human subject, wherein the method comprises administering to the subject an amount effective to elicit in a or the subject an immune response, preferably a desired immune response, of a recombinant nucleic acid construct according to the first, second, third, fourth and fifth aspect, including any embodiment thereof, a vector according to the sixth aspect, including any embodiment thereof, a cell according to the seventh aspect, including any embodiment thereof, a delivery vehicle according to the eight aspect, including any embodiment thereof and/or a composition according to the ninth aspect, including any embodiment thereof. In an embodiment thereof, the method is for the treatment of a disease, preferably a disease described herein in connection with the second, third, fourth and fifth aspect, including any embodiment thereof. In a preferred embodiment, the effector molecule is one which is capable of or suitable for eliciting such immune response.

According to the present invention, any embodiment of the second, third, fourth and fifth aspect is also an embodiment of the first aspect, including any embodiment thereof.

According to the present invention, any embodiment of the first aspect is also an embodiment of the second, third, fourth and fifth embodiment, including any embodiment thereof.

In an embodiment and as preferably used herein, a heterologous construct, heterologous nucleic acid construct, recombinant construct or recombinant nucleic acid construct is a construct, preferably a nucleic acid construct, which as such is not existing in a wild type biological system such as a virus, a cell, a tissue, an organ or an organism. More preferably, a heterologous construct, heterologous nucleic acid construct, a recombinant construct or recombinant nucleic acid construct is one, where at least one element contained in the construct is not combined with a coding region for the effector, wherein said element is selected from the group consisting of a 5' non-translated region, a 3' non-translated region, a cap structure, a signal sequence and a poly-A tail. Preferably, the 5' non-translated region is a 5' UTR and the 3' non-translated region is a 3' UTR.

In an embodiment and as preferably used herein, a therapeutic method or a method for the treatment and/or prevention of a disease is a therapy.

In an embodiment of each and any aspect of the present invention, the coding region coding for an effector molecule is expressed by an endothelial cell. In preferred embodiment thereof, the endothelial cell expressing the effector molecule is an endothelial cell the function of which is impaired. In an alternative preferred embodiment, the endothelial cell expressing the effector molecule is an endothelial cell the function of which is not impaired; in such case, the effector molecule is exported or secreted by the effector molecule producing endothelial cell and exerts its effect on any other cell, typically any other endothelial cell, whereby such other cell is one the function of which is impaired. In a preferred embodiment, an impaired function of a cell is function of such cell which is to be restored in according with the present invention in its various aspect.

As preferably used herein, a derivative of a sequence which displays the same function as the parent sequence; preferably the parent sequence is a or the wild type sequence. In a preferred embodiment, the sequence of the derivative and of the parent case have an identity of at least 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. It is within the present invention that the derivative is a truncated version of the parent sequence. Such truncation may exist at the 5' end, the 3' end or both the 5' end and the 3' end. If reference is made to a parent sequence in terms of identity, such identity preferable refers to a stretch of subsequent nucleotides shared by both the derivative and the parent sequence with the sequence of the parent sequence serving as the reference sequence.

In an embodiment and as preferably used herein, a coding region coding for an effector molecule comprises or consists of a nucleic acid sequence coding to said effector molecule.

In an embodiment and as preferably used herein, a 5' non-translated region is a 5' non-translated sequence.

In an embodiment and as preferably used herein, a 3' non-translated region is a 5' non-translated sequence.

In an embodiment and as preferably used herein, the indication of any source of any one of the elements contained in the recombinant nucleic acid construct of the invention is to be understood as a substitute of or an alternative description of a nucleotide sequence. Such nucleotide sequence is preferably the one of the respective wild type sequence, more preferably the one of the respective human wild type sequence. It will be appreciated by a person skilled in the art that other species than man may provide such wild type sequence; in an embodiment of the present invention such wild type sequence for such other species than man may be used in the practicing of the present invention. It will also be appreciated by a person skilled in the art that such wild type sequence may be taken from publicly available data bases such as GenBank. The wild type sequence may be subject to changes over time, typically to correct sequencing errors. In an embodiment of the invention, the sequence is the one described in said publicly available data bases such as GenBank at the date of filing of the instant application. To the extent various respective wild type sequences exist, each and any such wild type sequence shall be encompassed by the present invention. In another embodiment, where there is more than one wild type sequences and the more than one wild type sequence is provided by different groups of people such as different geographically defined groups of people and/or different genetically defined groups of people, the wild type sequence prevailing in the most comprehensive group of said different groups of people may be regarded as the wild type sequence.

In an embodiment of the present invention and as preferably used herein, if reference is made to a derivative of a 5' UTR of a gene indicating that the derivative has a nucleotide identity of at least 85 %, identity is identity referring or relative to the 5' UTR of the gene.

In an embodiment of the present invention and as preferably used herein, if reference is made to a derivative of a 3' UTR of a gene indicating that the derivative has a nucleotide identity of at least 85 %, identity is identity referring or relative to the 3' UTR of the gene.

In an embodiment and as preferably used herein, a signal peptide is a peptide which mediates directly or indirectly the transfer or secretion of a polypeptide or a protein fused or attached to the signal peptide into the extracellular space, whereupon, preferably, the signal sequence is removed from the polypeptide and protein, respectively.

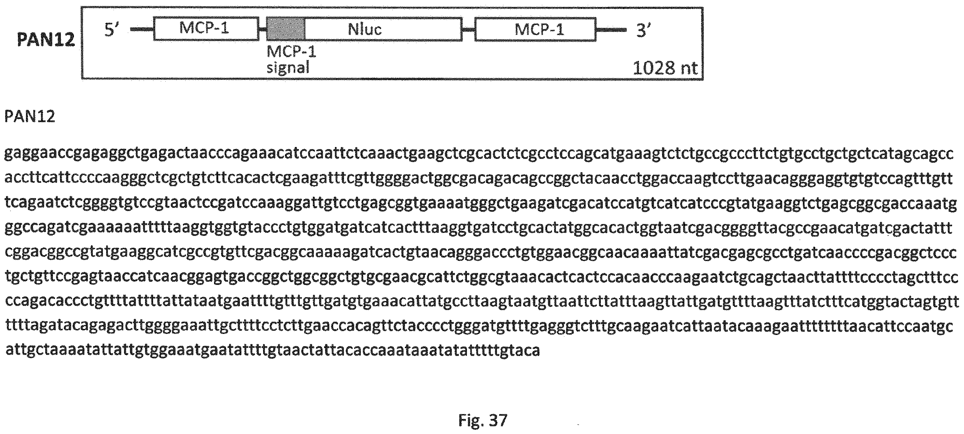

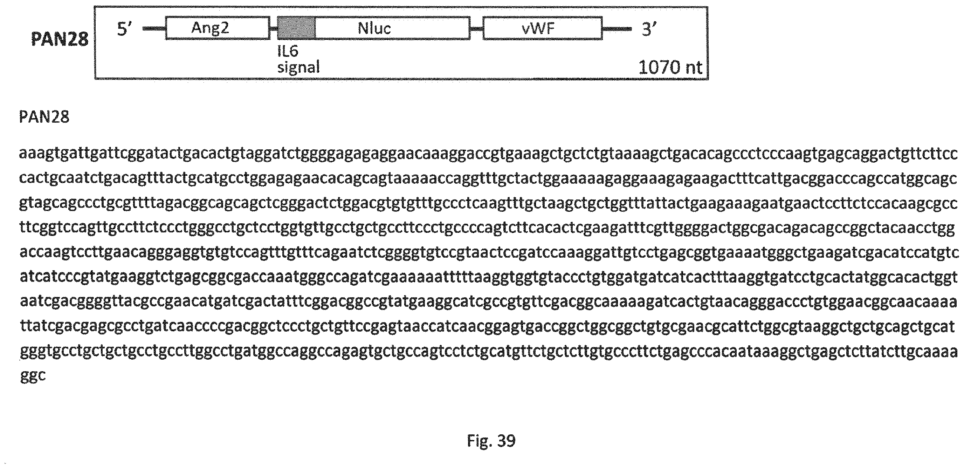

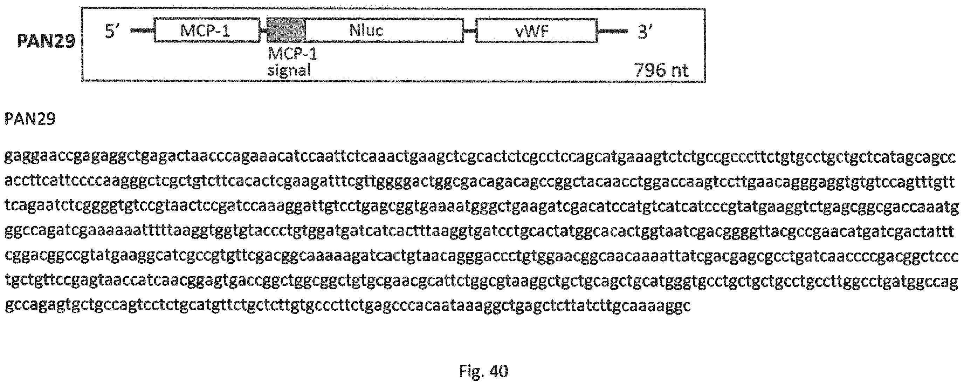

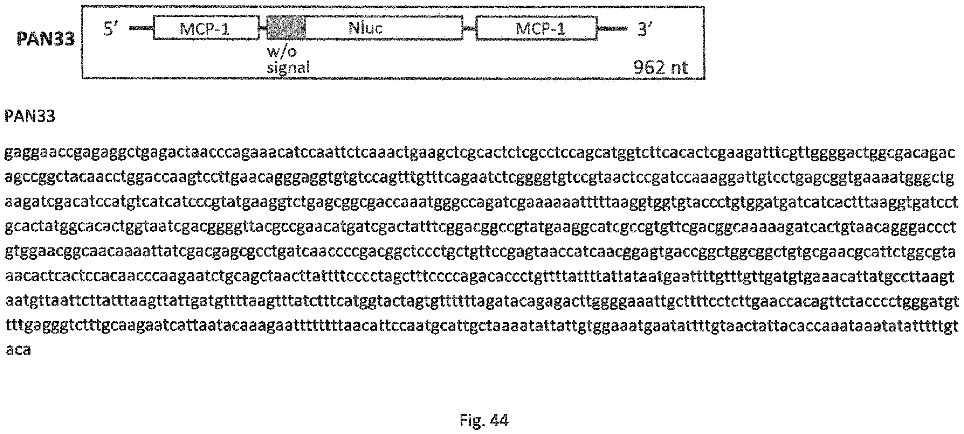

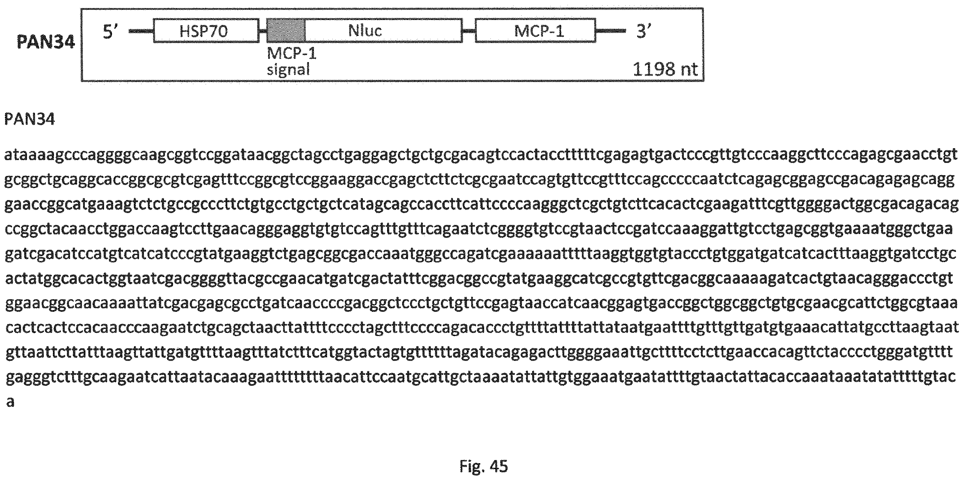

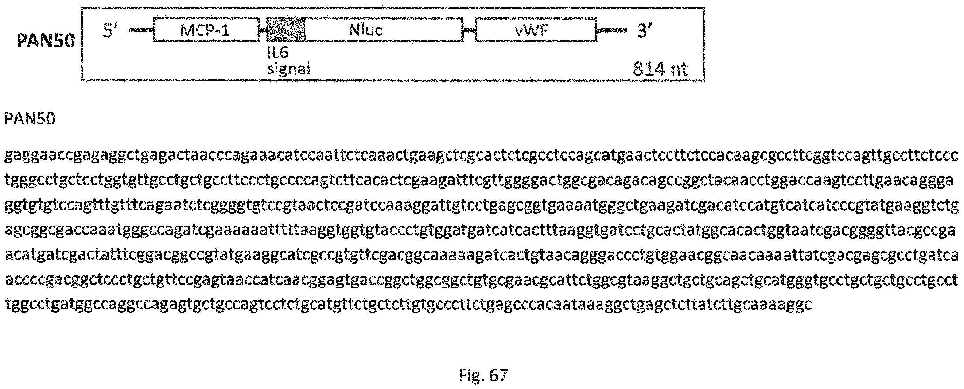

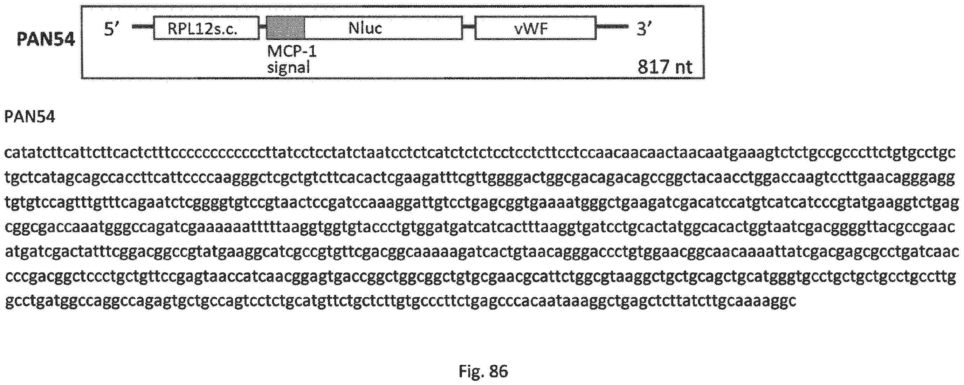

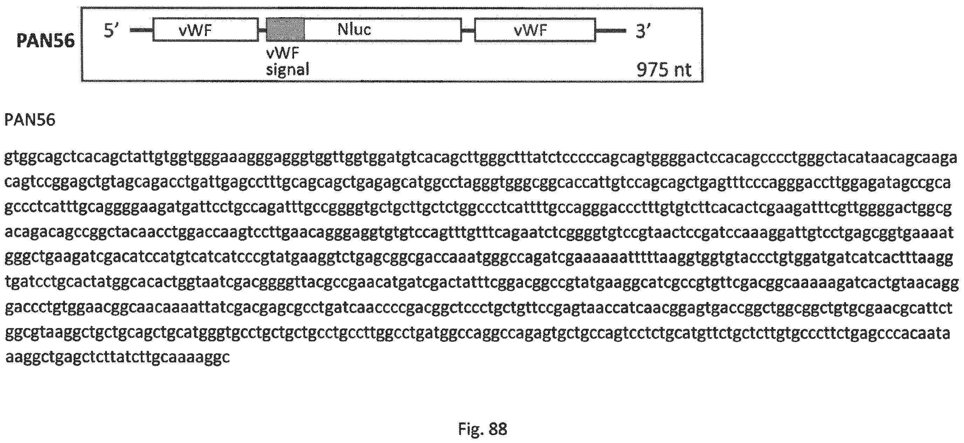

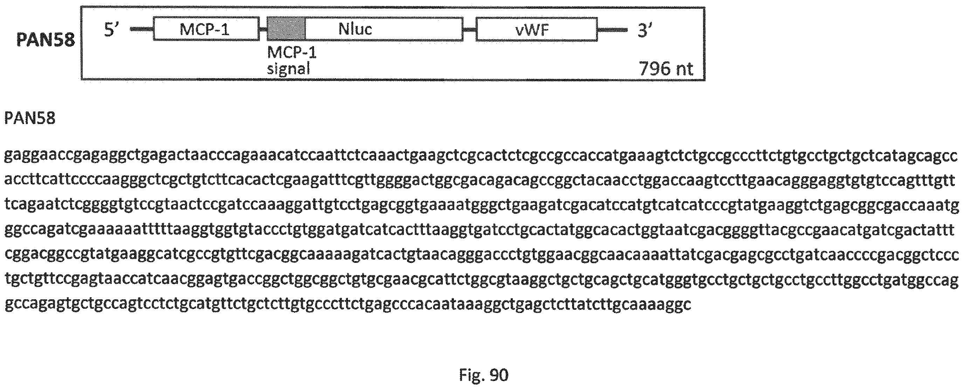

In an embodiment of the present invention, the recombinant nucleic acid construct is a recombinant nucleic acid construct disclosed in the Figs, more specifically a recombinant nucleic acid construct disclosed in Fig. 40 (PAN29), Fig 90 (PAN58), Fig 86 (PAN54), Fig 67 (PAN50), Fig 45 (PAN34), Fig 44 (PAN33), Fig 39 (PAN28), Fig 37 (PAN12), Fig 88 (PAN56).

In an embodiment of the present invention and as preferably used herein, a decoy is a mRNA encoded protein which binds a ligand and neutralizes the signaling activity.

In an embodiment and as preferably used herein, a poly-A tail is a nucleotide sequence comprising or consisting of a plurality of A's covalently attached to each other, whereby such covalent linkage is preferably a phosphodiester linkage. In an embodiment thereof, the poly-A tail comprises about 20 to about 240 As (i.e. adenosine phosphates) nucleotides, preferably about 60 to 120 As, more preferable about 100 to 140 As and most preferably about 120 As.

The poly A tail is thought to stabilize natural messengers and synthetic sense RNA. Therefore, in one embodiment a long poly A tail can be added to an mRNA molecule thus rendering the RNA more stable. Poly A tails can be added using a variety of art-recognized techniques. For example, long poly A tails can be added to synthetic or in vitro transcribed RNA using poly A polymerase (Yokoe, et al. Nature Biotechnology. 1996; 14: 1252-1256). A transcription vector can also encode long poly A tails. In addition, poly A tails can be added by transcription directly from PCR products. Poly A may also be ligated to the 3' end of a sense RNA with RNA ligase (see, e.g., Molecular Cloning A Laboratory Manual, 2nd Ed., ed. by Sambrook, Fritsch and Maniatis (Cold Spring Harbor Laboratory Press: 1991 edition)). In one embodiment, the length of the poly A tail is at least about 90, 200, 300, 400 at least 500 nucleotides. In one embodiment, the length of the poly A tail is adjusted to control the stability of a modified sense mRNA molecule of the invention and, thus, the transcription of protein. For example, since the length of the poly A tail can influence the half-life of a sense mRNA molecule, the length of the poly A tail can be adjusted to modify the level of resistance of the mRNA to nucleases and thereby control the time course of protein expression in a cell. In one embodiment, the stabilized nucleic acid molecules are sufficiently resistant to in vivo degradation (e.g., by nucleases), such that they may be delivered to the target cell without a transfer vehicle. In yet another embodiment the nucleic acid molecule can be purified by means of HPLC in order to remove double-stranded RNA contaminants and thus to reduce innate immune system activation (see e.g. Kariko et al., Nucleic Acids Res. 2011 Nov; 39(21):e142).

In an embodiment and as preferably used herein, the CAP structure is a nucleotide sequence forming a CAP structure which is known to a person skilled in the art. In eukaryotic cells the CAP-structure consists of a m7G(5')ppp(5')G 5'-5'-triphosphate linkage structure. The first two ribose sugars at the 5'-end of the mRNA can be methylated in the 2'-position which is known as CAP-1 (in case the first of the two ribose sugars is methylated) and CAP-2 structure (in case the second of the two ribose sugars is methylated). The CAP-structure can be incorporated during the mRNA transcription or post-transcriptionally by using capping and 2'-O-Methyltransferase enzymes and S-adenosylmethionine (SAM) as a methyl donor. In addition, a phosphatase treatment can be used in order to remove immune stimulatory 5'-phosphates.

In an embodiment and as preferably used herein, a gain of function mutation is a type of mutation in which the altered gene product possesses a new molecular function or a new pattern of gene expression. Gain of function mutations are almost always dominant or semi-dominant. In accordance therewith, a gain of function mutant effector molecule is an effector molecule possessing a new molecule function or a new pattern of expression.

In an embodiment and as preferably used herein, a hypermorphic mutation is a type of mutation in which the altered gene product possesses an increased level of activity. In accordance therewith, a hypermorphic mutant effector molecule is an effector molecule possessing an increased level of activity or an effector molecule which is expressed at an increased level. Preferably, such increase in expression level is caused by the mRNA coding the mutant effector molecule.
Hypermorphic mutations are, for example described in Muller, H. J. 1932. "Further studies on the nature and causes of gene mutations." Proceedings of the 6th International Congress of Genetics, pp. 213-255, the disclosure of which is incorporated herein by reference.

In an embodiment and as preferably used herein, the terms dominant applied to the phenotypic effect of a particular allele in reference to another allele (usually the standard wild-type allele) with respect to a given trait. An allele "A" is said to be dominant with respect to the allele "a" if the A/A homozygote and the A/a heterozygote are phenotypically identical and different from the a/a homozygote.

In an embodiment and as preferably used herein, an allele "A" is said to be semi-dominant with respect to the allele "a" if the A/A homozygote has a mutant phenotype, the A/a heterozygote has a less severe phenotype, while the a/a homozygote is wild type.

It will be acknowledged and appreciated by a person skilled in the art that these dominant mutations identified in human genome-wide-analysis can be selected from groups with different biological consequences such as mRNA stabilization phenotypes (e.g. functional deletion of microRNA binding sites), protein stabilization phenotypes (e.g. mutation of PEST sequence), phenotypes with attenuation of inhibitory domains and interfering with binding sites for inhibitors, phenotypes with constitutively activation of catalytic domains such as kinase domains, G-coupled protein receptor domains, ligand binding and dimerization domains.

In an embodiment of the first, second, third, fourth, fifth, sixth, seventh, eighth, ninth, tenth, eleventh and twelfth aspect, including any embodiment thereof, and as preferably used herein, the non-mutant effector molecule is a or the wild type form of the effector molecule. In a preferred embodiment, the wild type form of the effector molecule is a wild type form of the effector molecule in human.

In an embodiment of the first, second, third, fourth, fifth, sixth, seventh, eighth, ninth, tenth, eleventh and twelfth aspect, including any embodiment thereof, the mutant effector molecule comprises an amino acid sequence and the non-mutant effector molecule comprises an amino acid sequence, wherein the mutant effector molecule differs from the non-mutant effector molecule at the level of the amino acid sequence and/or at the level of the nucleotide sequence of the mRNA level under the proviso that the mutant effector molecule is a gain of function mutant effector molecule and/or a hypermorphic mutant effector molecule compared to the non-mutant effector molecule.

In a preferred embodiment of the first, second, third, fourth, fifth, sixth, seventh, eighth, ninth, tenth, eleventh and twelfth aspect, including any embodiment thereof, the mutant effector molecule differs from the non-mutant molecule by one or more amino acid mutations; preferably, an amino acid mutation is a type of mutation, wherein the type of mutation is selected from the group comprising an amino acid substitution, an amino acid deletion and an amino acid insertion. In an embodiment of the first, second, third, fourth, fifth, sixth, seventh, eighth, ninth, tenth, eleventh and twelfth aspect, including any embodiment thereof, the mutant effector molecule comprises a mutation at one amino acid position defined by the non-mutant effector molecule. The mutation at the one amino acid position comprises one type of mutation; alternatively, the mutation at the one amino acid position comprises more than one type of mutation.

In a further embodiment of the first, second, third, fourth, fifth, sixth, seventh, eighth, ninth, tenth, eleventh and twelfth aspect, including any embodiment thereof, the mutant effector molecule comprises a mutation at two or more amino acid positions defined by the non-mutant effector molecule. At each and any of the two or more amino acid positions, the mutation comprises, individually and independently from any other of the two or more amino acid positions, one type of mutation; alternatively, the mutation at the two or more amino acid positions comprises individually and independently from any other of the two or more amino acid positions, more than one type of mutation.

In a further embodiment of the first, second, third, fourth, fifth, sixth, seventh, eighth, ninth, tenth, eleventh and twelfth aspect, including any embodiment thereof, the mutant effector molecule comprises an additional stop, which leads to a truncated version of the effector molecule. In a preferred embodiment thereof, such additional stop codon results in deletion of an inhibitors domain, such as, e.g., a terminal inhibitory domain and a C-terminal inhibitory domain.

In an embodiment of the first, second, third, fourth, fifth, sixth, seventh, eighth, ninth, tenth, eleventh and twelfth aspect, including any embodiment thereof, the mutant effector molecule is a mutant effector molecule of the Tie-2 pathway, the PI3 kinase pathway, the Akt pathway, the mTor pathway, the MAPK (mitogen activated protein kinase) pathway, the RAS pathway and the TCA (tricarboxylic acid) cylce. The recombinant nucleic acid construct according to the present invention comprising as the effector molecule this kind of mutant effector molecule are particularly effective in the treatment of vascular leakage and any diseases involving, going along with or being caused by such vascular leakage. A mutant effector molecule that is particularly preferred for use in the recombinant nucleic acid construct is a molecule that is responsible for or results in vascular malformation; preferably, vascular malfunction includes congenital vascular anomalies of veins, lymph vessels, both veins and lymph vessels, or both arteries and veins.

In a preferred embodiment of the first, second, third, fourth, fifth, sixth, seventh, eighth, ninth, tenth, eleventh and twelfth aspect, including any embodiment thereof, such mutant effector molecule of the Tie-2 pathway, the PI3 kinase pathway, the Akt pathway or the mTor pathway is contained as the effector molecule in the recombinant nucleic acid construct PAN29, PAN54 or PAN58, whereby in case of constructs PAN29, PAN54 or PAN58 replacing the Nano Luciferase as the effector molecule.

In an embodiment of the first, second, third, fourth, fifth, sixth, seventh, eighth, ninth, tenth, eleventh and twelfth aspect, including any embodiment thereof, the recombinant nucleic acid construct comprising such mutant effector molecule of the Tie-2 pathway, of the PI3 kinase pathway of the Akt pathway or of the mTor pathway, is for use in the treatment of a diseases, preferably the treatment of acute respiratory distress syndrome. Due to the mutant effector molecule the Tie-2 pathway is activated leading, among others, to the expression of activating ligand angiopoetin-1 and, as a result thereof, to reduced vascular leakage. Effector molecules of the Tie-2 pathway are Tie-2, AKT, FOXO1, ABIN2, IQGAP1, RAP1, RAC1 and VE-cadherin, effector molecules of the PI3 kinase pathway are PIK3CA, PDK1, AKT, XIAP, eNOS, RAC, RAS ERK and S6K, effector molecules of the Akt pathway are Akt1, eNOS, IKK, MEJ1/2, ERK and API, effector molecules of the mTor pathway are mTor, SGK1, PKCα, Akt, HIF-1, S6K, eIF4G, Lipin 1, SKAR, PGC-1α, PPAPγ, PPARα, SREBP-1 and TFEB, effector molecules of the MAPK pathway are GNA11 and GNAQ, effector molecules of the RAS pathway are KRAS and NRAS, and an effector molecule of the TCA cycle is IDH1.

In an embodiment of the first, second, third, fourth, fifth, sixth, seventh, eighth, ninth, tenth, eleventh and twelfth aspect, including any embodiment thereof, the effector molecule is different from Tie-2 mutant (R849W).

In an embodiment of the first, second, third, fourth, fifth, sixth, seventh, eighth, ninth, tenth, eleventh and twelfth aspect, including any embodiment thereof, the effector molecule is different from a fusion or truncated version of PI3 kinase being hyperactive and/or being constitutively active.

In an embodiment of the first, second, third, fourth, fifth, sixth, seventh, eighth, ninth, tenth, eleventh and twelfth aspect, including any embodiment thereof, the effector molecule is different from a fusion or truncated version of Akt being hyperactive and/or being constitutively active.

In an embodiment of the first, second, third, fourth, fifth, sixth, seventh, eighth, ninth, tenth, eleventh and twelfth aspect, including any embodiment thereof, the effector molecule is a constitutively expressed Tie-2 receptor, preferably a constitutively expressed human Tie-2 receptor. The use of this embodiment allows restoration of vascular barrier function, preferably without the need for high concentrations of activating ligands to said Tie-2 receptor such as, e.g. Ang-1. The recombinant nucleic acid construct of this embodiment is particularly suitable for use in inflamed endothelium and for use in any diseases involving, going along with or being caused by inflamed endothelium, preferably inflamed endothelium showing decreased or down-regulated expression of Tie-2 ligand such as SEPSIS, acute respiratory distress syndrom, vascular-leak-syndrome, cerebral malaria syndrome, Malaria, acute pancreatitis, clarkson disease, dengue virus infection, leakage associated infections.

In an embodiment of the first, second, third, fourth, fifth, sixth, seventh, eighth, ninth, tenth, eleventh and twelfth aspect, including any embodiment thereof, the effector molecule is a wild type Tie-2 receptor, wherein the recombinant nucleic acid construct coding for such wild type Tie-2 receptor additionally comprises a coding region for Ang-1 or wherein the recombinant nucleic acid construct coding for such wild type Tie-2 receptor is used together with or is for use together with a recombinant nucleic acid construct coding for Ang-1 as effector molecule.

In an embodiment of the first, second, third, fourth, fifth, sixth, seventh, eighth, ninth, tenth, eleventh and twelfth aspect, including any embodiment thereof, the effector molecule is a mutant human Tie-2, wherein the mutant human Tie-2 comprises one or more than one mutations selected from the group comprising A925S, K1100N, L914F, R915L, R915H, R915C, R918C, R918L, R918G, R918H, T1105N, T1106P, V919L, Y897N, Y897C, Y897H, Y897F and Y897S. In a preferred embodiment, the mutant human Tie-2 is selected from the group comprising L914F, R915L, R915C, T1105N, T 1106P, Y897H and Y897F.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human Tie-2, wherein the mutant human Tie-2 comprises one or more than one mutations selected from the group comprising A925S, K1100N, L914F, R915L, R915H, R915C, R918C, R918L, R918G, R918H, T1105N, T1106P, V919L, Y897N, Y897C, Y897H, Y897F and Y897S, preferably one or more mutation selected from the group comprising L914F, R915L, R915C, T1105N, T 1106P, Y897H and Y897F; more preferably the mutant human Tie-2 comprises mutation L914F, the mutant human Tie-2 comprises mutations R849W and L914F, the mutant human Tie-2 comprises mutations Y897F and R915L, or the mutant human Tie-2 comprises mutations T1105N and T1106P.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human Tie-2 comprises mutation L914F, whereby the nucleotide sequence of the recombinant nucleic acid construct (PAN62) comprises SEQ ID NO: 128.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human Tie-2 comprises mutations R849W and L914F, whereby the nucleotide sequence of the recombinant nucleic acid construct (PAN63) comprises SEQ ID NO: 129.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human Tie-2 comprises mutations Y897F and R915L, whereby the nucleotide sequence of the recombinant nucleic acid construct (PAN64) comprises SEQ ID NO: 130.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human Tie-2 comprises mutations T1105N and T1106P, whereby the nucleotide sequence of the recombinant nucleic acid construct (PAN65) comprises SEQ ID NO: 131.

In an embodiment of the first, second, third, fourth, fifth, sixth, seventh, eighth, ninth, tenth, eleventh and twelfth aspect, including any embodiment thereof, the effector molecule is a mutant human PIK3CA (see, e.g., Tan FH et al.; Cells. 2019 Apr 9;8(4)) wherein the mutant human PIK3CA comprises one or more mutations selected from the group consisting of H1047R, E545K and E542K.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human PIK3CA, wherein the mutant human PIK3CA comprises one or more than one mutations selected from the group comprising H1047R, E545K and E542K.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human PIK3CA comprises mutation E542K, whereby the nucleotide sequence of the recombinant nucleic acid construct (PAN67) comprises SEQ ID NO: 134.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human PIK3CA comprises mutation E545K, whereby the nucleotide sequence of the recombinant nucleic acid construct (PAN68) comprises SEQ ID NO: 135.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human PIK3CA comprises mutation H1047R, whereby the nucleotide sequence of the recombinant nucleic acid construct (PAN69) comprises SEQ ID NO: 136.

In an embodiment of the first, second, third, fourth, fifth, sixth, seventh, eighth, ninth, tenth, eleventh and twelfth aspect, including any embodiment thereof, the effector molecule is a mutant human PIK3CA, wherein the mutant human PIK3CA comprises one or more mutations selected from the group consisting of R88Q, N345K, C420R, E542K, E545A, E545D, E545G, E545K, Q546E, Q546K, Q546L, Q546R, M1043I, H1047L, H1047R, H1047Y and G1049R.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human PIK3CA, wherein the mutant human PIK3CA comprises one or more than one mutations selected from the group comprising R88Q, N345K, C420R, E542K, E545A, E545D, E545G, E545K, Q546E, Q546K, Q546L, Q546R, M1043I, H1047L, H1047R, H1047Y and G1049R.

In an embodiment of the first, second, third, fourth, fifth, sixth, seventh, eighth, ninth, tenth, eleventh and twelfth aspect, including any embodiment thereof, the effector molecule is a mutant human Akt1. In a preferred embodiment, the mutant human Akt1 comprises one or more mutations, wherein the mutation is preferably E17K.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human Akt1, wherein the mutant human Akt1 comprises one or more than one mutations, preferably the mutation is E17K.

In an embodiment of the first, second, third, fourth, fifth, sixth, seventh, eighth, ninth, tenth, eleventh and twelfth aspect, including any embodiment thereof, the effector molecule is a mutant human GNA11. In a preferred embodiment, the mutant human GNA11 comprises one or more mutations, wherein the mutation is preferably selected from the group comprising Q209H, R183C and Q209L.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human GNA11, wherein the mutant human GNA11 comprises one or more than one mutations, preferably, the mutation is selected from the group comprising mutations Q209H, R183C and Q209L.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human GNA11 comprising mutation GNA11 Q 209H .

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human GNA11 comprising mutation GNA11 R183C.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human GNA11 comprising mutation GNA11 Q209L.

In an embodiment of the first, second, third, fourth, fifth, sixth, seventh, eighth, ninth, tenth, eleventh and twelfth aspect, including any embodiment thereof, the effector molecule is a mutant human GNAQ. In a preferred embodiment, the mutant human GNAQ comprises one or more mutations, wherein the mutation is preferably selected from the group comprising Q209H, R183Q and Q209L.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human GNAQ, wherein the mutant human GNAQ comprises one or more than one mutations, preferably, the mutation is selected from the group comprising mutations Q209H, R183Q and Q209L.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human GNAQ comprising mutation GNAQ Q 209H.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human GNAQ comprising mutation GNAQ R183Q.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human GNAQ comprising mutation GNAQ Q209L.

In an embodiment of the first, second, third, fourth, fifth, sixth, seventh, eighth, ninth, tenth, eleventh and twelfth aspect, including any embodiment thereof, the effector molecule is a mutant human KRAS. In a preferred embodiment, the mutant human KRAS comprises one or more mutations, wherein the mutation is preferably selected from the group comprising Q22K, G12A, A146T, G13C and G12D.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human KRAS, wherein the mutant human KRAS comprises one or more than one mutations, preferably, the mutation is selected from the group comprising mutations Q22K, G12A, A146T, G13C and G12D.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human KRAS comprising mutation KRAS Q22K.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human KRAS comprising mutation KRAS G12A.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human KRAS comprising mutation KRAS A146T.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human KRAS comprising mutation KRAS G13C.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human KRAS comprising mutation KRAS G12D.

In an embodiment of the first, second, third, fourth, fifth, sixth, seventh, eighth, ninth, tenth, eleventh and twelfth aspect, including any embodiment thereof, the effector molecule is a mutant human NRAS. In a preferred embodiment, the mutant human NRAS comprises one or more mutations, wherein the mutation is preferably mutation NRAS Q61R.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human NRAS, wherein the mutant human NRAS comprises one or more than one mutations, preferably, the mutation is NRAS Q61R.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human NRAS comprising mutation NRAS Q61R.

In an embodiment of the first, second, third, fourth, fifth, sixth, seventh, eighth, ninth, tenth, eleventh and twelfth aspect, including any embodiment thereof, the effector molecule is a mutant human IDH1. In a preferred embodiment, the mutant human IDH1 comprises one or more mutations, wherein the mutation is preferably mutation IDH1 R132C.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for a mutant human IDH1, wherein the mutant human IDH1 comprises one or more than one mutations, preferably, the mutation is IDH1 R132C.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human IDH1 comprising mutation IDH1 R132C.

In an embodiment of the first, second, third, fourth, fifth, sixth, seventh, eighth, ninth, tenth, eleventh and twelfth aspect, including any embodiment thereof, the effector molecule is a mutant PIK3CA as disclosed herein, wherein the recombinant nucleic acid construct coding for such mutant PIK3CA additionally comprises a coding region for a mutant GNAQ as disclosed herein or wherein the recombinant nucleic acid construct coding for such mutant PIK3CA is used together with or is for use together with a recombing nucleic acid construct coding for a mutant GNAQ as disclosed herein as the effector molecule.

In an embodiment of the first, second, third, fourth, fifth, sixth, seventh, eighth, ninth, tenth, eleventh and twelfth aspect, including any embodiment thereof, the effector molecule is a mutant PIK3CA as disclosed herein, wherein the recombinant nucleic acid construct coding for such mutant PIK3CA additionally comprises a coding region for a mutant GNA11 as disclosed herein or wherein the recombinant nucleic acid construct coding for such mutant PIK3CA is used together with or is for use together with a recombing nucleic acid construct coding for a mutant GNA11 as disclosed herein as the effector molecule.

In an embodiment of the first, second, third, fourth, fifth, sixth, seventh, eighth, ninth, tenth, eleventh and twelfth aspect, including any embodiment thereof, the effector molecule is a mutant PIK3CA as disclosed herein, wherein the recombinant nucleic acid construct coding for such mutant PIK3CA additionally comprises a coding region for a mutant IDH1 as disclosed herein or wherein the recombinant nucleic acid construct coding for such mutant PIK3CA is used together with or is for use together with a recombing nucleic acid construct coding for a mutant IDH1 as disclosed herein as the effector molecule.

In an embodiment of the first, second, third, fourth, fifth, sixth, seventh, eighth, ninth, tenth, eleventh and twelfth aspect, including any embodiment thereof, the effector molecule is a mutant human PI 3-kinase which is referred to as myrP110*. Briefly, myrP110* is based on a constitutively active PI 3-kinase molecule p110* carrying a myristylation signal. P110* is a chimeric protein in which the iSH2 region of p85 is covalently linked to its binding site at the N-terminus of p110, using a flexible hinge region. The myristylation sequence is at the N-terminal end of the molecule. (see, for example "Constitutively active Phosphatidylinositol 3-Kinase And Uses Thereof." Klippel et al. (1995); US Patent 6,043,062; and "Membrane Localization of Phosphatidylinositol 3-Kinase Is Sufficient To Activate Multiple Signal-Transducing Kinase Pathways.", Klippel et al. (1996); Molecular and Cellular Biology; Vol. 16, No.8, p4117-4127.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 or a derivative thereof having a nucleotide identity of at least 85 %, preferably the 5' UTR comprises a nucleotide sequence of SEQ ID NO: 20, a 3' UTR for a gene coding for von Willebrand factor (vWF) or a derivative thereof having a nucleotide identity of at least 85 %, preferably the 3' UTR comprises a nucleotide sequence of SEQ ID NO: 37, and a coding region coding for myrP110*.

In an embodiment of the first, second, third, fourth, fifth, sixth, seventh, eighth, ninth, tenth, eleventh and twelfth aspect, including any embodiment thereof, the recombinant nucleic acid construct comprises a 5' UTR for a gene coding for MCP-1 (preferably of SEQ ID NO: 20), a 3' UTR for a gene coding for von Willebrand factor (vWF) (preferably of SEQ ID NO: 37) and a coding region coding for the mutant human myrP110*, whereby the nucleotide sequence of the recombinant construct (PAN70) comprises SEQ ID NO: 151.

It will be acknowledged and appreciated by a person skilled in the art that the terms used herein preferably have the following meaning:
- Tie-2:: Tyrosine kinase with Ig and EGF homology domains-2 or Tyrosine-protein kinase receptor Tie-2
- PIK3CA:: Phosphatidylinositol-4,5-Bisphosphate 3-Kinase Catalytic Subunit Alpha
- Akt:: Protein kinase B (PKB
- GNA11:: G protein subunit alpha 11
- GNAQ:: G protein subunit alpha q
- KRAS:: Kirsten RAt Sarcoma virus
- NRAS:: Neuroblastoma RAS viral oncogene homolog
- HRAS:: Harvey murine sarcoma virus oncogene
- IDH1:: Isocitrat-Dehydrogenase 1
- myrP110*:: myristoylated form of the catalytic subunit p110alpha

In an embodiment of the first, second, third, fourth, fifth, sixth, seventh, eighth, ninth, tenth, eleventh and twelfth aspect, including any embodiment thereof, the effector molecule is a mutant of the EGFR gene (see, e.g., Lynch TJ et al. N Engl J Med. 2004 May 20;350(21):2129-39. Epub 2004 Apr 29).

In an embodiment of the first, second, third, fourth, fifth, sixth, seventh, eighth, ninth, tenth, eleventh and twelfth aspect, including any embodiment thereof, the effector molecule is a mutant of STAT3 and STAT1 (see, e.g. Fabre A et al.; J Allergy Clin Immunol Pract. 2019 Feb 27).

In an embodiment of the first, second, third, fourth, fifth, sixth, seventh, eighth, ninth, tenth, eleventh and twelfth aspect, including any embodiment thereof, the effector molecule is a mutant of KRAS, HRAS, NRAS (see, e.g., Ten Broek RW; Genes Chromosomes Cancer. 2019 Jan 24. doi: 10.1002/gcc.22739. [Epub ahead of print]).

In an embodiment of the first, second, third, fourth, fifth, sixth, seventh, eighth, ninth, tenth, eleventh and twelfth aspect, including any embodiment thereof, the effector molecule is a mutant of BRAF, KRAS, PIK3C, c-KIT, ABL, IDH1, IDH2 and JAK2 (see, e.g., Musaffe Tuna and Christopher I. Amos, "Activating Mutations and Targeted Therapy in Cancer" 2012 Oct 12. doi: 10.5772/48701.

In an embodiment of the first, second, third, fourth, fifth, sixth, seventh, eighth, ninth, tenth, eleventh and twelfth aspect, including any embodiment thereof, the effector molecule is a mutant of G-protein-coupled receptor genes (see, e.g., Fukami M et al. "Gain-of-function mutations in G-protein-coupled receptor genes associated with human endocrine disorders" Clin Endocrinol (Oxf). 2018 Mar; 88(3):351-359. doi: 0.1111/cen.13496. Epub 2017 Nov 7. Review)

In an embodiment of the first, second, third, fourth, fifth, sixth, seventh, eighth, ninth, tenth, eleventh and twelfth aspect, including any embodiment thereof, the effector molecule is a mutant of a receptor tyrosine kinase (RTK) (see, e.g., Du Z and Lovly CM; Mol Cancer. 2018 Feb 19; 17(1)). In an embodiment, the receptor tyrosine kinase is oncogenically activated. In an alternative embodiment, a mutation is one in extracellular domain (ECD), transmembrane domain (TMD) or juxtamembrane domain (JMD) of RTKs. Three missense mutations within the EGFR ECD (P596L, G598 V, and A289V) were previously reported in glioblastoma (GBM) which are associated with increased expression of EGFR protein. In another embodiment, a mutation is a mutation within ECD of other RTKs such as RET (Rezeptor-Tyrosinkinase Ret) in thyroid cancer and KIT (Tyrosine kinase KIT) in gastrointestinal stromal tumor (GIST). HER2 G660D and V659E mutations within the TMD act as driver mutations in non-small cell lung cancer (NSCLC). HER2 V659 mutations are also found in a patient with Li-Fraumeni syndrome. These mutations disrupt specific protein-protein and protein-lipid interactions within the HER2 TMD that are essential for proper receptor dimerization. It has been shown that these two TMD mutations exhibit lower protein turnover than wild-type HER2. Finally, mutations within the JMD release autoinhibitory juxtamembrane interactions and subsequently hyperactivate these RTKs, such as KIT V560G and PDGFRA V561D mutation in GIST. In light thereof, it will be acknowledged and appreciated that mutations within the ECD, TMD and JM of RTKs adopt alternative activating mechanisms compared to mutations within the TKD.

Just for reason of clarity, as used herein, if a mutation of a protein such as an effector molecule is indicated herein, the amino acid residue refers to the amino acid residue of the respective human wild type protein, and commensurate with the usual practice in the field, the amino acid to the left represents the amino acid residue as present in the wild type protein, and the amino acid to the right represents the amino acid residue as present in the mutant protein. For example, Tie-2 R849W is a mutant based on the human wild type of Tie-2, preferably as disclosed herein, where amino acid residue R of the wild type sequence is replaced by amino acid residue W in the mutant Tie-2.

Also just for reason of clarity, to the extent the nucleotide sequence of a recombinant nucleic acid construct is indicated as a DNA sequence, any T will be an U in case the recombinant nucleic acid construct is present in the embodiment where the nucleotides are ribonucleotides (rather than deoxribonucleotides).

In an embodiment of the first, second, third, fourth, fifth, sixth, seventh, eighth, ninth, tenth, eleventh and twelfth aspect, including any embodiment thereof, the effector molecule is a mutant of an oncogene. Preferably, the mutant effector molecule us a constitutive active growth factor, a constitutive active receptor or an oncogenic mutation affecting cell proliferation (see, e.g., Lodish H, Berk A, Zipursky SL, et al.; Molecular Cell Biology; New York: W. H. Freeman; 2000).

Most tumors express constitutively active forms of one or more intracellular signal-transduction proteins, causing growth-promoting signaling in the absence of normal growth factors. Cancer can result from expression of mutant forms of growth factors, growth factor receptors, signal-transduction proteins, transcription factors, pro- or anti-apoptotic proteins, and DNA repair proteins. Mutations changing the structure or expression of proteins in these classes generally give rise to dominantly active oncogenes greatly increasing the probability that the mutant cells will become tumor cells. Virus-encoded proteins that activate growth-factor receptors also can induce cancer.

This oncogenic protein expression is permanent in nature due to genetic changes allowing for a sustained protein expression. The use of these mutations in the practicing of the present invention and the recombinant nucleic acid construct of the present invention is possible due to the highly transient nature of the oncogenic activity of these growth-related proteins making a permanent oncogenic transformation highly unlikely.

Oncogenes rarely arise from genes encoding growth factors. In fact, only one naturally occurring growth-factor oncogene - sis - has been discovered. The sis oncogene, which encodes a type of PDGF, can aberrantly autostimulate proliferation of cells that normally express the PDGF receptor. Several types of mutations leading to production of constitutively active receptors have been identified, which transmit growth signals in the absence of the normal ligands. In some cases, a point mutation changes a normal Receptor-Tyrosine-Kinase into one that dimerizes and is activated in the absence of ligand. For instance, a single point mutation converts the normal Her2 receptor into the Neu oncoprotein, which is found in certain mouse cancers. A mutation that alters a single amino acid (valine to glutamine) in the transmembrane region of the Her2 receptor causes dimerization of two receptor proteins in the absence of the normal EGF-related ligand, making the protein constitutively active as a kinase. Because of this, such mutant Her2 receptor constitutes an effector molecule of the recombinant nucleic acid construct according to the first, second, third, fourth, fifth, sixth, seventh, eighth, ninth, tenth, eleventh and twelfth aspect, including any embodiment thereof.

A deletion that causes loss of the extracellular ligand binding domain in the EGF receptor leads, for unknown reasons, to constitutive activation of the protein kinase, ErbB. Because of this, such mutant ErbB constitutes an effector molecule of the recombinant nucleic acid construct according to the first, second, third, fourth, fifth, sixth, seventh, eighth, ninth, tenth, eleventh and twelfth aspect, including any embodiment thereof. It will be appreciated and acknowledged by a person skilled in the art that ErbB is actually a protein family consisting of, namely ErbB-1, also named epidermal growth factor receptor (EGFR), ErbB-2, also named HER2 in humans and neu in rodents, ErbB-3, also named HER3, and ErbB-4, also named HER4.v-ErbBs are homologous to EGFR, but lack sequences within the ligand binding ectodomain.

Similarly, human tumors called multiple endocrine neoplasia type 2 have been found to express a constitutively active dimeric GDNF (Glial cell-derived neurotrophic factor) receptor that results from a point mutation in the extracellular domain. In other cases, deletion of much of the extracellular ligand-binding domain produces a constitutively active oncoprotein receptor. Because of this, such mutant GDNF receptor constitutes an effector molecule of the recombinant nucleic acid construct according to the first, second, third, fourth, fifth, sixth, seventh, eighth, ninth, tenth, eleventh and twelfth aspect, including any embodiment thereof.

A large number of oncogenes are derived from protooncogenes whose encoded proteins act as intracellular transducers, proteins that aid in transmitting signals from a receptor to a cellular target. In this class are the ras oncogenes, which were the first non-viral oncogenes to be recognized. E.g. a single point mutation in Ras, a key transducing protein in many signaling pathways, reduces its GTPase activity, thereby maintaining it in an activated state. One common mutation by which the ras proto-oncogene becomes an oncogene causes only one change in the protein, substitution of any amino acid for glycine at position 12 of the sequence. This simple mutation reduces the protein's GTPase activity, thus linking GTP hydrolysis to the maintenance of normal, controlled Ras function. The oncogenic change causes only a slight alteration in the three-dimensional structure of Ras, but this is sufficient to change the normal protein into an oncoprotein. Constitutively active Ras oncoproteins are expressed by many types of human tumors including bladder, colon, mammary, skin, and lung carcinomas, neuroblastomas, and leukemias. Ras proteins are anchored to the inner side of the cell's plasma membrane by a particular type of covalently attached fatty acid called a farnesyl group, this attachment is essential for either normal or oncogenic Ras proteins to function as signal transducers. Because of this, such mutant Ras constitutes an effector molecule of the recombinant nucleic acid construct according to the first, second, third, fourth, fifth, sixth, seventh, eighth, ninth, tenth, eleventh and twelfth aspect, including any embodiment thereof.

Oncogenes encoding other components of the RTK-Ras - MAP kinase pathway also have been identified. One example, found in certain transforming mouse retroviruses, encodes a constitutively activated Raf serine/threonine kinase, which is in the pathway between Ras and MAP kinase. Another is the crk oncogene found in avian sarcoma virus, which causes certain tumors when overexpressed. The Crk protein, which has no known biochemical activity, contains one SH2 and two SH3 domains, and is similar to the GRB2 adapter protein that also functions between an RTK and Ras. The SH2 and SH3 domains in GRB2 and other adapter proteins mediate formation of specific protein aggregates that normally serve as signaling units for cellular events. However, overexpression of Crk leads to formation of protein aggregates that inappropriately signal the growth and metastatic abilities characteristic of cancer cells. Because of this, such mutant Raf serine/threonine kinase and such mutant Crk protein constitute an effector molecule of the recombinant nucleic acid construct according to the first, second, third, fourth, fifth, sixth, seventh, eighth, ninth, tenth, eleventh and twelfth aspect, including any embodiment thereof.

Several oncogenes, some initially identified in human tumors, others in transforming retroviruses, encode nonreceptor protein-tyrosine kinases. One of these is generated by a chromosomal translocation that results in fusion of a portion of the bcr gene (whose function is unknown but whose N-terminal segment forms a coiled-coil domain that links several bcr polypeptides together) with part of the c-abl gene, which encodes a protein-tyrosine kinase whose normal substrates are not known. The chimeric polypeptides expressed from the resulting Bcr-Abl oncogene form a tetramer that exhibits constitutive Abl kinase activity. Although Abl is normally localized to the nucleus, addition of the Bcr segment causes the Bcr-Abl oncoprotein to be localized to the cytosol. Through the Abl SH2 and SH3 domains Bcr-Abl binds to many intracellular signal-transduction proteins and then phosporylates them, proteins that Abl would not normally activate. As a consequence, these signaling proteins, including JAK2 protein-tyrosine kinase, Stat5 transcription factor, and PI-3 kinase become activated in the absence of growth factors. Because of this, such oncogenes and mutant oncogenes, respectively, constitute an effector molecule of the recombinant nucleic acid construct according to the first, second, third, fourth, fifth, sixth, seventh, eighth, ninth, tenth, eleventh and twelfth aspect, including any embodiment thereof.

Another oncogene that encodes a constitutively active cytosolic protein-tyrosine kinase is src. As we noted previously, v-src from Rous sarcoma retrovirus was the first oncogene to be discovered. The regulation of Src activity and the alterations by which the c-src proto-oncogene becomes an oncogene is known. The normal Src kinase (c-Src) contains an SH2, an SH3, and a protein-tyrosine kinase domain. The kinase activity of this 60-kDa protein is normally inactivated by phosphorylation of the tyrosine residue at position 527, which is six residues from the C-terminus. Hydrolysis of phosphotyrosine 527 by a specific phosphatase enzyme normally activates c-Src. Tyrosine 527 is often missing or altered in Src oncoproteins that have constitutive kinase activity; that is, they do not require activation by a phosphatase. In Rous sarcoma virus, for instance, the src gene has suffered a deletion that eliminates the C-terminal 18 amino acids of c-Src; as a consequence, the v-Src kinase is constitutively active. Phosphorylation of target proteins by aberrant Src oncoproteins contributes to abnormal proliferation of many types of cells. Because of this, such aberrant or mutant Src oncoproteins, respectively, constitute an effector molecule of the recombinant nucleic acid construct according to the first, second, third, fourth, fifth, sixth, seventh, eighth, ninth, tenth, eleventh and twelfth aspect, including any embodiment thereof.

In an embodiment of the first, second, third, fourth, fifth, sixth, seventh, eighth, ninth, tenth, eleventh and twelfth aspect, including any embodiment thereof, any condition of disease can thus be addressed where more cellular growth is beneficial e.g. indications related to regeneration of damaged tissue in indications such as wound healing, stroke, heart failure, muscle and bone regeneration, cartilage regeneration, organ transplantation, induction of stem cell growth. More preferably, the effector molecule of the recombinant nucleic acid construct used for such purpose is a hypermorphic mutant effector molecule.

In an embodiment of the first, second, third, fourth, fifth, sixth, seventh, eighth, ninth, tenth, eleventh and twelfth aspect, including any embodiment thereof, the effector molecule is a mutant effector molecule and more specifically a mutant effector molecule which is hypermorphic. It will be appreciated and acknowledged by a person skilled in the art that such mutant effector molecule which is hypermorphic is transiently expressed by the recombinant nucleic acid construct of the present invention so as to avoid oncogenic transformation by excessive proliferation of cells and to prevent the induction of several diseases associated with uncontrolled proliferation, including cancer, atherosclerosis, vascular malformation, rheumatoid arthritis, psoriasis, liver fibrosis, idiopathic pulmonary fibrosis, scleroderma and cirrhosis of the liver. On the other hand, such mutant effector molecule which is hypermorphic can be used in any regenerative treatment. Preferably, such regenerative treatment includes, but is not limited to the treatment of heart failure, vascular disease, stroke, pulmonary disease including fibrosis, arthritis, burns, wound healing and skin disorders, Crohn's disease, pediatric neurological disease, spinal cord injuries, Alzheimer, Parkinson, and frailty in the elderly.

Other conditions and diseases which can be treated by this kind of mutant effector molecule being contained in the recombinant nucleic acid construct of the present invention encompass any diseases in which an increased regenerative potential after tissue injury are potential fields of application. Humans are limited in their capacity for reparative regeneration, which occurs in response to injury. One of the most studied regenerative responses in humans is the hypertrophy of the liver following liver injury. Cardiac myocyte renewal has been found to occur in normal adult humans, and at a higher rate in adults following acute heart injury such as infarction. Even in adult myocardium following infarction, proliferation is only found in around 1% of myocytes around the area of injury, which is not enough to restore function of cardiac muscle. However, this is an important target for regenerative medicine as it implies that regeneration of cardiomyocytes, and consequently of myocardium, can be induced. When there is chronic or acute damage to tissue involving the immune system, as in inflammation or heart disease, the body loses the ability to repair itself.

In a preferred embodiment of the first, second, third, fourth, fifth, sixth, seventh, eighth, ninth, tenth, eleventh and twelfth aspect, including any embodiment thereof, the mutant effector molecule is phospholipase C-gamma (PLCG2) which is a functional hypermorphic mutant of PLCG2. A preferred mutant PLCG2 is PLCG2 P522R (see, e.g. Magno L et al., Alzheimers Res Ther. 2019 Feb 2;11(1):16). A recombinant nucleic acid construct of the present invention comprising as the effector molecule such mutant PLCG2 and PLCG2 P22R in particular, is useful in the treatment of Alzheimer's disease (Magno L et al., supra).

In an embodiment of the first, second, third, fourth, fifth, sixth, seventh, eighth, ninth, tenth, eleventh and twelfth aspect, including any embodiment thereof, the effector molecule is a mutant Factor IX and more specifically a mutant Factor IX which is hypermorphic (VandenDriessche T and Chual MK; Molecular Therapy, Vol. 26, No. 1, January 2018, p. 18 ff). More preferably the mutant effector molecule is Factor IX R338L). A recombinant nucleic acid construct of the present invention comprising as the effector molecule such mutant Factor IX and Factor IX R338L in particular, is useful in the treatment of hemophilia (VandenDriessche T and Chual MK; supra).

In a preferred embodiment of the recombinant nucleic acid construct according to each and any aspect, including any embodiment thereof, the recombinant nucleic acid construct is an mRNA.

In an embodiment of each and any aspect of the present invention, including any embodiment thereof, a 5' UTR of a gene coding for MCP-1 comprises a nucleotide sequence of SEQ ID NO: 20.

In an embodiment of each and any aspect of the present invention, including any embodiment thereof, a 3' UTR of a gene coding for MCP-1 comprises a nucleotide sequence of SEQ ID NO: 23.

In an embodiment of each and any aspect of the present invention, including any embodiment thereof, a 5' UTR of a gene coding for RPL12s.c. comprises a nucleotide sequence of SEQ ID NO: 24.

In an embodiment of each and any aspect of the present invention, including any embodiment thereof, a 3' UTR of a gene coding for RPL12s.c. comprises a nucleotide sequence of SEQ ID NO: 25.

In an embodiment of each and any aspect of the present invention, including any embodiment thereof, a 5' UTR of a gene coding for Ang-2 comprises a nucleotide sequence of SEQ ID NO: 26.

In an embodiment of each and any aspect of the present invention, including any embodiment thereof, a 3' UTR of a gene coding for Ang-2 comprises a nucleotide sequence of SEQ ID NO: 29.

In an embodiment of each and any aspect of the present invention, including any embodiment thereof, a 5' UTR of a gene coding for HSP70 comprises a nucleotide sequence of SEQ ID NO: 38 or 40.

In an embodiment of each and any aspect of the present invention, including any embodiment thereof, a 3' UTR of a gene coding for HSP70 comprises a nucleotide sequence of SEQ ID NO: 39 or 43.

In an embodiment of each and any aspect of the present invention, including any embodiment thereof, a 5' UTR of a gene coding for H3.3. comprises a nucleotide sequence of SEQ ID NO: 44.

In an embodiment of each and any aspect of the present invention, including any embodiment thereof, a 3' UTR of a gene coding for H3.3. comprises a nucleotide sequence of SEQ ID NO: 45.

In an embodiment of each and any aspect of the present invention, including any embodiment thereof, a 5' UTR of a gene coding for Galectin-9 (LHALS9) comprises a nucleotide sequence of SEQ ID NO: 46.

In an embodiment of each and any aspect of the present invention, including any embodiment thereof, a 3' UTR of a gene coding for Galectin-9 (LHALS9) comprises a nucleotide sequence of SEQ ID NO: 47.

In an embodiment of each and any aspect of the present invention, including any embodiment thereof, a 5' UTR of a gene coding for IL-6 comprises a nucleotide sequence of SEQ ID NO: 30.

In an embodiment of each and any aspect of the present invention, including any embodiment thereof, a 3' UTR of a gene coding for IL-6 comprises a nucleotide sequence of SEQ ID NO: 33.

In an embodiment of each and any aspect of the present invention, including any embodiment thereof, a 5' UTR of a gene coding for vWF comprises a nucleotide sequence of SEQ ID NO: 34.

In an embodiment of each and any aspect of the present invention, including any embodiment thereof, a 3' UTR of a gene coding for vWF comprises a nucleotide sequence of SEQ ID NO: 37.

In an embodiment of each and any aspect of the present invention, including any embodiment thereof, a 5' UTR of a gene coding for Ang-1 comprises a nucleotide sequence of SEQ ID NO: 83.

In an embodiment of each and any aspect of the present invention, including any embodiment thereof, a 3' UTR of a gene coding for Ang-1 comprises a nucleotide sequence of SEQ ID NO: 88.

In an embodiment of each and any aspect of the present invention, including any embodiment thereof, a 5' UTR of a gene coding for Ang-4 comprises a nucleotide sequence of SEQ ID NO: 89.

In an embodiment of each and any aspect of the present invention, including any embodiment thereof, a 3' UTR of a gene coding for Ang-4 comprises a nucleotide sequence of SEQ ID NO: 94.

In an embodiment of each and any aspect of the present invention, including any embodiment thereof, a 5' UTR of construct PAN57 comprises a nucleotide sequence of SEQ ID NO: 120.

In an embodiment of each and any aspect of the present invention, including any embodiment thereof, a 3' UTR of construct PAN57 comprises a nucleotide sequence of SEQ ID NO: 121.

In an embodiment of each and any aspect of the present invention, including any embodiment thereof, the various combinations of a 5' UTR and a 3' UTR as realized in each and any of the specific constructs disclosed herein is a combination of 5' UTR and a 3' UTR which may be realized in recombinant nucleic acid construct irrespective of any other constituent and in particular irrespective of any coding region coding for an effector molecule contained in a recombinant nucleic acid construct of the invention.

In an embodiment of each and any aspect of the present in invention, including any embodiment thereof, and as preferably used herein, an endogenous gene is a gene contained in any cell described herein. In accordance therewith, a recombinant nucleic acid construct where the 5' UTR and the 3' UTR are from different endogenous genes, comprises a 5' UTR from a first gene of a cell and a 3' UTR from a second gene of a cell, for example the 5' UTR is from a gene encoding MCP-1 or Ang-2, and the 3' UTR is from a gene coding for vWF. Said cell may be the same cell or a different cell and/or said cell may be the same kind of cell or a different kind of cell. Preferably, the cell is of human origin.

In a preferred embodiment of the recombinant nucleic acid construct according to each and any aspect, including any embodiment thereof, the recombinant nucleic acid construct is a recombinant nucleic acid molecule.

It is within the present invention defined by each and any aspect, including any embodiment thereof, that the nucleotides forming the recombinant nucleic acid molecule may be modified. Suitable modifications include alterations in one or more nucleotides of a codon such that the codon encodes the same amino acid but is more stable than the codon found in the wild-type version of the nucleic acid. For example, an inverse relationship between the stability of RNA and a higher number cytidines (C's) and/or uridines (U's) residues has been demonstrated, and RNA devoid of C and U residues have been found to be stable to most RNases (Heidenreich, et al. J Biol Chem 269, 2131-8 (1994)). In some embodiments, the number of C and/or U residues in an mRNA sequence is reduced. In another embodiment, the number of C and/or U residues is reduced by substitution of one codon encoding a particular amino acid for another codon encoding the same or a related amino acid. Contemplated modifications to the mRNA nucleic acids of the present invention also include the incorporation of pseudouridine, N1-methyl-pseudouridine, 5-methoxyuridine and 5'-Methylcytidine. The incorporation of such modified nucleotides into the recombinant nucleic acid construct, preferably the mRNA of the present invention may enhance stability and translational capacity, as well as diminishing immunogenicity in vivo. (See, e.g., Kariko, K., et al., Molecular Therapy 16 (11): 1833-1840 (2008)). Substitutions and modifications to the recombinant nucleic acid construct of the present invention, particularly at the level of the individual nucleotide of the recombinant nucleic acid construct, may be performed by methods readily known to one of ordinary skill in the art.

The constraints on reducing the number of C and U residues in a sequence will likely be greater within the coding region of an mRNA, compared to an untranslated region, (i.e., it will likely not be possible to eliminate all of the C and U residues present in the message while still retaining the ability of the message to encode the desired amino acid sequence). The degeneracy of the genetic code, however presents an opportunity to allow the number of C and/or U residues that are present in the sequence to be reduced, while maintaining the same coding capacity (i.e., depending on which amino acid is encoded by a codon, several different possibilities for modification of RNA sequences may be possible). For example, the codons for Gly can be altered to GGA or GGG instead of GGU or GGC.

The term modification also includes, for example, the incorporation of non-nucleotide linkages or modified nucleotides into the nucleic acid sequences of the present invention (e.g., modifications to one or both the 3' and 5' ends of an mRNA molecule encoding a functional protein or enzyme). Such modifications include the addition of bases to a nucleic acid sequence (e.g., the inclusion of a poly A tail or a longer poly A tail), the alteration of the 3' UTR or the 5' UTR, complexing the nucleic acid with an agent (e.g., a protein or a complementary nucleic acid molecule), and inclusion of elements which change the structure of a nucleic acid molecule (e.g., which form secondary structures).

The delivery vehicle according to the eighth aspect is preferably based on a composition of one or more suitable lipids (e.g. liposomes) which in combination with the recombinant nucleic acid construct according to the present invention result in overall cationic-lipid-nucleic acid particles (cLNPs). Such cLNPs can be used for functionally transferring a nucleic acid, particularly an mRNA, into endothelial cells of the mammalian vasculature.

Lipid-nucleic acid nanoparticles (LNPs) formed by complexation of nucleic acids such as the recombinant nucleic acid construct of the present invention with cationic lipids in combination with other lipidic components, such as zwitterionic phospholipids, cholesterol and PEGylated lipids, have already been used to block degradation of RNAs in plasma and to facilitate the functional cellular uptake of the RNAs.

However, in a therapeutic context, particularly in view of parenteral therapeutic applications, the overall charge of the lipid-nucleic acid nanoparticles, characterized by the Zeta-potential, is essential in directing the organ distribution of the nanoparticles. Someone skilled in the art will appreciate that overall neutrally charged LNPs are almost exclusively taken up by organs of the reticuloendothelial system, particularly by the liver and spleen. On the other hand, it is also known to those skilled in the art, that overall positively charged lipid nanoparticles are particularly useful to functionally deliver a pharmaceutical payload into cells of the mammalian vasculature.

Those skilled in the art will also appreciate that free nucleic acids, particularly mRNAs, are immune stimulatory if applied intravenously.

Thus, in view of parenteral therapeutic applications a thorough and stable complexation of the nucleic acid inside the lipidic nanoparticle will be essential in order to prevent undesired immunogenic side reactions. Furthermore, it is also known to those skilled in the art that the size of the lipid-nucleic acid nanoparticle is crucial in suppressing the reticuloendothelial clearance of the particles.

It has recently been described that highly potent limit sized siRNA LNPs (∼30 nm mean particle size) can be prepared using a microfluidic mixing process based on a staggered herringbone micromixer (SHM) device (Belliveau et al.; Molecular Therapy-Nucleic Acids (2012) 1(8)).

Due to the millisecond mixing of the lipids and the nucleic acid such as the recombinant nucleic acid construct of the invention on a nanoliter scale, the resulting particles are characterized by a very tight packaging of the nucleic acid inside a dense, solid particle core. On the contrary, those skilled in the art will appreciate that macrofluidic mixing processes using T-type connectors, generally result in larger nanoparticles with a more heterogeneous, multilamellar morphology.

In an embodiment, the present invention provides novel positively charged lipid-nucleic acid nanoparticles comprising a nucleic acid such as recombinant nucleic acid construct of the invention for the functional delivery of said recombinant nucleic acid construct of the invention, particularly mRNA, into cells of the mammalian vasculature, particularly into endothelial cells of the lung vasculature.

In an embodiment of the various aspects of the present invention, including any embodiment thereof, a cationic lipid selected from the group comprising β-(L-Arginyl)-L-2,3-diamino propionic acid-N-palmityl-N-oleyl-amide, L-Arginyl-β-alanine-N-palmityl-N-oleyl-amide, DOTAP (N-[1-(2,3-Dioleoyloxy)propyl]-N,N,N-trimethylammonium methyl-chloride), DOTMA (1,2-di-O-octadecenyl-3-trimethylammonium propane (chloride salt)) or DC-cholesterol is combined with neutral lipids and further shielding lipid components bearing a polyethylene glycol (PEG) modification. The neutral lipid can be either a zwitterionic phospholipid selected from a group comprising Diphytanoyl-PE (1,2-Diphytanoyl-sn-glycero-3-phosphoethanolamine), DOPE (1,2-Dioleoyl-sn-glycero-3-phosphoethanolamine), DLPE (1,2-Lauroyl-sn-glycero-3-phosphoethanolamine), DMPE, POPE, DSPE or an uncharged sterol lipid selected from a group comprising cholesterol and stigmasterol. The PEGylated lipid component is selected from a group comprising methoxyPEG-DSPE, methoxyPEG-DLG, methoxyPEG-DMG, methoxyPEG-DPG, methoxyPEG-DSG, methoxyPEG-c-DMA, methoxyPEG-C8-Ceramide, methoxyPEG-C16-Ceramide. In a particular embodiment the chain length of the PEG-chain corresponds to a molecular weight in the range of 750 Da to 5000 Da, preferably in a range of 1500 Da to 3000 Da.

In another embodiment of the present invention, the molar ratio of the lipids in the lipid mixture is in a range of 20 - 80 mol% cationic lipid, 10 - 70 mol% neutral lipid and 1 - 10 mol% PEGylated lipid, preferably in a range of 35-65 mol% cationic lipid, 35-65 mol% neutral lipid and 1-5 mol% PEGylated lipid (with the overall lipid content being set as 100 %).

In a preferred embodiment, the cationic lipid is β-(L-Arginyl)-L-2,3-diamino propionic acid-N-palmityl-N-oleyl-amide or L-Arginyl-β-alanine-N-palmityl-N-oleyl-amide, the neutral lipid is the phospholipid Diphytanoyl-PE and the PEGylated lipid is methoxyPEG2000-DSPE. The molar ratio is 50 mol% cationic lipid, 49 mol% Diphytanoyl-PE and 1 mol% mPEG2000-DSPE (1,2-distearoyl-sn-glycero-3-phosphoethanolamine-N-[methoxy(polyethylene glycol)-2000] (sodium salt)).

In yet another embodiment, the mixture of above mentioned lipids is dissolved in a water miscible solvent selected from a group comprising ethanol, acetone, 1-butanol, 2-butanol, tert.-butanol, 3-methyl-1-butanol, 2-methyl-1-propanol, 1-propanol, 2-propanol, dimethylsulfoxide, preferably from a group comprising ethanol, tert.-butanol and 1-butanol.

The dissolved lipid mixture is subsequently rapidly mixed with a dissolution of a nucleic acid such as the recombinant nucleic acid construct of the invention in an aqueous solvent, resulting in the formation of lipid-nucleic acid nanoparticles (LNPs). In a preferred embodiment of the present invention, the mixing is performed using a microfluidic mixing device, particularly using a staggered herringbone-type mixing chamber, e.g. NanoAssemblr™ (Precision NanoSystems Inc.; Vancouver, Canada) device.

In yet another embodiment, the mass ratio of total lipids to nucleic acid nucleic acid such as the recombinant nucleic acid construct of the invention is in a range of 5 to 60, preferably in a range of 15 to 40 and the volumetric mixing ratio of aqueous nucleic acid solution to organic total lipid solution is in the range of 1:1 to 6:1, preferably in the range of 2:1 to 4:1. The mixing flow-rate of the solutions is in the range of 5 ml/min to 25 ml/min, preferably in a range of 10 ml/min to 20 ml/min.

In a certain embodiment of the invention the organic solvent of the resulting mixture is removed after the mixing step by dialysis or by tangential flow filtration using ultrafiltration membranes, whereby the ultrafiltration membranes can be either hollow fiber membranes or flat-screen membranes. The pore size of the ultrafiltration membrane corresponds to a molecular weight cutoff in the range of 1500 Da to 500.000 Da, preferably in a range of 1500 Da to 100.000 Da and even more preferably in a range of 1500 Da to 30.000 Da. Within the tangential flow filtration step it is also possible to concentrate the LNPs to a desired concentration.

The mean particle size of the resulting LNPs can be measured using dynamic light scattering (DLS) technologies and is preferably in the range of 15 nm to 400 nm, more preferably in a range of 25 nm to 200 nm and even more preferable the particles have a size in the range of 25 nm to 100 nm. The overall particle charge of the LNPs is positive and the Zeta-potential of the particles is preferably in a range between +5 mV to +60 mV, more preferably in a range of +25 mV to +60mV.

In certain embodiments, the delivery vehicle of the invention is a liposomal transfer vehicle, e.g. a lipid nanoparticle or a lipidoid nanoparticle. In one embodiment, the transfer vehicle may be selected and/or prepared to optimize delivery of the recombinant nucleic acid construct of the invention to a target cell. For example, if the target cell is an endothelial cell, the properties of the transfer vehicle (e.g., size, charge and/or pH) may be optimized to effectively deliver such transfer vehicle to the target cell, reduce immune clearance and/or promote retention in that target cell. Alternatively, if the target cell is in the central nervous system (e.g., mRNA such as the recombinant nucleic acid construct of the invention administered for the treatment of neurodegenerative diseases may specifically target brain or spinal tissue), selection and preparation of the transfer vehicle must consider penetration of, and retention within, the blood brain barrier and/or the use of alternate means of directly delivering such transfer vehicle to such target cell. In one embodiment, the compositions of the present invention may be combined with agents that facilitate the transfer of exogenous mRNA (e.g., agents which disrupt or improve the permeability of the blood brain barrier and thereby enhance the transfer of exogenous mRNA to the target cells).

Liposomes (e.g., liposomal lipid nanoparticles) are known to be particularly suitable for their use as transfer vehicles of diagnostic or therapeutic compounds in vivo (Lasic, Trends Biotechnol, 16: 307-321, 1998; Drummond et al, Pharmacol. Rev., 51: 691-743, 1999) and are usually characterized as microscopic vesicles having an interior aqua space sequestered from an outer medium by a membrane of one or more bilayers. Bilayer membranes of liposomes are typically formed by amphiphilic molecules, such as lipids of synthetic or natural origin that comprise spatially separated hydrophilic and hydrophobic domains (Lasic, Trends Biotechnol, 16: 307- 321, 1998). Bilayer membranes of the liposomes can also be formed by amphiphilic polymers and surfactants (e.g., polymerosomes, niosomes, etc.). Such liposomes may also be used as the lipid moiety in the delivery vehicle of the present invention comprising a recombinant nucleic acid construct of the present invention.

It is to be acknowledged that any feature related to the recombinant nucleic acid construct as disclosed in connection with a particular aspect of the present invention is equally an embodiment of the recombinant nucleic acid construct of each and any other aspect of the present invention, including any embodiment thereof. More specifically, any embodiment of the recombinant nucleic acid construct disclosed in connection with the first aspect is also an embodiment of the recombinant nucleic acid construct of the second, third and fourth aspect; similarly, any embodiment of the recombinant nucleic acid construct disclosed in connection with the second, third and fourth aspect is also an embodiment of the recombinant nucleic acid construct of the first aspect.

It is generally acknowledged in the art that the UTRs flanking a coding region do not interfere with the coding region and, more specifically, that the UTRs may, in principle, be exchanged without interfering with the expression of the coding region (see, e.g. WO 2017/100551 A1; Trepotec et al.; Tissue Engineering Part A, Vol 0 Nr ja (Apr. 2018) (https://doi.org/10.1089/ten.TEA.2017.0485).

Without wishing to be bound by any theory, the present inventors have surprisingly found that the recombinant nucleic acid molecule of the present invention and more specifically the mutant effector molecule having a gain of function mutation or a hypermorphic mutation is suitable for increasing the level of protein expression of the effector molecule, for increasing the duration of the expression of the effector molecule and/or for increasing the functional activity of the effector molecule. The use of the mutant effector molecule leads to better therapeutic efficacy compared to the non-mutant effector molecule. Preferably, gain of function mutation can be derived by means of routine measures from sporadic somatic mutation of the non-mutant effector molecule or from inherited germline mutations of the non-mutant effector molecule causing the desired phenotype. Preferably, a mutant effector molecule is one verified by human genetic studies.

It will be appreciated by a person skilled in the art that due to the transient, i.e. timely restricted expression of the effector molecule from the recombinant nucleic acid construct of the present invention, no pathological phenotype will be caused despite the effector molecule in the mutant effector molecule in particular being potentially pathogenic. Rather, the transient increase in activity will provide for a better therapeutic response and/or will require less frequent dosing.

The present invention is further illustrated by the following Figs. and Examples form which further features, embodiments and advantages of the present invention may be taken.
Fig. 1 is a schematic representation of conventional mRNA molecule structure.
Fig. 2 is a schematic representation of selected examples for mRNA constructs with the coding sequence with an open reading frame being the one of Nano-luciferase, namely PAN02, PAN05, PAN07, PAN08, PAN09, PAN10, PAN11, PAN12, 13, PAN28, PAN29, PAN30, PAN31, PAN32, PAN33, PAN34, PAN35, PAN36, PAN37, PAN38, PAN39, PAN40, PAN41, PAN42, PAN43, PAN44, PAN45, PAN46, PAN47, PAN48 and PAN49.
Fig. 3 is a restriction map of plasmid pcDNA3.1(-) containing construct PAN11.
Fig. 4 is a restriction digest map of plasmid pdDNA3.1(-) of Fig. 3.
Fig. 5 is an 1% agarose gel stained with EtBr showing the non-linearized (uncut, supercoiled, right lanes) and linearization product (left lanes) of pcDNA3.1(-) plasmids containing constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05") upon BamHI restriction.
Fig. 6 shows 1% agarose gels stained with EtBr showing PCR products of Poly-A tailing PCRs for the addition of 120 nt of Poly-A tail.
Fig. 7A shows the 5' primers used for the 5' UTRs of the indicated gene; the first column indicates in connection with which construct such primer is to be used, the second column indicates the origin of the 5' UTR, and the third column indicates the position where the primer hybridizes to the nucleotide sequence.
Fig. 7B shows the 3' primers used for the 3' UTRs of the indicated gene; the first column indicates in connection with which construct such primer is to be used, the second column indicates the origin of the 3' UTR, and the third column indicates the position where the primer hybridizes to the nucleotide sequence.
Fig. 8 is an image of a non-denaturing agarose gel after EtBr staining showing the *in vitro* mRNA transcripts of various construct.

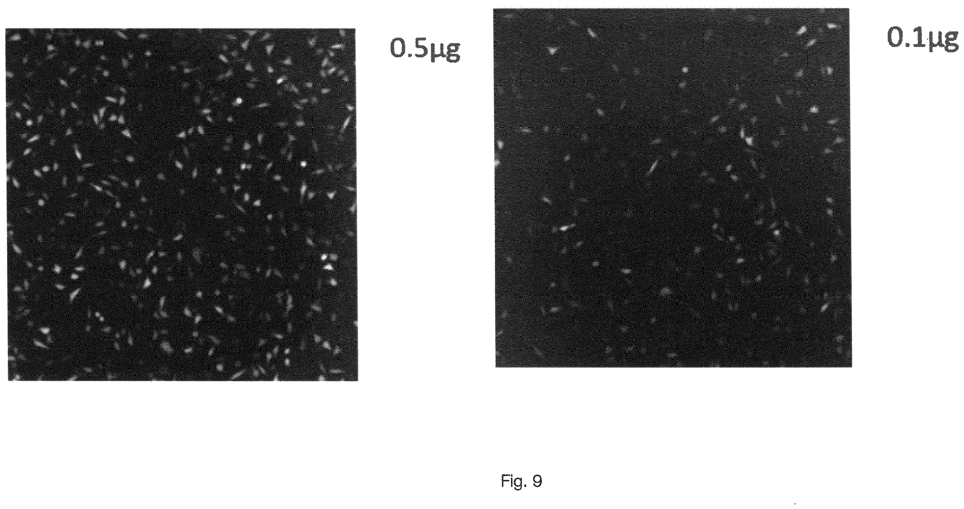

Fig. 9 are photographs of fluorescence microscopy 24 h post transfection of HeLa cells transfected with 0.5 µg (left) and 0.1 µg (right) of a commercially, enhanced mRNA version of the producing green fluorescent protein (TriLINK Biotechnology).
Fig. 10 is a bar diagram showing expression of luciferase, indicated as RLU (relative light units) of luciferase in medium, by the indicated recombinant nucleic acid constructs in HPMEC where luciferase is used as the effector molecule after 2 hours (left column), 6 hours (middle column) and 24 hours (right column) post transfection.
Fig. 11 is a bar diagram showing expression of luciferase, indicated as RLU (relative light units) of luciferase in whole cell ly sates of HPMEC, by the indicated recombinant nucleic acid constructs in HPMEC, where luciferase is used as the effector molecule after, 6 hours (left column) and 24 hours (right column) post transfection.
Fig. 12 is a bar diagram showing expression of luciferase, indicated as RLU (relative light units) of luciferase in medium, by the indicated recombinant nucleic acid constructs in HUVEC where luciferase is used as the effector molecule after 2 hours (left column), 6 hours (middle column) and 24 hours (right column) post transfection.
Fig. 13 is a bar diagram showing expression of luciferase, indicated as RLU (relative light units) of luciferase in whole cell lysates of HUVEC, by the indicated recombinant nucleic acid constructs after lysis of HUVEC, where luciferase is used as the effector molecule after 2 hours (left column), 6 hours (middle column) and 24 hours (right column) post transfection.
Fig. 14 is a bar diagram showing expression of luciferase, indicated as RLU [(relative light units) of luciferase in medium, by the indicated recombinant nucleic acid constructs in HeLa cells where luciferase is used as the effector molecule after 2 hours (left column), 6 hours (middle column) and 24 hours (right column) post transfection.

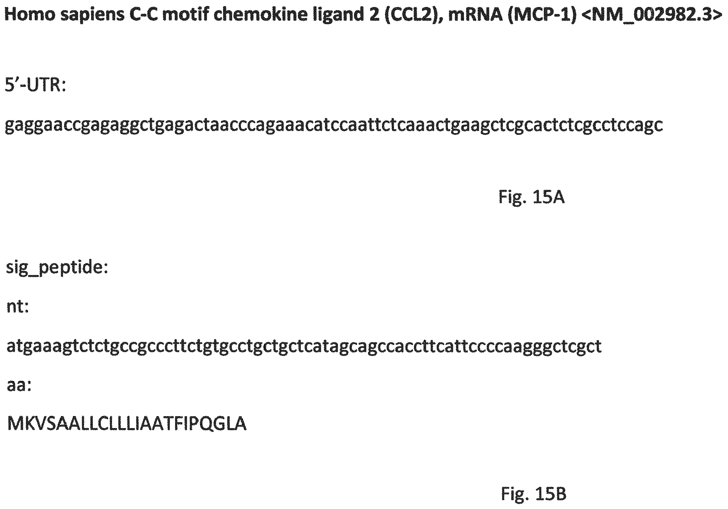

Fig. 15A shows the 5' UTR nucleotide sequence of the mRNA of human MCP-1 which is also referred to as homo sapiens C-C motif chemokine ligand 2 (CCL2) (GenBank entry NM_002982.3).
Fig. 15B shows both the nucleotide sequence and the amino acid sequence of the signal peptide sequence of human MCP-1.

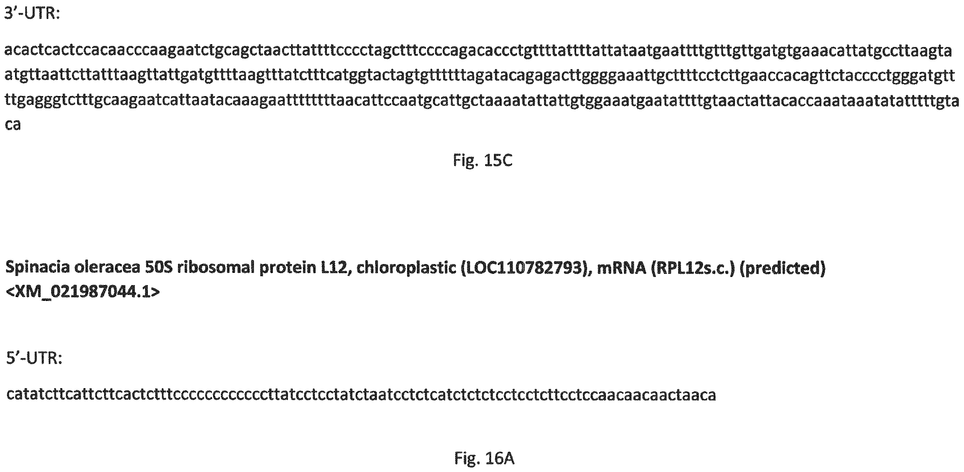

Fig. 15C shows the 3' UTR nucleotide sequence of the mRNA of human MCP-1.
Fig. 16A shows the 5' UTR nucleotide sequence of the mRNA of the 50S ribosomal protein L12, chloroplastic (LOC110782793), of Spinacia oleracea which is also referred to as RPL12s.c. (GenBank entry XM_021987044.1).

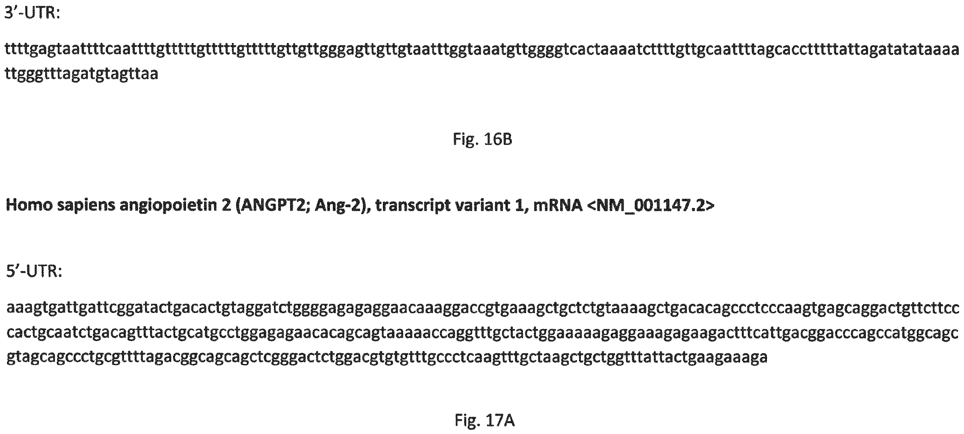

Fig. 16B shows the 3' UTR from the mRNA of the 50S ribosomal protein L12, chloroplastic (LOC110782793), of Spinacia oleracea.
Fig. 17A shows the 5' UTR nucleotide sequence of the mRNA of human angiopoietin 2 which is also referred to as ANGPT2 or Ang-2, transcript variant 1 (GenBank entry NM_001147.2).

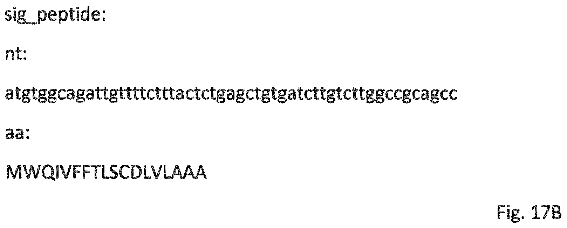

Fig. 17B shows both the nucleotide sequence and the amino acid sequence of the signal sequence of the mRNA of human Ang-2.

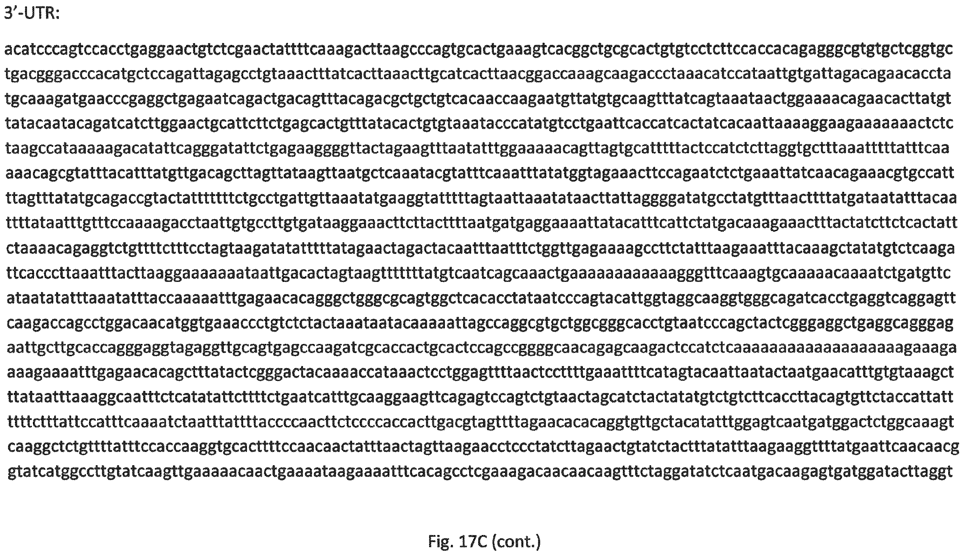

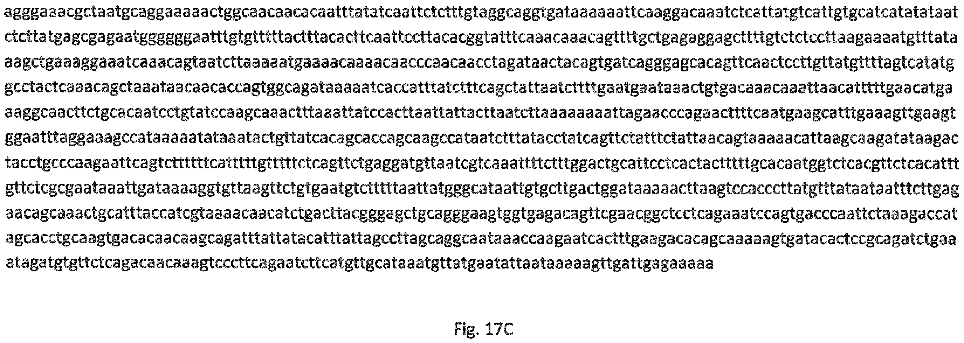

Fig. 17C shows the 3' UTR nucleotide sequence of the mRNA of human Ang-2.

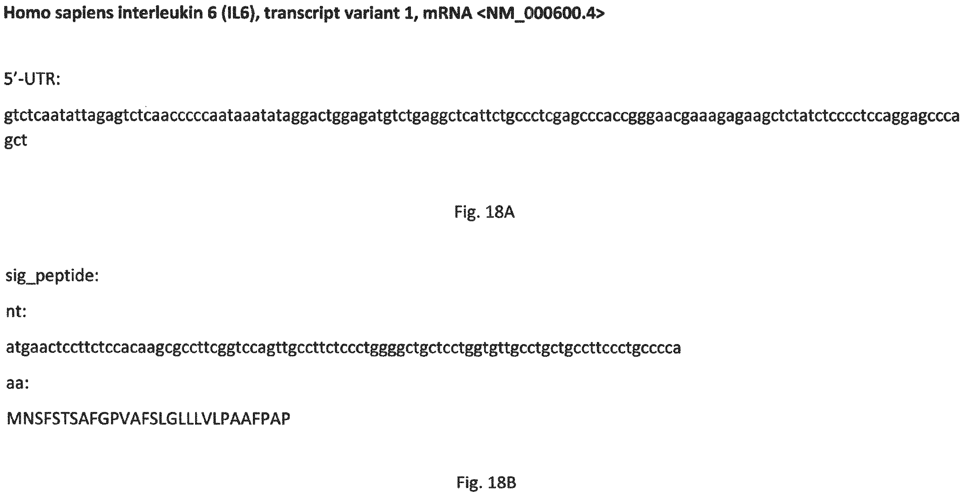

Fig. 18A shows the 5' UTR nucleotide sequence of the mRNA of human interleukin 6 (IL-6), transcript variant 1 (GenBank entry NM_000600.4).
Fig. 18B shows both the nucleotide sequence and the amino acid sequence of the signal sequence of the mRNA of human IL-6.

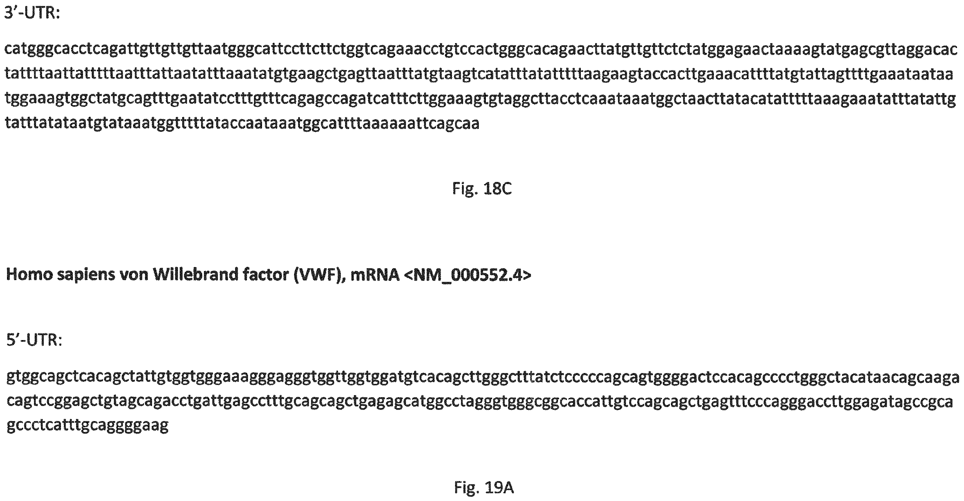

Fig. 18C shows the 3' UTR nucleotide sequence of the mRNA of human IL-6.
Fig. 19A shows the 5' UTR nucleotide sequence of the mRNA of human von Willebrand factor (vWF) (GenBank entry NM_000552.4).

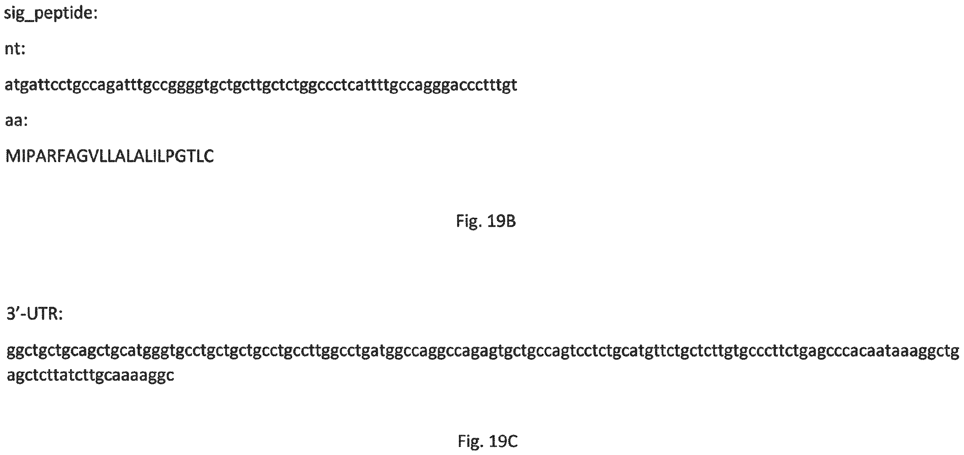

Fig. 19B shows both the nucleotide sequence and the amino acid sequence of the signal sequence of the mRNA of von Willebrand factor (vWF).
Fig. 19C shows the 3' UTR nucleotide sequence of the mRNA of von Willebrand factor (vWF).

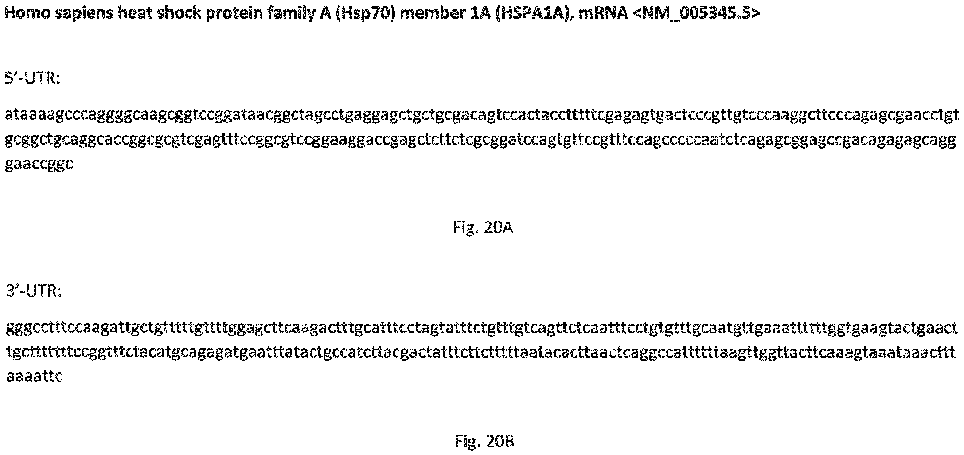

Fig. 20A shows the 5' UTR nucleotide sequence of the mRNA of human heat shock protein family A (Hsp70) member 1A, also referred to as HSPA1A (GenBank entry NM_005345.5).
Fig. 20B shows the 3' UTR nucleotide sequence of the mRNA of human HSPA1A.
Fig. 21A shows the 5' UTR nucleotide sequence of the mRNA of human heat shock protein family A (Hsp70) member 5, also referred to as HSPA5, (GenBank entry NM_005347.4).
Fig. 21B shows both the nucleotide sequence and the amino acid sequence of the signal sequence of the mRNA of human HSPA5.

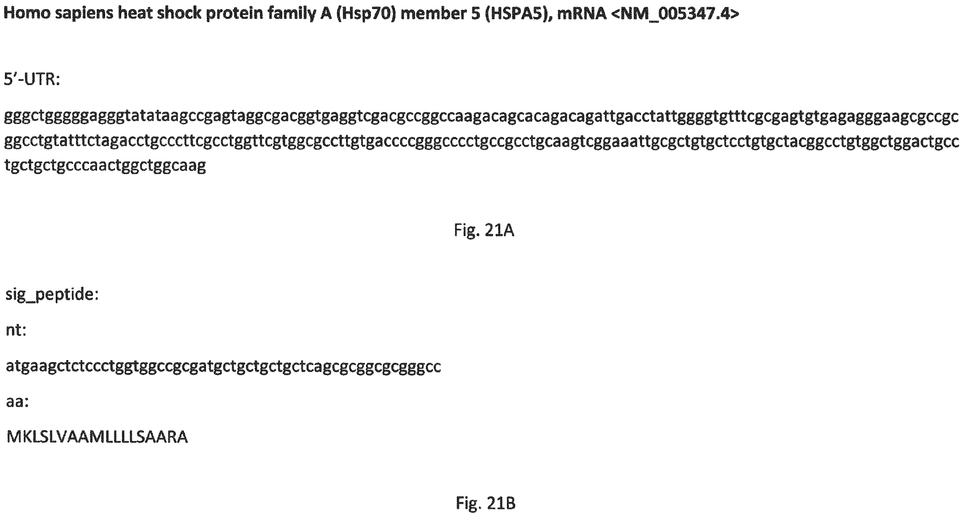

Fig. 21B shows both the nucleotide sequence and the amino acid sequence of the signal sequence of the mRNA of human HSPA5.

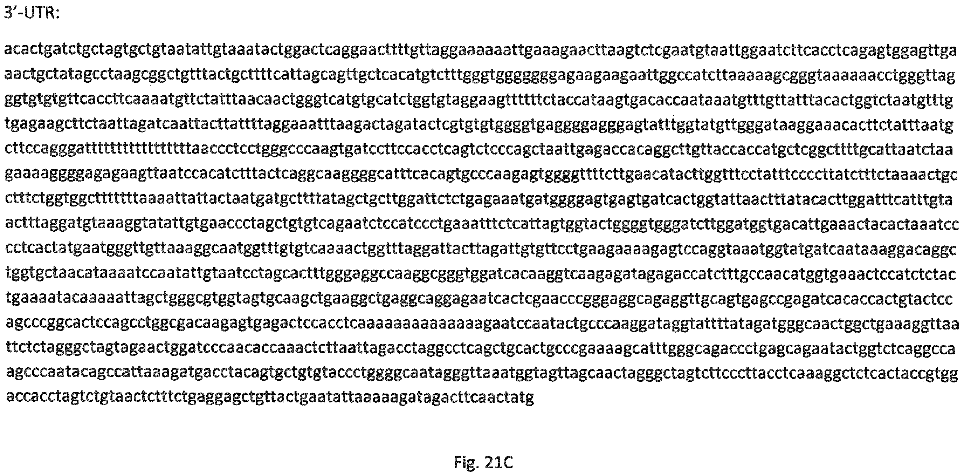

Fig. 21C shows the 3' UTR nucleotide sequence of the mRNA of human HSPA1A.

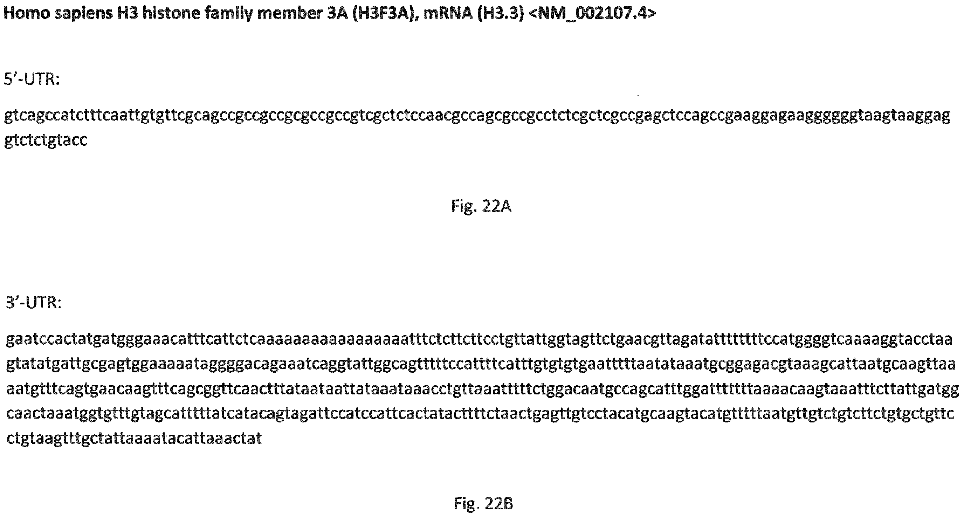

Fig. 22A shows the 5' UTR nucleotide sequence of the mRNA of human H3 histone family member 3A (H3F3A), also referred to as H3.3, (GenBank entry NM_002107.4).
Fig. 22B shows the 3' UTR nucleotide sequence of the mRNA of human H3.3.

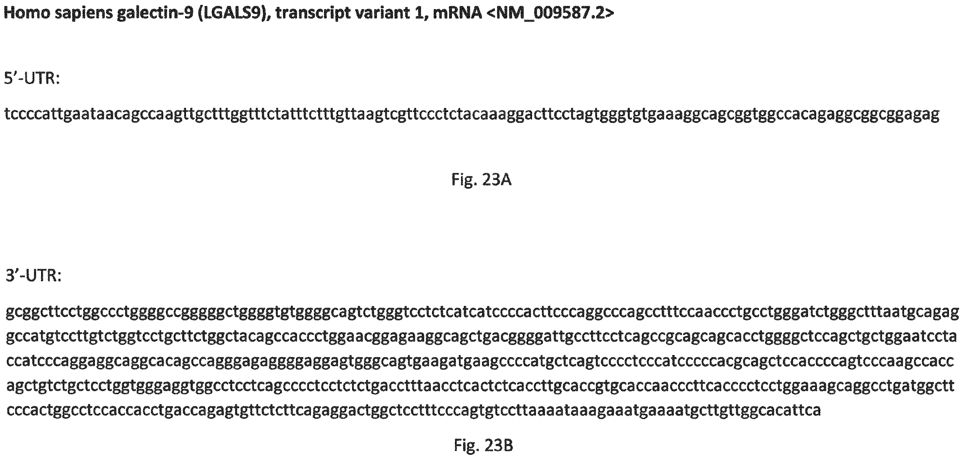

Fig. 23A shows the 5' UTR nucleotide sequence of the mRNA of human galectin-9 (LGALS9), transcript variant 1 (GenBank entry NM_009587.2).
Fig. 23B shows the 3' UTR nucleotide sequence of the mRNA of human galectin-9.

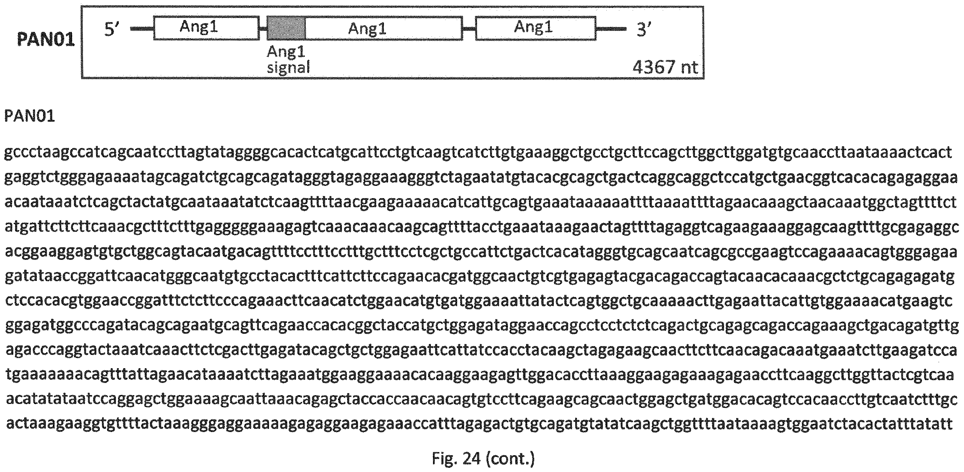

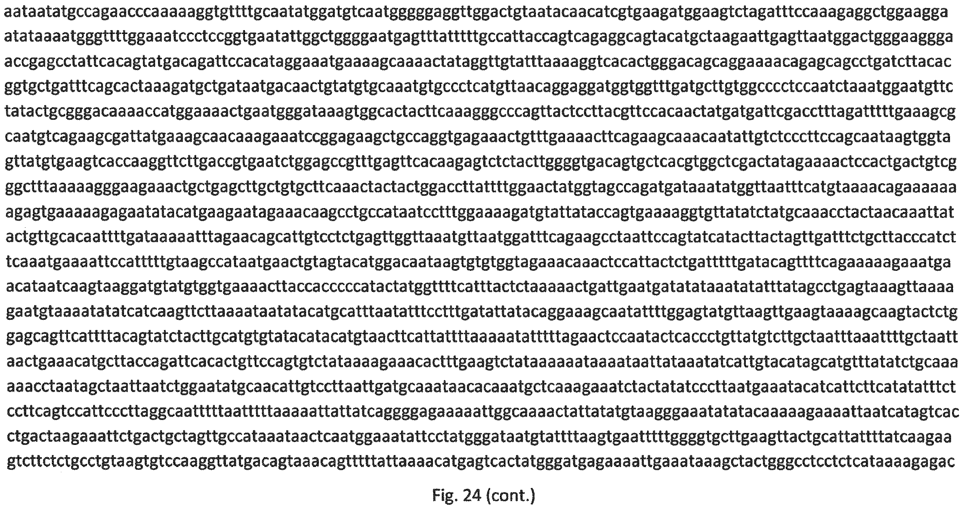

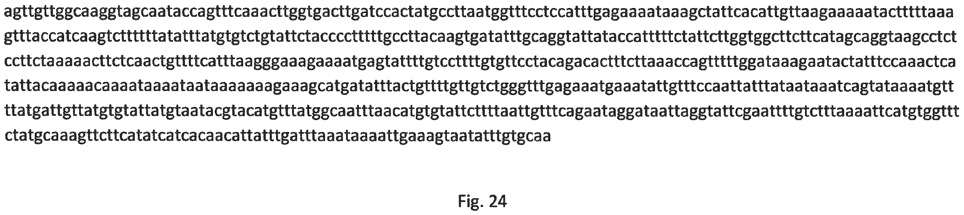

Fig. 24 shows the basic structure and nucleotide sequence of recombinant nucleic acid construct PAN01.

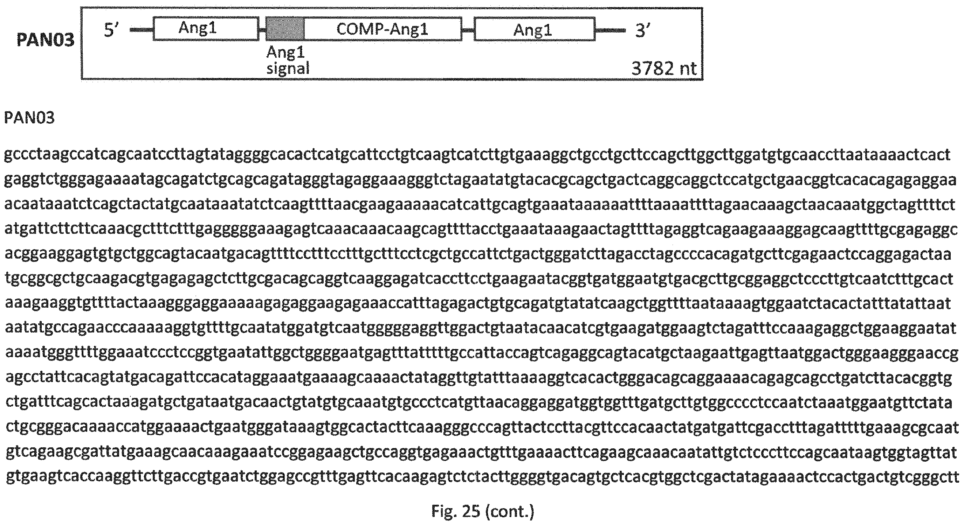

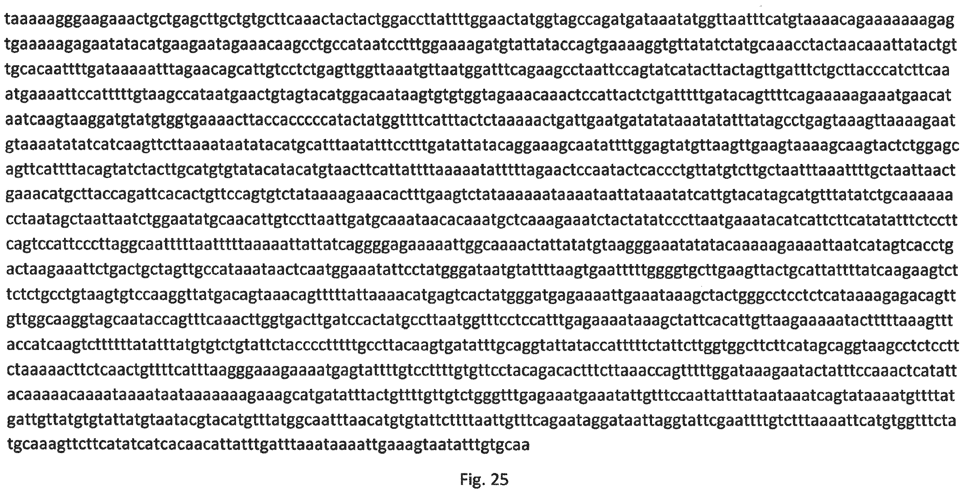

Fig. 25 shows the basic structure and nucleotide sequence of recombinant nucleic acid construct PAN03.

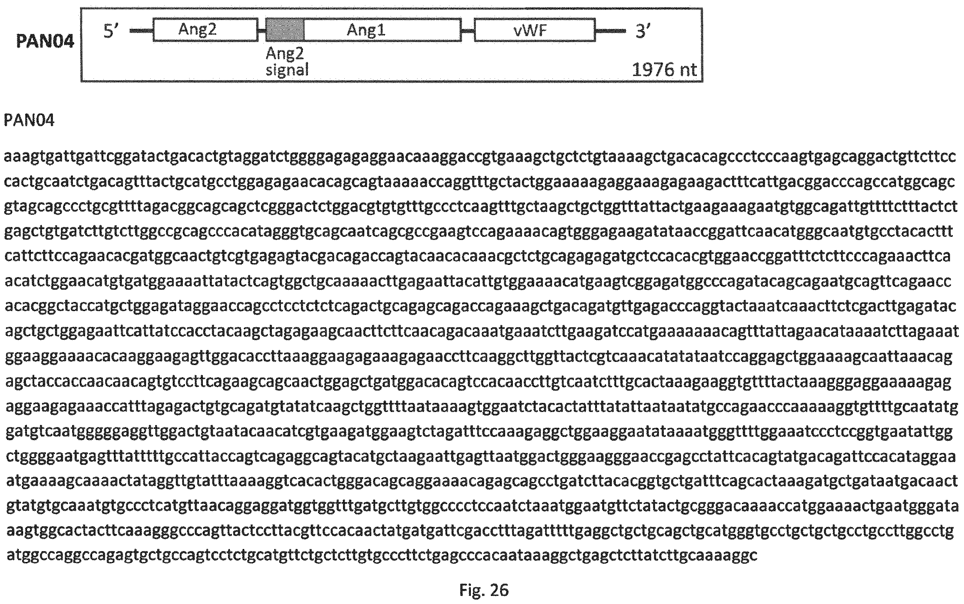

Fig. 26 shows the basic structure and nucleotide sequence of recombinant nucleic acid construct PAN04.

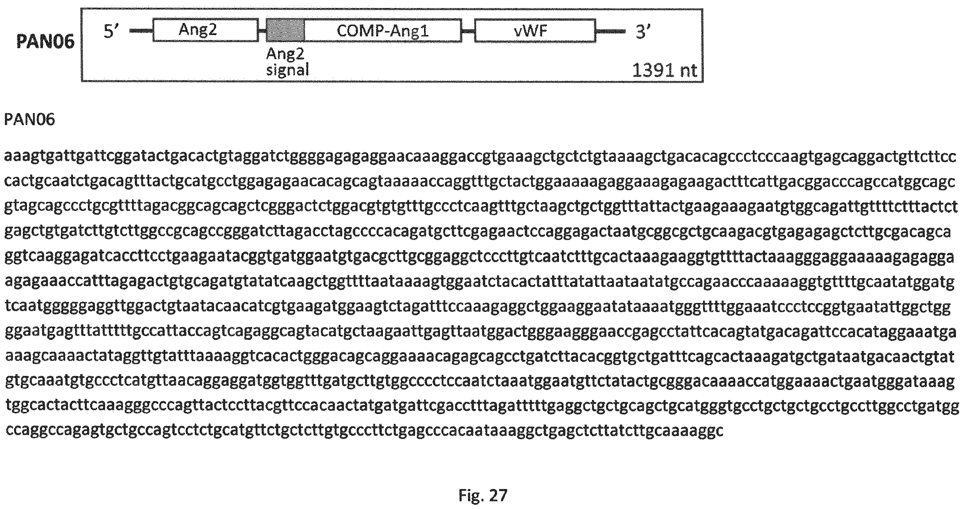

Fig. 27 shows the basic structure and nucleotide sequence of recombinant nucleic acid construct PAN06.

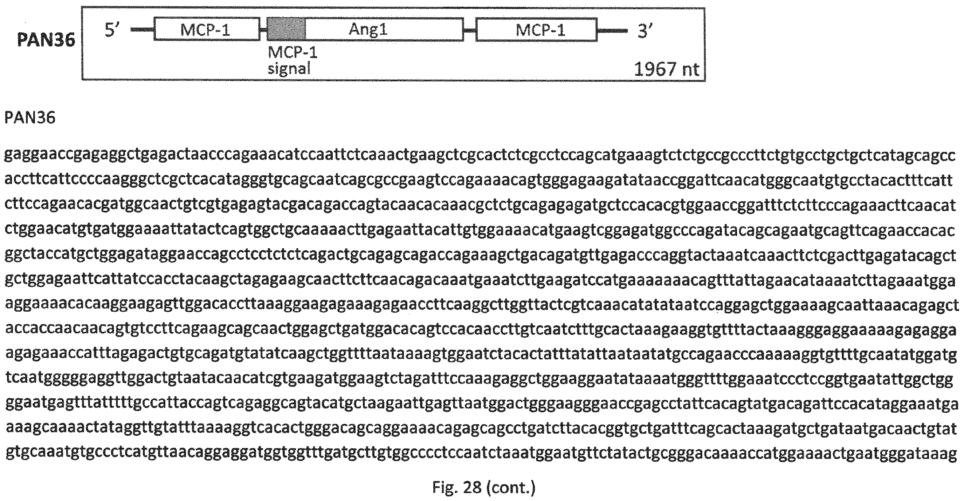

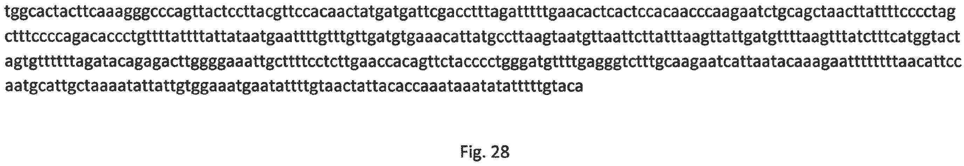

Fig. 28 shows the basic structure and nucleotide sequence of recombinant nucleic acid construct PAN36.

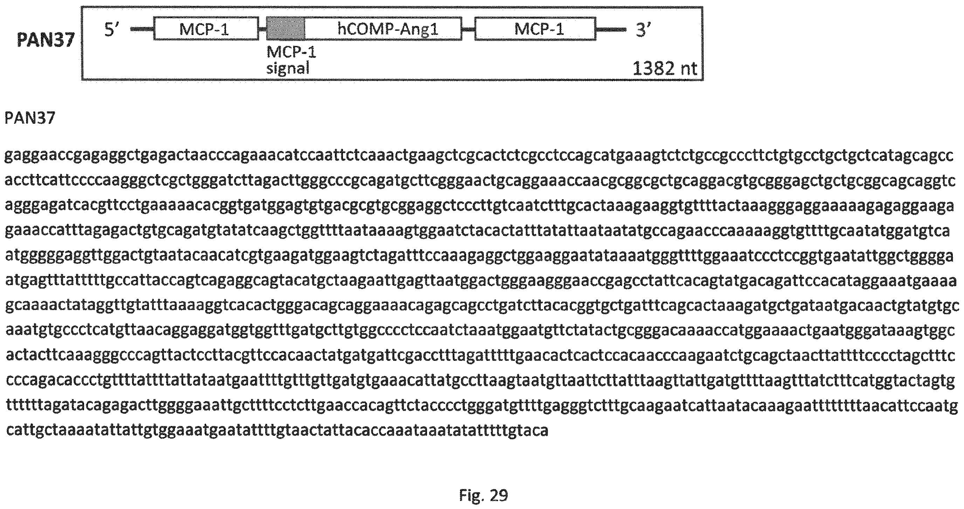

Fig. 29 shows the basic structure and nucleotide sequence of recombinant nucleic acid construct PAN37.

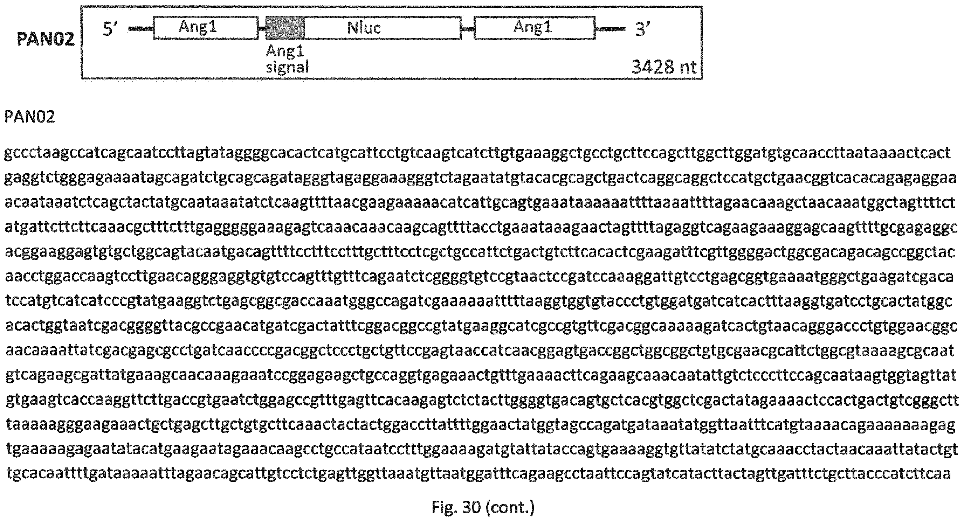

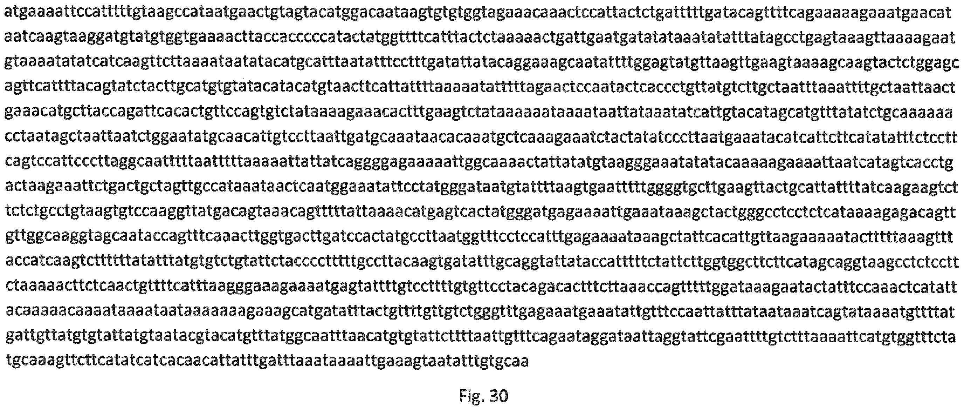

Fig. 30 shows the basic structure and nucleotide sequence of recombinant nucleic acid construct PAN02.

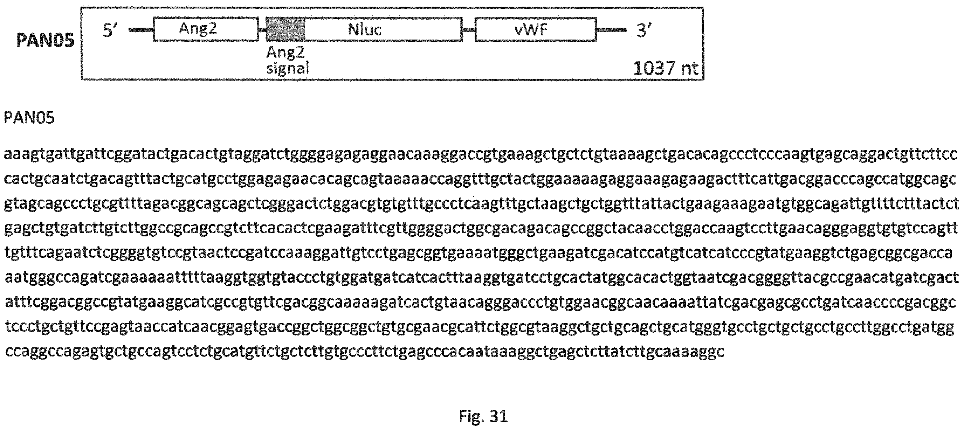

Fig. 31 shows the basic structure and nucleotide sequence of recombinant nucleic acid construct PAN05.

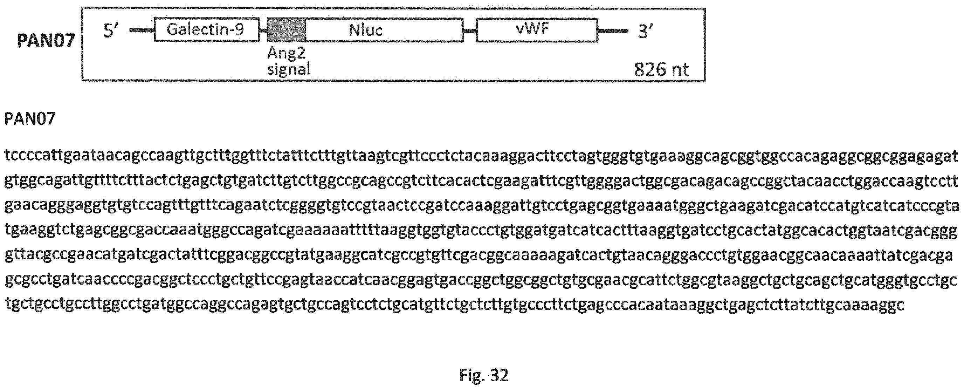

Fig. 32 shows the basic structure and nucleotide sequence of recombinant nucleic acid construct PAN07.

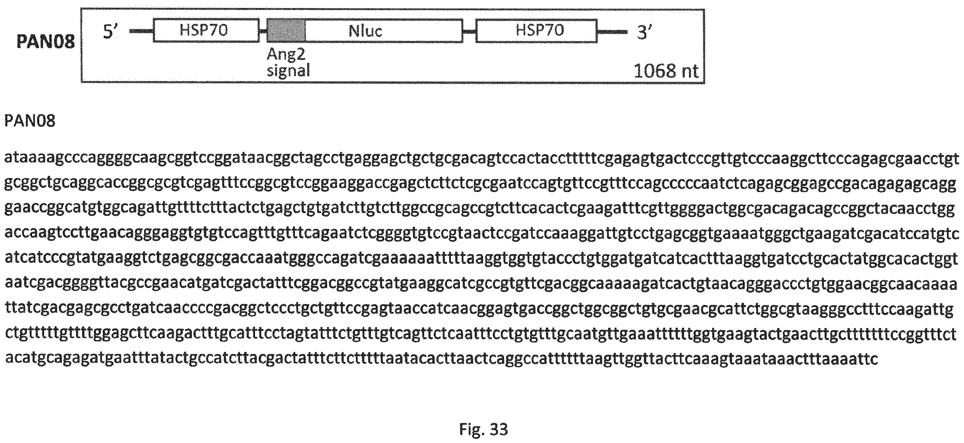

Fig. 33 shows the basic structure and nucleotide sequence of recombinant nucleic acid construct PAN08.

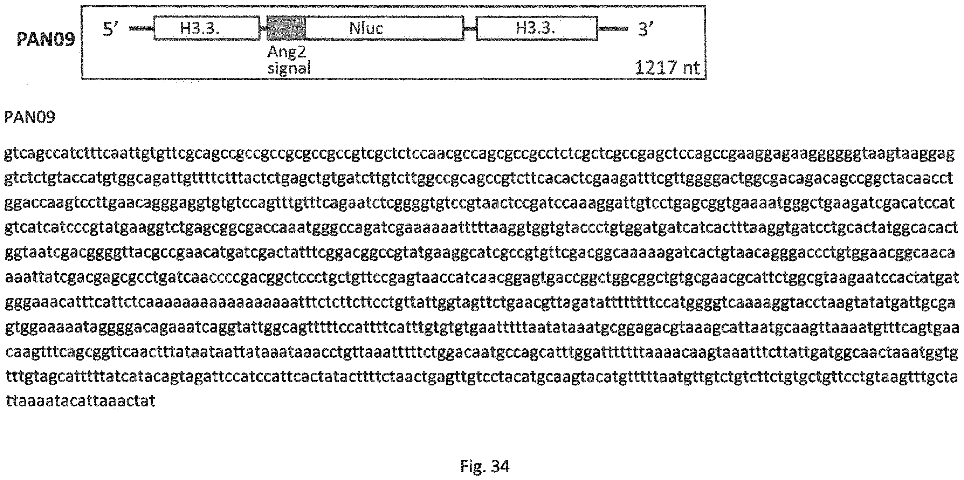

Fig. 34 shows the basic structure and nucleotide sequence of recombinant nucleic acid construct PAN09.

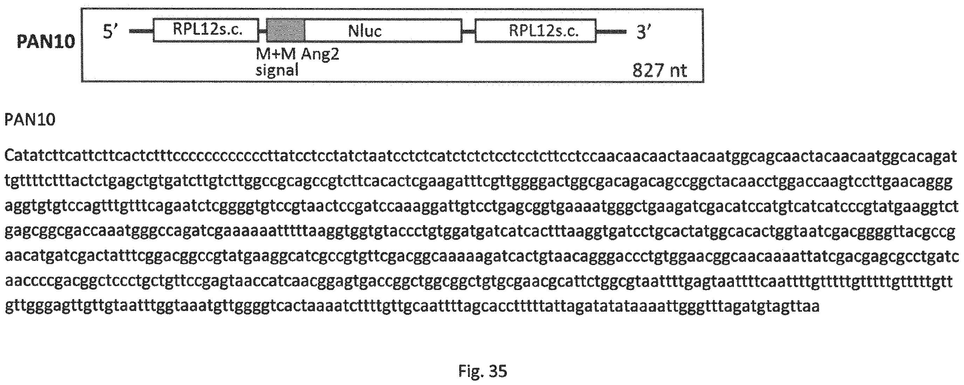

Fig. 35 shows the basic structure and nucleotide sequence of recombinant nucleic acid construct PAN10.

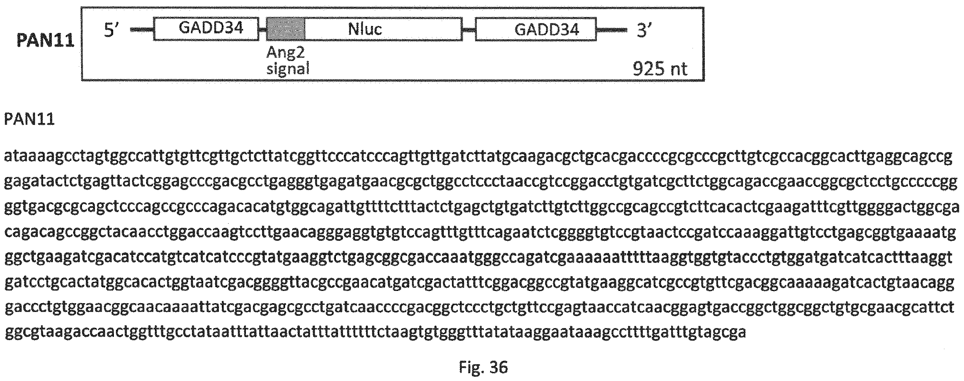

Fig. 36 shows the basic structure and nucleotide sequence of recombinant nucleic acid construct PAN11.
Fig. 37 shows the basic structure and nucleotide sequence of recombinant nucleic acid construct PAN12.

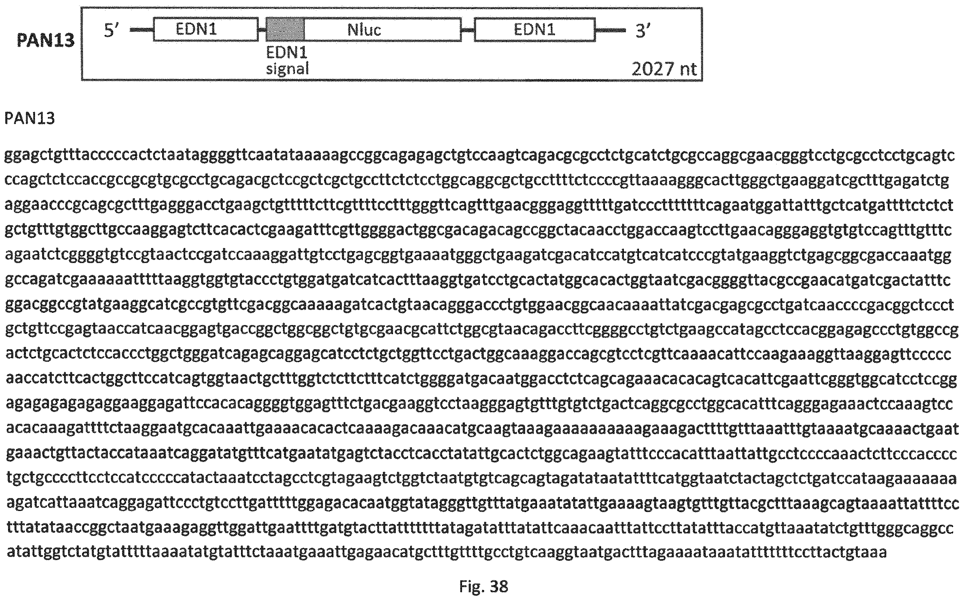

Fig. 38 shows the basic structure and nucleotide sequence of recombinant nucleic acid construct PAN13.
Fig. 39 shows the basic structure and nucleotide sequence of recombinant nucleic acid construct PAN28.
Fig. 40 shows the basic structure and nucleotide sequence of recombinant nucleic acid construct PAN29.

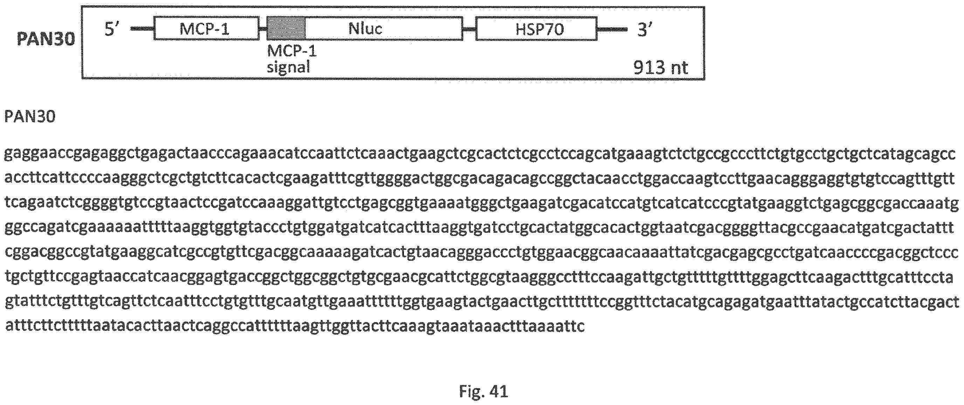

Fig. 41 shows the basic structure and nucleotide sequence of recombinant nucleic acid construct PAN30.

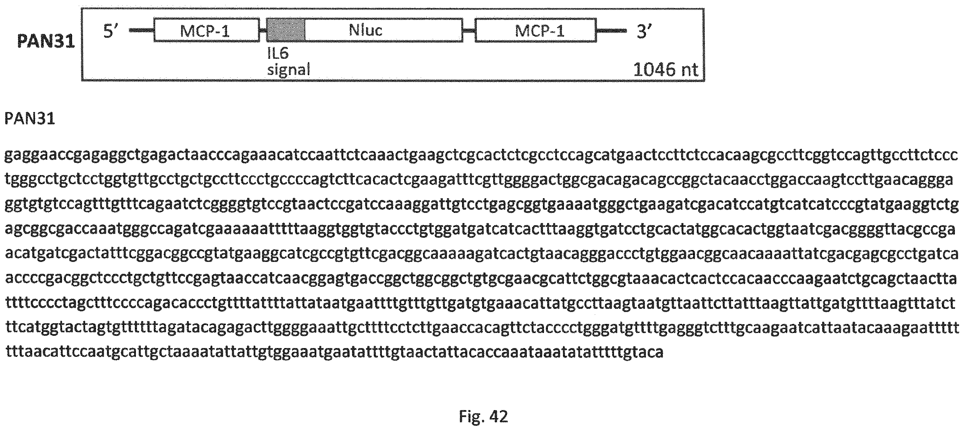

Fig. 42 shows the basic structure and nucleotide sequence of recombinant nucleic acid construct PAN31.

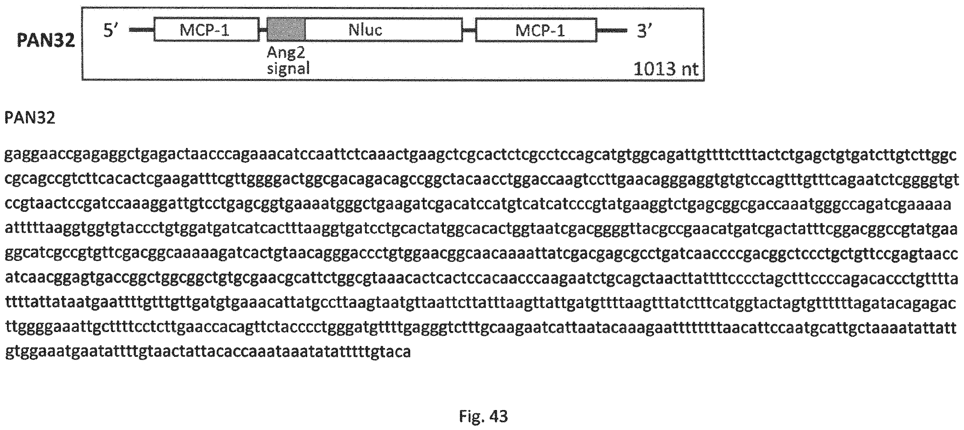

Fig. 43 shows the basic structure and nucleotide sequence of recombinant nucleic acid construct PAN32.
Fig. 44 shows the basic structure and nucleotide sequence of recombinant nucleic acid construct PAN33.
Fig. 45 shows the basic structure and nucleotide sequence of recombinant nucleic acid construct PAN34.

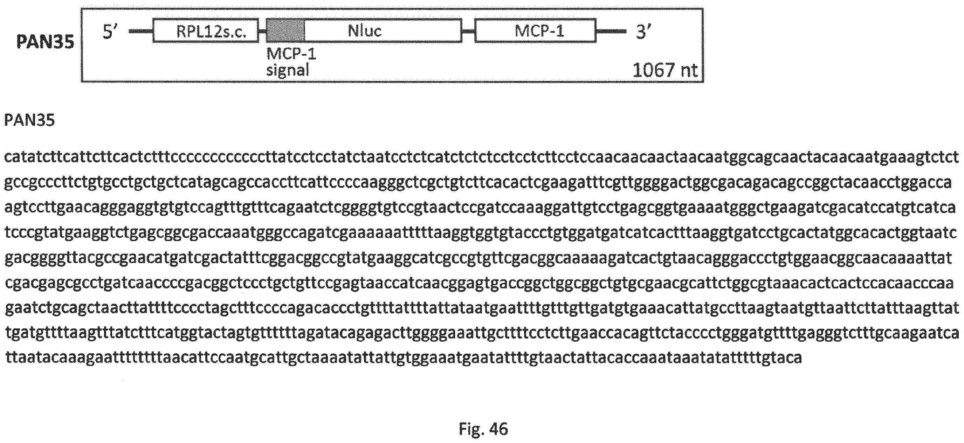

Fig. 46 shows the basic structure and nucleotide sequence of recombinant nucleic acid construct PAN35.

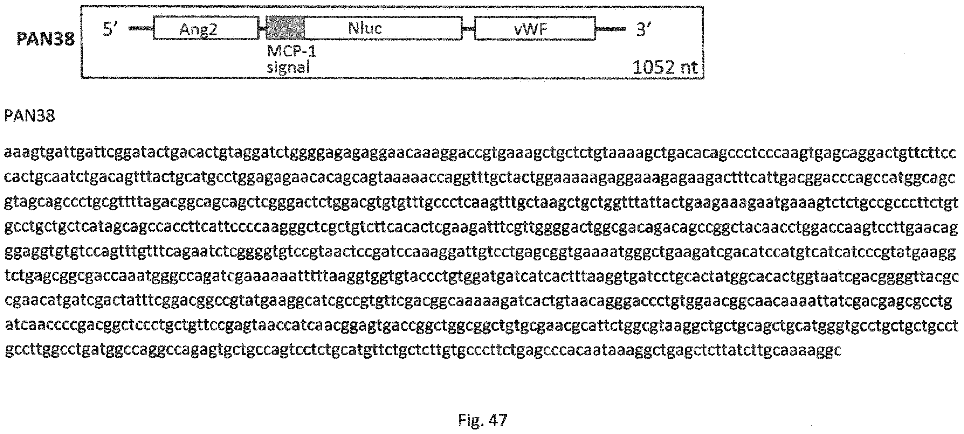

Fig. 47 shows the basic structure and nucleotide sequence of recombinant nucleic acid construct PAN38.

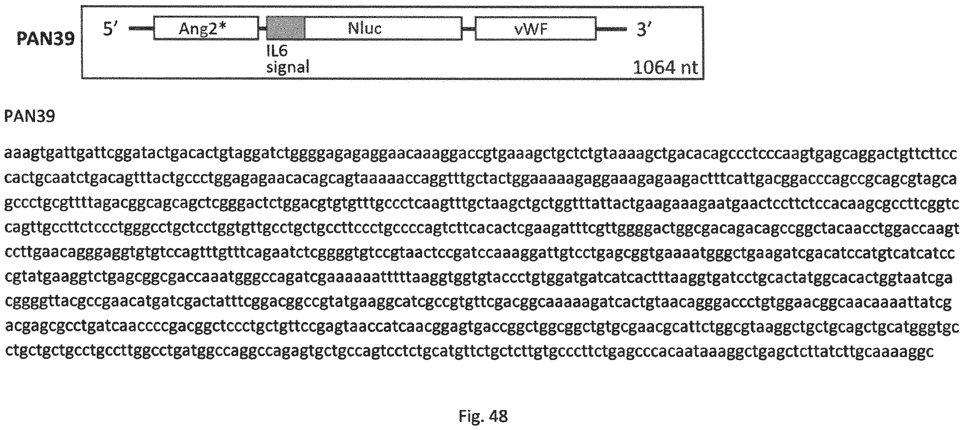

Fig. 48 shows the basic structure and nucleotide sequence of recombinant nucleic acid construct PAN39.

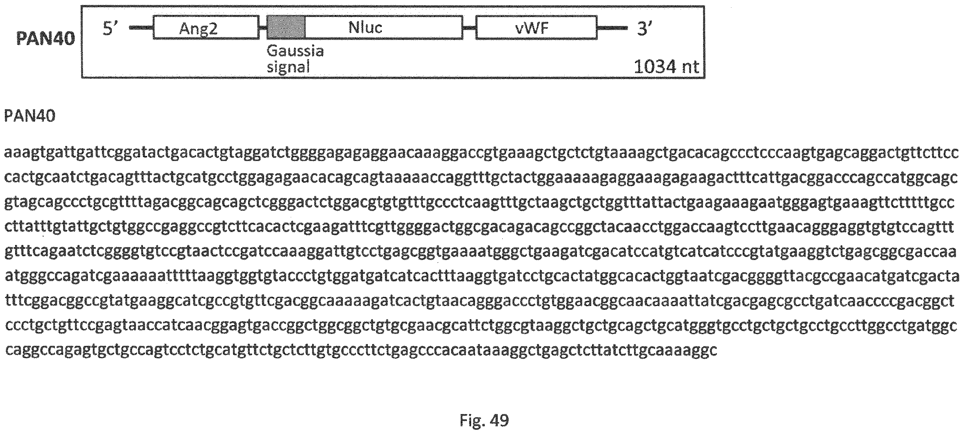

Fig. 49 shows the basic structure and nucleotide sequence of recombinant nucleic acid construct PAN40.

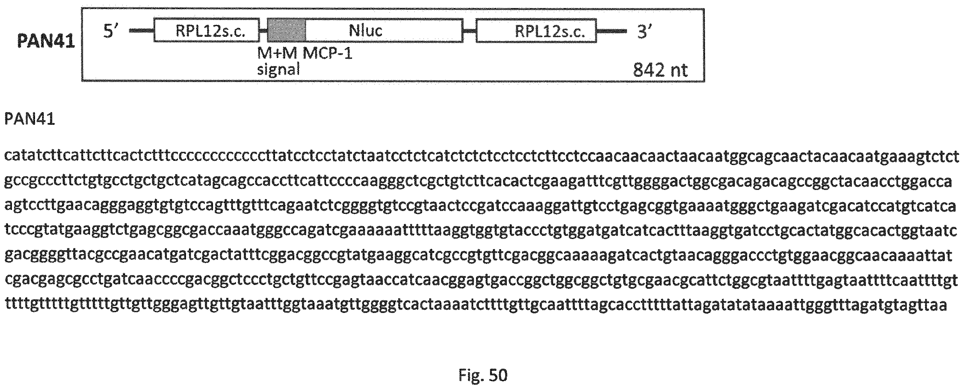

Fig. 50 shows the basic structure and nucleotide sequence of recombinant nucleic acid construct PAN41.

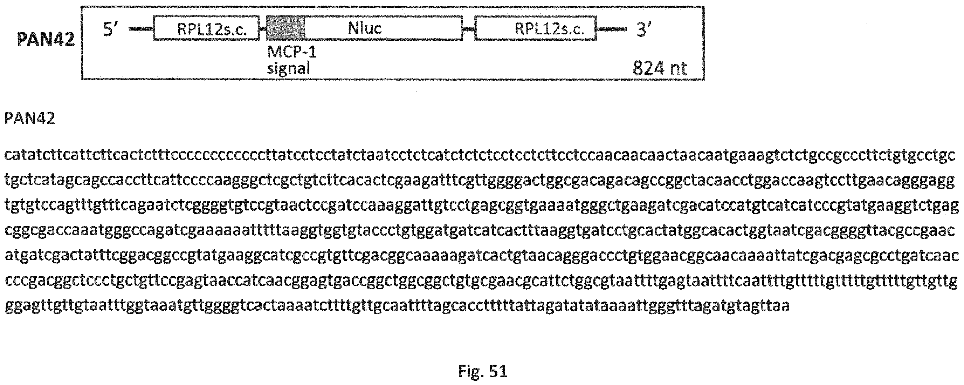

Fig. 51 shows the basic structure and nucleotide sequence of recombinant nucleic acid construct PAN42.

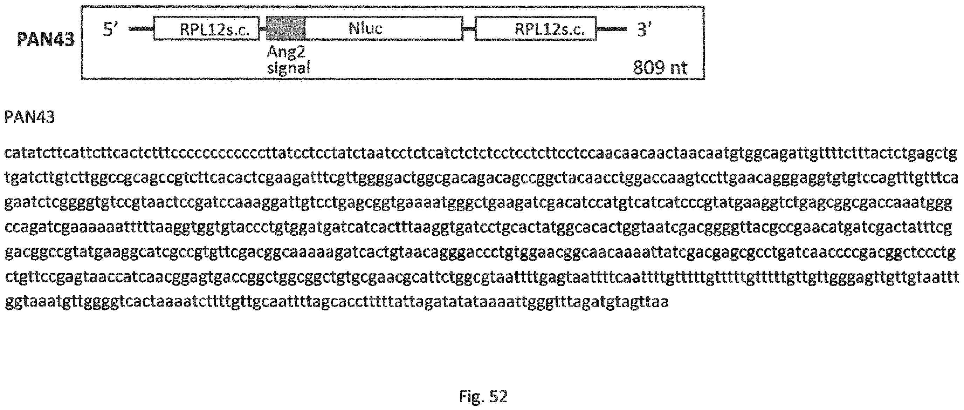

Fig. 52 shows the basic structure and nucleotide sequence of recombinant nucleic acid construct PAN43.

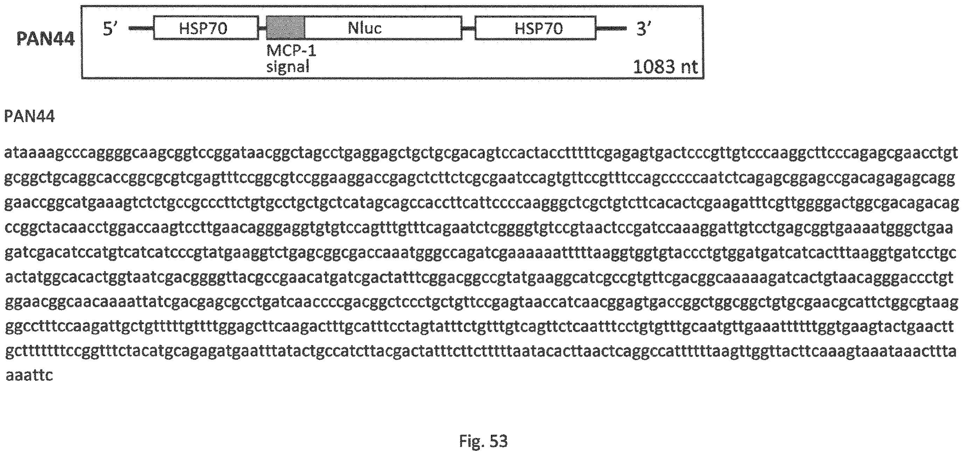

Fig. 53 shows the basic structure and nucleotide sequence of recombinant nucleic acid construct PAN44.

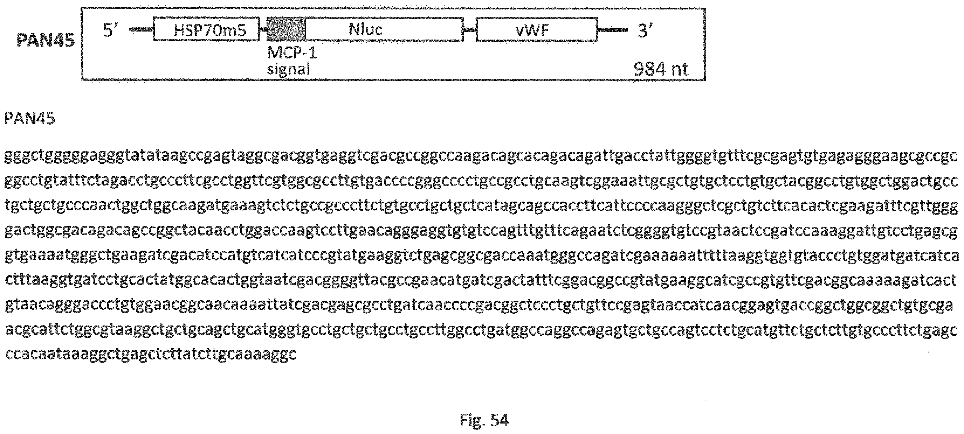

Fig. 54 shows the basic structure and nucleotide sequence of recombinant nucleic acid construct PAN45.

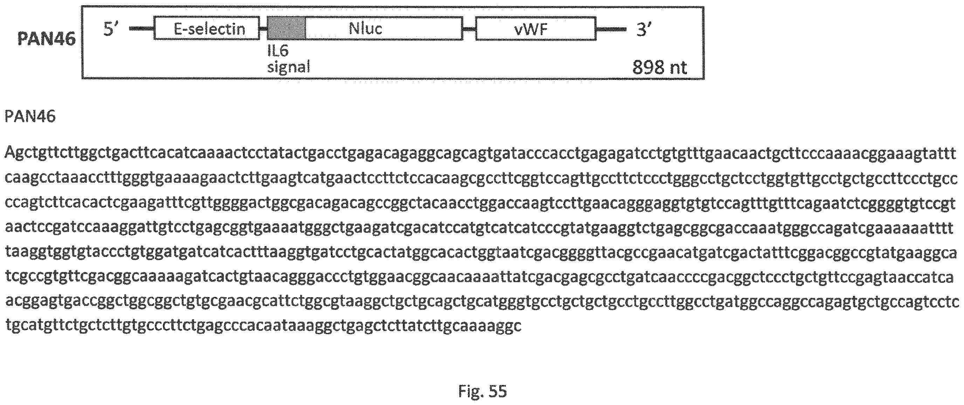

Fig. 55 shows the basic structure and nucleotide sequence of recombinant nucleic acid construct PAN46.

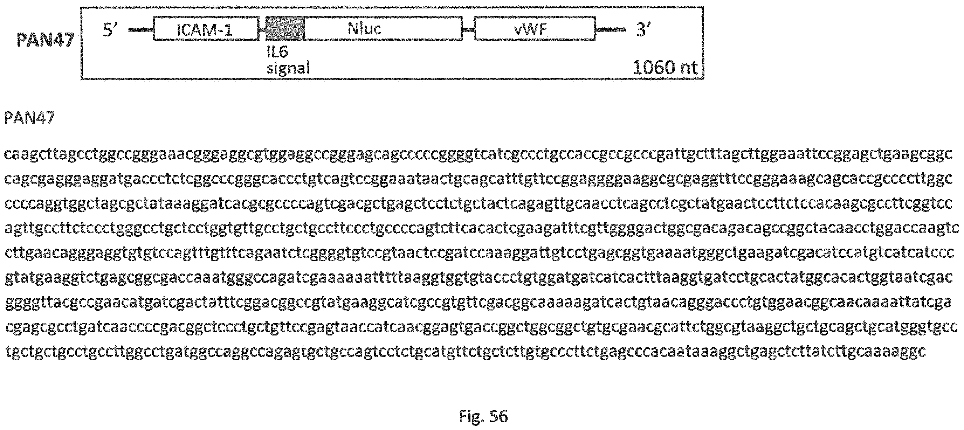

Fig. 56 shows the basic structure and nucleotide sequence of recombinant nucleic acid construct PAN47.

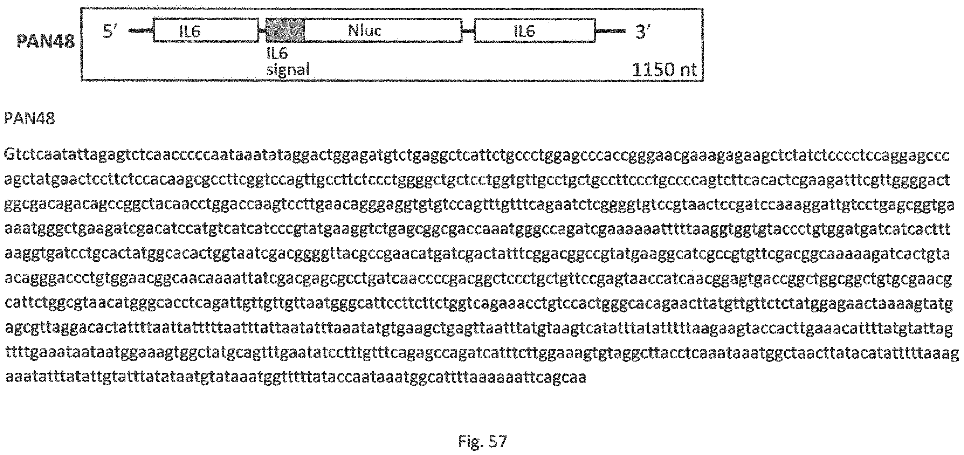

Fig. 57 shows the basic structure and nucleotide sequence of recombinant nucleic acid construct PAN48.

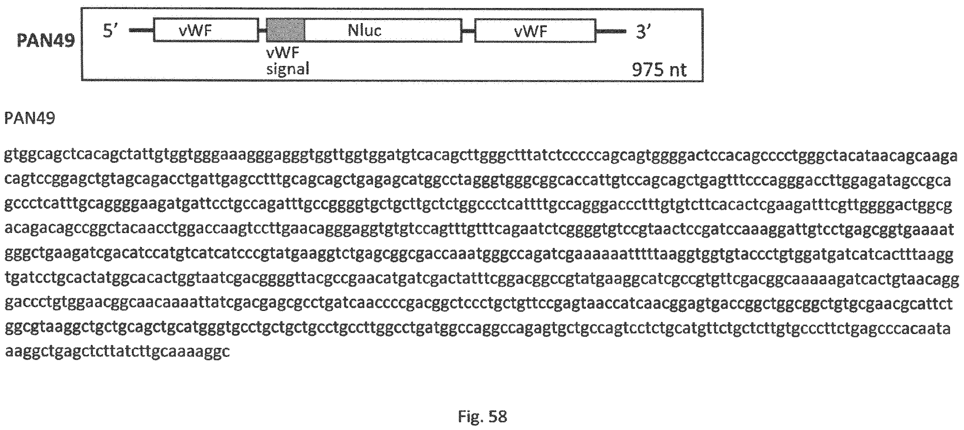

Fig. 58 shows the basic structure and nucleotide sequence of recombinant nucleic acid construct PAN49.

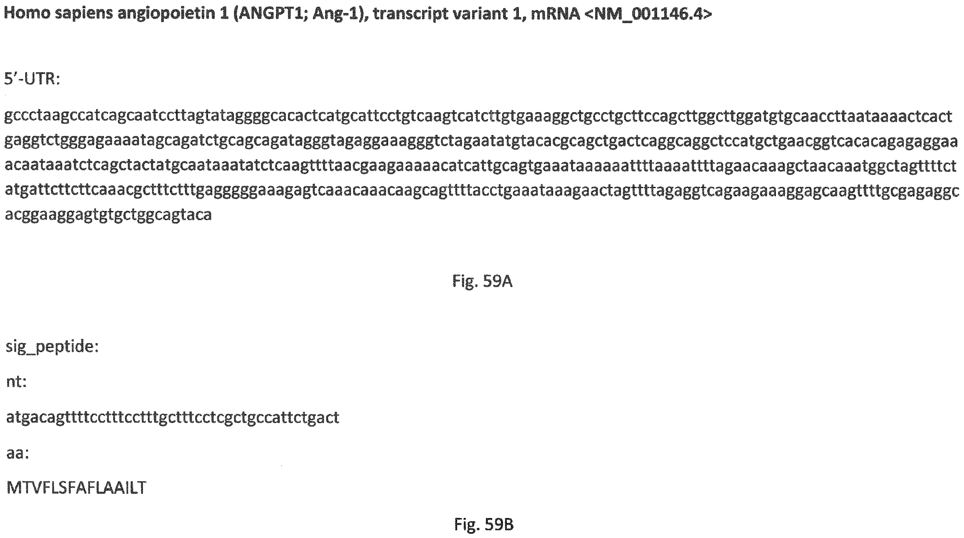

Fig. 59A shows the nucleotide sequence of mRNA of human angiopoietin 1, also referred to as ANGPT1 or Ang-1, transcript variant 1, GenBank entry NM_001146.4.
Fig. 59B shows both the nucleotide sequence and the amino acid sequence of the mRNA of human Ang-1.

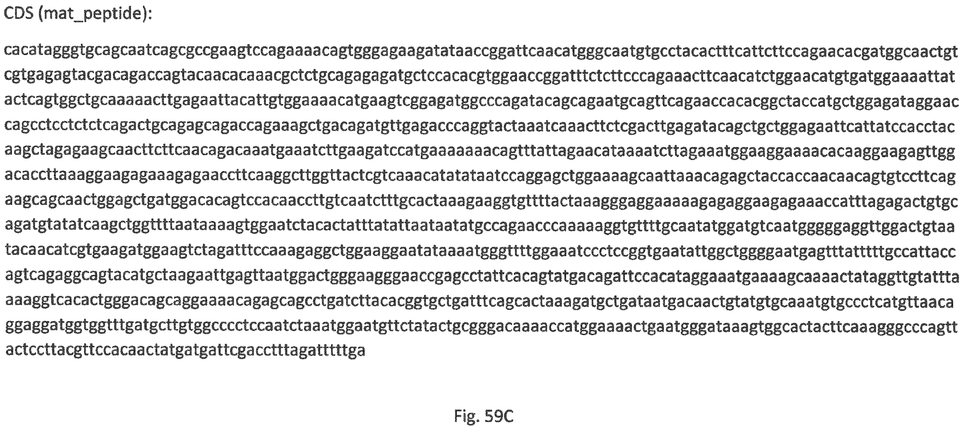

Fig. 59C shows the coding sequence (CDS) of the mature peptide of mRNA of human Ang-1.

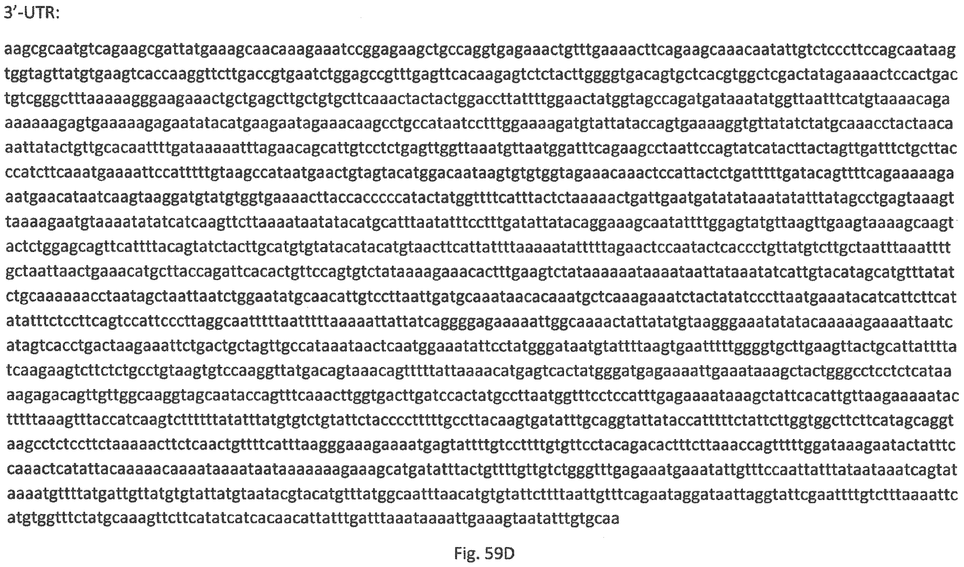

Fig. 59D shows the 3' UTR nucleotide sequence of the mRNA of human Ang-1.

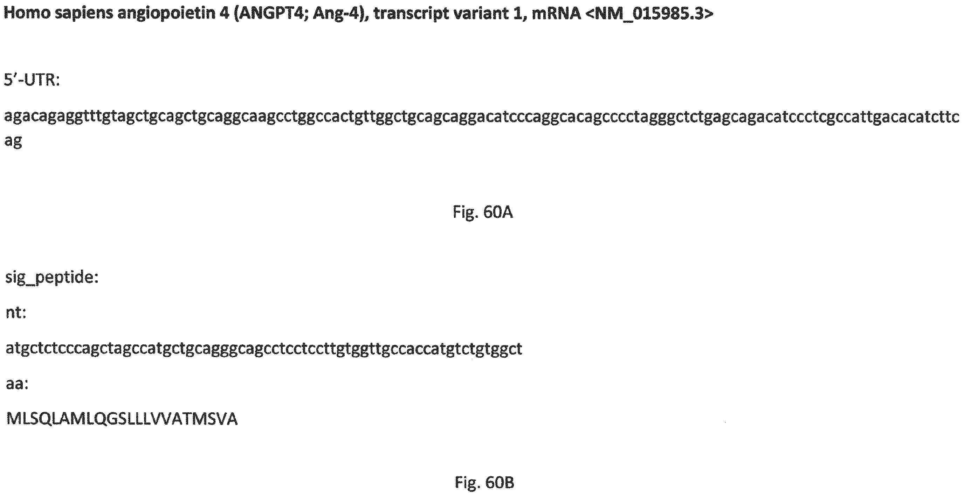

Fig. 60A shows the nucleotide sequence of mRNA of human angiopoietin 4, also referred to as ANGPT4 or Ang-4, transcript variant 1, GenBank entry NM_015985.3.
Fig. 60B shows both the nucleotide sequence and the amino acid sequence of the mRNA of human Ang-4.

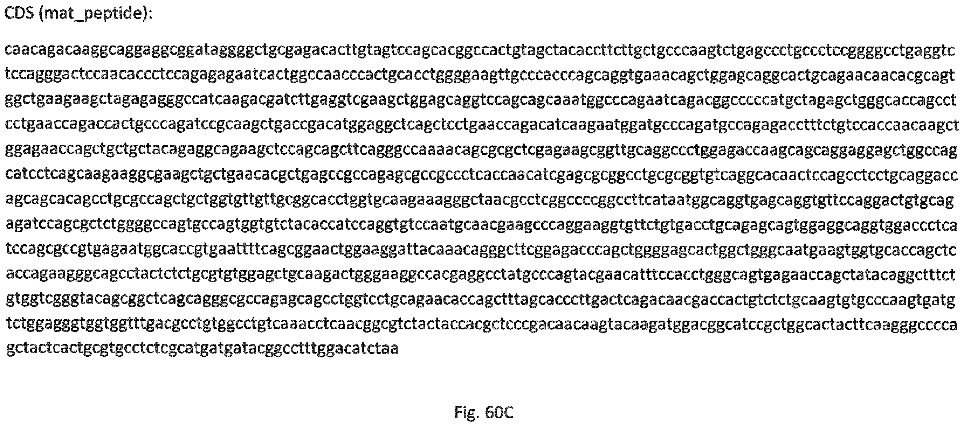

Fig. 60C shows the coding sequence (CDS) of the mature peptide of mRNA of human Ang-4.

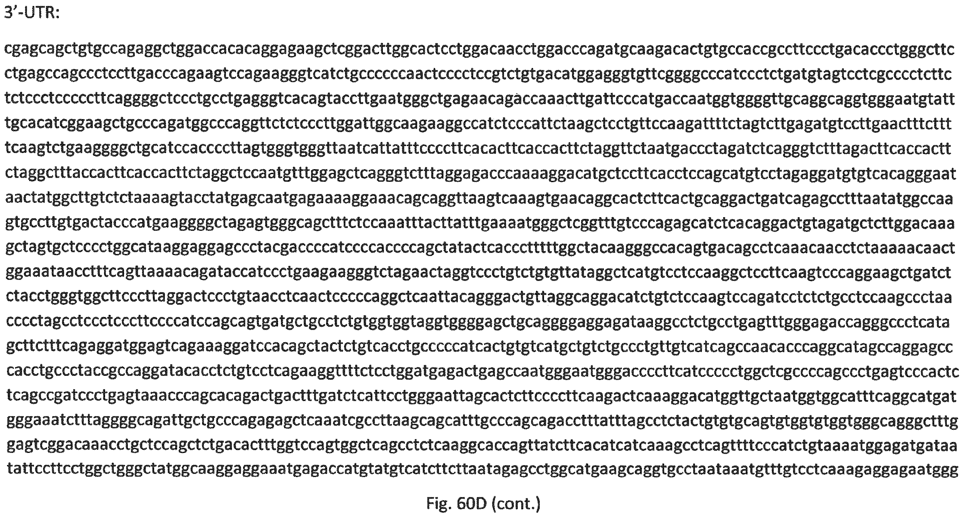

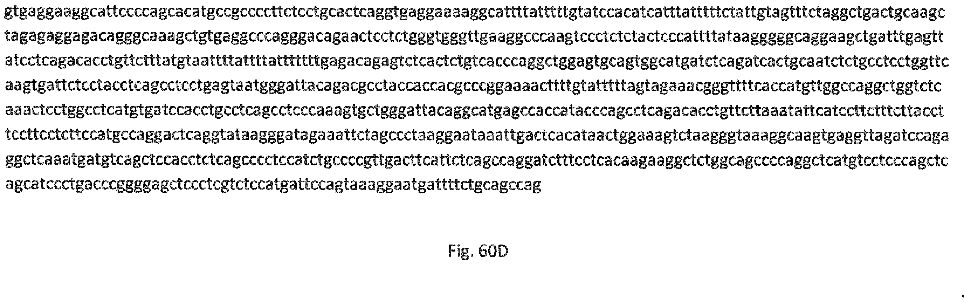

Fig. 60D shows the 3' UTR nucleotide sequence of the mRNA of human Ang-4.

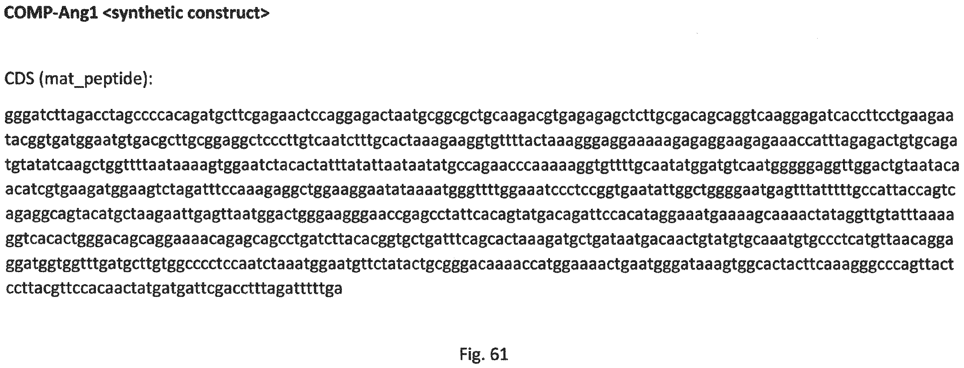

Fig. 61 shows the nucleotide sequence coding for COMP-Angl which is a synthetic construct.

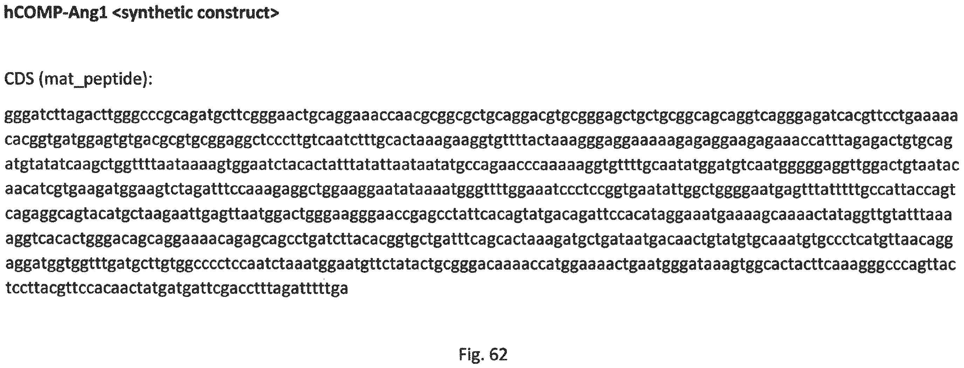

Fig. 62 shows the nucleotide sequence coding for hCOMP-Ang1 which is a synthetic construct.

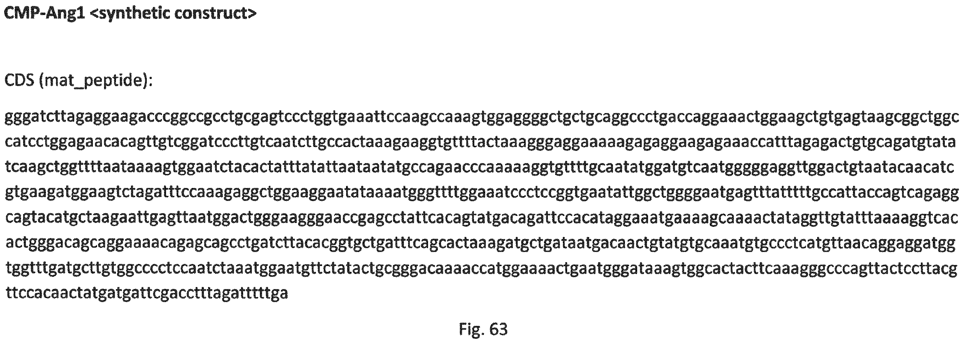

Fig. 63 shows the nucleotide sequence coding for CMP-Ang1 which is a synthetic construct.

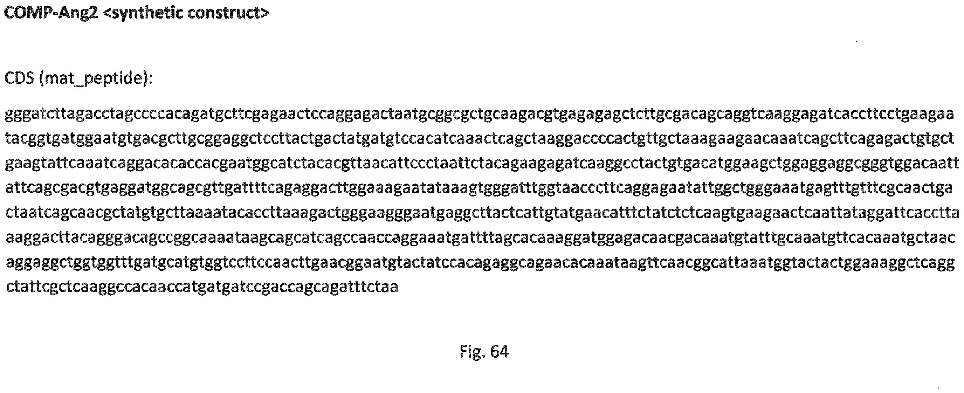

Fig. 64 shows the nucleotide sequence coding for COMP-Ang2 which is a synthetic construct.

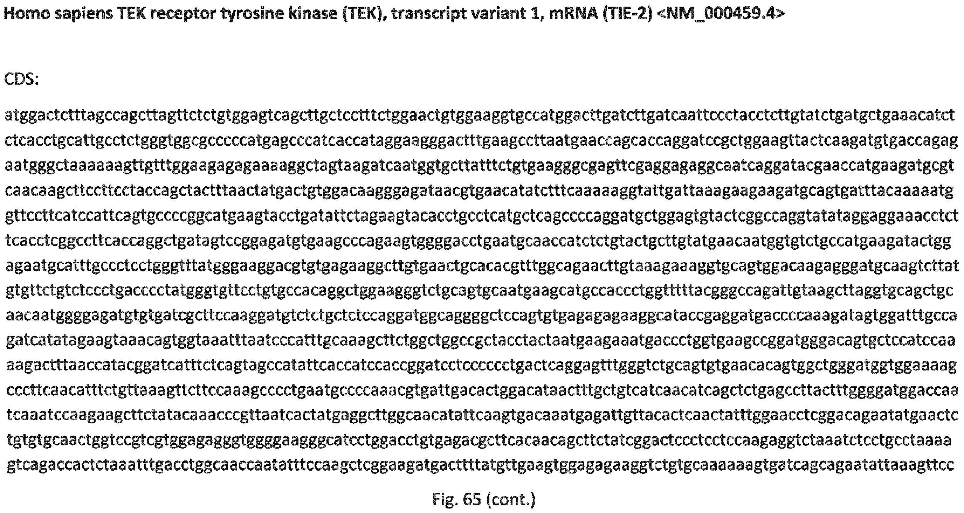

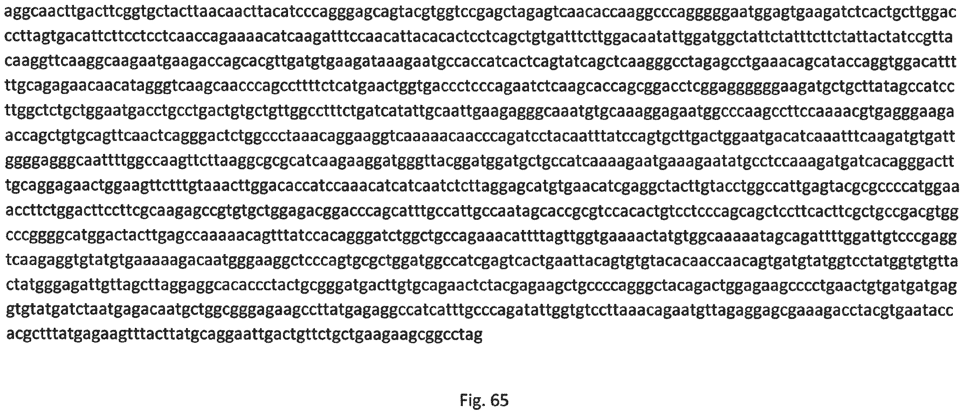

Fig. 65 shows the coding nucleotide sequence of mRNA of human TEK receptor tyrosine kinase (TEK), transcript variant 1, also referred to as Tie-2, GenBank entry.

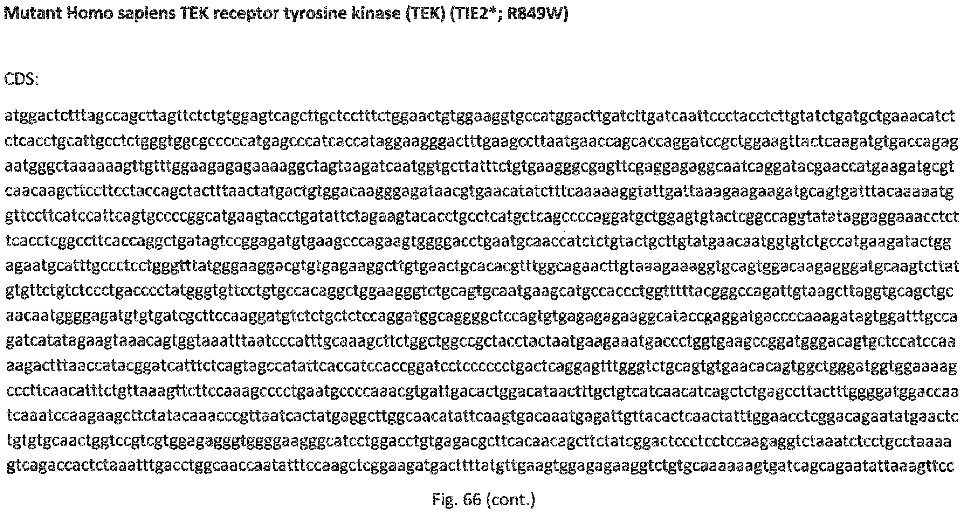

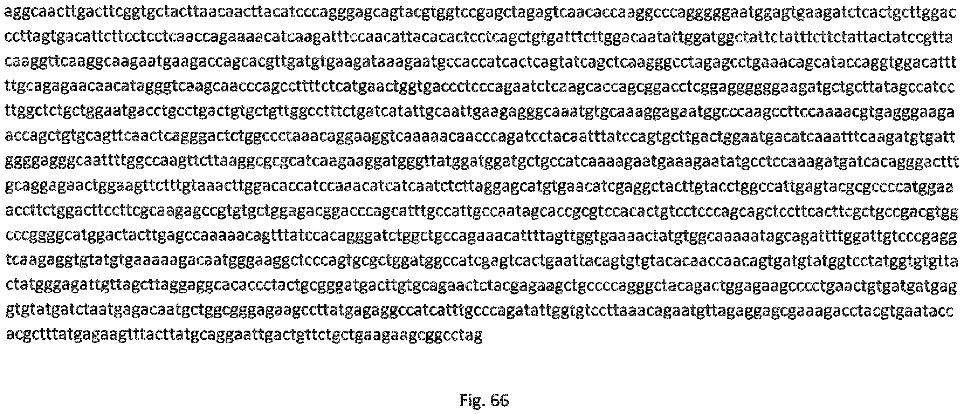

Fig. 66 shows the coding nucleotide sequence of mRNA of human mutant TEK receptor tyrosine kinase (TEK), which is also referred to as TIE* having a R849W mutation.
Fig. 67 shows the basic structure and nucleotide sequence of recombinant nucleic acid construct PAN50.

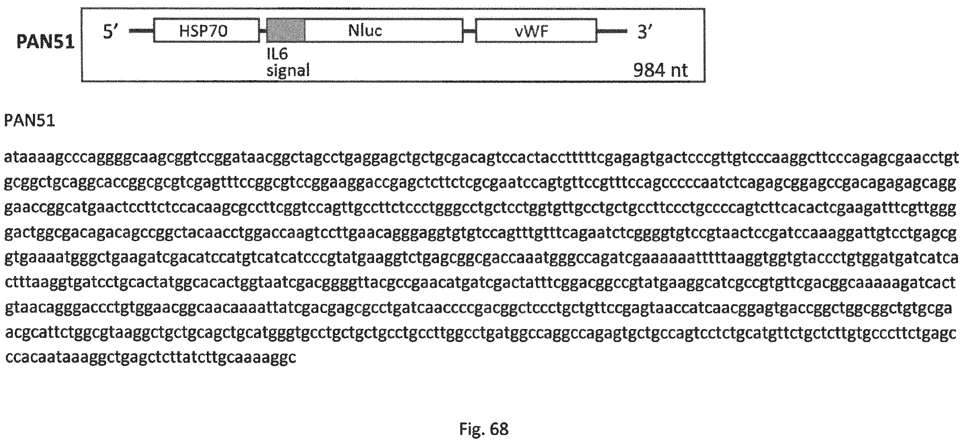

Fig. 68 shows the basic structure and nucleotide sequence of recombinant nucleic acid construct PAN51.

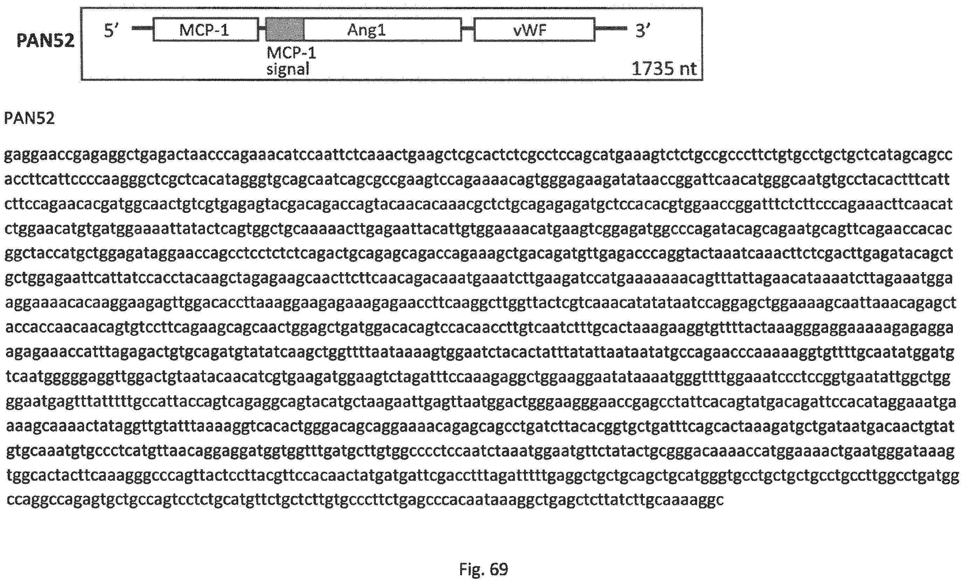

Fig. 69 shows the basic structure and nucleotide sequence of recombinant nucleic acid construct PAN52.

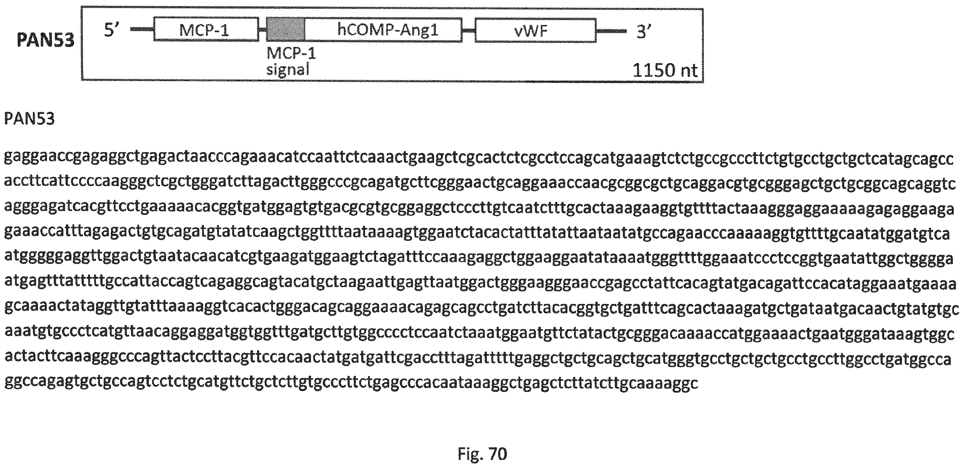

Fig. 70 shows the basic structure and nucleotide sequence of recombinant nucleic acid construct PAN53.

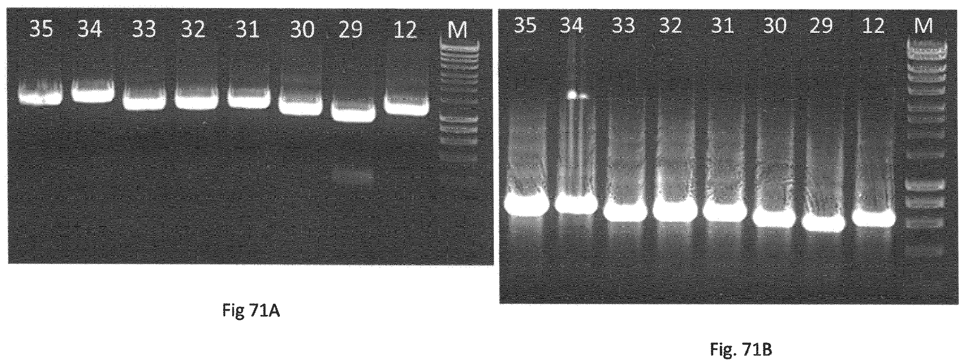

Fig. 71A is an image of a non-denaturing agarose gel after EtBr staining showing the tail-PCR products of various construct.
Fig. 71B is an image of a non-denaturing agarose gel after EtBr staining showing the *in vitro* mRNA transcripts of various construct.

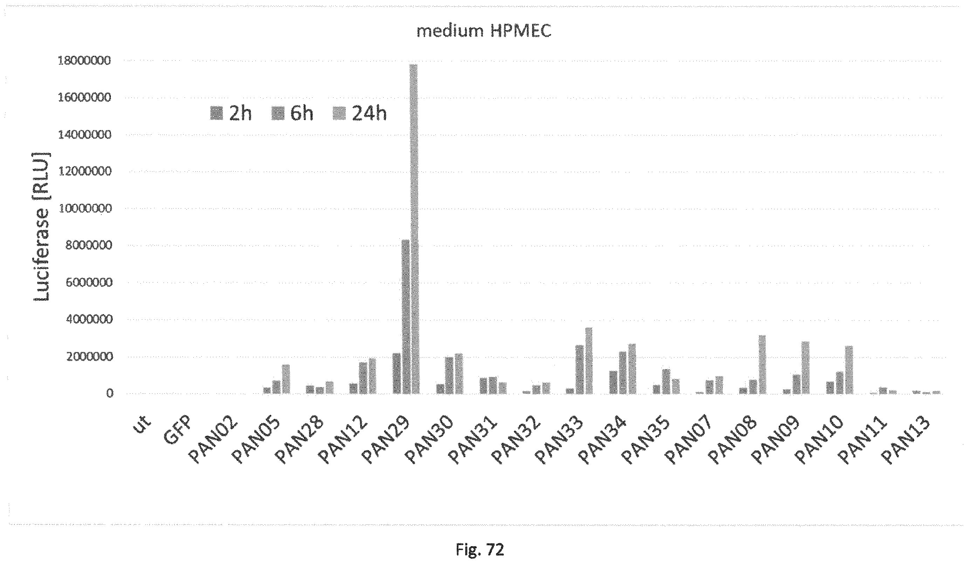

Fig. 72 is a bar diagram showing expression of luciferase, indicated as RLU (relative light units) of luciferase in medium, by the indicated recombinant nucleic acid constructs in HPMEC where luciferase is used as the effector molecule after 2 hours (left column), 6 hours (middle column) and 24 hours (right column) post transfection.

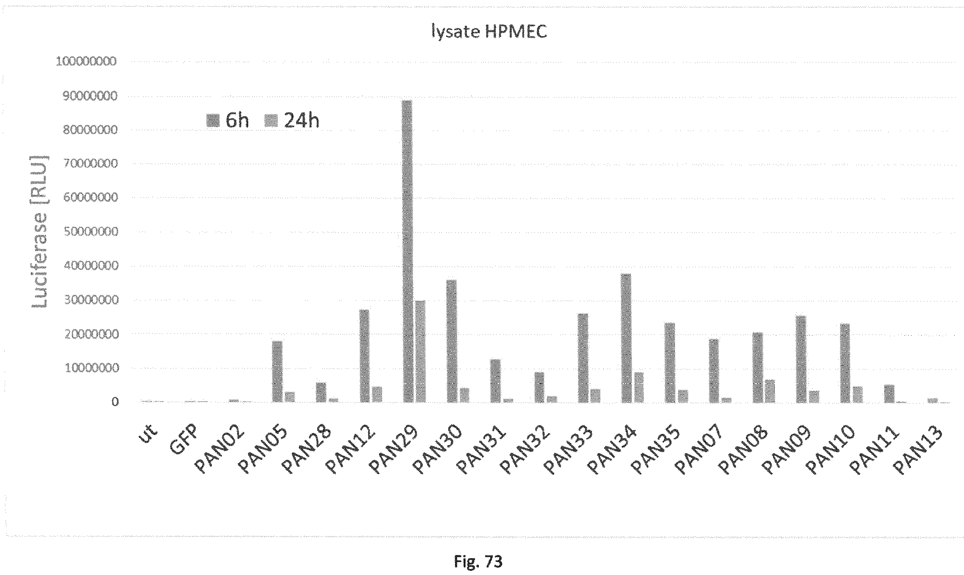

Fig. 73 is a bar diagram showing expression of luciferase, indicated as RLU (relative light units) of luciferase in whole cell ly sates of HPMEC, by the indicated recombinant nucleic acid constructs in HPMEC, where luciferase is used as the effector molecule after 6 hours (left column) and 24 hours (right column) post transfection.

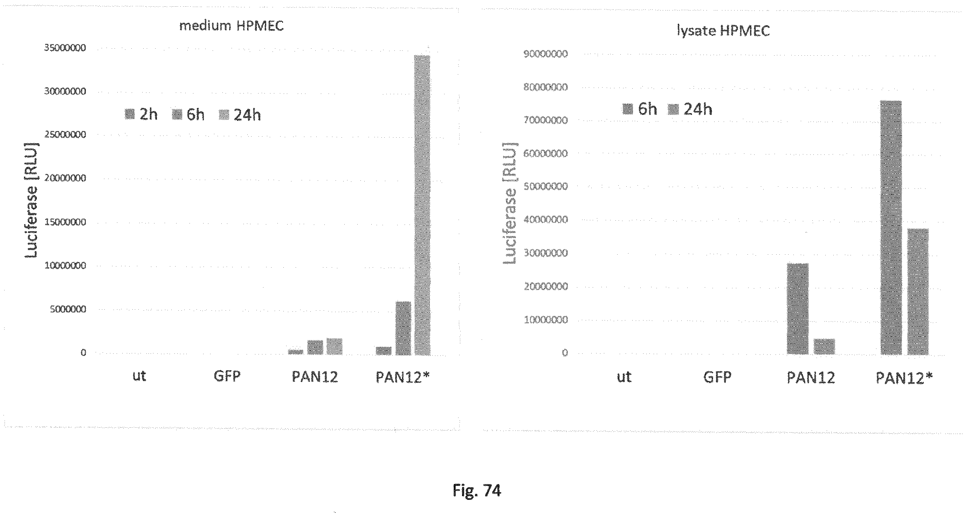

Fig. 74A (left) is a bar diagram showing expression of luciferase, indicated as RLU (relative light units) of luciferase in medium, by the indicated recombinant nucleic acid constructs in HPMEC (transfected with GFP mRNA, PAN12 mRNA and PAN12* mRNA) where luciferase is used as the effector molecule after 2 hours (left column), 6 hours (middle column) and 24 hours (right column) post transfection. In PAN12* 100% of uridine and 100% cytidine nucleotides are replaced by pseudo-uridine and 5-methyl-cytidine, respectively.
Fig. 74B (right) is a bar diagram showing expression of luciferase, indicated as RLU (relative light units) of luciferase in cell lysates, by the indicated recombinant nucleic acid constructs in HPMEC (transfected with GFP mRNA, PAN12 mRNA and PAN12* mRNA) where luciferase is used as the effector molecule after 6 hours (left column) and 24 hours (right column) post transfection. In PAN12* 100% of uridine and 100% cytidine nucleotides are replaced by pseudo-uridine and 5-methyl-cytidine, respectively.

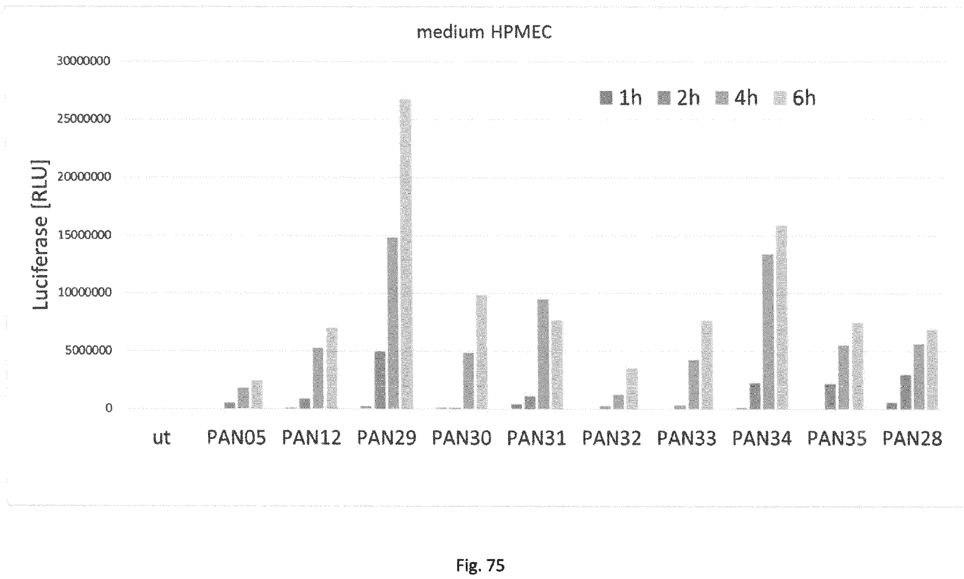

Fig. 75 is a bar diagram showing expression of luciferase, indicated as RLU (relative light units) of luciferase in medium, by the indicated recombinant nucleic acid constructs in HPMEC where luciferase is used as the effector molecule after 1 hour, 2 hours, 4 hours and 24 hours post transfection (from left to right, with 1 hour results being shown to the utmost left side and 24 hours result being shown to the utmost right side).

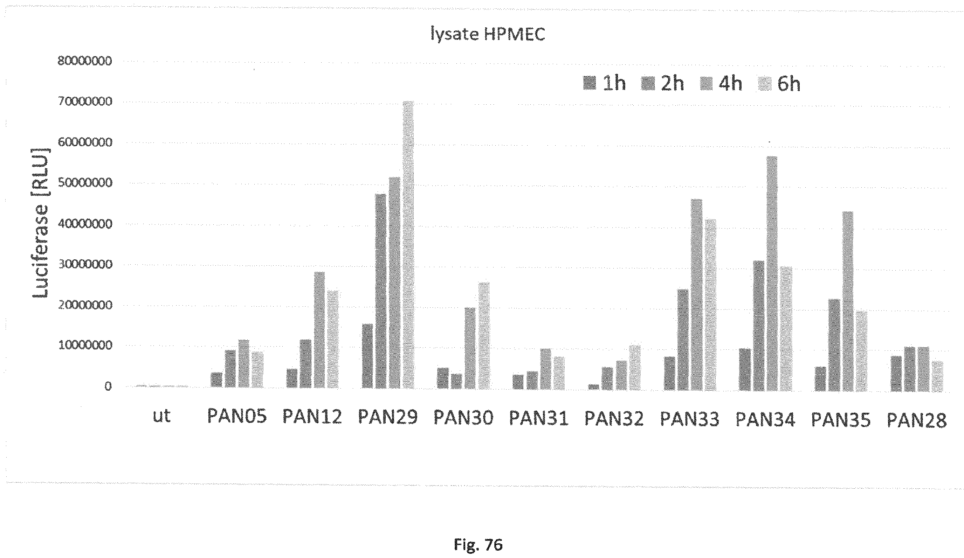

Fig. 76 is a bar diagram showing expression of luciferase, indicated as RLU (relative light units) of luciferase in whole cell lysates of HPMEC, by the indicated recombinant nucleic acid constructs in HPMEC, where luciferase is used as the effector molecule after 1 hour, 2 hours, 4 hours and 24 hours post transfection (from left to right, with 1 hour results being shown to the utmost left side and 24 hours result being shown to the utmost right side).

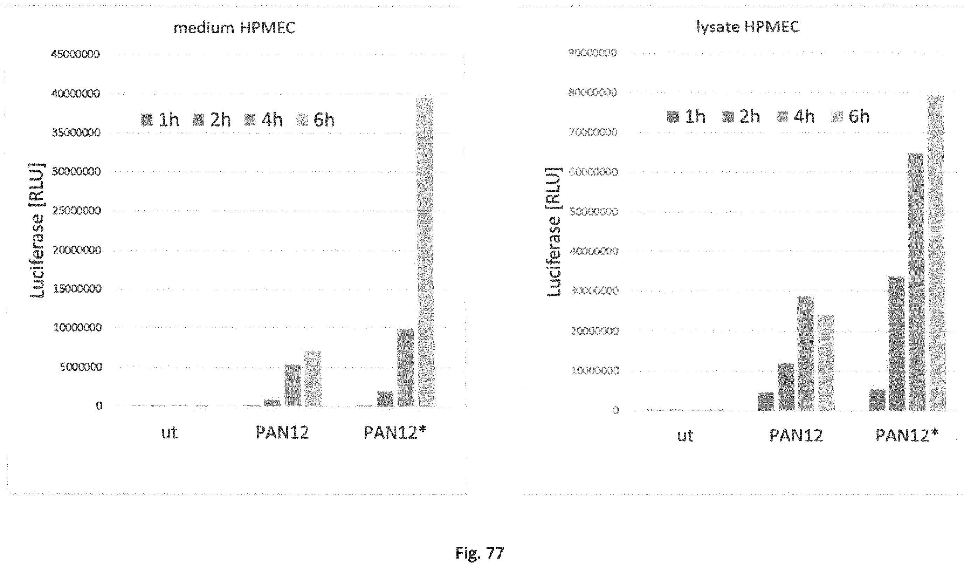

Fig. 77A (left) is a bar diagram showing expression of luciferase, indicated as RLU (relative light units) of luciferase in medium, by the indicated recombinant nucleic acid constructs in HPMEC (transfected with GFP mRNA, PAN12 mRNA and PAN12* mRNA) where luciferase is used as the effector molecule after 1 hour, 2 hours, 4 hours and 24 hours post transfection (from left to right, with 1 hour results being shown to the utmost left side and 24 hours result being shown to the utmost right side). In PAN12* 100% of uridine and 100% cytidine nucleotides are replaced by pseudo-uridine and 5-methyl-cytidine, respectively.
Fig. 77B (right) is a bar diagram showing expression of luciferase, indicated as RLU (relative light units) of luciferase in cell lysates, by the indicated recombinant nucleic acid constructs in HPMEC (transfected with GFP mRNA, PAN12 mRNA and PAN12* mRNA) where luciferase is used as the effector molecule after 1 hour, 2 hours, 4 hours and 24 hours post transfection (from left to right, with 1 hour results being shown to the utmost left side and 24 hours result being shown to the utmost right side). In PAN12* 100% of uridine and 100% cytidine nucleotides are replaced by pseudo-uridine and 5-methyl-cytidine, respectively.

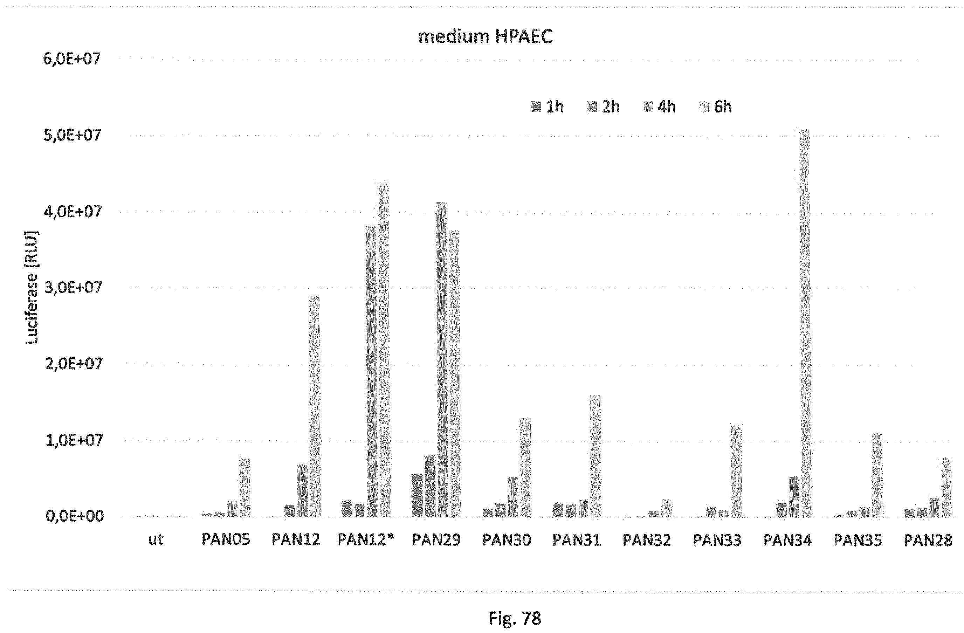

Fig. 78 is a bar diagram showing expression of luciferase, indicated as RLU (relative light units) of luciferase in medium, by the indicated recombinant nucleic acid constructs in HPAEC where luciferase is used as the effector molecule after 1 hour, 2 hours, 4 hours and 24 hours post transfection (from left to right, with 1 hour results being shown to the utmost left side and 24 hours result being shown to the utmost right side).

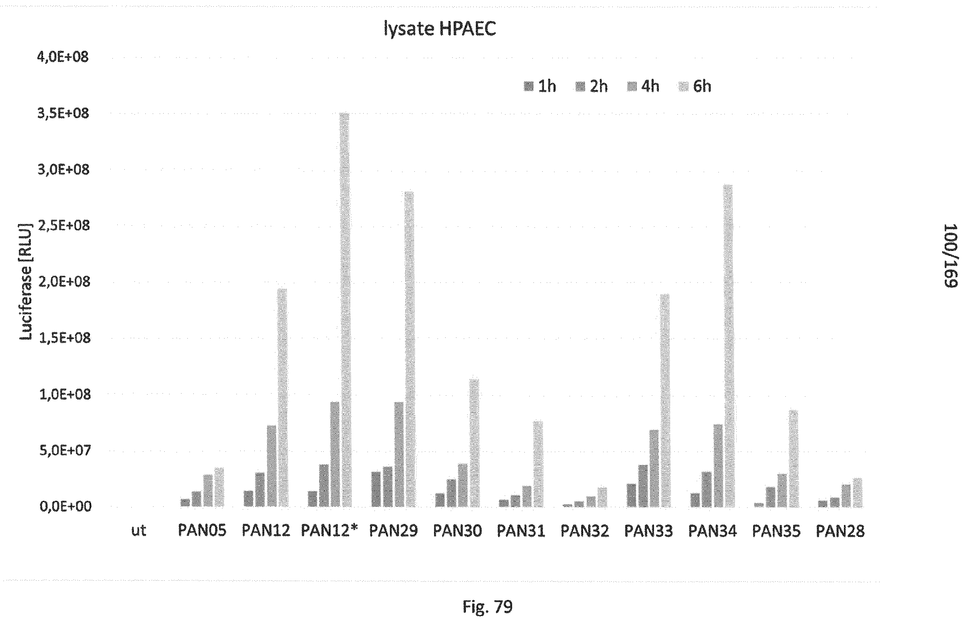

Fig. 79 is a bar diagram showing expression of luciferase, indicated as RLU (relative light units) of luciferase in whole cell lysates of HPAEC, by the indicated recombinant nucleic acid constructs in HPAEC, where luciferase is used as the effector molecule after 1 hour, 2 hours, 4 hours and 24 hours post transfection (from left to right, with 1 hour results being shown to the utmost left side and 24 hours result being shown to the utmost right side).

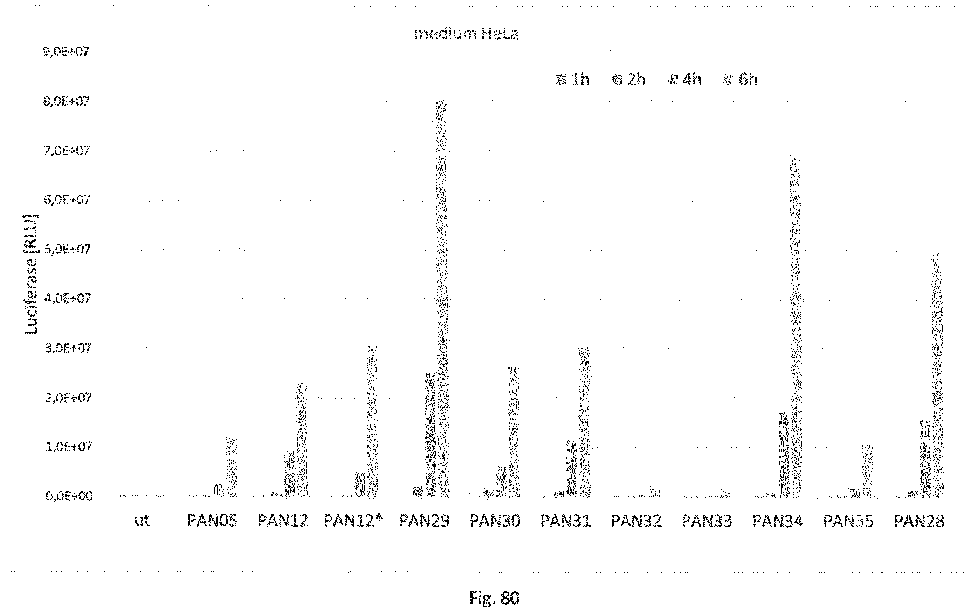

Fig. 80 is a bar diagram showing expression of luciferase, indicated as RLU (relative light units) of luciferase in medium, by the indicated recombinant nucleic acid constructs in HeLa cells where luciferase is used as the effector molecule after 1 hour, 2 hours, 4 hours and 24 hours post transfection (from left to right, with 1 hour results being shown to the utmost left side and 24 hours result being shown to the utmost right side).

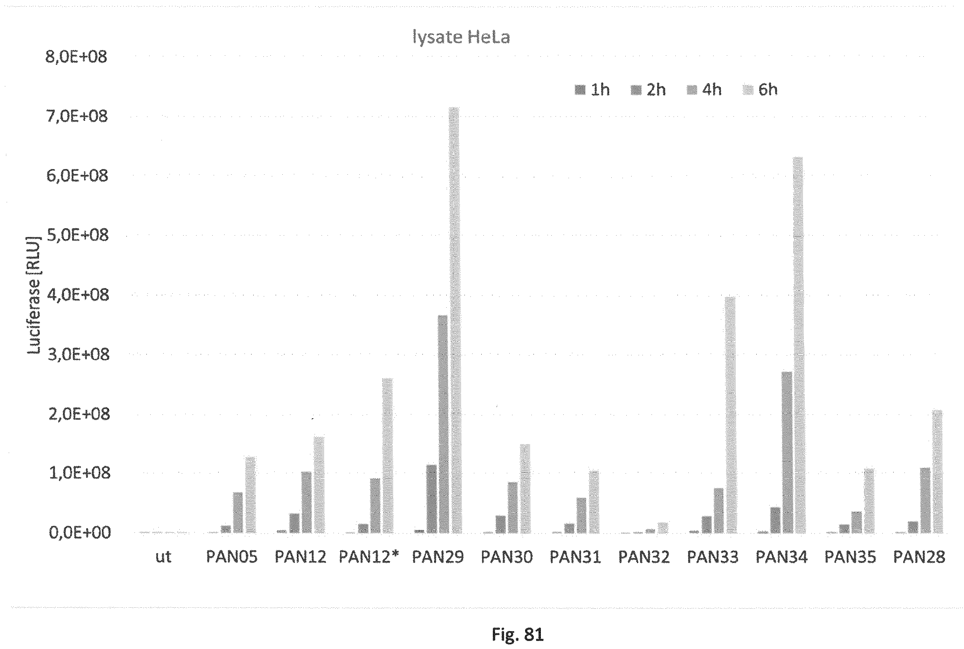

Fig. 81 is a bar diagram showing expression of luciferase, indicated as RLU (relative light units) of luciferase in whole cell lysates of HeLa cells, by the indicated recombinant nucleic acid constructs in HeLA cells, where luciferase is used as the effector molecule after 1 hour, 2 hours, 4 hours and 24 hours post transfection (from left to right, with 1 hour results being shown to the utmost left side and 24 hours result being shown to the utmost right side).

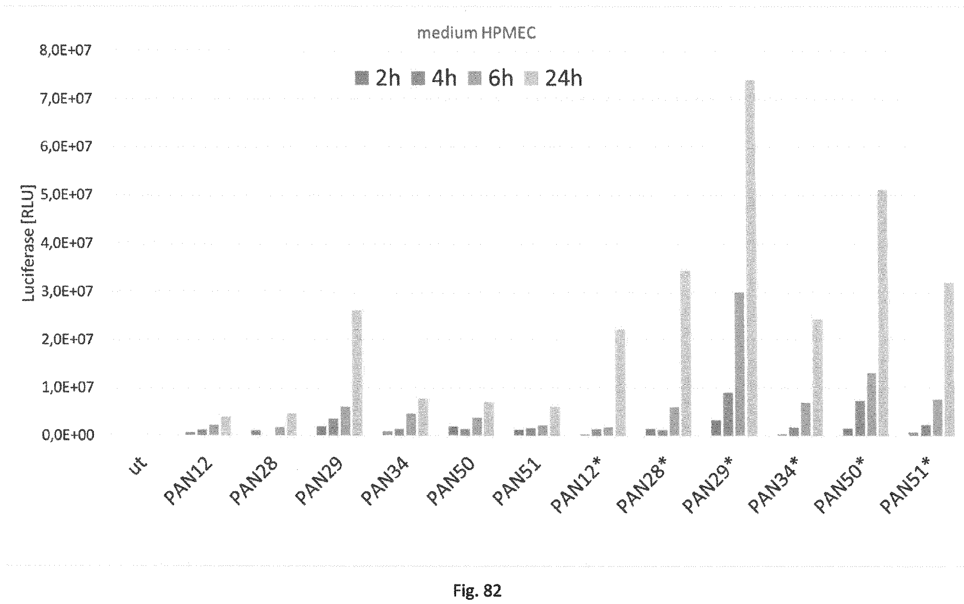

Fig. 82 is a bar diagram showing expression of luciferase, indicated as RLU (relative light units) of luciferase in medium, by the indicated recombinant nucleic acid constructs in HPMEC where luciferase is used as the effector molecule after 2 hours, 4 hours, 6 hours and 24 hours post transfection (from left to right, with 1 hour results being shown to the utmost left side and 24 hours result being shown to the utmost right side). In constructs PAN12*, PAN28*, PAN29*, PAN34*, PAN50* and PAN51*100% of uridine and 100% cytidine nucleotides are replaced by pseudo-uridine and 5-methyl-cytidine, respectively.

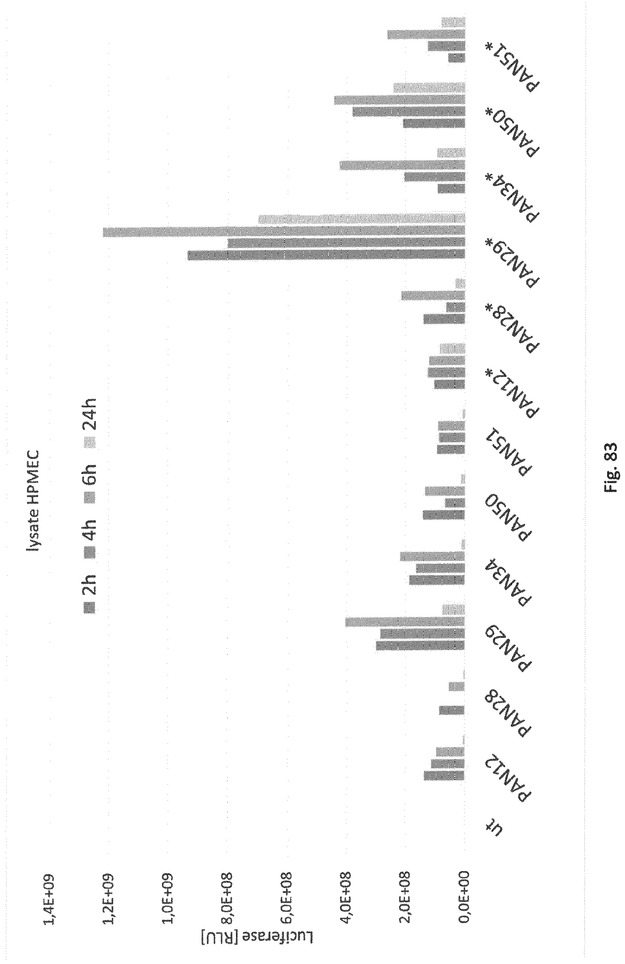

Fig. 83 is a bar diagram showing expression of luciferase, indicated as RLU (relative light units) of luciferase in whole cell ly sates of HPMEC, by the indicated recombinant nucleic acid constructs in HPMEC, where luciferase is used as the effector molecule after 2 hours, 4 hours, 6 hours and 24 hours post transfection (from left to right, with 1 hour results being shown to the utmost left side and 24 hours result being shown to the utmost right side). In constructs PAN12*, PAN28*, PAN29*, PAN34*, PAN50* and PAN51*100% of uridine and 100% cytidine nucleotides are replaced by pseudo-uridine and 5-methyl-cytidine, respectively.

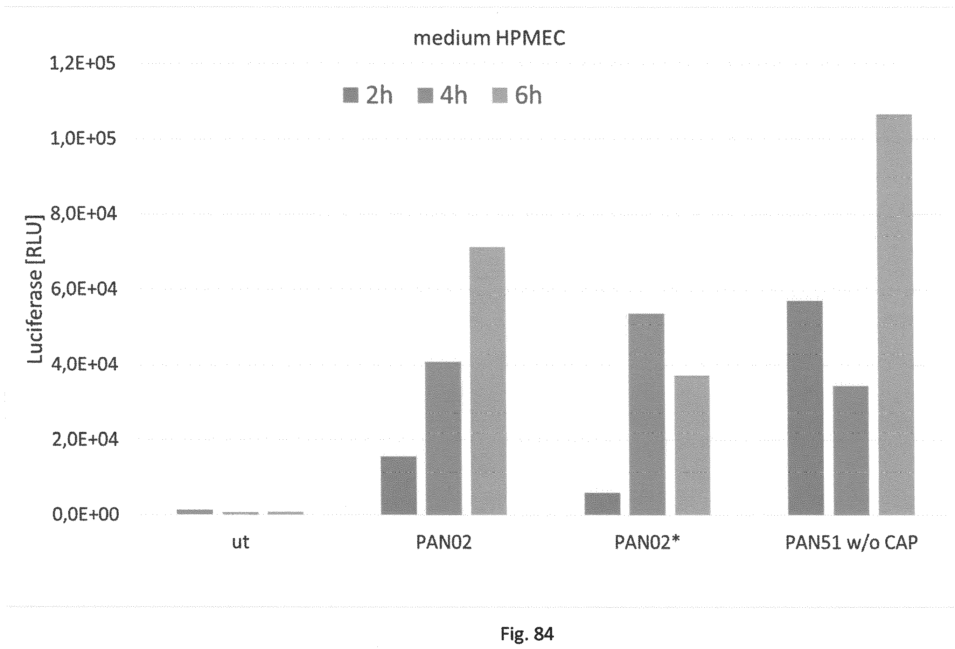

Fig. 84 is a bar diagram showing expression of luciferase, indicated as RLU (relative light units) of luciferase in medium, by the indicated recombinant nucleic acid constructs in HPMEC where luciferase is used as the effector molecule after 2 hours (left column), 4 hours (middle column) and 6 hours (right column) post transfection. In construct PAN02*, 100% of uridine and 100% cytidine nucleotides are replaced by pseudo-uridine and 5-methyl-cytidine, respectively; and in construct PAN51 w/o CAP the mRNA sequence is the one of construct PAN51, but without any 5'-capping structure.

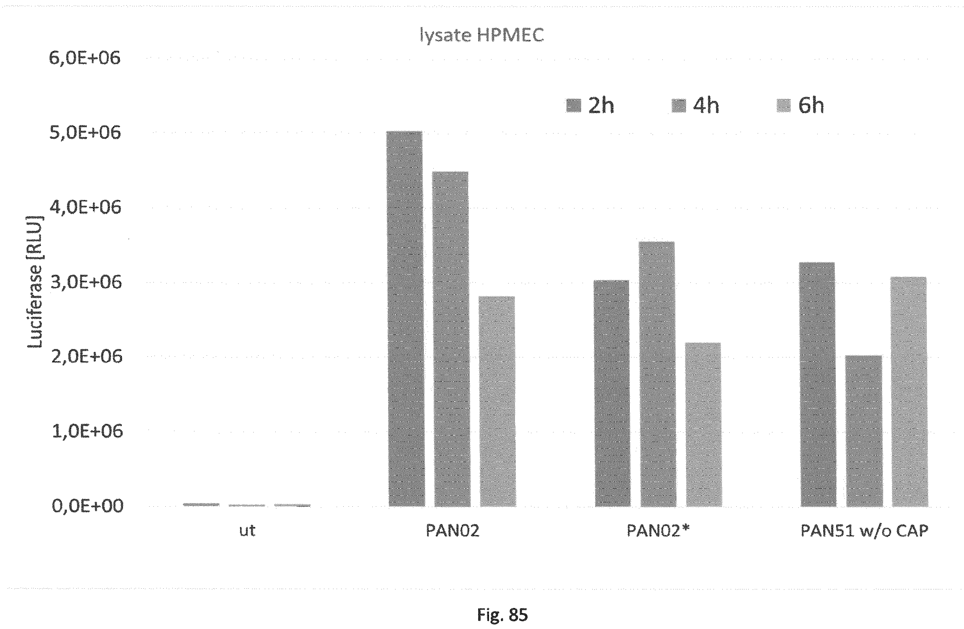

Fig. 85 is a bar diagram showing expression of luciferase, indicated as RLU (relative light units) of luciferase in whole cell lysates of HPMEC, by the indicated recombinant nucleic acid constructs in HPMEC, where luciferase is used as the effector molecule after 2 hours (left column) 4 hours (middle column) and 6 hours (right column) post transfection. In construct PAN02*, 100% of uridine and 100% cytidine nucleotides are replaced by pseudo-uridine and 5-methyl-cytidine, respectively; and in construct PAN51 w/o CAP the mRNA sequence is the one of construct PAN51, but without any 5'-capping structure.
Fig. 86 shows the basic structure and nucleotide sequence of recombinant nucleic acid construct PAN54.

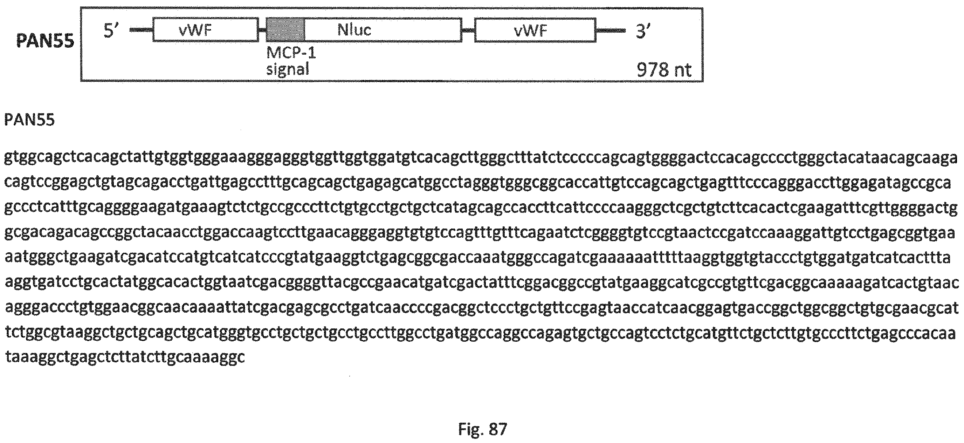

Fig. 87 shows the basic structure and nucleotide sequence of recombinant nucleic acid construct PAN55.
Fig. 88 shows the basic structure and nucleotide sequence of recombinant nucleic acid construct PAN56.

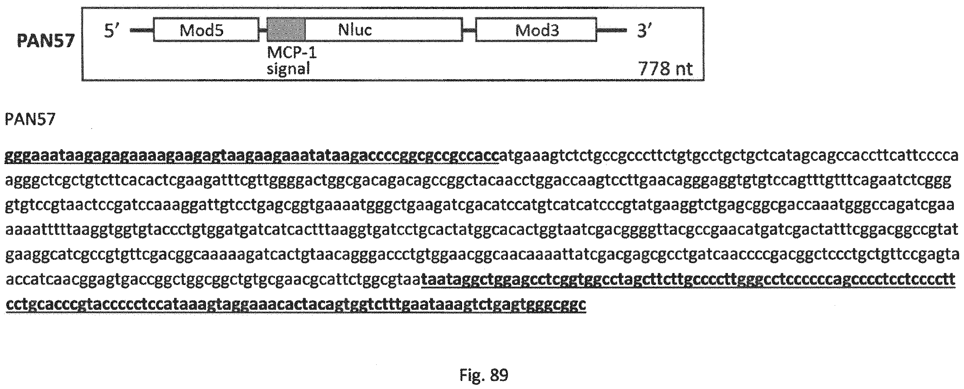

Fig. 89 shows the basic structure and nucleotide sequence of recombinant nucleic acid construct PAN57.
Fig. 90 shows the basic structure and nucleotide sequence of recombinant nucleic acid construct PAN58.

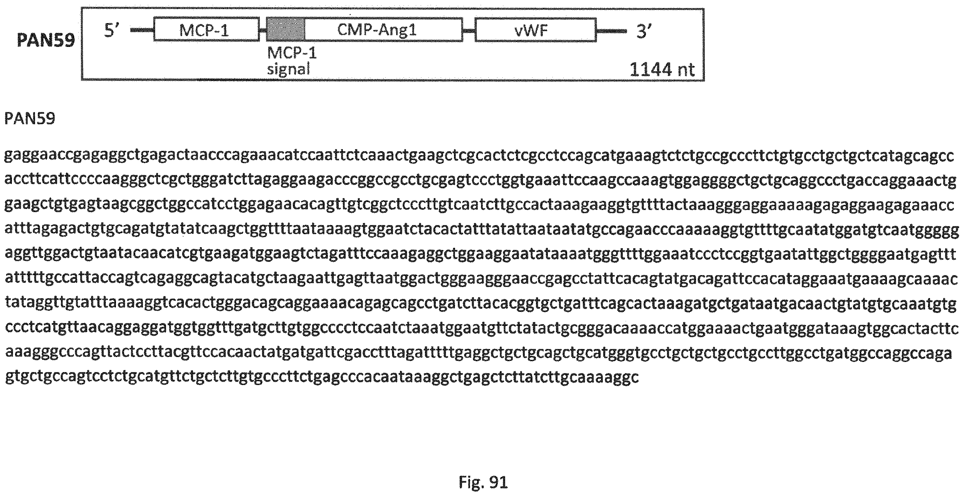

Fig. 91 shows the basic structure and nucleotide sequence of recombinant nucleic acid construct PAN59.

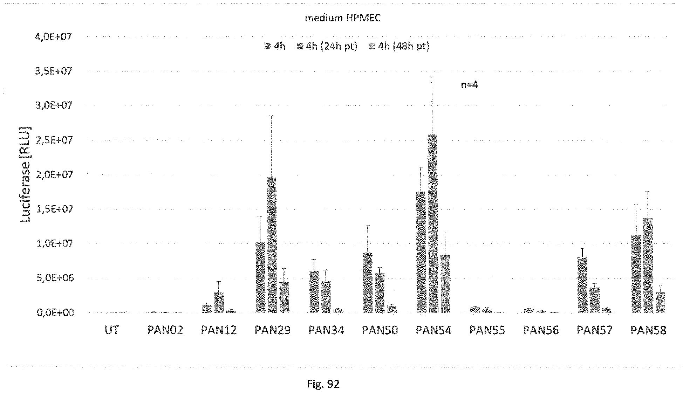

Fig. 92 is a bar diagram showing expression of luciferase, indicated as RLU (relative light units) of luciferase in medium, by the indicated recombinant nucleic acid constructs in HPMEC, where luciferase is used as the effector molecule after 4 hours (left column). Additional 4-hours luciferase activity values are assessed 24 hours (middle column) and 48 hours (right column) post transfection, after washing of the cells and supplementing the cells with fresh medium.

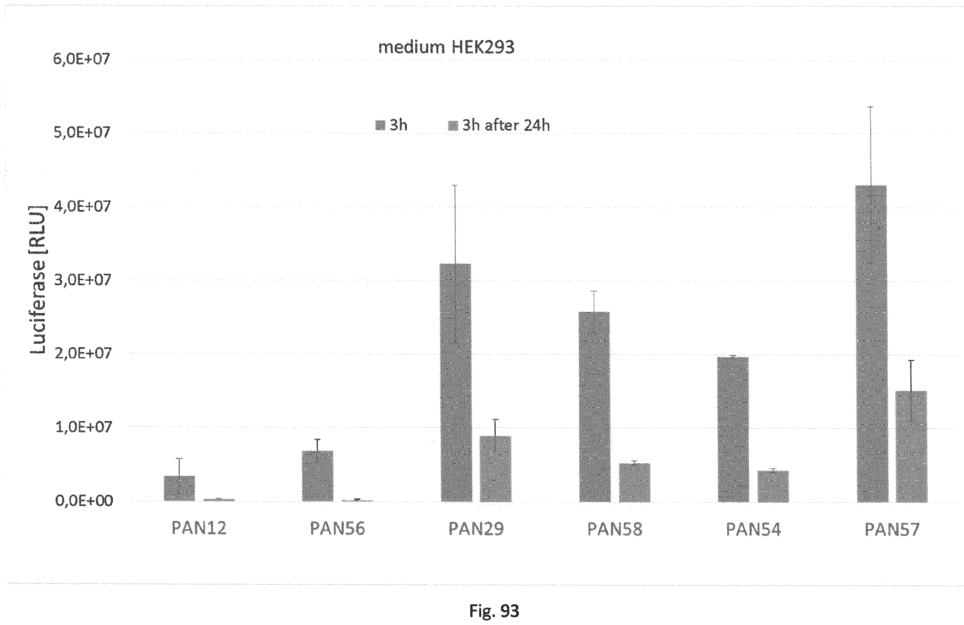

Fig. 93 is a bar diagram showing expression of luciferase, indicated as RLU (relative light units) of luciferase in medium, by the indicated recombinant nucleic acid constructs in HEK293 cells, where luciferase is used as the effector molecule after 3 hours (left column). An additional 3-hours luciferase activity value is assessed 24 hours (right column) post transfection, after washing of the cells and supplementing the cells with fresh medium.
   The human embryonic kidney 293 cell line (HEK293) is grown in EMEM (EBSS) + 2mM Glutamine + 1% Non-Essential Amino Acids (NEAA) + 10% FCS culture medium. Subculture Routine is to split sub-confluent cultures (70-80%) 1:2 to 1:6 i.e. seeding at 2-5x10,000 cells/cm² using 0.25% trypsin or trypsin/EDTA; 5% CO₂; 37°C. mRNA transfection are performed with the Lipofectamine™ MessengerMAX™ Transfection Reagent (Invitrogen™) according to the manufactures protocol.

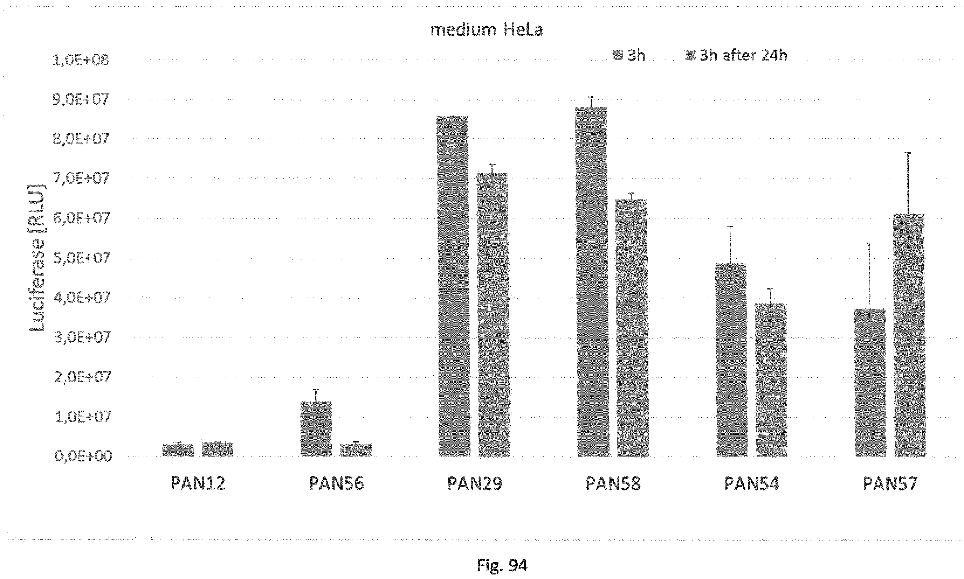

Fig. 94 is a bar diagram showing expression of luciferase, indicated as RLU (relative light units) of luciferase in medium, by the indicated recombinant nucleic acid constructs in HeLa cells, where luciferase is used as the effector molecule after 3 hours (left column). An additional 3-hours luciferase activity value is assessed 24 hours (right column) post transfection, after washing of the cells and supplementing the cells with fresh medium.

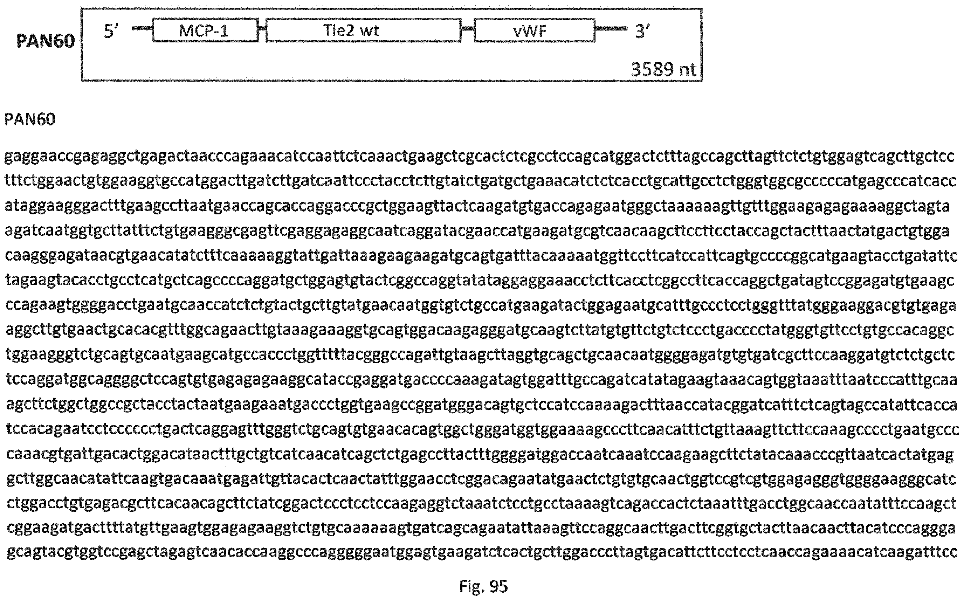

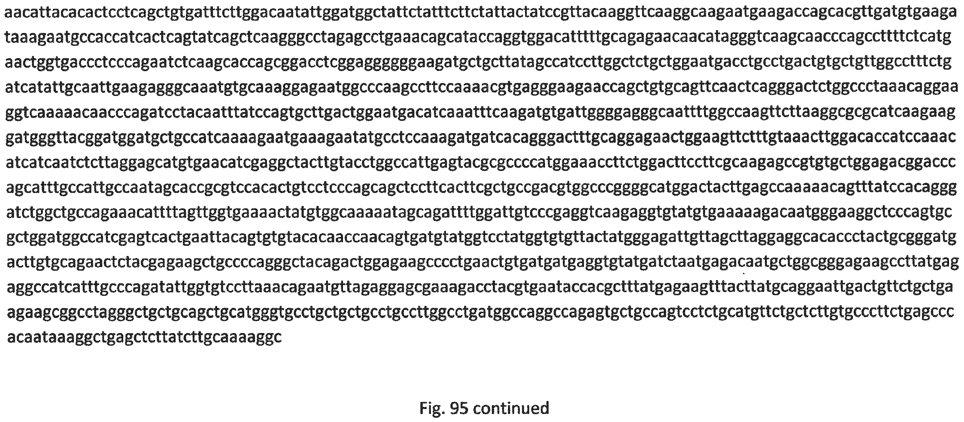

Fig. 95 shows the basic structure and nucleotide sequence of recombinant nucleic acid construct PAN60.

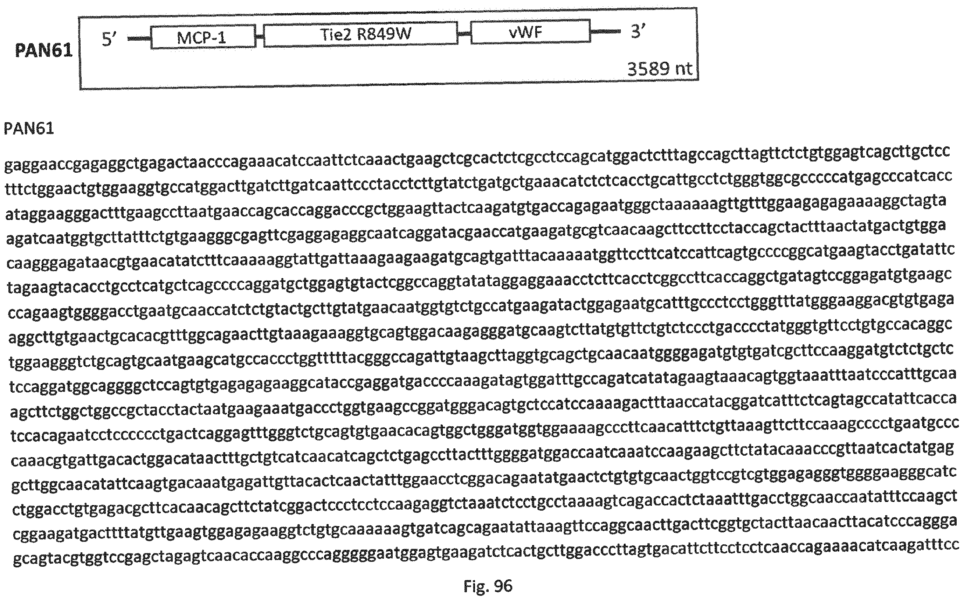

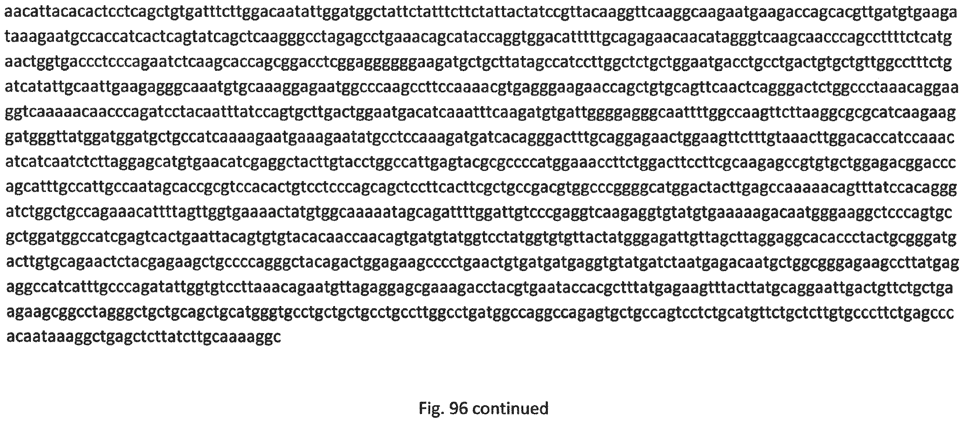

Fig. 96 shows the basic structure and nucleotide sequence of recombinant nucleic acid construct PAN61.

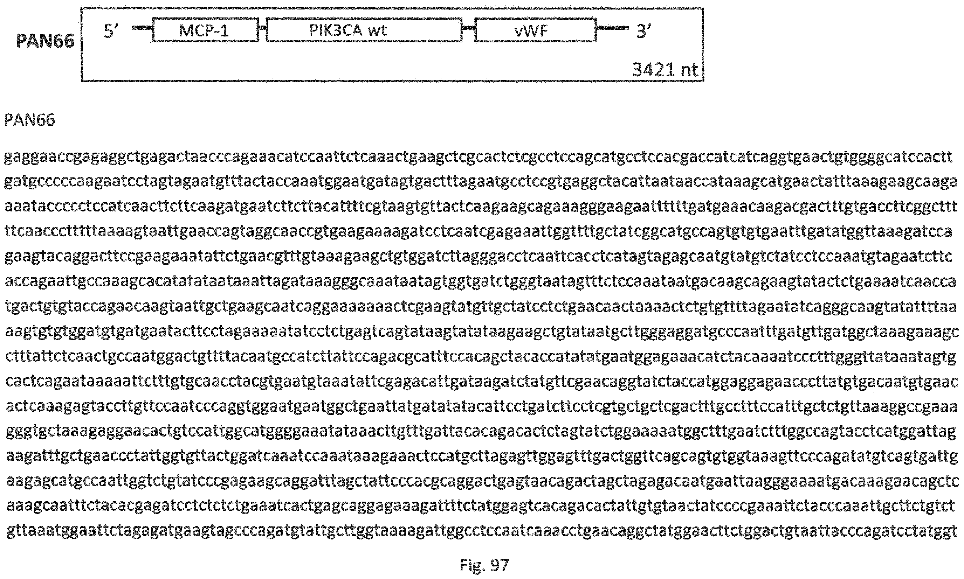

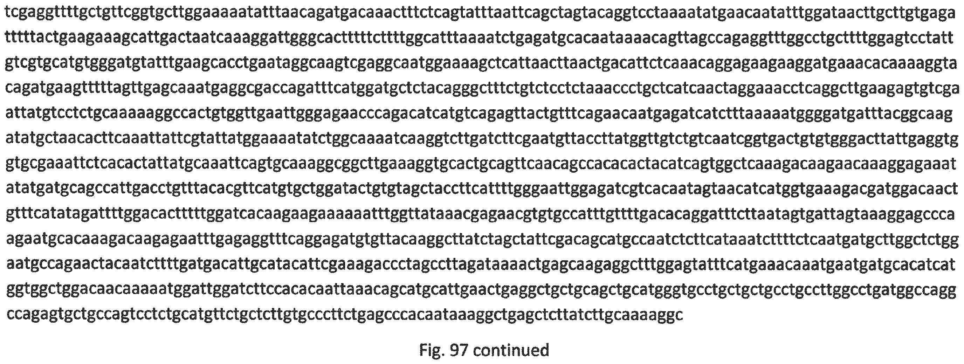

Fig. 97 shows the basic structure and nucleotide sequence of recombinant nucleic acid construct PAN66.

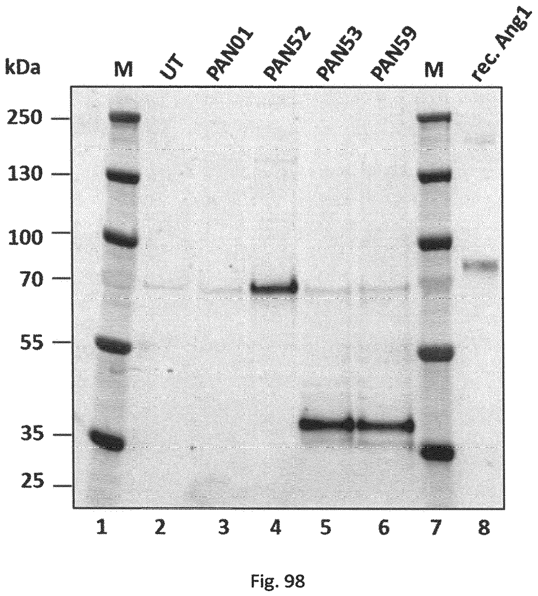

Fig. 98 shows Western Blot analysis of protein lysates from HPMEC cells transfected with indicated PAN mRNA constructs. Cells were transfected with 1µg the mRNAs and harvested after 6 hours. Total cell lysates were separated by SDS-PAGE and analyzed by immunoblot using anti-Angl antibody. M, Marker; UT, untreated cells, rec. Ang1, recombinant hAng1.

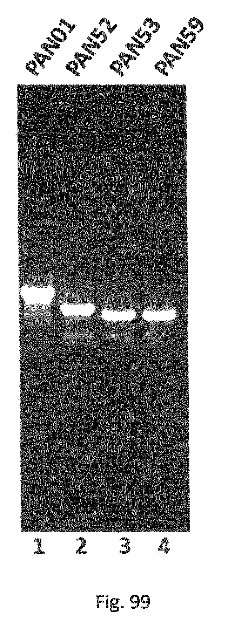

Fig. 99 shows Agarose gels of mRNAs after in vitro transcription. 2 µg of indicated mRNAs were separated by agarose gel electrophoresis and visualized by ethidium bromide staining and UV illumination.

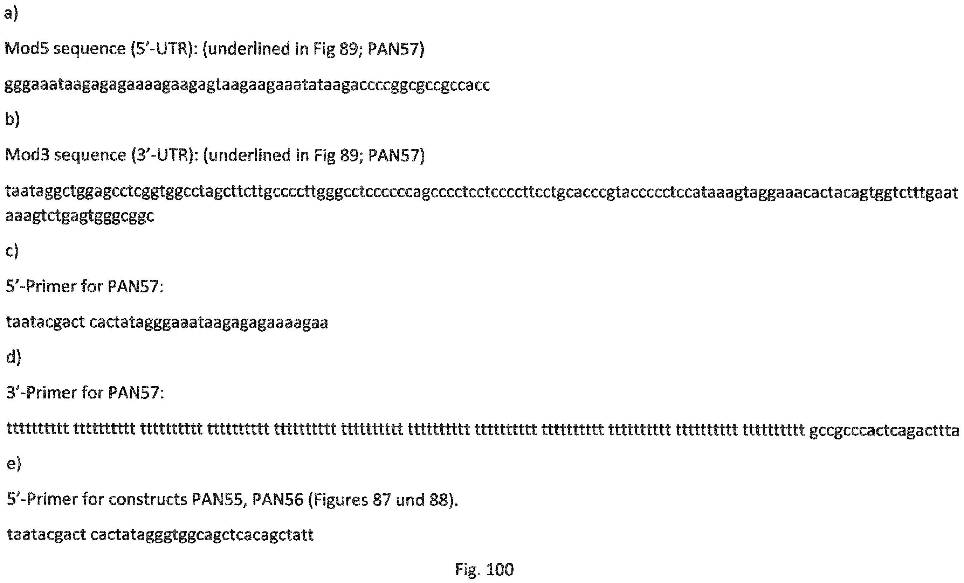

Fig. 100 shows the sequences of: a) the 5'UTR of PAN57; b) the 3'UTR of PAN57; c) the 5'PCR-Primer for PAN57; d) the 3'PCR-Primer for PAN57 and e) the 5'PCR-Primer for PAN55, PAN56.

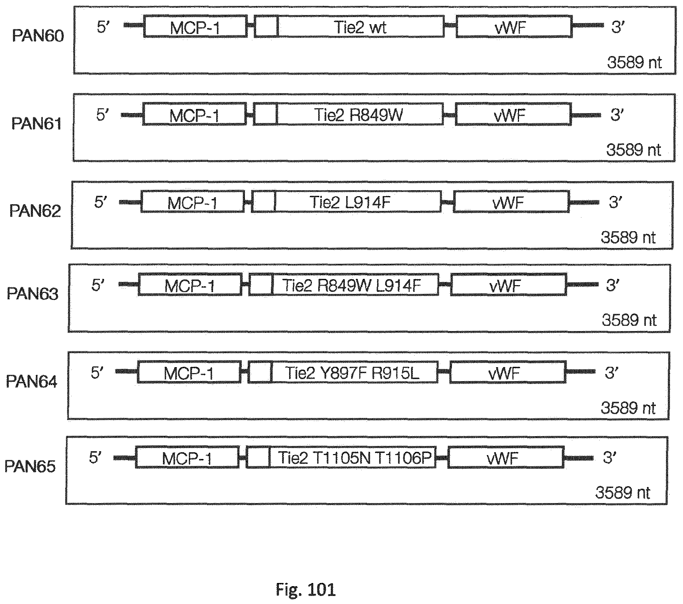

Fig. 101 shows the basic structure and length of recombinant nucleic acid constructs PAN60, PAN61, PAN62, PAN63, PAN64 and PAN65.

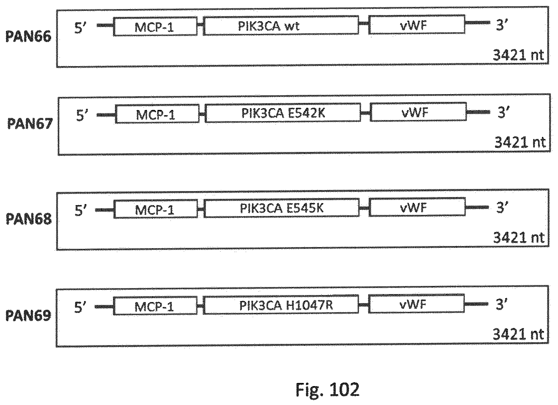

Fig. 102 shows the basic structure and length of recombinant nucleic acid constructs PAN66, PAN67, PAN68, and PAN69.

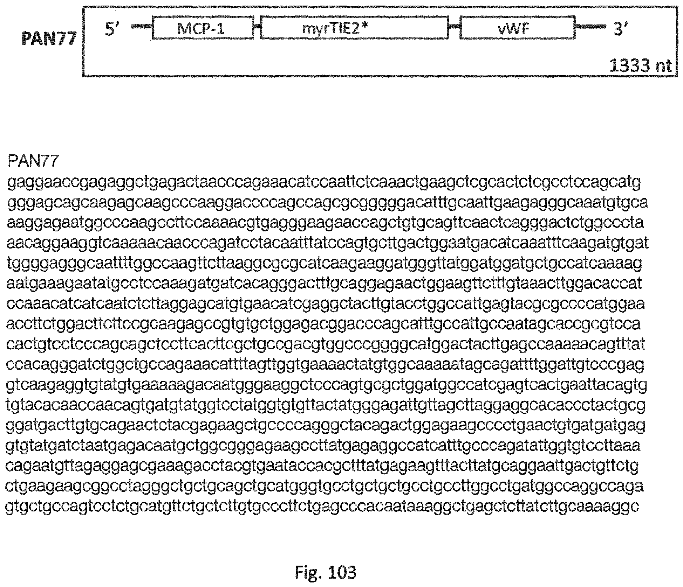

Fig. 103 shows the basic structure, length and nucleotide sequence of recombinant nucleic acid construct PAN77.

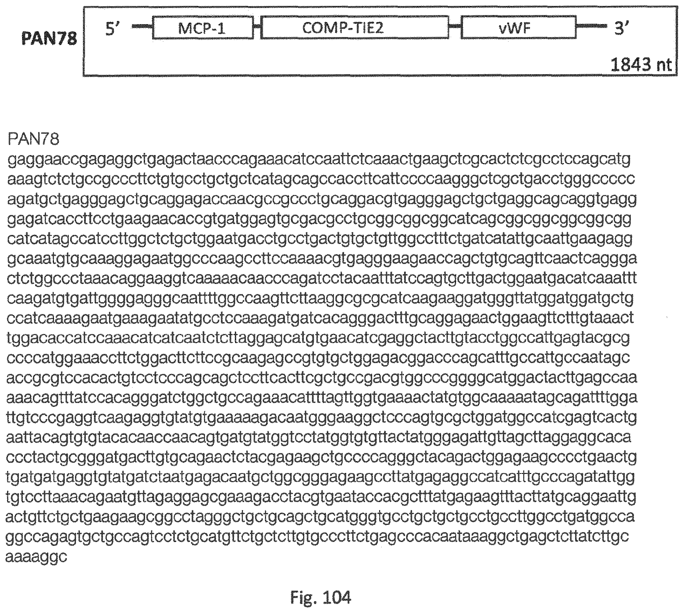

Fig. 104 shows the basic structure, length and nucleotide sequence of recombinant nucleic acid construct PAN78.

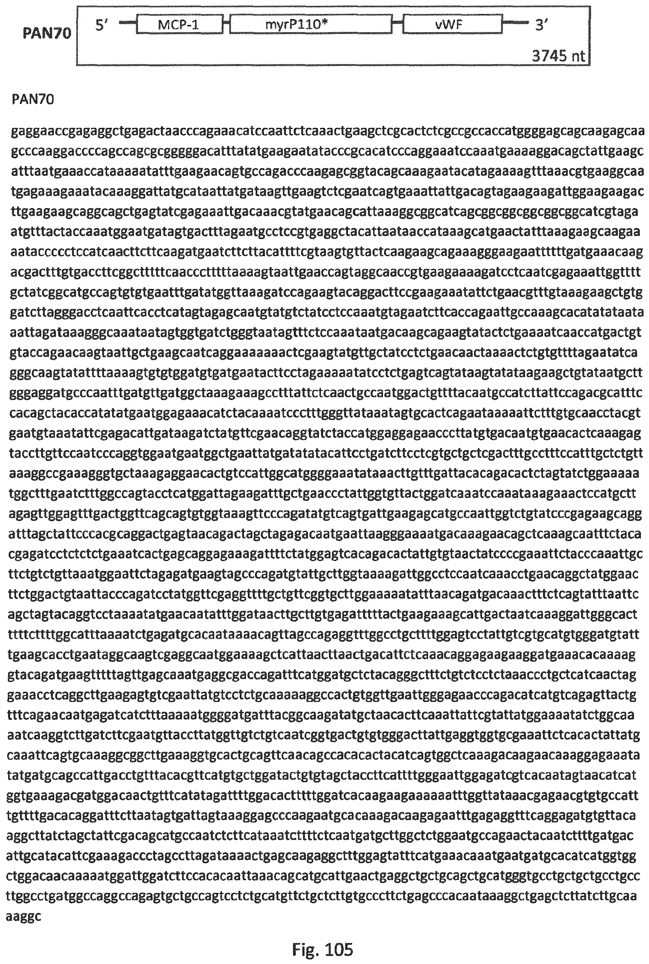

Fig. 105 shows the basic structure, the length and nucleotide sequence of recombinant nucleic acid and the nucleotide sequence of construct PAN70.

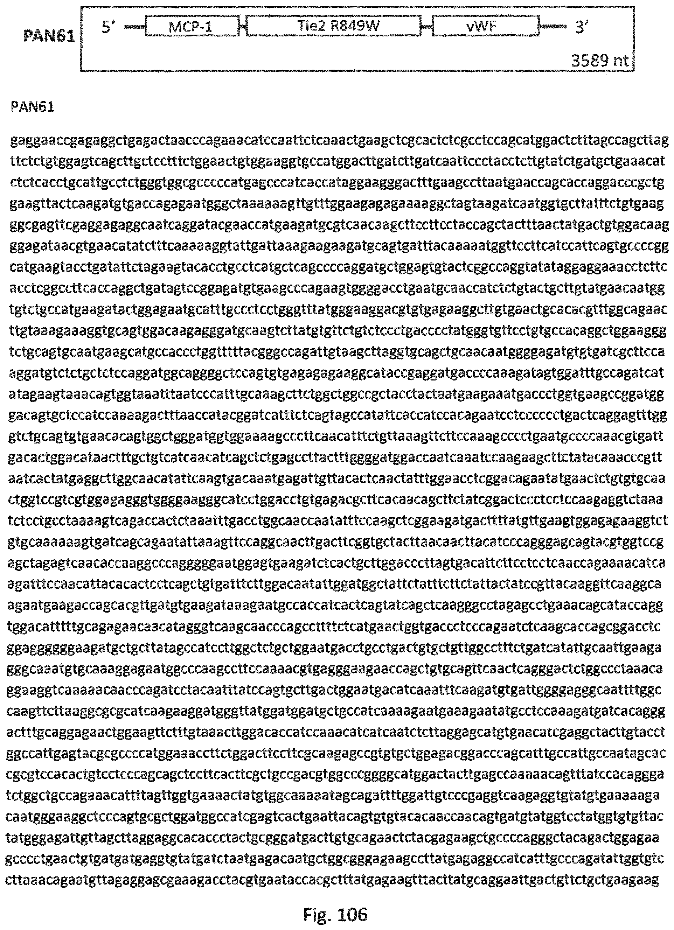

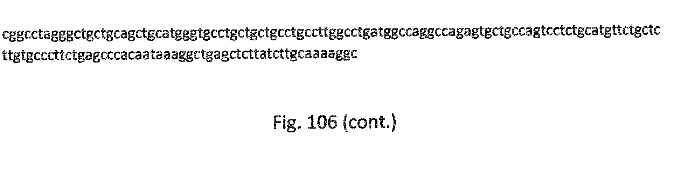

Fig. 106 shows the basic structure, length and nucleotide sequence of recombinant nucleic acid and the nucleotide sequence of construct PAN61.

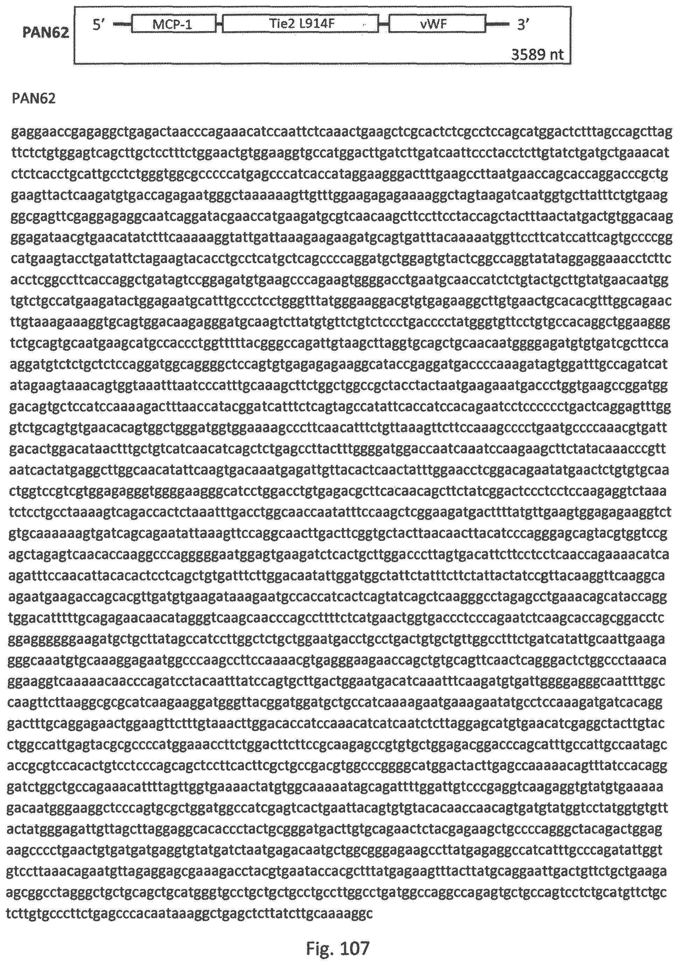

Fig. 107 shows the basic structure, length and nucleotide sequence of recombinant nucleic acid and the nucleotide sequence of construct PAN62.

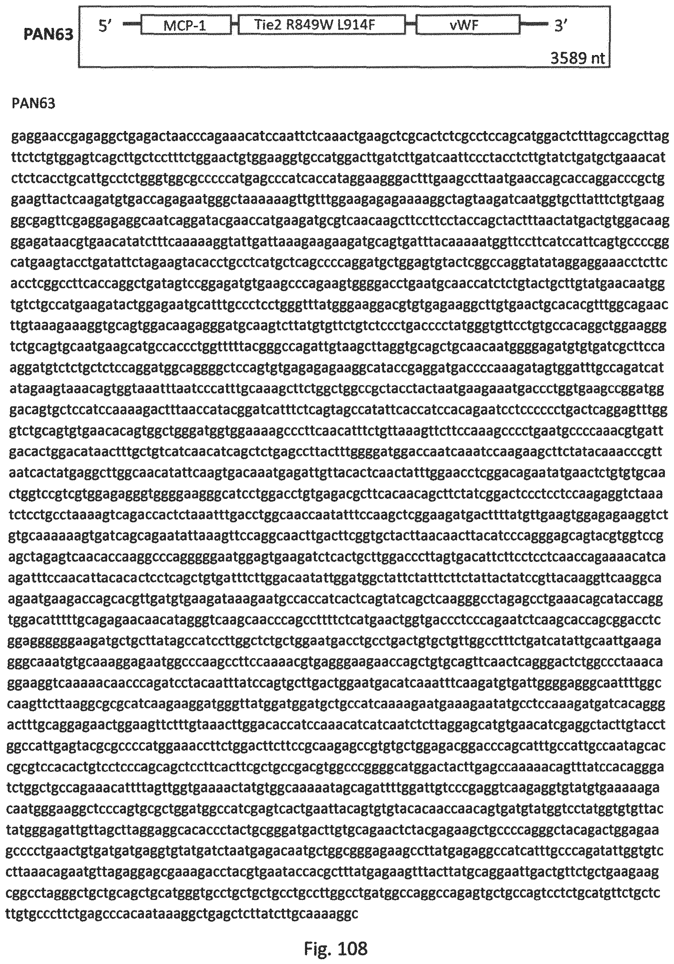

Fig. 108 shows the basic structure, length and nucleotide sequence of recombinant nucleic acid and the nucleotide sequence of construct PAN63.

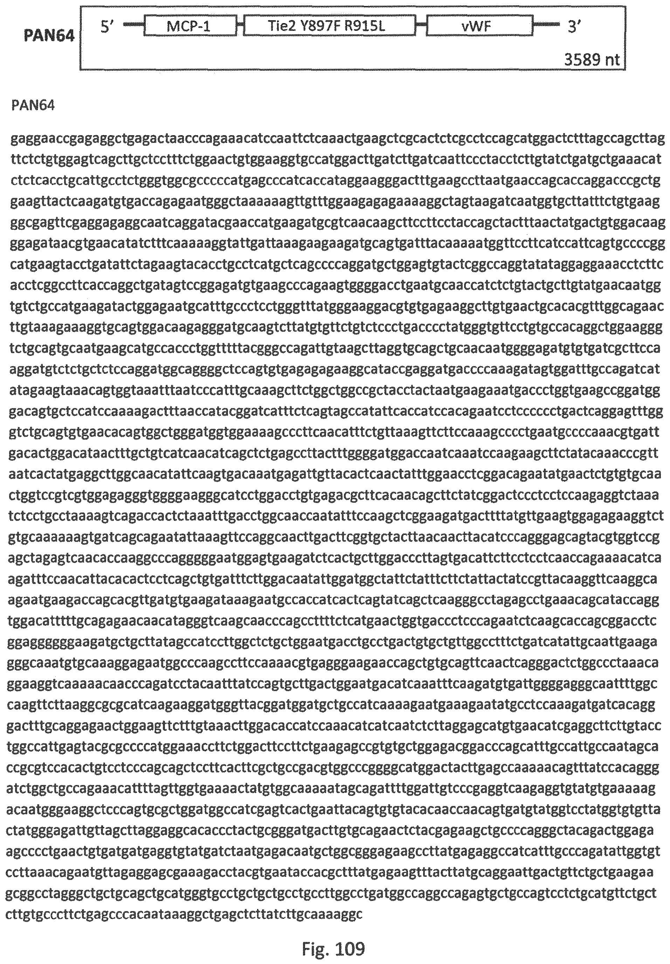

Fig. 109 shows the basic structure, length and nucleotide sequence of recombinant nucleic acid and the nucleotide sequence of construct PAN64.

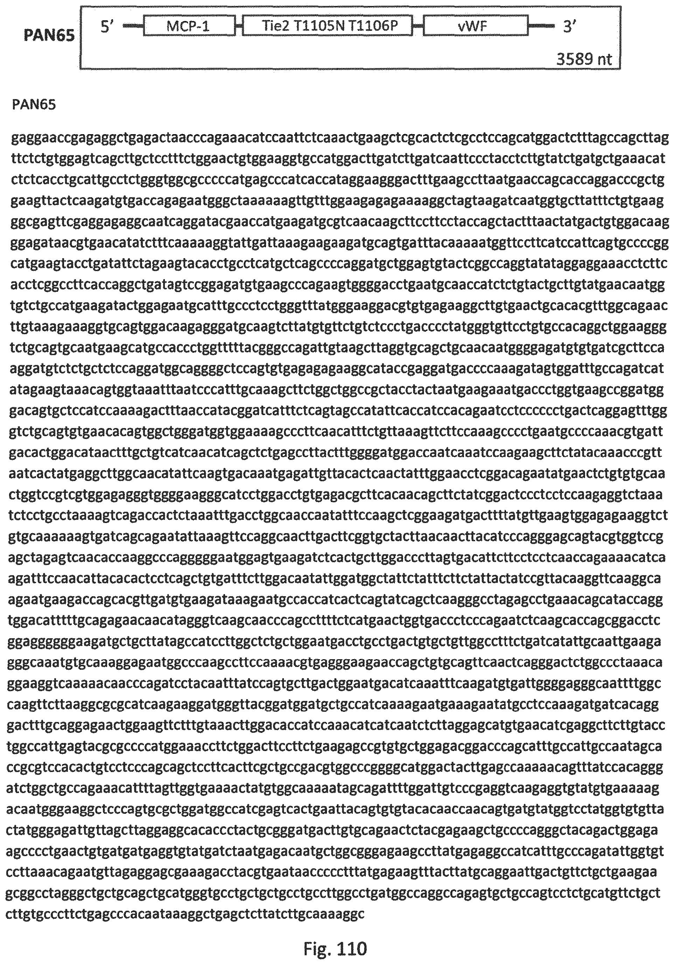

Fig. 110 shows the basic structure, length and nucleotide sequence of recombinant nucleic acid and the nucleotide sequence of construct PAN65.

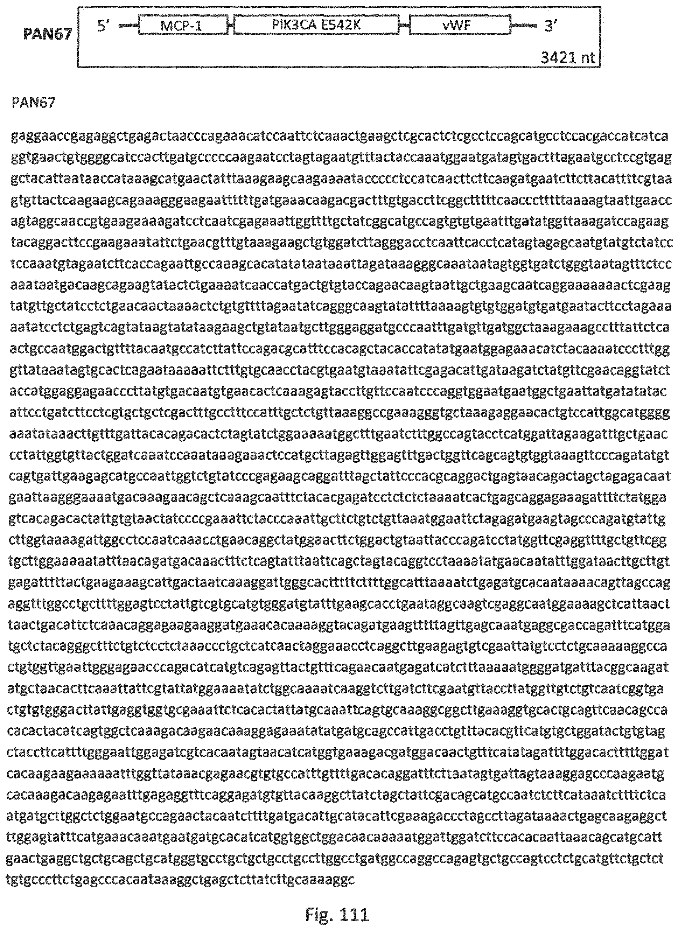

Fig. 111 shows the basic structure, length and nucleotide sequence of recombinant nucleic acid and the nucleotide sequence of construct PAN67.

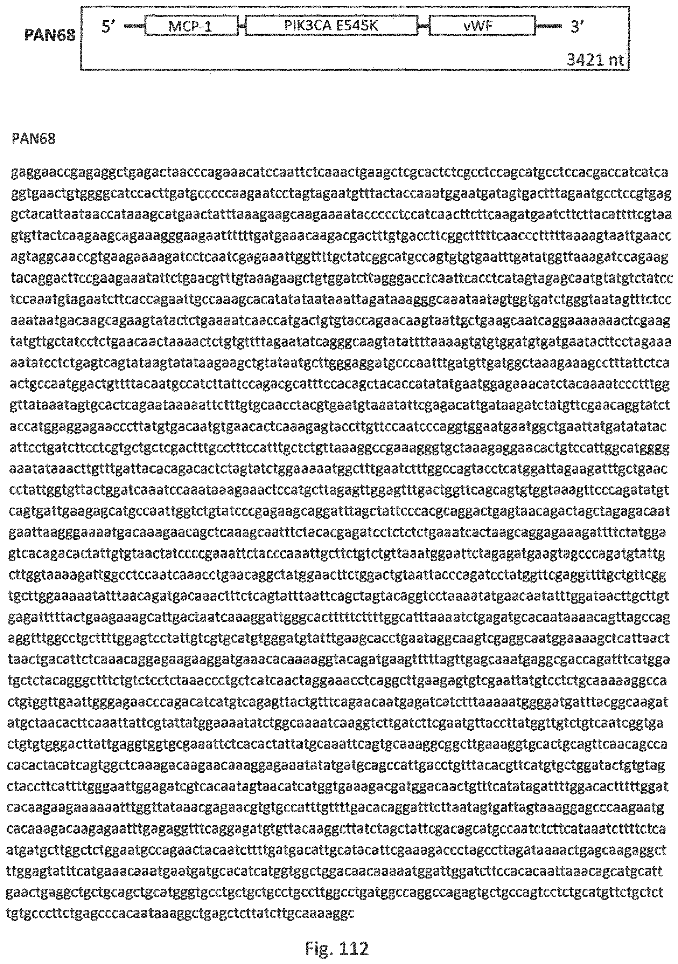

Fig. 112 shows the basic structure, length and nucleotide sequence of recombinant nucleic acid and the nucleotide sequence of construct PAN68.

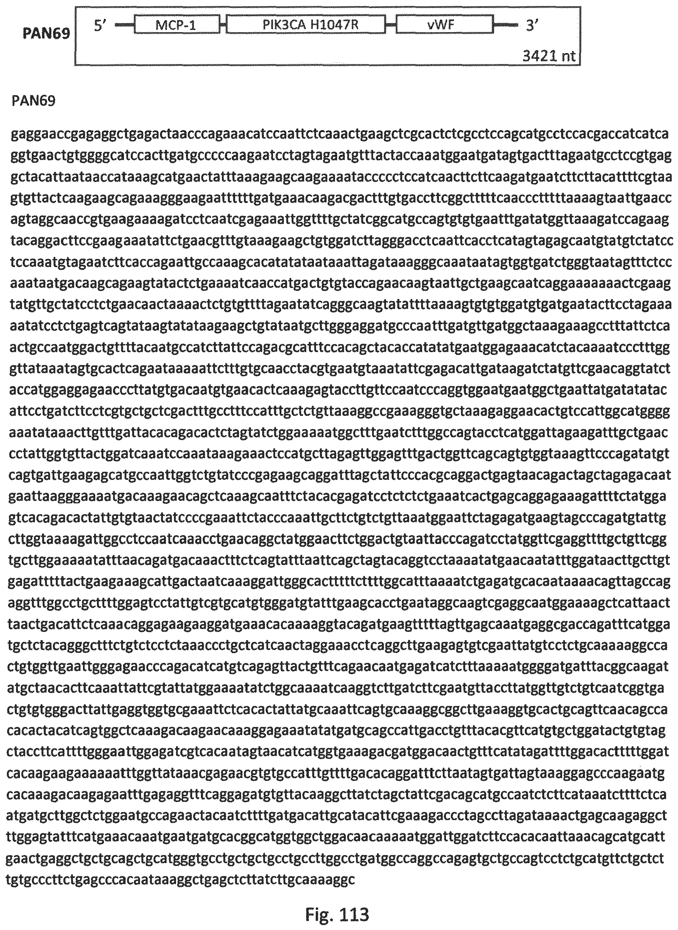

Fig. 113 shows the basic structure, length and nucleotide sequence of recombinant nucleic acid and the nucleotide sequence of construct PAN69.

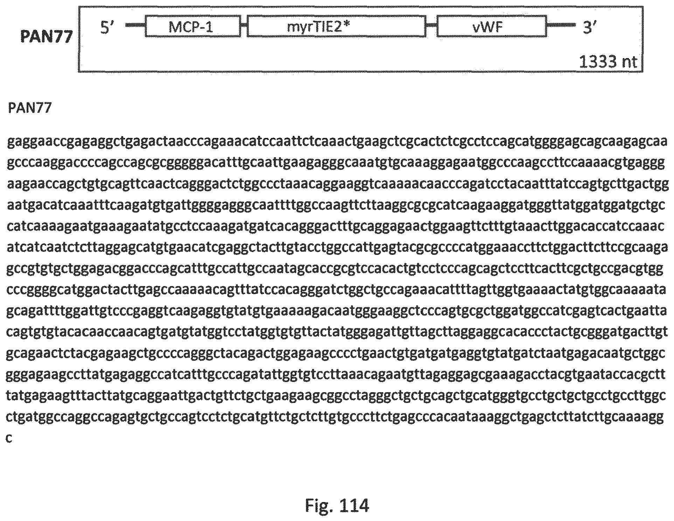

Fig. 114 shows the basic structure, length and nucleotide sequence of recombinant nucleic acid and the nucleotide sequence of construct PAN77.

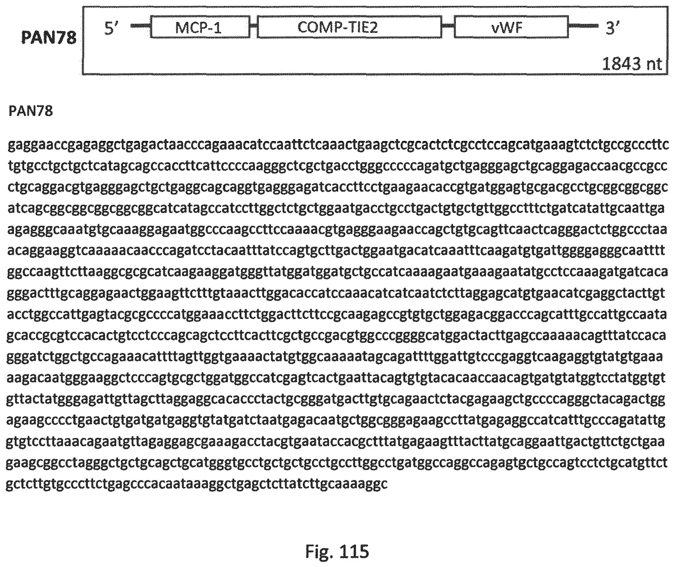

Fig. 115 shows the basic structure, length and nucleotide sequence of recombinant nucleic acid and the nucleotide sequence of construct PAN78.

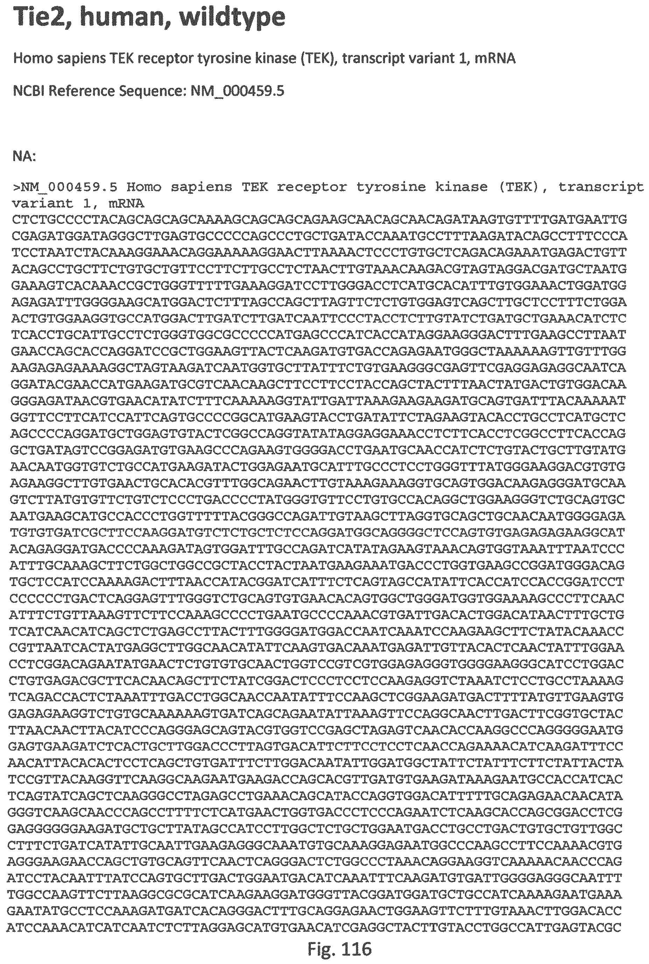

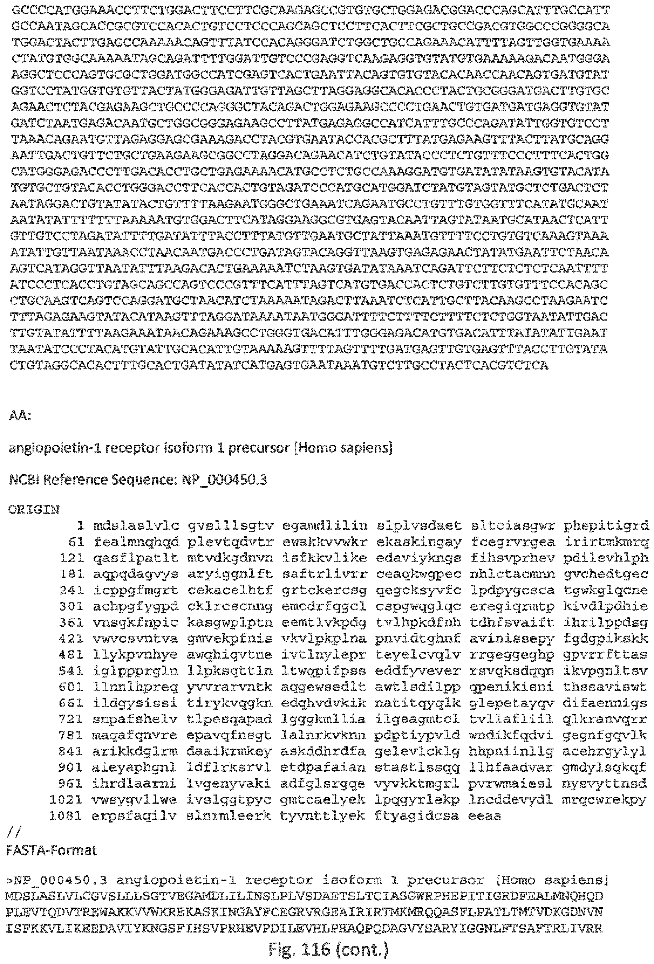

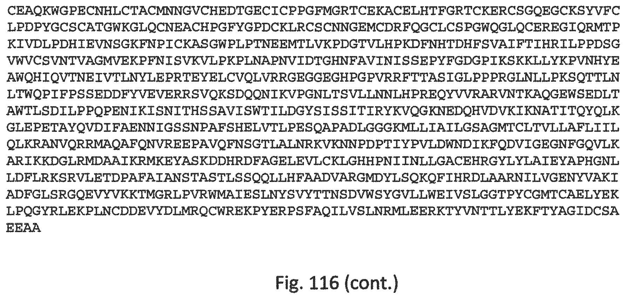

Fig. 116 shows the nucleotide sequence and amino acid sequence of human wild type Tie-2.

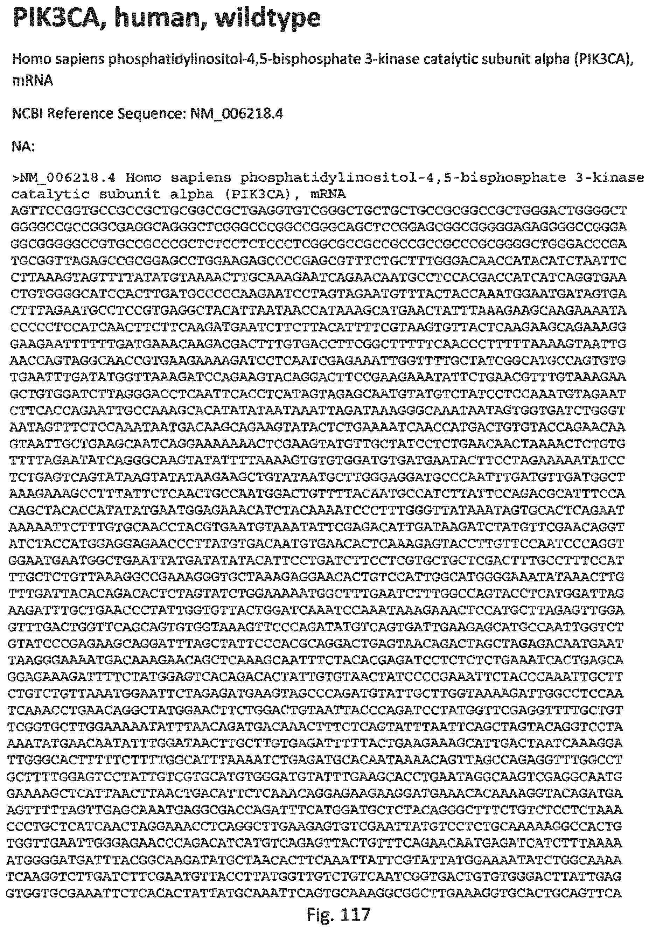

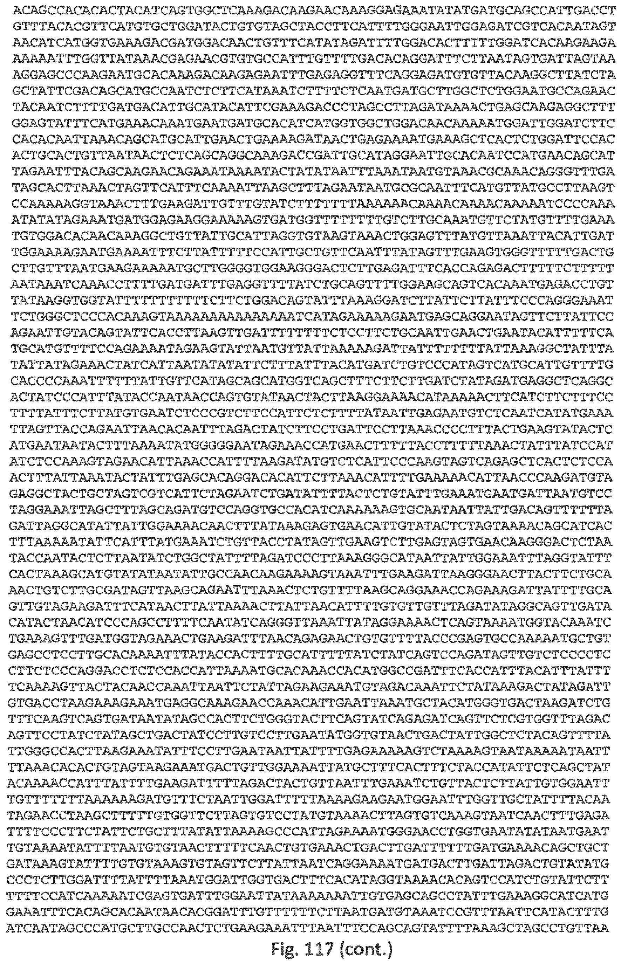

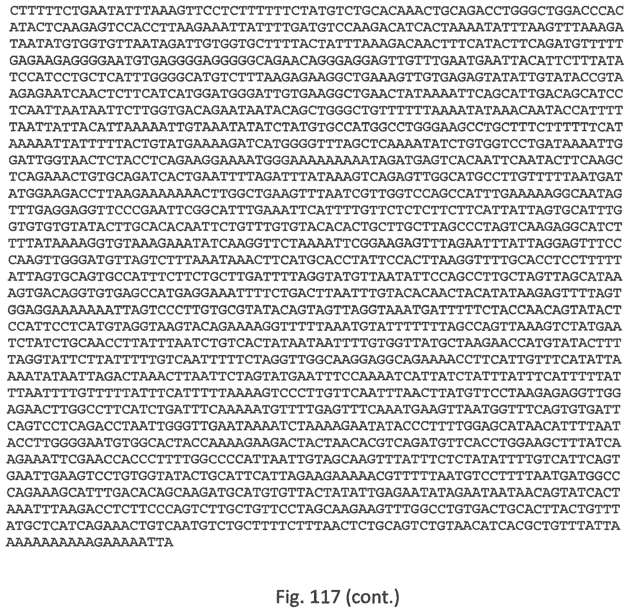

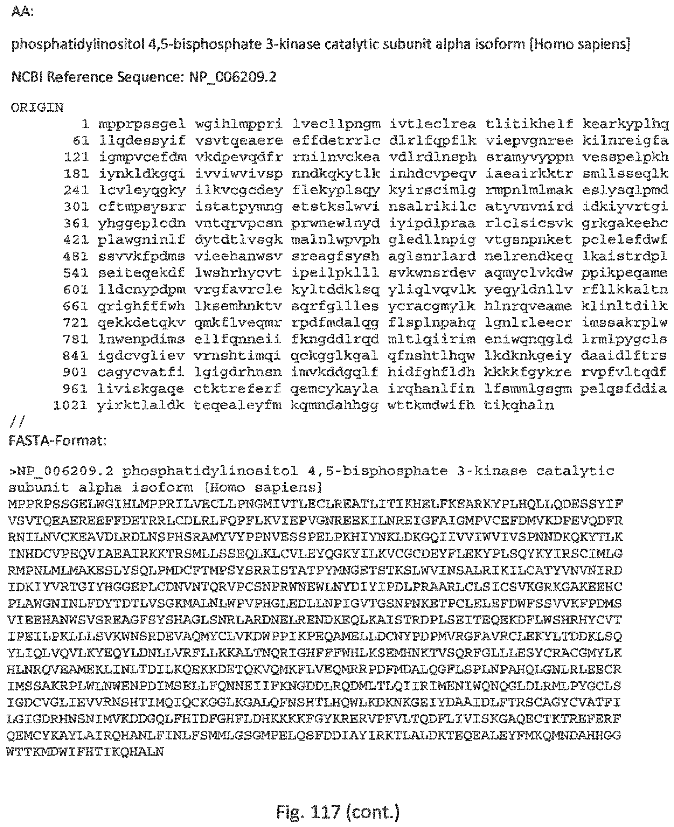

Fig. 117 shows the nucleotide sequence and amino acid sequence of human wild type PIK3CA.

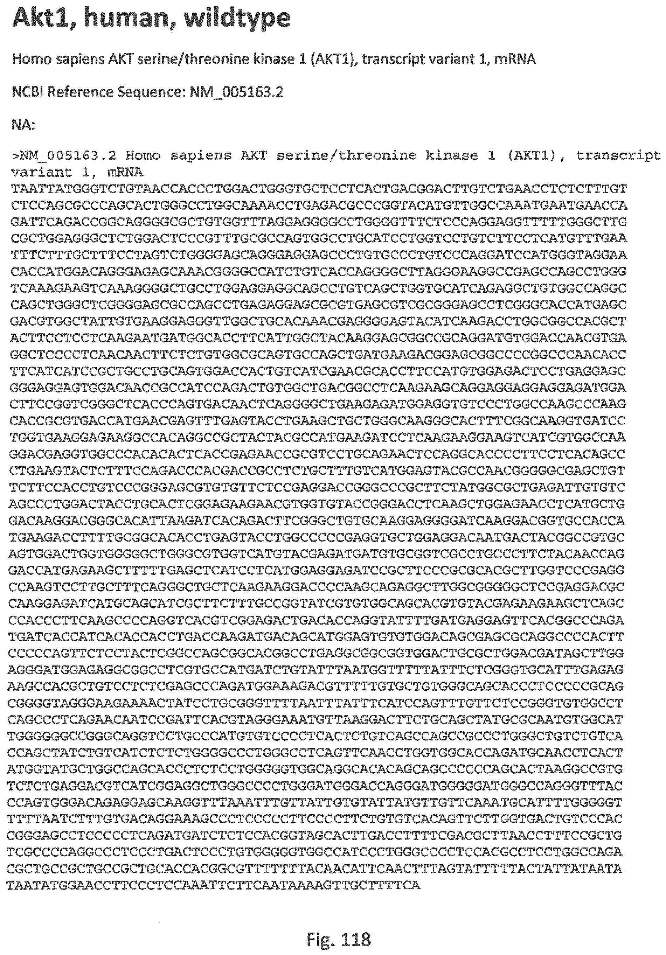

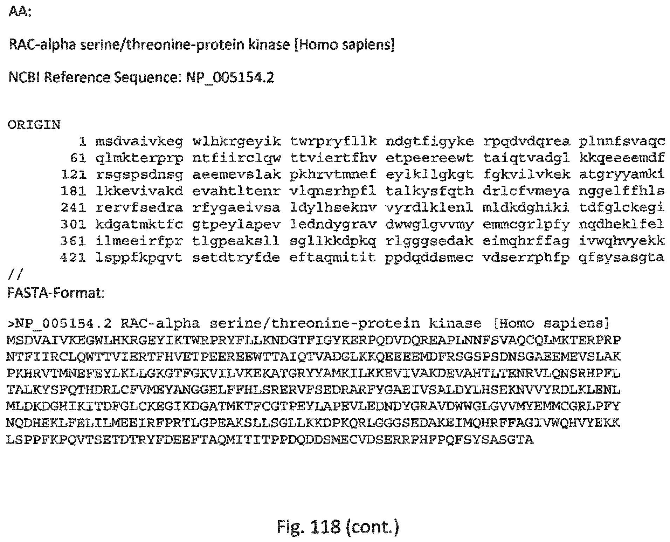

Fig. 118 shows the nucleotide sequence and amino acid sequence of human wild type Akt1.

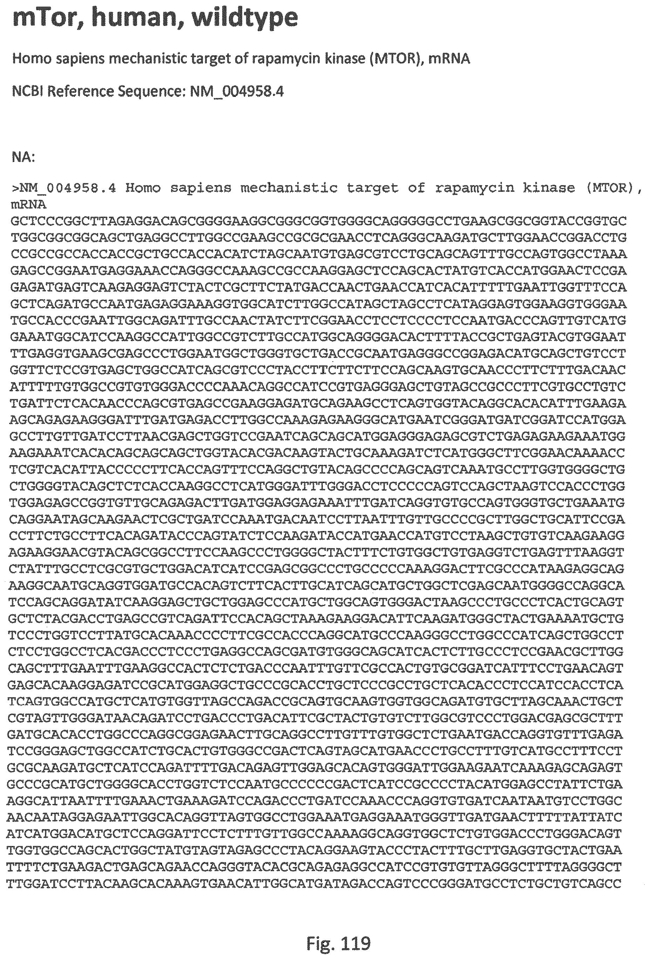

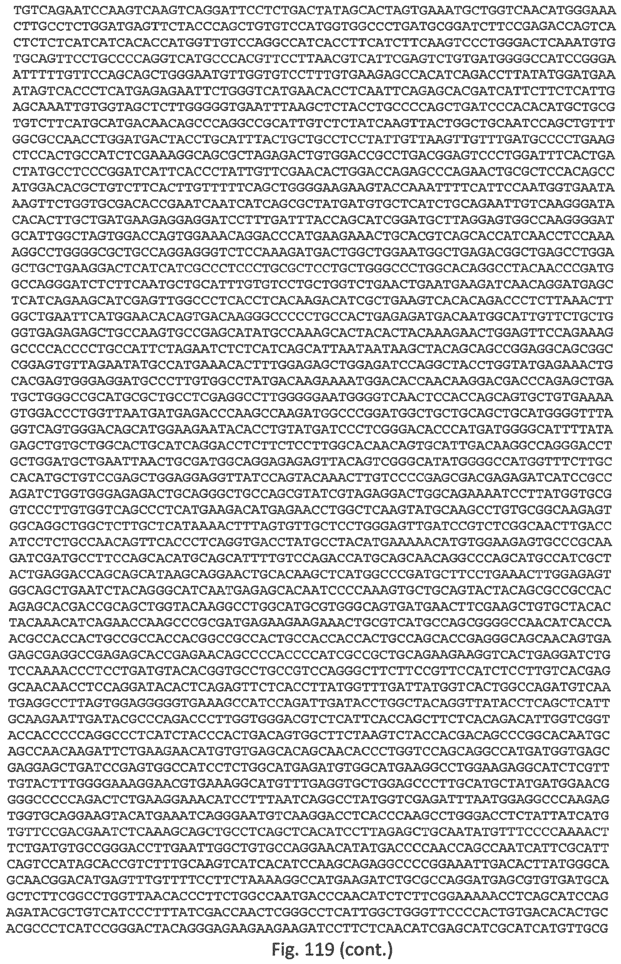

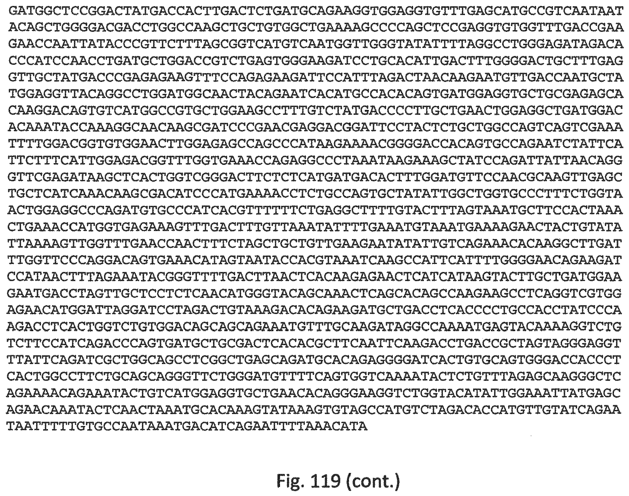

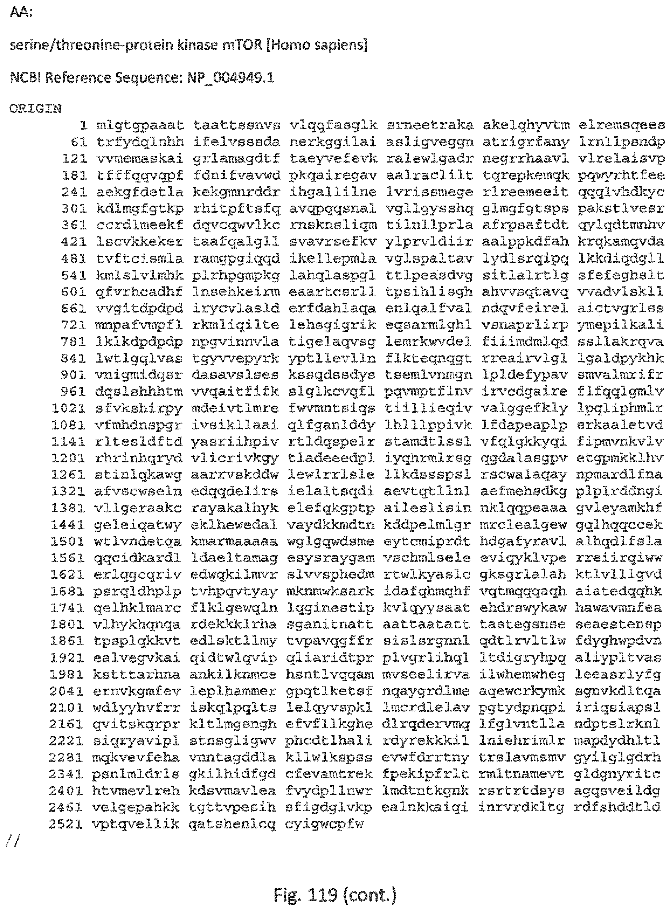

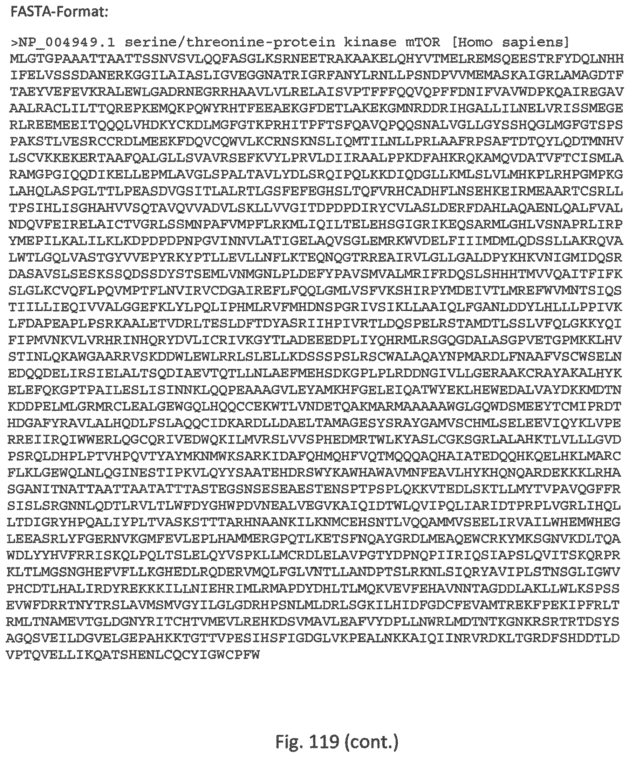

Fig. 119 shows the nucleotide sequence and amino acid sequence of human wild type mTOR.

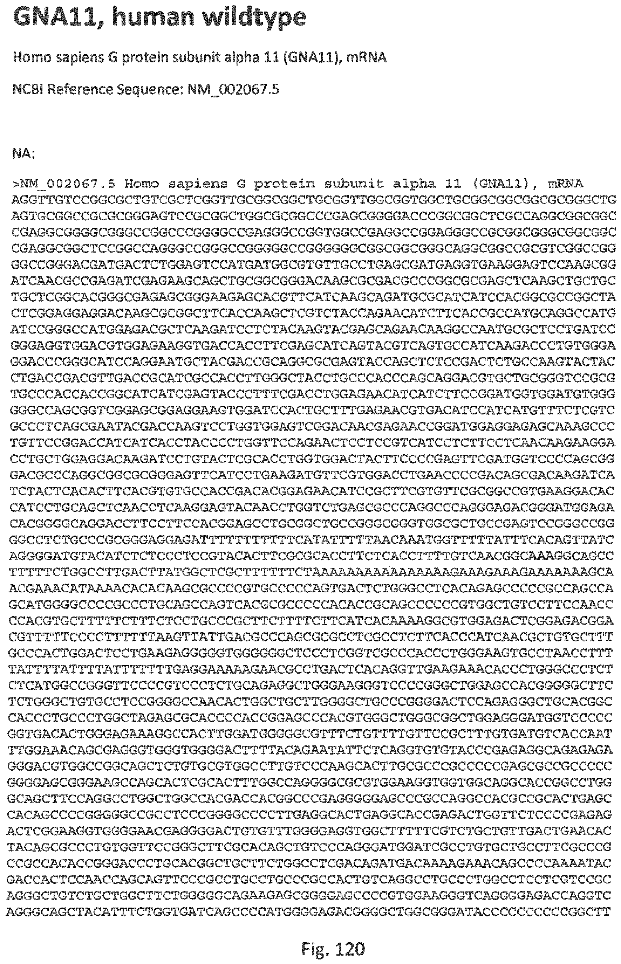

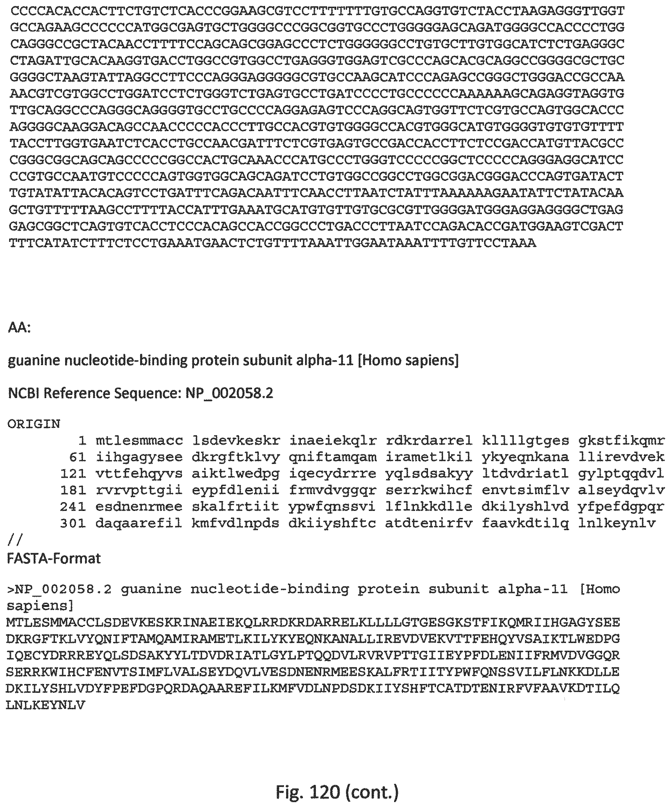

Fig. 120 shows the nucleotide sequence and amino acid sequence of human wild type GNA11.

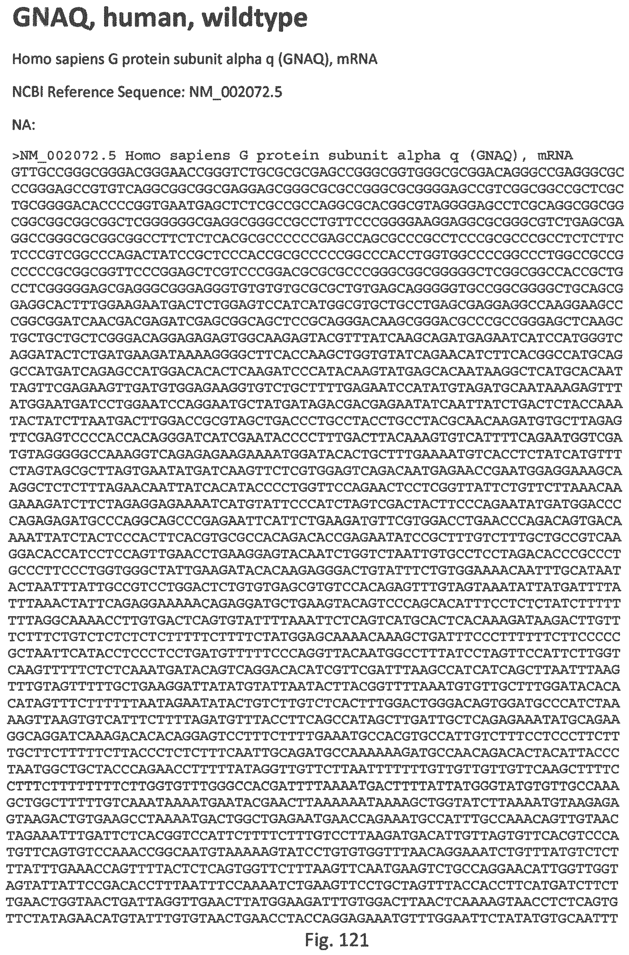

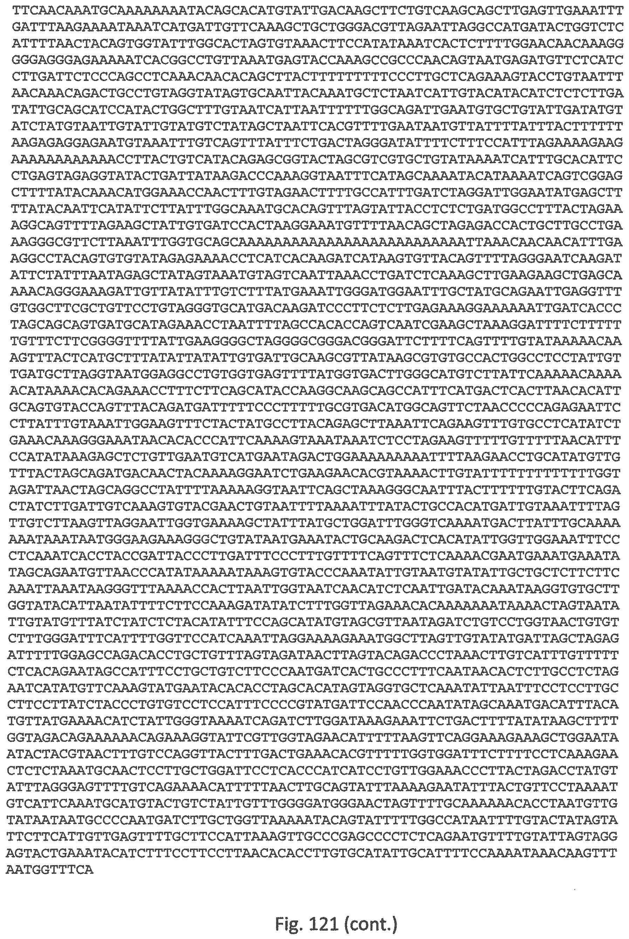

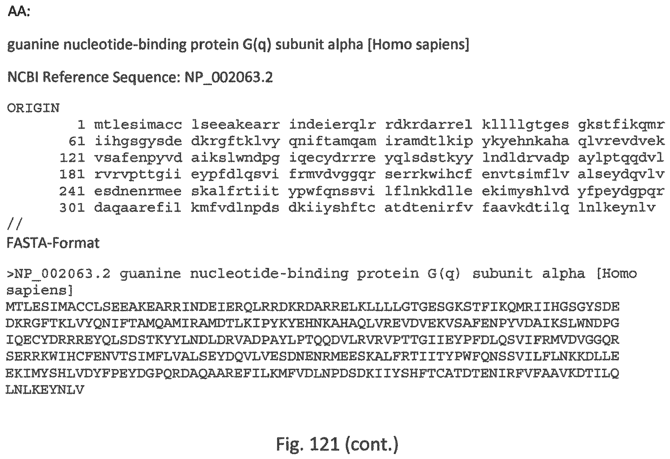

Fig. 121 shows the nucleotide sequence and amino acid sequence of human wild type GNAQ.

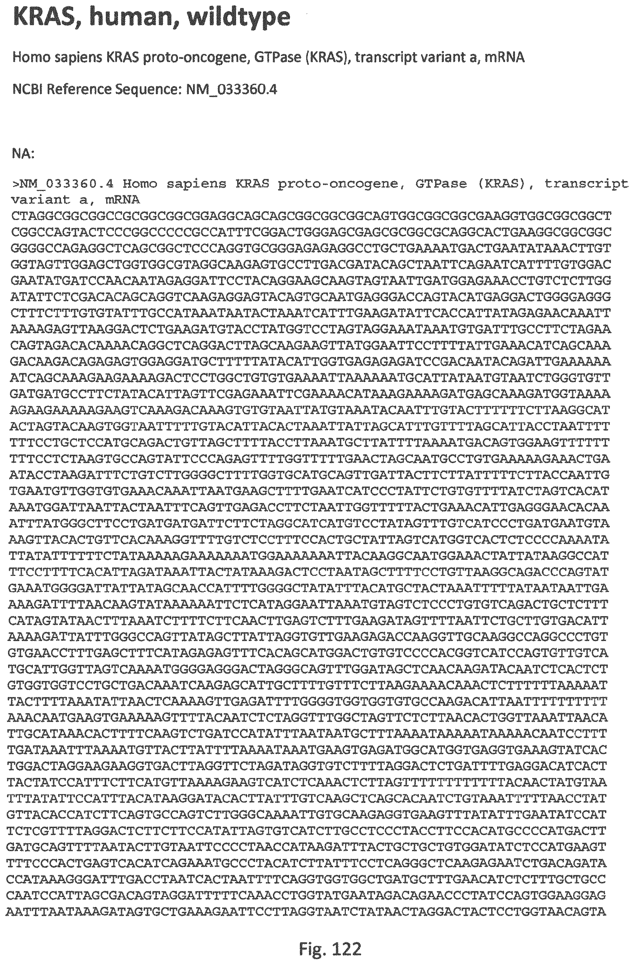

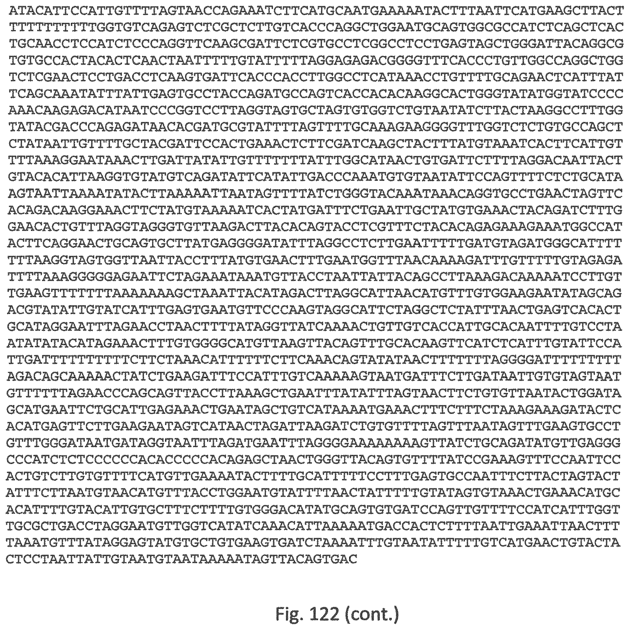

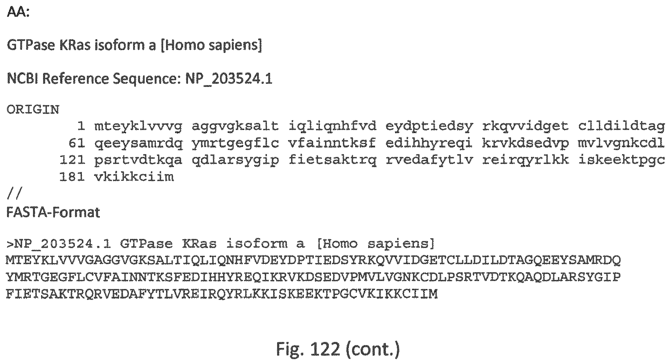

Fig. 122 shows the nucleotide sequence and amino acid sequence of human wild type KRAS.

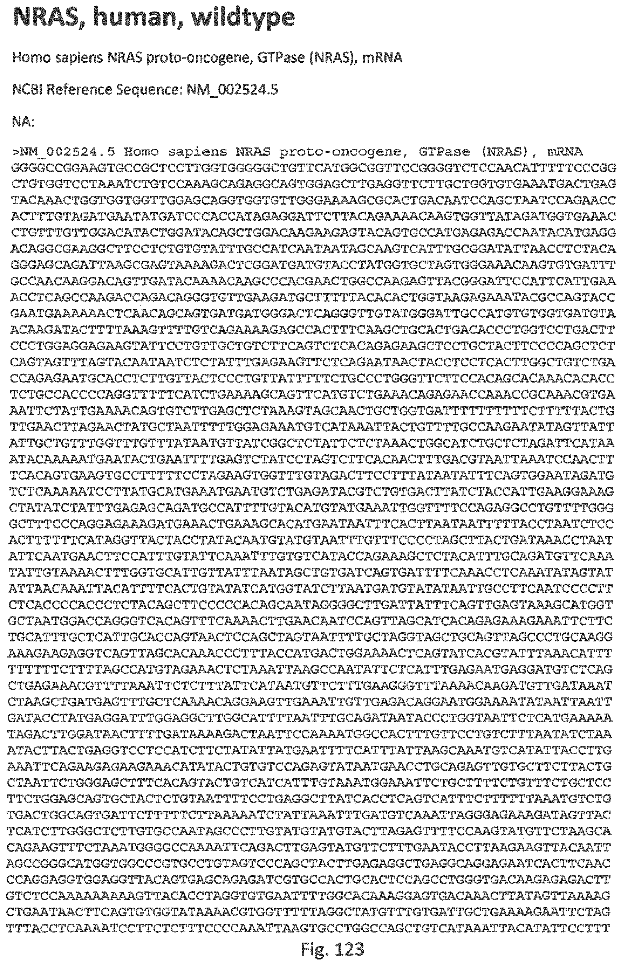

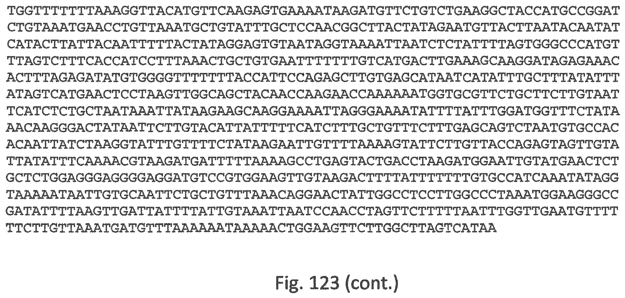

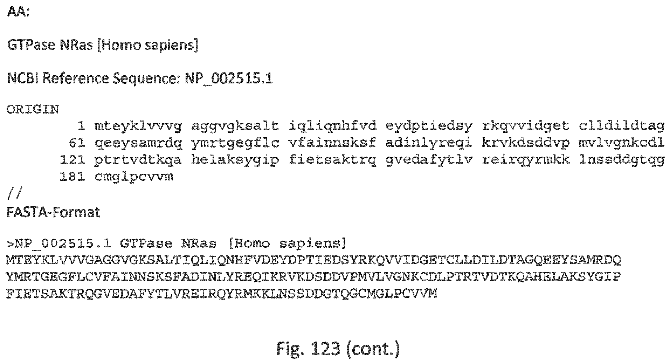

Fig. 123 shows the nucleotide sequence and amino acid sequence of human wild type NRAS.

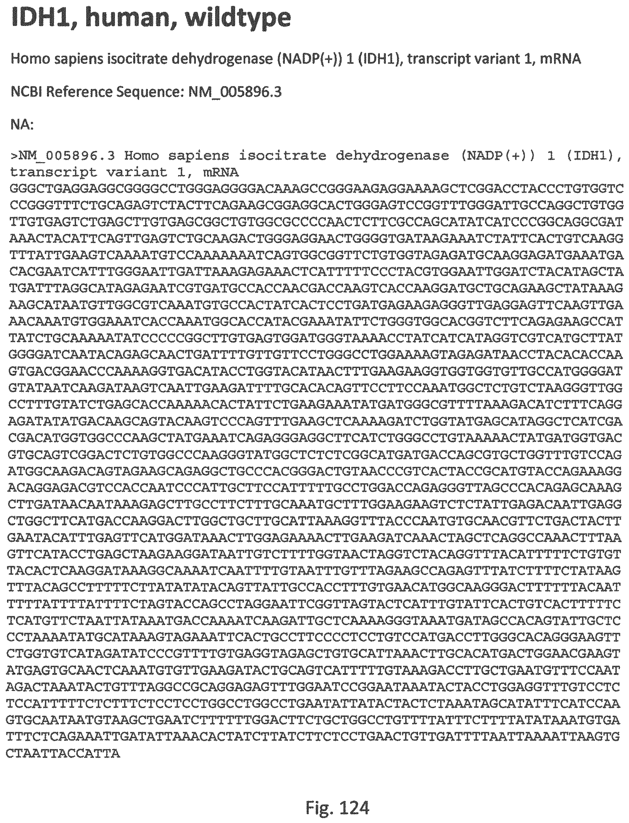

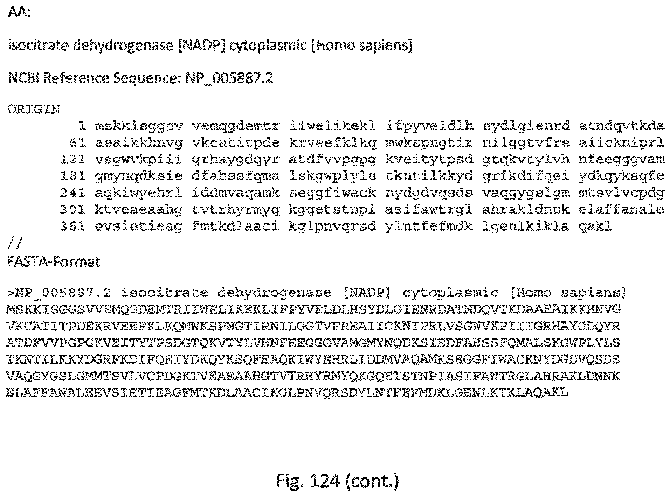

Fig. 124 shows the nucleotide sequence and amino acid sequence of human wild type IDH1.

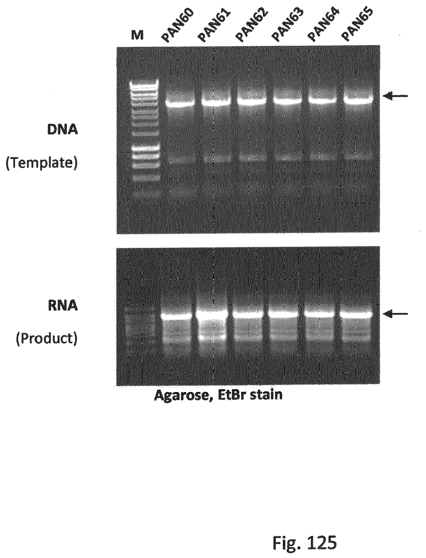

Fig. 125 shows 1% agarose gels stained with EtBr showing PCR products of Poly-A tailing PCRs.

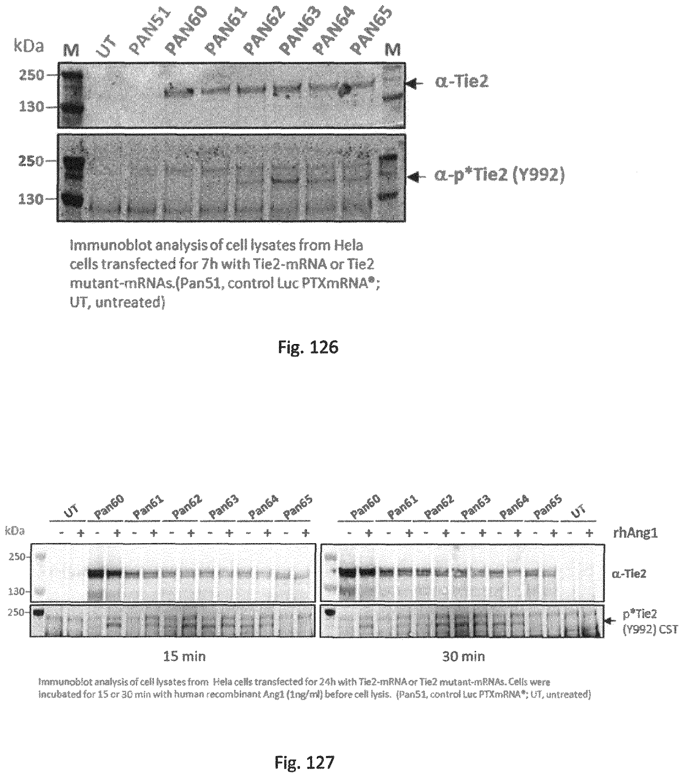

Fig. 126 and 127 show the result of an immunoblot analysis of cell lysates from Hela cells transfected with Tie-2 mRNA or Tie-2 mutant mRNA.

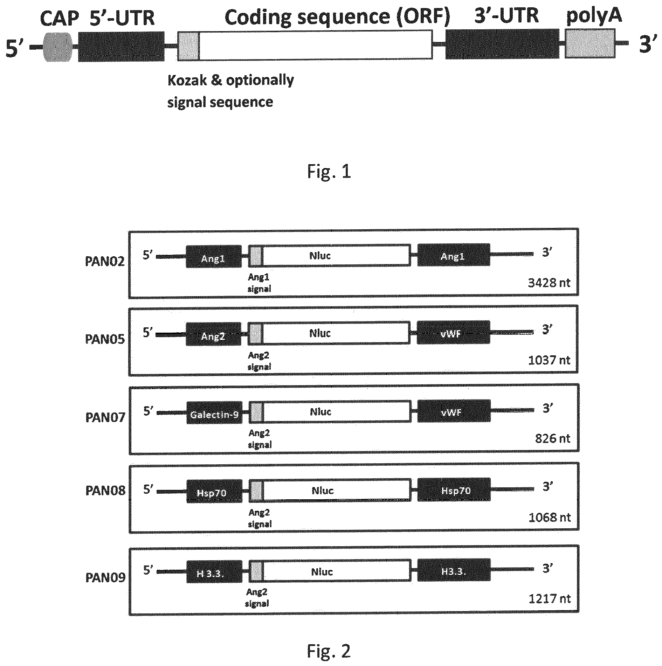

Fig. 1 is a schematic representation of conventional mRNA molecule structure. Eukaryotic including mammalian mRNAs consist of a cap region, a 5' untranslated region (UTR), the coding sequence (CDS) with an open reading frame (ORF) starting with a consensus Kozak sequence for optimal translation initiation and in case of secreted proteins followed by an signal peptide leader sequences, a 3' UTR, and a poly A-tail (>120 nt) at the 3' end.

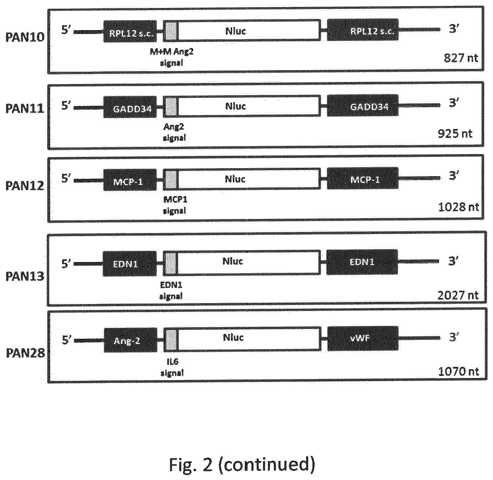

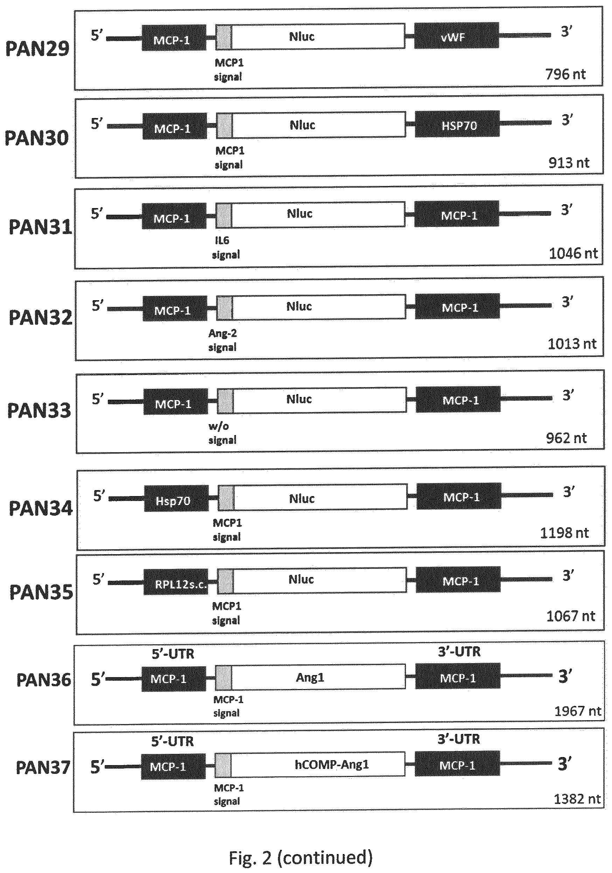

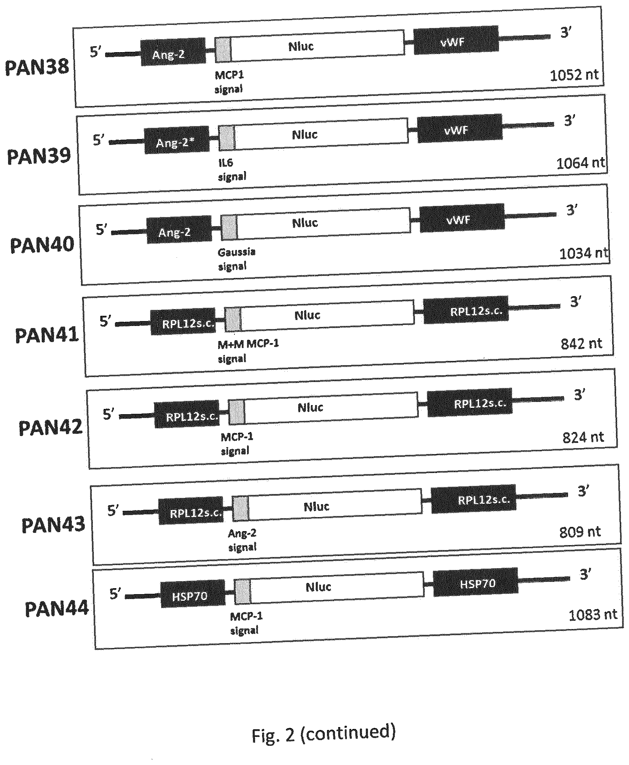

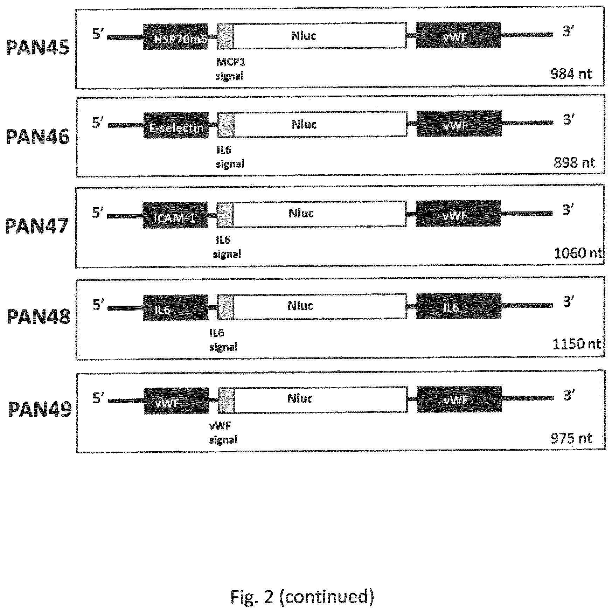

Fig. 2 is a schematic representation of selected examples for mRNA constructs with the coding sequence with an open reading frame being the one of Nano-luciferase. It is within the present invention that for each and any of the indicated mRNA constructs the coding region comprising a sequence with an open reading frame for Nano-luciferase may be replaced by a coding regions comprising a sequence coding for an effector molecule, particularly by a coding region comprising a sequence coding for an effector molecule disclosed herein; in a preferred embodiment thereof, the effector molecule is Ang1 or COMP-Angl. In accordance with the present invention, the recombinant nucleic acid constructs are designed as follows:

Recombinant nucleic acid construct PAN02 comprises as the 5' non-translated region the 5' UTR of Ang1, as the nucleic acid sequence coding for a signal peptide the nucleic acid sequence coding for a signal peptide of Ang1, as the coding region coding for an effector molecule the coding region coding for Nano luciferase, and as the 3' non-translated region the 3' UTR of Ang1.

Recombinant nucleic acid construct PAN05 comprises as the 5' non-translated region the 5' UTR of Ang2, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of von Willebrand Factor (vWF).

Recombinant nucleic acid construct PAN07 comprises as the 5' non-translated region the 5' UTR of Galectin-9, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of von Willebrand Factor (vWF).

Recombinant nucleic acid construct PAN08 comprises as the 5' non-translated region the 5' UTR of Hsp70, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of HSP70.

Recombinant nucleic acid construct PAN09 comprises as the 5' non-translated region the 5' UTR of H3.3, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of H3.3.

Recombinant nucleic acid construct PAN10 comprises as the 5' non-translated region the 5' UTR of RPL12s.c., as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of RPL12s.c. This construct additionally comprises another start codon preceding the start codon of the coding region for Nano-luciferase in-frame.

Recombinant nucleic acid construct PAN11 comprises as the 5' non-translated region the 5' UTR of GADD34, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of GADD34.

Recombinant nucleic acid construct PAN12 comprises as the 5' non-translated region the 5' UTR of MCP-1 as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN13 comprises as the 5' non-translated region the 5' UTR of EDN1, as the nucleic acid sequence coding for a signal peptide the nucleic acid sequence coding for a signal peptide of EDN1, as the coding region coding for an effector molecule the coding region coding for Nano luciferase, and as the 3' non-translated region the 3' UTR of EDN1.

Recombinant nucleic acid construct PAN28 comprises as the 5' non-translated region the 5' UTR of Ang2, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of vWF.

Recombinant nucleic acid construct PAN29 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN30 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of HSP70.

Recombinant nucleic acid construct PAN31 comprises as the 5' non-translated region the 5' UTR of MCP-1, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of MCP-1.

Recombinant nucleic acid construct PAN32 comprises as the 5' non-translated region the 5' UTR of MCP-1, as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of MCP-1.

Recombinant nucleic acid construct PAN33 comprises as the 5' non-translated region the 5' UTR of MCP-1, no nucleic acid sequence coding for a signal peptide, as the coding region coding for an effector molecule the coding region coding for Nano luciferase, and as the 3' non-translated region the 3' UTR of MCP-1.

Recombinant nucleic acid construct PAN34 comprises as the 5' non-translated region the 5' UTR of Hsp70, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN35 comprises as the 5' non-translated region the 5' UTR of RPL12 s.c. (spinach chloroplast), as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of MCP-1.

Recombinant nucleic acid construct PAN36 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Ang1, and as the 3' non-translated region the 3' UTR of MCP-1.

Recombinant nucleic acid construct PAN37 comprises as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for hCOMP-Ang1, and as the 3' non-translated region the 3' UTR of MCP-1.

Recombinant nucleic acid construct PAN38 comprises as the 5' non-translated region the 5' UTR of Ang2, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN39 comprises as the 5' non-translated region the 5' UTR of Ang2* (Ang2 5'-UTR with deleted upstream ATGs), as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN40 comprises as the 5' non-translated region the 5' UTR of Ang2, as the nucleic acid sequence coding for a signal peptide the nucleic acid sequence coding for a signal peptide of Gaussia luciferase, as the coding region coding for an effector molecule the coding region coding for Nano luciferase, and as the 3' non-translated region the 3' UTR of von Willebrand Factor (vWF).

Recombinant nucleic acid construct PAN41 comprises as the 5' non-translated region the 5' UTR of RPL12 s.c., as the nucleic acid sequence coding for a signal peptide the nucleic acid sequence coding for a signal peptide of MCP-1 with an additional upstream ATG for translational start, as the coding region coding for an effector molecule the coding region coding for Nano luciferase, and as the 3' non-translated region the 3' UTR of RPL12 s.c.. This construct additionally comprises another start codon preceding the start codon of the coding region for Nano-luciferase in-frame.

Recombinant nucleic acid construct PAN42 comprises as the 5' non-translated region the 5' UTR of RPL12 s.c., as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of RPL12 s.c._{•}

Recombinant nucleic acid construct PAN43 comprises as the 5' non-translated region the 5' UTR of RPL12 s.c., as the nucleic acid sequence coding for a signal peptide the nucleic acid sequence coding for a signal peptide of Ang2, as the coding region coding for an effector molecule the coding region coding for Nano luciferase, and as the 3' non-translated region the 3' UTR of RPL12 s.c..

Recombinant nucleic acid construct PAN44 comprises as the 5' non-translated region the 5' UTR of Hsp70, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of Hsp70.

Recombinant nucleic acid construct PAN45 comprises as the 5' non-translated region the 5' UTR of Hsp70m5, as the nucleic acid sequence coding for a signal peptide the nucleic acid sequence coding for a signal peptide of MCP-1, as the coding region coding for an effector molecule the coding region coding for Nano luciferase, and as the 3' non-translated region the 3' UTR of von Willebrand Factor (vWF).

Recombinant nucleic acid construct PAN46 comprises as the 5' non-translated region the 5' UTR of E-selectin, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN47 comprises as the 5' non-translated region the 5' UTR of ICAM1, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Recombinant nucleic acid construct PAN48 comprises as the 5' non-translated region the 5' UTR of IL-6, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of IL-6).

Recombinant nucleic acid construct PAN49 comprises as the 5' non-translated region the 5' UTR of von Willebrand Factor (vWF), as the nucleic acid sequence coding for a signal peptide the nucleic acid sequence coding for a signal peptide of von Willebrand Factor (vWF), as the coding region coding for an effector molecule the coding region coding for Nano luciferase, and as the 3' non-translated region the 3' UTR of von Willebrand Factor (vWF).

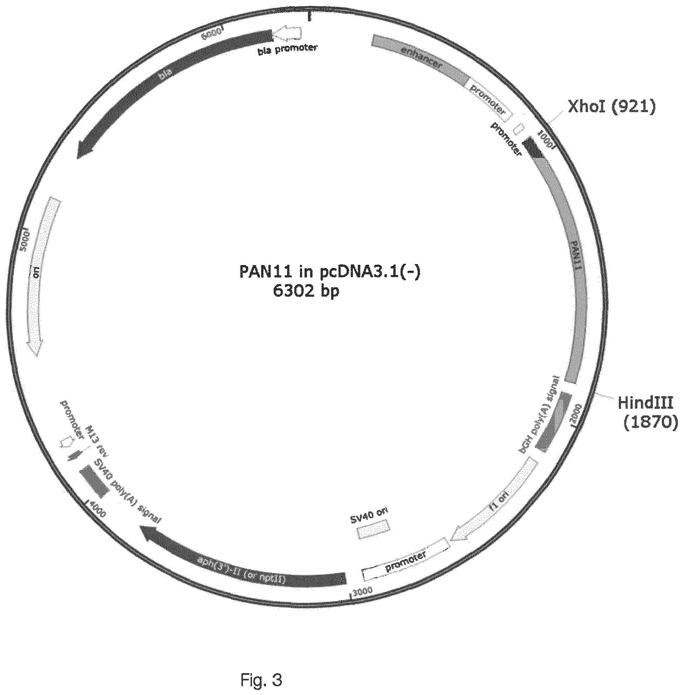

Fig. 3 shows a restriction map of a plasmid pcDNA3.1(-) used as an illustrative example for a plasmid for the expression of an exemplary recombinant nucleic acid construct of the present invention, whereby such exemplary recombinant nucleic acid construct is construct PAN 11; restriction sites XhoI/HindIII are shown.

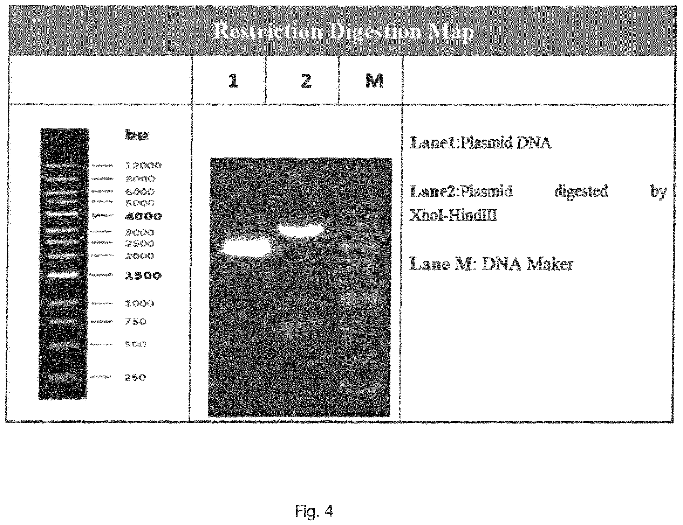

Fig. 4 shows a restriction digestion map of the PAN11 expressing pcDNA3.1- plasmid of Fig. 3; the insert represents PAN11 sequence with engineered 5' and 3' UTR, signal sequence and the coding region coding for Nano-Luciferase.

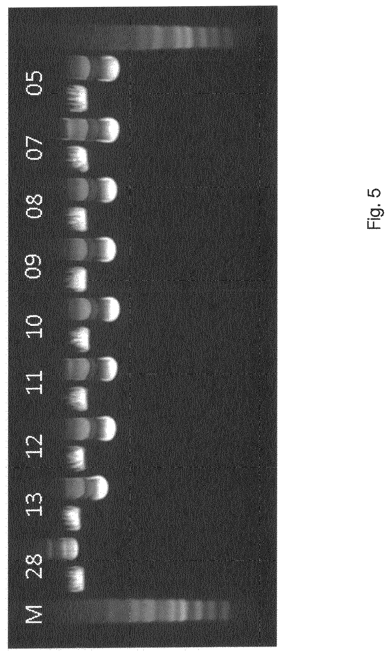

Fig. 5 is an 1% agarose gel stained with EtBr showing the non-linearized (uncut, supercoiled, right lanes) and linearization product (left lanes) of pcDNA3.1(-) plasmids upon BamHI restriction of the constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05").

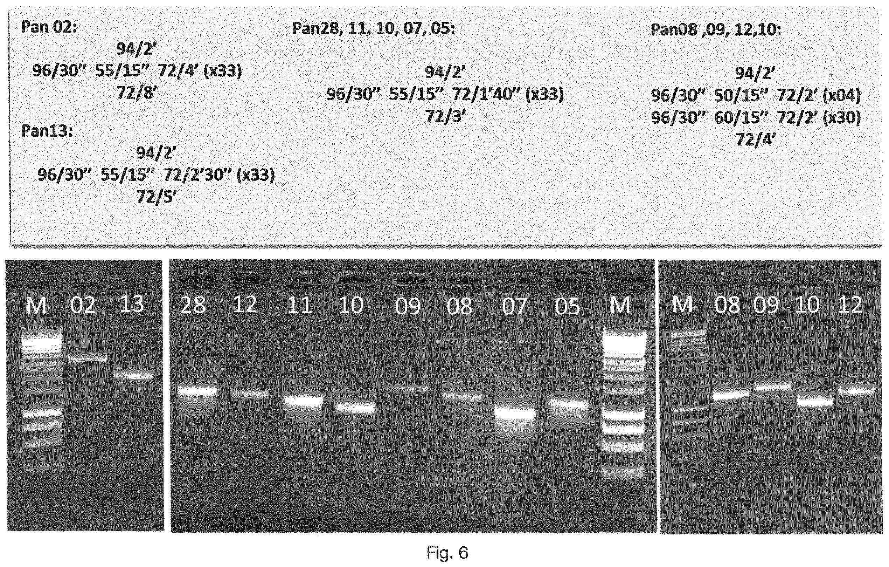

Fig. 6 shows 1% agarose gels stained with EtBr showing PCR products of Poly-A tailing PCRs for the addition of 120 nt of Poly-A tail by 120 nt long poly-T 3' primer flanking the different recombinant nucleic acid constructs. On top are the optimized PCR conditions for the indicated recombinant nucleic acid constructs. For example, for Pan02 the conditions are as follows: denaturation for 2 minutes at 94°C. 33 cycles of 30 seconds at 96°C, 15 seconds at 55°C and 4 minutes at 72°C. Final extension for 8 minutes at 72°C.

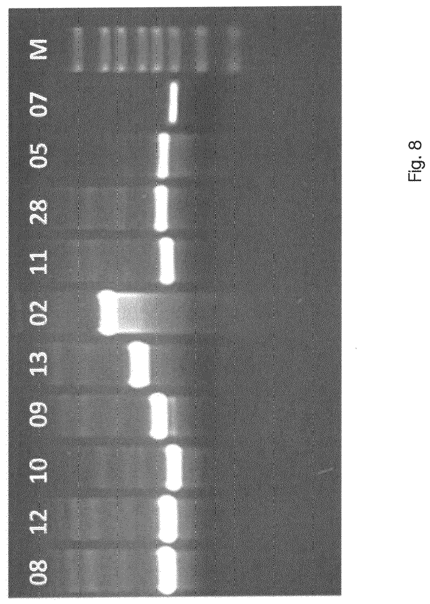

Fig. 8 is an image of a 1% non-denaturing agarose gel stained with EtBr showing the in vitro transcribed mRNAs of constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05"). M: is a lane with a high range RiboRuler RNA ladder. From this Fig. 8 may be taken that the transcribed mRNAs were intact and, more specifically, were not degraded.

Fig. 24 shows the basic structure and nucleotide sequence of recombinant nucleic acid construct PAN01. PAN1 is an embodiment of the present invention comprising as the 5' non-translated region the 5' UTR of Ang1, as the nucleic acid sequence coding for a signal peptide the nucleic acid sequence coding for a signal peptide of Ang1, as the coding region coding for an effector molecule the coding region coding for Ang1, and as the 3' non-translated region the 3' UTR of Ang1.

Fig. 25 shows the basic structure and nucleotide sequence of recombinant nucleic acid construct PAN03. PAN03 is an embodiment of the present invention comprising as the 5' non-translated region the 5' UTR of Ang1, as the nucleic acid sequence coding for a signal peptide the nucleic acid sequence coding for a signal peptide of Ang1, as the coding region coding for an effector molecule the coding region coding for COMP-Angl, and as the 3' non-translated region the 3' UTR of Ang1.

Fig. 26 shows the basic structure and nucleotide sequence of recombinant nucleic acid construct PAN04. PAN04 is an embodiment of the present invention comprising as the 5' non-translated region the 5' UTR of Ang2, as the nucleic acid sequence coding for a signal peptide the nucleic acid sequence coding for a signal peptide of Ang2, as the coding region coding for an effector molecule the coding region coding for Ang1, and as the 3' non-translated region the 3' UTR of von Willebrand Factor (vWF).

Fig. 27 shows the basic structure and nucleotide sequence of recombinant nucleic acid construct PAN06. PAN06 is an embodiment of the present invention comprising as the 5' non-translated region the 5' UTR of Ang2, as the nucleic acid sequence coding for a signal peptide the nucleic acid sequence coding for a signal peptide of Ang2, as the coding region coding for an effector molecule the coding region coding for COMP-Ang1, and as the 3' non-translated region the 3' UTR of von Willebrand Factor (vWF).

Fig. 28 shows the basic structure and nucleotide sequence of recombinant nucleic acid construct PAN36. PAN36 is an embodiment of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Ang1, and as the 3' non-translated region the 3' UTR of MCP-1.

Fig. 67 shows recombinant nucleic acid construct PAN50 which is another embodiment of an mRNA of the present invention comprising as the 5' non-translated region the 5' UTR of MCP-1, as the nucleic acid sequence coding for a signal peptide the nucleic acid sequence coding for a signal peptide of IL-6, as the coding region coding for an effector molecule the coding region coding for Nano luciferase, and as the 3' non-translated region the 3' UTR of von Willebrand Factor (vWF).

Fig. 68 shows recombinant nucleic acid construct PAN51 which is another embodiment of an mRNA of the present invention comprising as the 5' non-translated region the 5' UTR of HSP70, as the nucleic acid sequence coding for a signal peptide the nucleic acid sequence coding for a signal peptide of IL-6, as the coding region coding for an effector molecule the coding region coding for Nano luciferase (NLuc), and as the 3' non-translated region the 3' UTR of von Willebrand Factor (vWF).

Fig. 69 shows recombinant nucleic acid construct PAN52 which is another embodiment of an mRNA of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Ang1, and as the 3' non-translated region the 3' UTR of von Willebrand Factor (vWF).

Fig. 70 shows recombinant nucleic acid construct PAN53 which is another embodiment of an mRNA of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for hCOMP-Ang1, and as the 3' non-translated region the 3' UTR of von Willebrand Factor (vWF).

Fig. 71A is an image of a 1% non-denaturing agarose gel stained with EtBr showing the tail-PCR products of constructs PAN35 ("35"), PA34 ("34"), PAN33 ("33"), PAN32 ("32"), PAN31 ("31"), PAN30 ("30"), PAN29 ("29") and PAN12 ("12"). M: is a lane with a high range RiboRuler RNA ladder. From this Fig. 71A may be taken that the transcribed mRNAs were intact and, more specifically, were not degraded.

Fig. 71B is an image of a 1% non-denaturing agarose gel stained with EtBr showing the *in vitro* mRNA transcripts of constructs PAN35 ("35"), PA34 ("34"), PAN33 ("33"), PAN32 ("32"), PAN31 ("31"), PAN30 ("30"), PAN29 ("29") and PAN12 ("12"). M: is a lane with a high range RiboRuler RNA ladder. From this Fig. 71B may be taken that the transcribed mRNAs were intact and, more specifically, were not degraded.

Fig. 86 shows recombinant nucleic acid construct PAN54 which is another embodiment of an mRNA of the present invention comprising as the 5' non-translated region the 5' UTR of RPL12s.c. as the nucleic acid sequence coding for a signal peptide the nucleic acid sequence coding for a signal peptide of MCP-1, as the coding region coding for an effector molecule the coding region coding for Nano-Luciferase (NLuc), and as the 3' non-translated region the 3' UTR of von Willebrand Factor (vWF).

Fig. 87 shows recombinant nucleic acid construct PAN55 which is another embodiment of an mRNA of the present invention comprising as the 5' non-translated region the 5' UTR of vWF, as the nucleic acid sequence coding for a signal peptide the nucleic acid sequence coding for a signal peptide of MCP-1, as the coding region coding for an effector molecule the coding region coding for Nano Luciferase (NLuc), and as the 3' non-translated region the 3' UTR of von Willebrand Factor (vWF).

Fig. 88 shows recombinant nucleic acid construct PAN56 which is another embodiment of an mRNA of the present invention comprising as the 5' non-translated region the 5' UTR of vWF, as the nucleic acid sequence coding for a signal peptide the nucleic acid sequence coding for a signal peptide of vWF, as the coding region coding for an effector molecule the coding region coding for Nano Luciferase (NLuc), and as the 3' non-translated region the 3' UTR of von Willebrand Factor (vWF).

Fig. 89 shows recombinant nucleic acid construct PAN57 which is another embodiment of an mRNA of the present invention comprising as the 5' non-translated region a synthetic nucleic acid sequence as described in Jiang, Lei et al. (2018). Systemic messenger RNA as an etiological treatment for acute intermittent porphyria. Nature Medicine. 24, 1899-2909 (2018), as the nucleic acid sequence coding for a signal peptide the nucleic acid sequence coding for a signal peptide of MCP-1, as the coding region coding for an effector molecule the coding region coding for Nano Luciferase (NLuc), and as the 3' non-translated region the 3' UTR a nucleic acid sequence as described in Jiang, Lei et al. (2018). Systemic messenger RNA as an etiological treatment for acute intermittent porphyria. Nature Medicine. 24; 1899-1909 (2018). The 5'-and 3' sequences are shown underlined and in bold.

Fig. 90 shows recombinant nucleic acid construct PAN58 which is another embodiment of an mRNA of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for Nano Luciferase (NLuc), and as the 3' non-translated region the 3' UTR of von Willebrand Factor (vWF). In addition, PAN58 comprises a consensus Kozak sequence (GCCACC) in the six bases upstream of the start AUG.

Fig. 91 shows recombinant nucleic acid construct PAN59 which is another embodiment of an mRNA of the present invention comprising as the 5' non-translated region the 5' UTR of MCP-1, as the nucleic acid sequence coding for a signal peptide the nucleic acid sequence coding for a signal peptide of MCP-1, as the coding region coding for an effector molecule the coding region coding for CMP-Ang1, and as the 3' non-translated region the 3' UTR of von Willebrand Factor (vWF).

Fig. 95 shows recombinant nucleic acid construct PAN60 which is another embodiment of an mRNA of the present invention comprising as the 5' non-translated region the 5' UTR of MCP-1, as the coding region coding for an effector molecule the coding region coding for wild type (wt) Tie2, and as the 3' non-translated region the 3' UTR of von Willebrand Factor (vWF).

Fig. 96 shows recombinant nucleic acid construct PAN61 which is another embodiment of an mRNA of the present invention comprising as the 5' non-translated region the 5' UTR of MCP-1, as the coding region coding for an effector molecule the coding region coding for Tie2 mutant R849W , and as the 3' non-translated region the 3' UTR of von Willebrand Factor (vWF).

Fig. 97 shows recombinant nucleic acid construct PAN66 which is another embodiment of an mRNA of the present invention comprising as the 5' non-translated region the 5' UTR of MCP-1, as the coding region coding for an effector molecule the coding region coding for wild type PIK3CA which is, according to GenBank Homo sapiens phosphatidylinositol-4,5-bisphosphate 3-kinase catalytic subunit alpha (PIK3CA) with the respective reference number being 006218.4., and as the 3' non-translated region the 3' UTR of von Willebrand Factor (vWF).

Fig. 101 shows the basic structure and length of recombinant nucleic acid constructs PAN60, PAN61, PAN62, PAN63, PAN64 and PAN65.

Recombinant nucleic acid construct PAN60 comprises as the 5' non-translated region the 5' UTR of MCP-1, as the coding region coding for an effector molecule the coding region coding for wild type of Tie-2, and as the 3' non-translated region the 3' UTR of vWF.

Recombinant nucleic acid construct PAN61 comprises as the 5' non-translated region the 5' UTR of MCP-1, as the coding region coding for an effector molecule the coding region coding for mutant Tie-2 Tie2 R849W, and as the 3' non-translated region the 3' UTR of vWF.

Recombinant nucleic acid construct PAN62 comprises as the 5' non-translated region the 5' UTR of MCP-1, as the coding region coding for an effector molecule the coding region coding for mutant Tie-2 Tie2 L914R, and as the 3' non-translated region the 3' UTR of vWF.

Recombinant nucleic acid construct PAN63 comprises as the 5' non-translated region the 5' UTR of MCP-1, as the coding region coding for an effector molecule the coding region coding for mutant Tie-2 Tie2 R849W L914F, and as the 3' non-translated region the 3' UTR of vWF.

Recombinant nucleic acid construct PAN64 comprises as the 5' non-translated region the 5' UTR of MCP-1, as the coding region coding for an effector molecule the coding region coding for mutant Tie-2 Tie2 Y897F R915L, and as the 3' non-translated region the 3' UTR of vWF.

Recombinant nucleic acid construct PAN65 comprises as the 5' non-translated region the 5' UTR of MCP-1, as the coding region coding for an effector molecule the coding region coding for mutant Tie-2 Tie2 T1105N T 1106P, and as the 3' non-translated region the 3' UTR of vWF.

Fig. 102 shows the basic structure and length of recombinant nucleic acid constructs PAN66, PAN67, PAN68, and PAN69.

Recombinant nucleic acid construct PAN66 comprises as the 5' non-translated region the 5' UTR of MCP-1, as the coding region coding for an effector molecule the coding region coding for wild type of PIK3CA, and as the 3' non-translated region the 3' UTR of vWF.

Recombinant nucleic acid construct PAN67 comprises as the 5' non-translated region the 5' UTR of MCP-1, as the coding region coding for an effector molecule the coding region coding for mutant PIK3CA PIK3CA E542K, and as the 3' non-translated region the 3' UTR of vWF.

Recombinant nucleic acid construct PAN68 comprises as the 5' non-translated region the 5' UTR of MCP-1, as the coding region coding for an effector molecule the coding region coding for mutant PIK3CA PIK3CA E545K, and as the 3' non-translated region the 3' UTR of vWF.

Recombinant nucleic acid construct PAN69 comprises as the 5' non-translated region the 5' UTR of MCP-1, as the coding region coding for an effector molecule the coding region coding for mutant PIK3CA PIK3CA H1047R, and as the 3' non-translated region the 3' UTR of vWF.

Fig. 103 shows the basic structure and length of recombinant nucleic acid construct PAN77. Recombinant nucleic acid construct PAN77 comprises as the 5' non-translated region the 5' UTR of MCP-1, as the coding region coding for an effector molecule the sequence coding for mutant Tie-2, wherein said mutant Tie-2 is a truncated form of Tie-2, preferably the truncated form of Tie-2 comprises a nucleotide sequence of SEQ ID NO: 201, and as the 3' non-translated region the 3' UTR of vWF.

Fig. 104 shows the basic structure and length of recombinant nucleic acid construct PAN78. Recombinant nucleic acid construct PAN78 comprises as the 5' non-translated region the 5' UTR of MCP-1, as the coding region coding for an effector molecule the sequecne coding for mutant Tie-2, wherein said mutant Tie-2 is a COMP-Tie-2 fusion , preferably the truncated form of Tie-2 comprises a nucleotide sequence of SEQ ID NO: 202, and as the 3' non-translated region the 3' UTR of vWF.

Fig. 105 shows the basic structure, the length of recombinant nucleic acid and the nucleotide sequence of construct PAN70. Recombinant nucleic acid construct PAN70 comprises as the 5' non-translated region the 5' UTR of MCP-1, as the coding region coding for an effector molecule the coding sequence for myrP110*, and as the 3' non-translated region the 3' UTR of vWF.

The SEQ ID NOs: of the sequence listing are related to the instant disclosure as summarized in Table 1.

**Table 1:**

| **Seq ID No:** | **Subject to Fig.** | **Further information** |
|---|---|---|
| 1 | 7A | 5'Primer |
| 2 | 7A | 5'Primer |
| 3 | 7A | 5'Primer |
| 4 | 7A | 5'Primer |
| 5 | 7A | 5'Primer |
| 6 | 7A | 5'Primer |
| 7 | 7A | 5'Primer |
| 8 | 7A | 5'Primer |
| 9 | 7A | 5'Primer |
| 10 | 7A | 5'Primer |
| 11 | 7B | 3'Primer |
| 12 | 7B | 3'Primer |
| 13 | 7B | 3'Primer |
| 14 | 7B | 3'Primer |
| 15 | 7B | 3'Primer |
| 16 | 7B | 3'Primer |
| 17 | 7B | 3'Primer |
| 18 | 7B | 3'Primer |
| 19 | 7B | 3'Primer |
| 20 | 15A | 5'UTR MCP1 |
| 21 | 15B | SP (signal peptide) nucleotide sequence of MCP1 |
| 22 | 15B | SP amino acid sequence of MCP1 |
| 23 | 15C | 3'UTR of MCP1 |
| 24 | 16A | 5'UTR of RPL12 |
| 25 | 16B | 3'UTR of RPL12 |
| 26 | 17A | 5'UTR of Ang2 |
| 27 | 17B | SP (signal peptide) nucleotide sequence of Ang2 |
| 28 | 17B | SP (signal peptide) amino acid sequence of Ang2 |
| 29 | 17C | 3'UTR of Ang2 |
| 30 | 18A | 5'UTR of IL6 |
| 31 | 18B | SP (signal peptide) nucleotide sequence of IL6 |
| 32 | 18B | SP (signal peptide) amino acid sequence of IL6 |
| 33 | 18C | 3'UTR of IL6 |
| 34 | 19A | 5'UTR of vWF |
| 35 | 19B | SP(signal peptide) nucleotide sequence of vWF |
| 36 | 19B | SP (signal peptide) amino aicd sequence of vWF |
| 37 | 19C | 3'UTR of vWF |
| 38 | 20A | 5'UTR of HSP70 A1 |
| 39 | 20B | 3'UTR of HSP70 A1 |
| 40 | 21A | 5'UTR of HSP70 A5 |
| 41 | 21B | SP (signal peptide) nucleotide sequence of HSP70 A5 |
| 42 | 21B | SP (signal peptide) amino acid sequence of HSP70 A5 |
| 43 | 21C | 3'UTR of HSP70 A5 |
| 44 | 22A | 5'UTR of H3.3 |
| 45 | 22B | 3'UTR of H3.3 |
| 46 | 23A | 5'UTR of LGALS9 |
| 47 | 23B | 3'UTR of LGALS9 |
| 48 | 24 | Construct PAN01 |
| 49 | 25 | Construct PAN03 |
| 50 | 26 | Construct PAN04 |
| 51 | 27 | Construct PAN06 |
| 52 | 28 | Construct PAN36 |
| 53 | 29 | Construct PAN37 |
| 54 | 30 | Construct PAN02 |
| 55 | 31 | Construct PAN05 |
| 56 | 32 | Construct PAN07 |
| 57 | 33 | Construct PAN08 |
| 58 | 34 | Construct PAN09 |
| 59 | 35 | Construct PAN10 |
| 60 | 36 | Construct PAN11 |
| 61 | 37 | Construct PAN12 |
| 62 | 38 | Construct PAN13 |
| 63 | 39 | Construct PAN28 |
| 64 | 40 | Construct PAN29 |
| 65 | 41 | Construct PAN30 |
| 66 | 42 | Construct PAN31 |
| 67 | 43 | Construct PAN32 |
| 68 | 44 | Construct PAN33 |
| 69 | 45 | Construct PAN34 |
| 70 | 46 | Construct PAN35 |
| 71 | 47 | Construct PAN38 |
| 72 | 48 | Construct PAN39 |
| 73 | 49 | Construct PAN40 |
| 74 | 50 | Construct PAN41 |
| 75 | 51 | Construct PAN42 |
| 76 | 52 | Construct PAN43 |
| 77 | 53 | Construct PAN44 |
| 78 | 54 | Construct PAN45 |
| 79 | 55 | Construct PAN46 |
| 80 | 56 | Construct |
| 81 | 57 | Construct PAN48 |
| 82 | 58 | Construct PAN49 |
| 83 | 59A | 5'UTR of Ang1 |
| 84 | 59B | SP (signal peptide) nucleotide sequence of Ang1 |
| 85 | 59B | SP (signal peptide) amino acid sequence of Ang1 |
| 86 | 59C | CDS of Ang1 (mature peptide+stop codon) |
| 87 | - | Amino acid sequence derived from SEQ ID No. 86 |
| 88 | 59D | 3'UTR of Ang1 |
| 89 | 60A | 5'UTR of Ang4 |
| 90 | 60B | SP (signal peptide) nucleotide sequence of Ang4 |
| 91 | 60B | SP (signal peptide) amino acid sequecne of Ang4 |
| 92 | 60C | CDS of Ang4 (mature peptide+stop codon) |
| 93 | - | Amino acid sequence derived from SEQ ID No.92 |
| 94 | 60D | 3'UTR of Ang4 |
| 95 | 61 | COMP-Ang1 CDS (mature pep+stop codon) (rat) |
| 96 | - | Amino acid sequence derived from SEQ ID No.95 |
| 97 | 62 | hCOMP-Ang1 CDS (mature pep+stop codon) (human) |
| 98 | - | Amino acid sequence derived from SEQ ID No.97 |
| 99 | 63 | CMP-Ang1 CDS (mat peptide+stop codon) |
| 100 | 64 | COMP-Ang2 CDS (mat peptide+stop codon) |
| 101 | 65 | Tie2 CDS (start+mat pep+stop codon) |
| 102 | - | Amino acid sequence derived from SEQ ID No. 101 |
| 103 | 66 | Tie2* CDS (R849W)(start+mat pep+stop codon) |
| 104 | - | Amino acid sequence derived from SEQ ID No. 103 |
| 105 | 67 | Construct PAN50 |
| 106 | 68 | Construct PAN51 |
| 107 | 69 | Construct PAN52 |
| 108 | 70 | Construct PAN53 |
| 109 | 86 | Construct PAN54 |
| 110 | 87 | Construct PAN55 |
| 111 | 88 | Construct PAN56 |
| 112 | 89 | Construct PAN57 (Mod) |
| 113 | 90 | Construct PAN58 |
| 114 | 91 | Construct PAN59 |
| 115 | 95 | Construct PAN60 (Tie2 wt) |
| 116 | 96 | Construct PAN61 (Tie2 R849W) |
| 117 | 97 | Construct PAN66 (PIK3CA wt) |
| 118 | | Construct CDS (start+mat pep+stop codon) PIK3CA |
| 119 | | CDS (mature peptide+stop cod) Nluc |
| 120 | 100a | 5'UTR of (PAN57) |
| 121 | 100b | 3'UTR of (PAN57) |
| 122 | 7B | 3'Primer for constructs PAN01-PAN03 |
| 123 | 100c | 5'Primer for construct PAN57 |
| 124 | 100d | 3'Primer for construct PAN57 |
| 125 | 100e | 5'Primer for construct PAN55 (Fig. 87) and PAN56 (Fig 88) |
| 126 | 116 | nucleotide sequence of human wild type Tie-2 |
| 127 | 116 | amino acid sequence of human wild type Tie-2 |
| 128 | 107 | PAN62 |
| 129 | 108 | PAN63 |
| 130 | 109 | PAN64 |
| 131 | 110 | PAN65 |
| 132 | 117 | nucleotide sequence of human wild type PIK3CA |
| 133 | 117 | amino acid sequence of human wild type PIK3CA |
| 134 | 111 | PAN67 |
| 135 | 112 | PAN68 |
| 136 | 113 | PAN69 |
| 137 | 118 | nucleotide sequence of human wild type Akt1 |
| 138 | 118 | amino acid sequence of human wild type Akt1 |
| 139 | 119 | nucleotide sequence of human wild type mTor |
| 140 | 119 | amino acid sequence of human wild type mTor |
| 141 | 120 | nucleotide sequence of human wild type GNA11 |
| 142 | 120 | amino acid sequence of human wild type GNA11 |
| 143 | 121 | nucleotide sequence of human wild type GNAQ |
| 144 | 121 | amino acid sequence of human wild type GNAQ |
| 145 | 122 | nucleotide sequence of human wild type KRAS |
| 146 | 122 | amino acid sequence of human wild type KRAS |
| 147 | 123 | nucleotide sequence of human wild type NRAS |
| 148 | 123 | amino acid sequence of human wild type NRAS |
| 149 | 124 | nucleotide sequence of human wild type IDH1 |
| 150 | 124 | amino acid sequence of human wild type IDH1 |
| 151 | 105 | nucleotide sequence of PAN70 |
| 152 | 114 | nucleotide sequence of PAN77 |
| 153 | 115 | nucleotide sequence of PAN78 |

### Example 1: Materials and Methods

### Plasmid Template generation by gene synthesis and cloning

Sequences encoding Nluc reporter protein, Ang-1 protein and derivatives thereof (e.g. COMP-Ang-1, CMP-Ang-1) with different signal peptides were flanked by different heterologous 5' and 3' UTRs and were designed and produced by gene synthesis by BioCat (Heidelberg) and cloned (Xho1-BamHI) into pcDNA3.1- (Thermo Fisher).

Sequences encoding wt Tie-2 and sequences with the specified Tie-2 mutation in the coding region flanked by different heterologous 5' and 3' UTRs were designed (see Figures 95 and 96) and produced by gene synthesis by BioCat (Heidelberg) and cloned (Xho1-BamHI) into pcDNA3.1- (Thermo Fisher).

### In vitro transcription from PCR products to generate polyadenylated mRNAs

In vitro transcription is the synthesis of RNA transcripts by RNA polymerase from a linear DNA template containing the corresponding promoter sequence (T7, T3, SP6) and the gene to be transcribed. A typical transcription reaction consists of the template DNA, RNA polymerase, ribonucleotide triphosphates, RNase inhibitor and buffer containing Mg2⁺ ions. Linearized plasmid DNA, PCR products and synthetic DNA oligonucleotides can be used as templates for transcription as long as they have the T7 promoter sequence upstream of the gene to be transcribed. In order to generate DNA templates with a poly-(A) tail a tail-PCR using 5' primers containing T7 RNA polymerase sequences and 3' primers with a 120 nt Poly-T sequence were synthesized and purified (BioSpring, Frankfurt).

The Linear DNA templates for in vitro transcription were generated by PCR from linearized plasmid. Plasmids were digested with BamHI that cut once 3-terminal in the vector backbone to produce a linearized vector that can be used as the template for the poly-(A) tail PCR. 5µg plasmid are digested for 2h at 37°C with 5U BamHI restriction enzyme in 50µl and 1x HF Buffer. Small Aliquots of digested mix were analyzed by gel electrophoresis to check for complete digestion of the plasmid (see, for example, Fig. 5 for constructs PAN28 ("28"), PAN13 ("13"), PAN12 ("12"), PAN11 ("11"), PAN10 ("10"), PAN09 ("09"), PAN08 ("08"), PAN07 ("07") and PAN05 ("05")). The restriction enzyme is heat-inactivated by incubating at 80°C for 20 min and the digested plasmid is purified by a PCR purification column following the manufacturer's protocol (NucleoSpin Gel and PCR clean-up, Macherey-Nagel). The linearized plasmid can be stored at -20°C for several months and can be used for PCR template generation. The purpose of linearization was to eliminate circular templates that could potentially generate run-on transcripts during the IVT reaction.

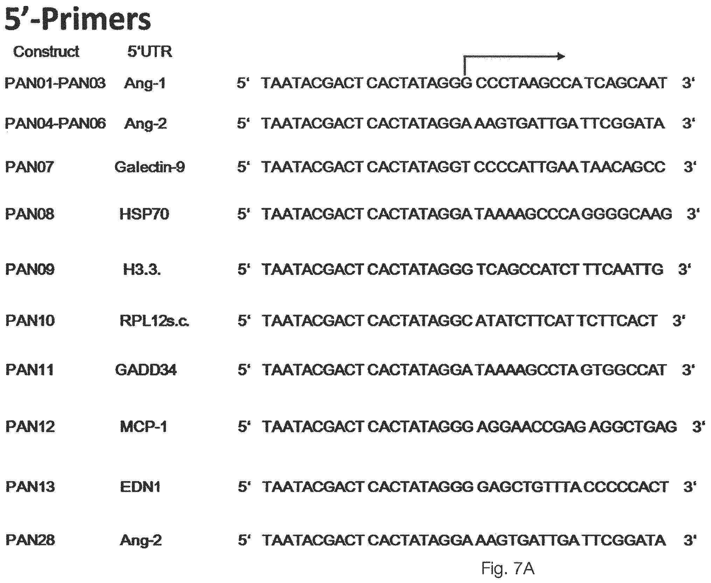

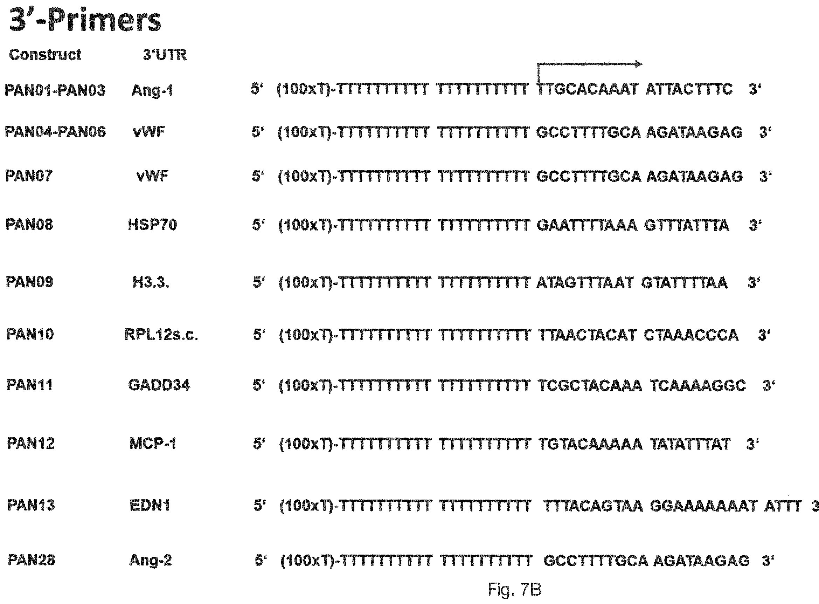

Addition of poly-(A) tail by PCR was performed by using Hot StarHiFidelity polymerase (Qiagen) or Taq DNA Polymerase (Roche) following the manufacturer's protocol. For this purpose, Adapter primers containing T7 promoter sequences and 3' Poly A tail sequences (120nt) were used to amplify linear DNA-templates. Each of the adapter primers had overlap sequences which fused regulatory sequences necessary for translation and transcription to the gene of interest (see, for example, Figures 7a and 7b). A typical 50µl PCR reaction contained final concentrations of 200µM (of each) dNTP, 0,5µM for each primer, 50 ng linearized plasmid DNA, 1x PCR reaction buffer (1,5 mM MgCl2) and 1,25 U Taq DNA Polymerase per reaction (0,25 µl of 5U/µl). The specific PCR cycle programs or thermal profiles were adjusted according to the Tm of the primer pairs, according to the expected length of the PCR product and according to the used thermal cycler. The quality of the PCR products was checked by analyzing an aliquot by gel electrophoresis and the reactions are purified by commercial PCR purification kits ((NucleoSpin Gel and PCR clean-up, Macherey-Nagel).

### In vitro transcription reaction

Addition of a 5' end cap structure to the RNA is an important process in eukaryotes. It is essential for RNA stability, efficient translation, nuclear transport and splicing. The process involved addition of a 7-methylguanosine cap at the 5' triphosphate end of the RNA. RNA capping can be carried out post-transcriptionally using capping enzymes or co-transcriptionally using cap analogs such as ARCA (Jena Bioscience) or CleanCap (Trilink Biotechnologies) or EZ-Cap (ApexBio). In the enzymatic method, the mRNA may be capped using the vaccinia virus mRNA capping enzyme (NEB) as per manufacturer's protocol. The enzyme adds on a 7-methylguanosine cap at the 5' end of the RNA using GTP and S-adenosyl methionine as donors (cap-0 structure). Both methods yield functionally active capped RNA suitable for transfection or other applications. In addition, a 2'-O-Methyltransferase can be used to introduce cap-1 and cap-2 structures.

In the example described below, the T7 High Yield Transcription Kit (Thermo Scientific) was used to synthesize capped RNA transcripts (PAN 01-XX) using cap analogs co-transriptionally.

The DNA template for the transcription reaction was a linearized plasmid or a PCR product. For the capped RNA the reaction at room temperature was set up in the following order:

| **Component** | **Volume (µl)** | **Final** |
|---|---|---|
| Nuclease free water* | To 20 | |
| 5X Reaction Buffer | 4 | 1X |
| ATP (100 mM) | 2 | 10mM |
| CTP (100 mM) or 5'-Methylcytidine-5'-Triphophate (100 mM) | 2 | 10mM |
| UTP (100 mM) or Pseudouridine-5'-Triphosphate (100 mM) or 5-Methoxyuridine-5'-Triphosphate (100 mM) N1-Methyl-pseudouridine (100mM) | 2 | 10 mm |
| GTP (30 mM) | 2 | 3mM |
| 3'-0-Me-m⁷G(5')ppp(5')G cap analog (ARCA; Anti Reverse Cap Analog) (100 mM) | 2 | 10mM |
| Template DNA* | X | 1µg |
| T7 RNA Polymerase Mix | 2 | |
| **Total** | **20** | |

1 µg DNA was added and the total reaction volume to 20 µl was obtained by adding nuclease free water. The amount of water to be added varied based on the concentration of the template DNA. The reactions at were well mixed by vortexing and incubated at 37 °C for 2 hours in a dry air incubator. The transcription reactions were treated with DNase I to remove the DNA template before proceeding with purification as follows:
1. 70 µl nuclease free water was added to the transcription reactions followed by 10 µl of DNase I reaction buffer.
2. 2 µl DNase I were added to the reactions.
3. The reaction was incubated at 37 °C for 15 minutes.

### Column Purification of Capped mRNA

1. The capped RNA was purified using the MEGAclearKit as per the manufacturer's instructions (any spin column based RNA purification kit may be used).
2. The RNA was quantified using a NanoDrop Spectrophotometer.
3. As per the manufacturer's instructions, RNA sample quality was assessed using the Agilent RNA 6000 Nano Kit and Agilent 2100 Bioanalyzer or by native 1% agarose gel electrophoresis and ethidium bromide staining (see, Fig. 8).

### mRNA transfection of HeLa cells, HEK293 cells, HPMEC cells, HPAEC cells and HUVEC cells

HeLa cells, were grown in DMEM complete medium. The human embryonic kidney 293 cell line (HEK293) is grown in EMEM (EBSS) + 2mM Glutamine + 1% Non Essential Amino Acids (NEAA) + 10% FCS culture medium.

The human embryonic kidney 293 cell line (HEK293) is grown in EMEM (EBSS) + 2mM Glutamine + 1% Non Essential Amino Acids (NEAA) + 10% FCS culture medium. Subculture Routine is to split sub-confluent cultures (70-80%) 1:2 to 1:6 i.e. seeding at 2-5x10,000 cells/cm² using 0.25% trypsin or trypsin/EDTA; 5% CO₂; 37°C. mRNA transfection are performed with the Lipofectamine™ MessengerMAX™ Transfection Reagent (Invitrogen™) according to the manufactures protocol.

Primary Human Pulmonary Microvascular Endothelial Cells (HPMEC) and Primary Human Pulmonary Artery Endothelial Cells (*HPAEC*) are isolated from human pulmonary arteries are most appropriate for studying human lung diseases and are isolated from the lung from a single donor. Since lung tissue contains blood and lymphatic capillaries, HPMEC comprise Blood and Lymphatic Microvascular Endothelial Cells.

The cell type of HPMEC's are characterized by immunofluorescent staining. They stain positive for CD31 and von Willebrand factor and negative for smooth muscle alpha-actin.

HUVEC, HPAEC and HPMEC cells were grown in Endothelial Cell Growth Medium as recommended from *P*romoCell (Heidelberg Germany,) respectively. The Endothelial Cell Growth Medium is basal Medium supplemented with Fetal Calf Serum (0.05 ml/ml), Endothelial Cell Growth Supplement (bovine hypothalamic extract; 0.004 ml ml), Heparin 90µg/ml and Hydrocortisone 1µg/ml. The experiments with HUVEC, HPAEC and HPMEC cells are performed on cells with passage numbers below 8.

RNA transfections and more mRNA transfections were carried out using Lipofectamin MessengerMAX (Invitrogen, Carlsbad, CA; Thermo Fisher Scientific).. For mRNA transfection in 24-well plate format 1x10⁵ cells/well were seeded 24h before transfection to reach 70-90 confluence immediately before transfection. For one single well 1.5µl Lipofectamine MessengerMAX Reagent was added to 25µl OptMEM (Invitrogen, Carlsbad, CA; Thermo Fisher Scientific), vortexed and incubated for 10 min at RT. In a second well 500ng or 1µg mRNA were added to 25µl OptiMEM, and vortexed. After incubation diluted mRNA was added to diluted Lipofectamin, mixed well, incubated for 5 min at RT and added dropwise to cells in the presence of 0,5 ml of complete medium for Hela and HEK293. For HUVEC, HPAEC and HPMEC the Endothelial Cell Growth medium was replaced and the cells washed with OptiMEM and then the Lipofectamin MessengerMAxX mRNA complex solution added to the well with 0,5 ml OptiMEM prewarmed to 37 °C. Cells were incubated at 37°C for 2h and the OptiMEM medium replaced with complete Endothelial Cell Growth Medium (PromoCell, Heidelberg Germany).

Cell Lysates from Transfected cells or serum were analyzed at the indicated time points. Alternatively, mRNA-LNPs based on the cationic lipids L-Arginyl-β-alanine-N-palmityl-N-oleyl-amide or β-(L-Arginyl)-L-2,3-diamino propionic acid-N-palmityl-N-oleyl-amide in combination with neutral and PEGylated co-lipids were used for in vitro transfection. The co-lipids were Diphytanoyl-PE and the PEGylated lipid is methoxyPEG2000-DSPE

Successful introduction of mRNA into host cells was monitored using various known methods, such as a fluorescent marker, such as Green Fluorescent Protein (GFP), such as reporter enzymes (e.g. luciferase derivatives). Alternatively, transfection of a modified mRNA could also be determined by measuring the protein expression level of the target polypeptide by e.g., Western Blotting or immunocytochemistry or ELISA.

Fully modified mRNAs with 5-methylcytidine and/or pseudouridine (or 5-methoxyuridine or N1-methylpseudouridine) are transfected into HUVEC or HPMEC (Human Pulmonary Microvascular Endothelial Cells, PromoCell, Heidelberg) using Lipofectamin MessengerMAX (Invitrogen, Carlsbad, CA; Thermo Fisher Scientific). The cell lysates are harvested and run by ELISA (or on immunoblot assays (western) at different time points (30", 1 h, 2h, 4h, 6h hours) after transfection to determine the protein expression.

### Nano-Luc Luciferase activity reporter measurement

A time dependent quantitative detection of Nano-Glo-Luciferase expression (Promega) was performed for secreted versions by analyzing samples from the tissue culture supernatant according to the reporter assay technical manual Nano-Glo Luciferase Assay System (Promega). In brief, 5-20µl of serum or lysate were diluted in 100µl H₂O final volume and equal volume of reconstituted Nano-Glo luciferase assay reagent was combined in a 96 well GloMax 96 Microplate. After at least 3 min incubation at RT luminescence were measured in an appropriate luminometer.

### Immunoblot detection of Tie-2 and derivates

For immunoblot detection of Tie-2 and derivatives protein lysates are loaded on NuPage SDS-PAGE system (chambers and power supply) with 1.5mm ready-to-use Bis-Tris gels and 4-12% acrylamide gradient with MOPS-buffer as running aid (all Life Technologies, Grand Island, NY). Each lysate sample is prepared to 40 µl final volume. This sample contains 25 µg protein lysate in variable volume, RIPA buffer to make up volume to 26 µl, 4 µl of 10x reducing agent and 10 µl 4x SDS loading buffer (both from Life Technologies, Grand Island, NY). Samples are heated at 95°C for 5min and loaded on the gel. Standard settings are chosen by the manufacturer, 200V, 120mA and max. 25 W. Run time is 60min, but no longer than running dye reaching the lower end of the gel.

After the run is terminated, the plastic case is cracked and the encased gel transferred to a ready-to-use nitrocellulose membrane kit and power supply (iBLOT; LifeTechnologies, Grand Island, NY). Using default settings, the protein lysate is transferred by high Ampere electricity from the gel to the membrane.

After the transfer, the membranes are incubated in 5% BSA in IX TBS for 15 minutes then in 5% BSA in IX TBS + 0.1 % Tween for another 15 minutes. Primary antibodies against human Tie-2 proteins or Phospho-specific Antibodies (P-Tie-2, P-Akt, see below) are applied in 3ml of 5% BSA in IX TBS solution at a 1:500 to 1:2000 dilution for 3 hours at room temperature and gentle agitation on an orbital shaker. Membranes are washed 3 times with IX TBS/0.1 % Tween, 5 minutes each time with gentle agitation. The secondary antibody (Goat anti-rabbit HRP conjugate; Abeam, Cambridge, MA) is conjugated to horse radish peroxidase and binds to the primary antibody antibodies. The secondary antibody is diluted from 1:1000 to 1:5000 in 5% BSA in IX TBS and incubated for 3 hrs at RT. At the end of incubation time, the membranes are washed 3 times with IX TBS/0.1 % Tween, 5 minutes each time with gentle agitation. The membranes are developed in 5ml Pierce WestPico Chemiluminescent Subtrate (Thermo Fisher, Rockford, IL) as directed.

### Example 2: Expression of luciferase, Ang-1 or Ang-1-derivatives expressing recombinant nucleic acid constructs in HPMEC, HUVEC, HPAEC and HeLa cells

### mRNA transfection of HeLa, HUVEC, HPAEC and HPMEC cells

Primary Human Pulmonary Microvascular Endothelial Cells (HPMEC) are most appropriate for studying human lung diseases and are isolated from the lung from a single donor. Since lung tissue contains blood and lymphatic capillaries, HPMEC comprise Blood and Lymphatic Microvascular Endothelial Cells. The cells were routinely analyzed by immunofluorescent staining: they stain positive for CD31 and von Willebrand factor and negative for smooth muscle alpha-actin. HeLa cells, HUVEC, HPMEC and HPAEC (human pulmonary artery endothelial cells) cells, respectively, were grown in DMEM complete medium or Endothelial Cell Growth Medium (PromoCell, Heidelberg Germany, CatNo.: C-22020) respectively. The Endothelial Cell Growth Medium was basal Medium supplemented with Fetal Calf Serum (0.05 ml/ml), Endothelial Cell Growth Supplement (bovine hypothalamic extract; 0.004 ml ml), Heparin 90µg/ml and Hydrocortisone 1µg/ml. The experiments with HUVEC and HPMEC cells were performed on cells with passage numbers below 8.

mRNA transfections were carried out using Lipofectamin MessengerMAX (Invitrogen, Carlsbad, CA; Thermo Fisher Scientific). For mRNA transfection in 24-well plate format 1x10⁵ cells/well were seeded 24h before transfection to reach 70-90 confluence immediate before transfection. For one single well 1.5µl Lipofectamine MessengerMAX Reagent was added to 25µl OptiMEM, vortex and incubated for 10 min at RT. In a second well add 500ng or 1µg mRNA were added to 25µl OptiMEM, vortex. After incubation diluted mRNA was added to diluted Lipofectamin, mixed well, incubated for 5 min at RT and added dropwise to cells in the presence of 0,5 ml of DMEM complete medium for HeLa. For HUVEC, HPMEC and HPAEC the Endothelial Cell Growth medium was replaced and the cells washed with OptiMEM and then added the Lipofectamin MessengerMAX mRNA complex solution to the well with 0,5 ml OptiMEM prewarmed to 37 °C. Cells were incubated at 37°C for 2h and the OptiMEM medium was replaced with complete Endothelial Cell Growth Medium (PromoCell, Heidelberg Germany) and transfected cells or serum were analyzed at the indicated time points. Alternatively, mRNA-LNPs based on the cationic lipids *L*-Arginyl-β-alanine-*N-*palmityl-*N*-oleyl-amide or *β*-(*L*-Arginyl)-*L*-2,3-diamino propionic acid-*N*-palmityl-*N*-oleyl-amide in combination with neutral and PEGylated co-lipids can be used for in vitro transfection.

Successful introduction of mRNA into host cells can be monitored using various known methods, such as a fluorescent marker, such as Green Fluorescent Protein (GFP), such as reporter enzymes (e.g. luciferase derivatives) or transfection of a modified mRNA can also be determined by measuring the protein expression level of the target polypeptide by e.g., Western Blotting or immunocytochemistry or ELISA.

### Nano-Luc Luciferase activity reporter measurement

A time dependent quantitative detection of Nano-Glo-Luciferase expression (Promega) was performed for secreted versions by analyzing samples from the tissue culture supernatant according to the reporter assay technical manual Nano-Glo Luciferase Assay System (Promega). In brief, 5-20µl of serum or lysate were diluted in 100µl H₂O final volume and equal volume of reconstituted Nano-Glo luciferase assay reagent was combined in a 96 well GloMax 96 Microplate. After at least 3 min incubation at RT luminescence could be measured in an appropriate luminometer.

### Detection of Ang-1, COM-Ang-1, CMP-Ang-1 protein in cell lysates and supernatant

A time dependent quantitative detection of secreted Ang-1 and secreted COMP-Ang-1 or COMP-Ang-2 expression is performed by analyzing samples from the supernatant tissue culture by ELISA (Human Angiopoietin-1 Quantikine ELISA Kit (DANG10, , R&D systems) or by Western blot according to the technical manual.

500 ng of COMP-Ang-1 (mRNA sequence shown in SEQ ID NO: PAN05) with poly A tail of approximately 120 nucleotides not shown in sequence; 5 'cap,) fully modified with 5-methylcytidine and pseudouridine (COMP-Ang-1, 5mC/pU), fully modified with 5-methylcytidine and Nl-methyl-pseudouridine (COMP-Ang-1,5mC/NlmpU) or unmodified (COMP-Ang-, unmod) is transfected into HUVEC or HPMEC (Human Pulmonary Microvascular Endothelial Cells, PromoCell, Heidelberg) using Lipofectamin MessengerMAX (Invitrogen, Carlsbad, CA; Thermo Fisher Scientific). The supernatant is harvested and run by ELISA 4 hours after transfection to determine the protein expression and cytokine induction.

For immunoblot detection of Ang-1 and derivatives protein lysates were loaded on NuPage SDS-PAGE system (chambers and power supply) with 1.5mm ready-to-use Bis-Tris gels and 4-12% acrylamide gradient with MOPS-buffer as running aid (all Life Technologies, Grand Island, NY). Each lysate sample was prepared to 40ul final volume. This sample contained 25ug protein lysate in variable volume, RIPA buffer to make up volume to 26ul, 4ul of lOx reducing agent and 10µl 4x SDS loading buffer (both from Life Technologies, Grand Island, NY). Samples were heated at 95oC for 5min and loaded on the gel. Standard settings were chosen by the manufacturer, 200V, 120mA and max. 25 W. Run time was 60min, but no longer than running dye reaching the lower end of the gel.

After the run is terminated, the plastic case is cracked and the encased gel transferred to a ready-to-use nitrocellulose membrane kit and power supply (iBLOT; LifeTechnologies, Grand Island, NY). Using default settings, the protein lysate is transferred by high Ampere electricity from the gel to the membrane.

After the transfer, the membranes were incubated in 5% BSA in IX TBS for 15 minutes then in 5% BSA in IX TBS + 0.1 % Tween for another 15 minutes. Primary antibodies (Ang-1 ab183701 Abcam, Cambridge, UK) against human Ang-1 proteins are applied in 3ml of 5% BSA in IX TBS solution at a 1:5000 dilution overnight at 4°C and gentle agitation on an orbital shaker. Membranes are washed 3 times with IX TBS/0.1 % Tween, 5 minutes each time with gentle agitation. The secondary antibody (Goat anti-rabbit HRP conjugate; Abeam, Cambridge, MA) is conjugated to horse radish peroxidase and binds to the primary antibody antibodies. The secondary antibody is diluted of 1:10000 in 5% BSA in IX TBS and incubated for 1 hr at RT. At the end of incubation time, the membranes are washed 3 times with IX TBS/0.1 % Tween, 5 minutes each time with gentle agitation. The membranes are developed in 5ml Pierce WestPico Chemiluminescent Subtrate (Thermo Fisher, Rockford, IL) as directed. The Western Blot detects protein around the expected size of 70 kd for human Ang-1, and of 37kDa for COMP-Ang-1 and CMP-Ang1. For reference, recombinant human Ang-1 protein was purchased from R&D Systems (biotechne).

### Results

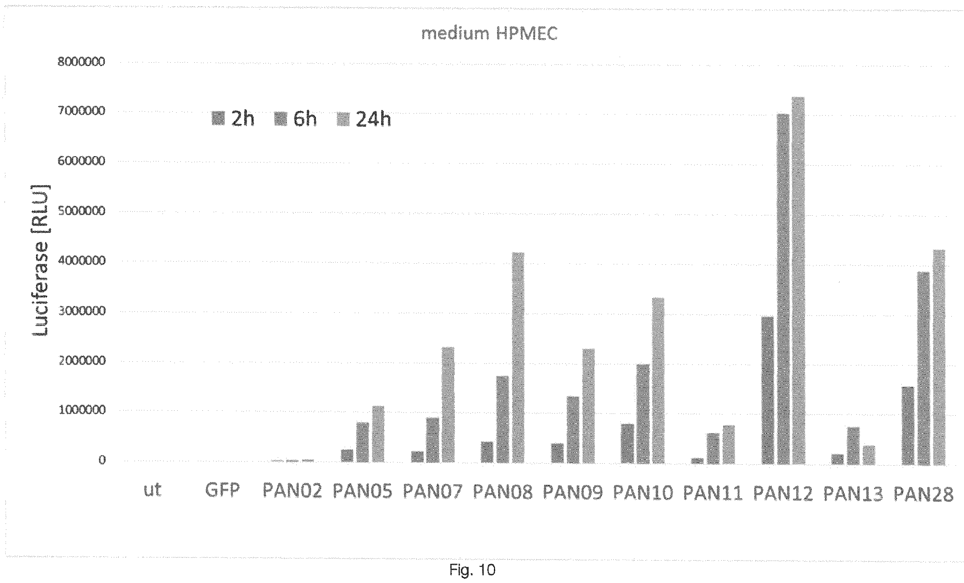

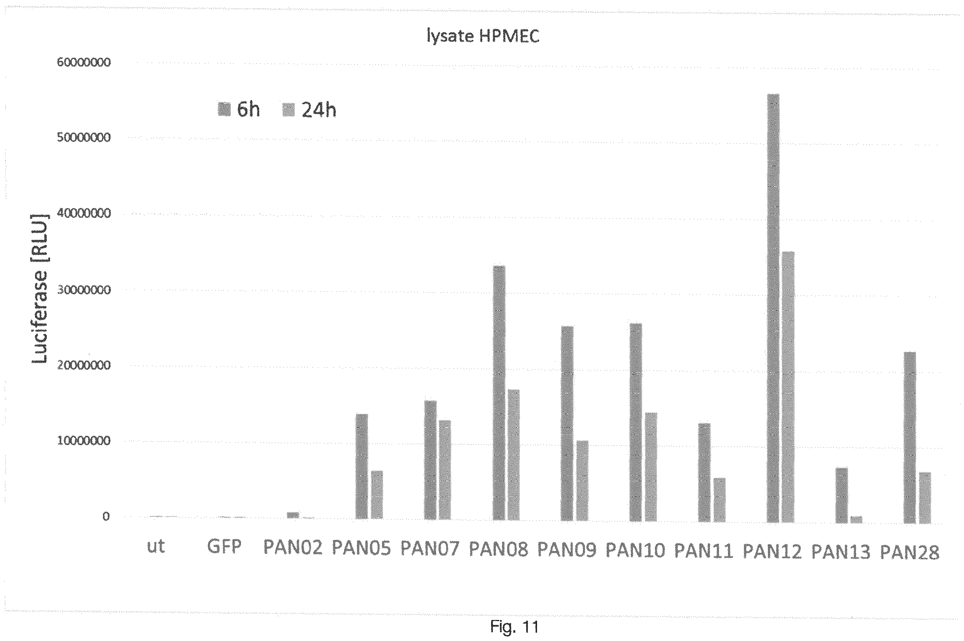

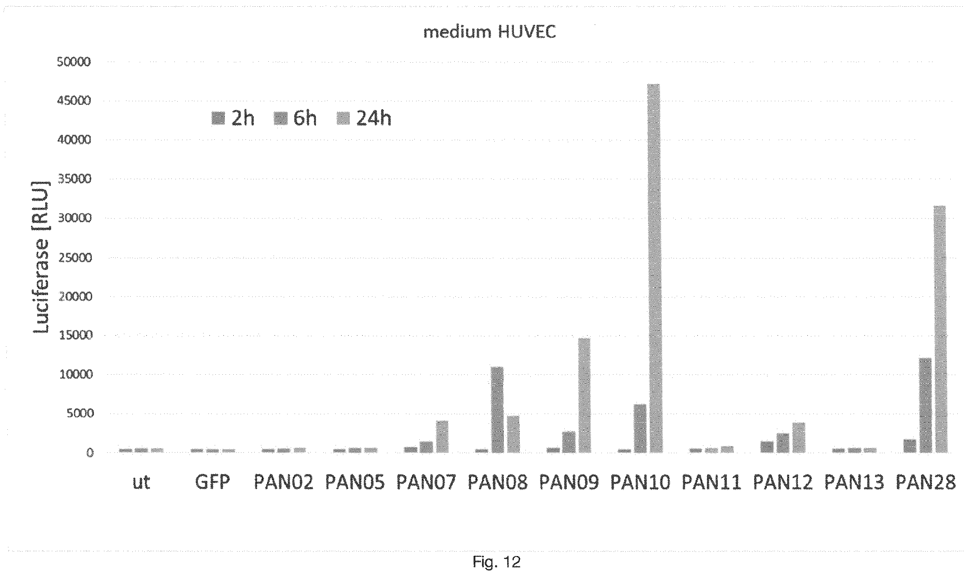

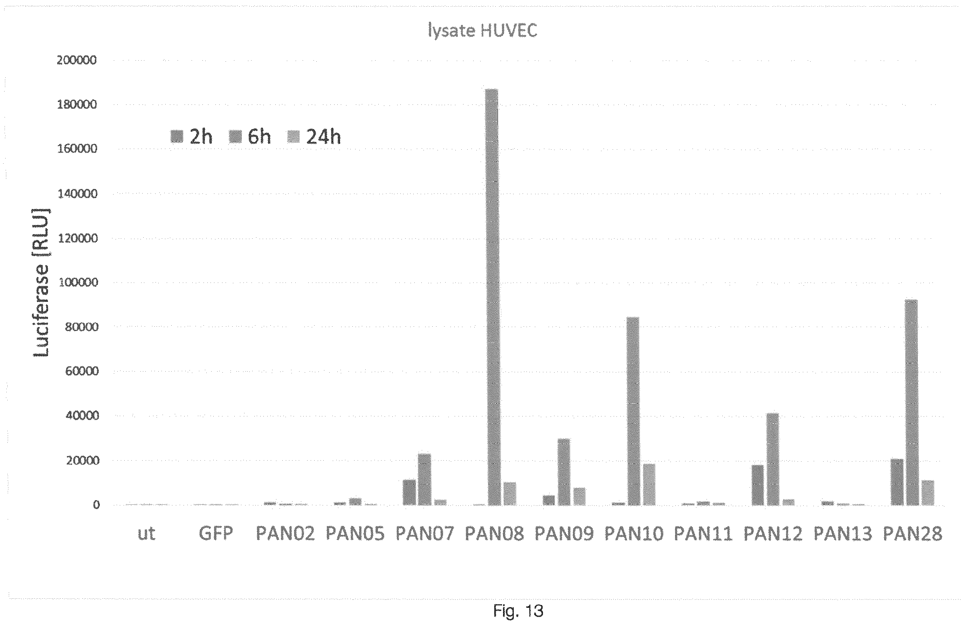

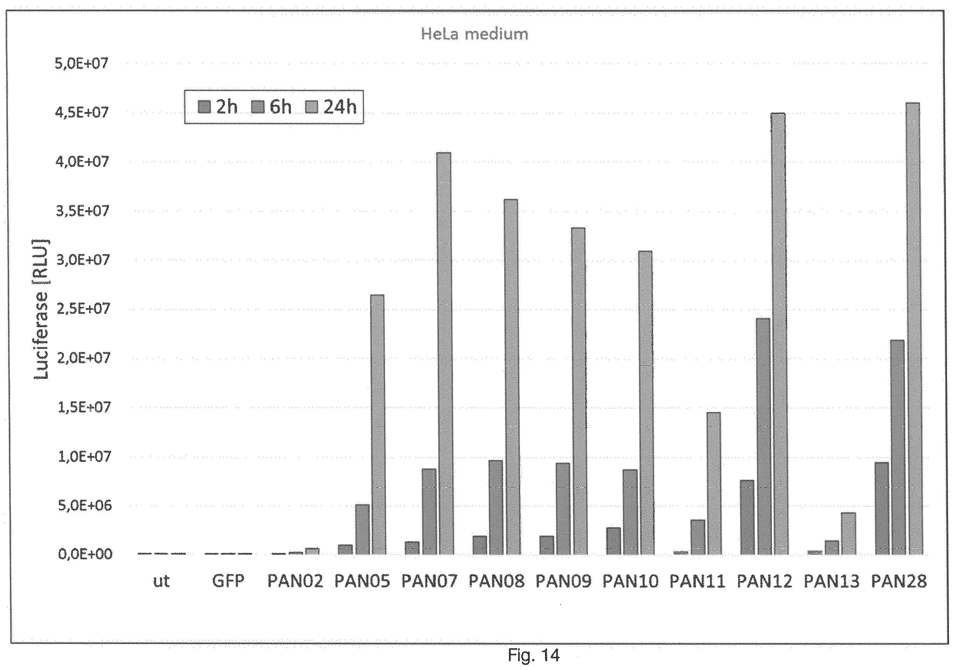

Protein expression data presented in the Figures 10 to 14, 72 to 85 and 92 to 94 underline that combinations of different regulatory nucleotide sequences flanking a coding region can significantly change the protein expression levels. Such change is further impacted by modification of the nucleotides forming the constructs as exemplified by replacing 100% of uridine and 100% cytidine nucleotides by pseudo-uridine and 5-methyl-cytidine (the constructs marked with "*" in Figure 74 and Figures 77 to 85. Changes are not only observed in a particular cell type but also across different cell types. For example, PAN02 (wild-type Ang-1 mRNA) shows the lowest Luciferase activity in all cell lines (Figures 10 to 14). All other constructs show enhanced protein expressions throughout all cell types. Therefore, the approach of using regulatory sequences from different genes can increase protein expression activity. To this end, replacing the endogenous Ang-1 sequences have shown superior effects on the protein expression of the reporter.

Worth noting, even the same regulatory sequences showed distinct protein expression activity in different cell types (Figures 75 to 81. These data further support our approach that the use of specific regulatory sequences can influence the activity of protein expression in a context-specific, i.e. cell-type specific cellular environment.

Furthermore, we have shown that our data are also concerning a different aspect i.e. secretion. For example, the regulatory sequences used in construct PAN12 show cell-type specific (HPMEC) expression in whole cell lysates but also in medium (supernatant) (Figures 10-14). This indicated that these sequences are not only optimal for protein expression (translation) but also secretion. In contrast, construct PAN28 shows expression in all cell types more or less equally. The regulatory sequences used in construct PAN29 (and PAN58), namely the 5'-UTR and the signal peptide sequence of MCP-1 in combination with the 3 '-UTR of vWF, were found to be particularly useful for highly efficient protein expression in human (microvascular) pulmonary endothelial cells (see e.g. Figures 72 and 75). The regulatory sequences used in construct PAN54, namely the 5'-UTR of spinach chloroplast RPL12, the signal peptide sequence of MCP-1 and the 3'-UTR of vWF, were found to be equally efficient for protein expression and secretion as shown in Figure 92. As shown in Fig. 98 the regulatory sequences of PAN29 are also functional using three different ORFs, namely wt Ang-1 in PAN52; hCOMP-Ang-1 in PAN53 and CMP-Ang-1 in PAN59 in directing expression in primary human pulmonary microendothelial cells (HPMEC). In contrast the mRNA construct containing the endogenous 5'-and 3'-UTRs of human Ang-1 (PAN01) is not translated (Fig. 98). Fig. 99 shows that all four mRNA constructs display comparable quality (integrity and purity).

### Example 3: Tie-2 pathway activation after transfection of modified mRNAs encoding the wildtype Tie-2 (PAN60) or the activating Tie-2 mutations (e.g. R849W (PAN61))

Modified mRNAs containing heterologous 5' and 3' UTRs are generated by in vitro transcription of PCR templates as described previously in example 1 above. Different concentration of this mRNA encoding the activating Tie-2 mutations (e.g. R849W) are transfected in different cell lines (HeLa, primary endothelial cells such as HUVECs or HPMEC or HPAEC) next to mRNAs encoding the wild-type Tie-2 using Lipofectamin MessengerMAX (Invitrogen, Carlsbad, CA; Thermo Fisher Scientific.). For qualitative Western blot cell lysates at different time points post transfection of mRNAs (1-24 h) are probed with anti-phosphotyrosine antibody (R&D systems) to evaluate Tie-2 phosphorylation (pTyr, 140 kD,). Blots are stripped and re-probed with anti-Tie-2 antibody (R&D systems) to detect total Tie-2. For quantitative measurement two different ELISAs are performed measuring human Tie-2 or Phospho-Tie-2 using the human Tie-2 DuoSet ELISA kit or human-Phospho-Tie-2 DuoSet IC ELISA according to the manufactures (e.g. both ELISA-Kits from R&D systems) protocol.

Transfection of Tie-2 mutation (PAN61, R849W) encoding mRNAs increase the presence of Phospho-Tie-2 in comparison to cell lysates derived from cells transfected with mRNAs encoding wildtype Tie-2 mRNA or in comparison to non-transfected cells. This increase in Tie-2 phosphorylation is observed in the presence or absence of co-stimulation with recombinant human Ang-1 ligand and can be observed in different cell lines. To confirm the activation of the Tie-2 signaling pathway and to demonstrate a biological relevant functional downstream signal transduction by expressing the Tie-2 (R849W) derivate a phosphorylation of Akt is demonstrated in a time course experiment. These experiments indicate that mRNAs encoding for the Tie-2 activating mutation (R849W) are functional and stronger in activating the Tie-2 signalling pathway than wt Tie-2 encoding mRNAs. These hyperactivation of the pathway can be demonstrated in the absence or presence of Tie-2 ligands (e.g. Ang-1) in human endothelial and/or non-endothelial cells.

### Example 4: mRNA formulation in cationic LNPs for in vivo applications by intravenous administration

For *in vivo* experiments mRNA-LNPs are prepared in a formulation process with β-(*L*-Arginyl)-*L*-2,3-diamino propionic acid-*N*-palmityl-*N*-oleyl-amide as cationic lipid. Alternatively, the cationic lipid *L*-Arginyl-β-alanine-*N*-palmityl-*N*-oleyl-amide can be used in an identical procedure to prepare mRNA-LNPs.

mRNA-LNP formulations are prepared using a modified procedure of a method described for siRNA (Chen, S., Tam, Y.Y., Lin, P.J., Sung, M.M., Tam, Y.K., and Cullis, P.R. (2016), Influence of particle size on the in vivo potency of lipid nanoparticle formulations of siRNA. J. Control. Release 235, 236-244. & (ii) patent application US20170121712).

Briefly, lipids are dissolved in ethanol at appropriate molar ratios (e.g. 50:49:1 β-(*L*-arginyl)-*L*-2,3-diamino propionic acid-*N*-palmityl-*N*-oleyl-amide: DPyPE: mPEG2000-DSPE). The lipid mixture is combined with an isotonic Sucrose solution of mRNA at a volume ratio of 2:1 (aqueous:ethanol) using a microfluidic mixer (NanoAssemblr®; Precision Nanosystems, Vancouver, BC) and flow rates of 18ml/min. Similarly, LNP formulations can be obtained using citrate or acetate buffered mRNA solutions (pH 3-4) and a slightly differing mixing ratio of 3:1 (v/v; aqueous:ethanol) and flow rates of 12ml/min.

After the mixing process, the formulations are dialyzed against 10mM HEPES or TRIS buffered isotonic Sucrose solution using 3.5 K MWCO Slide-A-Lyzer Dialysis Cassettes (Thermo Fisher Scientific) for at least 18 hours at 4°C. Instead of Sucrose, other sugars like Trehalose or Glucose can be equally used within the formulation process.

Subsequently, the formulations are tested for particle size (Zetasizer Nano ZS instrument (Malvern Instruments Ltd, Malvern, UK), RNA encapsulation (Quant-iT RiboGreen RNA Assay Kit following manufacturer's (Thermo Fisher Scientific) protocol), and endotoxin and are found to be between 30 to 100 nm in size with a Zeta-potential of >25mV, display greater than 90% mRNA encapsulation and < 1 EU/ml of endotoxin.

mRNA-LNP formulations are stored at -80 °C at a concentration of RNA of ∼ 0.3 µg/µl and an RNA to total lipid ratio of ∼ 0.03-0.05 (wt/wt) until further in vitro or in vivo use.

### Example 5: Material and methods - 2

### Plasmid Template generation by gene synthesis and cloning

Sequences encoding wt TIE-2 and sequences with the specified TIE-2 mutations in the coding region flanked by different heterologous 5' and 3' UTRs were designed and produced by gene synthesis by BioCat (Heidelberg) and cloned (Xho1-BamHI) into pcDNA3.1- (Thermo Fisher).

### In vitro transcription from PCR products to generate polyadenylated mRNAs

In vitro transcription is the synthesis of RNA transcripts by RNA polymerase from a linear DNA template containing the corresponding promoter sequence (T7, T3, SP6) and the gene to be transcribed. A typical transcription reaction consists of the template DNA, RNA polymerase, ribonucleotide triphosphates, RNase inhibitor and buffer containing Mg2+ ions. Linearized plasmid DNA, PCR products and synthetic DNA oligonucleotides can be used as templates for transcription as long as they have the T7 promoter sequence upstream of the gene to be transcribed. In order to generate DNA templates with a poly-(A) tail a tail-PCR using 5' primers containing T7 RNA polymerase sequences and 3' primers with a 120 nt Poly-T sequence are synthesized and purified (BioSpring, Frankfurt).

The Linear DNA templates for in vitro transcription are generated by PCR from linearized plasmid. Plasmids are digested with BamHI that cut once 3-terminal in the vector backbone to produce a linearized vector that can be used as the template for the poly-(A) tail PCR. 5µg plasmid are digested for 2h at 37oC with 5U BamHI restriction enzyme in 50µl and 1x HF Buffer. Small Aliquots of digested mix are analyzed by gel electrophoresis to check for complete digestion of the plasmid. The restriction enzyme is heat-inactivated by incubating at 80oC for 20 min and the digested plasmid is purified by a PCR purification column following the manufacturer's protocol (NucleoSpin Gel and PCR clean-up, Macherey-Nagel). The linearized plasmid can be stored at -20OC for several months and can be used for PCR template generation. The purpose of linearization is to eliminate circular templates that could potentially generate run-on transcripts during the IVT reaction.

Addition of poly-(A) tail by PCR is performed by using Hot StarHiFidelity polymerase (Qiagen) or Taq DNA Polymerase (Roche) following the manufacturer's protocol. For this purpose, Adapter primers containing T7 promoter sequences and 3' Poly A tail sequences (120nt) are used to amplify linear DNA-templates. Each of the adapter primers has overlap sequences which fuse regulatory sequences necessary for translation and transcription to the gene of interest (see Tab XX). A typical 50µl PCR reaction contains final concentrations of 200µM (of each) dNTP, 0,5µM for each primer, 50 ng linearized plasmid DNA, 1x PCR reaction buffer (1,5 mM MgCl2) and 1,25 U Taq DNA Polymerase per reaction (0,25µl of /5U/µl). The specific PCR cycle programs or thermal profiles are adjusted according to the Tm of the primer pairs, according to the expected length of the PCR product and according to the used thermal cycler. The quality of the PCR products are checked by analyzing an aliquot by gel electrophoresis and the reactions are purified by commercial PCR purification kits ((NucleoSpin Gel and PCR clean-up, Macherey-Nagel).

### In vitro transcription reaction

Addition of a 5' end cap structure to the RNA is an important process in eukaryotes. It is essential for RNA stability, efficient translation nuclear transport and splicing. The process involves addition of a 7-methylguanosine cap at the 5' triphosphate end of the RNA. RNA capping can be carried out post-transcriptionally using capping enzymes or co-transcriptionally using cap analogs such as ARCA (Jena Bioscience). In the enzymatic method, the mRNA may be capped using the Vaccinia virus capping enzyme 12,13. The enzyme adds on a 7-methylguanosine cap at the 5' end of the RNA using GTP and S-adenosyl methionine as donors (cap 0 structure). Both methods yield functionally active capped RNA suitable for transfection or other applications.

In the example 6 described below, the T7 High Yield Transcription Kit (Thermo Scientific) has been used to synthesize capped RNA transcripts (PAN 01-XX) using cap analogs co-transriptionally.

The DNA template for the transcription reaction is a linearized plasmid or a PCR product. For the capped RNA the reaction at room temperature is set up in the following order:

| Component | Volume (µl) | Final |
|---|---|---|
| Nuclease free water* | To 20 | |
| 5X Reaction Buffer | 4 | 1X |
| ATP (100 mM) | 2 | 10mM |
| CTP (100 mM) or 5-Methylcytidine-5'-Triphophate (100 mM) | 2 | 10mM |
| UTP (100 mM) or Pseudouridine-5'-Triphosphate (100 mM) or 5-Methoxyuridine-5'-Triphosphate (100 mM) | 2 | 10mM |
| GTP (30 mM) | 2 | 3mM |
| 3'-0-Me-m⁷G(5')ppp(5')G cap analog (ARCA; Anti Reverse Cap Analog) (100 mM) | 2 | 10mM |
| Template DNA* | X | 1µg |
| T7 RNA Polymerase Mix | 2 | |
| Total | 20 | |

Add 1 µg DNA and make up the total reaction volume to 20 µl with nuclease free water. The amount of water to be added will vary based on the concentration of the template DNA. Mix well by vortexing and incubate the reactions at 37 °C for 2 hours in a dry air incubator. The transcription reactions are treated with DNase I to remove the DNA template before proceeding with purification.
1. Add 70 µl nuclease free water to the transcription reactions followed by 10 µl of DNase I reaction buffer.
2. Add 2 µl DNase I to the reactions.
3. Incubate at 37 °C for 15 minutes.

### Column Purification of Capped mRNA

1. Purify the capped RNA using the MEGAclearKit as per the manufacturer's instructions (any spin column based RNA purification kit may be used).
2. Quantify the RNA using a NanoDrop Spectrophotometer.
3. As per the manufacturer's instructions, assess RNA sample quality using the Agilent RNA 6000 Nano Kit and Agilent 2100 Bioanalyzer or by native 1% agarose gelelectrophoresis and ethidium bromide staining.

### mRNA transfection of HeLa, HEK293, HUVEC and HPMEC cells

Primary Human Pulmonary Microvascular Endothelial Cells (HPMEC) are most appropriate for studying human lung diseases and are isolated from the lung from a single donor. Since lung tissue contains blood and lymphatic capillaries, HPMEC comprise Blood and Lymphatic Microvascular Endothelial Cells. The cells are routinely analyzed by immunofluorescent staining: they stain positive for CD31 and von Willebrand factor and negative for smooth muscle alpha-actin. HeLa cells, HUVEC, HPMEC cells were grown in DMEM complete medium or Endothelial Cell Growth Medium (PromoCell, Heidelberg Germany, CatNo.: C-22020) respectively. The Endothelial Cell Growth Medium is basal Medium supplemented with Fetal Calf Serum (0.05 ml/ml), Endothelial Cell Growth Supplement (bovine hypothalamic extract; 0.004 ml ml), Heparin 90µg/ml and Hydrocortisone 1µg/ml. The experiments with HUVEC and HPMEC cells are performed on cells with passage numbers below 8.

mRNA transfections are carried out using Lipofectamin MessengerMAX (Invitrogen, Carlsbad, CA; Thermo Fisher Scientific). For mRNA transfection in 24-well plate format 1x105 cells/well were seeded 24h before transfection to reach 70-90 confluence immediate before transfection. For one single well add 1.5µl Lipofectamine MessengerMAX Reagent to 25µl OpitMEM, vortex and incubate for 10 min at RT. In a second well add 500ng or 1µg mRNA to 25µl OptiMEM, vortex. After incubation add diluted mRNA to diluted Lipofectamin, mix well, incubate for 5 min at RT and add dropwise to cells in the presence of 0,5 ml of DMEM complete medium for HeLa. For HUVEC and HPMEC replace the Endothelial Cell Growth medium and wash the cells with OptiMEM and then add the Lipofectamin MessengerMAxX mRNA complex solution to the well with 0,5 ml OptiMEM prewarmed to 37 oC. Incubate cells at 37oC for 2h and replace the OptiMEM medium with complete Endothelial Cell Growth Medium (PromoCell, Heidelberg Germany) and analyze transfected cells or serum at the indicated time points. Alternatively mRNA-LNPs based on the cationic lipids L-Arginyl-β-alanine-N-palmityl-N-oleyl-amide or β-(L-Arginyl)-L-2,3-diamino propionic acid-N-palmityl-N-oleyl-amide in combination with neutral and PEGylated co-lipids can be used for in vitro transfection.

Successful introduction of mRNA into host cells can be monitored using various known methods, such as measuring the protein expression level of the target polypeptide by e.g., Western Blotting or immunocytochemistry or ELISA.

Fully modified mRNAs with 5-methylcytosine and/or pseudouridine (or 5-methoxyuridine) are transfected into HUVEC or HPMEC (Human Pulmonary Microvascular Endothelial Cells, PromoCell, Heidelberg) using Lipofectamin MessengerMAX (Invitrogen, Carlsbad, CA; Thermo Fisher Scientific). The cell lysates are harvested and run by ELISA at different time points (30", 1 h, 2h, 4h, 6h hours) after transfection to determine the protein expression.

For immunoblot detection of Tie-2 and derivatives protein lysates were loaded on NuPage SDS-PAGE system (chambers and power supply) with 1.5mm ready-to-use Bis-Tris gels and 4-12% acrylamide gradient with MOPS-buffer as running aid (all Life Technologies, Grand Island, NY). Each lysate sample was prepared to 40ul final volume. This sample contained 25ug protein lysate in variable volume, RIPA buffer to make up volume to 26ul, 4ul of lOx reducing agent and lOul 4x SDS loading buffer (both from Life Technologies, Grand Island, NY). Samples were heated at 95oC for 5min and loaded on the gel. Standard settings were chosen by the manufacturer, 200V, 120mA and max. 25 W. Run time was 60min, but no longer than running dye reaching the lower end of the gel.

After the run was terminated, the plastic case was cracked and the encased gel transferred to a ready-to-use nitrocellulose membrane kit and power supply (iBLOT; LifeTechnologies, Grand Island, NY). Using default settings, the protein lysate was transferred by high Ampere electricity from the gel to the membrane.

After the transfer, the membranes were incubated in 5% BSA in IX TBS for 15 minutes then in 5% BSA in IX TBS + 0.1 % Tween for another 15 minutes. Primary antibodies against human TIE-2 proteins or Phospho-specific Antibodies (P-TIE2, P-AKT, see below) were applied in 3ml of 5% BSA in IX TBS solution at a 1:500 to 1:2000 dilution for 3 hours at room temperature and gentle agitation on an orbital shaker. Membranes are washed 3 times with IX TBS/0.1 % Tween, 5 minutes each time with gentle agitation. The secondary antibody (Goat anti-rabbit HRP conjugate; Abeam, Cambridge, MA) was conjugated to horse radish peroxidase and binds to the primary antibody antibodies. The secondary antibody was diluted of 1:1000 to 1:5000 in 5% BSA in IX TBS and incubated for 3 hrs at RT. At the end of incubation time, the membranes were washed 3 times with IX TBS/0.1 % Tween, 5 minutes each time with gentle agitation. The membranes were developed in 5ml Pierce WestPico Chemiluminescent Subtrate (Thermo Fisher, Rockford, IL) as directed.

### Example 6: TIE-2 pathway activation after transfection of modified mRNAs encoding the wildtype TIE-2 (PAN60) or activating TIE-2 mutations (PAN61-65)

Different mRNA construct encoding for TIE-2 wt or activating TIE-2 point-mutation are prepared by in vitro transcription and transfected into mammalian cells including primary microvascular endothelial cells. TIE-2 expression and TIE-2 phosphorylation status are analyzed to demonstrate expression and activation. Examples for TIE-2 activation are, for example, increased Phopho-TIE-2, Phospho-Akt or downstream effector protein expression such as VE-cadherin.

Modified mRNAs containing heterologous 5' and 3' UTRs are generated by in vitro transcription of PCR templates as described previously in example 5. Different concentration of this mRNA encoding the activating TIE-2 mutations are transfected in different cell lines (HeLa, primary endothelial cells such as HUVECs or HPMEC) next to mRNAs encoding the wild-typeTIE-2 using Lipofectamin MessengerMAX (Invitrogen, Carlsbad, CA; Thermo Fisher Scientific.). For qualitative Western blot cell lysates at different time points post transfection of mRNAs (1-24 h) are probed with anti-phosphotyrosine antibody to evaluate TIE-2 phosphorylation (XXXX pTyr, 140 kD,). Blots are stripped and reprobed with anti-TIE-2 antibody to detect total TIE-2.

### Results

Interestingly transfection of TIE-2 mutations (R849W) encoding mRNAs do increase the presence of Phospho-TIE-2 in comparison to cell lysates derived from cells transfected with mRNAs encoding wildtype TIE-2 mRNA or in comparison to untransfected cells. This increase in TIE-2 phosphorylation is observed in the presence or absence of co-stimulation with recombinant human Ang-1 ligand and can be observed in different cell lines. To confirm the activation of the TIE-2 signaling pathway and to demonstrate a biological relevant functional downstream signal transduction by expressing the TIE-2 mutants derivate a phosphorylation of Akt is demonstrated in a time course experiment. These experiments indicate that mRNAs encoding for the TIE-2 activating mutations is functional and stronger in activating the TIE-2 signalling pathway than wt TIE-2 encoding mRNAs. These hyperactivation of the pathway is demonstrated in the absence or presence of TIE-2 agonist (e.g. Ang-1) in human endothelial and/or non-endothelial cells.

More specifically, Fig. 125 shows 1% agarose gels stained with EtBr showing PCR products of Poly-A tailing PCRs for the addition of 120 nt of Poly-A tail and mRNAs derived from these templates. Upper panel shows a quality control of PCR generated DNA templates of the indicated constructs (PAN 60 encoding for TIE2 wt, or for the mutated constructs PAN 61 encoding for TIE2 R849W, PAN 62 encoding for TIE2 L914F, PAN 63 encoding for TIE2 R849W L914F, PAN 64 encoding for Y897F R915L and PAN 65 encoding for TIE2 T1105N T1106P. Lower Panel show a quality control of IVT generated mRNA of the indicated constructs. 1µg of the DNA or mRNA were loaded on a non-denaturing 1% agarose gel containing EtBr, the electrophorese was performed at 120 V for 30 min and the nucleic acid was visualized under UV light.

Furthermore, as indicated in the immunoblots shown in Figs. 126 and 127 the constructs PAN62, PAN63 and PAN64, show a constitutive TIE-2 activation indicated by the phosphorylation of the TIE-2 receptor in the absence of Ang-1. Upper panel immunoblot on HeLa cell lysates 7h after mRNA transfection of the different constructs. PAN 51 serves as a negative control. Lower panel immunoblot on HeLa cells lysates transfected for 24 h with the indicated mRNA constructs and stimulated with recombinant ANG-1 for 15 or 30 min. A reduction of TIE-2 protein level in comparison to the wt (PAN60) is a result of the increased internalization and degradation of the TIE-2 receptor itself after the increased TIE-2 activation of the constutitve active mutants. Both experiments show a TIE-2 phosphorylation in the absence of TIE-2 agonist with mRNAs derived from constructs PAN62, PAN63 and PAN64.

The features of the present invention disclosed in the specification, the claims and/or the drawings may both separately and in any combination thereof be material for realizing the invention in various forms thereof.

## Claims

1. A recombinant nucleic acid construct comprising in 5'-> 3' direction
- a 5' UTR,
- a coding region coding for an effector molecule, and
- a 3' UTR,
wherein the 5' UTR is selected from the group consisting of a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for Ang-2 or a derivative thereof having a nucleotide identity of at least 85 %, a 5' UTR of a gene coding for HSP70 or a derivative thereof having a nucleotide identity of at least 85 %, a 5' UTR of a gene coding for H3.3. or a derivative thereof having a nucleotide identity of at least 85 %, a 5' UTR of a gene coding for Galectin-9 or a derivative thereof having a nucleotide identity of at least 85 %, a 5' UTR of a gene coding for GADD34 or a derivative thereof having a nucleotide identity of at least 85 %, a 5' UTR of a gene coding for EDN1 or a derivative thereof having a nucleotide identity of at least 85 %, a 5' UTR of a gene coding for HSP70m5 or a derivative thereof having a nucleotide identity of at least 85 %, a 5' UTR of a gene coding for E-selectin or a derivative thereof having a nucleotide identity of at least 85 % a 5' UTR of a gene coding for ICAM-1 or a derivative thereof having a nucleotide identity of at least 85 %, a 5' UTR of a gene coding for IL-6 or a derivative thereof having a nucleotide identity of at least 85 % and a 5' UTR of a gene coding for vWF or a derivative thereof having a nucleotide identity of at least 85 %;
wherein 3' UTR is selected from the group consisting of a 3' UTR of a gene coding for vWF or a derivative thereof having a nucleotide identity of at least 85 %, a 3' UTR of a gene coding for MCP-1 or a derivative thereof having a nucleotide identity of at least 85 %, , a 3' UTR of a gene coding for RPL12s.c. or a derivative thereof having a nucleotide identity of at least 85 %, a 3' UTR of a gene coding for HSP70 or a derivative thereof having a nucleotide identity of at least 85 %, a 3' UTR of a gene coding for H3.3. or a derivative thereof having a nucleotide identity of at least 85 %, a 3' UTR of a gene coding for GADD34 or a derivative thereof having a nucleotide identity of at least 85 %, a 3' UTR of a gene coding for EDN1 or a derivative thereof having a nucleotide identity of at least 85 %, and a 3' UTR of a gene coding for IL-6 or a derivative thereof having a nucleotide identity of at least 85 %,
wherein the effector molecule is effective in restoring a cellular function of a cell or is effective in exercising a therapeutic effect in or on a cell,
wherein the effector molecule is a mutant effector molecule, wherein the mutant effector molecule is a gain of function mutant effector molecule and/or a hypermorphic mutant effector molecule,
wherein the 5' UTR and the 3' UTR of the recombinant construct are of different origin, and wherein the recombinant nucleic acid construct is different from a wild type mRNA coding for the effector molecule.

2. The recombinant nucleic acid construct of claim 1, wherein the mutant effector molecule is a mutant Tie-2, preferably the mutant Tie-2 is selected from the group comprising Tie-2 L914 F, Tie-2 R849E L914F, Tie-2 Y897F R915L and Tie-2 T1105N T1106P.

3. The recombinant nucleic acid construct of claim 1, wherein the mutant effector molecule is a mutant PIK3, preferably the mutant PIK3CA is selected from the group comprising PIK3CA E542K, PIK2CA E545K and PIK3CA H1047R.

4. The recombinant nucleic acid construct of claim 1, wherein the mutant effector molecule is a mutant P110, preferably the mutant P110 is an engineered myristoylated form of the catalytic subunit p110alpha.

5. The recombinant nucleic acid construct of any one of claims 1 to 4, wherein
a) the 5' UTR is a 5' UTR of a gene coding for MCP-1 or a derivative thereof having a nucleotide identity of at least 85 %, and wherein the 3' UTR is selected from the group comprising a 3' UTR of a gene coding for vWF or a derivative thereof having a nucleotide identity of at least 85 %, a 3' UTR of a gene coding for HSP70 or a derivative thereof having a nucleotide identity of at least 85 %, a 3' UTR of a gene coding for RPL12s.c. or a derivative thereof having a nucleotide identity of at least 85 % and a 3' UTR of a gene coding for MCP-1 or a derivative thereof having a nucleotide identity of at least 85 %, or
b) the 5' UTR is a 5' UTR of a gene coding for RPL12s.c. or a derivative thereof having a nucleotide identity of at least 85 %, and wherein the 3' UTR is selected from the group comprising a 3' UTR of a gene coding for vWF or a derivative thereof having a nucleotide identity of at least 85 %, a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, and a 3' UTR of a gene coding for MCP-1 or a derivative thereof having a nucleotide identity of at least 85 %, or
c) the 5' UTR is a 5' UTR of a gene coding for HSP70 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL12s.c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %, or
d) the 5' UTR is a 5' UTR of a gene coding for ANG-2 or a derivative thereof having a nucleotide identity of at least 85 %, and wherein the 3' UTR is selected from the group comprising a 3' UTR of a gene coding for HSP70 or a derivative thereof having a nucleotide identity of at least 85 %, a 3' UTR of a gene coding for RPL 12s. c. or a derivative thereof having a nucleotide identity of at least 85 %, a 3' UTR of a gene coding for vWF or a derivative thereof having a nucleotide identity of at least 85 % and a 3' UTR of a gene coding for MCP-1 or a derivative thereof having a nucleotide identity of at least 85 %.

6. The recombinant nucleic acid construct of any one of claims 1 to 5, wherein the
a) 3'UTR is a 3' UTR of a gene coding for vWF or a derivative thereof having a nucleotide identity of at least 85 % and the 5' UTR is selected from the group comprising a 5' UTR of a gene coding for MCP-1 or a derivative thereof having a nucleotide identity of at least 85 %, a 5' UTR of a gene coding for RPL12s.c. or a derivative thereof having a nucleotide identity of at least 85 %, a 5' UTR of a gene coding for HSP70 or a derivative thereof having a nucleotide identity of at least 85 % and a 5' UTR of a gene coding for ANG-2 of a derivative thereof having a nucleotide identity of at least 85 %, or
b) 3'UTR is a 3' UTR of a gene coding for HSP70 or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %, or
c) 3'UTR is a 3' UTR of a gene coding for RPL12s.c. or a derivative thereof having a nucleotide identity of at least 85 % and the 5' UTR is selected from the group comprising a 5' UTR of a gene coding for HSP70 or a derivative thereof having a nucleotide identity of at least 85 % a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %, or
d) 3'UTR is a 3' UTR of a gene coding for MCP-1 or a derivative thereof having a nucleotide identity of at least 85 % and the 5' UTR is selected from the group comprising a 5' UTR of a gene coding for HSP70 or a derivative thereof having a nucleotide identity of at least 85 %, a 5' UTR of a gene coding for RPL12s.c. or a derivative thereof having a nucleotide identity of at least 85 %, a 5' UTR of a gene coding for ANG-2 of a derivative thereof having a nucleotide identity of at least 85 %, and a 5' UTR of a gene coding for MCP-1 or a derivative thereof having a nucleotide identity of at least 85 %.

7. The recombinant nucleic acid construct of any one of claims 1 to 6, wherein the construct is one selected from the group comprising
a construct, wherein the 5' UTR is a 5' UTR of a gene coding for MCP-1 or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for RPL12s.c. or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for MCP-1 or a derivative thereof having a nucleotide identity of at least 85 %, and the 3' UTR is a 3' UTR of a gene coding for MCP-1 or a derivative thereof having a nucleotide identity of at least 85 %,
a construct, wherein the 5' UTR is a 5' UTR of a gene coding for ANG-2 or a derivative thereof having a nucleotide identity of at least 85 %, and the 3' UTR is a 3' UTR of a gene coding for vWF or a derivative thereof having a nucleotide identity of at least 85 %,
a construct, wherein the 5' UTR is a 5' UTR of a gene coding for RPL12s.c. or a derivative thereof having a nucleotide identity of at least 85 %, and the 3' UTR is a 3' UTR of a gene coding for RPL12s.c. or a derivative thereof having a nucleotide identity of at least 85 %,
a construct, wherein the 5' UTR is a 5' UTR of a gene coding for HSP70 or a derivative thereof having a nucleotide identity of at least 85 %, and the 3' UTR is a 3' UTR of a gene coding for HSP70 or a derivative thereof having a nucleotide identity of at least 85 %,
a construct, wherein the 5' UTR is a 5' UTR of a gene coding for H3.3. or a derivative thereof having a nucleotide identity of at least 85 %, and the 3' UTR is a 3' UTR of a gene coding for H3.3. or a derivative thereof having a nucleotide identity of at least 85 %, and
a construct, wherein the 5' UTR is a 5' UTR of a gene coding for Galectin 9 or a derivative thereof having a nucleotide identity of at least 85 %, and the 3' UTR is a 3' UTR of a gene coding for Galectin 9 or a derivative thereof having a nucleotide identity of at least 85 %,
preferably the construct is
a construct, wherein the 5' UTR is a 5' UTR of a gene coding for MCP-1 or a derivative thereof having a nucleotide identity of at least 85 %, and the 3' UTR is a 3' UTR of a gene coding for vWF or a derivative thereof having a nucleotide identity of at least 85 %, or
a construct, wherein the 5' UTR is a 5' UTR of a gene coding for RPL12s.c. or a derivative thereof having a nucleotide identity of at least 85 %, and the 3' UTR is a 3' UTR of a gene coding for vWF or a derivative thereof having a nucleotide identity of at least 85 %.

8. The recombinant nucleic acid construct of any one of claims 1 to 7, wherein the construct comprises a poly-A tail, preferably at the 3' terminal end of the recombinant nucleic acid construct.

9. The recombinant nucleic acid construct of any one of claims 1 to 8, wherein the construct comprises a CAP structure, preferably at the 5' terminal end of the recombinant nucleic acid construct.

10. The recombinant nucleic acid construct of any one of claims 1 to 9, wherein the construct comprises a IRES (internal ribosomal entry site) sequence, preferably at the 5' terminal end of the recombinant nucleic acid construct.

11. The recombinant nucleic acid construct of any one of claims 1 to 10, wherein the construct comprises nucleic acid sequence coding for a signal peptide, preferably the signal peptide is in-frame with nucleic acid sequence coding for a signal peptide and is arranged between the 5' UTR and the coding region coding for an effector molecule.

12. The recombinant nucleic acid construct of claim 11, wherein the nucleotide sequence coding for a signal peptide is selected from the group comprising a nucleotide sequence coding for a signal peptide of MCP-1 or a derivative thereof having a nucleotide identity of at least 85 %, nucleotide sequence coding for a signal peptide of IL-6 or a derivative thereof having a nucleotide identity of at least 85 %,, a nucleotide sequence coding for a signal peptide of Ang-2 or a derivative thereof having a nucleotide identity of at least 85 %, and a nucleotide sequence coding for a signal peptide of Ang-1 or a derivative thereof having a nucleotide identity of at least 85 %.

13. The recombinant nucleic acid construct of any one of claims 1 to 12, wherein the cell a cellular function of which is restored and/or the cell in or on which a therapeutic effect is exercised is an endothelial cell, preferably a vascular endothelial cell, more preferably the vascular endothelial cell is a microvascular endothelial cell.

14. The recombinant nucleic acid construct of any one of claims 1 to 13, wherein the cellular function is one which can be restored by an effector molecule having anti-permeability effect of endothelial cells, an anti-vascular leakage effect, an anti-apoptotic effect of endothelial cells or an anti-inflammatory effect of endothelial cells or an anti-stress response effect, preferably the effect is linked to or associated with the TIE-2 signalling pathway, VEGF-receptor pathway, NOTCH signalling pathway, PI3-kinase pathway, eNOS signalling pathway, sirtuin-dependent metabolic and energy homeostasis pathway, oxidative stress pathway, shear stress response pathway, ET-1 signal transduction pathway, NO-mediated signal transduction pathway, and mechanochemical transduction pathway.

15. A vector comprising a nucleic acid construct of any one of claims 1 to 14.

16. A cell comprising a nucleic acid construct of any one of claims 1 to 14 and/or a vector of claim 15.

17. A delivery vehicle comprising a nucleic acid construct of any one of claims 1 to 14, wherein the delivery vehicle is a cationic lipid delivery particle, preferably the particle is a nanoparticle, more preferably the average size of the nanoparticle is from about 30 nm to about 200 nm, preferably from about 30 nm to about 140 nm and more preferably from about 30 nm to about 60 nm.

18. A pharmaceutical composition comprising a nucleic acid construct of any one of claims 1 to 14, a vector of claim 15, a cell of claim 16 and/or a delivery vehicle of claims 17, and a pharmaceutically acceptable diluent.

19. The recombinant nucleic acid construct of any one of claims 1 to 14, for use in a method for the treatment and/or prevention of a disease.

20. The recombinant nucleic acid construct of any one of claims 1 to 14, for use in a method for restoring a cellular function of a cell.


SEQUENCE LISTING

<110>  Pantherna Therapeutics GmbH

<120>  Recombniant nucleic acid construct and use thereof

<130>  P 10051 EP

<160>  153

<170>  PatentIn version 3.5

<210>  1
<211>  38
<212>  DNA
<213>  Artificial Sequence

<220>
<223>  Synthetic

<400>  1
taatacgact cactataggg ccctaagcca tcagcaat                              38


<210>  2
<211>  38
<212>  DNA
<213>  Artificial Sequence

<220>
<223>  Synthetic

<400>  2
taatacgact cactatagga aagtgattga ttcggata                              38


<210>  3
<211>  38
<212>  DNA
<213>  Artificial Sequence

<220>
<223>  Synthetic

<400>  3
taatacgact cactataggt ccccattgaa taacagcc                              38


<210>  4
<211>  38
<212>  DNA
<213>  Artificial Sequence

<220>
<223>  Synthetic

<400>  4
taatacgact cactatagga taaaagccca ggggcaag                              38


<210>  5
<211>  38
<212>  DNA

<213>  Artificial Sequence

<220>
<223>  Synthetic

<400>  5
taatacgact cactataggg tcagccatct ttcaattg                              38


<210>  6
<211>  38
<212>  DNA
<213>  Artificial Sequence

<220>
<223>  Synthetic

<400>  6
taatacgact cactataggc atatcttcat tcttcact                              38


<210>  7
<211>  38
<212>  DNA
<213>  Artificial Sequence

<220>
<223>  Synthetic

<400>  7
taatacgact cactatagga taaaagccta gtggccat                              38


<210>  8
<211>  38
<212>  DNA
<213>  Artificial Sequence

<220>
<223>  Synthetic

<400>  8
taatacgact cactataggg aggaaccgag aggctgag                              38


<210>  9
<211>  38
<212>  DNA
<213>  Artificial Sequence

<220>
<223>  Synthetic

<400>  9
taatacgact cactataggg gagctgttta cccccact                              38


<210>  10
<211>  38
<212>  DNA
<213>  Artificial Sequence

<220>

<223>  Synthetic

<400>  10
taatacgact cactatagga aagtgattga ttcggata                              38


<210>  11
<211>  139
<212>  DNA
<213>  Artificial Sequence

<220>
<223>  Synthetic

<400>  11
tttttttttt tttttttttt tttttttttt tttttttttt tttttttttt tttttttttt      60

tttttttttt tttttttttt tttttttttt tttttttttt tttttttttt tttttttttt     120

gccttttgca agataagag                                                  139


<210>  12
<211>  139
<212>  DNA
<213>  Artificial Sequence

<220>
<223>  Synthetic

<400>  12
tttttttttt tttttttttt tttttttttt tttttttttt tttttttttt tttttttttt      60

tttttttttt tttttttttt tttttttttt tttttttttt tttttttttt tttttttttt     120

gccttttgca agataagag                                                  139


<210>  13
<211>  139
<212>  DNA
<213>  Artificial Sequence

<220>
<223>  Synthetic

<400>  13
tttttttttt tttttttttt tttttttttt tttttttttt tttttttttt tttttttttt      60

tttttttttt tttttttttt tttttttttt tttttttttt tttttttttt tttttttttt     120

gaattttaaa gtttattta                                                  139


<210>  14
<211>  139
<212>  DNA
<213>  Artificial Sequence

<220>
<223>  Synthetic

<400>  14

tttttttttt tttttttttt tttttttttt tttttttttt tttttttttt tttttttttt 60

tttttttttt tttttttttt tttttttttt tttttttttt tttttttttt tttttttttt 120

atagtttaat gtattttaa 139


<210> 15
<211> 139
<212> DNA
<213> Artificial Sequence

<220>
<223> Synthetic

<400> 15
tttttttttt tttttttttt tttttttttt tttttttttt tttttttttt tttttttttt 60

tttttttttt tttttttttt tttttttttt tttttttttt tttttttttt tttttttttt 120

ttaactacat ctaaaccca 139


<210> 16
<211> 139
<212> DNA
<213> Artificial Sequence

<220>
<223> Synthetic

<400> 16
tttttttttt tttttttttt tttttttttt tttttttttt tttttttttt tttttttttt 60

tttttttttt tttttttttt tttttttttt tttttttttt tttttttttt tttttttttt 120

tcgctacaaa tcaaaaggc 139


<210> 17
<211> 139
<212> DNA
<213> Artificial Sequence

<220>
<223> Synthetic

<400> 17
tttttttttt tttttttttt tttttttttt tttttttttt tttttttttt tttttttttt 60

tttttttttt tttttttttt tttttttttt tttttttttt tttttttttt tttttttttt 120

tgtacaaaaa tatatttat 139


<210> 18
<211> 144
<212> DNA
<213> Artificial Sequence

<220>
<223> Synthetic

<400>  18
tttttttttt tttttttttt tttttttttt tttttttttt tttttttttt tttttttttt      60

tttttttttt tttttttttt tttttttttt tttttttttt tttttttttt tttttttttt     120

tttacagtaa ggaaaaaaat attt                                            144


<210>  19
<211>  139
<212>  DNA
<213>  Artificial Sequence

<220>
<223>  Synthetic

<400>  19
tttttttttt tttttttttt tttttttttt tttttttttt tttttttttt tttttttttt      60

tttttttttt tttttttttt tttttttttt tttttttttt tttttttttt tttttttttt     120

gccttttgca agataagag                                                  139


<210>  20
<211>  73
<212>  DNA
<213>  Homo sapiens


<220>
<221>  5'UTR
<222>  (1)..(73)

<400>  20
gaggaaccga gaggctgaga ctaacccaga aacatccaat tctcaaactg aagctcgcac      60

tctcgcctcc agc                                                         73


<210>  21
<211>  69
<212>  DNA
<213>  Homo sapiens


<220>
<221>  sig_peptide
<222>  (1)..(69)

<400>  21
atgaaagtct ctgccgccct tctgtgcctg ctgctcatag cagccacctt cattccccaa      60

gggctcgct                                                              69


<210>  22
<211>  23
<212>  PRT
<213>  Homo sapiens


<220>

<221>  SIGNAL
<222>  (1)..(23)

<400>  22

Met Lys Val Ser Ala Ala Leu Leu Cys Leu Leu Leu Ile Ala Ala Thr
1               5                   10                  15

Phe Ile Pro Gln Gly Leu Ala
            20

<210>  23
<211>  373
<212>  DNA
<213>  Homo sapiens

<220>
<221>  3'UTR
<222>  (1)..(373)

<400>  23
   acactcactc cacaacccaa gaatctgcag ctaacttatt ttcccctagc tttccccaga      60
   caccctgttt tattttatta taatgaattt tgtttgttga tgtgaaacat tatgccttaa     120
   gtaatgttaa ttcttattta agttattgat gttttaagtt tatctttcat ggtactagtg     180
   ttttttagat acagagactt ggggaaattg cttttcctct tgaaccacag ttctacccct     240
   gggatgtttt gagggtcttt gcaagaatca ttaatacaaa gaattttttt taacattcca     300
   atgcattgct aaaatattat tgtggaaatg aatattttgt aactattaca ccaaataaat     360
   atatttttgt aca                                                         373

<210>  24
<211>  94
<212>  DNA
<213>  Spinacia oleracea

<220>
<221>  5'UTR
<222>  (1)..(94)

<400>  24
catatcttca ttcttcactc tttccccccc cccccttatc ctcctatcta atcctctcat      60

ctctctcctc ctcttcctcc aacaacaact aaca                                 94

<210>  25
<211>  148
<212>  DNA
<213>  Spinacia oleracea

<220>
<221>  3'UTR

<222>  (1)..(148)

<400>  25
ttttgagtaa ttttcaattt tgtttttgtt tttgtttttg ttgttgggag ttgttgtaat      60

ttggtaaatg ttggggtcac taaaatcttt tgttgcaatt ttagcacctt tttattagat     120

atataaaatt gggtttagat gtagttaa                                        148


<210>  26
<211>  329
<212>  DNA
<213>  Homo sapiens


<220>
<221>  5'UTR
<222>  (1)..(329)

<400>  26
aaagtgattg attcggatac tgacactgta ggatctgggg agagaggaac aaaggaccgt      60

gaaagctgct ctgtaaaagc tgacacagcc ctcccaagtg agcaggactg ttcttcccac     120

tgcaatctga cagtttactg catgcctgga gagaacacag cagtaaaaac caggtttgct     180

actggaaaaa gaggaaagag aagactttca ttgacggacc cagccatggc agcgtagcag     240

ccctgcgttt tagacggcag cagctcggga ctctggacgt gtgtttgccc tcaagtttgc     300

taagctgctg gtttattact gaagaaaga                                       329


<210>  27
<211>  54
<212>  DNA
<213>  Homo sapiens


<220>
<221>  sig_peptide
<222>  (1)..(54)

<400>  27
atgtggcaga ttgttttctt tactctgagc tgtgatcttg tcttggccgc agcc            54


<210>  28
<211>  18
<212>  PRT
<213>  Homo sapiens


<220>
<221>  SIGNAL
<222>  (1)..(18)

<400>  28

Met Trp Gln Ile Val Phe Phe Thr Leu Ser Cys Asp Leu Val Leu Ala
1               5                   10                  15

Ala Ala

<210> 29
<211> 3450
<212> DNA
<213> Homo sapiens

<220>
<221> 3'UTR
<222> (1)..(3450)

<400> 29
acatcccagt ccacctgagg aactgtctcg aactattttc aaagacttaa gcccagtgca 60

ctgaaagtca cggctgcgca ctgtgtcctc ttccaccaca gagggcgtgt gctcggtgct 120

gacgggaccc acatgctcca gattagagcc tgtaaacttt atcacttaaa cttgcatcac 180

ttaacggacc aaagcaagac cctaaacatc cataattgtg attagacaga acacctatgc 240

aaagatgaac ccgaggctga gaatcagact gacagtttac agacgctgct gtcacaacca 300

agaatgttat gtgcaagttt atcagtaaat aactggaaaa cagaacactt atgttataca 360

atacagatca tcttggaact gcattcttct gagcactgtt tatacactgt gtaaataccc 420

atatgtcctg aattcaccat cactatcaca attaaaagga agaaaaaaac tctctaagcc 480

ataaaaagac atattcaggg atattctgag aaggggttac tagaagttta atatttggaa 540

aaacagttag tgcattttta ctccatctct taggtgcttt aaatttttat ttcaaaaaca 600

gcgtatttac atttatgttg acagcttagt tataagttaa tgctcaaata cgtatttcaa 660

atttatatgg tagaaacttc cagaatctct gaaattatca acagaaacgt gccattttag 720

tttatatgca gaccgtacta tttttttctg cctgattgtt aaatatgaag gtatttttag 780

taattaaata taacttatta ggggatatgc ctatgtttaa cttttatgat aatatttaca 840

attttataat ttgtttccaa aagacctaat tgtgccttgt gataaggaaa cttcttactt 900

ttaatgatga ggaaaattat acatttcatt ctatgacaaa gaaactttac tatcttctca 960

ctattctaaa acagaggtct gttttctttc ctagtaagat atatttttat agaactagac 1020

tacaatttaa tttctggttg agaaaagcct tctatttaag aaatttacaa agctatatgt 1080

ctcaagattc acccttaaat ttacttaagg aaaaaaataa ttgacactag taagtttttt 1140

tatgtcaatc agcaaactga aaaaaaaaaa agggtttcaa agtgcaaaaa caaaatctga 1200

tgttcataat atatttaaat atttaccaaa aatttgagaa cacaggctgg ggcgcagtgg 1260

ctcacaccta taatcccagt acattggtag gcaaggtggg cagatcacct gaggtcagga 1320

gttcaagacc agcctggaca acatggtgaa accctgtctc tactaaataa tacaaaaatt 1380

agccaggcgt gctggcgggc acctgtaatc ccagctactc gggaggctga ggcagggaga 1440

attgcttgca ccagggaggt agaggttgca gtgagccaag atcgcaccac tgcactccag 1500

ccggggcaac agagcaagac tccatctcaa aaaaaaaaaa aaaaaaagaa agaaaagaaa 1560

atttgagaac acagctttat actcgggact acaaaaccat aaactcctgg agttttaact 1620

ccttttgaaa ttttcatagt acaattaata ctaatgaaca tttgtgtaaa gctttataat 1680

ttaaaggcaa tttctcatat attcttttct gaatcatttg caaggaagtt cagagtccag 1740

tctgtaacta gcatctacta tatgtctgtc ttcaccttac agtgttctac cattattttt 1800

tctttattcc atttcaaaat ctaatttatt ttaccccaac ttctccccac cacttgacgt 1860

agttttagaa cacacaggtg ttgctacata tttggagtca atgatggact ctggcaaagt 1920

caaggctctg ttttatttcc accaaggtgc acttttccaa caactattta actagttaag 1980

aacctcccta tcttagaact gtatctactt tatatttaag aaggttttat gaattcaaca 2040

acggtatcat ggccttgtat caagttgaaa aacaactgaa aataagaaaa tttcacagcc 2100

tcgaaagaca acaacaagtt tctaggatat ctcaatgaca agagtgatgg atacttaggt 2160

agggaaacgc taatgcagga aaaactggca acaacacaat ttatatcaat tctctttgta 2220

ggcaggtgat aaaaaattca aggacaaatc tcattatgtc attgtgcatc atatataatc 2280

tcttatgagc gagaatgggg ggaatttgtg tttttacttt acacttcaat tccttacacg 2340

gtatttcaaa caaacagttt tgctgagagg agcttttgtc tctccttaag aaaatgttta 2400

taaagctgaa aggaaatcaa acagtaatct taaaaatgaa aacaaaacaa cccaacaacc 2460

tagataacta cagtgatcag ggagcacagt tcaactcctt gttatgtttt agtcatatgg 2520

cctactcaaa cagctaaata acaacaccag tggcagataa aaatcaccat ttatctttca 2580

gctattaatc ttttgaatga ataaactgtg acaaacaaat taacattttt gaacatgaaa 2640

ggcaacttct gcacaatcct gtatccaagc aaactttaaa ttatccactt aattattact 2700

taatcttaaa aaaaattaga acccagaact tttcaatgaa gcatttgaaa gttgaagtgg 2760

aatttaggaa agccataaaa atataaatac tgttatcaca gcaccagcaa gccataatct 2820

ttatacctat cagttctatt tctattaaca gtaaaaacat taagcaagat ataagactac 2880

ctgcccaaga attcagtctt ttttcatttt tgtttttctc agttctgagg atgttaatcg 2940

tcaaattttc tttggactgc attcctcact actttttgca caatggtctc acgttctcac 3000

atttgttctc gcgaataaat tgataaaagg tgttaagttc tgtgaatgtc tttttaatta 3060

tgggcataat tgtgcttgac tggataaaaa cttaagtcca cccttatgtt tataataatt 3120

tcttgagaac agcaaactgc atttaccatc gtaaaacaac atctgactta cgggagctgc 3180

agggaagtgg tgagacagtt cgaacggctc ctcagaaatc cagtgaccca attctaaaga 3240

ccatagcacc tgcaagtgac acaacaagca gatttattat acatttatta gccttagcag 3300

gcaataaacc aagaatcact ttgaagacac agcaaaaagt gatacactcc gcagatctga 3360

aatagatgtg ttctcagaca acaaagtccc ttcagaatct tcatgttgca taaatgttat 3420

gaatattaat aaaaagttga ttgagaaaaa 3450

<210> 30
<211> 121
<212> DNA
<213> Homo sapiens

<220>
<221> 5'UTR
<222> (1)..(121)

<400> 30
gtctcaatat tagagtctca acccccaata aatataggac tggagatgtc tgaggctcat 60

tctgccctcg agcccaccgg gaacgaaaga gaagctctat ctcccctcca ggagcccagc 120

t 121

<210> 31
<211> 87
<212> DNA
<213> Homo sapiens

<220>
<221> sig_peptide
<222> (1)..(87)

<400> 31
atgaactcct tctccacaag cgccttcggt ccagttgcct tctccctggg gctgctcctg 60

gtgttgcctg ctgccttccc tgcccca 87

<210> 32
<211> 29
<212> PRT
<213> Homo sapiens

<220>
<221> SIGNAL
<222> (1)..(29)

<400> 32

Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro
            20                  25

<210> 33
<211> 429

<212>  DNA
<213>  Homo sapiens

<220>
<221>  3'UTR
<222>  (1)..(429)

<400>  33
catgggcacc tcagattgtt gttgttaatg ggcattcctt cttctggtca gaaacctgtc      60

cactgggcac agaacttatg ttgttctcta tggagaacta aaagtatgag cgttaggaca     120

ctattttaat tatttttaat ttattaatat ttaaatatgt gaagctgagt taatttatgt     180

aagtcatatt tatattttta agaagtacca cttgaaacat tttatgtatt agttttgaaa     240

taataatgga aagtggctat gcagtttgaa tatcctttgt ttcagagcca gatcatttct     300

tggaaagtgt aggcttacct caaataaatg gctaacttat acatattttt aaagaaatat     360

ttatattgta tttatataat gtataaatgg tttttatacc aataaatggc attttaaaaa     420

attcagcaa                                                             429

<210>  34
<211>  255
<212>  DNA
<213>  Homo sapiens

<220>
<221>  5'UTR
<222>  (1)..(255)

<400>  34
gtggcagctc acagctattg tggtgggaaa gggagggtgg ttggtggatg tcacagcttg      60

ggctttatct cccccagcag tggggactcc acagcccctg ggctacataa cagcaagaca     120

gtccggagct gtagcagacc tgattgagcc tttgcagcag ctgagagcat ggcctagggt     180

gggcggcacc attgtccagc agctgagttt cccagggacc ttggagatag ccgcagccct     240

catttgcagg ggaag                                                      255

<210>  35
<211>  66
<212>  DNA
<213>  Homo sapiens

<220>
<221>  sig_peptide
<222>  (1)..(66)

<400>  35
atgattcctg ccagatttgc cggggtgctg cttgctctgg ccctcatttt gccagggacc      60

ctttgt                                                                 66

<210>  36
<211>  22
<212>  PRT
<213>  Homo sapiens

<220>
<221>  SIGNAL
<222>  (1)..(22)

<400>  36

Met Ile Pro Ala Arg Phe Ala Gly Val Leu Leu Ala Leu Ala Leu Ile
1               5                   10                  15

Leu Pro Gly Thr Leu Cys
            20

<210>  37
<211>  141
<212>  DNA
<213>  Homo sapiens

<220>
<221>  3'UTR
<222>  (1)..(141)

<400>  37
ggctgctgca gctgcatggg tgcctgctgc tgcctgcctt ggcctgatgg ccaggccaga      60

gtgctgccag tcctctgcat gttctgctct tgtgcccttc tgagcccaca ataaaggctg     120

agctcttatc ttgcaaaagg c                                               141

<210>  38
<211>  243
<212>  DNA
<213>  Homo sapiens

<220>
<221>  5'UTR
<222>  (1)..(243)

<400>  38
ataaaagccc aggggcaagc ggtccggata acggctagcc tgaggagctg ctgcgacagt      60

ccactacctt tttcgagagt gactcccgtt gtcccaaggc ttcccagagc gaacctgtgc     120

ggctgcaggc accggcgcgt cgagtttccg gcgtccggaa ggaccgagct cttctcgcgg     180

atccagtgtt ccgtttccag cccccaatct cagagcggag ccgacagaga gcagggaacc     240

ggc                                                                   243

<210>  39
<211>  258

<212>  DNA
<213>  Homo sapiens

<220>
<221>  3'UTR
<222>  (1)..(258)

<400>  39
gggcctttcc aagattgctg tttttgtttt ggagcttcaa gactttgcat ttcctagtat      60

ttctgtttgt cagttctcaa tttcctgtgt ttgcaatgtt gaaatttttt ggtgaagtac     120

tgaacttgct ttttttccgg tttctacatg cagagatgaa tttatactgc catcttacga     180

ctatttcttc tttttaatac acttaactca ggccattttt taagttggtt acttcaaagt     240

aaataaactt taaaattc                                                   258

<210>  40
<211>  261
<212>  DNA
<213>  Homo sapiens

<220>
<221>  5'UTR
<222>  (1)..(261)

<400>  40
gggctggggg agggtatata agccgagtag gcgacggtga ggtcgacgcc ggccaagaca      60

gcacagacag attgacctat tggggtgttt cgcgagtgtg agagggaagc gccgcggcct     120

gtatttctag acctgccctt cgcctggttc gtggcgcctt gtgaccccgg gcccctgccg     180

cctgcaagtc ggaaattgcg ctgtgctcct gtgctacggc ctgtggctgg actgcctgct     240

gctgcccaac tggctggcaa g                                               261

<210>  41
<211>  54
<212>  DNA
<213>  Homo sapiens

<220>
<221>  sig_peptide
<222>  (1)..(54)

<400>  41
atgaagctct ccctggtggc cgcgatgctg ctgctgctca gcgcggcgcg ggcc            54

<210>  42
<211>  18
<212>  PRT
<213>  Homo sapiens

<220>

<221>  SIGNAL
<222>  (1)..(18)

<400>  42

Met Lys Leu Ser Leu Val Ala Ala Met Leu Leu Leu Leu Ser Ala Ala
1               5                   10                  15


Arg Ala




<210>  43
<211>  1744
<212>  DNA
<213>  Homo sapiens


<220>
<221>  3'UTR
<222>  (1)..(1744)

<400>  43
acactgatct gctagtgctg taatattgta aatactggac tcaggaactt ttgttaggaa      60

aaaattgaaa gaacttaagt ctcgaatgta attggaatct tcacctcaga gtggagttga     120

aactgctata gcctaagcgg ctgtttactg cttttcatta gcagttgctc acatgtcttt     180

gggtgggggg gagaagaaga attggccatc ttaaaaagcg ggtaaaaaac ctgggttagg     240

gtgtgtgttc accttcaaaa tgttctattt aacaactggg tcatgtgcat ctggtgtagg     300

aagttttttc taccataagt gacaccaata aatgtttgtt atttacactg gtctaatgtt     360

tgtgagaagc ttctaattag atcaattact tattttagga aatttaagac tagatactcg     420

tgtgtggggt gaggggaggg agtatttggt atgttgggat aaggaaacac ttctatttaa     480

tgcttccagg gatttttttt ttttttttta accctcctgg gcccaagtga tccttccacc     540

tcagtctccc agctaattga gaccacaggc ttgttaccac catgctcggc ttttgcatta     600

atctaagaaa aggggagaga agttaatcca catctttact caggcaaggg gcatttcaca     660

gtgcccaaga gtggggtttt cttgaacata cttggtttcc tatttcccct tatctttcta     720

aaactgcctt tctggtggct ttttttaaaa ttattactaa tgatgctttt atagctgctt     780

ggattctctg agaaatgatg gggagtgagt gatcactggt attaacttta tacacttgga     840

tttcatttgt aactttagga tgtaaaggta tattgtgaac cctagctgtg tcagaatctc     900

catccctgaa atttctcatt agtggtactg gggtgggatc ttggatggtg acattgaaac     960

tacactaaat cccctcacta tgaatgggtt gttaaaggca atggtttgtg tcaaaactgg    1020

tttaggatta cttagattgt gttcctgaag aaaagagtcc aggtaaatgg tatgatcaat    1080

aaaggacagg ctggtgctaa cataaaatcc aatattgtaa tcctagcact ttgggaggcc    1140

aaggcgggtg gatcacaagg tcaagagata gagaccatct ttgccaacat ggtgaaactc    1200

catctctact gaaaatacaa aaattagctg ggcgtggtag tgcaagctga aggctgaggc 1260

aggagaatca ctcgaacccg ggaggcagag gttgcagtga gccgagatca caccactgta 1320

ctccagcccg gcactccagc ctggcgacaa gagtgagact ccacctcaaa aaaaaaaaaa 1380

agaatccaat actgcccaag gataggtatt ttatagatgg gcaactggct gaaaggttaa 1440

ttctctaggg ctagtagaac tggatcccaa caccaaactc ttaattagac ctaggcctca 1500

gctgcactgc ccgaaaagca tttgggcaga ccctgagcag aatactggtc tcaggccaag 1560

cccaatacag ccattaaaga tgacctacag tgctgtgtac cctggggcaa tagggttaaa 1620

tggtagttag caactagggc tagtcttccc ttacctcaaa ggctctcact accgtggacc 1680

acctagtctg taactctttc tgaggagctg ttactgaata ttaaaaagat agacttcaac 1740

tatg 1744

<210> 44
<211> 128
<212> DNA
<213> Homo sapiens

<220>
<221> 5'UTR
<222> (1)..(128)

<400> 44
gtcagccatc tttcaattgt gttcgcagcc gccgccgcgc cgccgtcgct ctccaacgcc 60

agcgccgcct ctcgctcgcc gagctccagc cgaaggagaa ggggggtaag taaggaggtc 120

tctgtacc 128

<210> 45
<211> 521
<212> DNA
<213> Homo sapiens

<220>
<221> 3'UTR
<222> (1)..(521)

<400> 45
gaatccacta tgatgggaaa catttcattc tcaaaaaaaa aaaaaaaaat ttctcttctt 60

cctgttattg gtagttctga acgttagata ttttttttcc atggggtcaa aaggtaccta 120

agtatatgat tgcgagtgga aaaatagggg acagaaatca ggtattggca gtttttccat 180

tttcatttgt gtgtgaattt ttaatataaa tgcggagacg taaagcatta atgcaagtta 240

aaatgtttca gtgaacaagt ttcagcggtt caactttata ataattataa ataaacctgt 300

taaatttttc tggacaatgc cagcatttgg atttttttaa aacaagtaaa tttcttattg 360

atggcaacta aatggtgttt gtagcatttt tatcatacag tagattccat ccattcacta 420

tacttttcta actgagttgt cctacatgca agtacatgtt tttaatgttg tctgtcttct 480

gtgctgttcc tgtaagtttg ctattaaaat acattaaact a 521

<210> 46
<211> 118
<212> DNA
<213> Homo sapiens

<220>
<221> 5'UTR
<222> (1)..(118)

<400> 46
tccccattga ataacagcca agttgctttg gtttctattt ctttgttaag tcgttccctc 60

tacaaaggac ttcctagtgg gtgtgaaagg cagcggtggc cacagaggcg gcggagag 118

<210> 47
<211> 578
<212> DNA
<213> Homo sapiens

<220>
<221> 3'UTR
<222> (1)..(578)

<400> 47
gcggcttcct ggccctgggg ccgggggctg gggtgtgggg cagtctgggt cctctcatca 60

tccccacttc ccaggcccag cctttccaac cctgcctggg atctgggctt taatgcagag 120

gccatgtcct tgtctggtcc tgcttctggc tacagccacc ctggaacgga gaaggcagct 180

gacggggatt gccttcctca gccgcagcag cacctggggc tccagctgct ggaatcctac 240

catcccagga ggcaggcaca gccagggaga ggggaggagt gggcagtgaa gatgaagccc 300

catgctcagt cccctcccat cccccacgca gctccacccc agtcccaagc caccagctgt 360

ctgctcctgg tgggaggtgg cctcctcagc ccctcctctc tgacctttaa cctcactctc 420

accttgcacc gtgcaccaac ccttcacccc tcctggaaag caggcctgat ggcttcccac 480

tggcctccac cacctgacca gagtgttctc ttcagaggac tggctccttt cccagtgtcc 540

ttaaaataaa gaaatgaaaa tgcttgttgg cacattca 578

<210> 48
<211> 4367
<212> DNA
<213> Artificial Sequence

<220>
<223> Synthetic

<400> 48
gccctaagcc atcagcaatc cttagtatag gggcacactc atgcattcct gtcaagtcat 60

cttgtgaaag gctgcctgct tccagcttgg cttggatgtg caaccttaat aaaactcact 120

gaggtctggg agaaaatagc agatctgcag cagatagggt agaggaaagg gtctagaata 180

tgtacacgca gctgactcag gcaggctcca tgctgaacgg tcacacagag aggaaacaat 240

aaatctcagc tactatgcaa taaatatctc aagttttaac gaagaaaaac atcattgcag 300

tgaaataaaa aattttaaaa ttttagaaca aagctaacaa atggctagtt ttctatgatt 360

cttcttcaaa cgctttcttt gagggggaaa gagtcaaaca aacaagcagt tttacctgaa 420

ataaagaact agttttagag gtcagaagaa aggagcaagt tttgcgagag gcacggaagg 480

agtgtgctgg cagtacaatg acagttttcc tttcctttgc tttcctcgct gccattctga 540

ctcacatagg gtgcagcaat cagcgccgaa gtccagaaaa cagtgggaga agatataacc 600

ggattcaaca tgggcaatgt gcctacactt tcattcttcc agaacacgat ggcaactgtc 660

gtgagagtac gacagaccag tacaacacaa acgctctgca gagagatgct ccacacgtgg 720

aaccggattt ctcttcccag aaacttcaac atctggaaca tgtgatggaa aattatactc 780

agtggctgca aaaacttgag aattacattg tggaaaacat gaagtcggag atggcccaga 840

tacagcagaa tgcagttcag aaccacacgg ctaccatgct ggagatagga accagcctcc 900

tctctcagac tgcagagcag accagaaagc tgacagatgt tgagacccag gtactaaatc 960

aaacttctcg acttgagata cagctgctgg agaattcatt atccacctac aagctagaga 1020

agcaacttct tcaacagaca aatgaaatct tgaagatcca tgaaaaaaac agtttattag 1080

aacataaaat cttagaaatg gaaggaaaac acaaggaaga gttggacacc ttaaaggaag 1140

agaaagagaa ccttcaaggc ttggttactc gtcaaacata tataatccag gagctggaaa 1200

agcaattaaa cagagctacc accaacaaca gtgtccttca gaagcagcaa ctggagctga 1260

tggacacagt ccacaacctt gtcaatcttt gcactaaaga aggtgtttta ctaaagggag 1320

gaaaaagaga ggaagagaaa ccatttagag actgtgcaga tgtatatcaa gctggtttta 1380

ataaaagtgg aatctacact atttatatta ataatatgcc agaacccaaa aaggtgtttt 1440

gcaatatgga tgtcaatggg ggaggttgga ctgtaataca acatcgtgaa gatggaagtc 1500

tagatttcca aagaggctgg aaggaatata aaatgggttt tggaaatccc tccggtgaat 1560

attggctggg gaatgagttt atttttgcca ttaccagtca gaggcagtac atgctaagaa 1620

ttgagttaat ggactgggaa gggaaccgag cctattcaca gtatgacaga ttccacatag 1680

gaaatgaaaa gcaaaactat aggttgtatt taaaaggtca cactgggaca gcaggaaaac 1740

agagcagcct gatcttacac ggtgctgatt tcagcactaa agatgctgat aatgacaact 1800

gtatgtgcaa atgtgccctc atgttaacag gaggatggtg gtttgatgct tgtggcccct 1860

ccaatctaaa tggaatgttc tatactgcgg gacaaaacca tggaaaactg aatgggataa 1920

agtggcacta cttcaaaggg cccagttact ccttacgttc cacaactatg atgattcgac 1980

ctttagattt ttgaaagcgc aatgtcagaa gcgattatga aagcaacaaa gaaatccgga 2040

gaagctgcca ggtgagaaac tgtttgaaaa cttcagaagc aaacaatatt gtctcccttc 2100

cagcaataag tggtagttat gtgaagtcac caaggttctt gaccgtgaat ctggagccgt 2160

ttgagttcac aagagtctct acttggggtg acagtgctca cgtggctcga ctatagaaaa 2220

ctccactgac tgtcgggctt taaaaaggga agaaactgct gagcttgctg tgcttcaaac 2280

tactactgga ccttattttg gaactatggt agccagatga taaatatggt taatttcatg 2340

taaaacagaa aaaaagagtg aaaaagagaa tatacatgaa gaatagaaac aagcctgcca 2400

taatcctttg gaaaagatgt attataccag tgaaaaggtg ttatatctat gcaaacctac 2460

taacaaatta tactgttgca caattttgat aaaaatttag aacagcattg tcctctgagt 2520

tggttaaatg ttaatggatt tcagaagcct aattccagta tcatacttac tagttgattt 2580

ctgcttaccc atcttcaaat gaaaattcca tttttgtaag ccataatgaa ctgtagtaca 2640

tggacaataa gtgtgtggta gaaacaaact ccattactct gatttttgat acagttttca 2700

gaaaaagaaa tgaacataat caagtaagga tgtatgtggt gaaaacttac cacccccata 2760

ctatggtttt catttactct aaaaactgat tgaatgatat ataaatatat ttatagcctg 2820

agtaaagtta aaagaatgta aaatatatca tcaagttctt aaaataatat acatgcattt 2880

aatatttcct ttgatattat acaggaaagc aatattttgg agtatgttaa gttgaagtaa 2940

aagcaagtac tctggagcag ttcattttac agtatctact tgcatgtgta tacatacatg 3000

taacttcatt attttaaaaa tatttttaga actccaatac tcaccctgtt atgtcttgct 3060

aatttaaatt ttgctaatta actgaaacat gcttaccaga ttcacactgt tccagtgtct 3120

ataaaagaaa cactttgaag tctataaaaa ataaaataat tataaatatc attgtacata 3180

gcatgtttat atctgcaaaa aacctaatag ctaattaatc tggaatatgc aacattgtcc 3240

ttaattgatg caaataacac aaatgctcaa agaaatctac tatatccctt aatgaaatac 3300

atcattcttc atatatttct ccttcagtcc attcccttag gcaattttta atttttaaaa 3360

attattatca ggggagaaaa attggcaaaa ctattatatg taagggaaat atatacaaaa 3420

agaaaattaa tcatagtcac ctgactaaga aattctgact gctagttgcc ataaataact 3480

caatggaaat attcctatgg gataatgtat tttaagtgaa tttttggggt gcttgaagtt 3540

actgcattat tttatcaaga agtcttctct gcctgtaagt gtccaaggtt atgacagtaa 3600

acagttttta ttaaaacatg agtcactatg ggatgagaaa attgaaataa agctactggg 3660

cctcctctca taaaagagac agttgttggc aaggtagcaa taccagtttc aaacttggtg 3720

acttgatcca ctatgcctta atggtttcct ccatttgaga aaataaagct attcacattg 3780

ttaagaaaaa tactttttaa agtttaccat caagtctttt ttatatttat gtgtctgtat 3840

tctacccctt tttgccttac aagtgatatt tgcaggtatt ataccatttt tctattcttg 3900

gtggcttctt catagcaggt aagcctctcc ttctaaaaac ttctcaactg ttttcattta 3960

agggaaagaa aatgagtatt ttgtcctttt gtgttcctac agacactttc ttaaaccagt 4020

ttttggataa agaatactat ttccaaactc atattacaaa aacaaaataa aataataaaa 4080

aaagaaagca tgatatttac tgttttgttg tctgggtttg agaaatgaaa tattgtttcc 4140

aattatttat aataaatcag tataaaatgt tttatgattg ttatgtgtat tatgtaatac 4200

gtacatgttt atggcaattt aacatgtgta ttcttttaat tgtttcagaa taggataatt 4260

aggtattcga attttgtctt taaaattcat gtggtttcta tgcaaagttc ttcatatcat 4320

cacaacatta tttgatttaa ataaaattga aagtaatatt tgtgcaa 4367

<210> 49
<211> 3782
<212> DNA
<213> Artificial Sequence

<220>
<223> Synthetic

<400> 49
gccctaagcc atcagcaatc cttagtatag gggcacactc atgcattcct gtcaagtcat 60

cttgtgaaag gctgcctgct tccagcttgg cttggatgtg caaccttaat aaaactcact 120

gaggtctggg agaaaatagc agatctgcag cagatagggt agaggaaagg gtctagaata 180

tgtacacgca gctgactcag gcaggctcca tgctgaacgg tcacacagag aggaaacaat 240

aaatctcagc tactatgcaa taaatatctc aagttttaac gaagaaaaac atcattgcag 300

tgaaataaaa aattttaaaa ttttagaaca aagctaacaa atggctagtt ttctatgatt 360

cttcttcaaa cgctttcttt gagggggaaa gagtcaaaca aacaagcagt tttacctgaa 420

ataaagaact agttttagag gtcagaagaa aggagcaagt tttgcgagag gcacggaagg 480

agtgtgctgg cagtacaatg acagttttcc tttcctttgc tttcctcgct gccattctga 540

ctgggatctt agacctagcc ccacagatgc ttcgagaact ccaggagact aatgcggcgc 600

tgcaagacgt gagagagctc ttgcgacagc aggtcaagga gatcaccttc ctgaagaata 660

cggtgatgga atgtgacgct tgcggaggct cccttgtcaa tctttgcact aaagaaggtg 720

ttttactaaa gggaggaaaa agagaggaag agaaaccatt tagagactgt gcagatgtat 780

atcaagctgg ttttaataaa agtggaatct acactattta tattaataat atgccagaac 840

ccaaaaaggt gttttgcaat atggatgtca atgggggagg ttggactgta atacaacatc 900

gtgaagatgg aagtctagat ttccaaagag gctggaagga atataaaatg ggttttggaa 960

atccctccgg tgaatattgg ctggggaatg agtttatttt tgccattacc agtcagaggc 1020

agtacatgct aagaattgag ttaatggact gggaagggaa ccgagcctat tcacagtatg 1080

acagattcca cataggaaat gaaaagcaaa actataggtt gtatttaaaa ggtcacactg 1140

ggacagcagg aaaacagagc agcctgatct tacacggtgc tgatttcagc actaaagatg 1200

ctgataatga caactgtatg tgcaaatgtg ccctcatgtt aacaggagga tggtggtttg 1260

atgcttgtgg cccctccaat ctaaatggaa tgttctatac tgcgggacaa aaccatggaa 1320

aactgaatgg gataaagtgg cactacttca aagggcccag ttactcctta cgttccacaa 1380

ctatgatgat tcgaccttta gatttttgaa agcgcaatgt cagaagcgat tatgaaagca 1440

acaaagaaat ccggagaagc tgccaggtga gaaactgttt gaaaacttca gaagcaaaca 1500

atattgtctc ccttccagca ataagtggta gttatgtgaa gtcaccaagg ttcttgaccg 1560

tgaatctgga gccgtttgag ttcacaagag tctctacttg gggtgacagt gctcacgtgg 1620

ctcgactata gaaaactcca ctgactgtcg ggctttaaaa agggaagaaa ctgctgagct 1680

tgctgtgctt caaactacta ctggacctta ttttggaact atggtagcca gatgataaat 1740

atggttaatt tcatgtaaaa cagaaaaaaa gagtgaaaaa gagaatatac atgaagaata 1800

gaaacaagcc tgccataatc ctttggaaaa gatgtattat accagtgaaa aggtgttata 1860

tctatgcaaa cctactaaca aattatactg ttgcacaatt ttgataaaaa tttagaacag 1920

cattgtcctc tgagttggtt aaatgttaat ggatttcaga agcctaattc cagtatcata 1980

cttactagtt gatttctgct tacccatctt caaatgaaaa ttccattttt gtaagccata 2040

atgaactgta gtacatggac aataagtgtg tggtagaaac aaactccatt actctgattt 2100

ttgatacagt tttcagaaaa agaaatgaac ataatcaagt aaggatgtat gtggtgaaaa 2160

cttaccaccc ccatactatg gttttcattt actctaaaaa ctgattgaat gatatataaa 2220

tatatttata gcctgagtaa agttaaaaga atgtaaaata tatcatcaag ttcttaaaat 2280

aatatacatg catttaatat ttcctttgat attatacagg aaagcaatat tttggagtat 2340

gttaagttga agtaaaagca agtactctgg agcagttcat tttacagtat ctacttgcat 2400

gtgtatacat acatgtaact tcattatttt aaaaatattt ttagaactcc aatactcacc 2460

ctgttatgtc ttgctaattt aaattttgct aattaactga aacatgctta ccagattcac 2520

actgttccag tgtctataaa agaaacactt tgaagtctat aaaaaataaa ataattataa 2580

atatcattgt acatagcatg tttatatctg caaaaaacct aatagctaat taatctggaa 2640

tatgcaacat tgtccttaat tgatgcaaat aacacaaatg ctcaaagaaa tctactatat 2700

cccttaatga aatacatcat tcttcatata tttctccttc agtccattcc cttaggcaat 2760

ttttaatttt taaaaattat tatcagggga gaaaaattgg caaaactatt atatgtaagg 2820

gaaatatata caaaaagaaa attaatcata gtcacctgac taagaaattc tgactgctag 2880

ttgccataaa taactcaatg gaaatattcc tatgggataa tgtattttaa gtgaattttt 2940

ggggtgcttg aagttactgc attattttat caagaagtct tctctgcctg taagtgtcca 3000

aggttatgac agtaaacagt ttttattaaa acatgagtca ctatgggatg agaaaattga 3060

aataaagcta ctgggcctcc tctcataaaa gagacagttg ttggcaaggt agcaatacca 3120

gtttcaaact tggtgacttg atccactatg ccttaatggt ttcctccatt tgagaaaata 3180

aagctattca cattgttaag aaaaatactt tttaaagttt accatcaagt cttttttata 3240

tttatgtgtc tgtattctac ccctttttgc cttacaagtg atatttgcag gtattatacc 3300

atttttctat tcttggtggc ttcttcatag caggtaagcc tctccttcta aaaacttctc 3360

aactgttttc atttaaggga aagaaaatga gtattttgtc cttttgtgtt cctacagaca 3420

ctttcttaaa ccagtttttg gataaagaat actatttcca aactcatatt acaaaaacaa 3480

aataaaataa taaaaaaaga aagcatgata tttactgttt tgttgtctgg gtttgagaaa 3540

tgaaatattg tttccaatta tttataataa atcagtataa aatgttttat gattgttatg 3600

tgtattatgt aatacgtaca tgtttatggc aatttaacat gtgtattctt ttaattgttt 3660

cagaatagga taattaggta ttcgaatttt gtctttaaaa ttcatgtggt ttctatgcaa 3720

agttcttcat atcatcacaa cattatttga tttaaataaa attgaaagta atatttgtgc 3780

aa 3782

<210> 50
<211> 1976
<212> DNA
<213> Artificial Sequence

<220>
<223> Synthetic

<400> 50
   aaagtgattg attcggatac tgacactgta ggatctgggg agagaggaac aaaggaccgt 60
   gaaagctgct ctgtaaaagc tgacacagcc ctcccaagtg agcaggactg ttcttcccac 120
   tgcaatctga cagtttactg catgcctgga gagaacacag cagtaaaaac caggtttgct 180
   actggaaaaa gaggaaagag aagactttca ttgacggacc cagccatggc agcgtagcag 240
   ccctgcgttt tagacggcag cagctcggga ctctggacgt gtgtttgccc tcaagtttgc 300
   taagctgctg gtttattact gaagaaagaa tgtggcagat tgttttcttt actctgagct 360
   gtgatcttgt cttggccgca gcccacatag ggtgcagcaa tcagcgccga agtccagaaa 420
   acagtgggag aagatataac cggattcaac atgggcaatg tgcctacact ttcattcttc 480
   cagaacacga tggcaactgt cgtgagagta cgacagacca gtacaacaca aacgctctgc 540
   agagagatgc tccacacgtg gaaccggatt tctcttccca gaaacttcaa catctggaac 600
   atgtgatgga aaattatact cagtggctgc aaaaacttga gaattacatt gtggaaaaca      660
   tgaagtcgga gatggcccag atacagcaga atgcagttca gaaccacacg gctaccatgc      720
   tggagatagg aaccagcctc ctctctcaga ctgcagagca gaccagaaag ctgacagatg      780
   ttgagaccca ggtactaaat caaacttctc gacttgagat acagctgctg gagaattcat      840
   tatccaccta caagctagag aagcaacttc ttcaacagac aaatgaaatc ttgaagatcc      900
   atgaaaaaaa cagtttatta gaacataaaa tcttagaaat ggaaggaaaa cacaaggaag      960
   agttggacac cttaaaggaa gagaaagaga accttcaagg cttggttact cgtcaaacat     1020
   atataatcca ggagctggaa aagcaattaa acagagctac caccaacaac agtgtccttc     1080
   agaagcagca actggagctg atggacacag tccacaacct tgtcaatctt tgcactaaag     1140
   aaggtgtttt actaaaggga ggaaaaagag aggaagagaa accatttaga gactgtgcag     1200
   atgtatatca agctggtttt aataaaagtg gaatctacac tatttatatt aataatatgc     1260
   cagaacccaa aaaggtgttt tgcaatatgg atgtcaatgg gggaggttgg actgtaatac     1320
   aacatcgtga agatggaagt ctagatttcc aaagaggctg gaaggaatat aaaatgggtt     1380
   ttggaaatcc ctccggtgaa tattggctgg ggaatgagtt tatttttgcc attaccagtc     1440
   agaggcagta catgctaaga attgagttaa tggactggga agggaaccga gcctattcac     1500
   agtatgacag attccacata ggaaatgaaa agcaaaacta taggttgtat ttaaaaggtc     1560
   acactgggac agcaggaaaa cagagcagcc tgatcttaca cggtgctgat ttcagcacta     1620
   aagatgctga taatgacaac tgtatgtgca aatgtgccct catgttaaca ggaggatggt     1680
   ggtttgatgc ttgtggcccc tccaatctaa atggaatgtt ctatactgcg ggacaaaacc     1740
   atggaaaact gaatgggata aagtggcact acttcaaagg gcccagttac tccttacgtt     1800
   ccacaactat gatgattcga cctttagatt tttgaggctg ctgcagctgc atgggtgcct     1860
   gctgctgcct gccttggcct gatggccagg ccagagtgct gccagtcctc tgcatgttct     1920
   gctcttgtgc ccttctgagc ccacaataaa ggctgagctc ttatcttgca aaaggc          1976

<210>  51
<211>  1391
<212>  DNA
<213>  Artificial Sequence

<220>
<223>  Synthetic

<400>  51
aaagtgattg attcggatac tgacactgta ggatctgggg agagaggaac aaaggaccgt       60

gaaagctgct ctgtaaaagc tgacacagcc ctcccaagtg agcaggactg ttcttcccac      120

tgcaatctga cagtttactg catgcctgga gagaacacag cagtaaaaac caggtttgct      180

actggaaaaa gaggaaagag aagactttca ttgacggacc cagccatggc agcgtagcag      240

ccctgcgttt tagacggcag cagctcggga ctctggacgt gtgtttgccc tcaagtttgc 300

taagctgctg gtttattact gaagaaagaa tgtggcagat tgttttcttt actctgagct 360

gtgatcttgt cttggccgca gccgggatct tagacctagc cccacagatg cttcgagaac 420

tccaggagac taatgcggcg ctgcaagacg tgagagagct cttgcgacag caggtcaagg 480

agatcacctt cctgaagaat acggtgatgg aatgtgacgc ttgcggaggc tcccttgtca 540

atctttgcac taaagaaggt gttttactaa agggaggaaa aagagaggaa gagaaaccat 600

ttagagactg tgcagatgta tatcaagctg gttttaataa aagtggaatc tacactattt 660

atattaataa tatgccagaa cccaaaaagg tgttttgcaa tatggatgtc aatgggggag 720

gttggactgt aatacaacat cgtgaagatg gaagtctaga tttccaaaga ggctggaagg 780

aatataaaat gggttttgga aatccctccg gtgaatattg gctggggaat gagtttattt 840

ttgccattac cagtcagagg cagtacatgc taagaattga gttaatggac tgggaaggga 900

accgagccta ttcacagtat gacagattcc acataggaaa tgaaaagcaa aactataggt 960

tgtatttaaa aggtcacact gggacagcag gaaaacagag cagcctgatc ttacacggtg 1020

ctgatttcag cactaaagat gctgataatg acaactgtat gtgcaaatgt gccctcatgt 1080

taacaggagg atggtggttt gatgcttgtg gcccctccaa tctaaatgga atgttctata 1140

ctgcgggaca aaaccatgga aaactgaatg ggataaagtg gcactacttc aaagggccca 1200

gttactcctt acgttccaca actatgatga ttcgaccttt agatttttga ggctgctgca 1260

gctgcatggg tgcctgctgc tgcctgcctt ggcctgatgg ccaggccaga gtgctgccag 1320

tcctctgcat gttctgctct tgtgcccttc tgagcccaca ataaaggctg agctcttatc 1380

ttgcaaaagg c 1391

<210> 52
<211> 1967
<212> DNA
<213> Artificial Sequence

<220>
<223> Synthetic

<400> 52
gaggaaccga gaggctgaga ctaacccaga aacatccaat tctcaaactg aagctcgcac 60

tctcgcctcc agcatgaaag tctctgccgc ccttctgtgc ctgctgctca tagcagccac 120

cttcattccc caagggctcg ctcacatagg gtgcagcaat cagcgccgaa gtccagaaaa 180

cagtgggaga agatataacc ggattcaaca tgggcaatgt gcctacactt tcattcttcc 240

agaacacgat ggcaactgtc gtgagagtac gacagaccag tacaacacaa acgctctgca 300

gagagatgct ccacacgtgg aaccggattt ctcttcccag aaacttcaac atctggaaca 360

tgtgatggaa aattatactc agtggctgca aaaacttgag aattacattg tggaaaacat 420

gaagtcggag atggcccaga tacagcagaa tgcagttcag aaccacacgg ctaccatgct 480

ggagatagga accagcctcc tctctcagac tgcagagcag accagaaagc tgacagatgt 540

tgagacccag gtactaaatc aaacttctcg acttgagata cagctgctgg agaattcatt 600

atccacctac aagctagaga agcaacttct tcaacagaca aatgaaatct tgaagatcca 660

tgaaaaaaac agtttattag aacataaaat cttagaaatg gaaggaaaac acaaggaaga 720

gttggacacc ttaaaggaag agaaagagaa ccttcaaggc ttggttactc gtcaaacata 780

tataatccag gagctggaaa agcaattaaa cagagctacc accaacaaca gtgtccttca 840

gaagcagcaa ctggagctga tggacacagt ccacaacctt gtcaatcttt gcactaaaga 900

aggtgtttta ctaaagggag gaaaaagaga ggaagagaaa ccatttagag actgtgcaga 960

tgtatatcaa gctggtttta ataaaagtgg aatctacact atttatatta ataatatgcc 1020

agaacccaaa aaggtgtttt gcaatatgga tgtcaatggg ggaggttgga ctgtaataca 1080

acatcgtgaa gatggaagtc tagatttcca aagaggctgg aaggaatata aaatgggttt 1140

tggaaatccc tccggtgaat attggctggg gaatgagttt atttttgcca ttaccagtca 1200

gaggcagtac atgctaagaa ttgagttaat ggactgggaa gggaaccgag cctattcaca 1260

gtatgacaga ttccacatag gaaatgaaaa gcaaaactat aggttgtatt taaaaggtca 1320

cactgggaca gcaggaaaac agagcagcct gatcttacac ggtgctgatt tcagcactaa 1380

agatgctgat aatgacaact gtatgtgcaa atgtgccctc atgttaacag gaggatggtg 1440

gtttgatgct tgtggcccct ccaatctaaa tggaatgttc tatactgcgg gacaaaacca 1500

tggaaaactg aatgggataa agtggcacta cttcaaaggg cccagttact ccttacgttc 1560

cacaactatg atgattcgac ctttagattt ttgaacactc actccacaac ccaagaatct 1620

gcagctaact tattttcccc tagctttccc cagacaccct gttttatttt attataatga 1680

attttgtttg ttgatgtgaa acattatgcc ttaagtaatg ttaattctta tttaagttat 1740

tgatgtttta agtttatctt tcatggtact agtgtttttt agatacagag acttggggaa 1800

attgcttttc ctcttgaacc acagttctac ccctgggatg ttttgagggt ctttgcaaga 1860

atcattaata caaagaattt tttttaacat tccaatgcat tgctaaaata ttattgtgga 1920

aatgaatatt ttgtaactat tacaccaaat aaatatattt ttgtaca 1967

<210> 53
<211> 1382
<212> DNA
<213> Artificial Sequence

<220>
<223> Synthetic

<400> 53
gaggaaccga gaggctgaga ctaacccaga aacatccaat tctcaaactg aagctcgcac 60

tctcgcctcc agcatgaaag tctctgccgc ccttctgtgc ctgctgctca tagcagccac 120

cttcattccc caagggctcg ctgggatctt agacttgggc ccgcagatgc ttcgggaact 180

gcaggaaacc aacgcggcgc tgcaggacgt gcgggagctg ctgcggcagc aggtcaggga 240

gatcacgttc ctgaaaaaca cggtgatgga gtgtgacgcg tgcggaggct cccttgtcaa 300

tctttgcact aaagaaggtg ttttactaaa gggaggaaaa agagaggaag agaaaccatt 360

tagagactgt gcagatgtat atcaagctgg ttttaataaa agtggaatct acactattta 420

tattaataat atgccagaac ccaaaaaggt gttttgcaat atggatgtca atgggggagg 480

ttggactgta atacaacatc gtgaagatgg aagtctagat ttccaaagag gctggaagga 540

atataaaatg ggttttggaa atccctccgg tgaatattgg ctggggaatg agtttatttt 600

tgccattacc agtcagaggc agtacatgct aagaattgag ttaatggact gggaagggaa 660

ccgagcctat tcacagtatg acagattcca cataggaaat gaaaagcaaa actataggtt 720

gtatttaaaa ggtcacactg ggacagcagg aaaacagagc agcctgatct tacacggtgc 780

tgatttcagc actaaagatg ctgataatga caactgtatg tgcaaatgtg ccctcatgtt 840

aacaggagga tggtggtttg atgcttgtgg cccctccaat ctaaatggaa tgttctatac 900

tgcgggacaa aaccatggaa aactgaatgg gataaagtgg cactacttca aagggcccag 960

ttactcctta cgttccacaa ctatgatgat tcgaccttta gatttttgaa cactcactcc 1020

acaacccaag aatctgcagc taacttattt tcccctagct ttccccagac accctgtttt 1080

attttattat aatgaatttt gtttgttgat gtgaaacatt atgccttaag taatgttaat 1140

tcttatttaa gttattgatg ttttaagttt atctttcatg gtactagtgt tttttagata 1200

cagagacttg gggaaattgc ttttcctctt gaaccacagt tctacccctg ggatgttttg 1260

agggtctttg caagaatcat taatacaaag aatttttttt aacattccaa tgcattgcta 1320

aaatattatt gtggaaatga atattttgta actattacac caaataaata tatttttgta 1380

ca 1382

<210> 54
<211> 3428
<212> DNA
<213> Artificial Sequence

<220>
<223> Synthetic

<400> 54
gccctaagcc atcagcaatc cttagtatag gggcacactc atgcattcct gtcaagtcat 60

cttgtgaaag gctgcctgct tccagcttgg cttggatgtg caaccttaat aaaactcact 120

gaggtctggg agaaaatagc agatctgcag cagatagggt agaggaaagg gtctagaata 180

tgtacacgca gctgactcag gcaggctcca tgctgaacgg tcacacagag aggaaacaat 240

aaatctcagc tactatgcaa taaatatctc aagttttaac gaagaaaaac atcattgcag 300

tgaaataaaa aattttaaaa ttttagaaca aagctaacaa atggctagtt ttctatgatt 360

cttcttcaaa cgctttcttt gagggggaaa gagtcaaaca aacaagcagt tttacctgaa 420

ataaagaact agttttagag gtcagaagaa aggagcaagt tttgcgagag gcacggaagg 480

agtgtgctgg cagtacaatg acagttttcc tttcctttgc tttcctcgct gccattctga 540

ctgtcttcac actcgaagat ttcgttgggg actggcgaca gacagccggc tacaacctgg 600

accaagtcct tgaacaggga ggtgtgtcca gtttgtttca gaatctcggg gtgtccgtaa 660

ctccgatcca aaggattgtc ctgagcggtg aaaatgggct gaagatcgac atccatgtca 720

tcatcccgta tgaaggtctg agcggcgacc aaatgggcca gatcgaaaaa atttttaagg 780

tggtgtaccc tgtggatgat catcacttta aggtgatcct gcactatggc acactggtaa 840

tcgacggggt tacgccgaac atgatcgact atttcggacg gccgtatgaa ggcatcgccg 900

tgttcgacgg caaaaagatc actgtaacag ggaccctgtg gaacggcaac aaaattatcg 960

acgagcgcct gatcaacccc gacggctccc tgctgttccg agtaaccatc aacggagtga 1020

ccggctggcg gctgtgcgaa cgcattctgg cgtaaaagcg caatgtcaga agcgattatg 1080

aaagcaacaa agaaatccgg agaagctgcc aggtgagaaa ctgtttgaaa acttcagaag 1140

caaacaatat tgtctccctt ccagcaataa gtggtagtta tgtgaagtca ccaaggttct 1200

tgaccgtgaa tctggagccg tttgagttca caagagtctc tacttggggt gacagtgctc 1260

acgtggctcg actatagaaa actccactga ctgtcgggct ttaaaaaggg aagaaactgc 1320

tgagcttgct gtgcttcaaa ctactactgg accttatttt ggaactatgg tagccagatg 1380

ataaatatgg ttaatttcat gtaaaacaga aaaaaagagt gaaaaagaga atatacatga 1440

agaatagaaa caagcctgcc ataatccttt ggaaaagatg tattatacca gtgaaaaggt 1500

gttatatcta tgcaaaccta ctaacaaatt atactgttgc acaattttga taaaaattta 1560

gaacagcatt gtcctctgag ttggttaaat gttaatggat ttcagaagcc taattccagt 1620

atcatactta ctagttgatt tctgcttacc catcttcaaa tgaaaattcc atttttgtaa 1680

gccataatga actgtagtac atggacaata agtgtgtggt agaaacaaac tccattactc 1740

tgatttttga tacagttttc agaaaaagaa atgaacataa tcaagtaagg atgtatgtgg 1800

tgaaaactta ccacccccat actatggttt tcatttactc taaaaactga ttgaatgata 1860

tataaatata tttatagcct gagtaaagtt aaaagaatgt aaaatatatc atcaagttct 1920

taaaataata tacatgcatt taatatttcc tttgatatta tacaggaaag caatattttg 1980

gagtatgtta agttgaagta aaagcaagta ctctggagca gttcatttta cagtatctac 2040

ttgcatgtgt atacatacat gtaacttcat tattttaaaa atatttttag aactccaata 2100

ctcaccctgt tatgtcttgc taatttaaat tttgctaatt aactgaaaca tgcttaccag 2160

attcacactg ttccagtgtc tataaaagaa acactttgaa gtctataaaa aataaaataa 2220

ttataaatat cattgtacat agcatgttta tatctgcaaa aaacctaata gctaattaat 2280

ctggaatatg caacattgtc cttaattgat gcaaataaca caaatgctca aagaaatcta 2340

ctatatccct taatgaaata catcattctt catatatttc tccttcagtc cattccctta 2400

ggcaattttt aatttttaaa aattattatc aggggagaaa aattggcaaa actattatat 2460

gtaagggaaa tatatacaaa aagaaaatta atcatagtca cctgactaag aaattctgac 2520

tgctagttgc cataaataac tcaatggaaa tattcctatg ggataatgta ttttaagtga 2580

atttttgggg tgcttgaagt tactgcatta ttttatcaag aagtcttctc tgcctgtaag 2640

tgtccaaggt tatgacagta aacagttttt attaaaacat gagtcactat gggatgagaa 2700

aattgaaata aagctactgg gcctcctctc ataaaagaga cagttgttgg caaggtagca 2760

ataccagttt caaacttggt gacttgatcc actatgcctt aatggtttcc tccatttgag 2820

aaaataaagc tattcacatt gttaagaaaa atacttttta aagtttacca tcaagtcttt 2880

tttatattta tgtgtctgta ttctacccct ttttgcctta caagtgatat ttgcaggtat 2940

tataccattt ttctattctt ggtggcttct tcatagcagg taagcctctc cttctaaaaa 3000

cttctcaact gttttcattt aagggaaaga aaatgagtat tttgtccttt tgtgttccta 3060

cagacacttt cttaaaccag tttttggata aagaatacta tttccaaact catattacaa 3120

aaacaaaata aaataataaa aaaagaaagc atgatattta ctgttttgtt gtctgggttt 3180

gagaaatgaa atattgtttc caattattta taataaatca gtataaaatg ttttatgatt 3240

gttatgtgta ttatgtaata cgtacatgtt tatggcaatt taacatgtgt attcttttaa 3300

ttgtttcaga ataggataat taggtattcg aattttgtct ttaaaattca tgtggtttct 3360

atgcaaagtt cttcatatca tcacaacatt atttgattta aataaaattg aaagtaatat 3420

ttgtgcaa 3428

<210> 55
<211> 1037
<212> DNA
<213> Artificial Sequence

<220>
<223> Synthetic

<400> 55
aaagtgattg attcggatac tgacactgta ggatctgggg agagaggaac aaaggaccgt 60

gaaagctgct ctgtaaaagc tgacacagcc ctcccaagtg agcaggactg ttcttcccac 120

tgcaatctga cagtttactg catgcctgga gagaacacag cagtaaaaac caggtttgct 180

actggaaaaa gaggaaagag aagactttca ttgacggacc cagccatggc agcgtagcag 240

ccctgcgttt tagacggcag cagctcggga ctctggacgt gtgtttgccc tcaagtttgc 300

taagctgctg gtttattact gaagaaagaa tgtggcagat tgttttcttt actctgagct 360

gtgatcttgt cttggccgca gccgtcttca cactcgaaga tttcgttggg gactggcgac 420

agacagccgg ctacaacctg gaccaagtcc ttgaacaggg aggtgtgtcc agtttgtttc 480

agaatctcgg ggtgtccgta actccgatcc aaaggattgt cctgagcggt gaaaatgggc 540

tgaagatcga catccatgtc atcatcccgt atgaaggtct gagcggcgac caaatgggcc 600

agatcgaaaa aatttttaag gtggtgtacc ctgtggatga tcatcacttt aaggtgatcc 660

tgcactatgg cacactggta atcgacgggg ttacgccgaa catgatcgac tatttcggac 720

ggccgtatga aggcatcgcc gtgttcgacg gcaaaaagat cactgtaaca gggaccctgt 780

ggaacggcaa caaaattatc gacgagcgcc tgatcaaccc cgacggctcc ctgctgttcc 840

gagtaaccat caacggagtg accggctggc ggctgtgcga acgcattctg gcgtaaggct 900

gctgcagctg catgggtgcc tgctgctgcc tgccttggcc tgatggccag gccagagtgc 960

tgccagtcct ctgcatgttc tgctcttgtg cccttctgag cccacaataa aggctgagct 1020

cttatcttgc aaaaggc 1037

<210> 56
<211> 826
<212> DNA
<213> Artificial Sequence

<220>
<223> Synthetic

<400> 56
tccccattga ataacagcca agttgctttg gtttctattt ctttgttaag tcgttccctc 60

tacaaaggac ttcctagtgg gtgtgaaagg cagcggtggc cacagaggcg gcggagagat 120

gtggcagatt gttttcttta ctctgagctg tgatcttgtc ttggccgcag ccgtcttcac 180

actcgaagat ttcgttgggg actggcgaca gacagccggc tacaacctgg accaagtcct 240

tgaacaggga ggtgtgtcca gtttgtttca gaatctcggg gtgtccgtaa ctccgatcca 300

aaggattgtc ctgagcggtg aaaatgggct gaagatcgac atccatgtca tcatcccgta 360

tgaaggtctg agcggcgacc aaatgggcca gatcgaaaaa atttttaagg tggtgtaccc 420

tgtggatgat catcacttta aggtgatcct gcactatggc acactggtaa tcgacggggt 480

tacgccgaac atgatcgact atttcggacg gccgtatgaa ggcatcgccg tgttcgacgg 540

caaaaagatc actgtaacag ggaccctgtg gaacggcaac aaaattatcg acgagcgcct 600

gatcaacccc gacggctccc tgctgttccg agtaaccatc aacggagtga ccggctggcg 660

gctgtgcgaa cgcattctgg cgtaaggctg ctgcagctgc atgggtgcct gctgctgcct 720

gccttggcct gatggccagg ccagagtgct gccagtcctc tgcatgttct gctcttgtgc 780

ccttctgagc ccacaataaa ggctgagctc ttatcttgca aaaggc 826

<210> 57
<211> 1068
<212> DNA
<213> Artificial Sequence

<220>
<223> Synthetic

<400> 57
ataaaagccc aggggcaagc ggtccggata acggctagcc tgaggagctg ctgcgacagt 60

ccactacctt tttcgagagt gactcccgtt gtcccaaggc ttcccagagc gaacctgtgc 120

ggctgcaggc accggcgcgt cgagtttccg gcgtccggaa ggaccgagct cttctcgcga 180

atccagtgtt ccgtttccag cccccaatct cagagcggag ccgacagaga gcagggaacc 240

ggcatgtggc agattgtttt ctttactctg agctgtgatc ttgtcttggc cgcagccgtc 300

ttcacactcg aagatttcgt tggggactgg cgacagacag ccggctacaa cctggaccaa 360

gtccttgaac agggaggtgt gtccagtttg tttcagaatc tcggggtgtc cgtaactccg 420

atccaaagga ttgtcctgag cggtgaaaat gggctgaaga tcgacatcca tgtcatcatc 480

ccgtatgaag gtctgagcgg cgaccaaatg ggccagatcg aaaaaatttt taaggtggtg 540

taccctgtgg atgatcatca ctttaaggtg atcctgcact atggcacact ggtaatcgac 600

ggggttacgc cgaacatgat cgactatttc ggacggccgt atgaaggcat cgccgtgttc 660

gacggcaaaa agatcactgt aacagggacc ctgtggaacg gcaacaaaat tatcgacgag 720

cgcctgatca accccgacgg ctccctgctg ttccgagtaa ccatcaacgg agtgaccggc 780

tggcggctgt gcgaacgcat tctggcgtaa gggcctttcc aagattgctg tttttgtttt 840

ggagcttcaa gactttgcat ttcctagtat ttctgtttgt cagttctcaa tttcctgtgt 900

ttgcaatgtt gaaatttttt ggtgaagtac tgaacttgct ttttttccgg tttctacatg 960

cagagatgaa tttatactgc catcttacga ctatttcttc tttttaatac acttaactca 1020

ggccattttt taagttggtt acttcaaagt aaataaactt taaaattc 1068

<210> 58
<211> 1217
<212> DNA
<213> Artificial Sequence

<220>
<223> Synthetic

<400> 58

gtcagccatc tttcaattgt gttcgcagcc gccgccgcgc cgccgtcgct ctccaacgcc 60

agcgccgcct ctcgctcgcc gagctccagc cgaaggagaa ggggggtaag taaggaggtc 120

tctgtaccat gtggcagatt gttttcttta ctctgagctg tgatcttgtc ttggccgcag 180

ccgtcttcac actcgaagat ttcgttgggg actggcgaca gacagccggc tacaacctgg 240

accaagtcct tgaacaggga ggtgtgtcca gtttgtttca gaatctcggg gtgtccgtaa 300

ctccgatcca aaggattgtc ctgagcggtg aaaatgggct gaagatcgac atccatgtca 360

tcatcccgta tgaaggtctg agcggcgacc aaatgggcca gatcgaaaaa atttttaagg 420

tggtgtaccc tgtggatgat catcacttta aggtgatcct gcactatggc acactggtaa 480

tcgacggggt tacgccgaac atgatcgact atttcggacg gccgtatgaa ggcatcgccg 540

tgttcgacgg caaaaagatc actgtaacag ggaccctgtg gaacggcaac aaaattatcg 600

acgagcgcct gatcaacccc gacggctccc tgctgttccg agtaaccatc aacggagtga 660

ccggctggcg gctgtgcgaa cgcattctgg cgtaagaatc cactatgatg ggaaacattt 720

cattctcaaa aaaaaaaaaa aaaatttctc ttcttcctgt tattggtagt tctgaacgtt 780

agatattttt tttccatggg gtcaaaaggt acctaagtat atgattgcga gtggaaaaat 840

aggggacaga aatcaggtat tggcagtttt tccattttca tttgtgtgtg aatttttaat 900

ataaatgcgg agacgtaaag cattaatgca agttaaaatg tttcagtgaa caagtttcag 960

cggttcaact ttataataat tataaataaa cctgttaaat ttttctggac aatgccagca 1020

tttggatttt tttaaaacaa gtaaatttct tattgatggc aactaaatgg tgtttgtagc 1080

atttttatca tacagtagat tccatccatt cactatactt ttctaactga gttgtcctac 1140

atgcaagtac atgtttttaa tgttgtctgt cttctgtgct gttcctgtaa gtttgctatt 1200

aaaatacatt aaactat 1217

<210> 59
<211> 827
<212> DNA
<213> Artificial Sequence

<220>
<223> Synthetic

<400> 59
catatcttca ttcttcactc tttccccccc cccccttatc ctcctatcta atcctctcat 60

ctctctcctc ctcttcctcc aacaacaact aacaatggca gcaactacaa caatggcaca 120

gattgttttc tttactctga gctgtgatct tgtcttggcc gcagccgtct tcacactcga 180

agatttcgtt ggggactggc gacagacagc cggctacaac ctggaccaag tccttgaaca 240

gggaggtgtg tccagtttgt ttcagaatct cggggtgtcc gtaactccga tccaaaggat 300

tgtcctgagc ggtgaaaatg ggctgaagat cgacatccat gtcatcatcc cgtatgaagg 360

tctgagcggc gaccaaatgg gccagatcga aaaaattttt aaggtggtgt accctgtgga 420

tgatcatcac tttaaggtga tcctgcacta tggcacactg gtaatcgacg gggttacgcc 480

gaacatgatc gactatttcg gacggccgta tgaaggcatc gccgtgttcg acggcaaaaa 540

gatcactgta acagggaccc tgtggaacgg caacaaaatt atcgacgagc gcctgatcaa 600

ccccgacggc tccctgctgt tccgagtaac catcaacgga gtgaccggct ggcggctgtg 660

cgaacgcatt ctggcgtaat tttgagtaat tttcaatttt gtttttgttt ttgtttttgt 720

tgttgggagt tgttgtaatt tggtaaatgt tggggtcact aaaatctttt gttgcaattt 780

tagcaccttt ttattagata tataaaattg ggtttagatg tagttaa 827

<210> 60
<211> 925
<212> DNA
<213> Artificial Sequence

<220>
<223> Synthetic

<400> 60
ataaaagcct agtggccatt gtgttcgttg ctcttatcgg ttcccatccc agttgttgat 60

cttatgcaag acgctgcacg accccgcgcc cgcttgtcgc cacggcactt gaggcagccg 120

gagatactct gagttactcg gagcccgacg cctgagggtg agatgaacgc gctggcctcc 180

ctaaccgtcc ggacctgtga tcgcttctgg cagaccgaac cggcgctcct gcccccgggg 240

tgacgcgcag ctcccagccg cccagacaca tgtggcagat tgttttcttt actctgagct 300

gtgatcttgt cttggccgca gccgtcttca cactcgaaga tttcgttggg gactggcgac 360

agacagccgg ctacaacctg gaccaagtcc ttgaacaggg aggtgtgtcc agtttgtttc 420

agaatctcgg ggtgtccgta actccgatcc aaaggattgt cctgagcggt gaaaatgggc 480

tgaagatcga catccatgtc atcatcccgt atgaaggtct gagcggcgac caaatgggcc 540

agatcgaaaa aatttttaag gtggtgtacc ctgtggatga tcatcacttt aaggtgatcc 600

tgcactatgg cacactggta atcgacgggg ttacgccgaa catgatcgac tatttcggac 660

ggccgtatga aggcatcgcc gtgttcgacg gcaaaaagat cactgtaaca gggaccctgt 720

ggaacggcaa caaaattatc gacgagcgcc tgatcaaccc cgacggctcc ctgctgttcc 780

gagtaaccat caacggagtg accggctggc ggctgtgcga acgcattctg gcgtaagacc 840

aactggtttg cctataattt attaactatt tatttttctt aagtgtgggt ttatataagg 900

aataaagcct tttgatttgt agcga 925

<210> 61
<211> 1028
<212> DNA

<213>  Artificial Sequence

<220>
<223>  Synthetic

<400>  61
gaggaaccga gaggctgaga ctaacccaga aacatccaat tctcaaactg aagctcgcac      60

tctcgcctcc agcatgaaag tctctgccgc ccttctgtgc ctgctgctca tagcagccac     120

cttcattccc caagggctcg ctgtcttcac actcgaagat ttcgttgggg actggcgaca     180

gacagccggc tacaacctgg accaagtcct tgaacaggga ggtgtgtcca gtttgtttca     240

gaatctcggg gtgtccgtaa ctccgatcca aaggattgtc ctgagcggtg aaaatgggct     300

gaagatcgac atccatgtca tcatcccgta tgaaggtctg agcggcgacc aaatgggcca     360

gatcgaaaaa atttttaagg tggtgtaccc tgtggatgat catcacttta aggtgatcct     420

gcactatggc acactggtaa tcgacggggt tacgccgaac atgatcgact atttcggacg     480

gccgtatgaa ggcatcgccg tgttcgacgg caaaaagatc actgtaacag ggaccctgtg     540

gaacggcaac aaaattatcg acgagcgcct gatcaacccc gacggctccc tgctgttccg     600

agtaaccatc aacggagtga ccggctggcg gctgtgcgaa cgcattctgg cgtaaacact     660

cactccacaa cccaagaatc tgcagctaac ttattttccc ctagctttcc ccagacaccc     720

tgttttattt tattataatg aattttgttt gttgatgtga aacattatgc cttaagtaat     780

gttaattctt atttaagtta ttgatgtttt aagtttatct ttcatggtac tagtgttttt     840

tagatacaga gacttgggga aattgctttt cctcttgaac cacagttcta cccctgggat     900

gttttgaggg tctttgcaag aatcattaat acaaagaatt ttttttaaca ttccaatgca     960

ttgctaaaat attattgtgg aaatgaatat tttgtaacta ttacaccaaa taaatatatt    1020

tttgtaca                                                             1028

<210>  62
<211>  2027
<212>  DNA
<213>  Artificial Sequence

<220>
<223>  Synthetic

<400>  62
ggagctgttt acccccactc taataggggt tcaatataaa aagccggcag agagctgtcc      60

aagtcagacg cgcctctgca tctgcgccag gcgaacgggt cctgcgcctc ctgcagtccc     120

agctctccac cgccgcgtgc gcctgcagac gctccgctcg ctgccttctc tcctggcagg     180

cgctgccttt tctccccgtt aaaagggcac ttgggctgaa ggatcgcttt gagatctgag     240

gaacccgcag cgctttgagg gacctgaagc tgtttttctt cgttttcctt tgggttcagt     300

ttgaacggga ggtttttgat cccttttttt cagaatggat tatttgctca tgattttctc     360

tctgctgttt gtggcttgcc aaggagtctt cacactcgaa gatttcgttg gggactggcg 420

acagacagcc ggctacaacc tggaccaagt ccttgaacag ggaggtgtgt ccagtttgtt 480

tcagaatctc ggggtgtccg taactccgat ccaaaggatt gtcctgagcg gtgaaaatgg 540

gctgaagatc gacatccatg tcatcatccc gtatgaaggt ctgagcggcg accaaatggg 600

ccagatcgaa aaaattttta aggtggtgta ccctgtggat gatcatcact ttaaggtgat 660

cctgcactat ggcacactgg taatcgacgg ggttacgccg aacatgatcg actatttcgg 720

acggccgtat gaaggcatcg ccgtgttcga cggcaaaaag atcactgtaa cagggaccct 780

gtggaacggc aacaaaatta tcgacgagcg cctgatcaac cccgacggct ccctgctgtt 840

ccgagtaacc atcaacggag tgaccggctg gcggctgtgc gaacgcattc tggcgtaaca 900

gaccttcggg gcctgtctga agccatagcc tccacggaga gccctgtggc cgactctgca 960

ctctccaccc tggctgggat cagagcagga gcatcctctg ctggttcctg actggcaaag 1020

gaccagcgtc ctcgttcaaa acattccaag aaaggttaag gagttccccc aaccatcttc 1080

actggcttcc atcagtggta actgctttgg tctcttcttt catctgggga tgacaatgga 1140

cctctcagca gaaacacaca gtcacattcg aattcgggtg gcatcctccg gagagagaga 1200

gaggaaggag attccacaca ggggtggagt ttctgacgaa ggtcctaagg gagtgtttgt 1260

gtctgactca ggcgcctggc acatttcagg gagaaactcc aaagtccaca caaagatttt 1320

ctaaggaatg cacaaattga aaacacactc aaaagacaaa catgcaagta aagaaaaaaa 1380

aaagaaagac ttttgtttaa atttgtaaaa tgcaaaactg aatgaaactg ttactaccat 1440

aaatcaggat atgtttcatg aatatgagtc tacctcacct atattgcact ctggcagaag 1500

tatttcccac atttaattat tgcctcccca aactcttccc acccctgctg ccccttcctc 1560

catcccccat actaaatcct agcctcgtag aagtctggtc taatgtgtca gcagtagata 1620

taatattttc atggtaatct actagctctg atccataaga aaaaaaagat cattaaatca 1680

ggagattccc tgtccttgat ttttggagac acaatggtat agggttgttt atgaaatata 1740

ttgaaaagta agtgtttgtt acgctttaaa gcagtaaaat tattttcctt tatataaccg 1800

gctaatgaaa gaggttggat tgaattttga tgtacttatt tttttataga tatttatatt 1860

caaacaattt attccttata tttaccatgt taaatatctg tttgggcagg ccatattggt 1920

ctatgtattt ttaaaatatg tatttctaaa tgaaattgag aacatgcttt gttttgcctg 1980

tcaaggtaat gactttagaa aataaatatt tttttcctta ctgtaaa 2027

<210> 63
<211> 1070
<212> DNA
<213> Artificial Sequence

<220>
<223>  Synthetic

<400>  63
aaagtgattg attcggatac tgacactgta ggatctgggg agagaggaac aaaggaccgt      60

gaaagctgct ctgtaaaagc tgacacagcc ctcccaagtg agcaggactg ttcttcccac     120

tgcaatctga cagtttactg catgcctgga gagaacacag cagtaaaaac caggtttgct     180

actggaaaaa gaggaaagag aagactttca ttgacggacc cagccatggc agcgtagcag     240

ccctgcgttt tagacggcag cagctcggga ctctggacgt gtgtttgccc tcaagtttgc     300

taagctgctg gtttattact gaagaaagaa tgaactcctt ctccacaagc gccttcggtc     360

cagttgcctt ctccctgggc ctgctcctgg tgttgcctgc tgccttccct gccccagtct     420

tcacactcga agatttcgtt ggggactggc gacagacagc cggctacaac ctggaccaag     480

tccttgaaca gggaggtgtg tccagtttgt ttcagaatct cggggtgtcc gtaactccga     540

tccaaaggat tgtcctgagc ggtgaaaatg ggctgaagat cgacatccat gtcatcatcc     600

cgtatgaagg tctgagcggc gaccaaatgg gccagatcga aaaaattttt aaggtggtgt     660

accctgtgga tgatcatcac tttaaggtga tcctgcacta tggcacactg gtaatcgacg     720

gggttacgcc gaacatgatc gactatttcg gacggccgta tgaaggcatc gccgtgttcg     780

acggcaaaaa gatcactgta acagggaccc tgtggaacgg caacaaaatt atcgacgagc     840

gcctgatcaa ccccgacggc tccctgctgt tccgagtaac catcaacgga gtgaccggct     900

ggcggctgtg cgaacgcatt ctggcgtaag gctgctgcag ctgcatgggt gcctgctgct     960

gcctgccttg gcctgatggc caggccagag tgctgccagt cctctgcatg ttctgctctt    1020

gtgcccttct gagcccacaa taaaggctga gctcttatct tgcaaaaggc              1070


<210>  64
<211>  796
<212>  DNA
<213>  Artificial Sequence

<220>
<223>  Synthetic

<400>  64
gaggaaccga gaggctgaga ctaacccaga aacatccaat tctcaaactg aagctcgcac      60

tctcgcctcc agcatgaaag tctctgccgc ccttctgtgc ctgctgctca tagcagccac     120

cttcattccc caaggctcgc ctgtcttcac actcgaagat ttcgttgggg actggcgaca     180

gacagccggc tacaacctgg accaagtcct tgaacaggga ggtgtgtcca gtttgtttca     240

gaatctcggg gtgtccgtaa ctccgatcca aaggattgtc ctgagcggtg aaaatgggct     300

gaagatcgac atccatgtca tcatcccgta tgaaggtctg agcggcgacc aaatgggcca     360

gatcgaaaaa atttttaagg tggtgtaccc tgtggatgat catcacttta aggtgatcct     420

gcactatggc acactggtaa tcgacggggt tacgccgaac atgatcgact atttcggacg 480

gccgtatgaa ggcatcgccg tgttcgacgg caaaaagatc actgtaacag ggaccctgtg 540

gaacggcaac aaaattatcg acgagcgcct gatcaacccc gacggctccc tgctgttccg 600

agtaaccatc aacggagtga ccggctggcg gctgtgcgaa cgcattctgg cgtaaggctg 660

ctgcagctgc atgggtgcct gctgctgcct gccttggcct gatggccagg ccagagtgct 720

gccagtcctc tgcatgttct gctcttgtgc ccttctgagc ccacaataaa ggctgagctc 780

ttatcttgca aaaggc 796

<210> 65
<211> 913
<212> DNA
<213> Artificial Sequence

<220>
<223> Synthetic

<400> 65
gaggaaccga gaggctgaga ctaacccaga aacatccaat tctcaaactg aagctcgcac 60

tctcgcctcc agcatgaaag tctctgccgc ccttctgtgc ctgctgctca tagcagccac 120

cttcattccc caagggctcg ctgtcttcac actcgaagat ttcgttgggg actggcgaca 180

gacagccggc tacaacctgg accaagtcct tgaacaggga ggtgtgtcca gtttgtttca 240

gaatctcggg gtgtccgtaa ctccgatcca aaggattgtc ctgagcggtg aaaatgggct 300

gaagatcgac atccatgtca tcatcccgta tgaaggtctg agcggcgacc aaatgggcca 360

gatcgaaaaa atttttaagg tggtgtaccc tgtggatgat catcacttta aggtgatcct 420

gcactatggc acactggtaa tcgacggggt tacgccgaac atgatcgact atttcggacg 480

gccgtatgaa ggcatcgccg tgttcgacgg caaaaagatc actgtaacag ggaccctgtg 540

gaacggcaac aaaattatcg acgagcgcct gatcaacccc gacggctccc tgctgttccg 600

agtaaccatc aacggagtga ccggctggcg gctgtgcgaa cgcattctgg cgtaagggcc 660

tttccaagat tgctgttttt gttttggagc ttcaagactt tgcatttcct agtatttctg 720

tttgtcagtt ctcaatttcc tgtgtttgca atgttgaaat tttttggtga agtactgaac 780

ttgctttttt tccggtttct acatgcagag atgaatttat actgccatct tacgactatt 840

tcttcttttt aatacactta actcaggcca ttttttaagt tggttacttc aaagtaaata 900

aactttaaaa ttc 913

<210> 66
<211> 1046
<212> DNA
<213> Artificial Sequence

<220>
<223>  Synthetic

<400>  66
gaggaaccga gaggctgaga ctaacccaga aacatccaat tctcaaactg aagctcgcac      60

tctcgcctcc agcatgaact ccttctccac aagcgccttc ggtccagttg ccttctccct     120

gggcctgctc ctggtgttgc ctgctgcctt ccctgcccca gtcttcacac tcgaagattt     180

cgttggggac tggcgacaga cagccggcta caacctggac caagtccttg aacagggagg     240

tgtgtccagt ttgtttcaga atctcggggt gtccgtaact ccgatccaaa ggattgtcct     300

gagcggtgaa aatgggctga agatcgacat ccatgtcatc atcccgtatg aaggtctgag     360

cggcgaccaa atgggccaga tcgaaaaaat ttttaaggtg gtgtaccctg tggatgatca     420

tcactttaag gtgatcctgc actatggcac actggtaatc gacggggtta cgccgaacat     480

gatcgactat ttcggacggc cgtatgaagg catcgccgtg ttcgacggca aaaagatcac     540

tgtaacaggg accctgtgga acggcaacaa aattatcgac gagcgcctga tcaaccccga     600

cggctccctg ctgttccgag taaccatcaa cggagtgacc ggctggcggc tgtgcgaacg     660

cattctggcg taaacactca ctccacaacc caagaatctg cagctaactt attttcccct     720

agctttcccc agacaccctg ttttatttta ttataatgaa ttttgtttgt tgatgtgaaa     780

cattatgcct taagtaatgt taattcttat ttaagttatt gatgttttaa gtttatcttt     840

catggtacta gtgtttttta gatacagaga cttggggaaa ttgcttttcc tcttgaacca     900

cagttctacc cctgggatgt tttgagggtc tttgcaagaa tcattaatac aaagaatttt     960

ttttaacatt ccaatgcatt gctaaaatat tattgtggaa atgaatattt tgtaactatt    1020

acaccaaata aatatatttt tgtaca                                         1046


<210>  67
<211>  1013
<212>  DNA
<213>  Artificial Sequence

<220>
<223>  Synthetic

<400>  67
gaggaaccga gaggctgaga ctaacccaga aacatccaat tctcaaactg aagctcgcac      60

tctcgcctcc agcatgtggc agattgtttt ctttactctg agctgtgatc ttgtcttggc     120

cgcagccgtc ttcacactcg aagatttcgt tggggactgg cgacagacag ccggctacaa     180

cctggaccaa gtccttgaac agggaggtgt gtccagtttg tttcagaatc tcggggtgtc     240

cgtaactccg atccaaagga ttgtcctgag cggtgaaaat gggctgaaga tcgacatcca     300

tgtcatcatc ccgtatgaag gtctgagcgg cgaccaaatg ggccagatcg aaaaaatttt     360

taaggtggtg taccctgtgg atgatcatca ctttaaggtg atcctgcact atggcacact     420

ggtaatcgac ggggttacgc cgaacatgat cgactatttc ggacggccgt atgaaggcat 480

cgccgtgttc gacggcaaaa agatcactgt aacagggacc ctgtggaacg gcaacaaaat 540

tatcgacgag cgcctgatca accccgacgg ctccctgctg ttccgagtaa ccatcaacgg 600

agtgaccggc tggcggctgt gcgaacgcat tctggcgtaa acactcactc cacaacccaa 660

gaatctgcag ctaacttatt ttcccctagc tttccccaga caccctgttt tattttatta 720

taatgaattt tgtttgttga tgtgaaacat tatgccttaa gtaatgttaa ttcttattta 780

agttattgat gttttaagtt tatctttcat ggtactagtg ttttttagat acagagactt 840

ggggaaattg cttttcctct tgaaccacag ttctacccct gggatgtttt gagggtcttt 900

gcaagaatca ttaatacaaa gaattttttt taacattcca atgcattgct aaaatattat 960

tgtggaaatg aatattttgt aactattaca ccaaataaat atatttttgt aca 1013

<210> 68
<211> 962
<212> DNA
<213> Artificial Sequence

<220>
<223> Synthetic

<400> 68
gaggaaccga gaggctgaga ctaacccaga aacatccaat tctcaaactg aagctcgcac 60

tctcgcctcc agcatggtct tcacactcga agatttcgtt ggggactggc gacagacagc 120

cggctacaac ctggaccaag tccttgaaca gggaggtgtg tccagtttgt ttcagaatct 180

cggggtgtcc gtaactccga tccaaaggat tgtcctgagc ggtgaaaatg ggctgaagat 240

cgacatccat gtcatcatcc cgtatgaagg tctgagcggc gaccaaatgg gccagatcga 300

aaaaattttt aaggtggtgt accctgtgga tgatcatcac tttaaggtga tcctgcacta 360

tggcacactg gtaatcgacg gggttacgcc gaacatgatc gactatttcg gacggccgta 420

tgaaggcatc gccgtgttcg acggcaaaaa gatcactgta acagggaccc tgtggaacgg 480

caacaaaatt atcgacgagc gcctgatcaa ccccgacggc tccctgctgt tccgagtaac 540

catcaacgga gtgaccggct ggcggctgtg cgaacgcatt ctggcgtaaa cactcactcc 600

acaacccaag aatctgcagc taacttattt tcccctagct ttccccagac accctgtttt 660

attttattat aatgaatttt gtttgttgat gtgaaacatt atgccttaag taatgttaat 720

tcttatttaa gttattgatg ttttaagttt atctttcatg gtactagtgt tttttagata 780

cagagacttg gggaaattgc ttttcctctt gaaccacagt tctacccctg ggatgttttg 840

agggtctttg caagaatcat taatacaaag aatttttttt aacattccaa tgcattgcta 900

aaatattatt gtggaaatga atattttgta actattacac caaataaata tatttttgta 960

ca                                                                   962

<210>  69
<211>  1198
<212>  DNA
<213>  Artificial Sequence

<220>
<223>  Synthetic

<400>  69
ataaaagccc aggggcaagc ggtccggata acggctagcc tgaggagctg ctgcgacagt      60

ccactacctt tttcgagagt gactcccgtt gtcccaaggc ttcccagagc gaacctgtgc     120

ggctgcaggc accggcgcgt cgagtttccg gcgtccggaa ggaccgagct cttctcgcga     180

atccagtgtt ccgtttccag cccccaatct cagagcggag ccgacagaga gcagggaacc     240

ggcatgaaag tctctgccgc ccttctgtgc ctgctgctca tagcagccac cttcattccc     300

caagggctcg ctgtcttcac actcgaagat ttcgttgggg actggcgaca gacagccggc     360

tacaacctgg accaagtcct tgaacaggga ggtgtgtcca gtttgtttca gaatctcggg     420

gtgtccgtaa ctccgatcca aaggattgtc ctgagcggtg aaaatgggct gaagatcgac     480

atccatgtca tcatcccgta tgaaggtctg agcggcgacc aaatgggcca gatcgaaaaa     540

atttttaagg tggtgtaccc tgtggatgat catcacttta aggtgatcct gcactatggc     600

acactggtaa tcgacggggt tacgccgaac atgatcgact atttcggacg gccgtatgaa     660

ggcatcgccg tgttcgacgg caaaaagatc actgtaacag ggaccctgtg gaacggcaac     720

aaaattatcg acgagcgcct gatcaacccc gacggctccc tgctgttccg agtaaccatc     780

aacggagtga ccggctggcg gctgtgcgaa cgcattctgg cgtaaacact cactccacaa     840

cccaagaatc tgcagctaac ttattttccc ctagctttcc ccagacaccc tgttttattt     900

tattataatg aattttgttt gttgatgtga aacattatgc cttaagtaat gttaattctt     960

atttaagtta ttgatgtttt aagtttatct ttcatggtac tagtgttttt tagatacaga    1020

gacttgggga aattgctttt cctcttgaac cacagttcta cccctgggat gttttgaggg    1080

tctttgcaag aatcattaat acaaagaatt ttttttaaca ttccaatgca ttgctaaaat    1140

attattgtgg aaatgaatat tttgtaacta ttacaccaaa taaatatatt tttgtaca      1198

<210>  70
<211>  1067
<212>  DNA
<213>  Artificial Sequence

<220>
<223>  Synthetic

<400>  70
catatcttca ttcttcactc tttccccccc cccccttatc ctcctatcta atcctctcat      60

ctctctcctc ctcttcctcc aacaacaact aacaatggca gcaactacaa caatgaaagt 120

ctctgccgcc cttctgtgcc tgctgctcat agcagccacc ttcattcccc aagggctcgc 180

tgtcttcaca ctcgaagatt tcgttgggga ctggcgacag acagccggct acaacctgga 240

ccaagtcctt gaacagggag gtgtgtccag tttgtttcag aatctcgggg tgtccgtaac 300

tccgatccaa aggattgtcc tgagcggtga aaatgggctg aagatcgaca tccatgtcat 360

catcccgtat gaaggtctga gcggcgacca aatgggccag atcgaaaaaa tttttaaggt 420

ggtgtaccct gtggatgatc atcactttaa ggtgatcctg cactatggca cactggtaat 480

cgacggggtt acgccgaaca tgatcgacta tttcggacgg ccgtatgaag gcatcgccgt 540

gttcgacggc aaaaagatca ctgtaacagg gaccctgtgg aacggcaaca aaattatcga 600

cgagcgcctg atcaaccccg acggctccct gctgttccga gtaaccatca acggagtgac 660

cggctggcgg ctgtgcgaac gcattctggc gtaaacactc actccacaac ccaagaatct 720

gcagctaact tattttcccc tagctttccc cagacaccct gttttatttt attataatga 780

attttgtttg ttgatgtgaa acattatgcc ttaagtaatg ttaattctta tttaagttat 840

tgatgtttta agtttatctt tcatggtact agtgtttttt agatacagag acttggggaa 900

attgcttttc ctcttgaacc acagttctac ccctgggatg ttttgagggt ctttgcaaga 960

atcattaata caaagaattt tttttaacat tccaatgcat tgctaaaata ttattgtgga 1020

aatgaatatt ttgtaactat tacaccaaat aaatatattt ttgtaca 1067

<210> 71
<211> 1052
<212> DNA
<213> Artificial Sequence

<220>
<223> Synthetic

<400> 71
aaagtgattg attcggatac tgacactgta ggatctgggg agagaggaac aaaggaccgt 60

gaaagctgct ctgtaaaagc tgacacagcc ctcccaagtg agcaggactg ttcttcccac 120

tgcaatctga cagtttactg catgcctgga gagaacacag cagtaaaaac caggtttgct 180

actggaaaaa gaggaaagag aagactttca ttgacggacc cagccatggc agcgtagcag 240

ccctgcgttt tagacggcag cagctcggga ctctggacgt gtgtttgccc tcaagtttgc 300

taagctgctg gtttattact gaagaaagaa tgaaagtctc tgccgccctt ctgtgcctgc 360

tgctcatagc agccaccttc attccccaag ggctcgctgt cttcacactc gaagatttcg 420

ttggggactg gcgacagaca gccggctaca acctggacca agtccttgaa cagggaggtg 480

tgtccagttt gtttcagaat ctcggggtgt ccgtaactcc gatccaaagg attgtcctga 540

gcggtgaaaa tgggctgaag atcgacatcc atgtcatcat cccgtatgaa ggtctgagcg 600

gcgaccaaat gggccagatc gaaaaaattt ttaaggtggt gtaccctgtg gatgatcatc 660

actttaaggt gatcctgcac tatggcacac tggtaatcga cggggttacg ccgaacatga 720

tcgactattt cggacggccg tatgaaggca tcgccgtgtt cgacggcaaa aagatcactg 780

taacagggac cctgtggaac ggcaacaaaa ttatcgacga gcgcctgatc aaccccgacg 840

gctccctgct gttccgagta accatcaacg gagtgaccgg ctggcggctg tgcgaacgca 900

ttctggcgta aggctgctgc agctgcatgg gtgcctgctg ctgcctgcct tggcctgatg 960

gccaggccag agtgctgcca gtcctctgca tgttctgctc ttgtgccctt ctgagcccac 1020

aataaaggct gagctcttat cttgcaaaag gc 1052

<210> 72
<211> 1064
<212> DNA
<213> Artificial Sequence

<220>
<223> Synthetic

<400> 72
aaagtgattg attcggatac tgacactgta ggatctgggg agagaggaac aaaggaccgt 60

gaaagctgct ctgtaaaagc tgacacagcc ctcccaagtg agcaggactg ttcttcccac 120

tgcaatctga cagtttactg ccctggagag aacacagcag taaaaaccag gtttgctact 180

ggaaaaagag gaaagagaag actttcattg acggacccag ccgcagcgta gcagccctgc 240

gttttagacg gcagcagctc gggactctgg acgtgtgttt gccctcaagt ttgctaagct 300

gctggtttat tactgaagaa agaatgaact ccttctccac aagcgccttc ggtccagttg 360

ccttctccct gggcctgctc ctggtgttgc ctgctgcctt ccctgcccca gtcttcacac 420

tcgaagattt cgttggggac tggcgacaga cagccggcta caacctggac caagtccttg 480

aacagggagg tgtgtccagt ttgtttcaga atctcggggt gtccgtaact ccgatccaaa 540

ggattgtcct gagcggtgaa aatgggctga agatcgacat ccatgtcatc atcccgtatg 600

aaggtctgag cggcgaccaa atgggccaga tcgaaaaaat ttttaaggtg gtgtaccctg 660

tggatgatca tcactttaag gtgatcctgc actatggcac actggtaatc gacggggtta 720

cgccgaacat gatcgactat ttcggacggc cgtatgaagg catcgccgtg ttcgacggca 780

aaaagatcac tgtaacaggg accctgtgga acggcaacaa aattatcgac gagcgcctga 840

tcaaccccga cggctccctg ctgttccgag taaccatcaa cggagtgacc ggctggcggc 900

tgtgcgaacg cattctggcg taaggctgct gcagctgcat gggtgcctgc tgctgcctgc 960

cttggcctga tggccaggcc agagtgctgc cagtcctctg catgttctgc tcttgtgccc 1020

ttctgagccc acaataaagg ctgagctctt atcttgcaaa aggc 1064

<210> 73
<211> 1034
<212> DNA
<213> Artificial Sequence

<220>
<223> Synthetic

<400> 73
   aaagtgattg attcggatac tgacactgta ggatctgggg agagaggaac aaaggaccgt 60
   gaaagctgct ctgtaaaagc tgacacagcc ctcccaagtg agcaggactg ttcttcccac 120
   tgcaatctga cagtttactg catgcctgga gagaacacag cagtaaaaac caggtttgct 180
   actggaaaaa gaggaaagag aagactttca ttgacggacc cagccatggc agcgtagcag 240
   ccctgcgttt tagacggcag cagctcggga ctctggacgt gtgtttgccc tcaagtttgc 300
   taagctgctg gtttattact gaagaaagaa tgggagtgaa agttcttttt gcccttattt 360
   gtattgctgt ggccgaggcc gtcttcacac tcgaagattt cgttggggac tggcgacaga 420
   cagccggcta caacctggac caagtccttg aacagggagg tgtgtccagt ttgtttcaga 480
   atctcggggt gtccgtaact ccgatccaaa ggattgtcct gagcggtgaa aatgggctga 540
   agatcgacat ccatgtcatc atcccgtatg aaggtctgag cggcgaccaa atgggccaga 600
   tcgaaaaaat ttttaaggtg gtgtaccctg tggatgatca tcactttaag gtgatcctgc 660
   actatggcac actggtaatc gacggggtta cgccgaacat gatcgactat ttcggacggc 720
   cgtatgaagg catcgccgtg ttcgacggca aaaagatcac tgtaacaggg accctgtgga 780
   acggcaacaa aattatcgac gagcgcctga tcaaccccga cggctccctg ctgttccgag 840
   taaccatcaa cggagtgacc ggctggcggc tgtgcgaacg cattctggcg taaggctgct 900
   gcagctgcat gggtgcctgc tgctgcctgc cttggcctga tggccaggcc agagtgctgc 960
   cagtcctctg catgttctgc tcttgtgccc ttctgagccc acaataaagg ctgagctctt 1020
   atcttgcaaa aggc 1034

<210> 74
<211> 842
<212> DNA
<213> Artificial Sequence

<220>
<223> Synthetic

<400> 74
catatcttca ttcttcactc tttccccccc cccccttatc ctcctatcta atcctctcat 60

ctctctcctc ctcttcctcc aacaacaact aacaatggca gcaactacaa caatgaaagt 120

ctctgccgcc cttctgtgcc tgctgctcat agcagccacc ttcattcccc aagggctcgc 180

tgtcttcaca ctcgaagatt tcgttgggga ctggcgacag acagccggct acaacctgga 240

ccaagtcctt gaacagggag gtgtgtccag tttgtttcag aatctcgggg tgtccgtaac 300

tccgatccaa aggattgtcc tgagcggtga aaatgggctg aagatcgaca tccatgtcat 360

catcccgtat gaaggtctga gcggcgacca aatgggccag atcgaaaaaa tttttaaggt 420

ggtgtaccct gtggatgatc atcactttaa ggtgatcctg cactatggca cactggtaat 480

cgacggggtt acgccgaaca tgatcgacta tttcggacgg ccgtatgaag gcatcgccgt 540

gttcgacggc aaaaagatca ctgtaacagg gaccctgtgg aacggcaaca aaattatcga 600

cgagcgcctg atcaaccccg acggctccct gctgttccga gtaaccatca acggagtgac 660

cggctggcgg ctgtgcgaac gcattctggc gtaattttga gtaattttca attttgtttt 720

tgtttttgtt tttgttgttg ggagttgttg taatttggta aatgttgggg tcactaaaat 780

cttttgttgc aattttagca cctttttatt agatatataa aattgggttt agatgtagtt 840

aa 842

<210> 75
<211> 824
<212> DNA
<213> Artificial Sequence

<220>
<223> Synthetic

<400> 75
catatcttca ttcttcactc tttccccccc cccccttatc ctcctatcta atcctctcat 60

ctctctcctc ctcttcctcc aacaacaact aacaatgaaa gtctctgccg cccttctgtg 120

cctgctgctc atagcagcca ccttcattcc ccaagggctc gctgtcttca cactcgaaga 180

tttcgttggg gactggcgac agacagccgg ctacaacctg gaccaagtcc ttgaacaggg 240

aggtgtgtcc agtttgtttc agaatctcgg ggtgtccgta actccgatcc aaaggattgt 300

cctgagcggt gaaaatgggc tgaagatcga catccatgtc atcatcccgt atgaaggtct 360

gagcggcgac caaatgggcc agatcgaaaa aatttttaag gtggtgtacc ctgtggatga 420

tcatcacttt aaggtgatcc tgcactatgg cacactggta atcgacgggg ttacgccgaa 480

catgatcgac tatttcggac ggccgtatga aggcatcgcc gtgttcgacg gcaaaaagat 540

cactgtaaca gggaccctgt ggaacggcaa caaaattatc gacgagcgcc tgatcaaccc 600

cgacggctcc ctgctgttcc gagtaaccat caacggagtg accggctggc ggctgtgcga 660

acgcattctg gcgtaatttt gagtaatttt caattttgtt tttgtttttg tttttgttgt 720

tgggagttgt tgtaatttgg taaatgttgg ggtcactaaa atcttttgtt gcaattttag 780

caccttttta ttagatatat aaaattgggt ttagatgtag ttaa 824

<210>  76
<211>  809
<212>  DNA
<213>  Artificial Sequence

<220>
<223>  Synthetic

<400>  76
catatcttca ttcttcactc tttccccccc cccccttatc ctcctatcta atcctctcat      60

ctctctcctc ctcttcctcc aacaacaact aacaatgtgg cagattgttt tctttactct     120

gagctgtgat cttgtcttgg ccgcagccgt cttcacactc gaagatttcg ttggggactg     180

gcgacagaca gccggctaca acctggacca agtccttgaa cagggaggtg tgtccagttt     240

gtttcagaat ctcggggtgt ccgtaactcc gatccaaagg attgtcctga gcggtgaaaa     300

tgggctgaag atcgacatcc atgtcatcat cccgtatgaa ggtctgagcg gcgaccaaat     360

gggccagatc gaaaaaattt ttaaggtggt gtaccctgtg gatgatcatc actttaaggt     420

gatcctgcac tatggcacac tggtaatcga cggggttacg ccgaacatga tcgactattt     480

cggacggccg tatgaaggca tcgccgtgtt cgacggcaaa aagatcactg taacagggac     540

cctgtggaac ggcaacaaaa ttatcgacga gcgcctgatc aaccccgacg gctccctgct     600

gttccgagta accatcaacg gagtgaccgg ctggcggctg tgcgaacgca ttctggcgta     660

attttgagta attttcaatt ttgtttttgt tttgtttttt gttgttggga gttgttgtaa     720

tttggtaaat gttggggtca ctaaaatctt ttgttgcaat tttagcacct ttttattaga     780

tatataaaat tgggtttaga tgtagttaa                                       809


<210>  77
<211>  1083
<212>  DNA
<213>  Artificial Sequence

<220>
<223>  Synthetic

<400>  77
ataaaagccc aggggcaagc ggtccggata acggctagcc tgaggagctg ctgcgacagt      60

ccactacctt tttcgagagt gactcccgtt gtcccaaggc ttcccagagc gaacctgtgc     120

ggctgcaggc accggcgcgt cgagtttccg gcgtccggaa ggaccgagct cttctcgcga     180

atccagtgtt ccgtttccag cccccaatct cagagcggag ccgacagaga gcagggaacc     240

ggcatgaaag tctctgccgc ccttctgtgc ctgctgctca tagcagccac cttcattccc     300

caagggctcg ctgtcttcac actcgaagat ttcgttgggg actggcgaca gacagccggc     360

tacaacctgg accaagtcct tgaacaggga ggtgtgtcca gtttgtttca gaatctcggg     420

gtgtccgtaa ctccgatcca aaggattgtc ctgagcggtg aaaatgggct gaagatcgac     480

atccatgtca tcatcccgta tgaaggtctg agcggcgacc aaatgggcca gatcgaaaaa 540

atttttaagg tggtgtaccc tgtggatgat catcacttta aggtgatcct gcactatggc 600

acactggtaa tcgacggggt tacgccgaac atgatcgact atttcggacg gccgtatgaa 660

ggcatcgccg tgttcgacgg caaaaagatc actgtaacag ggaccctgtg gaacggcaac 720

aaaattatcg acgagcgcct gatcaacccc gacggctccc tgctgttccg agtaaccatc 780

aacggagtga ccggctggcg gctgtgcgaa cgcattctgg cgtaagggcc tttccaagat 840

tgctgttttt gttttggagc ttcaagactt tgcatttcct agtatttctg tttgtcagtt 900

ctcaatttcc tgtgtttgca atgttgaaat tttttggtga agtactgaac ttgctttttt 960

tccggtttct acatgcagag atgaatttat actgccatct tacgactatt tcttcttttt 1020

aatacactta actcaggcca ttttttaagt tggttacttc aaagtaaata aactttaaaa 1080

ttc 1083

<210> 78
<211> 984
<212> DNA
<213> Artificial Sequence

<220>
<223> Synthetic

<400> 78
gggctggggg agggtatata agccgagtag gcgacggtga ggtcgacgcc ggccaagaca 60

gcacagacag attgacctat tggggtgttt cgcgagtgtg agagggaagc gccgcggcct 120

gtatttctag acctgccctt cgcctggttc gtggcgcctt gtgaccccgg gcccctgccg 180

cctgcaagtc ggaaattgcg ctgtgctcct gtgctacggc ctgtggctgg actgcctgct 240

gctgcccaac tggctggcaa gatgaaagtc tctgccgccc ttctgtgcct gctgctcata 300

gcagccacct tcattcccca agggctcgct gtcttcacac tcgaagattt cgttggggac 360

tggcgacaga cagccggcta caacctggac caagtccttg aacagggagg tgtgtccagt 420

ttgtttcaga atctcggggt gtccgtaact ccgatccaaa ggattgtcct gagcggtgaa 480

aatgggctga agatcgacat ccatgtcatc atcccgtatg aaggtctgag cggcgaccaa 540

atgggccaga tcgaaaaaat ttttaaggtg gtgtaccctg tggatgatca tcactttaag 600

gtgatcctgc actatggcac actggtaatc gacggggtta cgccgaacat gatcgactat 660

ttcggacggc cgtatgaagg catcgccgtg ttcgacggca aaaagatcac tgtaacaggg 720

accctgtgga acggcaacaa aattatcgac gagcgcctga tcaaccccga cggctccctg 780

ctgttccgag taaccatcaa cggagtgacc ggctggcggc tgtgcgaacg cattctggcg 840

taaggctgct gcagctgcat gggtgcctgc tgctgcctgc cttggcctga tggccaggcc 900

agagtgctgc cagtcctctg catgttctgc tcttgtgccc ttctgagccc acaataaagg 960

ctgagctctt atcttgcaaa aggc                                          984

<210>  79
<211>  898
<212>  DNA
<213>  Artificial Sequence

<220>
<223>  Synthetic

<400>  79
agctgttctt ggctgacttc acatcaaaac tcctatactg acctgagaca gaggcagcag      60

tgatacccac ctgagagatc ctgtgtttga acaactgctt cccaaaacgg aaagtatttc     120

aagcctaaac ctttgggtga aaagaactct tgaagtcatg aactccttct ccacaagcgc     180

cttcggtcca gttgccttct ccctgggcct gctcctggtg ttgcctgctg ccttccctgc     240

cccagtcttc acactcgaag atttcgttgg ggactggcga cagacagccg gctacaacct     300

ggaccaagtc cttgaacagg gaggtgtgtc cagtttgttt cagaatctcg gggtgtccgt     360

aactccgatc caaaggattg tcctgagcgg tgaaaatggg ctgaagatcg acatccatgt     420

catcatcccg tatgaaggtc tgagcggcga ccaaatgggc cagatcgaaa aaatttttaa     480

ggtggtgtac cctgtggatg atcatcactt taaggtgatc ctgcactatg gcacactggt     540

aatcgacggg gttacgccga acatgatcga ctatttcgga cggccgtatg aaggcatcgc     600

cgtgttcgac ggcaaaaaga tcactgtaac agggaccctg tggaacggca acaaaattat     660

cgacgagcgc ctgatcaacc ccgacggctc cctgctgttc cgagtaacca tcaacggagt     720

gaccggctgg cggctgtgcg aacgcattct ggcgtaaggc tgctgcagct gcatgggtgc     780

ctgctgctgc ctgccttggc ctgatggcca ggccagagtg ctgccagtcc tctgcatgtt     840

ctgctcttgt gcccttctga gcccacaata aaggctgagc tcttatcttg caaaaggc       898

<210>  80
<211>  1060
<212>  DNA
<213>  Artificial Sequence

<220>
<223>  Synthetic

<400>  80
caagcttagc ctggccggga aacgggaggc gtggaggccg ggagcagccc ccggggtcat      60

cgccctgcca ccgccgcccg attgctttag cttggaaatt ccggagctga agcggccagc     120

gagggaggat gaccctctcg gcccgggcac cctgtcagtc cggaaataac tgcagcattt     180

gttccggagg ggaaggcgcg aggtttccgg gaaagcagca ccgccccttg gcccccaggt     240

ggctagcgct ataaaggatc acgcgcccca gtcgacgctg agctcctctg ctactcagag     300

ttgcaacctc agcctcgcta tgaactcctt ctccacaagc gccttcggtc cagttgcctt 360

ctccctgggc ctgctcctgg tgttgcctgc tgccttccct gccccagtct tcacactcga 420

agatttcgtt ggggactggc gacagacagc cggctacaac ctggaccaag tccttgaaca 480

gggaggtgtg tccagtttgt ttcagaatct cggggtgtcc gtaactccga tccaaaggat 540

tgtcctgagc ggtgaaaatg ggctgaagat cgacatccat gtcatcatcc cgtatgaagg 600

tctgagcggc gaccaaatgg gccagatcga aaaaattttt aaggtggtgt accctgtgga 660

tgatcatcac tttaaggtga tcctgcacta tggcacactg gtaatcgacg gggttacgcc 720

gaacatgatc gactatttcg gacggccgta tgaaggcatc gccgtgttcg acggcaaaaa 780

gatcactgta acagggaccc tgtggaacgg caacaaaatt atcgacgagc gcctgatcaa 840

ccccgacggc tccctgctgt tccgagtaac catcaacgga gtgaccggct ggcggctgtg 900

cgaacgcatt ctggcgtaag gctgctgcag ctgcatgggt gcctgctgct gcctgccttg 960

gcctgatggc caggccagag tgctgccagt cctctgcatg ttctgctctt gtgcccttct 1020

gagcccacaa taaaggctga gctcttatct tgcaaaaggc 1060

<210> 81
<211> 1150
<212> DNA
<213> Artificial Sequence

<220>
<223> Synthetic

<400> 81
gtctcaatat tagagtctca acccccaata aatataggac tggagatgtc tgaggctcat 60

tctgccctgg agcccaccgg gaacgaaaga gaagctctat ctcccctcca ggagcccagc 120

tatgaactcc ttctccacaa gcgccttcgg tccagttgcc ttctccctgg ggctgctcct 180

ggtgttgcct gctgccttcc ctgccccagt cttcacactc gaagatttcg ttggggactg 240

gcgacagaca gccggctaca acctggacca agtccttgaa cagggaggtg tgtccagttt 300

gtttcagaat ctcggggtgt ccgtaactcc gatccaaagg attgtcctga gcggtgaaaa 360

tgggctgaag atcgacatcc atgtcatcat cccgtatgaa ggtctgagcg gcgaccaaat 420

gggccagatc gaaaaaattt ttaaggtggt gtaccctgtg gatgatcatc actttaaggt 480

gatcctgcac tatggcacac tggtaatcga cggggttacg ccgaacatga tcgactattt 540

cggacggccg tatgaaggca tcgccgtgtt cgacggcaaa aagatcactg taacagggac 600

cctgtggaac ggcaacaaaa ttatcgacga gcgcctgatc aaccccgacg gctccctgct 660

gttccgagta accatcaacg gagtgaccgg ctggcggctg tgcgaacgca ttctggcgta 720

acatgggcac ctcagattgt tgttgttaat gggcattcct tcttctggtc agaaacctgt 780

ccactgggca cagaacttat gttgttctct atggagaact aaaagtatga gcgttaggac 840

actattttaa ttatttttaa tttattaata tttaaatatg tgaagctgag ttaatttatg 900

taagtcatat ttatattttt aagaagtacc acttgaaaca ttttatgtat tagttttgaa 960

ataataatgg aaagtggcta tgcagtttga atatcctttg tttcagagcc agatcatttc 1020

ttggaaagtg taggcttacc tcaaataaat ggctaactta tacatatttt taaagaaata 1080

tttatattgt atttatataa tgtataaatg gtttttatac caataaatgg cattttaaaa 1140

aattcagcaa 1150

<210> 82
<211> 975
<212> DNA
<213> Artificial Sequence

<220>
<223> Synthetic

<400> 82
gtggcagctc acagctattg tggtgggaaa gggagggtgg ttggtggatg tcacagcttg 60

ggctttatct cccccagcag tggggactcc acagcccctg ggctacataa cagcaagaca 120

gtccggagct gtagcagacc tgattgagcc tttgcagcag ctgagagcat ggcctagggt 180

gggcggcacc attgtccagc agctgagttt cccagggacc ttggagatag ccgcagccct 240

catttgcagg ggaagatgat tcctgccaga tttgccgggg tgctgcttgc tctggccctc 300

attttgccag ggaccctttg tgtcttcaca ctcgaagatt tcgttgggga ctggcgacag 360

acagccggct acaacctgga ccaagtcctt gaacagggag gtgtgtccag tttgtttcag 420

aatctcgggg tgtccgtaac tccgatccaa aggattgtcc tgagcggtga aaatgggctg 480

aagatcgaca tccatgtcat catcccgtat gaaggtctga gcggcgacca aatgggccag 540

atcgaaaaaa tttttaaggt ggtgtaccct gtggatgatc atcactttaa ggtgatcctg 600

cactatggca cactggtaat cgacggggtt acgccgaaca tgatcgacta tttcggacgg 660

ccgtatgaag gcatcgccgt gttcgacggc aaaaagatca ctgtaacagg gaccctgtgg 720

aacggcaaca aaattatcga cgagcgcctg atcaaccccg acggctccct gctgttccga 780

gtaaccatca acggagtgac cggctggcgg ctgtgcgaac gcattctggc gtaaggctgc 840

tgcagctgca tgggtgcctg ctgctgcctg ccttggcctg atggccaggc cagagtgctg 900

ccagtcctct gcatgttctg ctcttgtgcc cttctgagcc cacaataaag gctgagctct 960

tatcttgcaa aaggc 975

<210> 83
<211> 497
<212> DNA
<213> Homo sapiens

<220>
<221>  5'UTR
<222>  (1)..(497)

<400>  83
gccctaagcc atcagcaatc cttagtatag gggcacactc atgcattcct gtcaagtcat      60

cttgtgaaag gctgcctgct tccagcttgg cttggatgtg caaccttaat aaaactcact     120

gaggtctggg agaaaatagc agatctgcag cagatagggt agaggaaagg gtctagaata     180

tgtacacgca gctgactcag gcaggctcca tgctgaacgg tcacacagag aggaaacaat     240

aaatctcagc tactatgcaa taaatatctc aagttttaac gaagaaaaac atcattgcag     300

tgaaataaaa aattttaaaa ttttagaaca aagctaacaa atggctagtt ttctatgatt     360

cttcttcaaa cgctttcttt gagggggaaa gagtcaaaca aacaagcagt tttacctgaa     420

ataaagaact agttttagag gtcagaagaa aggagcaagt tttgcgagag gcacggaagg     480

agtgtgctgg cagtaca                                                    497


<210>  84
<211>  45
<212>  DNA
<213>  Homo sapiens


<220>
<221>  sig_peptide
<222>  (1)..(45)

<400>  84
atgacagttt tcctttcctt tgctttcctc gctgccattc tgact                      45


<210>  85
<211>  15
<212>  PRT
<213>  Homo sapiens


<220>
<221>  SIGNAL
<222>  (1)..(15)

<400>  85

Met Thr Val Phe Leu Ser Phe Ala Phe Leu Ala Ala Ile Leu Thr
1               5                   10                  15


<210>  86
<211>  1452
<212>  DNA
<213>  Homo sapiens


<220>
<221>  CDS

<222>  (1)..(1452)

<400>  86
cac ata ggg tgc agc aat cag cgc cga agt cca gaa aac agt ggg aga      48
His Ile Gly Cys Ser Asn Gln Arg Arg Ser Pro Glu Asn Ser Gly Arg
1               5                   10                  15

aga tat aac cgg att caa cat ggg caa tgt gcc tac act ttc att ctt      96
Arg Tyr Asn Arg Ile Gln His Gly Gln Cys Ala Tyr Thr Phe Ile Leu
            20                  25                  30

cca gaa cac gat ggc aac tgt cgt gag agt acg aca gac cag tac aac      144
Pro Glu His Asp Gly Asn Cys Arg Glu Ser Thr Thr Asp Gln Tyr Asn
        35                  40                  45

aca aac gct ctg cag aga gat gct cca cac gtg gaa ccg gat ttc tct      192
Thr Asn Ala Leu Gln Arg Asp Ala Pro His Val Glu Pro Asp Phe Ser
    50                  55                  60

tcc cag aaa ctt caa cat ctg gaa cat gtg atg gaa aat tat act cag      240
Ser Gln Lys Leu Gln His Leu Glu His Val Met Glu Asn Tyr Thr Gln
65                  70                  75                  80

tgg ctg caa aaa ctt gag aat tac att gtg gaa aac atg aag tcg gag      288
Trp Leu Gln Lys Leu Glu Asn Tyr Ile Val Glu Asn Met Lys Ser Glu
                85                  90                  95

atg gcc cag ata cag cag aat gca gtt cag aac cac acg gct acc atg      336
Met Ala Gln Ile Gln Gln Asn Ala Val Gln Asn His Thr Ala Thr Met
            100                 105                 110

ctg gag ata gga acc agc ctc ctc tct cag act gca gag cag acc aga      384
Leu Glu Ile Gly Thr Ser Leu Leu Ser Gln Thr Ala Glu Gln Thr Arg
        115                 120                 125

aag ctg aca gat gtt gag acc cag gta cta aat caa act tct cga ctt      432
Lys Leu Thr Asp Val Glu Thr Gln Val Leu Asn Gln Thr Ser Arg Leu
    130                 135                 140

gag ata cag ctg ctg gag aat tca tta tcc acc tac aag cta gag aag      480
Glu Ile Gln Leu Leu Glu Asn Ser Leu Ser Thr Tyr Lys Leu Glu Lys
145                 150                 155                 160

caa ctt ctt caa cag aca aat gaa atc ttg aag atc cat gaa aaa aac      528
Gln Leu Leu Gln Gln Thr Asn Glu Ile Leu Lys Ile His Glu Lys Asn
                165                 170                 175

agt tta tta gaa cat aaa atc tta gaa atg gaa gga aaa cac aag gaa      576
Ser Leu Leu Glu His Lys Ile Leu Glu Met Glu Gly Lys His Lys Glu
            180                 185                 190

gag ttg gac acc tta aag gaa gag aaa gag aac ctt caa ggc ttg gtt      624
Glu Leu Asp Thr Leu Lys Glu Glu Lys Glu Asn Leu Gln Gly Leu Val
        195                 200                 205

act cgt caa aca tat ata atc cag gag ctg gaa aag caa tta aac aga      672
Thr Arg Gln Thr Tyr Ile Ile Gln Glu Leu Glu Lys Gln Leu Asn Arg
    210                 215                 220

gct acc acc aac aac agt gtc ctt cag aag cag caa ctg gag ctg atg      720
Ala Thr Thr Asn Asn Ser Val Leu Gln Lys Gln Gln Leu Glu Leu Met
225                 230                 235                 240

gac aca gtc cac aac ctt gtc aat ctt tgc act aaa gaa ggt gtt tta 768
Asp Thr Val His Asn Leu Val Asn Leu Cys Thr Lys Glu Gly Val Leu
                245                 250                 255

cta aag gga gga aaa aga gag gaa gag aaa cca ttt aga gac tgt gca 816
Leu Lys Gly Gly Lys Arg Glu Glu Glu Lys Pro Phe Arg Asp Cys Ala
            260                 265                 270

gat gta tat caa gct ggt ttt aat aaa agt gga atc tac act att tat 864
Asp Val Tyr Gln Ala Gly Phe Asn Lys Ser Gly Ile Tyr Thr Ile Tyr
        275                 280                 285

att aat aat atg cca gaa ccc aaa aag gtg ttt tgc aat atg gat gtc 912
Ile Asn Asn Met Pro Glu Pro Lys Lys Val Phe Cys Asn Met Asp Val
    290                 295                 300

aat ggg gga ggt tgg act gta ata caa cat cgt gaa gat gga agt cta 960
Asn Gly Gly Gly Trp Thr Val Ile Gln His Arg Glu Asp Gly Ser Leu
305                 310                 315                 320

gat ttc caa aga ggc tgg aag gaa tat aaa atg ggt ttt gga aat ccc 1008
Asp Phe Gln Arg Gly Trp Lys Glu Tyr Lys Met Gly Phe Gly Asn Pro
                325                 330                 335

tcc ggt gaa tat tgg ctg ggg aat gag ttt att ttt gcc att acc agt 1056
Ser Gly Glu Tyr Trp Leu Gly Asn Glu Phe Ile Phe Ala Ile Thr Ser
            340                 345                 350

cag agg cag tac atg cta aga att gag tta atg gac tgg gaa ggg aac 1104
Gln Arg Gln Tyr Met Leu Arg Ile Glu Leu Met Asp Trp Glu Gly Asn
        355                 360                 365

cga gcc tat tca cag tat gac aga ttc cac ata gga aat gaa aag caa 1152
Arg Ala Tyr Ser Gln Tyr Asp Arg Phe His Ile Gly Asn Glu Lys Gln
    370                 375                 380

aac tat agg ttg tat tta aaa ggt cac act ggg aca gca gga aaa cag 1200
Asn Tyr Arg Leu Tyr Leu Lys Gly His Thr Gly Thr Ala Gly Lys Gln
385                 390                 395                 400

agc agc ctg atc tta cac ggt gct gat ttc agc act aaa gat gct gat 1248
Ser Ser Leu Ile Leu His Gly Ala Asp Phe Ser Thr Lys Asp Ala Asp
                405                 410                 415

aat gac aac tgt atg tgc aaa tgt gcc ctc atg tta aca gga gga tgg 1296
Asn Asp Asn Cys Met Cys Lys Cys Ala Leu Met Leu Thr Gly Gly Trp
            420                 425                 430

tgg ttt gat gct tgt ggc ccc tcc aat cta aat gga atg ttc tat act 1344
Trp Phe Asp Ala Cys Gly Pro Ser Asn Leu Asn Gly Met Phe Tyr Thr
        435                 440                 445

gcg gga caa aac cat gga aaa ctg aat ggg ata aag tgg cac tac ttc 1392
Ala Gly Gln Asn His Gly Lys Leu Asn Gly Ile Lys Trp His Tyr Phe
    450                 455                 460

aaa ggg ccc agt tac tcc tta cgt tcc aca act atg atg att cga cct 1440
Lys Gly Pro Ser Tyr Ser Leu Arg Ser Thr Thr Met Met Ile Arg Pro
465                 470                 475                 480

tta gat ttt tga 1452
Leu Asp Phe

<210>  87
<211>  483
<212>  PRT
<213>  Homo sapiens

<400>  87

His Ile Gly Cys Ser Asn Gln Arg Arg Ser Pro Glu Asn Ser Gly Arg
1               5                   10                  15

Arg Tyr Asn Arg Ile Gln His Gly Gln Cys Ala Tyr Thr Phe Ile Leu
            20                  25                  30

Pro Glu His Asp Gly Asn Cys Arg Glu Ser Thr Thr Asp Gln Tyr Asn
        35                  40                  45

Thr Asn Ala Leu Gln Arg Asp Ala Pro His Val Glu Pro Asp Phe Ser
    50                  55                  60

Ser Gln Lys Leu Gln His Leu Glu His Val Met Glu Asn Tyr Thr Gln
65                  70                  75                  80

Trp Leu Gln Lys Leu Glu Asn Tyr Ile Val Glu Asn Met Lys Ser Glu
                85                  90                  95

Met Ala Gln Ile Gln Gln Asn Ala Val Gln Asn His Thr Ala Thr Met
            100                 105                 110

Leu Glu Ile Gly Thr Ser Leu Leu Ser Gln Thr Ala Glu Gln Thr Arg
        115                 120                 125

Lys Leu Thr Asp Val Glu Thr Gln Val Leu Asn Gln Thr Ser Arg Leu
    130                 135                 140

Glu Ile Gln Leu Leu Glu Asn Ser Leu Ser Thr Tyr Lys Leu Glu Lys
145                 150                 155                 160

Gln Leu Leu Gln Gln Thr Asn Glu Ile Leu Lys Ile His Glu Lys Asn
                165                 170                 175

Ser Leu Leu Glu His Lys Ile Leu Glu Met Glu Gly Lys His Lys Glu
            180                 185                 190

Glu Leu Asp Thr Leu Lys Glu Glu Lys Glu Asn Leu Gln Gly Leu Val
        195                 200                 205

Thr Arg Gln Thr Tyr Ile Ile Gln Glu Leu Glu Lys Gln Leu Asn Arg
    210                 215                 220

Ala Thr Thr Asn Asn Ser Val Leu Gln Lys Gln Gln Leu Glu Leu Met
225                 230                 235                 240

Asp Thr Val His Asn Leu Val Asn Leu Cys Thr Lys Glu Gly Val Leu
                245                 250                 255

Leu Lys Gly Gly Lys Arg Glu Glu Glu Lys Pro Phe Arg Asp Cys Ala
            260                 265                 270

Asp Val Tyr Gln Ala Gly Phe Asn Lys Ser Gly Ile Tyr Thr Ile Tyr
        275                 280                 285

Ile Asn Asn Met Pro Glu Pro Lys Lys Val Phe Cys Asn Met Asp Val
    290                 295                 300

Asn Gly Gly Gly Trp Thr Val Ile Gln His Arg Glu Asp Gly Ser Leu
305                 310                 315                 320

Asp Phe Gln Arg Gly Trp Lys Glu Tyr Lys Met Gly Phe Gly Asn Pro
                325                 330                 335

Ser Gly Glu Tyr Trp Leu Gly Asn Glu Phe Ile Phe Ala Ile Thr Ser
            340                 345                 350

Gln Arg Gln Tyr Met Leu Arg Ile Glu Leu Met Asp Trp Glu Gly Asn
        355                 360                 365

Arg Ala Tyr Ser Gln Tyr Asp Arg Phe His Ile Gly Asn Glu Lys Gln
    370                 375                 380

Asn Tyr Arg Leu Tyr Leu Lys Gly His Thr Gly Thr Ala Gly Lys Gln
385                 390                 395                 400

Ser Ser Leu Ile Leu His Gly Ala Asp Phe Ser Thr Lys Asp Ala Asp
                405                 410                 415

Asn Asp Asn Cys Met Cys Lys Cys Ala Leu Met Leu Thr Gly Gly Trp
            420                 425                 430

Trp Phe Asp Ala Cys Gly Pro Ser Asn Leu Asn Gly Met Phe Tyr Thr
        435                 440                 445

Ala Gly Gln Asn His Gly Lys Leu Asn Gly Ile Lys Trp His Tyr Phe
    450                 455                 460

Lys Gly Pro Ser Tyr Ser Leu Arg Ser Thr Thr Met Met Ile Arg Pro

465                 470                 475                 480

Leu Asp Phe

<210>  88
<211>  2373
<212>  DNA
<213>  Homo sapiens

<220>
<221>  5'UTR
<222>  (1)..(2373)

<400>  88
aagcgcaatg tcagaagcga ttatgaaagc aacaaagaaa tccggagaag ctgccaggtg      60

agaaactgtt tgaaaacttc agaagcaaac aatattgtct cccttccagc aataagtggt     120

agttatgtga agtcaccaag gttcttgacc gtgaatctgg agccgtttga gttcacaaga     180

gtctctactt ggggtgacag tgctcacgtg gctcgactat agaaaactcc actgactgtc     240

gggctttaaa aagggaagaa actgctgagc ttgctgtgct tcaaactact actggacctt     300

attttggaac tatggtagcc agatgataaa tatggttaat ttcatgtaaa acagaaaaaa     360

agagtgaaaa agagaatata catgaagaat agaaacaagc ctgccataat cctttggaaa     420

agatgtatta taccagtgaa aaggtgttat atctatgcaa acctactaac aaattatact     480

gttgcacaat tttgataaaa atttagaaca gcattgtcct ctgagttggt taaatgttaa     540

tggatttcag aagcctaatt ccagtatcat acttactagt tgatttctgc ttacccatct     600

tcaaatgaaa attccatttt tgtaagccat aatgaactgt agtacatgga caataagtgt     660

gtggtagaaa caaactccat tactctgatt tttgatacag ttttcagaaa aagaaatgaa     720

cataatcaag taaggatgta tgtggtgaaa acttaccacc cccatactat ggttttcatt     780

tactctaaaa actgattgaa tgatatataa atatatttat agcctgagta aagttaaaag     840

aatgtaaaat atatcatcaa gttcttaaaa taatatacat gcatttaata tttcctttga     900

tattatacag gaaagcaata ttttggagta tgttaagttg aagtaaaagc aagtactctg     960

gagcagttca ttttacagta tctacttgca tgtgtataca tacatgtaac ttcattattt    1020

taaaaatatt tttagaactc caatactcac cctgttatgt cttgctaatt taaattttgc    1080

taattaactg aaacatgctt accagattca cactgttcca gtgtctataa aagaaacact    1140

ttgaagtcta taaaaaataa aataattata aatatcattg tacatagcat gtttatatct    1200

gcaaaaaacc taatagctaa ttaatctgga atatgcaaca ttgtccttaa ttgatgcaaa    1260

taacacaaat gctcaaagaa atctactata tcccttaatg aaatacatca ttcttcatat    1320

atttctcctt cagtccattc ccttaggcaa tttttaattt ttaaaaatta ttatcagggg    1380

agaaaaattg gcaaaactat tatatgtaag ggaaatatat acaaaaagaa aattaatcat 1440

agtcacctga ctaagaaatt ctgactgcta gttgccataa ataactcaat ggaaatattc 1500

ctatgggata atgtatttta agtgaatttt tggggtgctt gaagttactg cattatttta 1560

tcaagaagtc ttctctgcct gtaagtgtcc aaggttatga cagtaaacag tttttattaa 1620

aacatgagtc actatgggat gagaaaattg aaataaagct actgggcctc ctctcataaa 1680

agagacagtt gttggcaagg tagcaatacc agtttcaaac ttggtgactt gatccactat 1740

gccttaatgg tttcctccat ttgagaaaat aaagctattc acattgttaa gaaaaatact 1800

ttttaaagtt taccatcaag tcttttttat atttatgtgt ctgtattcta cccctttttg 1860

ccttacaagt gatatttgca ggtattatac catttttcta ttcttggtgg cttcttcata 1920

gcaggtaagc ctctccttct aaaaacttct caactgtttt catttaaggg aaagaaaatg 1980

agtattttgt ccttttgtgt tcctacagac actttcttaa accagttttt ggataaagaa 2040

tactatttcc aaactcatat tacaaaaaca aaataaaata ataaaaaaag aaagcatgat 2100

atttactgtt ttgttgtctg ggtttgagaa atgaaatatt gtttccaatt atttataata 2160

aatcagtata aaatgtttta tgattgttat gtgtattatg taatacgtac atgtttatgg 2220

caatttaaca tgtgtattct tttaattgtt tcagaatagg ataattaggt attcgaattt 2280

tgtctttaaa attcatgtgg tttctatgca aagttcttca tatcatcaca acattatttg 2340

atttaaataa aattgaaagt aatatttgtg caa 2373

<210> 89
<211> 120
<212> DNA
<213> Homo sapiens

<220>
<221> 5'UTR
<222> (1)..(120)

<400> 89
agacagaggt ttgtagctgc agctgcaggc aagcctggcc actgttggct gcagcaggac 60

atcccaggca cagcccctag ggctctgagc agacatccct cgccattgac acatcttcag 120

<210> 90
<211> 66
<212> DNA
<213> Homo sapiens

<220>
<221> sig_peptide
<222> (1)..(66)

<400> 90

atgctctccc agctagccat gctgcagggc agcctcctcc ttgtggttgc caccatgtct     60

gtggct                                                                66

<210>  91
<211>  22
<212>  PRT
<213>  Homo sapiens

<220>
<221>  SIGNAL
<222>  (1)..(22)

<400>  91

Met Leu Ser Gln Leu Ala Met Leu Gln Gly Ser Leu Leu Leu Val Val
1               5                   10                  15

Ala Thr Met Ser Val Ala
            20

<210>  92
<211>  1446
<212>  DNA
<213>  Homo sapiens

<220>
<221>  CDS
<222>  (1)..(1446)

<400>  92
caa cag aca agg cag gag gcg gat agg ggc tgc gag aca ctt gta gtc      48
Gln Gln Thr Arg Gln Glu Ala Asp Arg Gly Cys Glu Thr Leu Val Val
1               5                   10                  15

cag cac ggc cac tgt agc tac acc ttc ttg ctg ccc aag tct gag ccc      96
Gln His Gly His Cys Ser Tyr Thr Phe Leu Leu Pro Lys Ser Glu Pro
            20                  25                  30

tgc cct ccg ggg cct gag gtc tcc agg gac tcc aac acc ctc cag aga      144
Cys Pro Pro Gly Pro Glu Val Ser Arg Asp Ser Asn Thr Leu Gln Arg
        35                  40                  45

gaa tca ctg gcc aac cca ctg cac ctg ggg aag ttg ccc acc cag cag      192
Glu Ser Leu Ala Asn Pro Leu His Leu Gly Lys Leu Pro Thr Gln Gln
    50                  55                  60

gtg aaa cag ctg gag cag gca ctg cag aac aac acg cag tgg ctg aag      240
Val Lys Gln Leu Glu Gln Ala Leu Gln Asn Asn Thr Gln Trp Leu Lys
65                  70                  75                  80

aag cta gag agg gcc atc aag acg atc ttg agg tcg aag ctg gag cag      288
Lys Leu Glu Arg Ala Ile Lys Thr Ile Leu Arg Ser Lys Leu Glu Gln
                85                  90                  95

gtc cag cag caa atg gcc cag aat cag acg gcc ccc atg cta gag ctg      336
Val Gln Gln Gln Met Ala Gln Asn Gln Thr Ala Pro Met Leu Glu Leu
            100                 105                 110

ggc acc agc ctc ctg aac cag acc act gcc cag atc cgc aag ctg acc 384
Gly Thr Ser Leu Leu Asn Gln Thr Thr Ala Gln Ile Arg Lys Leu Thr
        115                 120                 125

gac atg gag gct cag ctc ctg aac cag aca tca aga atg gat gcc cag 432
Asp Met Glu Ala Gln Leu Leu Asn Gln Thr Ser Arg Met Asp Ala Gln
    130                 135                 140

atg cca gag acc ttt ctg tcc acc aac aag ctg gag aac cag ctg ctg 480
Met Pro Glu Thr Phe Leu Ser Thr Asn Lys Leu Glu Asn Gln Leu Leu
145                 150                 155                 160

cta cag agg cag aag ctc cag cag ctt cag ggc caa aac agc gcg ctc 528
Leu Gln Arg Gln Lys Leu Gln Gln Leu Gln Gly Gln Asn Ser Ala Leu
                165                 170                 175

gag aag cgg ttg cag gcc ctg gag acc aag cag cag gag gag ctg gcc 576
Glu Lys Arg Leu Gln Ala Leu Glu Thr Lys Gln Gln Glu Glu Leu Ala
            180                 185                 190

agc atc ctc agc aag aag gcg aag ctg ctg aac acg ctg agc cgc cag 624
Ser Ile Leu Ser Lys Lys Ala Lys Leu Leu Asn Thr Leu Ser Arg Gln
        195                 200                 205

agc gcc gcc ctc acc aac atc gag cgc ggc ctg cgc ggt gtc agg cac 672
Ser Ala Ala Leu Thr Asn Ile Glu Arg Gly Leu Arg Gly Val Arg His
    210                 215                 220

aac tcc agc ctc ctg cag gac cag cag cac agc ctg cgc cag ctg ctg 720
Asn Ser Ser Leu Leu Gln Asp Gln Gln His Ser Leu Arg Gln Leu Leu
225                 230                 235                 240

gtg ttg ttg cgg cac ctg gtg caa gaa agg gct aac gcc tcg gcc ccg 768
Val Leu Leu Arg His Leu Val Gln Glu Arg Ala Asn Ala Ser Ala Pro
                245                 250                 255

gcc ttc ata atg gca ggt gag cag gtg ttc cag gac tgt gca gag atc 816
Ala Phe Ile Met Ala Gly Glu Gln Val Phe Gln Asp Cys Ala Glu Ile
            260                 265                 270

cag cgc tct ggg gcc agt gcc agt ggt gtc tac acc atc cag gtg tcc 864
Gln Arg Ser Gly Ala Ser Ala Ser Gly Val Tyr Thr Ile Gln Val Ser
        275                 280                 285

aat gca acg aag ccc agg aag gtg ttc tgt gac ctg cag agc agt gga 912
Asn Ala Thr Lys Pro Arg Lys Val Phe Cys Asp Leu Gln Ser Ser Gly
    290                 295                 300

ggc agg tgg acc ctc atc cag cgc cgt gag aat ggc acc gtg aat ttt 960
Gly Arg Trp Thr Leu Ile Gln Arg Arg Glu Asn Gly Thr Val Asn Phe
305                 310                 315                 320

cag cgg aac tgg aag gat tac aaa cag ggc ttc gga gac cca gct ggg 1008
Gln Arg Asn Trp Lys Asp Tyr Lys Gln Gly Phe Gly Asp Pro Ala Gly
                325                 330                 335

gag cac tgg ctg ggc aat gaa gtg gtg cac cag ctc acc aga agg gca 1056
Glu His Trp Leu Gly Asn Glu Val Val His Gln Leu Thr Arg Arg Ala
            340                 345                 350

gcc tac tct ctg cgt gtg gag ctg caa gac tgg gaa ggc cac gag gcc 1104
Ala Tyr Ser Leu Arg Val Glu Leu Gln Asp Trp Glu Gly His Glu Ala

355                 360                 365

tat gcc cag tac gaa cat ttc cac ctg ggc agt gag aac cag cta tac          1152
Tyr Ala Gln Tyr Glu His Phe His Leu Gly Ser Glu Asn Gln Leu Tyr
    370                 375                 380

agg ctt tct gtg gtc ggg tac agc ggc tca gca ggg cgc cag agc agc          1200
Arg Leu Ser Val Val Gly Tyr Ser Gly Ser Ala Gly Arg Gln Ser Ser
385                 390                 395                 400

ctg gtc ctg cag aac acc agc ttt agc acc ctt gac tca gac aac gac          1248
Leu Val Leu Gln Asn Thr Ser Phe Ser Thr Leu Asp Ser Asp Asn Asp
                405                 410                 415

cac tgt ctc tgc aag tgt gcc caa gtg atg tct gga ggg tgg tgg ttt          1296
His Cys Leu Cys Lys Cys Ala Gln Val Met Ser Gly Gly Trp Trp Phe
            420                 425                 430

gac gcc tgt ggc ctg tca aac ctc aac ggc gtc tac tac cac gct ccc          1344
Asp Ala Cys Gly Leu Ser Asn Leu Asn Gly Val Tyr Tyr His Ala Pro
        435                 440                 445

gac aac aag tac aag atg gac ggc atc cgc tgg cac tac ttc aag ggc          1392
Asp Asn Lys Tyr Lys Met Asp Gly Ile Arg Trp His Tyr Phe Lys Gly
    450                 455                 460

ccc agc tac tca ctg cgt gcc tct cgc atg atg ata cgg cct ttg gac          1440
Pro Ser Tyr Ser Leu Arg Ala Ser Arg Met Met Ile Arg Pro Leu Asp
465                 470                 475                 480

atc taa                                                                  1446
Ile

<210>  93
<211>  481
<212>  PRT
<213>  Homo sapiens

<400>  93

Gln Gln Thr Arg Gln Glu Ala Asp Arg Gly Cys Glu Thr Leu Val Val
1               5                   10                  15

Gln His Gly His Cys Ser Tyr Thr Phe Leu Leu Pro Lys Ser Glu Pro
            20                  25                  30

Cys Pro Pro Gly Pro Glu Val Ser Arg Asp Ser Asn Thr Leu Gln Arg
        35                  40                  45

Glu Ser Leu Ala Asn Pro Leu His Leu Gly Lys Leu Pro Thr Gln Gln
    50                  55                  60

Val Lys Gln Leu Glu Gln Ala Leu Gln Asn Asn Thr Gln Trp Leu Lys
65                  70                  75                  80

Lys Leu Glu Arg Ala Ile Lys Thr Ile Leu Arg Ser Lys Leu Glu Gln

85                  90                  95

Val Gln Gln Gln Met Ala Gln Asn Gln Thr Ala Pro Met Leu Glu Leu
            100                 105                 110

Gly Thr Ser Leu Leu Asn Gln Thr Thr Ala Gln Ile Arg Lys Leu Thr
        115                 120                 125

Asp Met Glu Ala Gln Leu Leu Asn Gln Thr Ser Arg Met Asp Ala Gln
    130                 135                 140

Met Pro Glu Thr Phe Leu Ser Thr Asn Lys Leu Glu Asn Gln Leu Leu
145                 150                 155                 160

Leu Gln Arg Gln Lys Leu Gln Gln Leu Gln Gly Gln Asn Ser Ala Leu
                165                 170                 175

Glu Lys Arg Leu Gln Ala Leu Glu Thr Lys Gln Gln Glu Glu Leu Ala
            180                 185                 190

Ser Ile Leu Ser Lys Lys Ala Lys Leu Leu Asn Thr Leu Ser Arg Gln
        195                 200                 205

Ser Ala Ala Leu Thr Asn Ile Glu Arg Gly Leu Arg Gly Val Arg His
    210                 215                 220

Asn Ser Ser Leu Leu Gln Asp Gln Gln His Ser Leu Arg Gln Leu Leu
225                 230                 235                 240

Val Leu Leu Arg His Leu Val Gln Glu Arg Ala Asn Ala Ser Ala Pro
                245                 250                 255

Ala Phe Ile Met Ala Gly Glu Gln Val Phe Gln Asp Cys Ala Glu Ile
            260                 265                 270

Gln Arg Ser Gly Ala Ser Ala Ser Gly Val Tyr Thr Ile Gln Val Ser
        275                 280                 285

Asn Ala Thr Lys Pro Arg Lys Val Phe Cys Asp Leu Gln Ser Ser Gly
    290                 295                 300

Gly Arg Trp Thr Leu Ile Gln Arg Arg Glu Asn Gly Thr Val Asn Phe
305                 310                 315                 320

Gln Arg Asn Trp Lys Asp Tyr Lys Gln Gly Phe Gly Asp Pro Ala Gly
                325                 330                 335

Glu His Trp Leu Gly Asn Glu Val Val His Gln Leu Thr Arg Arg Ala
            340                 345                 350

Ala Tyr Ser Leu Arg Val Glu Leu Gln Asp Trp Glu Gly His Glu Ala
        355                 360                 365

Tyr Ala Gln Tyr Glu His Phe His Leu Gly Ser Glu Asn Gln Leu Tyr
    370                 375                 380

Arg Leu Ser Val Val Gly Tyr Ser Gly Ser Ala Gly Arg Gln Ser Ser
385                 390                 395                 400

Leu Val Leu Gln Asn Thr Ser Phe Ser Thr Leu Asp Ser Asp Asn Asp
                405                 410                 415

His Cys Leu Cys Lys Cys Ala Gln Val Met Ser Gly Gly Trp Trp Phe
            420                 425                 430

Asp Ala Cys Gly Leu Ser Asn Leu Asn Gly Val Tyr Tyr His Ala Pro
        435                 440                 445

Asp Asn Lys Tyr Lys Met Asp Gly Ile Arg Trp His Tyr Phe Lys Gly
    450                 455                 460

Pro Ser Tyr Ser Leu Arg Ala Ser Arg Met Met Ile Arg Pro Leu Asp
465                 470                 475                 480

Ile

<210>  94
<211>  3061
<212>  DNA
<213>  Homo sapiens

<220>
<221>  3'UTR
<222>  (1)..(3061)

<400>  94
cgagcagctg tgccagaggc tggaccacac aggagaagct cggacttggc actcctggac      60

aacctggacc cagatgcaag acactgtgcc accgccttcc ctgacaccct gggcttcctg     120

agccagccct ccttgaccca gaagtccaga agggtcatct gccccccaac tcccctccgt     180

ctgtgacatg gagggtgttc ggggcccatc cctctgatgt agtcctcgcc cctcttctct     240

ccctccccct tcaggggctc cctgcctgag ggtcacagta ccttgaatgg gctgagaaca     300

gaccaaactt gattcccatg accaatggtg gggttgcagg caggtgggaa tgtatttgca     360

catcggaagc tgcccagatg gcccaggttc tctcccttgg attggcaaga aggccatctc 420
ccattctaag ctcctgttcc aagattttct agtcttgaga tgtccttgaa ctttcttttc 480
aagtctgaag gggctgcatc caccccttag tgggtgggtt aatcattatt tccccttcac 540
acttcaccac ttctaggttc taatgaccct agatctcagg gtctttagac ttcaccactt 600
ctaggcttta ccacttcacc acttctaggc tccaatgttt ggagctcagg gtctttagga 660
gacccaaaag gacatgctcc ttcacctcca gcatgtccta gaggatgtgt cacagggaat 720
aactatggct tgtctctaaa agtacctatg agcaatgaga aaaggaaaca gcaggttaag 780
tcaaagtgaa caggcactct tcactgcagg actgatcaga gcctttaata tggccaagtg 840
ccttgtgact acccatgaag gggctagagt gggcagcttt ctccaaattt acttatttga 900
aaatgggctc ggtttgtccc agagcatctc acaggactgt agatgctctt ggacaaagct 960
agtgctcccc tggcataagg aggagcccta cgaccccatc cccaccccag ctatactcac 1020
cctttttggc tacaagggcc acagtgacag cctcaaacaa cctctaaaaa caactggaaa 1080
taacctttca gttaaaacag ataccatccc tgaagaaggg tctagaacta ggtccctgtc 1140
tgtgttatag gctcatgtcc tccaaggctc cttcaagtcc caggaagctg atctctacct 1200
gggtggcttc ccttaggact ccctgtaacc tcaactcccc caggctcaat tacagggact 1260
gttaggcagg acatctgtct ccaagtccag atcctctctg cctccaagcc ctaaccccta 1320
gcctccctcc cttccccatc cagcagtgat gctgcctctg tggtggtagg tggggagctg 1380
caggggagga gataaggcct ctgcctgagt ttgggagacc agggccctca tagcttcttt 1440
cagaggatgg agtcagaaag gatccacagc tactctgtca cctgccccca tcactgtgtc 1500
atgctgtctg ccctgttgtc atcagccaac acccaggcat agccaggagc ccacctgccc 1560
taccgccagg atacacctct gtcctcagaa ggttttctcc tggatgagac tgagccaatg 1620
ggaatgggac cccttcatcc ccctggctcg ccccagccct gagtcccact ctcagccgat 1680
ccctgagtaa acccagcaca gactgacttt gatctcattc ctgggaatta gcactcttcc 1740
ccttcaagac tcaaaggaca tggttgctaa tggtggcatt tcaggcatga tgggaaatct 1800
ttaggggcag attgctgccc agagagctca aatcgcctta agcagcattt gcccagcaga 1860
cctttattta gcctctactg tgtgcagtgt ggtgtggtgg gcagggcttt ggagtcggac 1920
aaacctgctc cagctctgac actttggtcc agtggctcag cctctcaagg caccagttat 1980
cttcacatca tcaaagcctc agttttccca tctgtaaaat ggagatgata atattccttc 2040
ctggctgggc tatggcaagg aggaaatgag accatgtatg tcatcttctt aatagagcct 2100
ggcatgaagc aggtgcctaa taaatgtttg tcctcaaaga ggagaatggg gtgaggaagg 2160
cattccccag cacatgccgc cccttctcct gcactcaggt gaggaaaagg cattttattt 2220
ttgtatccac atcatttatt tttctattgt agtttctagg ctgactgcaa gctagagagg 2280

agacagggca aagctgtgag gcccagggac agaactcctc tgggtgggtt gaaggcccaa 2340

gtccctctct actcccattt tataaggggg caggaagctg atttgagtta tcctcagaca 2400

cctgttcttt atgtaatttt attttatttt tttgagacag agtctcactc tgtcacccag 2460

gctggagtgc agtggcatga tctcagatca ctgcaatctc tgcctcctgg ttcaagtgat 2520

tctcctacct cagcctcctg agtaatggga ttacagacgc ctaccaccac gcccggaaaa 2580

cttttgtatt tttagtagaa acgggttttc accatgttgg ccaggctggt ctcaaactcc 2640

tggcctcatg tgatccacct gcctcagcct cccaaagtgc tgggattaca ggcatgagcc 2700

accataccca gcctcagaca cctgttctta aatattcatc cttctttctt accttccttc 2760

ctcttccatg ccaggactca ggtataaggg atagaaattc tagccctaag gaataaattg 2820

actcacataa ctggaaagtc taagggtaaa ggcaagtgag gttagatcca gaggctcaaa 2880

tgatgtcagc tccacctctc agcccctcca tctgccccgt tgacttcatt ctcagccagg 2940

atctttcctc acaagaaggc tctggcagcc ccaggctcat gtcctcccag ctcagcatcc 3000

ctgacccggg gagctccctc gtctccatga ttccagtaaa ggaatgattt tctgcagcca 3060

g 3061

<210> 95
<211> 867
<212> DNA
<213> Artificial Sequence

<220>
<223> Synthetic


<220>
<221> CDS
<222> (1)..(867)

<400> 95
ggg atc tta gac cta gcc cca cag atg ctt cga gaa ctc cag gag act 48
Gly Ile Leu Asp Leu Ala Pro Gln Met Leu Arg Glu Leu Gln Glu Thr
1               5                   10                  15

aat gcg gcg ctg caa gac gtg aga gag ctc ttg cga cag cag gtc aag 96
Asn Ala Ala Leu Gln Asp Val Arg Glu Leu Leu Arg Gln Gln Val Lys
            20                  25                  30

gag atc acc ttc ctg aag aat acg gtg atg gaa tgt gac gct tgc gga 144
Glu Ile Thr Phe Leu Lys Asn Thr Val Met Glu Cys Asp Ala Cys Gly
        35                  40                  45

ggc tcc ctt gtc aat ctt tgc act aaa gaa ggt gtt tta cta aag gga 192
Gly Ser Leu Val Asn Leu Cys Thr Lys Glu Gly Val Leu Leu Lys Gly
    50                  55                  60

gga aaa aga gag gaa gag aaa cca ttt aga gac tgt gca gat gta tat 240
Gly Lys Arg Glu Glu Glu Lys Pro Phe Arg Asp Cys Ala Asp Val Tyr
65                  70                  75                  80

caa gct ggt ttt aat aaa agt gga atc tac act att tat att aat aat     288
Gln Ala Gly Phe Asn Lys Ser Gly Ile Tyr Thr Ile Tyr Ile Asn Asn
                85                  90                  95

atg cca gaa ccc aaa aag gtg ttt tgc aat atg gat gtc aat ggg gga     336
Met Pro Glu Pro Lys Lys Val Phe Cys Asn Met Asp Val Asn Gly Gly
            100                 105                 110

ggt tgg act gta ata caa cat cgt gaa gat gga agt cta gat ttc caa     384
Gly Trp Thr Val Ile Gln His Arg Glu Asp Gly Ser Leu Asp Phe Gln
        115                 120                 125

aga ggc tgg aag gaa tat aaa atg ggt ttt gga aat ccc tcc ggt gaa     432
Arg Gly Trp Lys Glu Tyr Lys Met Gly Phe Gly Asn Pro Ser Gly Glu
    130                 135                 140

tat tgg ctg ggg aat gag ttt att ttt gcc att acc agt cag agg cag     480
Tyr Trp Leu Gly Asn Glu Phe Ile Phe Ala Ile Thr Ser Gln Arg Gln
145                 150                 155                 160

tac atg cta aga att gag tta atg gac tgg gaa ggg aac cga gcc tat     528
Tyr Met Leu Arg Ile Glu Leu Met Asp Trp Glu Gly Asn Arg Ala Tyr
                165                 170                 175

tca cag tat gac aga ttc cac ata gga aat gaa aag caa aac tat agg     576
Ser Gln Tyr Asp Arg Phe His Ile Gly Asn Glu Lys Gln Asn Tyr Arg
            180                 185                 190

ttg tat tta aaa ggt cac act ggg aca gca gga aaa cag agc agc ctg     624
Leu Tyr Leu Lys Gly His Thr Gly Thr Ala Gly Lys Gln Ser Ser Leu
        195                 200                 205

atc tta cac ggt gct gat ttc agc act aaa gat gct gat aat gac aac     672
Ile Leu His Gly Ala Asp Phe Ser Thr Lys Asp Ala Asp Asn Asp Asn
    210                 215                 220

tgt atg tgc aaa tgt gcc ctc atg tta aca gga gga tgg tgg ttt gat     720
Cys Met Cys Lys Cys Ala Leu Met Leu Thr Gly Gly Trp Trp Phe Asp
225                 230                 235                 240

gct tgt ggc ccc tcc aat cta aat gga atg ttc tat act gcg gga caa     768
Ala Cys Gly Pro Ser Asn Leu Asn Gly Met Phe Tyr Thr Ala Gly Gln
                245                 250                 255

aac cat gga aaa ctg aat ggg ata aag tgg cac tac ttc aaa ggg ccc     816
Asn His Gly Lys Leu Asn Gly Ile Lys Trp His Tyr Phe Lys Gly Pro
            260                 265                 270

agt tac tcc tta cgt tcc aca act atg atg att cga cct tta gat ttt     864
Ser Tyr Ser Leu Arg Ser Thr Thr Met Met Ile Arg Pro Leu Asp Phe
        275                 280                 285

tga                                                                 867

<210>  96
<211>  288
<212>  PRT
<213>  Artificial Sequence

<220>
<223>  Synthetic Construct

<400>  96

Gly Ile Leu Asp Leu Ala Pro Gln Met Leu Arg Glu Leu Gln Glu Thr
1               5                   10                  15

Asn Ala Ala Leu Gln Asp Val Arg Glu Leu Leu Arg Gln Gln Val Lys
            20                  25                  30

Glu Ile Thr Phe Leu Lys Asn Thr Val Met Glu Cys Asp Ala Cys Gly
        35                  40                  45

Gly Ser Leu Val Asn Leu Cys Thr Lys Glu Gly Val Leu Leu Lys Gly
    50                  55                  60

Gly Lys Arg Glu Glu Glu Lys Pro Phe Arg Asp Cys Ala Asp Val Tyr
65                  70                  75                  80

Gln Ala Gly Phe Asn Lys Ser Gly Ile Tyr Thr Ile Tyr Ile Asn Asn
                85                  90                  95

Met Pro Glu Pro Lys Lys Val Phe Cys Asn Met Asp Val Asn Gly Gly
            100                 105                 110

Gly Trp Thr Val Ile Gln His Arg Glu Asp Gly Ser Leu Asp Phe Gln
        115                 120                 125

Arg Gly Trp Lys Glu Tyr Lys Met Gly Phe Gly Asn Pro Ser Gly Glu
    130                 135                 140

Tyr Trp Leu Gly Asn Glu Phe Ile Phe Ala Ile Thr Ser Gln Arg Gln
145                 150                 155                 160

Tyr Met Leu Arg Ile Glu Leu Met Asp Trp Glu Gly Asn Arg Ala Tyr
                165                 170                 175

Ser Gln Tyr Asp Arg Phe His Ile Gly Asn Glu Lys Gln Asn Tyr Arg
            180                 185                 190

Leu Tyr Leu Lys Gly His Thr Gly Thr Ala Gly Lys Gln Ser Ser Leu
        195                 200                 205

Ile Leu His Gly Ala Asp Phe Ser Thr Lys Asp Ala Asp Asn Asp Asn
    210                 215                 220

Cys Met Cys Lys Cys Ala Leu Met Leu Thr Gly Gly Trp Trp Phe Asp
225                 230                 235                 240

Ala Cys Gly Pro Ser Asn Leu Asn Gly Met Phe Tyr Thr Ala Gly Gln
                245                 250                 255

Asn His Gly Lys Leu Asn Gly Ile Lys Trp His Tyr Phe Lys Gly Pro
            260                 265                 270

Ser Tyr Ser Leu Arg Ser Thr Thr Met Met Ile Arg Pro Leu Asp Phe
        275                 280                 285

<210>  97
<211>  867
<212>  DNA
<213>  Artificial Sequence

<220>
<223>  Synthetic

<220>
<221>  CDS
<222>  (1)..(867)

<400>  97
ggg atc tta gac ttg ggc ccg cag atg ctt cgg gaa ctg cag gaa acc      48
Gly Ile Leu Asp Leu Gly Pro Gln Met Leu Arg Glu Leu Gln Glu Thr
1               5                   10                  15

aac gcg gcg ctg cag gac gtg cgg gag ctg ctg cgg cag cag gtc agg      96
Asn Ala Ala Leu Gln Asp Val Arg Glu Leu Leu Arg Gln Gln Val Arg
            20                  25                  30

gag atc acg ttc ctg aaa aac acg gtg atg gag tgt gac gcg tgc gga      144
Glu Ile Thr Phe Leu Lys Asn Thr Val Met Glu Cys Asp Ala Cys Gly
        35                  40                  45

ggc tcc ctt gtc aat ctt tgc act aaa gaa ggt gtt tta cta aag gga      192
Gly Ser Leu Val Asn Leu Cys Thr Lys Glu Gly Val Leu Leu Lys Gly
    50                  55                  60

gga aaa aga gag gaa gag aaa cca ttt aga gac tgt gca gat gta tat      240
Gly Lys Arg Glu Glu Glu Lys Pro Phe Arg Asp Cys Ala Asp Val Tyr
65                  70                  75                  80

caa gct ggt ttt aat aaa agt gga atc tac act att tat att aat aat      288
Gln Ala Gly Phe Asn Lys Ser Gly Ile Tyr Thr Ile Tyr Ile Asn Asn
                85                  90                  95

atg cca gaa ccc aaa aag gtg ttt tgc aat atg gat gtc aat ggg gga      336
Met Pro Glu Pro Lys Lys Val Phe Cys Asn Met Asp Val Asn Gly Gly
            100                 105                 110

ggt tgg act gta ata caa cat cgt gaa gat gga agt cta gat ttc caa      384
Gly Trp Thr Val Ile Gln His Arg Glu Asp Gly Ser Leu Asp Phe Gln
        115                 120                 125

aga ggc tgg aag gaa tat aaa atg ggt ttt gga aat ccc tcc ggt gaa      432
Arg Gly Trp Lys Glu Tyr Lys Met Gly Phe Gly Asn Pro Ser Gly Glu
    130                 135                 140

tat tgg ctg ggg aat gag ttt att ttt gcc att acc agt cag agg cag      480

Tyr Trp Leu Gly Asn Glu Phe Ile Phe Ala Ile Thr Ser Gln Arg Gln
145                 150                 155                 160

tac atg cta aga att gag tta atg gac tgg gaa ggg aac cga gcc tat     528
Tyr Met Leu Arg Ile Glu Leu Met Asp Trp Glu Gly Asn Arg Ala Tyr
                165                 170                 175

tca cag tat gac aga ttc cac ata gga aat gaa aag caa aac tat agg     576
Ser Gln Tyr Asp Arg Phe His Ile Gly Asn Glu Lys Gln Asn Tyr Arg
            180                 185                 190

ttg tat tta aaa ggt cac act ggg aca gca gga aaa cag agc agc ctg     624
Leu Tyr Leu Lys Gly His Thr Gly Thr Ala Gly Lys Gln Ser Ser Leu
        195                 200                 205

atc tta cac ggt gct gat ttc agc act aaa gat gct gat aat gac aac     672
Ile Leu His Gly Ala Asp Phe Ser Thr Lys Asp Ala Asp Asn Asp Asn
    210                 215                 220

tgt atg tgc aaa tgt gcc ctc atg tta aca gga gga tgg tgg ttt gat     720
Cys Met Cys Lys Cys Ala Leu Met Leu Thr Gly Gly Trp Trp Phe Asp
225                 230                 235                 240

gct tgt ggc ccc tcc aat cta aat gga atg ttc tat act gcg gga caa     768
Ala Cys Gly Pro Ser Asn Leu Asn Gly Met Phe Tyr Thr Ala Gly Gln
                245                 250                 255

aac cat gga aaa ctg aat ggg ata aag tgg cac tac ttc aaa ggg ccc     816
Asn His Gly Lys Leu Asn Gly Ile Lys Trp His Tyr Phe Lys Gly Pro
            260                 265                 270

agt tac tcc tta cgt tcc aca act atg atg att cga cct tta gat ttt     864
Ser Tyr Ser Leu Arg Ser Thr Thr Met Met Ile Arg Pro Leu Asp Phe
        275                 280                 285

tga                                                                 867


<210>  98
<211>  288
<212>  PRT
<213>  Artificial Sequence

<220>
<223>  Synthetic Construct

<400>  98

Gly Ile Leu Asp Leu Gly Pro Gln Met Leu Arg Glu Leu Gln Glu Thr
1               5                   10                  15


Asn Ala Ala Leu Gln Asp Val Arg Glu Leu Leu Arg Gln Gln Val Arg
            20                  25                  30


Glu Ile Thr Phe Leu Lys Asn Thr Val Met Glu Cys Asp Ala Cys Gly
        35                  40                  45


Gly Ser Leu Val Asn Leu Cys Thr Lys Glu Gly Val Leu Leu Lys Gly
    50                  55                  60

Gly Lys Arg Glu Glu Glu Lys Pro Phe Arg Asp Cys Ala Asp Val Tyr
65                  70                  75                  80

Gln Ala Gly Phe Asn Lys Ser Gly Ile Tyr Thr Ile Tyr Ile Asn Asn
                85                  90                  95

Met Pro Glu Pro Lys Lys Val Phe Cys Asn Met Asp Val Asn Gly Gly
            100                 105                 110

Gly Trp Thr Val Ile Gln His Arg Glu Asp Gly Ser Leu Asp Phe Gln
        115                 120                 125

Arg Gly Trp Lys Glu Tyr Lys Met Gly Phe Gly Asn Pro Ser Gly Glu
    130                 135                 140

Tyr Trp Leu Gly Asn Glu Phe Ile Phe Ala Ile Thr Ser Gln Arg Gln
145                 150                 155                 160

Tyr Met Leu Arg Ile Glu Leu Met Asp Trp Glu Gly Asn Arg Ala Tyr
                165                 170                 175

Ser Gln Tyr Asp Arg Phe His Ile Gly Asn Glu Lys Gln Asn Tyr Arg
            180                 185                 190

Leu Tyr Leu Lys Gly His Thr Gly Thr Ala Gly Lys Gln Ser Ser Leu
        195                 200                 205

Ile Leu His Gly Ala Asp Phe Ser Thr Lys Asp Ala Asp Asn Asp Asn
    210                 215                 220

Cys Met Cys Lys Cys Ala Leu Met Leu Thr Gly Gly Trp Trp Phe Asp
225                 230                 235                 240

Ala Cys Gly Pro Ser Asn Leu Asn Gly Met Phe Tyr Thr Ala Gly Gln
                245                 250                 255

Asn His Gly Lys Leu Asn Gly Ile Lys Trp His Tyr Phe Lys Gly Pro
            260                 265                 270

Ser Tyr Ser Leu Arg Ser Thr Thr Met Met Ile Arg Pro Leu Asp Phe
        275                 280                 285

<210>  99
<211>  861
<212>  DNA
<213>  Artificial Sequence

<220>

<223>  Synthetic

<400>  99
gggatcttag aggaagaccc ggccgcctgc gagtccctgg tgaaattcca agccaaagtg      60

gaggggctgc tgcaggccct gaccaggaaa ctggaagctg tgagtaagcg gctggccatc     120

ctggagaaca cagttgtcgg atcccttgtc aatcttgcca ctaaagaagg tgttttacta     180

aagggaggaa aaagagagga agagaaacca tttagagact gtgcagatgt atatcaagct     240

ggttttaata aaagtggaat ctacactatt tatattaata atatgccaga acccaaaaag     300

gtgttttgca atatggatgt caatggggga ggttggactg taatacaaca tcgtgaagat     360

ggaagtctag atttccaaag aggctggaag gaatataaaa tgggttttgg aaatccctcc     420

ggtgaatatt ggctggggaa tgagtttatt tttgccatta ccagtcagag gcagtacatg     480

ctaagaattg agttaatgga ctgggaaggg aaccgagcct attcacagta tgacagattc     540

cacataggaa atgaaaagca aaactatagg ttgtatttaa aaggtcacac tgggacagca     600

ggaaaacaga gcagcctgat cttacacggt gctgatttca gcactaaaga tgctgataat     660

gacaactgta tgtgcaaatg tgccctcatg ttaacaggag gatggtggtt tgatgcttgt     720

ggcccctcca atctaaatgg aatgttctat actgcgggac aaaaccatgg aaaactgaat     780

gggataaagt ggcactactt caaagggccc agttactcct tacgttccac aactatgatg     840

attcgacctt tagatttttg a                                               861


<210>  100
<211>  870
<212>  DNA
<213>  Artificial Sequence

<220>
<223>  Synthetic

<400>  100
gggatcttag acctagcccc acagatgctt cgagaactcc aggagactaa tgcggcgctg      60

caagacgtga gagagctctt gcgacagcag gtcaaggaga tcaccttcct gaagaatacg     120

gtgatggaat gtgacgcttg cggaggctcc ttactgacta tgatgtccac atcaaactca     180

gctaaggacc ccactgttgc taaagaagaa caaatcagct tcagagactg tgctgaagta     240

ttcaaatcag gacacaccac gaatggcatc tacacgttaa cattccctaa ttctacagaa     300

gagatcaagg cctactgtga catggaagct ggaggaggcg ggtggacaat tattcagcga     360

cgtgaggatg gcagcgttga ttttcagagg acttggaaag aatataaagt gggatttggt     420

aacccttcag gagaatattg gctgggaaat gagtttgttt cgcaactgac taatcagcaa     480

cgctatgtgc ttaaaataca ccttaaagac tgggaaggga atgaggctta ctcattgtat     540

gaacatttct atctctcaag tgaagaactc aattatagga ttcaccttaa aggacttaca     600

gggacagccg gcaaaataag cagcatcagc caaccaggaa atgattttag cacaaaggat 660

ggagacaacg acaaatgtat ttgcaaatgt tcacaaatgc taacaggagg ctggtggttt 720

gatgcatgtg gtccttccaa cttgaacgga atgtactatc cacagaggca gaacacaaat 780

aagttcaacg gcattaaatg gtactactgg aaaggctcag gctattcgct caaggccaca 840

accatgatga tccgaccagc agatttctaa 870

<210> 101
<211> 3375
<212> DNA
<213> Homo sapiens

<220>
<221> CDS
<222> (1)..(3375)

<400> 101
atg gac tct tta gcc agc tta gtt ctc tgt gga gtc agc ttg ctc ctt 48
Met Asp Ser Leu Ala Ser Leu Val Leu Cys Gly Val Ser Leu Leu Leu
1               5                   10                  15

tct gga act gtg gaa ggt gcc atg gac ttg atc ttg atc aat tcc cta 96
Ser Gly Thr Val Glu Gly Ala Met Asp Leu Ile Leu Ile Asn Ser Leu
            20                  25                  30

cct ctt gta tct gat gct gaa aca tct ctc acc tgc att gcc tct ggg 144
Pro Leu Val Ser Asp Ala Glu Thr Ser Leu Thr Cys Ile Ala Ser Gly
        35                  40                  45

tgg cgc ccc cat gag ccc atc acc ata gga agg gac ttt gaa gcc tta 192
Trp Arg Pro His Glu Pro Ile Thr Ile Gly Arg Asp Phe Glu Ala Leu
    50                  55                  60

atg aac cag cac cag gat ccg ctg gaa gtt act caa gat gtg acc aga 240
Met Asn Gln His Gln Asp Pro Leu Glu Val Thr Gln Asp Val Thr Arg
65                  70                  75                  80

gaa tgg gct aaa aaa gtt gtt tgg aag aga gaa aag gct agt aag atc 288
Glu Trp Ala Lys Lys Val Val Trp Lys Arg Glu Lys Ala Ser Lys Ile
                85                  90                  95

aat ggt gct tat ttc tgt gaa ggg cga gtt cga gga gag gca atc agg 336
Asn Gly Ala Tyr Phe Cys Glu Gly Arg Val Arg Gly Glu Ala Ile Arg
            100                 105                 110

ata cga acc atg aag atg cgt caa caa gct tcc ttc cta cca gct act 384
Ile Arg Thr Met Lys Met Arg Gln Gln Ala Ser Phe Leu Pro Ala Thr
        115                 120                 125

tta act atg act gtg gac aag gga gat aac gtg aac ata tct ttc aaa 432
Leu Thr Met Thr Val Asp Lys Gly Asp Asn Val Asn Ile Ser Phe Lys
    130                 135                 140

aag gta ttg att aaa gaa gaa gat gca gtg att tac aaa aat ggt tcc 480
Lys Val Leu Ile Lys Glu Glu Asp Ala Val Ile Tyr Lys Asn Gly Ser
145                 150                 155                 160

ttc atc cat tca gtg ccc cgg cat gaa gta cct gat att cta gaa gta 528

Phe Ile His Ser Val Pro Arg His Glu Val Pro Asp Ile Leu Glu Val
                165                 170                 175

cac ctg cct cat gct cag ccc cag gat gct gga gtg tac tcg gcc agg      576
His Leu Pro His Ala Gln Pro Gln Asp Ala Gly Val Tyr Ser Ala Arg
            180                 185                 190

tat ata gga gga aac ctc ttc acc tcg gcc ttc acc agg ctg ata gtc      624
Tyr Ile Gly Gly Asn Leu Phe Thr Ser Ala Phe Thr Arg Leu Ile Val
        195                 200                 205

cgg aga tgt gaa gcc cag aag tgg gga cct gaa tgc aac cat ctc tgt      672
Arg Arg Cys Glu Ala Gln Lys Trp Gly Pro Glu Cys Asn His Leu Cys
    210                 215                 220

act gct tgt atg aac aat ggt gtc tgc cat gaa gat act gga gaa tgc      720
Thr Ala Cys Met Asn Asn Gly Val Cys His Glu Asp Thr Gly Glu Cys
225                 230                 235                 240

att tgc cct cct ggg ttt atg gga agg acg tgt gag aag gct tgt gaa      768
Ile Cys Pro Pro Gly Phe Met Gly Arg Thr Cys Glu Lys Ala Cys Glu
                245                 250                 255

ctg cac acg ttt ggc aga act tgt aaa gaa agg tgc agt gga caa gag      816
Leu His Thr Phe Gly Arg Thr Cys Lys Glu Arg Cys Ser Gly Gln Glu
            260                 265                 270

gga tgc aag tct tat gtg ttc tgt ctc cct gac ccc tat ggg tgt tcc      864
Gly Cys Lys Ser Tyr Val Phe Cys Leu Pro Asp Pro Tyr Gly Cys Ser
        275                 280                 285

tgt gcc aca ggc tgg aag ggt ctg cag tgc aat gaa gca tgc cac cct      912
Cys Ala Thr Gly Trp Lys Gly Leu Gln Cys Asn Glu Ala Cys His Pro
    290                 295                 300

ggt ttt tac ggg cca gat tgt aag ctt agg tgc agc tgc aac aat ggg      960
Gly Phe Tyr Gly Pro Asp Cys Lys Leu Arg Cys Ser Cys Asn Asn Gly
305                 310                 315                 320

gag atg tgt gat cgc ttc caa gga tgt ctc tgc tct cca gga tgg cag     1008
Glu Met Cys Asp Arg Phe Gln Gly Cys Leu Cys Ser Pro Gly Trp Gln
                325                 330                 335

ggg ctc cag tgt gag aga gaa ggc ata ccg agg atg acc cca aag ata     1056
Gly Leu Gln Cys Glu Arg Glu Gly Ile Pro Arg Met Thr Pro Lys Ile
            340                 345                 350

gtg gat ttg cca gat cat ata gaa gta aac agt ggt aaa ttt aat ccc     1104
Val Asp Leu Pro Asp His Ile Glu Val Asn Ser Gly Lys Phe Asn Pro
        355                 360                 365

att tgc aaa gct tct ggc tgg ccg cta cct act aat gaa gaa atg acc     1152
Ile Cys Lys Ala Ser Gly Trp Pro Leu Pro Thr Asn Glu Glu Met Thr
    370                 375                 380

ctg gtg aag ccg gat ggg aca gtg ctc cat cca aaa gac ttt aac cat     1200
Leu Val Lys Pro Asp Gly Thr Val Leu His Pro Lys Asp Phe Asn His
385                 390                 395                 400

acg gat cat ttc tca gta gcc ata ttc acc atc cac cgg atc ctc ccc     1248
Thr Asp His Phe Ser Val Ala Ile Phe Thr Ile His Arg Ile Leu Pro
                405                 410                 415

cct gac tca gga gtt tgg gtc tgc agt gtg aac aca gtg gct ggg atg 1296
Pro Asp Ser Gly Val Trp Val Cys Ser Val Asn Thr Val Ala Gly Met
            420                 425                 430

gtg gaa aag ccc ttc aac att tct gtt aaa gtt ctt cca aag ccc ctg 1344
Val Glu Lys Pro Phe Asn Ile Ser Val Lys Val Leu Pro Lys Pro Leu
        435                 440                 445

aat gcc cca aac gtg att gac act gga cat aac ttt gct gtc atc aac 1392
Asn Ala Pro Asn Val Ile Asp Thr Gly His Asn Phe Ala Val Ile Asn
    450                 455                 460

atc agc tct gag cct tac ttt ggg gat gga cca atc aaa tcc aag aag 1440
Ile Ser Ser Glu Pro Tyr Phe Gly Asp Gly Pro Ile Lys Ser Lys Lys
465                 470                 475                 480

ctt cta tac aaa ccc gtt aat cac tat gag gct tgg caa cat att caa 1488
Leu Leu Tyr Lys Pro Val Asn His Tyr Glu Ala Trp Gln His Ile Gln
                485                 490                 495

gtg aca aat gag att gtt aca ctc aac tat ttg gaa cct cgg aca gaa 1536
Val Thr Asn Glu Ile Val Thr Leu Asn Tyr Leu Glu Pro Arg Thr Glu
            500                 505                 510

tat gaa ctc tgt gtg caa ctg gtc cgt cgt gga gag ggt ggg gaa ggg 1584
Tyr Glu Leu Cys Val Gln Leu Val Arg Arg Gly Glu Gly Gly Glu Gly
        515                 520                 525

cat cct gga cct gtg aga cgc ttc aca aca gct tct atc gga ctc cct 1632
His Pro Gly Pro Val Arg Arg Phe Thr Thr Ala Ser Ile Gly Leu Pro
    530                 535                 540

cct cca aga ggt cta aat ctc ctg cct aaa agt cag acc act cta aat 1680
Pro Pro Arg Gly Leu Asn Leu Leu Pro Lys Ser Gln Thr Thr Leu Asn
545                 550                 555                 560

ttg acc tgg caa cca ata ttt cca agc tcg gaa gat gac ttt tat gtt 1728
Leu Thr Trp Gln Pro Ile Phe Pro Ser Ser Glu Asp Asp Phe Tyr Val
                565                 570                 575

gaa gtg gag aga agg tct gtg caa aaa agt gat cag cag aat att aaa 1776
Glu Val Glu Arg Arg Ser Val Gln Lys Ser Asp Gln Gln Asn Ile Lys
            580                 585                 590

gtt cca ggc aac ttg act tcg gtg cta ctt aac aac tta cat ccc agg 1824
Val Pro Gly Asn Leu Thr Ser Val Leu Leu Asn Asn Leu His Pro Arg
        595                 600                 605

gag cag tac gtg gtc cga gct aga gtc aac acc aag gcc cag ggg gaa 1872
Glu Gln Tyr Val Val Arg Ala Arg Val Asn Thr Lys Ala Gln Gly Glu
    610                 615                 620

tgg agt gaa gat ctc act gct tgg acc ctt agt gac att ctt cct cct 1920
Trp Ser Glu Asp Leu Thr Ala Trp Thr Leu Ser Asp Ile Leu Pro Pro
625                 630                 635                 640

caa cca gaa aac atc aag att tcc aac att aca cac tcc tca gct gtg 1968
Gln Pro Glu Asn Ile Lys Ile Ser Asn Ile Thr His Ser Ser Ala Val
                645                 650                 655

att tct tgg aca ata ttg gat ggc tat tct att tct tct att act atc 2016
Ile Ser Trp Thr Ile Leu Asp Gly Tyr Ser Ile Ser Ser Ile Thr Ile
            660                 665                 670

cgt tac aag gtt caa ggc aag aat gaa gac cag cac gtt gat gtg aag     2064
Arg Tyr Lys Val Gln Gly Lys Asn Glu Asp Gln His Val Asp Val Lys
        675                 680                 685

ata aag aat gcc acc atc act cag tat cag ctc aag ggc cta gag cct     2112
Ile Lys Asn Ala Thr Ile Thr Gln Tyr Gln Leu Lys Gly Leu Glu Pro
    690                 695                 700

gaa aca gca tac cag gtg gac att ttt gca gag aac aac ata ggg tca     2160
Glu Thr Ala Tyr Gln Val Asp Ile Phe Ala Glu Asn Asn Ile Gly Ser
705                 710                 715                 720

agc aac cca gcc ttt tct cat gaa ctg gtg acc ctc cca gaa tct caa     2208
Ser Asn Pro Ala Phe Ser His Glu Leu Val Thr Leu Pro Glu Ser Gln
                725                 730                 735

gca cca gcg gac ctc gga ggg ggg aag atg ctg ctt ata gcc atc ctt     2256
Ala Pro Ala Asp Leu Gly Gly Gly Lys Met Leu Leu Ile Ala Ile Leu
            740                 745                 750

ggc tct gct gga atg acc tgc ctg act gtg ctg ttg gcc ttt ctg atc     2304
Gly Ser Ala Gly Met Thr Cys Leu Thr Val Leu Leu Ala Phe Leu Ile
        755                 760                 765

ata ttg caa ttg aag agg gca aat gtg caa agg aga atg gcc caa gcc     2352
Ile Leu Gln Leu Lys Arg Ala Asn Val Gln Arg Arg Met Ala Gln Ala
    770                 775                 780

ttc caa aac gtg agg gaa gaa cca gct gtg cag ttc aac tca ggg act     2400
Phe Gln Asn Val Arg Glu Glu Pro Ala Val Gln Phe Asn Ser Gly Thr
785                 790                 795                 800

ctg gcc cta aac agg aag gtc aaa aac aac cca gat cct aca att tat     2448
Leu Ala Leu Asn Arg Lys Val Lys Asn Asn Pro Asp Pro Thr Ile Tyr
                805                 810                 815

cca gtg ctt gac tgg aat gac atc aaa ttt caa gat gtg att ggg gag     2496
Pro Val Leu Asp Trp Asn Asp Ile Lys Phe Gln Asp Val Ile Gly Glu
            820                 825                 830

ggc aat ttt ggc caa gtt ctt aag gcg cgc atc aag aag gat ggg tta     2544
Gly Asn Phe Gly Gln Val Leu Lys Ala Arg Ile Lys Lys Asp Gly Leu
        835                 840                 845

cgg atg gat gct gcc atc aaa aga atg aaa gaa tat gcc tcc aaa gat     2592
Arg Met Asp Ala Ala Ile Lys Arg Met Lys Glu Tyr Ala Ser Lys Asp
    850                 855                 860

gat cac agg gac ttt gca gga gaa ctg gaa gtt ctt tgt aaa ctt gga     2640
Asp His Arg Asp Phe Ala Gly Glu Leu Glu Val Leu Cys Lys Leu Gly
865                 870                 875                 880

cac cat cca aac atc atc aat ctc tta gga gca tgt gaa cat cga ggc     2688
His His Pro Asn Ile Ile Asn Leu Leu Gly Ala Cys Glu His Arg Gly
                885                 890                 895

tac ttg tac ctg gcc att gag tac gcg ccc cat gga aac ctt ctg gac     2736
Tyr Leu Tyr Leu Ala Ile Glu Tyr Ala Pro His Gly Asn Leu Leu Asp
            900                 905                 910

ttc ctt cgc aag agc cgt gtg ctg gag acg gac cca gca ttt gcc att     2784
Phe Leu Arg Lys Ser Arg Val Leu Glu Thr Asp Pro Ala Phe Ala Ile

915                 920                 925

gcc aat agc acc gcg tcc aca ctg tcc tcc cag cag ctc ctt cac ttc       2832
Ala Asn Ser Thr Ala Ser Thr Leu Ser Ser Gln Gln Leu Leu His Phe
    930                 935                 940

gct gcc gac gtg gcc cgg ggc atg gac tac ttg agc caa aaa cag ttt       2880
Ala Ala Asp Val Ala Arg Gly Met Asp Tyr Leu Ser Gln Lys Gln Phe
945                 950                 955                 960

atc cac agg gat ctg gct gcc aga aac att tta gtt ggt gaa aac tat       2928
Ile His Arg Asp Leu Ala Ala Arg Asn Ile Leu Val Gly Glu Asn Tyr
                965                 970                 975

gtg gca aaa ata gca gat ttt gga ttg tcc cga ggt caa gag gtg tat       2976
Val Ala Lys Ile Ala Asp Phe Gly Leu Ser Arg Gly Gln Glu Val Tyr
            980                 985                 990

gtg aaa aag aca atg gga agg ctc  cca gtg cgc tgg atg  gcc atc gag     3024
Val Lys Lys Thr Met Gly Arg Leu  Pro Val Arg Trp Met  Ala Ile Glu
        995                 1000                1005

tca ctg  aat tac agt gtg tac  aca acc aac agt gat  gta tgg tcc       3069
Ser Leu  Asn Tyr Ser Val Tyr  Thr Thr Asn Ser Asp  Val Trp Ser
    1010                1015                1020

tat ggt  gtg tta cta tgg gag  att gtt agc tta gga  ggc aca ccc       3114
Tyr Gly  Val Leu Leu Trp Glu  Ile Val Ser Leu Gly  Gly Thr Pro
    1025                1030                1035

tac tgc  ggg atg act tgt gca  gaa ctc tac gag aag  ctg ccc cag       3159
Tyr Cys  Gly Met Thr Cys Ala  Glu Leu Tyr Glu Lys  Leu Pro Gln
    1040                1045                1050

ggc tac  aga ctg gag aag ccc  ctg aac tgt gat gat  gag gtg tat       3204
Gly Tyr  Arg Leu Glu Lys Pro  Leu Asn Cys Asp Asp  Glu Val Tyr
    1055                1060                1065

gat cta  atg aga caa tgc tgg  cgg gag aag cct tat  gag agg cca       3249
Asp Leu  Met Arg Gln Cys Trp  Arg Glu Lys Pro Tyr  Glu Arg Pro
    1070                1075                1080

tca ttt  gcc cag ata ttg gtg  tcc tta aac aga atg  tta gag gag       3294
Ser Phe  Ala Gln Ile Leu Val  Ser Leu Asn Arg Met  Leu Glu Glu
    1085                1090                1095

cga aag  acc tac gtg aat acc  acg ctt tat gag aag  ttt act tat       3339
Arg Lys  Thr Tyr Val Asn Thr  Thr Leu Tyr Glu Lys  Phe Thr Tyr
    1100                1105                1110

gca gga  att gac tgt tct gct  gaa gaa gcg gcc tag                    3375
Ala Gly  Ile Asp Cys Ser Ala  Glu Glu Ala Ala
    1115                1120

<210>  102
<211>  1124
<212>  PRT
<213>  Homo sapiens

<400>  102

Met Asp Ser Leu Ala Ser Leu Val Leu Cys Gly Val Ser Leu Leu Leu

1               5                   10                  15

Ser Gly Thr Val Glu Gly Ala Met Asp Leu Ile Leu Ile Asn Ser Leu
            20                  25                  30

Pro Leu Val Ser Asp Ala Glu Thr Ser Leu Thr Cys Ile Ala Ser Gly
        35                  40                  45

Trp Arg Pro His Glu Pro Ile Thr Ile Gly Arg Asp Phe Glu Ala Leu
    50                  55                  60

Met Asn Gln His Gln Asp Pro Leu Glu Val Thr Gln Asp Val Thr Arg
65                  70                  75                  80

Glu Trp Ala Lys Lys Val Val Trp Lys Arg Glu Lys Ala Ser Lys Ile
                85                  90                  95

Asn Gly Ala Tyr Phe Cys Glu Gly Arg Val Arg Gly Glu Ala Ile Arg
            100                 105                 110

Ile Arg Thr Met Lys Met Arg Gln Gln Ala Ser Phe Leu Pro Ala Thr
        115                 120                 125

Leu Thr Met Thr Val Asp Lys Gly Asp Asn Val Asn Ile Ser Phe Lys
    130                 135                 140

Lys Val Leu Ile Lys Glu Glu Asp Ala Val Ile Tyr Lys Asn Gly Ser
145                 150                 155                 160

Phe Ile His Ser Val Pro Arg His Glu Val Pro Asp Ile Leu Glu Val
                165                 170                 175

His Leu Pro His Ala Gln Pro Gln Asp Ala Gly Val Tyr Ser Ala Arg
            180                 185                 190

Tyr Ile Gly Gly Asn Leu Phe Thr Ser Ala Phe Thr Arg Leu Ile Val
        195                 200                 205

Arg Arg Cys Glu Ala Gln Lys Trp Gly Pro Glu Cys Asn His Leu Cys
    210                 215                 220

Thr Ala Cys Met Asn Asn Gly Val Cys His Glu Asp Thr Gly Glu Cys
225                 230                 235                 240

Ile Cys Pro Pro Gly Phe Met Gly Arg Thr Cys Glu Lys Ala Cys Glu
                245                 250                 255

Leu His Thr Phe Gly Arg Thr Cys Lys Glu Arg Cys Ser Gly Gln Glu
            260                 265                 270

Gly Cys Lys Ser Tyr Val Phe Cys Leu Pro Asp Pro Tyr Gly Cys Ser
        275                 280                 285

Cys Ala Thr Gly Trp Lys Gly Leu Gln Cys Asn Glu Ala Cys His Pro
    290                 295                 300

Gly Phe Tyr Gly Pro Asp Cys Lys Leu Arg Cys Ser Cys Asn Asn Gly
305                 310                 315                 320

Glu Met Cys Asp Arg Phe Gln Gly Cys Leu Cys Ser Pro Gly Trp Gln
                325                 330                 335

Gly Leu Gln Cys Glu Arg Glu Gly Ile Pro Arg Met Thr Pro Lys Ile
            340                 345                 350

Val Asp Leu Pro Asp His Ile Glu Val Asn Ser Gly Lys Phe Asn Pro
        355                 360                 365

Ile Cys Lys Ala Ser Gly Trp Pro Leu Pro Thr Asn Glu Glu Met Thr
    370                 375                 380

Leu Val Lys Pro Asp Gly Thr Val Leu His Pro Lys Asp Phe Asn His
385                 390                 395                 400

Thr Asp His Phe Ser Val Ala Ile Phe Thr Ile His Arg Ile Leu Pro
                405                 410                 415

Pro Asp Ser Gly Val Trp Val Cys Ser Val Asn Thr Val Ala Gly Met
            420                 425                 430

Val Glu Lys Pro Phe Asn Ile Ser Val Lys Val Leu Pro Lys Pro Leu
        435                 440                 445

Asn Ala Pro Asn Val Ile Asp Thr Gly His Asn Phe Ala Val Ile Asn
    450                 455                 460

Ile Ser Ser Glu Pro Tyr Phe Gly Asp Gly Pro Ile Lys Ser Lys Lys
465                 470                 475                 480

Leu Leu Tyr Lys Pro Val Asn His Tyr Glu Ala Trp Gln His Ile Gln
                485                 490                 495

Val Thr Asn Glu Ile Val Thr Leu Asn Tyr Leu Glu Pro Arg Thr Glu
            500                 505                 510

Tyr Glu Leu Cys Val Gln Leu Val Arg Arg Gly Glu Gly Gly Glu Gly
        515                 520                 525

His Pro Gly Pro Val Arg Arg Phe Thr Thr Ala Ser Ile Gly Leu Pro
    530                 535                 540

Pro Pro Arg Gly Leu Asn Leu Leu Pro Lys Ser Gln Thr Thr Leu Asn
545                 550                 555                 560

Leu Thr Trp Gln Pro Ile Phe Pro Ser Ser Glu Asp Asp Phe Tyr Val
                565                 570                 575

Glu Val Glu Arg Arg Ser Val Gln Lys Ser Asp Gln Gln Asn Ile Lys
            580                 585                 590

Val Pro Gly Asn Leu Thr Ser Val Leu Leu Asn Asn Leu His Pro Arg
        595                 600                 605

Glu Gln Tyr Val Val Arg Ala Arg Val Asn Thr Lys Ala Gln Gly Glu
    610                 615                 620

Trp Ser Glu Asp Leu Thr Ala Trp Thr Leu Ser Asp Ile Leu Pro Pro
625                 630                 635                 640

Gln Pro Glu Asn Ile Lys Ile Ser Asn Ile Thr His Ser Ser Ala Val
                645                 650                 655

Ile Ser Trp Thr Ile Leu Asp Gly Tyr Ser Ile Ser Ser Ile Thr Ile
            660                 665                 670

Arg Tyr Lys Val Gln Gly Lys Asn Glu Asp Gln His Val Asp Val Lys
        675                 680                 685

Ile Lys Asn Ala Thr Ile Thr Gln Tyr Gln Leu Lys Gly Leu Glu Pro
    690                 695                 700

Glu Thr Ala Tyr Gln Val Asp Ile Phe Ala Glu Asn Asn Ile Gly Ser
705                 710                 715                 720

Ser Asn Pro Ala Phe Ser His Glu Leu Val Thr Leu Pro Glu Ser Gln
                725                 730                 735

Ala Pro Ala Asp Leu Gly Gly Gly Lys Met Leu Leu Ile Ala Ile Leu
            740                 745                 750

Gly Ser Ala Gly Met Thr Cys Leu Thr Val Leu Leu Ala Phe Leu Ile
        755                 760                 765

Ile Leu Gln Leu Lys Arg Ala Asn Val Gln Arg Arg Met Ala Gln Ala
    770                 775                 780

Phe Gln Asn Val Arg Glu Glu Pro Ala Val Gln Phe Asn Ser Gly Thr
785                 790                 795                 800

Leu Ala Leu Asn Arg Lys Val Lys Asn Asn Pro Asp Pro Thr Ile Tyr
                805                 810                 815

Pro Val Leu Asp Trp Asn Asp Ile Lys Phe Gln Asp Val Ile Gly Glu
            820                 825                 830

Gly Asn Phe Gly Gln Val Leu Lys Ala Arg Ile Lys Lys Asp Gly Leu
        835                 840                 845

Arg Met Asp Ala Ala Ile Lys Arg Met Lys Glu Tyr Ala Ser Lys Asp
    850                 855                 860

Asp His Arg Asp Phe Ala Gly Glu Leu Glu Val Leu Cys Lys Leu Gly
865                 870                 875                 880

His His Pro Asn Ile Ile Asn Leu Leu Gly Ala Cys Glu His Arg Gly
                885                 890                 895

Tyr Leu Tyr Leu Ala Ile Glu Tyr Ala Pro His Gly Asn Leu Leu Asp
            900                 905                 910

Phe Leu Arg Lys Ser Arg Val Leu Glu Thr Asp Pro Ala Phe Ala Ile
        915                 920                 925

Ala Asn Ser Thr Ala Ser Thr Leu Ser Ser Gln Gln Leu Leu His Phe
    930                 935                 940

Ala Ala Asp Val Ala Arg Gly Met Asp Tyr Leu Ser Gln Lys Gln Phe
945                 950                 955                 960

Ile His Arg Asp Leu Ala Ala Arg Asn Ile Leu Val Gly Glu Asn Tyr
                965                 970                 975

Val Ala Lys Ile Ala Asp Phe Gly Leu Ser Arg Gly Gln Glu Val Tyr
            980                 985                 990

Val Lys Lys Thr Met Gly Arg Leu  Pro Val Arg Trp Met  Ala Ile Glu
        995                 1000                1005

Ser Leu  Asn Tyr Ser Val Tyr  Thr Thr Asn Ser Asp  Val Trp Ser

1010                1015                1020

Tyr Gly Val Leu Leu Trp Glu Ile Val Ser Leu Gly Gly Thr Pro
    1025                1030                1035

Tyr Cys Gly Met Thr Cys Ala Glu Leu Tyr Glu Lys Leu Pro Gln
    1040                1045                1050

Gly Tyr Arg Leu Glu Lys Pro Leu Asn Cys Asp Asp Glu Val Tyr
    1055                1060                1065

Asp Leu Met Arg Gln Cys Trp Arg Glu Lys Pro Tyr Glu Arg Pro
    1070                1075                1080

Ser Phe Ala Gln Ile Leu Val Ser Leu Asn Arg Met Leu Glu Glu
    1085                1090                1095

Arg Lys Thr Tyr Val Asn Thr Thr Leu Tyr Glu Lys Phe Thr Tyr
    1100                1105                1110

Ala Gly Ile Asp Cys Ser Ala Glu Glu Ala Ala
    1115                1120

<210>  103
<211>  3375
<212>  DNA
<213>  Homo sapiens

<220>
<221>  CDS
<222>  (1)..(3375)

<400>  103
atg gac tct tta gcc agc tta gtt ctc tgt gga gtc agc ttg ctc ctt        48
Met Asp Ser Leu Ala Ser Leu Val Leu Cys Gly Val Ser Leu Leu Leu
1               5                   10                  15

tct gga act gtg gaa ggt gcc atg gac ttg atc ttg atc aat tcc cta        96
Ser Gly Thr Val Glu Gly Ala Met Asp Leu Ile Leu Ile Asn Ser Leu
            20                  25                  30

cct ctt gta tct gat gct gaa aca tct ctc acc tgc att gcc tct ggg       144
Pro Leu Val Ser Asp Ala Glu Thr Ser Leu Thr Cys Ile Ala Ser Gly
        35                  40                  45

tgg cgc ccc cat gag ccc atc acc ata gga agg gac ttt gaa gcc tta       192
Trp Arg Pro His Glu Pro Ile Thr Ile Gly Arg Asp Phe Glu Ala Leu
    50                  55                  60

atg aac cag cac cag gat ccg ctg gaa gtt act caa gat gtg acc aga       240
Met Asn Gln His Gln Asp Pro Leu Glu Val Thr Gln Asp Val Thr Arg
65                  70                  75                  80

gaa tgg gct aaa aaa gtt gtt tgg aag aga gaa aag gct agt aag atc       288

Glu Trp Ala Lys Lys Val Val Trp Lys Arg Glu Lys Ala Ser Lys Ile
                85                  90                  95

aat ggt gct tat ttc tgt gaa ggg cga gtt cga gga gag gca atc agg      336
Asn Gly Ala Tyr Phe Cys Glu Gly Arg Val Arg Gly Glu Ala Ile Arg
            100                 105                 110

ata cga acc atg aag atg cgt caa caa gct tcc ttc cta cca gct act      384
Ile Arg Thr Met Lys Met Arg Gln Gln Ala Ser Phe Leu Pro Ala Thr
        115                 120                 125

tta act atg act gtg gac aag gga gat aac gtg aac ata tct ttc aaa      432
Leu Thr Met Thr Val Asp Lys Gly Asp Asn Val Asn Ile Ser Phe Lys
    130                 135                 140

aag gta ttg att aaa gaa gaa gat gca gtg att tac aaa aat ggt tcc      480
Lys Val Leu Ile Lys Glu Glu Asp Ala Val Ile Tyr Lys Asn Gly Ser
145                 150                 155                 160

ttc atc cat tca gtg ccc cgg cat gaa gta cct gat att cta gaa gta      528
Phe Ile His Ser Val Pro Arg His Glu Val Pro Asp Ile Leu Glu Val
                165                 170                 175

cac ctg cct cat gct cag ccc cag gat gct gga gtg tac tcg gcc agg      576
His Leu Pro His Ala Gln Pro Gln Asp Ala Gly Val Tyr Ser Ala Arg
            180                 185                 190

tat ata gga gga aac ctc ttc acc tcg gcc ttc acc agg ctg ata gtc      624
Tyr Ile Gly Gly Asn Leu Phe Thr Ser Ala Phe Thr Arg Leu Ile Val
        195                 200                 205

cgg aga tgt gaa gcc cag aag tgg gga cct gaa tgc aac cat ctc tgt      672
Arg Arg Cys Glu Ala Gln Lys Trp Gly Pro Glu Cys Asn His Leu Cys
    210                 215                 220

act gct tgt atg aac aat ggt gtc tgc cat gaa gat act gga gaa tgc      720
Thr Ala Cys Met Asn Asn Gly Val Cys His Glu Asp Thr Gly Glu Cys
225                 230                 235                 240

att tgc cct cct ggg ttt atg gga agg acg tgt gag aag gct tgt gaa      768
Ile Cys Pro Pro Gly Phe Met Gly Arg Thr Cys Glu Lys Ala Cys Glu
                245                 250                 255

ctg cac acg ttt ggc aga act tgt aaa gaa agg tgc agt gga caa gag      816
Leu His Thr Phe Gly Arg Thr Cys Lys Glu Arg Cys Ser Gly Gln Glu
            260                 265                 270

gga tgc aag tct tat gtg ttc tgt ctc cct gac ccc tat ggg tgt tcc      864
Gly Cys Lys Ser Tyr Val Phe Cys Leu Pro Asp Pro Tyr Gly Cys Ser
        275                 280                 285

tgt gcc aca ggc tgg aag ggt ctg cag tgc aat gaa gca tgc cac cct      912
Cys Ala Thr Gly Trp Lys Gly Leu Gln Cys Asn Glu Ala Cys His Pro
    290                 295                 300

ggt ttt tac ggg cca gat tgt aag ctt agg tgc agc tgc aac aat ggg      960
Gly Phe Tyr Gly Pro Asp Cys Lys Leu Arg Cys Ser Cys Asn Asn Gly
305                 310                 315                 320

gag atg tgt gat cgc ttc caa gga tgt ctc tgc tct cca gga tgg cag     1008
Glu Met Cys Asp Arg Phe Gln Gly Cys Leu Cys Ser Pro Gly Trp Gln
                325                 330                 335

ggg ctc cag tgt gag aga gaa ggc ata ccg agg atg acc cca aag ata 1056
Gly Leu Gln Cys Glu Arg Glu Gly Ile Pro Arg Met Thr Pro Lys Ile
            340                 345                 350

gtg gat ttg cca gat cat ata gaa gta aac agt ggt aaa ttt aat ccc 1104
Val Asp Leu Pro Asp His Ile Glu Val Asn Ser Gly Lys Phe Asn Pro
        355                 360                 365

att tgc aaa gct tct ggc tgg ccg cta cct act aat gaa gaa atg acc 1152
Ile Cys Lys Ala Ser Gly Trp Pro Leu Pro Thr Asn Glu Glu Met Thr
    370                 375                 380

ctg gtg aag ccg gat ggg aca gtg ctc cat cca aaa gac ttt aac cat 1200
Leu Val Lys Pro Asp Gly Thr Val Leu His Pro Lys Asp Phe Asn His
385                 390                 395                 400

acg gat cat ttc tca gta gcc ata ttc acc atc cac cgg atc ctc ccc 1248
Thr Asp His Phe Ser Val Ala Ile Phe Thr Ile His Arg Ile Leu Pro
                405                 410                 415

cct gac tca gga gtt tgg gtc tgc agt gtg aac aca gtg gct ggg atg 1296
Pro Asp Ser Gly Val Trp Val Cys Ser Val Asn Thr Val Ala Gly Met
            420                 425                 430

gtg gaa aag ccc ttc aac att tct gtt aaa gtt ctt cca aag ccc ctg 1344
Val Glu Lys Pro Phe Asn Ile Ser Val Lys Val Leu Pro Lys Pro Leu
        435                 440                 445

aat gcc cca aac gtg att gac act gga cat aac ttt gct gtc atc aac 1392
Asn Ala Pro Asn Val Ile Asp Thr Gly His Asn Phe Ala Val Ile Asn
    450                 455                 460

atc agc tct gag cct tac ttt ggg gat gga cca atc aaa tcc aag aag 1440
Ile Ser Ser Glu Pro Tyr Phe Gly Asp Gly Pro Ile Lys Ser Lys Lys
465                 470                 475                 480

ctt cta tac aaa ccc gtt aat cac tat gag gct tgg caa cat att caa 1488
Leu Leu Tyr Lys Pro Val Asn His Tyr Glu Ala Trp Gln His Ile Gln
                485                 490                 495

gtg aca aat gag att gtt aca ctc aac tat ttg gaa cct cgg aca gaa 1536
Val Thr Asn Glu Ile Val Thr Leu Asn Tyr Leu Glu Pro Arg Thr Glu
            500                 505                 510

tat gaa ctc tgt gtg caa ctg gtc cgt cgt gga gag ggt ggg gaa ggg 1584
Tyr Glu Leu Cys Val Gln Leu Val Arg Arg Gly Glu Gly Gly Glu Gly
        515                 520                 525

cat cct gga cct gtg aga cgc ttc aca aca gct tct atc gga ctc cct 1632
His Pro Gly Pro Val Arg Arg Phe Thr Thr Ala Ser Ile Gly Leu Pro
    530                 535                 540

cct cca aga ggt cta aat ctc ctg cct aaa agt cag acc act cta aat 1680
Pro Pro Arg Gly Leu Asn Leu Leu Pro Lys Ser Gln Thr Thr Leu Asn
545                 550                 555                 560

ttg acc tgg caa cca ata ttt cca agc tcg gaa gat gac ttt tat gtt 1728
Leu Thr Trp Gln Pro Ile Phe Pro Ser Ser Glu Asp Asp Phe Tyr Val
                565                 570                 575

gaa gtg gag aga agg tct gtg caa aaa agt gat cag cag aat att aaa 1776
Glu Val Glu Arg Arg Ser Val Gln Lys Ser Asp Gln Gln Asn Ile Lys
            580                 585                 590

gtt cca ggc aac ttg act tcg gtg cta ctt aac aac tta cat ccc agg 1824
Val Pro Gly Asn Leu Thr Ser Val Leu Leu Asn Asn Leu His Pro Arg
        595                 600                 605

gag cag tac gtg gtc cga gct aga gtc aac acc aag gcc cag ggg gaa 1872
Glu Gln Tyr Val Val Arg Ala Arg Val Asn Thr Lys Ala Gln Gly Glu
    610                 615                 620

tgg agt gaa gat ctc act gct tgg acc ctt agt gac att ctt cct cct 1920
Trp Ser Glu Asp Leu Thr Ala Trp Thr Leu Ser Asp Ile Leu Pro Pro
625                 630                 635                 640

caa cca gaa aac atc aag att tcc aac att aca cac tcc tca gct gtg 1968
Gln Pro Glu Asn Ile Lys Ile Ser Asn Ile Thr His Ser Ser Ala Val
                645                 650                 655

att tct tgg aca ata ttg gat ggc tat tct att tct tct att act atc 2016
Ile Ser Trp Thr Ile Leu Asp Gly Tyr Ser Ile Ser Ser Ile Thr Ile
            660                 665                 670

cgt tac aag gtt caa ggc aag aat gaa gac cag cac gtt gat gtg aag 2064
Arg Tyr Lys Val Gln Gly Lys Asn Glu Asp Gln His Val Asp Val Lys
        675                 680                 685

ata aag aat gcc acc atc act cag tat cag ctc aag ggc cta gag cct 2112
Ile Lys Asn Ala Thr Ile Thr Gln Tyr Gln Leu Lys Gly Leu Glu Pro
    690                 695                 700

gaa aca gca tac cag gtg gac att ttt gca gag aac aac ata ggg tca 2160
Glu Thr Ala Tyr Gln Val Asp Ile Phe Ala Glu Asn Asn Ile Gly Ser
705                 710                 715                 720

agc aac cca gcc ttt tct cat gaa ctg gtg acc ctc cca gaa tct caa 2208
Ser Asn Pro Ala Phe Ser His Glu Leu Val Thr Leu Pro Glu Ser Gln
                725                 730                 735

gca cca gcg gac ctc gga ggg ggg aag atg ctg ctt ata gcc atc ctt 2256
Ala Pro Ala Asp Leu Gly Gly Gly Lys Met Leu Leu Ile Ala Ile Leu
            740                 745                 750

ggc tct gct gga atg acc tgc ctg act gtg ctg ttg gcc ttt ctg atc 2304
Gly Ser Ala Gly Met Thr Cys Leu Thr Val Leu Leu Ala Phe Leu Ile
        755                 760                 765

ata ttg caa ttg aag agg gca aat gtg caa agg aga atg gcc caa gcc 2352
Ile Leu Gln Leu Lys Arg Ala Asn Val Gln Arg Arg Met Ala Gln Ala
    770                 775                 780

ttc caa aac gtg agg gaa gaa cca gct gtg cag ttc aac tca ggg act 2400
Phe Gln Asn Val Arg Glu Glu Pro Ala Val Gln Phe Asn Ser Gly Thr
785                 790                 795                 800

ctg gcc cta aac agg aag gtc aaa aac aac cca gat cct aca att tat 2448
Leu Ala Leu Asn Arg Lys Val Lys Asn Asn Pro Asp Pro Thr Ile Tyr
                805                 810                 815

cca gtg ctt gac tgg aat gac atc aaa ttt caa gat gtg att ggg gag 2496
Pro Val Leu Asp Trp Asn Asp Ile Lys Phe Gln Asp Val Ile Gly Glu
            820                 825                 830

ggc aat ttt ggc caa gtt ctt aag gcg cgc atc aag aag gat ggg tta 2544
Gly Asn Phe Gly Gln Val Leu Lys Ala Arg Ile Lys Lys Asp Gly Leu

        835                 840                 845

tgg atg gat gct gcc atc aaa aga atg aaa gaa tat gcc tcc aaa gat 2592
Trp Met Asp Ala Ala Ile Lys Arg Met Lys Glu Tyr Ala Ser Lys Asp
    850                 855                 860

gat cac agg gac ttt gca gga gaa ctg gaa gtt ctt tgt aaa ctt gga 2640
Asp His Arg Asp Phe Ala Gly Glu Leu Glu Val Leu Cys Lys Leu Gly
865                 870                 875                 880

cac cat cca aac atc atc aat ctc tta gga gca tgt gaa cat cga ggc 2688
His His Pro Asn Ile Ile Asn Leu Leu Gly Ala Cys Glu His Arg Gly
                885                 890                 895

tac ttg tac ctg gcc att gag tac gcg ccc cat gga aac ctt ctg gac 2736
Tyr Leu Tyr Leu Ala Ile Glu Tyr Ala Pro His Gly Asn Leu Leu Asp
            900                 905                 910

ttc ctt cgc aag agc cgt gtg ctg gag acg gac cca gca ttt gcc att 2784
Phe Leu Arg Lys Ser Arg Val Leu Glu Thr Asp Pro Ala Phe Ala Ile
        915                 920                 925

gcc aat agc acc gcg tcc aca ctg tcc tcc cag cag ctc ctt cac ttc 2832
Ala Asn Ser Thr Ala Ser Thr Leu Ser Ser Gln Gln Leu Leu His Phe
    930                 935                 940

gct gcc gac gtg gcc cgg ggc atg gac tac ttg agc caa aaa cag ttt 2880
Ala Ala Asp Val Ala Arg Gly Met Asp Tyr Leu Ser Gln Lys Gln Phe
945                 950                 955                 960

atc cac agg gat ctg gct gcc aga aac att tta gtt ggt gaa aac tat 2928
Ile His Arg Asp Leu Ala Ala Arg Asn Ile Leu Val Gly Glu Asn Tyr
                965                 970                 975

gtg gca aaa ata gca gat ttt gga ttg tcc cga ggt caa gag gtg tat 2976
Val Ala Lys Ile Ala Asp Phe Gly Leu Ser Arg Gly Gln Glu Val Tyr
            980                 985                 990

gtg aaa aag aca atg gga agg ctc cca gtg cgc tgg atg gcc atc gag 3024
Val Lys Lys Thr Met Gly Arg Leu Pro Val Arg Trp Met Ala Ile Glu
        995                 1000                1005

tca ctg aat tac agt gtg tac aca acc aac agt gat gta tgg tcc 3069
Ser Leu Asn Tyr Ser Val Tyr Thr Thr Asn Ser Asp Val Trp Ser
    1010                1015                1020

tat ggt gtg tta cta tgg gag att gtt agc tta gga ggc aca ccc 3114
Tyr Gly Val Leu Leu Trp Glu Ile Val Ser Leu Gly Gly Thr Pro
    1025                1030                1035

tac tgc ggg atg act tgt gca gaa ctc tac gag aag ctg ccc cag 3159
Tyr Cys Gly Met Thr Cys Ala Glu Leu Tyr Glu Lys Leu Pro Gln
    1040                1045                1050

ggc tac aga ctg gag aag ccc ctg aac tgt gat gat gag gtg tat 3204
Gly Tyr Arg Leu Glu Lys Pro Leu Asn Cys Asp Asp Glu Val Tyr
    1055                1060                1065

gat cta atg aga caa tgc tgg cgg gag aag cct tat gag agg cca 3249
Asp Leu Met Arg Gln Cys Trp Arg Glu Lys Pro Tyr Glu Arg Pro
    1070                1075                1080

tca ttt gcc cag ata ttg gtg tcc tta aac aga atg tta gag gag 3294

Ser Phe Ala Gln Ile Leu Val Ser Leu Asn Arg Met Leu Glu Glu
    1085                1090                1095

cga aag acc tac gtg aat acc acg ctt tat gag aag ttt act tat 3339
Arg Lys Thr Tyr Val Asn Thr Thr Leu Tyr Glu Lys Phe Thr Tyr
    1100                1105                1110

gca gga att gac tgt tct gct gaa gaa gcg gcc tag 3375
Ala Gly Ile Asp Cys Ser Ala Glu Glu Ala Ala
    1115                1120

<210> 104
<211> 1124
<212> PRT
<213> Homo sapiens

<400> 104

Met Asp Ser Leu Ala Ser Leu Val Leu Cys Gly Val Ser Leu Leu Leu
1               5                   10                  15

Ser Gly Thr Val Glu Gly Ala Met Asp Leu Ile Leu Ile Asn Ser Leu
            20                  25                  30

Pro Leu Val Ser Asp Ala Glu Thr Ser Leu Thr Cys Ile Ala Ser Gly
        35                  40                  45

Trp Arg Pro His Glu Pro Ile Thr Ile Gly Arg Asp Phe Glu Ala Leu
    50                  55                  60

Met Asn Gln His Gln Asp Pro Leu Glu Val Thr Gln Asp Val Thr Arg
65                  70                  75                  80

Glu Trp Ala Lys Lys Val Val Trp Lys Arg Glu Lys Ala Ser Lys Ile
                85                  90                  95

Asn Gly Ala Tyr Phe Cys Glu Gly Arg Val Arg Gly Glu Ala Ile Arg
            100                 105                 110

Ile Arg Thr Met Lys Met Arg Gln Gln Ala Ser Phe Leu Pro Ala Thr
        115                 120                 125

Leu Thr Met Thr Val Asp Lys Gly Asp Asn Val Asn Ile Ser Phe Lys
    130                 135                 140

Lys Val Leu Ile Lys Glu Glu Asp Ala Val Ile Tyr Lys Asn Gly Ser
145                 150                 155                 160

Phe Ile His Ser Val Pro Arg His Glu Val Pro Asp Ile Leu Glu Val
                165                 170                 175

His Leu Pro His Ala Gln Pro Gln Asp Ala Gly Val Tyr Ser Ala Arg
        180                 185                 190

Tyr Ile Gly Gly Asn Leu Phe Thr Ser Ala Phe Thr Arg Leu Ile Val
    195                 200                 205

Arg Arg Cys Glu Ala Gln Lys Trp Gly Pro Glu Cys Asn His Leu Cys
    210                 215                 220

Thr Ala Cys Met Asn Asn Gly Val Cys His Glu Asp Thr Gly Glu Cys
225                 230                 235                 240

Ile Cys Pro Pro Gly Phe Met Gly Arg Thr Cys Glu Lys Ala Cys Glu
                245                 250                 255

Leu His Thr Phe Gly Arg Thr Cys Lys Glu Arg Cys Ser Gly Gln Glu
            260                 265                 270

Gly Cys Lys Ser Tyr Val Phe Cys Leu Pro Asp Pro Tyr Gly Cys Ser
        275                 280                 285

Cys Ala Thr Gly Trp Lys Gly Leu Gln Cys Asn Glu Ala Cys His Pro
    290                 295                 300

Gly Phe Tyr Gly Pro Asp Cys Lys Leu Arg Cys Ser Cys Asn Asn Gly
305                 310                 315                 320

Glu Met Cys Asp Arg Phe Gln Gly Cys Leu Cys Ser Pro Gly Trp Gln
                325                 330                 335

Gly Leu Gln Cys Glu Arg Glu Gly Ile Pro Arg Met Thr Pro Lys Ile
            340                 345                 350

Val Asp Leu Pro Asp His Ile Glu Val Asn Ser Gly Lys Phe Asn Pro
        355                 360                 365

Ile Cys Lys Ala Ser Gly Trp Pro Leu Pro Thr Asn Glu Glu Met Thr
    370                 375                 380

Leu Val Lys Pro Asp Gly Thr Val Leu His Pro Lys Asp Phe Asn His
385                 390                 395                 400

Thr Asp His Phe Ser Val Ala Ile Phe Thr Ile His Arg Ile Leu Pro
                405                 410                 415

Pro Asp Ser Gly Val Trp Val Cys Ser Val Asn Thr Val Ala Gly Met
            420                 425                 430

Val Glu Lys Pro Phe Asn Ile Ser Val Lys Val Leu Pro Lys Pro Leu
        435                 440                 445

Asn Ala Pro Asn Val Ile Asp Thr Gly His Asn Phe Ala Val Ile Asn
    450                 455                 460

Ile Ser Ser Glu Pro Tyr Phe Gly Asp Gly Pro Ile Lys Ser Lys Lys
465                 470                 475                 480

Leu Leu Tyr Lys Pro Val Asn His Tyr Glu Ala Trp Gln His Ile Gln
                485                 490                 495

Val Thr Asn Glu Ile Val Thr Leu Asn Tyr Leu Glu Pro Arg Thr Glu
            500                 505                 510

Tyr Glu Leu Cys Val Gln Leu Val Arg Arg Gly Glu Gly Gly Glu Gly
        515                 520                 525

His Pro Gly Pro Val Arg Arg Phe Thr Thr Ala Ser Ile Gly Leu Pro
    530                 535                 540

Pro Pro Arg Gly Leu Asn Leu Leu Pro Lys Ser Gln Thr Thr Leu Asn
545                 550                 555                 560

Leu Thr Trp Gln Pro Ile Phe Pro Ser Ser Glu Asp Asp Phe Tyr Val
                565                 570                 575

Glu Val Glu Arg Arg Ser Val Gln Lys Ser Asp Gln Gln Asn Ile Lys
            580                 585                 590

Val Pro Gly Asn Leu Thr Ser Val Leu Leu Asn Asn Leu His Pro Arg
        595                 600                 605

Glu Gln Tyr Val Val Arg Ala Arg Val Asn Thr Lys Ala Gln Gly Glu
    610                 615                 620

Trp Ser Glu Asp Leu Thr Ala Trp Thr Leu Ser Asp Ile Leu Pro Pro
625                 630                 635                 640

Gln Pro Glu Asn Ile Lys Ile Ser Asn Ile Thr His Ser Ser Ala Val
                645                 650                 655

Ile Ser Trp Thr Ile Leu Asp Gly Tyr Ser Ile Ser Ser Ile Thr Ile
            660                 665                 670

Arg Tyr Lys Val Gln Gly Lys Asn Glu Asp Gln His Val Asp Val Lys
        675                 680                 685

Ile Lys Asn Ala Thr Ile Thr Gln Tyr Gln Leu Lys Gly Leu Glu Pro
    690                 695                 700

Glu Thr Ala Tyr Gln Val Asp Ile Phe Ala Glu Asn Asn Ile Gly Ser
705                 710                 715                 720

Ser Asn Pro Ala Phe Ser His Glu Leu Val Thr Leu Pro Glu Ser Gln
                725                 730                 735

Ala Pro Ala Asp Leu Gly Gly Gly Lys Met Leu Leu Ile Ala Ile Leu
            740                 745                 750

Gly Ser Ala Gly Met Thr Cys Leu Thr Val Leu Leu Ala Phe Leu Ile
        755                 760                 765

Ile Leu Gln Leu Lys Arg Ala Asn Val Gln Arg Arg Met Ala Gln Ala
    770                 775                 780

Phe Gln Asn Val Arg Glu Glu Pro Ala Val Gln Phe Asn Ser Gly Thr
785                 790                 795                 800

Leu Ala Leu Asn Arg Lys Val Lys Asn Asn Pro Asp Pro Thr Ile Tyr
                805                 810                 815

Pro Val Leu Asp Trp Asn Asp Ile Lys Phe Gln Asp Val Ile Gly Glu
            820                 825                 830

Gly Asn Phe Gly Gln Val Leu Lys Ala Arg Ile Lys Lys Asp Gly Leu
        835                 840                 845

Trp Met Asp Ala Ala Ile Lys Arg Met Lys Glu Tyr Ala Ser Lys Asp
    850                 855                 860

Asp His Arg Asp Phe Ala Gly Glu Leu Glu Val Leu Cys Lys Leu Gly
865                 870                 875                 880

His His Pro Asn Ile Ile Asn Leu Leu Gly Ala Cys Glu His Arg Gly
                885                 890                 895

Tyr Leu Tyr Leu Ala Ile Glu Tyr Ala Pro His Gly Asn Leu Leu Asp
            900                 905                 910

Phe Leu Arg Lys Ser Arg Val Leu Glu Thr Asp Pro Ala Phe Ala Ile
        915                 920                 925

Ala Asn Ser Thr Ala Ser Thr Leu Ser Ser Gln Gln Leu Leu His Phe

930                 935                 940

Ala Ala Asp Val Ala Arg Gly Met Asp Tyr Leu Ser Gln Lys Gln Phe
945                 950                 955                 960

Ile His Arg Asp Leu Ala Ala Arg Asn Ile Leu Val Gly Glu Asn Tyr
                965                 970                 975

Val Ala Lys Ile Ala Asp Phe Gly Leu Ser Arg Gly Gln Glu Val Tyr
            980                 985                 990

Val Lys Lys Thr Met Gly Arg Leu  Pro Val Arg Trp Met  Ala Ile Glu
        995                 1000                1005

Ser Leu  Asn Tyr Ser Val Tyr  Thr Thr Asn Ser Asp  Val Trp Ser
    1010                 1015                1020

Tyr Gly  Val Leu Leu Trp Glu  Ile Val Ser Leu Gly  Gly Thr Pro
    1025                 1030                1035

Tyr Cys  Gly Met Thr Cys Ala  Glu Leu Tyr Glu Lys  Leu Pro Gln
    1040                 1045                1050

Gly Tyr  Arg Leu Glu Lys Pro  Leu Asn Cys Asp Asp  Glu Val Tyr
    1055                 1060                1065

Asp Leu  Met Arg Gln Cys Trp  Arg Glu Lys Pro Tyr  Glu Arg Pro
    1070                 1075                1080

Ser Phe  Ala Gln Ile Leu Val  Ser Leu Asn Arg Met  Leu Glu Glu
    1085                 1090                1095

Arg Lys  Thr Tyr Val Asn Thr  Thr Leu Tyr Glu Lys  Phe Thr Tyr
    1100                 1105                1110

Ala Gly  Ile Asp Cys Ser Ala  Glu Glu Ala Ala
    1115                 1120

<210>  105
<211>  814
<212>  DNA
<213>  Artificial Sequence

<220>
<223>  Synthetic

<400>  105
gaggaaccga gaggctgaga ctaacccaga aacatccaat tctcaaactg aagctcgcac       60

tctcgcctcc agcatgaact ccttctccac aagcgccttc ggtccagttg ccttctccct      120

gggcctgctc ctggtgttgc ctgctgcctt ccctgcccca gtcttcacac tcgaagattt 180

cgttggggac tggcgacaga cagccggcta caacctggac caagtccttg aacagggagg 240

tgtgtccagt ttgtttcaga atctcggggt gtccgtaact ccgatccaaa ggattgtcct 300

gagcggtgaa aatgggctga agatcgacat ccatgtcatc atcccgtatg aaggtctgag 360

cggcgaccaa atgggccaga tcgaaaaaat ttttaaggtg gtgtaccctg tggatgatca 420

tcactttaag gtgatcctgc actatggcac actggtaatc gacggggtta cgccgaacat 480

gatcgactat ttcggacggc cgtatgaagg catcgccgtg ttcgacggca aaaagatcac 540

tgtaacaggg accctgtgga acggcaacaa aattatcgac gagcgcctga tcaaccccga 600

cggctccctg ctgttccgag taaccatcaa cggagtgacc ggctggcggc tgtgcgaacg 660

cattctggcg taaggctgct gcagctgcat gggtgcctgc tgctgcctgc cttggcctga 720

tggccaggcc agagtgctgc cagtcctctg catgttctgc tcttgtgccc ttctgagccc 780

acaataaagg ctgagctctt atcttgcaaa aggc 814

<210> 106
<211> 984
<212> DNA
<213> Artificial Sequence

<220>
<223> Synthetic

<400> 106
ataaaagccc aggggcaagc ggtccggata acggctagcc tgaggagctg ctgcgacagt 60

ccactacctt tttcgagagt gactcccgtt gtcccaaggc ttcccagagc gaacctgtgc 120

ggctgcaggc accggcgcgt cgagtttccg gcgtccggaa ggaccgagct cttctcgcga 180

atccagtgtt ccgtttccag cccccaatct cagagcggag ccgacagaga gcagggaacc 240

ggcatgaact ccttctccac aagcgccttc ggtccagttg ccttctccct gggcctgctc 300

ctggtgttgc ctgctgcctt ccctgcccca gtcttcacac tcgaagattt cgttggggac 360

tggcgacaga cagccggcta caacctggac caagtccttg aacagggagg tgtgtccagt 420

ttgtttcaga atctcggggt gtccgtaact ccgatccaaa ggattgtcct gagcggtgaa 480

aatgggctga agatcgacat ccatgtcatc atcccgtatg aaggtctgag cggcgaccaa 540

atgggccaga tcgaaaaaat ttttaaggtg gtgtaccctg tggatgatca tcactttaag 600

gtgatcctgc actatggcac actggtaatc gacggggtta cgccgaacat gatcgactat 660

ttcggacggc cgtatgaagg catcgccgtg ttcgacggca aaaagatcac tgtaacaggg 720

accctgtgga acggcaacaa aattatcgac gagcgcctga tcaaccccga cggctccctg 780

ctgttccgag taaccatcaa cggagtgacc ggctggcggc tgtgcgaacg cattctggcg 840

taaggctgct gcagctgcat gggtgcctgc tgctgcctgc cttggcctga tggccaggcc 900

agagtgctgc cagtcctctg catgttctgc tcttgtgccc ttctgagccc acaataaagg 960

ctgagctctt atcttgcaaa aggc 984

<210> 107
<211> 1735
<212> DNA
<213> Artificial Sequence

<220>
<223> Synthetic

<400> 107
gaggaaccga gaggctgaga ctaacccaga aacatccaat tctcaaactg aagctcgcac 60

tctcgcctcc agcatgaaag tctctgccgc ccttctgtgc ctgctgctca tagcagccac 120

cttcattccc caagggctcg ctcacatagg gtgcagcaat cagcgccgaa gtccagaaaa 180

cagtgggaga agatataacc ggattcaaca tgggcaatgt gcctacactt tcattcttcc 240

agaacacgat ggcaactgtc gtgagagtac gacagaccag tacaacacaa acgctctgca 300

gagagatgct ccacacgtgg aaccggattt ctcttcccag aaacttcaac atctggaaca 360

tgtgatggaa aattatactc agtggctgca aaaacttgag aattacattg tggaaaacat 420

gaagtcggag atggcccaga tacagcagaa tgcagttcag aaccacacgg ctaccatgct 480

ggagatagga accagcctcc tctctcagac tgcagagcag accagaaagc tgacagatgt 540

tgagacccag gtactaaatc aaacttctcg acttgagata cagctgctgg agaattcatt 600

atccacctac aagctagaga agcaacttct tcaacagaca aatgaaatct tgaagatcca 660

tgaaaaaaac agtttattag aacataaaat cttagaaatg gaaggaaaac acaaggaaga 720

gttggacacc ttaaaggaag agaaagagaa ccttcaaggc ttggttactc gtcaaacata 780

tataatccag gagctggaaa agcaattaaa cagagctacc accaacaaca gtgtccttca 840

gaagcagcaa ctggagctga tggacacagt ccacaacctt gtcaatcttt gcactaaaga 900

aggtgtttta ctaaagggag gaaaaagaga ggaagagaaa ccatttagag actgtgcaga 960

tgtatatcaa gctggtttta ataaaagtgg aatctacact atttatatta ataatatgcc 1020

agaacccaaa aaggtgtttt gcaatatgga tgtcaatggg ggaggttgga ctgtaataca 1080

acatcgtgaa gatggaagtc tagatttcca aagaggctgg aaggaatata aaatgggttt 1140

tggaaatccc tccggtgaat attggctggg gaatgagttt atttttgcca ttaccagtca 1200

gaggcagtac atgctaagaa ttgagttaat ggactgggaa gggaaccgag cctattcaca 1260

gtatgacaga ttccacatag gaaatgaaaa gcaaaactat aggttgtatt taaaaggtca 1320

cactgggaca gcaggaaaac agagcagcct gatcttacac ggtgctgatt tcagcactaa 1380

agatgctgat aatgacaact gtatgtgcaa atgtgccctc atgttaacag gaggatggtg 1440

gtttgatgct tgtggcccct ccaatctaaa tggaatgttc tatactgcgg gacaaaacca 1500

tggaaaactg aatgggataa agtggcacta cttcaaaggg cccagttact ccttacgttc 1560

cacaactatg atgattcgac ctttagattt ttgaggctgc tgcagctgca tgggtgcctg 1620

ctgctgcctg ccttggcctg atggccaggc cagagtgctg ccagtcctct gcatgttctg 1680

ctcttgtgcc cttctgagcc cacaataaag gctgagctct tatcttgcaa aaggc 1735

<210> 108
<211> 1150
<212> DNA
<213> Artificial Sequence

<220>
<223> Synthetic

<400> 108
gaggaaccga gaggctgaga ctaacccaga aacatccaat tctcaaactg aagctcgcac 60

tctcgcctcc agcatgaaag tctctgccgc ccttctgtgc ctgctgctca tagcagccac 120

cttcattccc caagggctcg ctgggatctt agacttgggc ccgcagatgc ttcgggaact 180

gcaggaaacc aacgcggcgc tgcaggacgt gcgggagctg ctgcggcagc aggtcaggga 240

gatcacgttc ctgaaaaaca cggtgatgga gtgtgacgcg tgcggaggct cccttgtcaa 300

tctttgcact aaagaaggtg ttttactaaa gggaggaaaa agagaggaag agaaaccatt 360

tagagactgt gcagatgtat atcaagctgg ttttaataaa agtggaatct acactattta 420

tattaataat atgccagaac ccaaaaaggt gttttgcaat atggatgtca atgggggagg 480

ttggactgta atacaacatc gtgaagatgg aagtctagat ttccaaagag gctggaagga 540

atataaaatg ggttttggaa atccctccgg tgaatattgg ctggggaatg agtttatttt 600

tgccattacc agtcagaggc agtacatgct aagaattgag ttaatggact gggaagggaa 660

ccgagcctat tcacagtatg acagattcca cataggaaat gaaaagcaaa actataggtt 720

gtatttaaaa ggtcacactg ggacagcagg aaaacagagc agcctgatct tacacggtgc 780

tgatttcagc actaaagatg ctgataatga caactgtatg tgcaaatgtg ccctcatgtt 840

aacaggagga tggtggtttg atgcttgtgg cccctccaat ctaaatggaa tgttctatac 900

tgcgggacaa aaccatggaa aactgaatgg gataaagtgg cactacttca aagggcccag 960

ttactcctta cgttccacaa ctatgatgat tcgaccttta gatttttgag gctgctgcag 1020

ctgcatgggt gcctgctgct gcctgccttg gcctgatggc caggccagag tgctgccagt 1080

cctctgcatg ttctgctctt gtgcccttct gagcccacaa taaaggctga gctcttatct 1140

tgcaaaaggc 1150

<210> 109

<211>  817
<212>  DNA
<213>  Artificial Sequence

<220>
<223>  Synthetic

<400>  109
catatcttca ttcttcactc tttccccccc cccccttatc ctcctatcta atcctctcat      60

ctctctcctc ctcttcctcc aacaacaact aacaatgaaa gtctctgccg cccttctgtg     120

cctgctgctc atagcagcca ccttcattcc ccaagggctc gctgtcttca cactcgaaga     180

tttcgttggg gactggcgac agacagccgg ctacaacctg gaccaagtcc ttgaacaggg     240

aggtgtgtcc agtttgtttc agaatctcgg ggtgtccgta actccgatcc aaaggattgt     300

cctgagcggt gaaaatgggc tgaagatcga catccatgtc atcatcccgt atgaaggtct     360

gagcggcgac caaatgggcc agatcgaaaa aatttttaag gtggtgtacc ctgtggatga     420

tcatcacttt aaggtgatcc tgcactatgg cacactggta atcgacgggg ttacgccgaa     480

catgatcgac tatttcggac ggccgtatga aggcatcgcc gtgttcgacg gcaaaaagat     540

cactgtaaca gggaccctgt ggaacggcaa caaaattatc gacgagcgcc tgatcaaccc     600

cgacggctcc ctgctgttcc gagtaaccat caacggagtg accggctggc ggctgtgcga     660

acgcattctg gcgtaaggct gctgcagctg catgggtgcc tgctgctgcc tgccttggcc     720

tgatggccag gccagagtgc tgccagtcct ctgcatgttc tgctcttgtg cccttctgag     780

cccacaataa aggctgagct cttatcttgc aaaaggc                              817


<210>  110
<211>  978
<212>  DNA
<213>  Artificial Sequence

<220>
<223>  Synthetic

<400>  110
gtggcagctc acagctattg tggtgggaaa gggagggtgg ttggtggatg tcacagcttg      60

ggctttatct cccccagcag tggggactcc acagcccctg ggctacataa cagcaagaca     120

gtccggagct gtagcagacc tgattgagcc tttgcagcag ctgagagcat ggcctagggt     180

gggcggcacc attgtccagc agctgagttt cccagggacc ttggagatag ccgcagccct     240

catttgcagg ggaagatgaa agtctctgcc gcccttctgt gcctgctgct catagcagcc     300

accttcattc cccaagggct cgctgtcttc acactcgaag atttcgttgg ggactggcga     360

cagacagccg gctacaacct ggaccaagtc cttgaacagg gaggtgtgtc cagtttgttt     420

cagaatctcg gggtgtccgt aactccgatc caaaggattg tcctgagcgg tgaaaatggg     480

ctgaagatcg acatccatgt catcatcccg tatgaaggtc tgagcggcga ccaaatgggc     540

cagatcgaaa aaatttttaa ggtggtgtac cctgtggatg atcatcactt taaggtgatc 600

ctgcactatg gcacactggt aatcgacggg gttacgccga acatgatcga ctatttcgga 660

cggccgtatg aaggcatcgc cgtgttcgac ggcaaaaaga tcactgtaac agggaccctg 720

tggaacggca acaaaattat cgacgagcgc ctgatcaacc ccgacggctc cctgctgttc 780

cgagtaacca tcaacggagt gaccggctgg cggctgtgcg aacgcattct ggcgtaaggc 840

tgctgcagct gcatgggtgc ctgctgctgc ctgccttggc ctgatggcca ggccagagtg 900

ctgccagtcc tctgcatgtt ctgctcttgt gcccttctga gcccacaata aaggctgagc 960

tcttatcttg caaaaggc 978

<210> 111
<211> 975
<212> DNA
<213> Artificial Sequence

<220>
<223> Synthetic

<400> 111
gtggcagctc acagctattg tggtgggaaa gggagggtgg ttggtggatg tcacagcttg 60

ggctttatct cccccagcag tggggactcc acagcccctg ggctacataa cagcaagaca 120

gtccggagct gtagcagacc tgattgagcc tttgcagcag ctgagagcat ggcctagggt 180

gggcggcacc attgtccagc agctgagttt cccagggacc ttggagatag ccgcagccct 240

catttgcagg ggaagatgat tcctgccaga tttgccgggg tgctgcttgc tctggccctc 300

attttgccag ggaccctttg tgtcttcaca ctcgaagatt tcgttgggga ctggcgacag 360

acagccggct acaacctgga ccaagtcctt gaacagggag gtgtgtccag tttgtttcag 420

aatctcgggg tgtccgtaac tccgatccaa aggattgtcc tgagcggtga aaatgggctg 480

aagatcgaca tccatgtcat catcccgtat gaaggtctga gcggcgacca aatgggccag 540

atcgaaaaaa tttttaaggt ggtgtaccct gtggatgatc atcactttaa ggtgatcctg 600

cactatggca cactggtaat cgacggggtt acgccgaaca tgatcgacta tttcggacgg 660

ccgtatgaag gcatcgccgt gttcgacggc aaaaagatca ctgtaacagg gaccctgtgg 720

aacggcaaca aaattatcga cgagcgcctg atcaaccccg acggctccct gctgttccga 780

gtaaccatca acggagtgac cggctggcgg ctgtgcgaac gcattctggc gtaaggctgc 840

tgcagctgca tgggtgcctg ctgctgcctg ccttggcctg atggccaggc cagagtgctg 900

ccagtcctct gcatgttctg ctcttgtgcc cttctgagcc cacaataaag gctgagctct 960

tatcttgcaa aaggc 975

<210> 112

<211>  778
<212>  DNA
<213>  Artificial Sequence

<220>
<223>  Synthetic

<400>  112
gggaaataag agagaaaaga agagtaagaa gaaatataag accccggcgc cgccaccatg      60

aaagtctctg ccgcccttct gtgcctgctg ctcatagcag ccaccttcat tccccaaggg     120

ctcgctgtct tcacactcga agatttcgtt ggggactggc gacagacagc cggctacaac     180

ctggaccaag tccttgaaca gggaggtgtg tccagtttgt ttcagaatct cggggtgtcc     240

gtaactccga tccaaaggat tgtcctgagc ggtgaaaatg ggctgaagat cgacatccat     300

gtcatcatcc cgtatgaagg tctgagcggc gaccaaatgg gccagatcga aaaaattttt     360

aaggtggtgt accctgtgga tgatcatcac tttaaggtga tcctgcacta tggcacactg     420

gtaatcgacg gggttacgcc gaacatgatc gactatttcg gacggccgta tgaaggcatc     480

gccgtgttcg acggcaaaaa gatcactgta acagggaccc tgtggaacgg caacaaaatt     540

atcgacgagc gcctgatcaa ccccgacggc tccctgctgt tccgagtaac catcaacgga     600

gtgaccggct ggcggctgtg cgaacgcatt ctggcgtaat aataggctgg agcctcggtg     660

gcctagcttc ttgccccttg ggcctccccc cagcccctcc tccccttcct gcacccgtac     720

cccctccata aagtaggaaa cactacagtg gtctttgaat aaagtctgag tgggcggc       778


<210>  113
<211>  796
<212>  DNA
<213>  Artificial Sequence

<220>
<223>  Synthetic

<400>  113
gaggaaccga gaggctgaga ctaacccaga aacatccaat tctcaaactg aagctcgcac      60

tctcgccgcc accatgaaag tctctgccgc ccttctgtgc ctgctgctca tagcagccac     120

cttcattccc caagggctcg ctgtcttcac actcgaagat ttcgttgggg actggcgaca     180

gacagccggc tacaacctgg accaagtcct tgaacaggga ggtgtgtcca gtttgtttca     240

gaatctcggg gtgtccgtaa ctccgatcca aaggattgtc ctgagcggtg aaaatgggct     300

gaagatcgac atccatgtca tcatcccgta tgaaggtctg agcggcgacc aaatgggcca     360

gatcgaaaaa atttttaagg tggtgtaccc tgtggatgat catcacttta aggtgatcct     420

gcactatggc acactggtaa tcgacggggt tacgccgaac atgatcgact atttcggacg     480

gccgtatgaa ggcatcgccg tgttcgacgg caaaaagatc actgtaacag ggaccctgtg     540

gaacggcaac aaaattatcg acgagcgcct gatcaacccc gacggctccc tgctgttccg     600

agtaaccatc aacggagtga ccggctggcg gctgtgcgaa cgcattctgg cgtaaggctg 660

ctgcagctgc atgggtgcct gctgctgcct gccttggcct gatggccagg ccagagtgct 720

gccagtcctc tgcatgttct gctcttgtgc ccttctgagc ccacaataaa ggctgagctc 780

ttatcttgca aaaggc 796

<210> 114
<211> 1144
<212> DNA
<213> Artificial Sequence

<220>
<223> Synthetic

<400> 114
gaggaaccga gaggctgaga ctaacccaga aacatccaat tctcaaactg aagctcgcac 60

tctcgcctcc agcatgaaag tctctgccgc ccttctgtgc ctgctgctca tagcagccac 120

cttcattccc caagggctcg ctgggatctt agaggaagac ccggccgcct gcgagtccct 180

ggtgaaattc caagccaaag tggaggggct gctgcaggcc ctgaccagga aactggaagc 240

tgtgagtaag cggctggcca tcctggagaa cacagttgtc ggctcccttg tcaatcttgc 300

cactaaagaa ggtgttttac taaagggagg aaaaagagag gaagagaaac catttagaga 360

ctgtgcagat gtatatcaag ctggttttaa taaaagtgga atctacacta tttatattaa 420

taatatgcca gaacccaaaa aggtgttttg caatatggat gtcaatgggg gaggttggac 480

tgtaatacaa catcgtgaag atggaagtct agatttccaa agaggctgga aggaatataa 540

aatgggtttt ggaaatccct ccggtgaata ttggctgggg aatgagttta tttttgccat 600

taccagtcag aggcagtaca tgctaagaat tgagttaatg gactgggaag ggaaccgagc 660

ctattcacag tatgacagat tccacatagg aaatgaaaag caaaactata ggttgtattt 720

aaaaggtcac actgggacag caggaaaaca gagcagcctg atcttacacg gtgctgattt 780

cagcactaaa gatgctgata atgacaactg tatgtgcaaa tgtgccctca tgttaacagg 840

aggatggtgg tttgatgctt gtggcccctc caatctaaat ggaatgttct atactgcggg 900

acaaaaccat ggaaaactga atgggataaa gtggcactac ttcaaagggc ccagttactc 960

cttacgttcc acaactatga tgattcgacc tttagatttt tgaggctgct gcagctgcat 1020

gggtgcctgc tgctgcctgc cttggcctga tggccaggcc agagtgctgc cagtcctctg 1080

catgttctgc tcttgtgccc ttctgagccc acaataaagg ctgagctctt atcttgcaaa 1140

aggc 1144

<210> 115
<211> 3589
<212> DNA

<213>  Artificial Sequence

<220>
<223>  Synthetic

<400>  115
gaggaaccga gaggctgaga ctaacccaga aacatccaat tctcaaactg aagctcgcac      60

tctcgcctcc agcatggact ctttagccag cttagttctc tgtggagtca gcttgctcct     120

ttctggaact gtggaaggtg ccatggactt gatcttgatc aattccctac ctcttgtatc     180

tgatgctgaa acatctctca cctgcattgc ctctgggtgg cgcccccatg agcccatcac     240

cataggaagg gactttgaag ccttaatgaa ccagcaccag gacccgctgg aagttactca     300

agatgtgacc agagaatggg ctaaaaaagt tgtttggaag agagaaaagg ctagtaagat     360

caatggtgct tatttctgtg aagggcgagt tcgaggagag gcaatcagga tacgaaccat     420

gaagatgcgt caacaagctt ccttcctacc agctacttta actatgactg tggacaaggg     480

agataacgtg aacatatctt tcaaaaaggt attgattaaa gaagaagatg cagtgattta     540

caaaaatggt tccttcatcc attcagtgcc ccggcatgaa gtacctgata ttctagaagt     600

acacctgcct catgctcagc cccaggatgc tggagtgtac tcggccaggt atataggagg     660

aaacctcttc acctcggcct tcaccaggct gatagtccgg agatgtgaag cccagaagtg     720

gggacctgaa tgcaaccatc tctgtactgc ttgtatgaac aatggtgtct gccatgaaga     780

tactggagaa tgcatttgcc ctcctgggtt tatgggaagg acgtgtgaga aggcttgtga     840

actgcacacg tttggcagaa cttgtaaaga aaggtgcagt ggacaagagg gatgcaagtc     900

ttatgtgttc tgtctccctg acccctatgg gtgttcctgt gccacaggct ggaagggtct     960

gcagtgcaat gaagcatgcc accctggttt ttacgggcca gattgtaagc ttaggtgcag    1020

ctgcaacaat ggggagatgt gtgatcgctt ccaaggatgt ctctgctctc caggatggca    1080

ggggctccag tgtgagagag aaggcatacc gaggatgacc ccaaagatag tggatttgcc    1140

agatcatata gaagtaaaca gtggtaaatt taatcccatt tgcaaagctt ctggctggcc    1200

gctacctact aatgaagaaa tgaccctggt gaagccggat gggacagtgc tccatccaaa    1260

agactttaac catacggatc atttctcagt agccatattc accatccaca gaatcctccc    1320

ccctgactca ggagtttggg tctgcagtgt gaacacagtg gctgggatgg tggaaaagcc    1380

cttcaacatt tctgttaaag ttcttccaaa gcccctgaat gccccaaacg tgattgacac    1440

tggacataac tttgctgtca tcaacatcag ctctgagcct tactttgggg atggaccaat    1500

caaatccaag aagcttctat acaaacccgt taatcactat gaggcttggc aacatattca    1560

agtgacaaat gagattgtta cactcaacta tttggaacct cggacagaat atgaactctg    1620

tgtgcaactg gtccgtcgtg gagagggtgg ggaagggcat cctggacctg tgagacgctt    1680

cacaacagct tctatcggac tccctcctcc aagaggtcta aatctcctgc ctaaaagtca    1740

gaccactcta aatttgacct ggcaaccaat atttccaagc tcggaagatg acttttatgt 1800

tgaagtggag agaaggtctg tgcaaaaaag tgatcagcag aatattaaag ttccaggcaa 1860

cttgacttcg gtgctactta acaacttaca tcccagggag cagtacgtgg tccgagctag 1920

agtcaacacc aaggcccagg gggaatggag tgaagatctc actgcttgga cccttagtga 1980

cattcttcct cctcaaccag aaaacatcaa gatttccaac attacacact cctcagctgt 2040

gatttcttgg acaatattgg atggctattc tatttcttct attactatcc gttacaaggt 2100

tcaaggcaag aatgaagacc agcacgttga tgtgaagata aagaatgcca ccatcactca 2160

gtatcagctc aagggcctag agcctgaaac agcataccag gtggacattt ttgcagagaa 2220

caacataggg tcaagcaacc cagccttttc tcatgaactg gtgaccctcc cagaatctca 2280

agcaccagcg gacctcggag gggggaagat gctgcttata gccatccttg gctctgctgg 2340

aatgacctgc ctgactgtgc tgttggcctt tctgatcata ttgcaattga agagggcaaa 2400

tgtgcaaagg agaatggccc aagccttcca aaacgtgagg gaagaaccag ctgtgcagtt 2460

caactcaggg actctggccc taaacaggaa ggtcaaaaac aacccagatc ctacaattta 2520

tccagtgctt gactggaatg acatcaaatt tcaagatgtg attggggagg gcaattttgg 2580

ccaagttctt aaggcgcgca tcaagaagga tgggttacgg atggatgctg ccatcaaaag 2640

aatgaaagaa tatgcctcca aagatgatca cagggacttt gcaggagaac tggaagttct 2700

ttgtaaactt ggacaccatc caaacatcat caatctctta ggagcatgtg aacatcgagg 2760

ctacttgtac ctggccattg agtacgcgcc ccatggaaac cttctggact tccttcgcaa 2820

gagccgtgtg ctggagacgg acccagcatt tgccattgcc aatagcaccg cgtccacact 2880

gtcctcccag cagctccttc acttcgctgc cgacgtggcc cggggcatgg actacttgag 2940

ccaaaaacag tttatccaca gggatctggc tgccagaaac attttagttg gtgaaaacta 3000

tgtggcaaaa atagcagatt ttggattgtc ccgaggtcaa gaggtgtatg tgaaaaagac 3060

aatgggaagg ctcccagtgc gctggatggc catcgagtca ctgaattaca gtgtgtacac 3120

aaccaacagt gatgtatggt cctatggtgt gttactatgg gagattgtta gcttaggagg 3180

cacaccctac tgcgggatga cttgtgcaga actctacgag aagctgcccc agggctacag 3240

actggagaag cccctgaact gtgatgatga ggtgtatgat ctaatgagac aatgctggcg 3300

ggagaagcct tatgagaggc catcatttgc ccagatattg gtgtccttaa acagaatgtt 3360

agaggagcga aagacctacg tgaataccac gctttatgag aagtttactt atgcaggaat 3420

tgactgttct gctgaagaag cggcctaggg ctgctgcagc tgcatgggtg cctgctgctg 3480

cctgccttgg cctgatggcc aggccagagt gctgccagtc ctctgcatgt tctgctcttg 3540

tgcccttctg agcccacaat aaaggctgag ctcttatctt gcaaaaggc 3589

<210>  116
<211>  3589
<212>  DNA
<213>  Artificial Sequence

<220>
<223>  Synthetic

<400>  116
gaggaaccga gaggctgaga ctaacccaga aacatccaat tctcaaactg aagctcgcac      60

tctcgcctcc agcatggact ctttagccag cttagttctc tgtggagtca gcttgctcct     120

ttctggaact gtggaaggtg ccatggactt gatcttgatc aattccctac ctcttgtatc     180

tgatgctgaa acatctctca cctgcattgc ctctgggtgg cgcccccatg agcccatcac     240

cataggaagg gactttgaag ccttaatgaa ccagcaccag gacccgctgg aagttactca     300

agatgtgacc agagaatggg ctaaaaaagt tgtttggaag agagaaaagg ctagtaagat     360

caatggtgct tatttctgtg aagggcgagt tcgaggagag gcaatcagga tacgaaccat     420

gaagatgcgt caacaagctt ccttcctacc agctacttta actatgactg tggacaaggg     480

agataacgtg aacatatctt tcaaaaaggt attgattaaa gaagaagatg cagtgattta     540

caaaaatggt tccttcatcc attcagtgcc ccggcatgaa gtacctgata ttctagaagt     600

acacctgcct catgctcagc cccaggatgc tggagtgtac tcggccaggt atataggagg     660

aaacctcttc acctcggcct tcaccaggct gatagtccgg agatgtgaag cccagaagtg     720

gggacctgaa tgcaaccatc tctgtactgc ttgtatgaac aatggtgtct gccatgaaga     780

tactggagaa tgcatttgcc ctcctgggtt tatgggaagg acgtgtgaga aggcttgtga     840

actgcacacg tttggcagaa cttgtaaaga aaggtgcagt ggacaagagg gatgcaagtc     900

ttatgtgttc tgtctccctg acccctatgg gtgttcctgt gccacaggct ggaagggtct     960

gcagtgcaat gaagcatgcc accctggttt ttacgggcca gattgtaagc ttaggtgcag    1020

ctgcaacaat ggggagatgt gtgatcgctt ccaaggatgt ctctgctctc caggatggca    1080

ggggctccag tgtgagagag aaggcatacc gaggatgacc ccaaagatag tggatttgcc    1140

agatcatata gaagtaaaca gtggtaaatt taatcccatt tgcaaagctt ctggctggcc    1200

gctacctact aatgaagaaa tgaccctggt gaagccggat gggacagtgc tccatccaaa    1260

agactttaac catacggatc atttctcagt agccatattc accatccaca gaatcctccc    1320

ccctgactca ggagtttggg tctgcagtgt gaacacagtg gctgggatgg tggaaaagcc    1380

cttcaacatt tctgttaaag ttcttccaaa gcccctgaat gccccaaacg tgattgacac    1440

tggacataac tttgctgtca tcaacatcag ctctgagcct tactttgggg atggaccaat    1500

caaatccaag aagcttctat acaaacccgt taatcactat gaggcttggc aacatattca    1560

agtgacaaat gagattgtta cactcaacta tttggaacct cggacagaat atgaactctg    1620

tgtgcaactg gtccgtcgtg gagagggtgg ggaagggcat cctggacctg tgagacgctt 1680

cacaacagct tctatcggac tccctcctcc aagaggtcta aatctcctgc ctaaaagtca 1740

gaccactcta aatttgacct ggcaaccaat atttccaagc tcggaagatg acttttatgt 1800

tgaagtggag agaaggtctg tgcaaaaaag tgatcagcag aatattaaag ttccaggcaa 1860

cttgacttcg gtgctactta acaacttaca tcccagggag cagtacgtgg tccgagctag 1920

agtcaacacc aaggcccagg gggaatggag tgaagatctc actgcttgga cccttagtga 1980

cattcttcct cctcaaccag aaaacatcaa gatttccaac attacacact cctcagctgt 2040

gatttcttgg acaatattgg atggctattc tatttcttct attactatcc gttacaaggt 2100

tcaaggcaag aatgaagacc agcacgttga tgtgaagata aagaatgcca ccatcactca 2160

gtatcagctc aagggcctag agcctgaaac agcataccag gtggacattt ttgcagagaa 2220

caacataggg tcaagcaacc cagccttttc tcatgaactg gtgaccctcc cagaatctca 2280

agcaccagcg gacctcggag gggggaagat gctgcttata gccatccttg gctctgctgg 2340

aatgacctgc ctgactgtgc tgttggcctt tctgatcata ttgcaattga agagggcaaa 2400

tgtgcaaagg agaatggccc aagccttcca aaacgtgagg gaagaaccag ctgtgcagtt 2460

caactcaggg actctggccc taaacaggaa ggtcaaaaac aacccagatc ctacaattta 2520

tccagtgctt gactggaatg acatcaaatt tcaagatgtg attggggagg gcaattttgg 2580

ccaagttctt aaggcgcgca tcaagaagga tgggttatgg atggatgctg ccatcaaaag 2640

aatgaaagaa tatgcctcca aagatgatca cagggacttt gcaggagaac tggaagttct 2700

ttgtaaactt ggacaccatc caaacatcat caatctctta ggagcatgtg aacatcgagg 2760

ctacttgtac ctggccattg agtacgcgcc ccatggaaac cttctggact tccttcgcaa 2820

gagccgtgtg ctggagacgg acccagcatt tgccattgcc aatagcaccg cgtccacact 2880

gtcctcccag cagctccttc acttcgctgc cgacgtggcc cggggcatgg actacttgag 2940

ccaaaaacag tttatccaca gggatctggc tgccagaaac attttagttg gtgaaaacta 3000

tgtggcaaaa atagcagatt ttggattgtc ccgaggtcaa gaggtgtatg tgaaaaagac 3060

aatgggaagg ctcccagtgc gctggatggc catcgagtca ctgaattaca gtgtgtacac 3120

aaccaacagt gatgtatggt cctatggtgt gttactatgg gagattgtta gcttaggagg 3180

cacaccctac tgcgggatga cttgtgcaga actctacgag aagctgcccc agggctacag 3240

actggagaag cccctgaact gtgatgatga ggtgtatgat ctaatgagac aatgctggcg 3300

ggagaagcct tatgagaggc catcatttgc ccagatattg gtgtccttaa acagaatgtt 3360

agaggagcga aagacctacg tgaataccac gctttatgag aagtttactt atgcaggaat 3420

tgactgttct gctgaagaag cggcctaggg ctgctgcagc tgcatgggtg cctgctgctg 3480

cctgccttgg cctgatggcc aggccagagt gctgccagtc ctctgcatgt tctgctcttg      3540

tgcccttctg agcccacaat aaaggctgag ctcttatctt gcaaaaggc                 3589

<210>  117
<211>  3207
<212>  DNA
<213>  Homo sapiens

<220>
<221>  CDS
<222>  (1)..(3207)

<400>  117
atg cct cca cga cca tca tca ggt gaa ctg tgg ggc atc cac ttg atg      48
ccc cca aga atc cta gta gaa tgt tta cta cca aat gga atg ata gtg      96
act tta gaa tgc ctc cgt gag gct aca tta ata acc ata aag cat gaa      144
cta ttt aaa gaa gca aga aaa tac ccc ctc cat caa ctt ctt caa gat      192
gaa tct tct tac att ttc gta agt gtt act caa gaa gca gaa agg gaa      240
gaa ttt ttt gat gaa aca aga cga ctt tgt gac ctt cgg ctt ttt caa      288
ccc ttt tta aaa gta att gaa cca gta ggc aac cgt gaa gaa aag atc      336
ctc aat cga gaa att ggt ttt gct atc ggc atg cca gtg tgt gaa ttt      384
gat atg gtt aaa gat cca gaa gta cag gac ttc cga aga aat att ctg      432
aac gtt tgt aaa gaa gct gtg gat ctt agg gac ctc aat tca cct cat      480
agt aga gca atg tat gtc tat cct cca aat gta gaa tct tca cca gaa      528
ttg cca aag cac ata tat aat aaa tta gat aaa ggg caa ata ata gtg      576
gtg atc tgg gta ata gtt tct cca aat aat gac aag cag aag tat act 624
ctg aaa atc aac cat gac tgt gta cca gaa caa gta att gct gaa gca 672
atc agg aaa aaa act cga agt atg ttg cta tcc tct gaa caa cta aaa 720
ctc tgt gtt tta gaa tat cag ggc aag tat att tta aaa gtg tgt gga 768
tgt gat gaa tac ttc cta gaa aaa tat cct ctg agt cag tat aag tat 816
ata aga agc tgt ata atg ctt ggg agg atg ccc aat ttg atg ttg atg 864
gct aaa gaa agc ctt tat tct caa ctg cca atg gac tgt ttt aca atg 912
cca tct tat tcc aga cgc att tcc aca gct aca cca tat atg aat gga 960
gaa aca tct aca aaa tcc ctt tgg gtt ata aat agt gca ctc aga ata 1008
aaa att ctt tgt gca acc tac gtg aat gta aat att cga gac att gat 1056
aag atc tat gtt cga aca ggt atc tac cat gga gga gaa ccc tta tgt 1104
gac aat gtg aac act caa aga gta cct tgt tcc aat ccc agg tgg aat 1152
gaa tgg ctg aat tat gat ata tac att cct gat ctt cct cgt gct gct 1200
cga ctt tgc ctt tcc att tgc tct gtt aaa ggc cga aag ggt gct aaa 1248
gag gaa cac tgt cca ttg gca tgg gga aat ata aac ttg ttt gat tac 1296
aca gac act cta gta tct gga aaa atg gct ttg aat ctt tgg cca gta 1344
cct cat gga tta gaa gat ttg ctg aac cct att ggt gtt act gga tca        1392
aat cca aat aaa gaa act cca tgc tta gag ttg gag ttt gac tgg ttc        1440
agc agt gtg gta aag ttc cca gat atg tca gtg att gaa gag cat gcc        1488
aat tgg tct gta tcc cga gaa gca gga ttt agc tat tcc cac gca gga        1536
ctg agt aac aga cta gct aga gac aat gaa tta agg gaa aat gac aaa        1584
gaa cag ctc aaa gca att tct aca cga gat cct ctc tct gaa atc act        1632
gag cag gag aaa gat ttt cta tgg agt cac aga cac tat tgt gta act        1680
atc ccc gaa att cta ccc aaa ttg ctt ctg tct gtt aaa tgg aat tct        1728
aga gat gaa gta gcc cag atg tat tgc ttg gta aaa gat tgg cct cca        1776
atc aaa cct gaa cag gct atg gaa ctt ctg gac tgt aat tac cca gat        1824
cct atg gtt cga ggt ttt gct gtt cgg tgc ttg gaa aaa tat tta aca        1872
gat gac aaa ctt tct cag tat tta att cag cta gta cag gtc cta aaa        1920
tat gaa caa tat ttg gat aac ttg ctt gtg aga ttt tta ctg aag aaa        1968
gca ttg act aat caa agg att ggg cac ttt ttc ttt tgg cat tta aaa        2016
tct gag atg cac aat aaa aca gtt agc cag agg ttt ggc ctg ctt ttg        2064
gag tcc tat tgt cgt gca tgt ggg atg tat ttg aag cac ctg aat agg        2112
caa gtc gag gca atg gaa aag ctc att aac tta act gac att ctc aaa      2160
cag gag aag aag gat gaa aca caa aag gta cag atg aag ttt tta gtt      2208
gag caa atg agg cga cca gat ttc atg gat gct cta cag ggc ttt ctg      2256
tct cct cta aac cct gct cat caa cta gga aac ctc agg ctt gaa gag      2304
tgt cga att atg tcc tct gca aaa agg cca ctg tgg ttg aat tgg gag      2352
aac cca gac atc atg tca gag tta ctg ttt cag aac aat gag atc atc      2400
ttt aaa aat ggg gat gat tta cgg caa gat atg cta aca ctt caa att      2448
att cgt att atg gaa aat atc tgg caa aat caa ggt ctt gat ctt cga      2496
atg tta cct tat ggt tgt ctg tca atc ggt gac tgt gtg gga ctt att      2544
gag gtg gtg cga aat tct cac act att atg caa att cag tgc aaa ggc      2592
ggc ttg aaa ggt gca ctg cag ttc aac agc cac aca cta cat cag tgg      2640
ctc aaa gac aag aac aaa gga gaa ata tat gat gca gcc att gac ctg      2688
ttt aca cgt tca tgt gct gga tac tgt gta gct acc ttc att ttg gga      2736
att gga gat cgt cac aat agt aac atc atg gtg aaa gac gat gga caa      2784
ctg ttt cat ata gat ttt gga cac ttt ttg gat cac aag aag aaa aaa      2832
ttt ggt tat aaa cga gaa cgt gtg cca ttt gtt ttg aca cag gat ttc 2880
tta ata gtg att agt aaa gga gcc caa gaa tgc aca aag aca aga gaa 2928
ttt gag agg ttt cag gag atg tgt tac aag gct tat cta gct att cga 2976
cag cat gcc aat ctc ttc ata aat ctt ttc tca atg atg ctt ggc tct 3024
gga atg cca gaa cta caa tct ttt gat gac att gca tac att cga 3069
aag acc cta gcc tta gat aaa act gag caa gag gct ttg gag tat 3114
ttc atg aaa caa atg aat gat gca cat cat ggt ggc tgg aca aca 3159
aaa atg gat tgg atc ttc cac aca att aaa cag cat gca ttg aac 3204
tga 3207

<210> 118
<211> 1068
<212> PRT
<213> Homo sapiens

<400> 118

<210>  119
<211>  513
<212>  DNA
<213>  Artificial Sequence

<220>
<223>  Synthetic

<400>  119
gtcttcacac tcgaagattt cgttggggac tggcgacaga cagccggcta caacctggac      60

caagtccttg aacagggagg tgtgtccagt ttgtttcaga atctcggggt gtccgtaact     120

ccgatccaaa ggattgtcct gagcggtgaa aatgggctga agatcgacat ccatgtcatc     180

atcccgtatg aaggtctgag cggcgaccaa atgggccaga tcgaaaaaat ttttaaggtg     240

gtgtaccctg tggatgatca tcactttaag gtgatcctgc actatggcac actggtaatc     300

gacggggtta cgccgaacat gatcgactat ttcggacggc cgtatgaagg catcgccgtg     360

ttcgacggca aaaagatcac tgtaacaggg accctgtgga acggcaacaa aattatcgac     420

gagcgcctga tcaaccccga cggctccctg ctgttccgag taaccatcaa cggagtgacc     480

ggctggcggc tgtgcgaacg cattctggcg taa                                  513


<210>  120
<211>  57
<212>  DNA
<213>  Artificial Sequence

<220>
<223>  Synthetic

<400>  120
gggaaataag agagaaaaga agagtaagaa gaaatataag accccggcgc cgccacc        57


<210>  121
<211>  139
<212>  DNA
<213>  Artificial Sequence

<220>
<223>  Synthetic

<400>  121
taataggctg gagcctcggt ggcctagctt cttgcccctt gggcctcccc ccagcccctc      60

ctccccttcc tgcacccgta ccccctccat aaagtaggaa acactacagt ggtctttgaa     120

taaagtctga gtgggcggc                                                  139


<210>  122
<211>  139
<212>  DNA
<213>  Artificial Sequence

<220>
<223>  Synthetic

<400>  122
tttttttttt tttttttttt tttttttttt tttttttttt tttttttttt tttttttttt      60

tttttttttt tttttttttt tttttttttt tttttttttt tttttttttt tttttttttt     120

ttgcacaaat attactttc                                                    139

<210>  123
<211>  38
<212>  DNA
<213>  Artificial Sequence

<220>
<223>  Synthetic

<400>  123
taatacgact cactataggg aaataagaga gaaaagaa                               38

<210>  124
<211>  139
<212>  DNA
<213>  Artificial Sequence

<220>
<223>  Synthetic

<400>  124
tttttttttt tttttttttt tttttttttt tttttttttt tttttttttt tttttttttt       60

tttttttttt tttttttttt tttttttttt tttttttttt tttttttttt tttttttttt      120

gccgcccact cagacttta                                                   139

<210>  125
<211>  38
<212>  DNA
<213>  Artificial Sequence

<220>
<223>  Synthetic

<400>  125
taatacgact cactataggg tggcagctca cagctatt                               38

<210>  126
<211>  4683
<212>  DNA
<213>  Homo sapiens

<220>
<221>  misc_feature
<223>  Tie-2, wild type

<400>  126
ctctgcccct acagcagcag caaaagcagc agcagaagca acagcaacag ataagtgttt       60

tgatgaattg cgagatggat agggcttgag tgcccccagc cctgctgata ccaaatgcct      120

ttaagataca gcctttccca tcctaatcta caaaggaaac aggaaaaagg aacttaaaac      180

tccctgtgct cagacagaaa tgagactgtt acagcctgct tctgtgctgt tccttcttgc      240

ctctaacttg taaacaagac gtagtaggac gatgctaatg gaaagtcaca aaccgctggg 300
tttttgaaag gatccttggg acctcatgca catttgtgga aactggatgg agagatttgg 360
ggaagcatgg actctttagc cagcttagtt ctctgtggag tcagcttgct cctttctgga 420
actgtggaag gtgccatgga cttgatcttg atcaattccc tacctcttgt atctgatgct 480
gaaacatctc tcacctgcat tgcctctggg tggcgccccc atgagcccat caccatagga 540
agggactttg aagccttaat gaaccagcac caggatccgc tggaagttac tcaagatgtg 600
accagagaat gggctaaaaa agttgtttgg aagagagaaa aggctagtaa gatcaatggt 660
gcttatttct gtgaagggcg agttcgagga gaggcaatca ggatacgaac catgaagatg 720
cgtcaacaag cttccttcct accagctact ttaactatga ctgtggacaa gggagataac 780
gtgaacatat ctttcaaaaa ggtattgatt aaagaagaag atgcagtgat ttacaaaaat 840
ggttccttca tccattcagt gccccggcat gaagtacctg atattctaga agtacacctg 900
cctcatgctc agccccagga tgctggagtg tactcggcca ggtatatagg aggaaacctc 960
ttcacctcgg ccttcaccag gctgatagtc cggagatgtg aagcccagaa gtggggacct 1020
gaatgcaacc atctctgtac tgcttgtatg aacaatggtg tctgccatga agatactgga 1080
gaatgcattt gccctcctgg gtttatggga aggacgtgtg agaaggcttg tgaactgcac 1140
acgtttggca gaacttgtaa agaaaggtgc agtggacaag agggatgcaa gtcttatgtg 1200
ttctgtctcc ctgaccccta tgggtgttcc tgtgccacag gctggaaggg tctgcagtgc 1260
aatgaagcat gccaccctgg tttttacggg ccagattgta agcttaggtg cagctgcaac 1320
aatggggaga tgtgtgatcg cttccaagga tgtctctgct ctccaggatg gcaggggctc 1380
cagtgtgaga gagaaggcat acagaggatg accccaaaga tagtggattt gccagatcat 1440
atagaagtaa acagtggtaa atttaatccc atttgcaaag cttctggctg gccgctacct 1500
actaatgaag aaatgaccct ggtgaagccg gatgggacag tgctccatcc aaaagacttt 1560
aaccatacgg atcatttctc agtagccata ttcaccatcc accggatcct cccccctgac 1620
tcaggagttt gggtctgcag tgtgaacaca gtggctggga tggtggaaaa gcccttcaac 1680
atttctgtta aagttcttcc aaagcccctg aatgccccaa acgtgattga cactggacat 1740
aactttgctg tcatcaacat cagctctgag ccttactttg gggatggacc aatcaaatcc 1800
aagaagcttc tatacaaacc cgttaatcac tatgaggctt ggcaacatat tcaagtgaca 1860
aatgagattg ttacactcaa ctatttggaa cctcggacag aatatgaact ctgtgtgcaa 1920
ctggtccgtc gtggagaggg tggggaaggg catcctggac ctgtgagacg cttcacaaca 1980
gcttctatcg gactccctcc tccaagaggt ctaaatctcc tgcctaaaag tcagaccact 2040
ctaaatttga cctggcaacc aatatttcca agctcggaag atgactttta tgttgaagtg 2100
gagagaaggt ctgtgcaaaa aagtgatcag cagaatatta aagttccagg caacttgact 2160

tcggtgctac ttaacaactt acatcccagg gagcagtacg tggtccgagc tagagtcaac 2220

accaaggccc agggggaatg gagtgaagat ctcactgctt ggacccttag tgacattctt 2280

cctcctcaac cagaaaacat caagatttcc aacattacac actcctcagc tgtgatttct 2340

tggacaatat tggatggcta ttctatttct tctattacta tccgttacaa ggttcaaggc 2400

aagaatgaag accagcacgt tgatgtgaag ataaagaatg ccaccatcac tcagtatcag 2460

ctcaagggcc tagagcctga aacagcatac caggtggaca tttttgcaga gaacaacata 2520

gggtcaagca acccagcctt ttctcatgaa ctggtgaccc tcccagaatc tcaagcacca 2580

gcggacctcg gaggggggaa gatgctgctt atagccatcc ttggctctgc tggaatgacc 2640

tgcctgactg tgctgttggc ctttctgatc atattgcaat tgaagagggc aaatgtgcaa 2700

aggagaatgg cccaagcctt ccaaaacgtg agggaagaac cagctgtgca gttcaactca 2760

gggactctgg ccctaaacag gaaggtcaaa aacaacccag atcctacaat ttatccagtg 2820

cttgactgga atgacatcaa atttcaagat gtgattgggg agggcaattt tggccaagtt 2880

cttaaggcgc gcatcaagaa ggatgggtta cggatggatg ctgccatcaa aagaatgaaa 2940

gaatatgcct ccaaagatga tcacagggac tttgcaggag aactggaagt tctttgtaaa 3000

cttggacacc atccaaacat catcaatctc ttaggagcat gtgaacatcg aggctacttg 3060

tacctggcca ttgagtacgc gccccatgga aaccttctgg acttccttcg caagagccgt 3120

gtgctggaga cggacccagc atttgccatt gccaatagca ccgcgtccac actgtcctcc 3180

cagcagctcc ttcacttcgc tgccgacgtg gcccggggca tggactactt gagccaaaaa 3240

cagtttatcc acagggatct ggctgccaga aacattttag ttggtgaaaa ctatgtggca 3300

aaaatagcag attttggatt gtcccgaggt caagaggtgt atgtgaaaaa gacaatggga 3360

aggctcccag tgcgctggat ggccatcgag tcactgaatt acagtgtgta cacaaccaac 3420

agtgatgtat ggtcctatgg tgtgttacta tgggagattg ttagcttagg aggcacaccc 3480

tactgcggga tgacttgtgc agaactctac gagaagctgc cccagggcta cagactggag 3540

aagcccctga actgtgatga tgaggtgtat gatctaatga gacaatgctg gcgggagaag 3600

ccttatgaga ggccatcatt tgcccagata ttggtgtcct taaacagaat gttagaggag 3660

cgaaagacct acgtgaatac cacgctttat gagaagttta cttatgcagg aattgactgt 3720

tctgctgaag aagcggccta ggacagaaca tctgtatacc ctctgtttcc ctttcactgg 3780

catgggagac ccttgacacc tgctgagaaa acatgcctct gccaaaggat gtgatatata 3840

agtgtacata tgtgctgtac acctgggacc ttcaccactg tagatcccat gcatggatct 3900

atgtagtatg ctctgactct aataggactg tatatactgt tttaagaatg ggctgaaatc 3960

agaatgcctg tttgtggttt catatgcaat aatatatttt tttaaaaatg tggacttcat 4020

aggaaggcgt gagtacaatt agtataatgc ataactcatt gttgtcctag atattttgat 4080

atttaccttt atgttgaatg ctattaaatg ttttcctgtg tcaaagtaaa atattgttaa 4140

taaacctaac aatgaccctg atagtacagg ttaagtgaga gaactatatg aattctaaca 4200

agtcataggt taatatttaa gacactgaaa aatctaagtg atataaatca gattcttctc 4260

tctcaatttt atccctcacc tgtagcagcc agtcccgttt catttagtca tgtgaccact 4320

ctgtcttgtg tttccacagc ctgcaagtca gtccaggatg ctaacatcta aaaatagact 4380

taaatctcat tgcttacaag cctaagaatc tttagagaag tatacataag tttaggataa 4440

aataatggga ttttcttttc ttttctctgg taatattgac ttgtatattt taagaaataa 4500

cagaaagcct gggtgacatt tgggagacat gtgacattta tatattgaat taatatccct 4560

acatgtattg cacattgtaa aaagttttag ttttgatgag ttgtgagttt accttgtata 4620

ctgtaggcac actttgcact gatatatcat gagtgaataa atgtcttgcc tactcacgtc 4680

tca 4683

<210> 127
<211> 1124
<212> PRT
<213> Homo sapiens

<220>
<221> MISC_FEATURE
<223> Tie-2, wild type

<400> 127

<210> 128
<211> 3589
<212> DNA
<213> Artificial Sequence

<220>
<223> Synthetic

<220>
<221> misc_feature
<223> PAN62

<400> 128
gaggaaccga gaggctgaga ctaacccaga aacatccaat tctcaaactg aagctcgcac 60

tctcgcctcc agcatggact ctttagccag cttagttctc tgtggagtca gcttgctcct 120

ttctggaact gtggaaggtg ccatggactt gatcttgatc aattccctac ctcttgtatc 180

tgatgctgaa acatctctca cctgcattgc ctctgggtgg cgcccccatg agcccatcac 240

cataggaagg gactttgaag ccttaatgaa ccagcaccag gacccgctgg aagttactca 300

agatgtgacc agagaatggg ctaaaaaagt tgtttggaag agagaaaagg ctagtaagat 360

caatggtgct tatttctgtg aagggcgagt tcgaggagag gcaatcagga tacgaaccat 420

gaagatgcgt caacaagctt ccttcctacc agctacttta actatgactg tggacaaggg 480

agataacgtg aacatatctt tcaaaaaggt attgattaaa gaagaagatg cagtgattta 540

caaaaatggt tccttcatcc attcagtgcc ccggcatgaa gtacctgata ttctagaagt 600

acacctgcct catgctcagc cccaggatgc tggagtgtac tcggccaggt atataggagg 660

aaacctcttc acctcggcct tcaccaggct gatagtccgg agatgtgaag cccagaagtg 720

gggacctgaa tgcaaccatc tctgtactgc ttgtatgaac aatggtgtct gccatgaaga 780

tactggagaa tgcatttgcc ctcctgggtt tatgggaagg acgtgtgaga aggcttgtga 840

actgcacacg tttggcagaa cttgtaaaga aaggtgcagt ggacaagagg gatgcaagtc 900

ttatgtgttc tgtctccctg acccctatgg gtgttcctgt gccacaggct ggaagggtct 960

gcagtgcaat gaagcatgcc accctggttt ttacgggcca gattgtaagc ttaggtgcag 1020

ctgcaacaat ggggagatgt gtgatcgctt ccaaggatgt ctctgctctc caggatggca 1080

ggggctccag tgtgagagag aaggcatacc gaggatgacc ccaaagatag tggatttgcc 1140

agatcatata gaagtaaaca gtggtaaatt taatcccatt tgcaaagctt ctggctggcc 1200

gctacctact aatgaagaaa tgaccctggt gaagccggat gggacagtgc tccatccaaa 1260

agactttaac catacggatc atttctcagt agccatattc accatccaca gaatcctccc 1320

ccctgactca ggagtttggg tctgcagtgt gaacacagtg gctgggatgg tggaaaagcc 1380

cttcaacatt tctgttaaag ttcttccaaa gcccctgaat gccccaaacg tgattgacac 1440

tggacataac tttgctgtca tcaacatcag ctctgagcct tactttgggg atggaccaat 1500

caaatccaag aagcttctat acaaacccgt taatcactat gaggcttggc aacatattca 1560

agtgacaaat gagattgtta cactcaacta tttggaacct cggacagaat atgaactctg 1620

tgtgcaactg gtccgtcgtg gagagggtgg ggaagggcat cctggacctg tgagacgctt 1680

cacaacagct tctatcggac tccctcctcc aagaggtcta aatctcctgc ctaaaagtca 1740

gaccactcta aatttgacct ggcaaccaat atttccaagc tcggaagatg acttttatgt 1800

tgaagtggag agaaggtctg tgcaaaaaag tgatcagcag aatattaaag ttccaggcaa 1860

cttgacttcg gtgctactta acaacttaca tcccagggag cagtacgtgg tccgagctag 1920

agtcaacacc aaggcccagg gggaatggag tgaagatctc actgcttgga cccttagtga 1980

cattcttcct cctcaaccag aaaacatcaa gatttccaac attacacact cctcagctgt 2040

gatttcttgg acaatattgg atggctattc tatttcttct attactatcc gttacaaggt 2100

tcaaggcaag aatgaagacc agcacgttga tgtgaagata aagaatgcca ccatcactca 2160

gtatcagctc aagggcctag agcctgaaac agcataccag gtggacattt ttgcagagaa 2220

caacataggg tcaagcaacc cagccttttc tcatgaactg gtgaccctcc cagaatctca 2280

agcaccagcg gacctcggag gggggaagat gctgcttata gccatccttg gctctgctgg 2340

aatgacctgc ctgactgtgc tgttggcctt tctgatcata ttgcaattga agagggcaaa 2400

tgtgcaaagg agaatggccc aagccttcca aaacgtgagg gaagaaccag ctgtgcagtt 2460

caactcaggg actctggccc taaacaggaa ggtcaaaaac aacccagatc ctacaattta 2520

tccagtgctt gactggaatg acatcaaatt tcaagatgtg attggggagg gcaattttgg 2580

ccaagttctt aaggcgcgca tcaagaagga tgggttacgg atggatgctg ccatcaaaag 2640

aatgaaagaa tatgcctcca aagatgatca cagggacttt gcaggagaac tggaagttct 2700

ttgtaaactt ggacaccatc caaacatcat caatctctta ggagcatgtg aacatcgagg 2760

ctacttgtac ctggccattg agtacgcgcc ccatggaaac cttctggact tcttccgcaa 2820

gagccgtgtg ctggagacgg acccagcatt tgccattgcc aatagcaccg cgtccacact 2880

gtcctcccag cagctccttc acttcgctgc cgacgtggcc cggggcatgg actacttgag 2940

ccaaaaacag tttatccaca gggatctggc tgccagaaac attttagttg gtgaaaacta 3000

tgtggcaaaa atagcagatt ttggattgtc ccgaggtcaa gaggtgtatg tgaaaaagac 3060

aatgggaagg ctcccagtgc gctggatggc catcgagtca ctgaattaca gtgtgtacac 3120

aaccaacagt gatgtatggt cctatggtgt gttactatgg gagattgtta gcttaggagg 3180

cacaccctac tgcgggatga cttgtgcaga actctacgag aagctgcccc agggctacag 3240

actggagaag cccctgaact gtgatgatga ggtgtatgat ctaatgagac aatgctggcg 3300

ggagaagcct tatgagaggc catcatttgc ccagatattg gtgtccttaa acagaatgtt 3360

agaggagcga aagacctacg tgaataccac gctttatgag aagtttactt atgcaggaat 3420

tgactgttct gctgaagaag cggcctaggg ctgctgcagc tgcatgggtg cctgctgctg 3480

cctgccttgg cctgatggcc aggccagagt gctgccagtc ctctgcatgt tctgctcttg 3540

tgcccttctg agcccacaat aaaggctgag ctcttatctt gcaaaaggc 3589

<210> 129
<211> 3589
<212> DNA
<213> Artificial Sequence

<220>
<223> Synthetic

<220>
<221> misc_feature
<223> PAN63

<400> 129
gaggaaccga gaggctgaga ctaacccaga aacatccaat tctcaaactg aagctcgcac 60

tctcgcctcc agcatggact ctttagccag cttagttctc tgtggagtca gcttgctcct 120

ttctggaact gtggaaggtg ccatggactt gatcttgatc aattccctac ctcttgtatc 180

tgatgctgaa acatctctca cctgcattgc ctctgggtgg cgcccccatg agcccatcac 240

cataggaagg gactttgaag ccttaatgaa ccagcaccag gacccgctgg aagttactca 300

agatgtgacc agagaatggg ctaaaaaagt tgtttggaag agagaaaagg ctagtaagat 360

caatggtgct tatttctgtg aagggcgagt tcgaggagag gcaatcagga tacgaaccat 420

gaagatgcgt caacaagctt ccttcctacc agctacttta actatgactg tggacaaggg 480

agataacgtg aacatatctt tcaaaaaggt attgattaaa gaagaagatg cagtgattta 540

caaaaatggt tccttcatcc attcagtgcc ccggcatgaa gtacctgata ttctagaagt 600

acacctgcct catgctcagc cccaggatgc tggagtgtac tcggccaggt atataggagg 660

aaacctcttc acctcggcct tcaccaggct gatagtccgg agatgtgaag cccagaagtg 720

gggacctgaa tgcaaccatc tctgtactgc ttgtatgaac aatggtgtct gccatgaaga 780

tactggagaa tgcatttgcc ctcctgggtt tatgggaagg acgtgtgaga aggcttgtga 840

actgcacacg tttggcagaa cttgtaaaga aaggtgcagt ggacaagagg gatgcaagtc 900

ttatgtgttc tgtctccctg acccctatgg gtgttcctgt gccacaggct ggaagggtct 960

gcagtgcaat gaagcatgcc accctggttt ttacgggcca gattgtaagc ttaggtgcag 1020

ctgcaacaat ggggagatgt gtgatcgctt ccaaggatgt ctctgctctc caggatggca 1080

ggggctccag tgtgagagag aaggcatacc gaggatgacc ccaaagatag tggatttgcc 1140

agatcatata gaagtaaaca gtggtaaatt taatcccatt tgcaaagctt ctggctggcc 1200

gctacctact aatgaagaaa tgaccctggt gaagccggat gggacagtgc tccatccaaa 1260

agactttaac catacggatc atttctcagt agccatattc accatccaca gaatcctccc 1320

ccctgactca ggagtttggg tctgcagtgt gaacacagtg gctgggatgg tggaaaagcc 1380

cttcaacatt tctgttaaag ttcttccaaa gcccctgaat gccccaaacg tgattgacac 1440

tggacataac tttgctgtca tcaacatcag ctctgagcct tactttgggg atggaccaat 1500

caaatccaag aagcttctat acaaacccgt taatcactat gaggcttggc aacatattca 1560

agtgacaaat gagattgtta cactcaacta tttggaacct cggacagaat atgaactctg 1620

tgtgcaactg gtccgtcgtg gagagggtgg ggaagggcat cctggacctg tgagacgctt 1680

cacaacagct tctatcggac tccctcctcc aagaggtcta aatctcctgc ctaaaagtca 1740

gaccactcta aatttgacct ggcaaccaat atttccaagc tcggaagatg acttttatgt 1800

tgaagtggag agaaggtctg tgcaaaaaag tgatcagcag aatattaaag ttccaggcaa 1860

cttgacttcg gtgctactta acaacttaca tcccagggag cagtacgtgg tccgagctag 1920

agtcaacacc aaggcccagg gggaatggag tgaagatctc actgcttgga cccttagtga 1980

cattcttcct cctcaaccag aaaacatcaa gatttccaac attacacact cctcagctgt 2040

gatttcttgg acaatattgg atggctattc tatttcttct attactatcc gttacaaggt 2100

tcaaggcaag aatgaagacc agcacgttga tgtgaagata aagaatgcca ccatcactca 2160

gtatcagctc aagggcctag agcctgaaac agcataccag gtggacattt ttgcagagaa 2220

caacataggg tcaagcaacc cagccttttc tcatgaactg gtgaccctcc cagaatctca 2280

agcaccagcg gacctcggag gggggaagat gctgcttata gccatccttg gctctgctgg 2340

aatgacctgc ctgactgtgc tgttggcctt tctgatcata ttgcaattga agagggcaaa 2400

tgtgcaaagg agaatggccc aagccttcca aaacgtgagg gaagaaccag ctgtgcagtt 2460

caactcaggg actctggccc taaacaggaa ggtcaaaaac aacccagatc ctacaattta 2520

tccagtgctt gactggaatg acatcaaatt tcaagatgtg attggggagg gcaattttgg 2580

ccaagttctt aaggcgcgca tcaagaagga tgggttatgg atggatgctg ccatcaaaag 2640

aatgaaagaa tatgcctcca aagatgatca cagggacttt gcaggagaac tggaagttct 2700

ttgtaaactt ggacaccatc caaacatcat caatctctta ggagcatgtg aacatcgagg 2760

ctacttgtac ctggccattg agtacgcgcc ccatggaaac cttctggact tcttccgcaa 2820

gagccgtgtg ctggagacgg acccagcatt tgccattgcc aatagcaccg cgtccacact 2880

gtcctcccag cagctccttc acttcgctgc cgacgtggcc cggggcatgg actacttgag 2940

ccaaaaacag tttatccaca gggatctggc tgccagaaac attttagttg gtgaaaacta 3000

tgtggcaaaa atagcagatt ttggattgtc ccgaggtcaa gaggtgtatg tgaaaaagac 3060

aatgggaagg ctcccagtgc gctggatggc catcgagtca ctgaattaca gtgtgtacac 3120

aaccaacagt gatgtatggt cctatggtgt gttactatgg gagattgtta gcttaggagg 3180

cacaccctac tgcgggatga cttgtgcaga actctacgag aagctgcccc agggctacag 3240

actggagaag cccctgaact gtgatgatga ggtgtatgat ctaatgagac aatgctggcg 3300

ggagaagcct tatgagaggc catcatttgc ccagatattg gtgtccttaa acagaatgtt 3360

agaggagcga aagacctacg tgaataccac gctttatgag aagtttactt atgcaggaat 3420

tgactgttct gctgaagaag cggcctaggg ctgctgcagc tgcatgggtg cctgctgctg 3480

cctgccttgg cctgatggcc aggccagagt gctgccagtc ctctgcatgt tctgctcttg 3540

tgcccttctg agcccacaat aaaggctgag ctcttatctt gcaaaaggc 3589

<210> 130
<211> 3589
<212> DNA
<213> Artificial Sequence

<220>
<223> Synthetic


<220>
<221> misc_feature
<223> PAN64

<400> 130
gaggaaccga gaggctgaga ctaacccaga aacatccaat tctcaaactg aagctcgcac 60

tctcgcctcc agcatggact ctttagccag cttagttctc tgtggagtca gcttgctcct 120

ttctggaact gtggaaggtg ccatggactt gatcttgatc aattccctac ctcttgtatc 180

tgatgctgaa acatctctca cctgcattgc ctctgggtgg cgcccccatg agcccatcac 240

cataggaagg gactttgaag ccttaatgaa ccagcaccag gacccgctgg aagttactca 300

agatgtgacc agagaatggg ctaaaaaagt tgtttggaag agagaaaagg ctagtaagat 360

caatggtgct tatttctgtg aagggcgagt tcgaggagag gcaatcagga tacgaaccat 420

gaagatgcgt caacaagctt ccttcctacc agctacttta actatgactg tggacaaggg 480

agataacgtg aacatatctt tcaaaaaggt attgattaaa gaagaagatg cagtgattta 540

caaaaatggt tccttcatcc attcagtgcc ccggcatgaa gtacctgata ttctagaagt 600

acacctgcct catgctcagc cccaggatgc tggagtgtac tcggccaggt atataggagg 660

aaacctcttc acctcggcct tcaccaggct gatagtccgg agatgtgaag cccagaagtg 720

gggacctgaa tgcaaccatc tctgtactgc ttgtatgaac aatggtgtct gccatgaaga 780

tactggagaa tgcatttgcc ctcctgggtt tatgggaagg acgtgtgaga aggcttgtga 840

actgcacacg tttggcagaa cttgtaaaga aaggtgcagt ggacaagagg gatgcaagtc 900

ttatgtgttc tgtctccctg acccctatgg gtgttcctgt gccacaggct ggaagggtct 960

gcagtgcaat gaagcatgcc accctggttt ttacgggcca gattgtaagc ttaggtgcag 1020

ctgcaacaat ggggagatgt gtgatcgctt ccaaggatgt ctctgctctc caggatggca 1080

ggggctccag tgtgagagag aaggcatacc gaggatgacc ccaaagatag tggatttgcc 1140

agatcatata gaagtaaaca gtggtaaatt taatcccatt tgcaaagctt ctggctggcc 1200

gctacctact aatgaagaaa tgaccctggt gaagccggat gggacagtgc tccatccaaa 1260

agactttaac catacggatc atttctcagt agccatattc accatccaca gaatcctccc 1320

ccctgactca ggagtttggg tctgcagtgt gaacacagtg gctgggatgg tggaaaagcc 1380

cttcaacatt tctgttaaag ttcttccaaa gcccctgaat gccccaaacg tgattgacac 1440

tggacataac tttgctgtca tcaacatcag ctctgagcct tactttgggg atggaccaat 1500

caaatccaag aagcttctat acaaacccgt taatcactat gaggcttggc aacatattca 1560

agtgacaaat gagattgtta cactcaacta tttggaacct cggacagaat atgaactctg 1620

tgtgcaactg gtccgtcgtg gagagggtgg ggaagggcat cctggacctg tgagacgctt 1680

cacaacagct tctatcggac tccctcctcc aagaggtcta aatctcctgc ctaaaagtca 1740

gaccactcta aatttgacct ggcaaccaat atttccaagc tcggaagatg acttttatgt 1800

tgaagtggag agaaggtctg tgcaaaaaag tgatcagcag aatattaaag ttccaggcaa 1860

cttgacttcg gtgctactta acaacttaca tcccagggag cagtacgtgg tccgagctag 1920

agtcaacacc aaggcccagg gggaatggag tgaagatctc actgcttgga cccttagtga 1980

cattcttcct cctcaaccag aaaacatcaa gatttccaac attacacact cctcagctgt 2040

gatttcttgg acaatattgg atggctattc tatttcttct attactatcc gttacaaggt 2100

tcaaggcaag aatgaagacc agcacgttga tgtgaagata aagaatgcca ccatcactca 2160

gtatcagctc aagggcctag agcctgaaac agcataccag gtggacattt ttgcagagaa 2220

caacataggg tcaagcaacc cagccttttc tcatgaactg gtgaccctcc cagaatctca 2280

agcaccagcg gacctcggag gggggaagat gctgcttata gccatccttg gctctgctgg 2340

aatgacctgc ctgactgtgc tgttggcctt tctgatcata ttgcaattga agagggcaaa 2400

tgtgcaaagg agaatggccc aagccttcca aaacgtgagg gaagaaccag ctgtgcagtt 2460

caactcaggg actctggccc taaacaggaa ggtcaaaaac aacccagatc ctacaattta 2520

tccagtgctt gactggaatg acatcaaatt tcaagatgtg attggggagg gcaattttgg 2580

ccaagttctt aaggcgcgca tcaagaagga tgggttacgg atggatgctg ccatcaaaag 2640

aatgaaagaa tatgcctcca aagatgatca cagggacttt gcaggagaac tggaagttct 2700

ttgtaaactt ggacaccatc caaacatcat caatctctta ggagcatgtg aacatcgagg 2760

cttcttgtac ctggccattg agtacgcgcc ccatggaaac cttctggact tccttctgaa 2820

gagccgtgtg ctggagacgg acccagcatt tgccattgcc aatagcaccg cgtccacact 2880

gtcctcccag cagctccttc acttcgctgc cgacgtggcc cggggcatgg actacttgag 2940

ccaaaaacag tttatccaca gggatctggc tgccagaaac attttagttg gtgaaaacta 3000

tgtggcaaaa atagcagatt ttggattgtc ccgaggtcaa gaggtgtatg tgaaaaagac 3060

aatgggaagg ctcccagtgc gctggatggc catcgagtca ctgaattaca gtgtgtacac 3120

aaccaacagt gatgtatggt cctatggtgt gttactatgg gagattgtta gcttaggagg 3180

cacaccctac tgcgggatga cttgtgcaga actctacgag aagctgcccc agggctacag 3240

actggagaag cccctgaact gtgatgatga ggtgtatgat ctaatgagac aatgctggcg 3300

ggagaagcct tatgagaggc catcatttgc ccagatattg gtgtccttaa acagaatgtt 3360

agaggagcga aagacctacg tgaataccac gctttatgag aagtttactt atgcaggaat 3420

tgactgttct gctgaagaag cggcctaggg ctgctgcagc tgcatgggtg cctgctgctg 3480

cctgccttgg cctgatggcc aggccagagt gctgccagtc ctctgcatgt tctgctcttg 3540

tgcccttctg agcccacaat aaaggctgag ctcttatctt gcaaaaggc 3589

<210> 131
<211> 3589
<212> DNA
<213> Artificial Sequence

<220>
<223> Synthetic


<220>
<221> misc_feature
<223> PAN65

<400> 131
gaggaaccga gaggctgaga ctaacccaga aacatccaat tctcaaactg aagctcgcac 60

tctcgcctcc agcatggact ctttagccag cttagttctc tgtggagtca gcttgctcct 120

ttctggaact gtggaaggtg ccatggactt gatcttgatc aattccctac ctcttgtatc 180

tgatgctgaa acatctctca cctgcattgc ctctgggtgg cgcccccatg agcccatcac 240

cataggaagg gactttgaag ccttaatgaa ccagcaccag gacccgctgg aagttactca 300

agatgtgacc agagaatggg ctaaaaaagt tgtttggaag agagaaaagg ctagtaagat 360

caatggtgct tatttctgtg aagggcgagt tcgaggagag gcaatcagga tacgaaccat 420

gaagatgcgt caacaagctt ccttcctacc agctacttta actatgactg tggacaaggg 480

agataacgtg aacatatctt tcaaaaaggt attgattaaa gaagaagatg cagtgattta 540
caaaaatggt tccttcatcc attcagtgcc ccggcatgaa gtacctgata ttctagaagt 600
acacctgcct catgctcagc cccaggatgc tggagtgtac tcggccaggt atataggagg 660
aaacctcttc acctcggcct tcaccaggct gatagtccgg agatgtgaag cccagaagtg 720
gggacctgaa tgcaaccatc tctgtactgc ttgtatgaac aatggtgtct gccatgaaga 780
tactggagaa tgcatttgcc ctcctgggtt tatgggaagg acgtgtgaga aggcttgtga 840
actgcacacg tttggcagaa cttgtaaaga aaggtgcagt ggacaagagg gatgcaagtc 900
ttatgtgttc tgtctccctg acccctatgg gtgttcctgt gccacaggct ggaagggtct 960
gcagtgcaat gaagcatgcc accctggttt ttacgggcca gattgtaagc ttaggtgcag 1020
ctgcaacaat ggggagatgt gtgatcgctt ccaaggatgt ctctgctctc caggatggca 1080
ggggctccag tgtgagagag aaggcatacc gaggatgacc ccaaagatag tggatttgcc 1140
agatcatata gaagtaaaca gtggtaaatt taatcccatt tgcaaagctt ctggctggcc 1200
gctacctact aatgaagaaa tgaccctggt gaagccggat gggacagtgc tccatccaaa 1260
agactttaac catacggatc atttctcagt agccatattc accatccaca gaatcctccc 1320
ccctgactca ggagtttggg tctgcagtgt gaacacagtg gctgggatgg tggaaaagcc 1380
cttcaacatt tctgttaaag ttcttccaaa gcccctgaat gccccaaacg tgattgacac 1440
tggacataac tttgctgtca tcaacatcag ctctgagcct tactttgggg atggaccaat 1500
caaatccaag aagcttctat acaaacccgt taatcactat gaggcttggc aacatattca 1560
agtgacaaat gagattgtta cactcaacta tttggaacct cggacagaat atgaactctg 1620
tgtgcaactg gtccgtcgtg gagagggtgg ggaagggcat cctggacctg tgagacgctt 1680
cacaacagct tctatcggac tccctcctcc aagaggtcta aatctcctgc ctaaaagtca 1740
gaccactcta aatttgacct ggcaaccaat atttccaagc tcggaagatg acttttatgt 1800
tgaagtggag agaaggtctg tgcaaaaaag tgatcagcag aatattaaag ttccaggcaa 1860
cttgacttcg gtgctactta acaacttaca tcccagggag cagtacgtgg tccgagctag 1920
agtcaacacc aaggcccagg gggaatggag tgaagatctc actgcttgga cccttagtga 1980
cattcttcct cctcaaccag aaaacatcaa gatttccaac attacacact cctcagctgt 2040
gatttcttgg acaatattgg atggctattc tatttcttct attactatcc gttacaaggt 2100
tcaaggcaag aatgaagacc agcacgttga tgtgaagata aagaatgcca ccatcactca 2160
gtatcagctc aagggcctag agcctgaaac agcataccag gtggacattt ttgcagagaa 2220
caacataggg tcaagcaacc cagccttttc tcatgaactg gtgaccctcc cagaatctca 2280
agcaccagcg gacctcggag gggggaagat gctgcttata gccatccttg gctctgctgg 2340
aatgacctgc ctgactgtgc tgttggcctt tctgatcata ttgcaattga agagggcaaa 2400

tgtgcaaagg agaatggccc aagccttcca aaacgtgagg gaagaaccag ctgtgcagtt 2460

caactcaggg actctggccc taaacaggaa ggtcaaaaac aacccagatc ctacaattta 2520

tccagtgctt gactggaatg acatcaaatt tcaagatgtg attggggagg gcaattttgg 2580

ccaagttctt aaggcgcgca tcaagaagga tgggttacgg atggatgctg ccatcaaaag 2640

aatgaaagaa tatgcctcca aagatgatca cagggacttt gcaggagaac tggaagttct 2700

ttgtaaactt ggacaccatc caaacatcat caatctctta ggagcatgtg aacatcgagg 2760

cttcttgtac ctggccattg agtacgcgcc ccatggaaac cttctggact tccttctgaa 2820

gagccgtgtg ctggagacgg acccagcatt tgccattgcc aatagcaccg cgtccacact 2880

gtcctcccag cagctccttc acttcgctgc cgacgtggcc cggggcatgg actacttgag 2940

ccaaaaacag tttatccaca gggatctggc tgccagaaac attttagttg gtgaaaacta 3000

tgtggcaaaa atagcagatt ttggattgtc ccgaggtcaa gaggtgtatg tgaaaaagac 3060

aatgggaagg ctcccagtgc gctggatggc catcgagtca ctgaattaca gtgtgtacac 3120

aaccaacagt gatgtatggt cctatggtgt gttactatgg gagattgtta gcttaggagg 3180

cacaccctac tgcgggatga cttgtgcaga actctacgag aagctgcccc agggctacag 3240

actggagaag cccctgaact gtgatgatga ggtgtatgat ctaatgagac aatgctggcg 3300

ggagaagcct tatgagaggc catcatttgc ccagatattg gtgtccttaa acagaatgtt 3360

agaggagcga aagacctacg tgaataaccc cctttatgag aagtttactt atgcaggaat 3420

tgactgttct gctgaagaag cggcctaggg ctgctgcagc tgcatgggtg cctgctgctg 3480

cctgccttgg cctgatggcc aggccagagt gctgccagtc ctctgcatgt tctgctcttg 3540

tgcccttctg agcccacaat aaaggctgag ctcttatctt gcaaaaggc 3589

<210> 132
<211> 9259
<212> DNA
<213> Homo sapiens

<220>
<221> misc_feature
<222> (1)..(9259)
<223> PIK3CA, wild type

<400> 132
   agttccggtg ccgccgctgc ggccgctgag gtgtcgggct gctgctgccg cggccgctgg 60
   gactggggct ggggccgccg gcgaggcagg gctcgggccc ggccgggcag ctccggagcg 120
   gcgggggaga ggggccggga ggcgggggcc gtgccgcccg ctctcctctc cctcggcgcc 180
   gccgccgccg cccgcggggc tgggacccga tgcggttaga gccgcggagc ctggaagagc 240
   cccgagcgtt tctgctttgg gacaaccata catctaattc cttaaagtag ttttatatgt 300
   aaaacttgca aagaatcaga acaatgcctc cacgaccatc atcaggtgaa ctgtggggca 360
   tccacttgat gcccccaaga atcctagtag aatgtttact accaaatgga atgatagtga 420
   ctttagaatg cctccgtgag gctacattaa taaccataaa gcatgaacta tttaaagaag 480
   caagaaaata ccccctccat caacttcttc aagatgaatc ttcttacatt ttcgtaagtg 540
   ttactcaaga agcagaaagg gaagaatttt ttgatgaaac aagacgactt tgtgaccttc 600
   ggctttttca acccttttta aaagtaattg aaccagtagg caaccgtgaa gaaaagatcc 660
   tcaatcgaga aattggtttt gctatcggca tgccagtgtg tgaatttgat atggttaaag 720
   atccagaagt acaggacttc cgaagaaata ttctgaacgt ttgtaaagaa gctgtggatc 780
   ttagggacct caattcacct catagtagag caatgtatgt ctatcctcca aatgtagaat 840
   cttcaccaga attgccaaag cacatatata ataaattaga taaagggcaa ataatagtgg 900
   tgatctgggt aatagtttct ccaaataatg acaagcagaa gtatactctg aaaatcaacc 960
   atgactgtgt accagaacaa gtaattgctg aagcaatcag gaaaaaaact cgaagtatgt 1020
   tgctatcctc tgaacaacta aaactctgtg ttttagaata tcagggcaag tatattttaa 1080
   aagtgtgtgg atgtgatgaa tacttcctag aaaaatatcc tctgagtcag tataagtata 1140
   taagaagctg tataatgctt gggaggatgc ccaatttgat gttgatggct aaagaaagcc 1200
   tttattctca actgccaatg gactgtttta caatgccatc ttattccaga cgcatttcca 1260
   cagctacacc atatatgaat ggagaaacat ctacaaaatc cctttgggtt ataaatagtg 1320
   cactcagaat aaaaattctt tgtgcaacct acgtgaatgt aaatattcga gacattgata 1380
   agatctatgt tcgaacaggt atctaccatg gaggagaacc cttatgtgac aatgtgaaca 1440
   ctcaaagagt accttgttcc aatcccaggt ggaatgaatg gctgaattat gatatataca 1500
   ttcctgatct tcctcgtgct gctcgacttt gcctttccat ttgctctgtt aaaggccgaa 1560
   agggtgctaa agaggaacac tgtccattgg catggggaaa tataaacttg tttgattaca 1620
   cagacactct agtatctgga aaaatggctt tgaatctttg gccagtacct catggattag 1680
   aagatttgct gaaccctatt ggtgttactg gatcaaatcc aaataaagaa actccatgct 1740
   tagagttgga gtttgactgg ttcagcagtg tggtaaagtt cccagatatg tcagtgattg 1800
   aagagcatgc caattggtct gtatcccgag aagcaggatt tagctattcc cacgcaggac 1860
   tgagtaacag actagctaga gacaatgaat taaggaaaaa tgacaaagaa cagctcaaag 1920
   caatttctac acgagatcct ctctctgaaa tcactgagca ggagaaagat tttctatgga 1980
   gtcacagaca ctattgtgta actatccccg aaattctacc caaattgctt ctgtctgtta 2040
   aatggaattc tagagatgaa gtagcccaga tgtattgctt ggtaaaagat tggcctccaa 2100
   tcaaacctga acaggctatg gaacttctgg actgtaatta cccagatcct atggttcgag 2160
   gttttgctgt tcggtgcttg gaaaaatatt taacagatga caaactttct cagtatttaa 2220
   ttcagctagt acaggtccta aaatatgaac aatatttgga taacttgctt gtgagatttt 2280
   tactgaagaa agcattgact aatcaaagga ttgggcactt tttcttttgg catttaaaat 2340
   ctgagatgca caataaaaca gttagccaga ggtttggcct gcttttggag tcctattgtc 2400
   gtgcatgtgg gatgtatttg aagcacctga ataggcaagt cgaggcaatg gaaaagctca 2460
   ttaacttaac tgacattctc aaacaggaga agaaggatga aacacaaaag gtacagatga 2520
   agtttttagt tgagcaaatg aggcgaccag atttcatgga tgctctacag ggctttctgt 2580
   ctcctctaaa ccctgctcat caactaggaa acctcaggct tgaagagtgt cgaattatgt 2640
   cctctgcaaa aaggccactg tggttgaatt gggagaaccc agacatcatg tcagagttac 2700
   tgtttcagaa caatgagatc atctttaaaa atggggatga tttacggcaa gatatgctaa 2760
   cacttcaaat tattcgtatt atggaaaata tctggcaaaa tcaaggtctt gatcttcgaa 2820
   tgttacctta tggttgtctg tcaatcggtg actgtgtggg acttattgag gtggtgcgaa 2880
   attctcacac tattatgcaa attcagtgca aaggcggctt gaaaggtgca ctgcagttca 2940
   acagccacac actacatcag tggctcaaag acaagaacaa aggagaaata tatgatgcag 3000
   ccattgacct gtttacacgt tcatgtgctg gatactgtgt agctaccttc attttgggaa 3060
   ttggagatcg tcacaatagt aacatcatgg tgaaagacga tggacaactg tttcatatag 3120
   attttggaca ctttttggat cacaagaaga aaaaatttgg ttataaacga gaacgtgtgc 3180
   catttgtttt gacacaggat ttcttaatag tgattagtaa aggagcccaa gaatgcacaa 3240
   agacaagaga atttgagagg tttcaggaga tgtgttacaa ggcttatcta gctattcgac 3300
   agcatgccaa tctcttcata aatcttttct caatgatgct tggctctgga atgccagaac 3360
   tacaatcttt tgatgacatt gcatacattc gaaagaccct agccttagat aaaactgagc 3420
   aagaggcttt ggagtatttc atgaaacaaa tgaatgatgc acatcatggt ggctggacaa 3480
   caaaaatgga ttggatcttc cacacaatta aacagcatgc attgaactga aaagataact 3540
   gagaaaatga aagctcactc tggattccac actgcactgt taataactct cagcaggcaa 3600
   agaccgattg cataggaatt gcacaatcca tgaacagcat tagaatttac agcaagaaca 3660
   gaaataaaat actatataat ttaaataatg taaacgcaaa cagggtttga tagcacttaa 3720
   actagttcat ttcaaaatta agctttagaa taatgcgcaa tttcatgtta tgccttaagt 3780
   ccaaaaaggt aaactttgaa gattgtttgt atcttttttt aaaaaacaaa acaaaacaaa 3840
   aatccccaaa atatatagaa atgatggaga aggaaaaagt gatggttttt tttgtcttgc 3900
   aaatgttcta tgttttgaaa tgtggacaca acaaaggctg ttattgcatt aggtgtaagt 3960
   aaactggagt ttatgttaaa ttacattgat tggaaaagaa tgaaaatttc ttatttttcc 4020
   attgctgttc aatttatagt ttgaagtggg tttttgactg cttgtttaat gaagaaaaat 4080
   gcttggggtg gaagggactc ttgagatttc accagagact ttttcttttt aataaatcaa 4140
   accttttgat gatttgaggt tttatctgca gttttggaag cagtcacaaa tgagacctgt 4200
   tataaggtgg tatttttttt tttcttctgg acagtattta aaggatctta ttcttatttc 4260
   ccagggaaat tctgggctcc cacaaagtaa aaaaaaaaaa aaatcataga aaaagaatga 4320
   gcaggaatag ttcttattcc agaattgtac agtattcacc ttaagttgat tttttttctc 4380
   cttctgcaat tgaactgaat acatttttca tgcatgtttt ccagaaaata gaagtattaa 4440
   tgttattaaa aagattattt tttttattaa aggctattta tattatagaa actatcatta 4500
   atatatattc tttatttaca tgatctgtcc catagtcatg cattgttttg caccccaaat 4560
   tttttattgt tcatagcagc atggtcagct ttcttcttga tctatagatg aggctcaggc 4620
   actatcccat ttataccaat aaccagtgta taactactta aggaaaacat aaaaacttca 4680
   tcttctttcc ttttatttct tatgtgaatc tcccgtcttc cattctcttt tataattgag 4740
   aatgtctcaa tcatatgaaa ttagttacca gaattaacac aatttagact atcttcctga 4800
   ttccttaaac ccctttactg aagtatactc atgaataata ctttaaaata tgggggaata 4860
   gaaaccatga actttttacc tttttaaact atttatccat atctccaaag tagaacatta 4920
   aaccatttta agatatgtct cattcccaag tagtcagagc tcactctcca actttattaa 4980
   atactatttg agcacaggac acattcttaa acattttgaa aaacattaac ccaagatgta 5040
   gaggctactg ctagtcgtca ttctagaatc tgatatttta ctctgtattt gaaatgaatg 5100
   attaatgtcc taggaaatta gctttagcag atgtccaggt gccacatcaa aaaagtgcaa 5160
   taattattga cagtttttta gattaggcat attattggaa aacaacttta taaagagtga 5220
   acattgtata ctctagtaaa acagcatcac tttaaaaata ttcatttatg aaatctgtta 5280
   cctatagttg aagtcttgag tagtgaacaa gggactctaa taccaatact cttaatatct 5340
   ggctatttta gatcccttaa agggcataat tattggaaat ttaggtattt cactaaagca 5400
   tgtatataat attgccaaca agaaaagtaa atttgaagat taagggaact tacttctgca 5460
   aactgtcttg cgatagttaa gcagaattta aactctgttt taagcaggaa accagaaaga 5520
   ttattttgca gttgtagaag atttcataac ttattaaaac ttattaacat tttgtgttgt 5580
   ttagatatag gcagttgata catactaaca tcccagcctt ttcaatatca gggttaaatt 5640
   ataggaaaac tcagtaaaat ggtacaaatc tgaaagtttg atggtagaaa ctgaagattt 5700
   aacagagaac tgtgttttac ccgagtgcca aaaatgctgt gagcctcctt gcacaaaatt 5760
   tataccactt ttgcattttt atctatcagt ccagatagtt gtctcccctc cttctcccag 5820
   gacctctcca ccattaaaat gcacaaacca catggccgat ttcaccattt acatttattt 5880
   tcaaaagtta ctacaaccaa attaattcta ttagaagaaa tgtagacaaa ttctataaag 5940
   actatagatt gtgacctaag aaagaaatga ggcaaagaac caaacattga attaaatgct 6000
   acatgggtga ctaagatctg tttcaagtca gtgataatat agccacttct gggtacttca 6060
   gtatcagaga tcagttctcg tggtttagac agttcctatc tatagctgac tatccttgtc 6120
   cttgaatatg gtgtaactga ctattggctc tacagtttta ttgggccact taagaaatat 6180
   ttccttgaat aattattttg agaaaaagtc taaaagtaat aaaaataatt ttaaacacac 6240
   tgtagtaaga aatgactgtt ggaaaattat gctttcactt tctaccatat tctcagctat 6300
   acaaaaccat ttattttgaa gatttttaga ctactgttaa tttgaaatct gttactctta 6360
   ttgtggaatt tgttttttta aaaaagatgt ttctaattgg atttttaaaa gaagaatgga 6420
   atttggttgc tattttacaa tagaacctaa gctttttgtg gttcttagtg tcctatgtaa 6480
   aacttagtgt caaagtaatc aactttgaga ttttcccttc tattctgctt tatattaaaa 6540
   gcccattaga aaatgggaac ctggtgaata tataatgaat tgtaaaatat tttaatgtgt 6600
   aactttttca actgtgaaac tgacttgatt ttttgatgaa aacagctgct gataaagtat 6660
   tttgtgtaaa gtgtagttct tattaatcag gaaaatgatg acttgattag actgtatatg 6720
   ccctcttgga ttttatttta aatggattgg tgactttcac ataggtaaaa cacagtccat 6780
   ctgtattctt ttttccatca aaaatcgagt gatttggaat tataaaaaaa ttgtgagcag 6840
   cctatttgaa aggcatcatg gaaatttcac agcacaataa cacggatttg tttttttcta 6900
   atgatgtaaa tccgtttaat tcatactttg atcaatagcc catgcttgcc aactctgaag 6960
   aaatttaatt tccagcagta ttttaaagct agcctgttaa ctttttctga atatttaaag 7020
   ttcctctttt ttctatgtct gcacaaactg cagacctggg ctggacccac atactcaaga 7080
   gtccacctta agaaattatt ttgatgtcca agacatcact aaaatattta agtttaaaga 7140
   taatatgtgg tgttaataga ttgtggtgct tttactattt aaagacaact ttcatacttc 7200
   agatgttttt gagaagaggg gaatgtgagg ggagggggca gaacagggag gagttgtttg 7260
   aatgaattac attctttata tccatcctgc tcatttgggg catgtcttta agagaaggct 7320
   gaaagttgtg agagtatatt gtataccgta agagaatcaa ctcttcatca tggatgggat 7380
   tgtgaaggct gaactataaa attcagcatt gacagcatcc tcaattaata attcttggtg 7440
   acagaataat acagctgggc tgttttttaa aatataaaca ataccatttt taattattac 7500
   attaaaaatt gtaaatatat ctatgtgcca tggcctggga agcctgcttt cttttttcat 7560
   aaaaattatt tttactgtat gaaaagatca tggggtttag ctcaaaatat ctgtggtcct 7620
   gataaaattg gattggtaac tctacctcag aaggaaaatg ggaaaaaaaa atagatgagt 7680
   cacaattcaa tacttcaagc tcagaaactg tgcagatcac tgaattttag atttataaag 7740
   tcagagttgg catgccttgt ttttaatgat atggaagacc ttaagaaaaa aacttggctg 7800
   aagtttaatc gttggtccag ccatttgaaa aaggcaatag tttgaggagg ttcccgaatt 7860
   cggcatttga aattcatttt gttctctctt cttcattatt agtgcatttg gtgtgtgtat 7920
   acttgcacac aattctgttt gtgtacacac tgcttgctta gccctagtca agaggcatct 7980
   tttataaaag gtgtaaagaa atatcaaggt tctaaaattc ggaagagttt agaatttatt 8040
   aggagtttcc caagttggga tgttagtctt taaataaact tcatgcacct attccactta 8100
   aggttttgca cctccttttt attagtgcag tgccatttct tctgcttgat tttaggtatg 8160
   ttaatattcc agccttgcta gttagcataa agtgacaggt gtgagccatg aggaaatttt 8220
   ctgacttaat ttgtacacaa ctacatataa gagttttagt ggaggaaaaa aattagtccc 8280
   ttgtgcgtat acagtagtta ggtaaatgat ttttctacca acagtatact ccattcctca 8340
   tgtaggtaag tacagaaaag gtttttaaat gtattttttt agccagttaa agtctatgaa 8400
   tctatctgca accttattta atctgtcact ataataattt tgtggttatg ctaagaacca 8460
   tgtatacttt taggtattct tatttttgtc aatttttcta ggttggcaag gaggcagaaa 8520
   accttcattg tttcatatta aaatataatt agactaaact taattctagt atgaatttcc 8580
   aaaatcatta tctatttatt tcatttttat ttaattttgt ttttatttca tttttaaaag 8640
   tcccttgttc aatttaactt atgttcctaa gagaggttgg agaacttggc cttcatctga 8700
   tttcaaaaat gttttgagtt tcaaatgaag ttaatggttt cagtgtgatt cagtcctcag 8760
   acctaattgg gttgaataaa atctaaaaga atataccctt ttggagcata acattttaat 8820
   accttgggga atgtggcact accaaaagaa gactactaac acgtcagatg ttcacctgga 8880
   agctttatca agaaattcga accacccttt tggccccatt aattgtagca agtttatttc 8940
   tctatatttt gtcattcagt gaattgaagt cctgtggtat actgcattca ttagaagaaa 9000
   aacgttttta atgtcctttt aatgatggcc cagaaagcat ttgacacagc aagatgcatg 9060
   tgttactata ttgagaatat agaataataa cagtatcact aaatttaaga cctcttccca 9120
   gtcttgctgt tcctagcaag aagtttggcc tgtgactgca cttactgttt atgctcatca 9180
   gaaactgtca atgtctgctt ttctttaact ctgcagtctg taacatcacg ctgtttatta 9240
   aaaaaaaaaa gaaaaatta 9259

<210> 133
<211> 1068
<212> PRT
<213> Homo sapiens

<220>
<221> MISC_FEATURE
<223> PIK3CA, wild type

<400> 133

<210>  134
<211>  3421
<212>  DNA
<213>  Artificial Sequence

<220>
<223>  Synthetic

<220>
<221>  misc_feature
<223>  PAN67

<400>  134
gaggaaccga gaggctgaga ctaacccaga aacatccaat tctcaaactg aagctcgcac      60

tctcgcctcc agcatgcctc cacgaccatc atcaggtgaa ctgtggggca tccacttgat     120

gcccccaaga atcctagtag aatgtttact accaaatgga atgatagtga ctttagaatg     180

cctccgtgag gctacattaa taaccataaa gcatgaacta tttaaagaag caagaaaata     240

ccccctccat caacttcttc aagatgaatc ttcttacatt ttcgtaagtg ttactcaaga     300

agcagaaagg gaagaatttt ttgatgaaac aagacgactt tgtgaccttc ggctttttca     360

acccttttta aaagtaattg aaccagtagg caaccgtgaa gaaaagatcc tcaatcgaga     420

aattggtttt gctatcggca tgccagtgtg tgaatttgat atggttaaag atccagaagt     480

acaggacttc cgaagaaata ttctgaacgt ttgtaaagaa gctgtggatc ttagggacct     540

caattcacct catagtagag caatgtatgt ctatcctcca aatgtagaat cttcaccaga     600

attgccaaag cacatatata ataaattaga taaagggcaa ataatagtgg tgatctgggt     660

aatagtttct ccaaataatg acaagcagaa gtatactctg aaaatcaacc atgactgtgt     720

accagaacaa gtaattgctg aagcaatcag gaaaaaaact cgaagtatgt tgctatcctc     780

tgaacaacta aaactctgtg ttttagaata tcagggcaag tatattttaa aagtgtgtgg     840

atgtgatgaa tacttcctag aaaaatatcc tctgagtcag tataagtata taagaagctg     900

tataatgctt gggaggatgc ccaatttgat gttgatggct aaagaaagcc tttattctca     960

actgccaatg gactgtttta caatgccatc ttattccaga cgcatttcca cagctacacc    1020

atatatgaat ggagaaacat ctacaaaatc cctttgggtt ataaatagtg cactcagaat 1080

aaaaattctt tgtgcaacct acgtgaatgt aaatattcga gacattgata agatctatgt 1140

tcgaacaggt atctaccatg gaggagaacc cttatgtgac aatgtgaaca ctcaaagagt 1200

accttgttcc aatcccaggt ggaatgaatg gctgaattat gatatataca ttcctgatct 1260

tcctcgtgct gctcgacttt gcctttccat ttgctctgtt aaaggccgaa agggtgctaa 1320

agaggaacac tgtccattgg catggggaaa tataaacttg tttgattaca cagacactct 1380

agtatctgga aaaatggctt tgaatctttg gccagtacct catggattag aagatttgct 1440

gaaccctatt ggtgttactg gatcaaatcc aaataaagaa actccatgct tagagttgga 1500

gtttgactgg ttcagcagtg tggtaaagtt cccagatatg tcagtgattg aagagcatgc 1560

caattggtct gtatcccgag aagcaggatt tagctattcc cacgcaggac tgagtaacag 1620

actagctaga gacaatgaat taagggaaaa tgacaaagaa cagctcaaag caatttctac 1680

acgagatcct ctctctaaaa tcactgagca ggagaaagat tttctatgga gtcacagaca 1740

ctattgtgta actatccccg aaattctacc caaattgctt ctgtctgtta aatggaattc 1800

tagagatgaa gtagcccaga tgtattgctt ggtaaaagat tggcctccaa tcaaacctga 1860

acaggctatg gaacttctgg actgtaatta cccagatcct atggttcgag gttttgctgt 1920

tcggtgcttg gaaaaatatt taacagatga caaactttct cagtatttaa ttcagctagt 1980

acaggtccta aaatatgaac aatatttgga taacttgctt gtgagatttt tactgaagaa 2040

agcattgact aatcaaagga ttgggcactt tttcttttgg catttaaaat ctgagatgca 2100

caataaaaca gttagccaga ggtttggcct gcttttggag tcctattgtc gtgcatgtgg 2160

gatgtatttg aagcacctga ataggcaagt cgaggcaatg gaaaagctca ttaacttaac 2220

tgacattctc aaacaggaga agaaggatga aacacaaaag gtacagatga agtttttagt 2280

tgagcaaatg aggcgaccag atttcatgga tgctctacag ggctttctgt ctcctctaaa 2340

ccctgctcat caactaggaa acctcaggct tgaagagtgt cgaattatgt cctctgcaaa 2400

aaggccactg tggttgaatt gggagaaccc agacatcatg tcagagttac tgtttcagaa 2460

caatgagatc atctttaaaa atggggatga tttacggcaa gatatgctaa cacttcaaat 2520

tattcgtatt atggaaaata tctggcaaaa tcaaggtctt gatcttcgaa tgttacctta 2580

tggttgtctg tcaatcggtg actgtgtggg acttattgag gtggtgcgaa attctcacac 2640

tattatgcaa attcagtgca aaggcggctt gaaaggtgca ctgcagttca acagccacac 2700

actacatcag tggctcaaag acaagaacaa aggagaaata tatgatgcag ccattgacct 2760

gtttacacgt tcatgtgctg gatactgtgt agctaccttc attttgggaa ttggagatcg 2820

tcacaatagt aacatcatgg tgaaagacga tggacaactg tttcatatag attttggaca 2880

ctttttggat cacaagaaga aaaaatttgg ttataaacga gaacgtgtgc catttgtttt 2940

gacacaggat ttcttaatag tgattagtaa aggagcccaa gaatgcacaa agacaagaga 3000

atttgagagg tttcaggaga tgtgttacaa ggcttatcta gctattcgac agcatgccaa 3060

tctcttcata aatcttttct caatgatgct tggctctgga atgccagaac tacaatcttt 3120

tgatgacatt gcatacattc gaaagaccct agccttagat aaaactgagc aagaggcttt 3180

ggagtatttc atgaaacaaa tgaatgatgc acatcatggt ggctggacaa caaaaatgga 3240

ttggatcttc cacacaatta aacagcatgc attgaactga ggctgctgca gctgcatggg 3300

tgcctgctgc tgcctgcctt ggcctgatgg ccaggccaga gtgctgccag tcctctgcat 3360

gttctgctct tgtgcccttc tgagcccaca ataaaggctg agctcttatc ttgcaaaagg 3420

c 3421

<210> 135
<211> 3421
<212> DNA
<213> Artificial Sequence

<220>
<223> Synthetic

<220>
<221> misc_feature
<223> PAN68

<400> 135
gaggaaccga gaggctgaga ctaacccaga aacatccaat tctcaaactg aagctcgcac 60

tctcgcctcc agcatgcctc cacgaccatc atcaggtgaa ctgtggggca tccacttgat 120

gcccccaaga atcctagtag aatgtttact accaaatgga atgatagtga ctttagaatg 180

cctccgtgag gctacattaa taaccataaa gcatgaacta tttaaagaag caagaaaata 240

ccccctccat caacttcttc aagatgaatc ttcttacatt ttcgtaagtg ttactcaaga 300

agcagaaagg gaagaatttt ttgatgaaac aagacgactt tgtgaccttc ggctttttca 360

acccttttta aaagtaattg aaccagtagg caaccgtgaa gaaaagatcc tcaatcgaga 420

aattggtttt gctatcggca tgccagtgtg tgaatttgat atggttaaag atccagaagt 480

acaggacttc cgaagaaata ttctgaacgt ttgtaaagaa gctgtggatc ttagggacct 540

caattcacct catagtagag caatgtatgt ctatcctcca aatgtagaat cttcaccaga 600

attgccaaag cacatatata ataaattaga taaagggcaa ataatagtgg tgatctgggt 660

aatagtttct ccaaataatg acaagcagaa gtatactctg aaaatcaacc atgactgtgt 720

accagaacaa gtaattgctg aagcaatcag gaaaaaaact cgaagtatgt tgctatcctc 780

tgaacaacta aaactctgtg ttttagaata tcagggcaag tatattttaa aagtgtgtgg 840

atgtgatgaa tacttcctag aaaaatatcc tctgagtcag tataagtata taagaagctg 900

tataatgctt gggaggatgc ccaatttgat gttgatggct aaagaaagcc tttattctca 960

actgccaatg gactgtttta caatgccatc ttattccaga cgcatttcca cagctacacc 1020

atatatgaat ggagaaacat ctacaaaatc cctttgggtt ataaatagtg cactcagaat 1080

aaaaattctt tgtgcaacct acgtgaatgt aaatattcga gacattgata agatctatgt 1140

tcgaacaggt atctaccatg gaggagaacc cttatgtgac aatgtgaaca ctcaaagagt 1200

accttgttcc aatcccaggt ggaatgaatg gctgaattat gatatataca ttcctgatct 1260

tcctcgtgct gctcgacttt gcctttccat ttgctctgtt aaaggccgaa agggtgctaa 1320

agaggaacac tgtccattgg catgggggaa tataaacttg tttgattaca cagacactct 1380

agtatctgga aaaatggctt tgaatctttg gccagtacct catggattag aagatttgct 1440

gaaccctatt ggtgttactg gatcaaatcc aaataaagaa actccatgct tagagttgga 1500

gtttgactgg ttcagcagtg tggtaaagtt cccagatatg tcagtgattg aagagcatgc 1560

caattggtct gtatcccgag aagcaggatt tagctattcc cacgcaggac tgagtaacag 1620

actagctaga gacaatgaat taagggaaaa tgacaaagaa cagctcaaag caatttctac 1680

acgagatcct ctctctgaaa tcactaagca ggagaaagat tttctatgga gtcacagaca 1740

ctattgtgta actatccccg aaattctacc caaattgctt ctgtctgtta aatggaattc 1800

tagagatgaa gtagcccaga tgtattgctt ggtaaaagat tggcctccaa tcaaacctga 1860

acaggctatg gaacttctgg actgtaatta cccagatcct atggttcgag gttttgctgt 1920

tcggtgcttg gaaaaatatt taacagatga caaactttct cagtatttaa ttcagctagt 1980

acaggtccta aaatatgaac aatatttgga taacttgctt gtgagatttt tactgaagaa 2040

agcattgact aatcaaagga ttgggcactt tttcttttgg catttaaaat ctgagatgca 2100

caataaaaca gttagccaga ggtttggcct gcttttggag tcctattgtc gtgcatgtgg 2160

gatgtatttg aagcacctga ataggcaagt cgaggcaatg gaaaagctca ttaacttaac 2220

tgacattctc aaacaggaga agaaggatga aacacaaaag gtacagatga agtttttagt 2280

tgagcaaatg aggcgaccag atttcatgga tgctctacag ggctttctgt ctcctctaaa 2340

ccctgctcat caactaggaa acctcaggct tgaagagtgt cgaattatgt cctctgcaaa 2400

aaggccactg tggttgaatt gggagaaccc agacatcatg tcagagttac tgtttcagaa 2460

caatgagatc atctttaaaa atggggatga tttacggcaa gatatgctaa cacttcaaat 2520

tattcgtatt atggaaaata tctggcaaaa tcaaggtctt gatcttcgaa tgttacctta 2580

tggttgtctg tcaatcggtg actgtgtggg acttattgag gtggtgcgaa attctcacac 2640

tattatgcaa attcagtgca aaggcggctt gaaaggtgca ctgcagttca acagccacac 2700

actacatcag tggctcaaag acaagaacaa aggagaaata tatgatgcag ccattgacct 2760

gtttacacgt tcatgtgctg gatactgtgt agctaccttc attttgggaa ttggagatcg 2820

tcacaatagt aacatcatgg tgaaagacga tggacaactg tttcatatag attttggaca 2880

ctttttggat cacaagaaga aaaaatttgg ttataaacga gaacgtgtgc catttgtttt 2940

gacacaggat ttcttaatag tgattagtaa aggagcccaa gaatgcacaa agacaagaga 3000

atttgagagg tttcaggaga tgtgttacaa ggcttatcta gctattcgac agcatgccaa 3060

tctcttcata aatcttttct caatgatgct tggctctgga atgccagaac tacaatcttt 3120

tgatgacatt gcatacattc gaaagaccct agccttagat aaaactgagc aagaggcttt 3180

ggagtatttc atgaaacaaa tgaatgatgc acatcatggt ggctggacaa caaaaatgga 3240

ttggatcttc cacacaatta aacagcatgc attgaactga ggctgctgca gctgcatggg 3300

tgcctgctgc tgcctgcctt ggcctgatgg ccaggccaga gtgctgccag tcctctgcat 3360

gttctgctct tgtgcccttc tgagcccaca ataaaggctg agctcttatc ttgcaaaagg 3420

c 3421

<210> 136
<211> 3421
<212> DNA
<213> Artificial Sequence

<220>
<223> Synthetic

<220>
<221> misc_feature
<223> PAN69

<400> 136
gaggaaccga gaggctgaga ctaacccaga aacatccaat tctcaaactg aagctcgcac 60

tctcgcctcc agcatgcctc cacgaccatc atcaggtgaa ctgtggggca tccacttgat 120

gcccccaaga atcctagtag aatgtttact accaaatgga atgatagtga ctttagaatg 180

cctccgtgag gctacattaa taaccataaa gcatgaacta tttaaagaag caagaaaata 240

ccccctccat caacttcttc aagatgaatc ttcttacatt ttcgtaagtg ttactcaaga 300

agcagaaagg gaagaatttt ttgatgaaac aagacgactt tgtgaccttc ggctttttca 360

acccttttta aaagtaattg aaccagtagg caaccgtgaa gaaaagatcc tcaatcgaga 420

aattggtttt gctatcggca tgccagtgtg tgaatttgat atggttaaag atccagaagt 480

acaggacttc cgaagaaata ttctgaacgt ttgtaaagaa gctgtggatc ttagggacct 540

caattcacct catagtagag caatgtatgt ctatcctcca aatgtagaat cttcaccaga 600

attgccaaag cacatatata ataaattaga taaagggcaa ataatagtgg tgatctgggt 660

aatagtttct ccaaataatg acaagcagaa gtatactctg aaaatcaacc atgactgtgt 720

accagaacaa gtaattgctg aagcaatcag gaaaaaaact cgaagtatgt tgctatcctc 780

tgaacaacta aaactctgtg ttttagaata tcagggcaag tatattttaa aagtgtgtgg 840

atgtgatgaa tacttcctag aaaaatatcc tctgagtcag tataagtata taagaagctg 900

tataatgctt gggaggatgc ccaatttgat gttgatggct aaagaaagcc tttattctca 960

actgccaatg gactgtttta caatgccatc ttattccaga cgcatttcca cagctacacc 1020

atatatgaat ggagaaacat ctacaaaatc cctttgggtt ataaatagtg cactcagaat 1080

aaaaattctt tgtgcaacct acgtgaatgt aaatattcga gacattgata agatctatgt 1140

tcgaacaggt atctaccatg gaggagaacc cttatgtgac aatgtgaaca ctcaaagagt 1200

accttgttcc aatcccaggt ggaatgaatg gctgaattat gatatataca ttcctgatct 1260

tcctcgtgct gctcgacttt gcctttccat ttgctctgtt aaaggccgaa agggtgctaa 1320

agaggaacac tgtccattgg catggggaaa tataaacttg tttgattaca cagacactct 1380

agtatctgga aaaatggctt tgaatctttg gccagtacct catggattag aagatttgct 1440

gaaccctatt ggtgttactg gatcaaatcc aaataaagaa actccatgct tagagttgga 1500

gtttgactgg ttcagcagtg tggtaaagtt cccagatatg tcagtgattg aagagcatgc 1560

caattggtct gtatcccgag aagcaggatt tagctattcc cacgcaggac tgagtaacag 1620

actagctaga gacaatgaat taaggaaaaa tgacaaagaa cagctcaaag caatttctac 1680

acgagatcct ctctctgaaa tcactgagca ggagaaagat tttctatgga gtcacagaca 1740

ctattgtgta actatccccg aaattctacc caaattgctt ctgtctgtta aatggaattc 1800

tagagatgaa gtagcccaga tgtattgctt ggtaaaagat tggcctccaa tcaaacctga 1860

acaggctatg gaacttctgg actgtaatta cccagatcct atggttcgag gttttgctgt 1920

tcggtgcttg gaaaaatatt taacagatga caaactttct cagtatttaa ttcagctagt 1980

acaggtccta aaatatgaac aatatttgga taacttgctt gtgagatttt tactgaagaa 2040

agcattgact aatcaaagga ttgggcactt tttcttttgg catttaaaat ctgagatgca 2100

caataaaaca gttagccaga ggtttggcct gcttttggag tcctattgtc gtgcatgtgg 2160

gatgtatttg aagcacctga ataggcaagt cgaggcaatg gaaaagctca ttaacttaac 2220

tgacattctc aaacaggaga agaaggatga aacacaaaag gtacagatga agtttttagt 2280

tgagcaaatg aggcgaccag atttcatgga tgctctacag ggctttctgt ctcctctaaa 2340

ccctgctcat caactaggaa acctcaggct tgaagagtgt cgaattatgt cctctgcaaa 2400

aaggccactg tggttgaatt gggagaaccc agacatcatg tcagagttac tgtttcagaa 2460

caatgagatc atctttaaaa atggggatga tttacggcaa gatatgctaa cacttcaaat 2520

tattcgtatt atggaaaata tctggcaaaa tcaaggtctt gatcttcgaa tgttacctta 2580

tggttgtctg tcaatcggtg actgtgtggg acttattgag gtggtgcgaa attctcacac 2640

tattatgcaa attcagtgca aaggcggctt gaaaggtgca ctgcagttca acagccacac 2700

actacatcag tggctcaaag acaagaacaa aggagaaata tatgatgcag ccattgacct 2760

gtttacacgt tcatgtgctg gatactgtgt agctaccttc attttgggaa ttggagatcg 2820

tcacaatagt aacatcatgg tgaaagacga tggacaactg tttcatatag attttggaca 2880

ctttttggat cacaagaaga aaaaatttgg ttataaacga gaacgtgtgc catttgtttt 2940

gacacaggat ttcttaatag tgattagtaa aggagcccaa gaatgcacaa agacaagaga 3000

atttgagagg tttcaggaga tgtgttacaa ggcttatcta gctattcgac agcatgccaa 3060

tctcttcata aatcttttct caatgatgct tggctctgga atgccagaac tacaatcttt 3120

tgatgacatt gcatacattc gaaagaccct agccttagat aaaactgagc aagaggcttt 3180

ggagtatttc atgaaacaaa tgaatgatgc acggcatggt ggctggacaa caaaaatgga 3240

ttggatcttc cacacaatta aacagcatgc attgaactga ggctgctgca gctgcatggg 3300

tgcctgctgc tgcctgcctt ggcctgatgg ccaggccaga gtgctgccag tcctctgcat 3360

gttctgctct tgtgcccttc tgagcccaca ataaaggctg agctcttatc ttgcaaaagg 3420

c 3421

<210> 137
<211> 2987
<212> DNA
<213> Homo sapiens

<220>
<221> misc_feature
<223> Akt1, wild type

<400> 137
taattatggg tctgtaacca ccctggactg ggtgctcctc actgacggac ttgtctgaac 60

ctctctttgt ctccagcgcc cagcactggg cctggcaaaa cctgagacgc ccggtacatg 120

ttggccaaat gaatgaacca gattcagacc ggcaggggcg ctgtggttta ggaggggcct 180

ggggtttctc ccaggaggtt tttgggcttg cgctggaggg ctctggactc ccgtttgcgc 240

cagtggcctg catcctggtc ctgtcttcct catgtttgaa tttctttgct ttcctagtct 300

ggggagcagg gaggagccct gtgccctgtc ccaggatcca tgggtaggaa caccatggac 360

agggagagca aacgggccca tctgtcacca ggggcttagg gaaggccgag ccagcctggg 420

tcaaagaagt caaaggggct gcctggagga ggcagcctgt cagctggtgc atcagaggct 480

gtggccaggc cagctgggct cggggagcgc cagcctgaga ggagcgcgtg agcgtcgcgg 540

gagcctcggg caccatgagc gacgtggcta ttgtgaagga gggttggctg cacaaacgag 600

gggagtacat caagacctgg cggccacgct acttcctcct caagaatgat ggcaccttca 660

ttggctacaa ggagcggccg caggatgtgg accaacgtga ggctcccctc aacaacttct 720

ctgtggcgca gtgccagctg atgaagacgg agcggccccg gcccaacacc ttcatcatcc 780

gctgcctgca gtggaccact gtcatcgaac gcaccttcca tgtggagact cctgaggagc 840

gggaggagtg gacaaccgcc atccagactg tggctgacgg cctcaagaag caggaggagg 900

aggagatgga cttccggtcg ggctcaccca gtgacaactc aggggctgaa gagatggagg 960

tgtccctggc caagcccaag caccgcgtga ccatgaacga gtttgagtac ctgaagctgc 1020

tgggcaaggg cactttcggc aaggtgatcc tggtgaagga gaaggccaca ggccgctact 1080

acgccatgaa gatcctcaag aaggaagtca tcgtggccaa ggacgaggtg gcccacacac 1140

tcaccgagaa ccgcgtcctg cagaactcca ggcacccctt cctcacagcc ctgaagtact 1200

ctttccagac ccacgaccgc ctctgctttg tcatggagta cgccaacggg ggcgagctgt 1260

tcttccacct gtcccgggag cgtgtgttct ccgaggaccg ggcccgcttc tatggcgctg 1320

agattgtgtc agccctggac tacctgcact cggagaagaa cgtggtgtac cgggacctca 1380

agctggagaa cctcatgctg gacaaggacg ggcacattaa gatcacagac ttcgggctgt 1440

gcaaggaggg gatcaaggac ggtgccacca tgaagacctt ttgcggcaca cctgagtacc 1500

tggcccccga ggtgctggag gacaatgact acggccgtgc agtggactgg tgggggctgg 1560

gcgtggtcat gtacgagatg atgtgcggtc gcctgccctt ctacaaccag gaccatgaga 1620

agctttttga gctcatcctc atggaggaga tccgcttccc gcgcacgctt ggtcccgagg 1680

ccaagtcctt gctttcaggg ctgctcaaga aggaccccaa gcagaggctt ggcgggggct 1740

ccgaggacgc caaggagatc atgcagcatc gcttctttgc cggtatcgtg tggcagcacg 1800

tgtacgagaa gaagctcagc ccacccttca agccccaggt cacgtcggag actgacacca 1860

ggtattttga tgaggagttc acggcccaga tgatcaccat cacaccacct gaccaagatg 1920

acagcatgga gtgtgtggac agcgagcgca ggccccactt cccccagttc tcctactcgg 1980

ccagcggcac ggcctgaggc ggcggtggac tgcgctggac gatagcttgg agggatggag 2040

aggcggcctc gtgccatgat ctgtatttaa tggtttttat ttctcgggtg catttgagag 2100

aagccacgct gtcctctcga gcccagatgg aaagacgttt ttgtgctgtg ggcagcaccc 2160

tcccccgcag cggggtaggg aagaaaacta tcctgcgggt tttaatttat ttcatccagt 2220

ttgttctccg ggtgtggcct cagccctcag aacaatccga ttcacgtagg gaaatgttaa 2280

ggacttctgc agctatgcgc aatgtggcat tgggggccgg ggcaggtcct gcccatgtgt 2340

cccctcactc tgtcagccag ccgccctggg ctgtctgtca ccagctatct gtcatctctc 2400

tggggccctg ggcctcagtt caacctggtg gcaccagatg caacctcact atggtatgct 2460

ggccagcacc ctctcctggg ggtggcaggc acacagcagc cccccagcac taaggccgtg 2520

tctctgagga cgtcatcgga ggctgggccc ctgggatggg accagggatg gggggatggc 2580

cagggtttac ccagtgggac agaggagcaa ggtttaaatt tgttattgtg tattatgttg 2640

ttcaaatgca ttttgggggt ttttaatctt tgtgacagga aagccctccc ccttcccctt 2700

ctgtgtcaca gttcttggtg actgtcccac cgggagcctc cccctcagat gatctctcca 2760

cggtagcact tgaccttttc gacgcttaac ctttccgctg tcgccccagg ccctccctga 2820

ctccctgtgg gggtggccat ccctgggccc ctccacgcct cctggccaga cgctgccgct 2880

gccgctgcac cacggcgttt ttttacaaca ttcaacttta gtatttttac tattataata 2940

taatatggaa ccttccctcc aaattcttca ataaaagttg cttttca 2987

<210> 138
<211> 480
<212> PRT
<213> Homo sapiens

<220>
<221> MISC_FEATURE
<223> Akt1, wild type

<400> 138

<210>  139
<211>  8721
<212>  DNA
<213>  Homo sapiens

<220>
<221>  misc_feature
<223>  mTor, wild type

<400>  139
gctcccggct tagaggacag cggggaaggc gggcggtggg gcagggggcc tgaagcggcg      60

gtaccggtgc tggcggcggc agctgaggcc ttggccgaag ccgcgcgaac ctcagggcaa     120

gatgcttgga accggacctg ccgccgccac caccgctgcc accacatcta gcaatgtgag     180

cgtcctgcag cagtttgcca gtggcctaaa gagccggaat gaggaaacca gggccaaagc     240

cgccaaggag ctccagcact atgtcaccat ggaactccga gagatgagtc aagaggagtc     300

tactcgcttc tatgaccaac tgaaccatca catttttgaa ttggtttcca gctcagatgc     360

caatgagagg aaaggtggca tcttggccat agctagcctc ataggagtgg aaggtgggaa     420

tgccacccga attggcagat ttgccaacta tcttcggaac ctcctcccct ccaatgaccc     480

agttgtcatg gaaatggcat ccaaggccat tggccgtctt gccatggcag gggacacttt     540

taccgctgag tacgtggaat ttgaggtgaa gcgagccctg gaatggctgg gtgctgaccg     600

caatgagggc cggagacatg cagctgtcct ggttctccgt gagctggcca tcagcgtccc     660

taccttcttc ttccagcaag tgcaaccctt ctttgacaac atttttgtgg ccgtgtggga     720

ccccaaacag gccatccgtg agggagctgt agccgccctt cgtgcctgtc tgattctcac     780

aacccagcgt gagccgaagg agatgcagaa gcctcagtgg tacaggcaca catttgaaga     840

agcagagaag ggatttgatg agaccttggc caaagagaag ggcatgaatc gggatgatcg     900

gatccatgga gccttgttga tccttaacga gctggtccga atcagcagca tggagggaga 960

gcgtctgaga gaagaaatgg aagaaatcac acagcagcag ctggtacacg acaagtactg 1020

caaagatctc atgggcttcg gaacaaaacc tcgtcacatt acccccttca ccagtttcca 1080

ggctgtacag ccccagcagt caaatgcctt ggtggggctg ctggggtaca gctctcacca 1140

aggcctcatg ggatttggga cctcccccag tccagctaag tccaccctgg tggagagccg 1200

gtgttgcaga gacttgatgg aggagaaatt tgatcaggtg tgccagtggg tgctgaaatg 1260

caggaatagc aagaactcgc tgatccaaat gacaatcctt aatttgttgc cccgcttggc 1320

tgcattccga ccttctgcct tcacagatac ccagtatctc caagatacca tgaaccatgt 1380

cctaagctgt gtcaagaagg agaaggaacg tacagcggcc ttccaagccc tggggctact 1440

ttctgtggct gtgaggtctg agtttaaggt ctatttgcct cgcgtgctgg acatcatccg 1500

agcggccctg cccccaaagg acttcgccca taagaggcag aaggcaatgc aggtggatgc 1560

cacagtcttc acttgcatca gcatgctggc tcgagcaatg gggccaggca tccagcagga 1620

tatcaaggag ctgctggagc ccatgctggc agtgggacta agccctgccc tcactgcagt 1680

gctctacgac ctgagccgtc agattccaca gctaaagaag gacattcaag atgggctact 1740

gaaaatgctg tccctggtcc ttatgcacaa accccttcgc cacccaggca tgcccaaggg 1800

cctggcccat cagctggcct ctcctggcct cacgaccctc cctgaggcca gcgatgtggg 1860

cagcatcact cttgccctcc gaacgcttgg cagctttgaa tttgaaggcc actctctgac 1920

ccaatttgtt cgccactgtg cggatcattt cctgaacagt gagcacaagg agatccgcat 1980

ggaggctgcc cgcacctgct cccgcctgct cacaccctcc atccacctca tcagtggcca 2040

tgctcatgtg gttagccaga ccgcagtgca agtggtggca gatgtgctta gcaaactgct 2100

cgtagttggg ataacagatc ctgaccctga cattcgctac tgtgtcttgg cgtccctgga 2160

cgagcgcttt gatgcacacc tggcccaggc ggagaacttg caggccttgt ttgtggctct 2220

gaatgaccag gtgtttgaga tccgggagct ggccatctgc actgtgggcc gactcagtag 2280

catgaaccct gcctttgtca tgcctttcct gcgcaagatg ctcatccaga ttttgacaga 2340

gttggagcac agtgggattg gaagaatcaa agagcagagt gcccgcatgc tggggcacct 2400

ggtctccaat gccccccgac tcatccgccc ctacatggag cctattctga aggcattaat 2460

tttgaaactg aaagatccag accctgatcc aaacccaggt gtgatcaata atgtcctggc 2520

aacaatagga gaattggcac aggttagtgg cctggaaatg aggaaatggg ttgatgaact 2580

ttttattatc atcatggaca tgctccagga ttcctctttg ttggccaaaa ggcaggtggc 2640

tctgtggacc ctgggacagt tggtggccag cactggctat gtagtagagc cctacaggaa 2700

gtaccctact ttgcttgagg tgctactgaa ttttctgaag actgagcaga accagggtac 2760

acgcagagag gccatccgtg tgttagggct tttaggggct ttggatcctt acaagcacaa 2820

agtgaacatt ggcatgatag accagtcccg ggatgcctct gctgtcagcc tgtcagaatc 2880

caagtcaagt caggattcct ctgactatag cactagtgaa atgctggtca acatgggaaa 2940

cttgcctctg gatgagttct acccagctgt gtccatggtg gccctgatgc ggatcttccg 3000

agaccagtca ctctctcatc atcacaccat ggttgtccag gccatcacct tcatcttcaa 3060

gtccctggga ctcaaatgtg tgcagttcct gccccaggtc atgcccacgt tccttaacgt 3120

cattcgagtc tgtgatgggg ccatccggga atttttgttc cagcagctgg gaatgttggt 3180

gtcctttgtg aagagccaca tcagacctta tatggatgaa atagtcaccc tcatgagaga 3240

attctgggtc atgaacacct caattcagag cacgatcatt cttctcattg agcaaattgt 3300

ggtagctctt gggggtgaat ttaagctcta cctgccccag ctgatcccac acatgctgcg 3360

tgtcttcatg catgacaaca gcccaggccg cattgtctct atcaagttac tggctgcaat 3420

ccagctgttt ggcgccaacc tggatgacta cctgcattta ctgctgcctc ctattgttaa 3480

gttgtttgat gcccctgaag ctccactgcc atctcgaaag gcagcgctag agactgtgga 3540

ccgcctgacg gagtccctgg atttcactga ctatgcctcc cggatcattc accctattgt 3600

tcgaacactg gaccagagcc cagaactgcg ctccacagcc atggacacgc tgtcttcact 3660

tgtttttcag ctggggaaga agtaccaaat tttcattcca atggtgaata aagttctggt 3720

gcgacaccga atcaatcatc agcgctatga tgtgctcatc tgcagaattg tcaagggata 3780

cacacttgct gatgaagagg aggatccttt gatttaccag catcggatgc ttaggagtgg 3840

ccaaggggat gcattggcta gtggaccagt ggaaacagga cccatgaaga aactgcacgt 3900

cagcaccatc aacctccaaa aggcctgggg cgctgccagg agggtctcca aagatgactg 3960

gctggaatgg ctgagacggc tgagcctgga gctgctgaag gactcatcat cgccctccct 4020

gcgctcctgc tgggccctgg cacaggccta caacccgatg gccagggatc tcttcaatgc 4080

tgcatttgtg tcctgctggt ctgaactgaa tgaagatcaa caggatgagc tcatcagaag 4140

catcgagttg gccctcacct cacaagacat cgctgaagtc acacagaccc tcttaaactt 4200

ggctgaattc atggaacaca gtgacaaggg ccccctgcca ctgagagatg acaatggcat 4260

tgttctgctg ggtgagagag ctgccaagtg ccgagcatat gccaaagcac tacactacaa 4320

agaactggag ttccagaaag gccccacccc tgccattcta gaatctctca tcagcattaa 4380

taataagcta cagcagccgg aggcagcggc cggagtgtta gaatatgcca tgaaacactt 4440

tggagagctg gagatccagg ctacctggta tgagaaactg cacgagtggg aggatgccct 4500

tgtggcctat gacaagaaaa tggacaccaa caaggacgac ccagagctga tgctgggccg 4560

catgcgctgc ctcgaggcct tgggggaatg gggtcaactc caccagcagt gctgtgaaaa 4620

gtggaccctg gttaatgatg agacccaagc caagatggcc cggatggctg ctgcagctgc 4680

atggggttta ggtcagtggg acagcatgga agaatacacc tgtatgatcc ctcgggacac 4740
ccatgatggg gcattttata gagctgtgct ggcactgcat caggacctct tctccttggc 4800
acaacagtgc attgacaagg ccagggacct gctggatgct gaattaactg cgatggcagg 4860
agagagttac agtcgggcat atggggccat ggtttcttgc cacatgctgt ccgagctgga 4920
ggaggttatc cagtacaaac ttgtccccga gcgacgagag atcatccgcc agatctggtg 4980
ggagagactg caggcctgcc agcgtatcgt agaggactgg cagaaaatcc ttatggtgcg 5040
gtcccttgtg gtcagccctc atgaagacat gagaacctgg ctcaagtatg caagcctgtg 5100
cggcaagagt ggcaggctgg ctcttgctca taaaacttta gtgttgctcc tgggagttga 5160
tccgtctcgg caacttgacc atcctctgcc aacagttcac cctcaggtga cctatgccta 5220
catgaaaaac atgtggaaga gtgcccgcaa gatcgatgcc ttccagcaca tgcagcattt 5280
tgtccagacc atgcagcaac aggcccagca tgccatcgct actgaggacc agcagcataa 5340
gcaggaactg cacaagctca tggcccgatg cttcctgaaa cttggagagt ggcagctgaa 5400
tctacagggc atcaatgaga gcacaatccc caaagtgctg cagtactaca gcgccgccac 5460
agagcacgac cgcagctggt acaaggcctg gcatgcgtgg gcagtgatga acttcgaagc 5520
tgtgctacac tacaaacatc agaaccaagc ccgcgatgag aagaagaaac tgcgtcatgc 5580
cagcggggcc aacatcacca acgccaccac tgccgccacc acggccgcca ctgccaccac 5640
cactgccagc accgagggca gcaacagtga gagcgaggcc gagagcaccg agaacagccc 5700
caccccatcg ccgctgcaga agaaggtcac tgaggatctg tccaaaaccc tcctgatgta 5760
cacggtgcct gccgtccagg gcttcttccg ttccatctcc ttgtcacgag gcaacaacct 5820
ccaggataca ctcagagttc tcaccttatg gtttgattat ggtcactggc cagatgtcaa 5880
tgaggcctta gtggaggggg tgaaagccat ccagattgat acctggctac aggttatacc 5940
tcagctcatt gcaagaattg atacgcccag acccttggtg ggacgtctca ttcaccagct 6000
tctcacagac attggtcggt accaccccca ggccctcatc tacccactga cagtggcttc 6060
taagtctacc acgacagccc ggcacaatgc agccaacaag attctgaaga acatgtgtga 6120
gcacagcaac accctggtcc agcaggccat gatggtgagc gaggagctga tccgagtggc 6180
catcctctgg catgagatgt ggcatgaagg cctggaagag gcatctcgtt tgtactttgg 6240
ggaaaggaac gtgaaaggca tgtttgaggt gctggagccc ttgcatgcta tgatggaacg 6300
gggcccccag actctgaagg aaacatcctt taatcaggcc tatggtcgag atttaatgga 6360
ggcccaagag tggtgcagga agtacatgaa atcagggaat gtcaaggacc tcacccaagc 6420
ctgggacctc tattatcatg tgttccgacg aatctcaaag cagctgcctc agctcacatc 6480
cttagagctg caatatgttt ccccaaaact tctgatgtgc cgggaccttg aattggctgt 6540
gccaggaaca tatgacccca accagccaat cattcgcatt cagtccatag caccgtcttt 6600

gcaagtcatc acatccaagc agaggccccg gaaattgaca cttatgggca gcaacggaca 6660

tgagtttgtt ttccttctaa aaggccatga agatctgcgc caggatgagc gtgtgatgca 6720

gctcttcggc ctggttaaca cccttctggc caatgaccca acatctcttc ggaaaaacct 6780

cagcatccag agatacgctg tcatcccttt atcgaccaac tcgggcctca ttggctgggt 6840

tccccactgt gacacactgc acgccctcat ccgggactac agggagaaga agaagatcct 6900

tctcaacatc gagcatcgca tcatgttgcg gatggctccg gactatgacc acttgactct 6960

gatgcagaag gtggaggtgt ttgagcatgc cgtcaataat acagctgggg acgacctggc 7020

caagctgctg tggctgaaaa gccccagctc cgaggtgtgg tttgaccgaa gaaccaatta 7080

tacccgttct ttagcggtca tgtcaatggt tgggtatatt ttaggcctgg gagatagaca 7140

cccatccaac ctgatgctgg accgtctgag tgggaagatc ctgcacattg actttgggga 7200

ctgctttgag gttgctatga cccgagagaa gtttccagag aagattccat ttagactaac 7260

aagaatgttg accaatgcta tggaggttac aggcctggat ggcaactaca gaatcacatg 7320

ccacacagtg atggaggtgc tgcgagagca caaggacagt gtcatggccg tgctggaagc 7380

ctttgtctat gaccccttgc tgaactggag gctgatggac acaaatacca aaggcaacaa 7440

gcgatcccga acgaggacgg attcctactc tgctggccag tcagtcgaaa ttttggacgg 7500

tgtggaactt ggagagccag cccataagaa aacggggacc acagtgccag aatctattca 7560

ttctttcatt ggagacggtt tggtgaaacc agaggcccta aataagaaag ctatccagat 7620

tattaacagg gttcgagata agctcactgg tcgggacttc tctcatgatg acactttgga 7680

tgttccaacg caagttgagc tgctcatcaa acaagcgaca tcccatgaaa acctctgcca 7740

gtgctatatt ggctggtgcc ctttctggta actggaggcc cagatgtgcc catcacgttt 7800

tttctgaggc ttttgtactt tagtaaatgc ttccactaaa ctgaaaccat ggtgagaaag 7860

tttgactttg ttaaatattt tgaaatgtaa atgaaaagaa ctactgtata ttaaaagttg 7920

gtttgaacca actttctagc tgctgttgaa gaatatattg tcagaaacac aaggcttgat 7980

ttggttccca ggacagtgaa acatagtaat accacgtaaa tcaagccatt cattttgggg 8040

aacagaagat ccataacttt agaaatacgg gttttgactt aactcacaag agaactcatc 8100

ataagtactt gctgatggaa gaatgaccta gttgctcctc tcaacatggg tacagcaaac 8160

tcagcacagc caagaagcct caggtcgtgg agaacatgga ttaggatcct agactgtaaa 8220

gacacagaag atgctgacct cacccctgcc acctatccca agacctcact ggtctgtgga 8280

cagcagcaga aatgtttgca agataggcca aaatgagtac aaaaggtctg tcttccatca 8340

gacccagtga tgctgcgact cacacgcttc aattcaagac ctgaccgcta gtagggaggt 8400

ttattcagat cgctggcagc ctcggctgag cagatgcaca gaggggatca ctgtgcagtg 8460

ggaccaccct cactggcctt ctgcagcagg gttctgggat gttttcagtg gtcaaaatac 8520

tctgtttaga gcaagggctc agaaaacaga aatactgtca tggaggtgct gaacacaggg 8580

aaggtctggt acatattgga aattatgagc agaacaaata ctcaactaaa tgcacaaagt 8640

ataaagtgta gccatgtcta gacaccatgt tgtatcagaa taatttttgt gccaataaat 8700

gacatcagaa ttttaaacat a 8721

<210> 140
<211> 2549
<212> PRT
<213> Homo sapiens

<220>
<221> MISC_FEATURE
<223> mTor, wild type

<400> 140

Met Leu Gly Thr Gly Pro Ala Ala Ala Thr Thr Ala Ala Thr Thr Ser
1               5                   10                  15

Ser Asn Val Ser Val Leu Gln Gln Phe Ala Ser Gly Leu Lys Ser Arg
            20                  25                  30

Asn Glu Glu Thr Arg Ala Lys Ala Ala Lys Glu Leu Gln His Tyr Val
        35                  40                  45

Thr Met Glu Leu Arg Glu Met Ser Gln Glu Glu Ser Thr Arg Phe Tyr
    50                  55                  60

Asp Gln Leu Asn His His Ile Phe Glu Leu Val Ser Ser Ser Asp Ala
65                  70                  75                  80

Asn Glu Arg Lys Gly Gly Ile Leu Ala Ile Ala Ser Leu Ile Gly Val
                85                  90                  95

Glu Gly Gly Asn Ala Thr Arg Ile Gly Arg Phe Ala Asn Tyr Leu Arg
            100                 105                 110

Asn Leu Leu Pro Ser Asn Asp Pro Val Val Met Glu Met Ala Ser Lys
        115                 120                 125

Ala Ile Gly Arg Leu Ala Met Ala Gly Asp Thr Phe Thr Ala Glu Tyr
    130                 135                 140

Val Glu Phe Glu Val Lys Arg Ala Leu Glu Trp Leu Gly Ala Asp Arg
145                 150                 155                 160

Asn Glu Gly Arg Arg His Ala Ala Val Leu Val Leu Arg Glu Leu Ala
                165                 170                 175

Ile Ser Val Pro Thr Phe Phe Phe Gln Gln Val Gln Pro Phe Phe Asp
            180                 185                 190

Asn Ile Phe Val Ala Val Trp Asp Pro Lys Gln Ala Ile Arg Glu Gly
        195                 200                 205

Ala Val Ala Ala Leu Arg Ala Cys Leu Ile Leu Thr Thr Gln Arg Glu
    210                 215                 220

Pro Lys Glu Met Gln Lys Pro Gln Trp Tyr Arg His Thr Phe Glu Glu
225                 230                 235                 240

Ala Glu Lys Gly Phe Asp Glu Thr Leu Ala Lys Glu Lys Gly Met Asn
                245                 250                 255

Arg Asp Asp Arg Ile His Gly Ala Leu Leu Ile Leu Asn Glu Leu Val
            260                 265                 270

Arg Ile Ser Ser Met Glu Gly Glu Arg Leu Arg Glu Glu Met Glu Glu
        275                 280                 285

Ile Thr Gln Gln Gln Leu Val His Asp Lys Tyr Cys Lys Asp Leu Met
    290                 295                 300

Gly Phe Gly Thr Lys Pro Arg His Ile Thr Pro Phe Thr Ser Phe Gln
305                 310                 315                 320

Ala Val Gln Pro Gln Gln Ser Asn Ala Leu Val Gly Leu Leu Gly Tyr
                325                 330                 335

Ser Ser His Gln Gly Leu Met Gly Phe Gly Thr Ser Pro Ser Pro Ala
            340                 345                 350

Lys Ser Thr Leu Val Glu Ser Arg Cys Cys Arg Asp Leu Met Glu Glu
        355                 360                 365

Lys Phe Asp Gln Val Cys Gln Trp Val Leu Lys Cys Arg Asn Ser Lys
    370                 375                 380

Asn Ser Leu Ile Gln Met Thr Ile Leu Asn Leu Leu Pro Arg Leu Ala
385                 390                 395                 400

Ala Phe Arg Pro Ser Ala Phe Thr Asp Thr Gln Tyr Leu Gln Asp Thr
                405                 410                 415

Met Asn His Val Leu Ser Cys Val Lys Lys Glu Lys Glu Arg Thr Ala
            420                 425                 430

Ala Phe Gln Ala Leu Gly Leu Leu Ser Val Ala Val Arg Ser Glu Phe
        435                 440                 445

Lys Val Tyr Leu Pro Arg Val Leu Asp Ile Ile Arg Ala Ala Leu Pro
    450                 455                 460

Pro Lys Asp Phe Ala His Lys Arg Gln Lys Ala Met Gln Val Asp Ala
465                 470                 475                 480

Thr Val Phe Thr Cys Ile Ser Met Leu Ala Arg Ala Met Gly Pro Gly
                485                 490                 495

Ile Gln Gln Asp Ile Lys Glu Leu Leu Glu Pro Met Leu Ala Val Gly
            500                 505                 510

Leu Ser Pro Ala Leu Thr Ala Val Leu Tyr Asp Leu Ser Arg Gln Ile
        515                 520                 525

Pro Gln Leu Lys Lys Asp Ile Gln Asp Gly Leu Leu Lys Met Leu Ser
    530                 535                 540

Leu Val Leu Met His Lys Pro Leu Arg His Pro Gly Met Pro Lys Gly
545                 550                 555                 560

Leu Ala His Gln Leu Ala Ser Pro Gly Leu Thr Thr Leu Pro Glu Ala
                565                 570                 575

Ser Asp Val Gly Ser Ile Thr Leu Ala Leu Arg Thr Leu Gly Ser Phe
            580                 585                 590

Glu Phe Glu Gly His Ser Leu Thr Gln Phe Val Arg His Cys Ala Asp
        595                 600                 605

His Phe Leu Asn Ser Glu His Lys Glu Ile Arg Met Glu Ala Ala Arg
    610                 615                 620

Thr Cys Ser Arg Leu Leu Thr Pro Ser Ile His Leu Ile Ser Gly His
625                 630                 635                 640

Ala His Val Val Ser Gln Thr Ala Val Gln Val Val Ala Asp Val Leu
                645                 650                 655

Ser Lys Leu Leu Val Val Gly Ile Thr Asp Pro Asp Pro Asp Ile Arg
            660                 665                 670

Tyr Cys Val Leu Ala Ser Leu Asp Glu Arg Phe Asp Ala His Leu Ala
        675                 680                 685

Gln Ala Glu Asn Leu Gln Ala Leu Phe Val Ala Leu Asn Asp Gln Val
    690                 695                 700

Phe Glu Ile Arg Glu Leu Ala Ile Cys Thr Val Gly Arg Leu Ser Ser
705                 710                 715                 720

Met Asn Pro Ala Phe Val Met Pro Phe Leu Arg Lys Met Leu Ile Gln
                725                 730                 735

Ile Leu Thr Glu Leu Glu His Ser Gly Ile Gly Arg Ile Lys Glu Gln
            740                 745                 750

Ser Ala Arg Met Leu Gly His Leu Val Ser Asn Ala Pro Arg Leu Ile
        755                 760                 765

Arg Pro Tyr Met Glu Pro Ile Leu Lys Ala Leu Ile Leu Lys Leu Lys
    770                 775                 780

Asp Pro Asp Pro Asp Pro Asn Pro Gly Val Ile Asn Asn Val Leu Ala
785                 790                 795                 800

Thr Ile Gly Glu Leu Ala Gln Val Ser Gly Leu Glu Met Arg Lys Trp
                805                 810                 815

Val Asp Glu Leu Phe Ile Ile Ile Met Asp Met Leu Gln Asp Ser Ser
            820                 825                 830

Leu Leu Ala Lys Arg Gln Val Ala Leu Trp Thr Leu Gly Gln Leu Val
        835                 840                 845

Ala Ser Thr Gly Tyr Val Val Glu Pro Tyr Arg Lys Tyr Pro Thr Leu
    850                 855                 860

Leu Glu Val Leu Leu Asn Phe Leu Lys Thr Glu Gln Asn Gln Gly Thr
865                 870                 875                 880

Arg Arg Glu Ala Ile Arg Val Leu Gly Leu Leu Gly Ala Leu Asp Pro
                885                 890                 895

Tyr Lys His Lys Val Asn Ile Gly Met Ile Asp Gln Ser Arg Asp Ala
            900                 905                 910

Ser Ala Val Ser Leu Ser Glu Ser Lys Ser Ser Gln Asp Ser Ser Asp

        915                 920                 925

Tyr Ser Thr Ser Glu Met Leu Val Asn Met Gly Asn Leu Pro Leu Asp
    930                 935                 940

Glu Phe Tyr Pro Ala Val Ser Met Val Ala Leu Met Arg Ile Phe Arg
945                 950                 955                 960

Asp Gln Ser Leu Ser His His His Thr Met Val Val Gln Ala Ile Thr
                965                 970                 975

Phe Ile Phe Lys Ser Leu Gly Leu Lys Cys Val Gln Phe Leu Pro Gln
            980                 985                 990

Val Met Pro Thr Phe Leu Asn Val Ile Arg Val Cys Asp Gly Ala Ile
        995                 1000                1005

Arg Glu Phe Leu Phe Gln Gln Leu Gly Met Leu Val Ser Phe Val
    1010                1015                1020

Lys Ser His Ile Arg Pro Tyr Met Asp Glu Ile Val Thr Leu Met
    1025                1030                1035

Arg Glu Phe Trp Val Met Asn Thr Ser Ile Gln Ser Thr Ile Ile
    1040                1045                1050

Leu Leu Ile Glu Gln Ile Val Val Ala Leu Gly Gly Glu Phe Lys
    1055                1060                1065

Leu Tyr Leu Pro Gln Leu Ile Pro His Met Leu Arg Val Phe Met
    1070                1075                1080

His Asp Asn Ser Pro Gly Arg Ile Val Ser Ile Lys Leu Leu Ala
    1085                1090                1095

Ala Ile Gln Leu Phe Gly Ala Asn Leu Asp Asp Tyr Leu His Leu
    1100                1105                1110

Leu Leu Pro Pro Ile Val Lys Leu Phe Asp Ala Pro Glu Ala Pro
    1115                1120                1125

Leu Pro Ser Arg Lys Ala Ala Leu Glu Thr Val Asp Arg Leu Thr
    1130                1135                1140

Glu Ser Leu Asp Phe Thr Asp Tyr Ala Ser Arg Ile Ile His Pro
    1145                1150                1155

Ile Val Arg Thr Leu Asp Gln Ser Pro Glu Leu Arg Ser Thr Ala
    1160                1165                1170

Met Asp Thr Leu Ser Ser Leu Val Phe Gln Leu Gly Lys Lys Tyr
    1175                1180                1185

Gln Ile Phe Ile Pro Met Val Asn Lys Val Leu Val Arg His Arg
    1190                1195                1200

Ile Asn His Gln Arg Tyr Asp Val Leu Ile Cys Arg Ile Val Lys
    1205                1210                1215

Gly Tyr Thr Leu Ala Asp Glu Glu Glu Asp Pro Leu Ile Tyr Gln
    1220                1225                1230

His Arg Met Leu Arg Ser Gly Gln Gly Asp Ala Leu Ala Ser Gly
    1235                1240                1245

Pro Val Glu Thr Gly Pro Met Lys Lys Leu His Val Ser Thr Ile
    1250                1255                1260

Asn Leu Gln Lys Ala Trp Gly Ala Ala Arg Arg Val Ser Lys Asp
    1265                1270                1275

Asp Trp Leu Glu Trp Leu Arg Arg Leu Ser Leu Glu Leu Leu Lys
    1280                1285                1290

Asp Ser Ser Ser Pro Ser Leu Arg Ser Cys Trp Ala Leu Ala Gln
    1295                1300                1305

Ala Tyr Asn Pro Met Ala Arg Asp Leu Phe Asn Ala Ala Phe Val
    1310                1315                1320

Ser Cys Trp Ser Glu Leu Asn Glu Asp Gln Gln Asp Glu Leu Ile
    1325                1330                1335

Arg Ser Ile Glu Leu Ala Leu Thr Ser Gln Asp Ile Ala Glu Val
    1340                1345                1350

Thr Gln Thr Leu Leu Asn Leu Ala Glu Phe Met Glu His Ser Asp
    1355                1360                1365

Lys Gly Pro Leu Pro Leu Arg Asp Asp Asn Gly Ile Val Leu Leu
    1370                1375                1380

Gly Glu Arg Ala Ala Lys Cys Arg Ala Tyr Ala Lys Ala Leu His
    1385                1390                1395

Tyr Lys Glu Leu Glu Phe Gln Lys Gly Pro Thr Pro Ala Ile Leu
    1400                1405                1410

Glu Ser Leu Ile Ser Ile Asn Asn Lys Leu Gln Gln Pro Glu Ala
    1415                1420                1425

Ala Ala Gly Val Leu Glu Tyr Ala Met Lys His Phe Gly Glu Leu
    1430                1435                1440

Glu Ile Gln Ala Thr Trp Tyr Glu Lys Leu His Glu Trp Glu Asp
    1445                1450                1455

Ala Leu Val Ala Tyr Asp Lys Lys Met Asp Thr Asn Lys Asp Asp
    1460                1465                1470

Pro Glu Leu Met Leu Gly Arg Met Arg Cys Leu Glu Ala Leu Gly
    1475                1480                1485

Glu Trp Gly Gln Leu His Gln Gln Cys Cys Glu Lys Trp Thr Leu
    1490                1495                1500

Val Asn Asp Glu Thr Gln Ala Lys Met Ala Arg Met Ala Ala Ala
    1505                1510                1515

Ala Ala Trp Gly Leu Gly Gln Trp Asp Ser Met Glu Glu Tyr Thr
    1520                1525                1530

Cys Met Ile Pro Arg Asp Thr His Asp Gly Ala Phe Tyr Arg Ala
    1535                1540                1545

Val Leu Ala Leu His Gln Asp Leu Phe Ser Leu Ala Gln Gln Cys
    1550                1555                1560

Ile Asp Lys Ala Arg Asp Leu Leu Asp Ala Glu Leu Thr Ala Met
    1565                1570                1575

Ala Gly Glu Ser Tyr Ser Arg Ala Tyr Gly Ala Met Val Ser Cys
    1580                1585                1590

His Met Leu Ser Glu Leu Glu Glu Val Ile Gln Tyr Lys Leu Val
    1595                1600                1605

Pro Glu Arg Arg Glu Ile Ile Arg Gln Ile Trp Trp Glu Arg Leu
    1610                1615                1620

Gln Gly Cys Gln Arg Ile Val Glu Asp Trp Gln Lys Ile Leu Met
    1625                1630                1635

Val Arg Ser Leu Val Val Ser Pro His Glu Asp Met Arg Thr Trp
    1640                1645                1650

Leu Lys Tyr Ala Ser Leu Cys Gly Lys Ser Gly Arg Leu Ala Leu
    1655                1660                1665

Ala His Lys Thr Leu Val Leu Leu Leu Gly Val Asp Pro Ser Arg
    1670                1675                1680

Gln Leu Asp His Pro Leu Pro Thr Val His Pro Gln Val Thr Tyr
    1685                1690                1695

Ala Tyr Met Lys Asn Met Trp Lys Ser Ala Arg Lys Ile Asp Ala
    1700                1705                1710

Phe Gln His Met Gln His Phe Val Gln Thr Met Gln Gln Gln Ala
    1715                1720                1725

Gln His Ala Ile Ala Thr Glu Asp Gln Gln His Lys Gln Glu Leu
    1730                1735                1740

His Lys Leu Met Ala Arg Cys Phe Leu Lys Leu Gly Glu Trp Gln
    1745                1750                1755

Leu Asn Leu Gln Gly Ile Asn Glu Ser Thr Ile Pro Lys Val Leu
    1760                1765                1770

Gln Tyr Tyr Ser Ala Ala Thr Glu His Asp Arg Ser Trp Tyr Lys
    1775                1780                1785

Ala Trp His Ala Trp Ala Val Met Asn Phe Glu Ala Val Leu His
    1790                1795                1800

Tyr Lys His Gln Asn Gln Ala Arg Asp Glu Lys Lys Lys Leu Arg
    1805                1810                1815

His Ala Ser Gly Ala Asn Ile Thr Asn Ala Thr Thr Ala Ala Thr
    1820                1825                1830

Thr Ala Ala Thr Ala Thr Thr Thr Ala Ser Thr Glu Gly Ser Asn
    1835                1840                1845

Ser Glu Ser Glu Ala Glu Ser Thr Glu Asn Ser Pro Thr Pro Ser
    1850                1855                1860

Pro Leu Gln Lys Lys Val Thr Glu Asp Leu Ser Lys Thr Leu Leu

1865                 1870                 1875

Met Tyr Thr Val Pro Ala Val Gln Gly Phe Phe Arg Ser Ile Ser
    1880                 1885                 1890

Leu Ser Arg Gly Asn Asn Leu Gln Asp Thr Leu Arg Val Leu Thr
    1895                 1900                 1905

Leu Trp Phe Asp Tyr Gly His Trp Pro Asp Val Asn Glu Ala Leu
    1910                 1915                 1920

Val Glu Gly Val Lys Ala Ile Gln Ile Asp Thr Trp Leu Gln Val
    1925                 1930                 1935

Ile Pro Gln Leu Ile Ala Arg Ile Asp Thr Pro Arg Pro Leu Val
    1940                 1945                 1950

Gly Arg Leu Ile His Gln Leu Leu Thr Asp Ile Gly Arg Tyr His
    1955                 1960                 1965

Pro Gln Ala Leu Ile Tyr Pro Leu Thr Val Ala Ser Lys Ser Thr
    1970                 1975                 1980

Thr Thr Ala Arg His Asn Ala Ala Asn Lys Ile Leu Lys Asn Met
    1985                 1990                 1995

Cys Glu His Ser Asn Thr Leu Val Gln Gln Ala Met Met Val Ser
    2000                 2005                 2010

Glu Glu Leu Ile Arg Val Ala Ile Leu Trp His Glu Met Trp His
    2015                 2020                 2025

Glu Gly Leu Glu Glu Ala Ser Arg Leu Tyr Phe Gly Glu Arg Asn
    2030                 2035                 2040

Val Lys Gly Met Phe Glu Val Leu Glu Pro Leu His Ala Met Met
    2045                 2050                 2055

Glu Arg Gly Pro Gln Thr Leu Lys Glu Thr Ser Phe Asn Gln Ala
    2060                 2065                 2070

Tyr Gly Arg Asp Leu Met Glu Ala Gln Glu Trp Cys Arg Lys Tyr
    2075                 2080                 2085

Met Lys Ser Gly Asn Val Lys Asp Leu Thr Gln Ala Trp Asp Leu
    2090                 2095                 2100

Tyr Tyr His Val Phe Arg Arg Ile Ser Lys Gln Leu Pro Gln Leu
    2105                2110                2115

Thr Ser Leu Glu Leu Gln Tyr Val Ser Pro Lys Leu Leu Met Cys
    2120                2125                2130

Arg Asp Leu Glu Leu Ala Val Pro Gly Thr Tyr Asp Pro Asn Gln
    2135                2140                2145

Pro Ile Ile Arg Ile Gln Ser Ile Ala Pro Ser Leu Gln Val Ile
    2150                2155                2160

Thr Ser Lys Gln Arg Pro Arg Lys Leu Thr Leu Met Gly Ser Asn
    2165                2170                2175

Gly His Glu Phe Val Phe Leu Leu Lys Gly His Glu Asp Leu Arg
    2180                2185                2190

Gln Asp Glu Arg Val Met Gln Leu Phe Gly Leu Val Asn Thr Leu
    2195                2200                2205

Leu Ala Asn Asp Pro Thr Ser Leu Arg Lys Asn Leu Ser Ile Gln
    2210                2215                2220

Arg Tyr Ala Val Ile Pro Leu Ser Thr Asn Ser Gly Leu Ile Gly
    2225                2230                2235

Trp Val Pro His Cys Asp Thr Leu His Ala Leu Ile Arg Asp Tyr
    2240                2245                2250

Arg Glu Lys Lys Lys Ile Leu Leu Asn Ile Glu His Arg Ile Met
    2255                2260                2265

Leu Arg Met Ala Pro Asp Tyr Asp His Leu Thr Leu Met Gln Lys
    2270                2275                2280

Val Glu Val Phe Glu His Ala Val Asn Asn Thr Ala Gly Asp Asp
    2285                2290                2295

Leu Ala Lys Leu Leu Trp Leu Lys Ser Pro Ser Ser Glu Val Trp
    2300                2305                2310

Phe Asp Arg Arg Thr Asn Tyr Thr Arg Ser Leu Ala Val Met Ser
    2315                2320                2325

Met Val Gly Tyr Ile Leu Gly Leu Gly Asp Arg His Pro Ser Asn
    2330                2335                2340

Leu Met Leu Asp Arg Leu Ser Gly Lys Ile Leu His Ile Asp Phe
    2345                2350                2355

Gly Asp Cys Phe Glu Val Ala Met Thr Arg Glu Lys Phe Pro Glu
    2360                2365                2370

Lys Ile Pro Phe Arg Leu Thr Arg Met Leu Thr Asn Ala Met Glu
    2375                2380                2385

Val Thr Gly Leu Asp Gly Asn Tyr Arg Ile Thr Cys His Thr Val
    2390                2395                2400

Met Glu Val Leu Arg Glu His Lys Asp Ser Val Met Ala Val Leu
    2405                2410                2415

Glu Ala Phe Val Tyr Asp Pro Leu Leu Asn Trp Arg Leu Met Asp
    2420                2425                2430

Thr Asn Thr Lys Gly Asn Lys Arg Ser Arg Thr Arg Thr Asp Ser
    2435                2440                2445

Tyr Ser Ala Gly Gln Ser Val Glu Ile Leu Asp Gly Val Glu Leu
    2450                2455                2460

Gly Glu Pro Ala His Lys Lys Thr Gly Thr Thr Val Pro Glu Ser
    2465                2470                2475

Ile His Ser Phe Ile Gly Asp Gly Leu Val Lys Pro Glu Ala Leu
    2480                2485                2490

Asn Lys Lys Ala Ile Gln Ile Ile Asn Arg Val Arg Asp Lys Leu
    2495                2500                2505

Thr Gly Arg Asp Phe Ser His Asp Asp Thr Leu Asp Val Pro Thr
    2510                2515                2520

Gln Val Glu Leu Leu Ile Lys Gln Ala Thr Ser His Glu Asn Leu
    2525                2530                2535

Cys Gln Cys Tyr Ile Gly Trp Cys Pro Phe Trp
    2540                2545

<210>  141
<211>  4190
<212>  DNA
<213>  Homo sapiens

<220>
<221>  misc_feature
<223>  GNA11

<400>  141
aggttgtccg gcgctgtcgc tcggttgcgg cggctgcggt tggcggtggc tgcggcggcg      60

gcgcgggctg agtgcggccg cgcgggagtc cgcggctggc gcggcccgag cggggacccg     120

gcggctcgcc aggcggcggc cgaggcgggg cgggccggcc cggggccgag ggccggtggc     180

cgaggccgga gggccgcggc gggcggcggc cgaggcggct ccggccaggg ccgggccggg     240

ggccgggggg cggcggcggg caggcggccg cgtcggccgg ggccgggacg atgactctgg     300

agtccatgat ggcgtgttgc ctgagcgatg aggtgaagga gtccaagcgg atcaacgccg     360

agatcgagaa gcagctgcgg cgggacaagc gcgacgcccg gcgcgagctc aagctgctgc     420

tgctcggcac gggcgagagc gggaagagca cgttcatcaa gcagatgcgc atcatccacg     480

gcgccggcta ctcggaggag gacaagcgcg gcttcaccaa gctcgtctac cagaacatct     540

tcaccgccat gcaggccatg atccgggcca tggagacgct caagatcctc tacaagtacg     600

agcagaacaa ggccaatgcg ctcctgatcc gggaggtgga cgtggagaag gtgaccacct     660

tcgagcatca gtacgtcagt gccatcaaga ccctgtggga ggacccgggc atccaggaat     720

gctacgaccg caggcgcgag taccagctct ccgactctgc caagtactac ctgaccgacg     780

ttgaccgcat cgccaccttg ggctacctgc ccacccagca ggacgtgctg cgggtccgcg     840

tgcccaccac cggcatcatc gagtaccctt tcgacctgga gaacatcatc ttccggatgg     900

tggatgtggg gggccagcgg tcggagcgga ggaagtggat ccactgcttt gagaacgtga     960

catccatcat gtttctcgtc gccctcagcg aatacgacca agtcctggtg gagtcggaca    1020

acgagaaccg gatggaggag agcaaagccc tgttccggac catcatcacc tacccctggt    1080

tccagaactc ctccgtcatc ctcttcctca acaagaagga cctgctggag gacaagatcc    1140

tgtactcgca cctggtggac tacttccccg agttcgatgg tccccagcgg gacgcccagg    1200

cggcgcggga gttcatcctg aagatgttcg tggacctgaa ccccgacagc gacaagatca    1260

tctactcaca cttcacgtgt gccaccgaca cggagaacat ccgcttcgtg ttcgcggccg    1320

tgaaggacac catcctgcag ctcaacctca aggagtacaa cctggtctga gcgcccaggc    1380

ccagggagac gggatggaga cacggggcag gaccttcctt ccacggagcc tgcggctgcc    1440

gggcgggtgg cgctgccgag tccgggccgg ggcctctgcc cgcgggagga gatttttttt    1500

tttcatattt ttaacaaatg gtttttattt cacagttatc aggggatgta catctctccc    1560

tccgtacact tcgcgcacct tctcaccttt tgtcaacggc aaaggcagcc tttttctggc    1620

cttgacttat ggctcgcttt tttctaaaaa aaaaaaaaaa agaaagaaag aaaaaaagca    1680

acgaaacata aaacacacaa gcgccccgtg cccccagtga ctctgggcct cacagagccc    1740

ccgccagcca gcatggggcc ccgccctgca gccagtcacg cgcccccaca ccgcagcccc 1800

ccgtggctgt ccttccaacc ccacgtgctt tttctttctc ctgcccgctt cttttcttca 1860

tcacaaaagg cgtggagact cggagacgga cgtttttccc cttttttaag ttattgacgc 1920

ccagcgcgcc tcgcctcttc acccatcaac gctgtgcttt gcccactgga ctcctgaaga 1980

gggggtgggg ggctccctcg gtcgcccacc ctgggaagtg cctaaccttt tattttattt 2040

tatttttttg aggaaaaaga acgcctgact cacaggttga agaaacaccc tgggccctct 2100

ctcatggccg ggttccccgt ccctctgcag aggctgggaa gggtccccgg gctggagcca 2160

cgggggcttc tctgggctgt gcctccgggg ccaacactgg ctgcttgggg ctgcccgggg 2220

actccagagg gctgcacggc caccctgccc tggctagagc gcaccccacc ggagcccacg 2280

tgggctgggc ggctggaggg atggtccccc ggtgacactg ggagaaaggc cacttggatg 2340

ggggcgtttc tgttttgttc cgctttgtga tgtcaccaat ttggaaacag cgagggtggg 2400

tggggacttt tacagaatat tctcaggtgt gtacccgaga ggcagagaga gggacgtggc 2460

cggcagctct gtgcgtggcc ttgtcccaag cacttgcgcc cgcccccgag cgccgccccc 2520

ggggagcggg aagccagcac tcgcactttg gccaggggcg cgtggaaggt ggtggcaggc 2580

accggcctgg gcagcttcca ggcctggctg gccacgacca cggcccgagg gggagcccgc 2640

caggccacgc cgcactgagc cacagccccg ggggccgcct cccggggccc cttgaggcac 2700

tgaggcaccg agactggttc tccccgagag actcggaagg tggggaacga ggggactgtg 2760

tttggggagg tggctttttc gtctgctgtt gactgaacac tacagcgccc tgtggttccg 2820

ggcttcgcac agctgtccca gggatggatc gcctgtgctg ccttcgcccg ccgccacacc 2880

gggaccctgc acggctgctt ctggcctcga cagatgacaa aagaaacagc cccaaaatac 2940

gaccactcca accagcagtt cccgcctgcc tgcccgccac tgtcaggcct gccctggcct 3000

cctcgtccgc agggctgtct gctggcttct gggggcagaa gagcggggag ccccgtggaa 3060

gggtcagggg agaccaggtc agggcagcta catttctggt gatcagcccc atggggagac 3120

ggggctggcg ggataccccc cccccggctt ccccacacca cttctgtctc acccggaagc 3180

gtcctttttt tgtgccaggt gtctacctaa gagggttggt gccagaagcc ccccatggcg 3240

agtgctgggg cccggcggtg ccctggggga gcagatgggg ccacccctgg cagggccgct 3300

acaacctttt ccagcagcgg agccctctgg ggggcctgtg cttgtggcat ctctgagggc 3360

ctagattgca caaggtgacc tggccgtggc ctgagggtgg agtcgcccag cacgcaggcc 3420

ggggcgctgc ggggctaagt attaggcctt cccagggagg gggcgtgcca agcatcccag 3480

agccgggctg ggaccgccaa aacgtcgtgg cctggatcct ctgggtctga gtgcctgatc 3540

ccctgccccc caaaaaagca gaggtaggtg ttgcaggccc agggcagggg tgcctgcccc 3600

aggagagtcc caggcagtgg ttctcgtgcc agtggcaccc aggggcaagg acagccaacc 3660

cccacccttg ccacgtgtgg ggccacgtgg gcatgtgggg tgtgtgtttt taccttggtg 3720

aatctcacct gccaacgatt tctcgtgagt gccgaccacc ttctccgacc atgttacgcc 3780

cgggcggcag cagcccccgg ccactgcaaa cccatgccct gggtcccccg gctcccccag 3840

ggaggcatcc ccgtgccaat gtcccccagt ggtggcagca gatcctgtgg ccggcctggc 3900

ggacgggacc cagtgatact tgtatattac acagtcctga tttcagacaa tttcaacctt 3960

aatctattta aaaaagaata ttctatacaa gctgttttta agccttttac catttgaaat 4020

gcatgtgttg tgcgcgttgg ggatgggagg aggggctgag gagcggctca gtgtcacctc 4080

ccacagccac cggccctgac ccttaatcca gacaccgatg gaagtcgact tttcatatct 4140

ttctcctgaa atgaactctg ttttaaattg gaataaattt tgttcctaaa 4190

<210> 142
<211> 359
<212> PRT
<213> Homo sapiens

<220>
<221> MISC_FEATURE
<223> GNA11, wild type

<400> 142

<210>  143
<211>  6870

<212>  DNA
<213>  Homo sapiens

<220>
<221>  misc_feature
<223>  GNAQ, wild type

<400>  143
gttgccgggc gggacgggaa ccgggtctgc gcgcgagccg ggcggtgggc gcggacaggg      60

ccgagggcgc ccgggagccg tgtcaggcgg cggcgaggag cgggcgcgcc gggcgcgggg     120

agccgtcggc ggccgctcgc tgcggggaca ccccggtgaa tgagctctcg ccgccaggcg     180

cacggcgtag gggagcctcg caggcggcgg cggcggcggc ggctcggggg gcgaggcggg     240

ccgcctgttc ccggggaagg aggcgcgggc gtctgagcga ggccgggcgc ggcggccttc     300

tctcacgcgc cccccgagcc agcgcccgcc tcccgcgccc gcctctcttc tcccgtcggc     360

ccagactatc cgctcccacc gcgcccccgg cccacctggt ggccccggcc ctggccgccg     420

cccccgcggc ggttcccgga gctcgtcccg gacgcgcgcc cgggcggcgg gggctcggcg     480

gccaccgctg cctcggggga gcgagggcgg gagggtgtgt gtgcgcgctg tgagcagggg     540

gtgccggcgg ggctgcagcg gaggcacttt ggaagaatga ctctggagtc catcatggcg     600

tgctgcctga gcgaggaggc caaggaagcc cggcggatca acgacgagat cgagcggcag     660

ctccgcaggg acaagcggga cgcccgccgg gagctcaagc tgctgctgct cgggacagga     720

gagagtggca agagtacgtt tatcaagcag atgagaatca tccatgggtc aggatactct     780

gatgaagata aaagggcttt caccaagctg gtgtatcaga acatcttcac ggccatgcag     840

gccatgatca gagccatgga cacactcaag atcccataca agtatgagca caataaggct     900

catgcacaat tagttcgaga agttgatgtg gagaaggtgt ctgcttttga gaatccatat     960

gtagatgcaa taaagagttt atggaatgat cctggaatcc aggaatgcta tgatagacga    1020

cgagaatatc aattatctga ctctaccaaa tactatctta atgacttgga ccgcgtagct    1080

gaccctgcct acctgcctac gcaacaagat gtgcttagag ttcgagtccc caccacaggg    1140

atcatcgaat acccctttga cttacaaagt gtcattttca gaatggtcga tgtagggggc    1200

caaaggtcag agagaagaaa atggatacac tgctttgaaa atgtcacctc tatcatgttt    1260

ctagtagcgc ttagtgaata tgatcaagtt ctcgtggagt cagacaatga gaaccgaatg    1320

gaggaaagca aggctctctt tagaacaatt atcacatacc cctggttcca gaactcctcg    1380

gttattctgt tcttaaacaa gaaagatctt ctagaggaga aaatcatgta ttcccatcta    1440

gtcgactact tcccagaata tgatggaccc cagagagatg cccaggcagc ccgagaattc    1500

attctgaaga tgttcgtgga cctgaaccca gacagtgaca aaattatcta ctcccacttc    1560

acgtgcgcca cagacaccga gaatatccgc tttgtctttg ctgccgtcaa ggacaccatc    1620

ctccagttga acctgaagga gtacaatctg gtctaattgt gcctcctaga cacccgccct 1680

gcccttccct ggtgggctat tgaagataca caagagggac tgtatttctg tggaaaacaa 1740

tttgcataat actaatttat tgccgtcctg gactctgtgt gagcgtgtcc acagagtttg 1800

tagtaaatat tatgatttta tttaaactat tcagaggaaa aacagaggat gctgaagtac 1860

agtcccagca catttcctct ctatcttttt tttaggcaaa accttgtgac tcagtgtatt 1920

ttaaattctc agtcatgcac tcacaaagat aagacttgtt tctttctgtc tctctctctt 1980

tttcttttct atggagcaaa acaaagctga tttccctttt ttcttccccc gctaattcat 2040

acctccctcc tgatgttttt cccaggttac aatggccttt atcctagttc cattcttggt 2100

caagtttttc tctcaaatga tacagtcagg acacatcgtt cgatttaagc catcatcagc 2160

ttaatttaag tttgtagttt ttgctgaagg attatatgta ttaatactta cggttttaaa 2220

tgtgttgctt tggatacaca catagtttct tttttaatag aatatactgt cttgtctcac 2280

tttggactgg gacagtggat gcccatctaa aagttaagtg tcatttcttt tagatgttta 2340

ccttcagcca tagcttgatt gctcagagaa atatgcagaa ggcaggatca aagacacaca 2400

ggagtccttt cttttgaaat gccacgtgcc attgtctttc ctcccttctt tgcttctttt 2460

tcttaccctc tctttcaatt gcagatgcca aaaaagatgc caacagacac tacattaccc 2520

taatggctgc tacccagaac ctttttatag gttgttctta atttttttgt tgttgttgtt 2580

caagcttttc ctttcttttt tttcttggtg tttgggccac gattttaaaa tgacttttat 2640

tatgggtatg tgttgccaaa gctggctttt tgtcaaataa aatgaatacg aacttaaaaa 2700

ataaaagctg gtatcttaaa atgtaagaga gtaagactgt gaagcctaaa atgactggct 2760

gagaatgaac cagaaatgcc atttgccaaa cagttgtaac tagaaatttg attctcacgg 2820

tccattcttt tctttgtcct taagatgaca ttgttagtgt tcacgtccca tgttcagtgt 2880

ccaaaccggc aatgtaaaaa gtatcctgtg tggtttaaca ggaaatctgt ttatgtctct 2940

ttatttgaaa ccagttttac tctcagtggt tctttaagtt caatgaagtc tgccaggaac 3000

attggttggt agtattattc cgacaccttt aatttccaaa atctgaagtt cctgctagtt 3060

taccaccttc atgatcttct tgaactggta actgattagg ttgaacttat ggaagatttg 3120

tggacttaac tcaaaagtaa cctctcagtg ttctatagaa catgtatttg tgtaactgaa 3180

cctaccagga gaaatgtttg gaattctata tgtgcaattt ttcaacaaat gcaaaaaaaa 3240

tacagcacat gtattgacaa gcttctgtca agcagcttga gttgaaattt gatttaagaa 3300

aataaatcat gattgttcaa agctgctggg acgttagaat taggccatga tactggtctc 3360

attttaacta cagtggtatt tggcactagt gtaaacttcc atataaatca ctcttttgga 3420

acaacaaagg gggagggaga aaaatcacgg cctgttaaat gagtaccaaa gccgcccaac 3480

agtaatgaga tgttctcatc cttgattctc ccagcctcaa acaacacagc ttactttttt 3540

tttcccttgc tcagaaagta cctgtaattt aacaaacaga ctgcctgtag gtatagtgca 3600

attacaaatg ctctaatcat tgtacataca tctctcttga tattgcagca tccatactgg 3660

ctttgtaatc attaattttt tggcagattg aatgtgctgt attgatatgt atctatgtaa 3720

ttgtattgta tgtctatagc taattcacgt tttgaataat gttattttat ttactttttt 3780

aagagaggag aatgtaaatt tgtcagttta tttctgacta gggatatttt ctttccattt 3840

agaaaagaag aaaaaaaaaa aaccttactg tcatacagag cggtactagc gtcgtgctgt 3900

ataaaatcat ttgcacattc ctgagtagag gtatactgat tataagaccc aaaggtaatt 3960

tcatagcaaa atacataaaa tcagtcggag cttttataca aacatggaaa ccaactttgt 4020

agaacttttg ccatttgatc taggattgga atatgagctt ttatacaatt catattctta 4080

tttggcaaat gcacagttta gtattacctc tctgatggcc tttactagaa aggcagtttt 4140

agaagctatt gtgatccact aaggaaatgt tttaacagct agagaccact gcttgcctga 4200

aagggcgttc ttaaatttgg tgcagcaaaa aaaaaaaaaa aaaaaaaaaa aattaaacaa 4260

caacatttga aggcctacag tgtgtataga gaaaacctca tcacaagatc ataagtgtta 4320

cagttttagg gaatcaagat attctattta atagagctat agtaaatgta gtcaattaaa 4380

cctgatctca aagcttgaag aagctgagca aaacagggaa agattgttat atttgtcttt 4440

atgaaattgg gatggaattt gctatgcaga attgaggttt gtggcttcgc tgttcctgta 4500

gggtgcatga caagatccct tctcttgaga aaggaaaaaa ttgatcaccc tagcagcagt 4560

gatgcataga aacctaattt tagccacacc agtcaatcga agctaaagga ttttcttttt 4620

tgtttcttcg ggttttattt gaaggggcta ggggcgggac gggattcttt tcagttttgt 4680

ataaaaacaa agtttactca tgctttatat tatattgtga ttgcaagcgt tataagcgtg 4740

tgccactggc ctcctattgt tgatgcttag gtaatggagg cctgtggtga gttttatggt 4800

gacttgggca tgtcttattc aaaaacaaaa acataaaaca cagaaacctt tcttcagcat 4860

accaaggcaa gcagccattt catgactcac ttaacacatt gcagtgtacc agtttacaga 4920

tgatttttcc ctttttgcgt gacatggcag ttctaacccc cagagaattc cttatttgta 4980

aattggaagt ttctactatg ccttacagag cttaaattca gaagtttgtg cctcatatct 5040

gaaacaaagg gaaataacac acccattcaa aagtaaataa atctcctaga agtttttgtt 5100

tttaacattt ccatataaag agctctgttg aatgtcatga atagactgga aaaaaaaatt 5160

ttaagaacct gcatatgttg tttactagca gatgacaact acaaaaggaa tctgaagaac 5220

acgtaaaact tgtatttttt tttttttggt agattaacta gcaggcctat tttaaaaagg 5280

taattcagct aaagggcaat ttactttttt gtacttcaga ctatcttgat tgtcaaagtg 5340

tacgaactgt aattttaaaa tttatactgc cacatgattg taaattttag ttgtcttaag 5400

ttaggaattg gtgaaaagct atttatgctg gatttgggtc aaaatgactt atttgcaaaa 5460

aaataaataa tgggaagaaa gggctgtata atgaaatact gcaagactca catattggtt 5520

ggaaatttcc ctcaaatcac ctaccgatta cccttgattt ccctttgttt tcagtttctc 5580

aaaacgaatg aaatgaaata tagcagaatg ttaacccata taaaaataaa gtgtacccaa 5640

atattgtaat gtatattgct gctcttcttc aaattaaata agggtttaaa accacttaat 5700

tggtaatcaa catctcaatt gatacaaata aggtgtgctt ggtatacatt aatattttct 5760

tccaaagata tatctttggt tagaaacaca aaaaaataaa actagtaata ttgtatgttt 5820

atctatctct acatatttcc agcatatgta gcgttaatag atctgtcctg gtaactgtgt 5880

ctttgggatt tcattttggt tccatcaaat taggaaaaga aatggcttag ttgtatatga 5940

ttagctagag atttttggag ccagacacct gctgtttagt agataactta gtacagaccc 6000

taaacttgtc atttgttttt ctcacagaat agccatttcc tgctgtcttc ccaatgatca 6060

ctgccctttc aataacactc ttgcctctag aatcatatgt tcaaagtatg aatacacacc 6120

tagcacatag taggtgctca aatattaatt tcctccttgc cttccttatc taccctgtgt 6180

cctccatttc cccgtatgat tccaacccaa tatagcaaat gacatttaca tgttatgaaa 6240

acatctattg ggtaaaatca gatcttggat aaagaaattc tgacttttat ataagctttt 6300

ggtagacaga aaaaacagaa aggtattcgt tggtagaaca tttttaagtt caggaaagaa 6360

agctggaata atactacgta actttgtcca ggttactttg actgaaacac gtttttggtg 6420

gatttctttt cctcaaagaa ctctctaaat gcaactcctt gctggattcc tcacccatca 6480

tcctgttgga aacccttact agacctatgt atttagggag ttttgtcaga aaacattttt 6540

aacttgcagt atttaaaaga atatttactg ttcctaaaat gtcattcaaa tgcatgtact 6600

gtctattgtt tggggatggg aactagtttt gcaaaaaaca cctaatgttg tataataatg 6660

ccccaatgat cttgctggtt aaaaatacag tatttttggc cataattttg tactatagta 6720

ttcttcattg ttgagttttg cttccattaa agttgcccga gcccctctca gaatgttttg 6780

tattagtagg agtactgaaa tacatctttc cttccttaac acaccttgtg catattgcat 6840

tttccaaaat aaacaagttt aatggtttca 6870

<210> 144
<211> 359
<212> PRT
<213> Homo sapiens

<220>
<221> MISC_FEATURE
<223> GNAQ

<400> 144

<210>  145
<211>  5430
<212>  DNA
<213>  Homo sapiens

<220>
<221>  misc_feature
<223>  KRAS, wild type

<400>  145
ctaggcggcg gccgcggcgg cggaggcagc agcggcggcg gcagtggcgg cggcgaaggt      60

ggcggcggct cggccagtac tcccggcccc cgccatttcg gactgggagc gagcgcggcg     120

caggcactga aggcggcggc ggggccagag gctcagcggc tcccaggtgc gggagagagg     180

cctgctgaaa atgactgaat ataaacttgt ggtagttgga gctggtggcg taggcaagag     240

tgccttgacg atacagctaa ttcagaatca ttttgtggac gaatatgatc caacaataga     300

ggattcctac aggaagcaag tagtaattga tggagaaacc tgtctcttgg atattctcga     360

cacagcaggt caagaggagt acagtgcaat gagggaccag tacatgagga ctggggaggg     420

ctttctttgt gtatttgcca taaataatac taaatcattt gaagatattc accattatag     480

agaacaaatt aaaagagtta aggactctga agatgtacct atggtcctag taggaaataa     540

atgtgatttg ccttctagaa cagtagacac aaaacaggct caggacttag caagaagtta     600

tggaattcct tttattgaaa catcagcaaa gacaagacag agagtggagg atgcttttta     660

tacattggtg agagagatcc gacaatacag attgaaaaaa atcagcaaag aagaaaagac     720

tcctggctgt gtgaaaatta aaaaatgcat tataatgtaa tctgggtgtt gatgatgcct 780

tctatacatt agttcgagaa attcgaaaac ataaagaaaa gatgagcaaa gatggtaaaa 840

agaagaaaaa gaagtcaaag acaaagtgtg taattatgta aatacaattt gtactttttt 900

cttaaggcat actagtacaa gtggtaattt ttgtacatta cactaaatta ttagcatttg 960

ttttagcatt acctaatttt tttcctgctc catgcagact gttagctttt accttaaatg 1020

cttattttaa aatgacagtg gaagtttttt tttcctctaa gtgccagtat tcccagagtt 1080

ttggtttttg aactagcaat gcctgtgaaa aagaaactga atacctaaga tttctgtctt 1140

ggggcttttg gtgcatgcag ttgattactt cttatttttc ttaccaattg tgaatgttgg 1200

tgtgaaacaa attaatgaag cttttgaatc atccctattc tgtgttttat ctagtcacat 1260

aaatggatta attactaatt tcagttgaga ccttctaatt ggtttttact gaaacattga 1320

gggaacacaa atttatgggc ttcctgatga tgattcttct aggcatcatg tcctatagtt 1380

tgtcatccct gatgaatgta aagttacact gttcacaaag gttttgtctc ctttccactg 1440

ctattagtca tggtcactct ccccaaaata ttatattttt tctataaaaa gaaaaaaatg 1500

gaaaaaaatt acaaggcaat ggaaactatt ataaggccat ttccttttca cattagataa 1560

attactataa agactcctaa tagcttttcc tgttaaggca gacccagtat gaaatgggga 1620

ttattatagc aaccatttgg gggctatatt tacatgctac taaattttta taataattga 1680

aaagatttta acaagtataa aaaattctca taggaattaa atgtagtctc cctgtgtcag 1740

actgctcttt catagtataa ctttaaatct tttcttcaac ttgagtcttt gaagatagtt 1800

ttaattctgc ttgtgacatt aaaagattat ttgggccagt tatagcttat taggtgttga 1860

agagaccaag gttgcaaggc caggccctgt gtgaaccttt gagctttcat agagagtttc 1920

acagcatgga ctgtgtcccc acggtcatcc agtgttgtca tgcattggtt agtcaaaatg 1980

gggagggact agggcagttt ggatagctca acaagataca atctcactct gtggtggtcc 2040

tgctgacaaa tcaagagcat tgcttttgtt tcttaagaaa acaaactctt ttttaaaaat 2100

tacttttaaa tattaactca aaagttgaga ttttggggtg gtggtgtgcc aagacattaa 2160

tttttttttt aaacaatgaa gtgaaaaagt tttacaatct ctaggtttgg ctagttctct 2220

taacactggt taaattaaca ttgcataaac acttttcaag tctgatccat atttaataat 2280

gctttaaaat aaaaataaaa acaatccttt tgataaattt aaaatgttac ttattttaaa 2340

ataaatgaag tgagatggca tggtgaggtg aaagtatcac tggactagga agaaggtgac 2400

ttaggttcta gataggtgtc ttttaggact ctgattttga ggacatcact tactatccat 2460

ttcttcatgt taaaagaagt catctcaaac tcttagtttt ttttttttac aactatgtaa 2520

tttatattcc atttacataa ggatacactt atttgtcaag ctcagcacaa tctgtaaatt 2580

tttaacctat gttacaccat cttcagtgcc agtcttgggc aaaattgtgc aagaggtgaa 2640

gtttatattt gaatatccat tctcgtttta ggactcttct tccatattag tgtcatcttg 2700

cctccctacc ttccacatgc cccatgactt gatgcagttt taatacttgt aattccccta 2760

accataagat ttactgctgc tgtggatatc tccatgaagt tttcccactg agtcacatca 2820

gaaatgccct acatcttatt tcctcagggc tcaagagaat ctgacagata ccataaaggg 2880

atttgaccta atcactaatt ttcaggtggt ggctgatgct ttgaacatct ctttgctgcc 2940

caatccatta gcgacagtag gatttttcaa acctggtatg aatagacaga accctatcca 3000

gtggaaggag aatttaataa agatagtgct gaaagaattc cttaggtaat ctataactag 3060

gactactcct ggtaacagta atacattcca ttgttttagt aaccagaaat cttcatgcaa 3120

tgaaaaatac tttaattcat gaagcttact tttttttttt ggtgtcagag tctcgctctt 3180

gtcacccagg ctggaatgca gtggcgccat ctcagctcac tgcaacctcc atctcccagg 3240

ttcaagcgat tctcgtgcct cggcctcctg agtagctggg attacaggcg tgtgccacta 3300

cactcaacta atttttgtat ttttaggaga gacggggttt caccctgttg gccaggctgg 3360

tctcgaactc ctgacctcaa gtgattcacc caccttggcc tcataaacct gttttgcaga 3420

actcatttat tcagcaaata tttattgagt gcctaccaga tgccagtcac cacacaaggc 3480

actgggtata tggtatcccc aaacaagaga cataatcccg gtccttaggt agtgctagtg 3540

tggtctgtaa tatcttacta aggcctttgg tatacgaccc agagataaca cgatgcgtat 3600

tttagttttg caaagaaggg gtttggtctc tgtgccagct ctataattgt tttgctacga 3660

ttccactgaa actcttcgat caagctactt tatgtaaatc acttcattgt tttaaaggaa 3720

taaacttgat tatattgttt ttttatttgg cataactgtg attcttttag gacaattact 3780

gtacacatta aggtgtatgt cagatattca tattgaccca aatgtgtaat attccagttt 3840

tctctgcata agtaattaaa atatacttaa aaattaatag ttttatctgg gtacaaataa 3900

acaggtgcct gaactagttc acagacaagg aaacttctat gtaaaaatca ctatgatttc 3960

tgaattgcta tgtgaaacta cagatctttg gaacactgtt taggtagggt gttaagactt 4020

acacagtacc tcgtttctac acagagaaag aaatggccat acttcaggaa ctgcagtgct 4080

tatgagggga tatttaggcc tcttgaattt ttgatgtaga tgggcatttt tttaaggtag 4140

tggttaatta cctttatgtg aactttgaat ggtttaacaa aagatttgtt tttgtagaga 4200

ttttaaaggg ggagaattct agaaataaat gttacctaat tattacagcc ttaaagacaa 4260

aaatccttgt tgaagttttt ttaaaaaaag ctaaattaca tagacttagg cattaacatg 4320

tttgtggaag aatatagcag acgtatattg tatcatttga gtgaatgttc ccaagtaggc 4380

attctaggct ctatttaact gagtcacact gcataggaat ttagaaccta acttttatag 4440

gttatcaaaa ctgttgtcac cattgcacaa ttttgtccta atatatacat agaaactttg 4500

tggggcatgt taagttacag tttgcacaag ttcatctcat ttgtattcca ttgatttttt 4560

ttttcttcta aacatttttt cttcaaacag tatataactt tttttagggg attttttttt 4620

agacagcaaa aactatctga agatttccat ttgtcaaaaa gtaatgattt cttgataatt 4680

gtgtagtaat gttttttaga acccagcagt taccttaaag ctgaatttat atttagtaac 4740

ttctgtgtta atactggata gcatgaattc tgcattgaga aactgaatag ctgtcataaa 4800

atgaaacttt ctttctaaag aaagatactc acatgagttc ttgaagaata gtcataacta 4860

gattaagatc tgtgttttag tttaatagtt tgaagtgcct gtttgggata atgataggta 4920

atttagatga atttagggga aaaaaaagtt atctgcagat atgttgaggg cccatctctc 4980

cccccacacc cccacagagc taactgggtt acagtgtttt atccgaaagt ttccaattcc 5040

actgtcttgt gttttcatgt tgaaaatact tttgcatttt tcctttgagt gccaatttct 5100

tactagtact atttcttaat gtaacatgtt tacctggaat gtattttaac tatttttgta 5160

tagtgtaaac tgaaacatgc acattttgta cattgtgctt tcttttgtgg gacatatgca 5220

gtgtgatcca gttgttttcc atcatttggt tgcgctgacc taggaatgtt ggtcatatca 5280

aacattaaaa atgaccactc ttttaattga aattaacttt taaatgttta taggagtatg 5340

tgctgtgaag tgatctaaaa tttgtaatat ttttgtcatg aactgtacta ctcctaatta 5400

ttgtaatgta ataaaaatag ttacagtgac 5430

<210> 146
<211> 189
<212> PRT
<213> Homo sapiens

<220>
<221> MISC_FEATURE
<223> KRAS, wild type

<400> 146

<210>  147
<211>  4326
<212>  DNA
<213>  Homo sapiens

<220>
<221>  misc_feature
<223>  NRAS, wild type

<400>  147
   ggggccggaa gtgccgctcc ttggtggggg ctgttcatgg cggttccggg gtctccaaca      60
   tttttcccgg ctgtggtcct aaatctgtcc aaagcagagg cagtggagct tgaggttctt     120
   gctggtgtga aatgactgag tacaaactgg tggtggttgg agcaggtggt gttgggaaaa     180
   gcgcactgac aatccagcta atccagaacc actttgtaga tgaatatgat cccaccatag     240
   aggattctta cagaaaacaa gtggttatag atggtgaaac ctgtttgttg gacatactgg     300
   atacagctgg acaagaagag tacagtgcca tgagagacca atacatgagg acaggcgaag     360
   gcttcctctg tgtatttgcc atcaataata gcaagtcatt tgcggatatt aacctctaca     420
   gggagcagat taagcgagta aaagactcgg atgatgtacc tatggtgcta gtgggaaaca     480
   agtgtgattt gccaacaagg acagttgata caaaacaagc ccacgaactg gccaagagtt     540
   acgggattcc attcattgaa acctcagcca agaccagaca gggtgttgaa gatgcttttt     600
   acacactggt aagagaaata cgccagtacc gaatgaaaaa actcaacagc agtgatgatg     660
   ggactcaggg ttgtatggga ttgccatgtg tggtgatgta acaagatact tttaaagttt 720
   tgtcagaaaa gagccacttt caagctgcac tgacaccctg gtcctgactt ccctggagga 780
   gaagtattcc tgttgctgtc ttcagtctca cagagaagct cctgctactt ccccagctct 840
   cagtagttta gtacaataat ctctatttga gaagttctca gaataactac ctcctcactt 900
   ggctgtctga ccagagaatg cacctcttgt tactccctgt tatttttctg ccctgggttc 960
   ttccacagca caaacacacc tctgccaccc caggtttttc atctgaaaag cagttcatgt 1020
   ctgaaacaga gaaccaaacc gcaaacgtga aattctattg aaaacagtgt cttgagctct 1080
   aaagtagcaa ctgctggtga tttttttttt ctttttactg ttgaacttag aactatgcta 1140
   atttttggag aaatgtcata aattactgtt ttgccaagaa tatagttatt attgctgttt 1200
   ggtttgttta taatgttatc ggctctattc tctaaactgg catctgctct agattcataa 1260
   atacaaaaat gaatactgaa ttttgagtct atcctagtct tcacaacttt gacgtaatta 1320
   aatccaactt tcacagtgaa gtgccttttt cctagaagtg gtttgtagac ttcctttata 1380
   atatttcagt ggaatagatg tctcaaaaat ccttatgcat gaaatgaatg tctgagatac 1440
   gtctgtgact tatctaccat tgaaggaaag ctatatctat ttgagagcag atgccatttt 1500
   gtacatgtat gaaattggtt ttccagaggc ctgttttggg gctttcccag gagaaagatg 1560
   aaactgaaag cacatgaata atttcactta ataattttta cctaatctcc acttttttca 1620
   taggttacta cctatacaat gtatgtaatt tgtttcccct agcttactga taaacctaat 1680
   attcaatgaa cttccatttg tattcaaatt tgtgtcatac cagaaagctc tacatttgca 1740
   gatgttcaaa tattgtaaaa ctttggtgca ttgttattta atagctgtga tcagtgattt 1800
   tcaaacctca aatatagtat attaacaaat tacattttca ctgtatatca tggtatctta 1860
   atgatgtata taattgcctt caatcccctt ctcaccccac cctctacagc ttcccccaca 1920
   gcaatagggg cttgattatt tcagttgagt aaagcatggt gctaatggac cagggtcaca 1980
   gtttcaaaac ttgaacaatc cagttagcat cacagagaaa gaaattcttc tgcatttgct 2040
   cattgcacca gtaactccag ctagtaattt tgctaggtag ctgcagttag ccctgcaagg 2100
   aaagaagagg tcagttagca caaacccttt accatgactg gaaaactcag tatcacgtat 2160
   ttaaacattt ttttttcttt tagccatgta gaaactctaa attaagccaa tattctcatt 2220
   tgagaatgag gatgtctcag ctgagaaacg ttttaaattc tctttattca taatgttctt 2280
   tgaagggttt aaaacaagat gttgataaat ctaagctgat gagtttgctc aaaacaggaa 2340
   gttgaaattg ttgagacagg aatggaaaat ataattaatt gatacctatg aggatttgga 2400
   ggcttggcat tttaatttgc agataatacc ctggtaattc tcatgaaaaa tagacttgga 2460
   taacttttga taaaagacta attccaaaat ggccactttg ttcctgtctt taatatctaa 2520
   atacttactg aggtcctcca tcttctatat tatgaatttt catttattaa gcaaatgtca 2580
   tattaccttg aaattcagaa gagaagaaac atatactgtg tccagagtat aatgaacctg 2640
   cagagttgtg cttcttactg ctaattctgg gagctttcac agtactgtca tcatttgtaa 2700
   atggaaattc tgcttttctg tttctgctcc ttctggagca gtgctactct gtaattttcc 2760
   tgaggcttat cacctcagtc atttcttttt taaatgtctg tgactggcag tgattctttt 2820
   tcttaaaaat ctattaaatt tgatgtcaaa ttagggagaa agatagttac tcatcttggg 2880
   ctcttgtgcc aatagccctt gtatgtatgt acttagagtt ttccaagtat gttctaagca 2940
   cagaagtttc taaatggggc caaaattcag acttgagtat gttctttgaa taccttaaga 3000
   agttacaatt agccgggcat ggtggcccgt gcctgtagtc ccagctactt gagaggctga 3060
   ggcaggagaa tcacttcaac ccaggaggtg gaggttacag tgagcagaga tcgtgccact 3120
   gcactccagc ctgggtgaca agagagactt gtctccaaaa aaaaagttac acctaggtgt 3180
   gaattttggc acaaaggagt gacaaactta tagttaaaag ctgaataact tcagtgtggt 3240
   ataaaacgtg gtttttaggc tatgtttgtg attgctgaaa agaattctag tttacctcaa 3300
   aatccttctc tttccccaaa ttaagtgcct ggccagctgt cataaattac atattccttt 3360
   tggttttttt aaaggttaca tgttcaagag tgaaaataag atgttctgtc tgaaggctac 3420
   catgccggat ctgtaaatga acctgttaaa tgctgtattt gctccaacgg cttactatag 3480
   aatgttactt aatacaatat catacttatt acaattttta ctataggagt gtaataggta 3540
   aaattaatct ctattttagt gggcccatgt ttagtctttc accatccttt aaactgctgt 3600
   gaattttttt gtcatgactt gaaagcaagg atagagaaac actttagaga tatgtggggt 3660
   tttttaccca ttccagagct tgtgagcata atcatatttg ctttatattt atagtcatga 3720
   actcctaagt tggcagctac aaccaagaac caaaaaatgg tgcgttctgc ttcttgtaat 3780
   tcatctctgc taataaatta taagaagcaa ggaaaattag ggaaaatatt ttatttggat 3840
   ggtttctata aacaagggac tataattctt gtacattatt tttcatcttt gctgtttctt 3900
   tgagcagtct aatgtgccac acaattatct aaggtatttg ttttctataa gaattgtttt 3960
   aaaagtattc ttgttaccag agtagttgta ttatatttca aaacgtaaga tgatttttaa 4020
   aagcctgagt actgacctaa gatggaattg tatgaactct gctctggagg gaggggagga 4080
   tgtccgtgga agttgtaaga cttttatttt tttgtgccat caaatatagg taaaaataat 4140
   tgtgcaattc tgctgtttaa acaggaacta ttggcctcct tggccctaaa tggaagggcc 4200
   gatattttaa gttgattatt ttattgtaaa ttaatccaac ctagttcttt ttaatttggt 4260
   tgaatgtttt ttcttgttaa atgatgttta aaaaataaaa actggaagtt cttggcttag 4320
   tcataa 4326

<210>  148
<211>  189
<212>  PRT
<213>  Homo sapiens

<220>
<221>  MISC_FEATURE
<223>  NRAS, widl type

<400>  148

Met Thr Glu Tyr Lys Leu Val Val Val Gly Ala Gly Gly Val Gly Lys
1               5                   10                  15

Ser Ala Leu Thr Ile Gln Leu Ile Gln Asn His Phe Val Asp Glu Tyr
            20                  25                  30

Asp Pro Thr Ile Glu Asp Ser Tyr Arg Lys Gln Val Val Ile Asp Gly
        35                  40                  45

Glu Thr Cys Leu Leu Asp Ile Leu Asp Thr Ala Gly Gln Glu Glu Tyr
    50                  55                  60

Ser Ala Met Arg Asp Gln Tyr Met Arg Thr Gly Glu Gly Phe Leu Cys
65                  70                  75                  80

Val Phe Ala Ile Asn Asn Ser Lys Ser Phe Ala Asp Ile Asn Leu Tyr
                85                  90                  95

Arg Glu Gln Ile Lys Arg Val Lys Asp Ser Asp Asp Val Pro Met Val
            100                 105                 110

Leu Val Gly Asn Lys Cys Asp Leu Pro Thr Arg Thr Val Asp Thr Lys
        115                 120                 125

Gln Ala His Glu Leu Ala Lys Ser Tyr Gly Ile Pro Phe Ile Glu Thr
    130                 135                 140

Ser Ala Lys Thr Arg Gln Gly Val Glu Asp Ala Phe Tyr Thr Leu Val
145                 150                 155                 160

Arg Glu Ile Arg Gln Tyr Arg Met Lys Lys Leu Asn Ser Ser Asp Asp
                165                 170                 175

Gly Thr Gln Gly Cys Met Gly Leu Pro Cys Val Val Met
            180                 185

<210>  149
<211>  2393
<212>  DNA

<213>  Homo sapiens

<220>
<221>  misc_feature
<223>  IDH1, wild type

<400>  149
gggctgagga ggcggggcct gggaggggac aaagccggga agaggaaaag ctcggaccta      60

ccctgtggtc ccgggtttct gcagagtcta cttcagaagc ggaggcactg ggagtccggt     120

ttgggattgc caggctgtgg ttgtgagtct gagcttgtga gcggctgtgg cgccccaact     180

cttcgccagc atatcatccc ggcaggcgat aaactacatt cagttgagtc tgcaagactg     240

ggaggaactg gggtgataag aaatctattc actgtcaagg tttattgaag tcaaaatgtc     300

caaaaaaatc agtggcggtt ctgtggtaga gatgcaagga gatgaaatga cacgaatcat     360

ttgggaattg attaaagaga aactcatttt tccctacgtg gaattggatc tacatagcta     420

tgatttaggc atagagaatc gtgatgccac caacgaccaa gtcaccaagg atgctgcaga     480

agctataaag aagcataatg ttggcgtcaa atgtgccact atcactcctg atgagaagag     540

ggttgaggag ttcaagttga aacaaatgtg gaaatcacca aatggcacca tacgaaatat     600

tctgggtggc acggtcttca gagaagccat tatctgcaaa aatatccccc ggcttgtgag     660

tggatgggta aaacctatca tcataggtcg tcatgcttat ggggatcaat acagagcaac     720

tgattttgtt gttcctgggc ctggaaaagt agagataacc tacacaccaa gtgacggaac     780

ccaaaaggtg acatacctgg tacataactt tgaagaaggt ggtggtgttg ccatggggat     840

gtataatcaa gataagtcaa ttgaagattt tgcacacagt tccttccaaa tggctctgtc     900

taagggttgg cctttgtatc tgagcaccaa aaacactatt ctgaagaaat atgatgggcg     960

ttttaaagac atctttcagg agatatatga caagcagtac aagtcccagt ttgaagctca    1020

aaagatctgg tatgagcata ggctcatcga cgacatggtg gcccaagcta tgaaatcaga    1080

gggaggcttc atctgggcct gtaaaaacta tgatggtgac gtgcagtcgg actctgtggc    1140

ccaagggtat ggctctctcg gcatgatgac cagcgtgctg gtttgtccag atggcaagac    1200

agtagaagca gaggctgccc acgggactgt aacccgtcac taccgcatgt accagaaagg    1260

acaggagacg tccaccaatc ccattgcttc catttttgcc tggaccagag ggttagccca    1320

cagagcaaag cttgataaca ataaagagct tgccttcttt gcaaatgctt tggaagaagt    1380

ctctattgag acaattgagg ctggcttcat gaccaaggac ttggctgctt gcattaaagg    1440

tttacccaat gtgcaacgtt ctgactactt gaatacattt gagttcatgg ataaacttgg    1500

agaaaacttg aagatcaaac tagctcaggc caaactttaa gttcatacct gagctaagaa    1560

ggataattgt cttttggtaa ctaggtctac aggtttacat ttttctgtgt tacactcaag    1620

gataaaggca aaatcaattt tgtaatttgt ttagaagcca gagtttatct tttctataag    1680

tttacagcct ttttcttata tatacagtta ttgccacctt tgtgaacatg gcaagggact 1740

tttttacaat ttttatttta ttttctagta ccagcctagg aattcggtta gtactcattt 1800

gtattcactg tcactttttc tcatgttcta attataaatg accaaaatca agattgctca 1860

aaagggtaaa tgatagccac agtattgctc cctaaaatat gcataaagta gaaattcact 1920

gccttcccct cctgtccatg accttgggca cagggaagtt ctggtgtcat agatatcccg 1980

ttttgtgagg tagagctgtg cattaaactt gcacatgact ggaacgaagt atgagtgcaa 2040

ctcaaatgtg ttgaagatac tgcagtcatt tttgtaaaga ccttgctgaa tgtttccaat 2100

agactaaata ctgtttaggc cgcaggagag tttggaatcc ggaataaata ctacctggag 2160

gtttgtcctc tccatttttc tctttctcct cctggcctgg cctgaatatt atactactct 2220

aaatagcata tttcatccaa gtgcaataat gtaagctgaa tcttttttgg acttctgctg 2280

gcctgtttta tttcttttat ataaatgtga tttctcagaa attgatatta aacactatct 2340

tatcttctcc tgaactgttg attttaatta aaattaagtg ctaattacca tta 2393

<210> 150
<211> 414
<212> PRT
<213> Homo sapiens

<220>
<221> MISC_FEATURE
<223> IDH1, wild type

<400> 150

<210>  151
<211>  3745
<212>  DNA
<213>  Artificial Sequence

<220>
<223>  Synthetic - PAN 70

<400>  151
gaggaaccga gaggctgaga ctaacccaga aacatccaat tctcaaactg aagctcgcac      60

tctcgccgcc accatgggga gcagcaagag caagcccaag gaccccagcc agcgcggggg     120

acatttatat gaagaatata cccgcacatc ccaggaaatc caaatgaaaa ggacagctat     180

tgaagcattt aatgaaacca taaaaatatt tgaagaacag tgccagaccc aagagcggta     240

cagcaaagaa tacatagaaa agtttaaacg tgaaggcaat gagaaagaaa tacaaaggat     300

tatgcataat tatgataagt tgaagtctcg aatcagtgaa attattgaca gtagaagaag     360

attggaagaa gacttgaaga agcaggcagc tgagtatcga gaaattgaca aacgtatgaa     420

cagcattaaa ggcggcatca gcggcggcgg cggcggcatc gtagaatgtt tactaccaaa     480

tggaatgata gtgactttag aatgcctccg tgaggctaca ttaataacca taaagcatga     540

actatttaaa gaagcaagaa aataccccct ccatcaactt cttcaagatg aatcttctta     600

cattttcgta agtgttactc aagaagcaga aagggaagaa ttttttgatg aaacaagacg     660

actttgtgac cttcggcttt ttcaaccctt tttaaaagta attgaaccag taggcaaccg     720

tgaagaaaag atcctcaatc gagaaattgg ttttgctatc ggcatgccag tgtgtgaatt     780

tgatatggtt aaagatccag aagtacagga cttccgaaga aatattctga acgtttgtaa     840

agaagctgtg gatcttaggg acctcaattc acctcatagt agagcaatgt atgtctatcc     900

tccaaatgta gaatcttcac cagaattgcc aaagcacata tataataaat tagataaagg     960

gcaaataata gtggtgatct gggtaatagt ttctccaaat aatgacaagc agaagtatac    1020

tctgaaaatc aaccatgact gtgtaccaga acaagtaatt gctgaagcaa tcaggaaaaa    1080

aactcgaagt atgttgctat cctctgaaca actaaaactc tgtgttttag aatatcaggg    1140

caagtatatt ttaaaagtgt gtggatgtga tgaatacttc ctagaaaaat atcctctgag 1200

tcagtataag tatataagaa gctgtataat gcttgggagg atgcccaatt tgatgttgat 1260

ggctaaagaa agcctttatt ctcaactgcc aatggactgt tttacaatgc catcttattc 1320

cagacgcatt tccacagcta caccatatat gaatggagaa acatctacaa aatccctttg 1380

ggttataaat agtgcactca gaataaaaat tctttgtgca acctacgtga atgtaaatat 1440

tcgagacatt gataagatct atgttcgaac aggtatctac catggaggag aacccttatg 1500

tgacaatgtg aacactcaaa gagtaccttg ttccaatccc aggtggaatg aatggctgaa 1560

ttatgatata tacattcctg atcttcctcg tgctgctcga ctttgccttt ccatttgctc 1620

tgttaaaggc cgaaagggtg ctaaagagga acactgtcca ttggcatggg gaaatataaa 1680

cttgtttgat tacacagaca ctctagtatc tggaaaaatg gctttgaatc tttggccagt 1740

acctcatgga ttagaagatt tgctgaaccc tattggtgtt actggatcaa atccaaataa 1800

agaaactcca tgcttagagt tggagtttga ctggttcagc agtgtggtaa agttcccaga 1860

tatgtcagtg attgaagagc atgccaattg gtctgtatcc cgagaagcag gatttagcta 1920

ttcccacgca ggactgagta acagactagc tagagacaat gaattaaggg aaaatgacaa 1980

agaacagctc aaagcaattt ctacacgaga tcctctctct gaaatcactg agcaggagaa 2040

agatttctta tggagtcaca gacactattg tgtaactatc cccgaaattc tacccaaatt 2100

gcttctgtct gttaaatgga attctagaga tgaagtagcc cagatgtatt gcttggtaaa 2160

agattggcct ccaatcaaac ctgaacaggc tatggaactt ctggactgta attacccaga 2220

tcctatggtt cgaggttttg ctgttcggtg cttggaaaaa tatttaacag atgacaaact 2280

ttctcagtat ttaattcagc tagtacaggt cctaaaatat gaacaatatt tggataactt 2340

gcttgtgaga tttttactga agaaagcatt gactaatcaa aggattgggc actttttctt 2400

ttggcattta aaatctgaga tgcacaataa aacagttagc cagaggtttg gcctgctttt 2460

ggagtcctat tgtcgtgcat gtgggatgta tttgaagcac ctgaataggc aagtcgaggc 2520

aatggaaaag ctcattaact taactgacat tctcaaacag gagaagaagg atgaaacaca 2580

aaaggtacag atgaagtttt tagttgagca aatgaggcga ccagatttca tggatgctct 2640

acagggcttt ctgtctcctc taaaccctgc tcatcaacta ggaaacctca ggcttgaaga 2700

gtgtcgaatt atgtcctctg caaaaaggcc actgtggttg aattgggaga acccagacat 2760

catgtcagag ttactgtttc agaacaatga gatcatcttt aaaaatgggg atgatttacg 2820

gcaagatatg ctaacacttc aaattattcg tattatggaa aatatctggc aaaatcaagg 2880

tcttgatctt cgaatgttac cttatggttg tctgtcaatc ggtgactgtg tgggacttat 2940

tgaggtggtg cgaaattctc acactattat gcaaattcag tgcaaaggcg gcttgaaagg 3000

tgcactgcag ttcaacagcc acacactaca tcagtggctc aaagacaaga acaaaggaga 3060

aatatatgat gcagccattg acctgtttac acgttcatgt gctggatact gtgtagctac 3120

cttcattttg ggaattggag atcgtcacaa tagtaacatc atggtgaaag acgatggaca 3180

actgtttcat atagattttg gacacttttt ggatcacaag aagaaaaaat ttggttataa 3240

acgagaacgt gtgccatttg ttttgacaca ggatttctta atagtgatta gtaaaggagc 3300

ccaagaatgc acaaagacaa gagaatttga gaggtttcag gagatgtgtt acaaggctta 3360

tctagctatt cgacagcatg ccaatctctt cataaatctt ttctcaatga tgcttggctc 3420

tggaatgcca gaactacaat cttttgatga cattgcatac attcgaaaga ccctagcctt 3480

agataaaact gagcaagagg ctttggagta tttcatgaaa caaatgaatg atgcacatca 3540

tggtggctgg acaacaaaaa tggattggat cttccacaca attaaacagc atgcattgaa 3600

ctgaggctgc tgcagctgca tgggtgcctg ctgctgcctg ccttggcctg atggccaggc 3660

cagagtgctg ccagtcctct gcatgttctg ctcttgtgcc cttctgagcc cacaataaag 3720

gctgagctct tatcttgcaa aaggc 3745

<210> 152
<211> 1333
<212> DNA
<213> Artificial Sequence

<220>
<223> Synthetic - PAN77

<400> 152
gaggaaccga gaggctgaga ctaacccaga aacatccaat tctcaaactg aagctcgcac 60

tctcgcctcc agcatgggga gcagcaagag caagcccaag gaccccagcc agcgcggggg 120

acatttgcaa ttgaagaggg caaatgtgca aaggagaatg gcccaagcct tccaaaacgt 180

gagggaagaa ccagctgtgc agttcaactc agggactctg gccctaaaca ggaaggtcaa 240

aaacaaccca gatcctacaa tttatccagt gcttgactgg aatgacatca aatttcaaga 300

tgtgattggg gagggcaatt ttggccaagt tcttaaggcg cgcatcaaga aggatgggtt 360

atggatggat gctgccatca aaagaatgaa agaatatgcc tccaaagatg atcacaggga 420

ctttgcagga gaactggaag ttctttgtaa acttggacac catccaaaca tcatcaatct 480

cttaggagca tgtgaacatc gaggctactt gtacctggcc attgagtacg cgccccatgg 540

aaaccttctg gacttcttcc gcaagagccg tgtgctggag acggacccag catttgccat 600

tgccaatagc accgcgtcca cactgtcctc ccagcagctc cttcacttcg ctgccgacgt 660

ggcccggggc atggactact tgagccaaaa acagtttatc cacagggatc tggctgccag 720

aaacatttta gttggtgaaa actatgtggc aaaaatagca gattttggat tgtcccgagg 780

tcaagaggtg tatgtgaaaa agacaatggg aaggctccca gtgcgctgga tggccatcga 840

gtcactgaat tacagtgtgt acacaaccaa cagtgatgta tggtcctatg gtgtgttact 900

atgggagatt gttagcttag gaggcacacc ctactgcggg atgacttgtg cagaactcta 960

cgagaagctg ccccagggct acagactgga gaagcccctg aactgtgatg atgaggtgta 1020

tgatctaatg agacaatgct ggcgggagaa gccttatgag aggccatcat ttgcccagat 1080

attggtgtcc ttaaacagaa tgttagagga gcgaaagacc tacgtgaata ccacgcttta 1140

tgagaagttt acttatgcag gaattgactg ttctgctgaa gaagcggcct agggctgctg 1200

cagctgcatg ggtgcctgct gctgcctgcc ttggcctgat ggccaggcca gagtgctgcc 1260

agtcctctgc atgttctgct cttgtgccct tctgagccca caataaaggc tgagctctta 1320

tcttgcaaaa ggc 1333

<210> 153
<211> 1579
<212> DNA
<213> Artificial Sequence

<220>
<223> Synthetic - PAN78

<400> 153
gaggaaccga gaggctgaga ctaacccaga aacatccaat tctcaaactg aagctcgcac 60

tctcgcctcc agcatgaaag tctctgccgc ccttctgtgc ctgctgctca tagcagccac 120

cttcattccc caagggctcg ctgacctggg cccccagatg ctgagggagc tgcaggagac 180

caacgccgcc ctgcaggacg tgagggagct gctgaggcag caggtgaggg agatcacctt 240

cctgaagaac accgtgatgg agtgcgacgc ctgcggcggc ggcatcagcg gcggcggcgg 300

cggcatcata gccatccttg gctctgctgg aatgacctgc ctgactgtgc tgttggcctt 360

tctgatcata ttgcaattga agagggcaaa tgtgcaaagg agaatggccc aagccttcca 420

aaacgtgagg gaagaaccag ctgtgcagtt caactcaggg actctggccc taaacaggaa 480

ggtcaaaaac aacccagatc ctacaattta tccagtgctt gactggaatg acatcaaatt 540

tcaagatgtg attggggagg gcaattttgg ccaagttctt aaggcgcgca tcaagaagga 600

tgggttatgg atggatgctg ccatcaaaag aatgaaagaa tatgcctcca aagatgatca 660

cagggacttt gcaggagaac tggaagttct ttgtaaactt ggacaccatc caaacatcat 720

caatctctta ggagcatgtg aacatcgagg ctacttgtac ctggccattg agtacgcgcc 780

ccatggaaac cttctggact tcttccgcaa gagccgtgtg ctggagacgg acccagcatt 840

tgccattgcc aatagcaccg cgtccacact gtcctcccag cagctccttc acttcgctgc 900

cgacgtggcc cggggcatgg actacttgag ccaaaaacag tttatccaca gggatctggc 960

tgccagaaac attttagttg gtgaaaacta tgtggcaaaa atagcagatt ttggattgtc 1020

ccgaggtcaa gaggtgtatg tgaaaaagac aatgggaagg ctcccagtgc gctggatggc 1080

catcgagtca ctgaattaca gtgtgtacac aaccaacagt gatgtatggt cctatggtgt 1140

gttactatgg gagattgtta gcttaggagg cacaccctac tgcgggatga cttgtgcaga 1200

actctacgag aagctgcccc agggctacag actggagaag cccctgaact gtgatgatga 1260

ggtgtatgat ctaatgagac aatgctggcg ggagaagcct tatgagaggc catcatttgc 1320

ccagatattg gtgtccttaa acagaatgtt agaggagcga aagacctacg tgaataccac 1380

gctttatgag aagtttactt atgcaggaat tgactgttct gctgaagaag cggcctaggg 1440

ctgctgcagc tgcatgggtg cctgctgctg cctgccttgg cctgatggcc aggccagagt 1500

gctgccagtc ctctgcatgt tctgctcttg tgcccttctg agcccacaat aaaggctgag 1560

ctcttatctt gcaaaaggc 1579